# EUROPEAN PATENT APPLICATION

(11) **EP 3 584 253 A1**
(43) Date of publication of application: **25.12.2019**
(21) Application number: 18178359.8
(22) Date of filing: 18.06.2018
(51) Int. Cl.: C07K 14/415, C12N 15/82

(54) **BALANCED RESISTANCE AND AVIRULENCE GENE EXPRESSION**

(71) Applicant: KWS SAAT SE & Co. KGaA, 37574 Einbeck (DE)
(72) Inventor: STAHL, Dietmar, 37574 Einbeck (DE); GYETVAI, Gabor Miklos, 37085 Göttingen (DE); STIRNWEIS, Daniel Fabian, 37085 Göttingen (DE)

(57) **Abstract**

The invention is related to nucleic acids encoding resistance proteins, avirulence proteins, or both, wherein at least one of the proteins include at least one amino acid substitution in comparison to the wild type amino acid sequence of the resistance and/or avirulence protein, said substitution leading to a decrease of cell death during a hypersensitive reaction. Furthermore, the invention does also encompass an expression cassette, a vector, a cell, a plant, plant tissue or plant seed comprising on of the nucleic acids or one of the proteins. Finaly, the invention provides a method for increasing a plant's resistance towards at least one plant pathogen.

## Description

### Field of the invention

The present invention relates to a nucleic acid for increasing the resistance of a plant or a part thereof towards at least one pathogen, as well as to methods using the nucleic acid.

### Background of the invention

Plants possess a highly efficient, two layered innate immune system that makes them resistant against most microbial pathogens (Jones and Dangl, 2006).

The first layer of defense relies on the recognition of evolutionary conserved pathogen- or microbial-associated molecular patterns (PAMPS or MAMPs) by pattern recognition receptors (PRRs). PAMPs or MAMPs are invariant structures broadly represented among microbial taxa and have essential roles in microbial physiology. Only an extremely select group of molecules have been found to function as PAMPs (Gaudet and Gray-Owen, 2016). PRRs are generally plasma membrane receptors, which are often coupled to intracellular kinase domains or require a co-receptor to provide signaling function (Dangl et al., 2013). Depending on the presence of the signal transduction domain, the plant PRRs are classified either as receptor-like kinases (RLKs) or as receptor-like proteins (RLPs), as described by Macho and Zipfel (2014). Recognition of pathogen-associated molecular patterns (PAMPs) in the apoplast by pattern recognition receptors (PRRs) initiates a complex signaling cascade leading to PRR-triggered immunity (PTI). Adapted pathogens are able to suppress the first defense layer through the secretion of effector proteins that interfere with the signaling (Jones and Dangl, 2006, Césari et al., 2014).

The second layer of plant defense, the effector triggered immunity (ETI), relies on the specific recognition of effectors by disease resistance genes (Jones and Dangl, 2006). This recognition leads to a strong defense response which is often associated with a local programmed cell death, the hypersensitive reaction. Since effectors are generally species- or isolate-specific, this second layer of immunity is only efficient against isolates that carry the recognized effector, which is then called an avirulence gene (Césari et al., 2014). Naturally occurring avirulence gene comprise a signaling sequence allowing for the intracellular processing within the pathogen.

The co-expression of a resistance gene and the corresponding avirulence gene in a plant cell is a promising concept to design genetically modified plants with a broad fungal resistance. The activation of resistance gene dependent defense reactions is frequently associated with cell death, the hallmark of innate immunity.

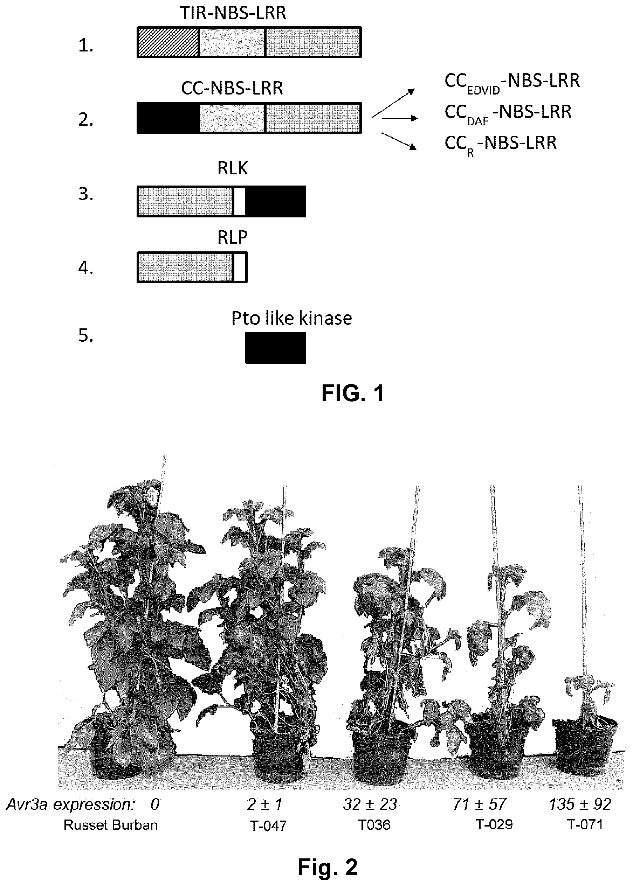

Plant resistance proteins (R-proteins) are classified into five main classes (Fig. 1), based on their structure and localization (Dangl and Jones 2001). Most resistance proteins belong to the nucleotide-binding-site leucine-rich repeat (NBS-LRR) family and are localized intracellularly. LRR domains are found in diverse proteins and function as sites of protein-protein interaction and protein-ligand binding. The recognition specificity of an NBS-LRR R-protein is usually determined by the C-terminal LRR domain. The conserved nucleotide-binding-site (NBS) domain is part of the larger NBS-ARC domain, which functions as a molecular switch which is involved in the Avr-dependent conformation change of the R-protein (Takken et al., 2006).

Depending on the N-terminal domain, NBS-LRR resistance proteins can be subdivided into CC-NBS-LRR and TIR-NBS-LRR proteins. CC-NBS-LRR proteins carry a coiled-coil (CC) domain at their N-terminus, and TIR-NBS-LRR proteins possess an N-terminus with homology to the TOLL/interleukin 1 receptor (TIR), as reviewed by Dangl and Jones, 2001. TIR-NBS-LRR proteins are completely absent from cereal species (McHale et al., 2006). The N-terminal domains are involved in downstream signaling.

The CC-domain of CC-NBS-LRR resistance proteins are classified into 2 groups according Collier and Moffet (2009) and Collier et al. (2011). The majority of CC-domains possesses a small "EDVID" consensus motif and was termed the CC_{EDVID} subtype. The EDVID motif or variations are required for the Avr dependent induction of a hypersensitive reaction as shown by amino acid substitution of the Rx protein (Rairdan *et al.* 2008).

The EDVID motif is absent in the CC_{RPW8} or CC_{R} domain, a less abundant subclass of CC domain. Few CC_{R}-NB-LRR proteins have been cloned to date. The best-studied members of this group include N-required gene 1 (NRG1) of *Nicotiana benthamiana* and activated disease resistance gene 1 (ADR1) of *Arabidopsis thaliana* (Collier et al., 2011). It was assumed that overexpression of an isolated CC_{EDVID} domain is not sufficient for inducing defense responses, unlike the CC_{R} domain of NRG1 or certain TIR domains (Collier et al., 2011). However, a subclass of the CC_{EDVID} domain is sufficient for cell-death induction (WO/2006/128444). This subclass is termed CC_{DAE} or CC_{DAE + EDVID} subtype, based on the presence of a typical conserved DAE motif in addition to the EDVID motif. Following this classification, the CC-NBS-LRR proteins can be divided into 3 subclasses (Fig. 1).

The functionality of a single R-gene is usually restricted to a plant family or even to a species. This phenomenon is named "restricted taxonomic functionality". Heterologous expression of NB-LRR type R genes in a taxonomically distinct family triggers either no response or inappropriate immunity, suggesting that the regulatory or signaling components associated with NB-LRR protein based resistance are family-specific (Brutus et al., 2010). Resistance gene transfer between species may be limited not only by divergence of signaling effector molecules and pathogen avirulence ligands, but potentially also by more fundamental gene expression and transcript processing limitations (Ayliffe et al., 2004).

A break of the restricted taxonomic functionality was reported by the transfer of a pair of NB-LRR-type R-genes, RPS4 and RRS1 (Narusaka et al., 2013) from a *Brassicaceae* plant (*Arabidopsis thaliana*) into two *Solanaceae* plants (*Nicotiana benthamiana, Solanum lycopersicum*)*.* An interfamily transfer from a *Solanaceae* plant (*Solanum lycopersicum*) to a *Brassicaceae* plant (*Arabidopsis thaliana*) was described for the RLP-type R-protein Ve1 (Fradin et al., 2011). However, although Ve1 is considered to encode a race-specific resistance protein, several observations support the hypothesis that Ve1 is an ancient pathogen receptor with traits of typical PRRs (Fradin et al., 2011). It is known that pattern recognition receptors (PRRs) can be transferred across species boundaries, as exemplified by the transfer of the PRRs EF-Tu and FLS2 from *A. thaliana* to *N. benthamiana* and S. *lycopersicum.* Until now, only alleles of certain single NB-LRR R-genes, as well as one two-component NB-LRR resistance gene pair were found to be suitable for an interfamily transfer from the family of Poaceae or *Gramineae* into the plant families of *Solanaceae* and *Brassicaceae.* The barley powdery mildew resistance gene Mla1 triggers cell death in *A. thaliana* after co-expression with the corresponding avirulence gene AVRₐ₁ (Lu et al., 2016). Its wheat orthologs Sr33 and Sr50 trigger cell death when overexpressed in *N. benthamiana* leaves (Cesari et al., 2016). The wheat powdery mildew resistance genes Pm3a and Pm3f trigger cell death after co-expression with the corresponding avirulence gene *AvrPm3*^{*a2*/*f2*} in *N. benthamiana* (Bourras et al., 2015). The avirulence gene AvrPia of *Magnaporthe grisea* was recognized in *N. benthamiana* by co-expression with the two-component NB-LRR resistance gene pair Pia_Rga4/Rga5 from rice. Generally, the distant relationship between monocot plants and dicot plants makes it difficult to predict whether a resistance gene originating from the one group is able to trigger an immune response in the other group.

The NBS-LRR resistance gene R3a of potato was cloned by a comparative genomics approach using synteny between the complex R3a locus of potato and the I2 complex locus of tomato (Huang et al., 2005). To date, R3a function was only shown for two members of the *Solanaceae* family (*Solanum tuberosum, N. benthamiana*). The *Phytophthora infestans* effector Avr3a, the corresponding avirulence gene of R3a, was identified with association genetics. *Avr3a* encodes a protein that is recognized in the host cytoplasm, where it triggers R3a-dependent cell death (Amstrong et al., 2005). The avirulence gene Avr3a occurs in *P*. *infestans* in two forms, the Avr3a_K80I103 (Avr3a^{KI}) or as Avr3a_E80M103 (Avr3a^{EM}) allele. The Avr3a^{KI} allele activates R3a resistance gene dependent innate immunity, whereas Avr3a^{EM} will not be recognized by the R3a gene (Amstrong et al. 2005). Avr3a is essential for the virulence of *P. infestans* and manipulates plant immunity by stabilizing potato E3 ligase CMPG1 (Bos et al., 2010). In addition, the AVR3a protein associates in potato cells with the dynamin-related protein 2 and suppresses defense responses triggered by flg22 and reduce internalization of the activated PRR FLS2 (Chaparro-Garcia et al., 2015).

An alternative approach is the expression of the avirulence gene under the control of a pathogen-inducible promoter. In the presence of a constitutively expressed R-gene, the avirulence gene should only be expressed at the time and site of pathogen infection (De Wit, 1992; Joosten and de Wit, 1999). This strategy was applied to the Cf-9/Avr9 gene pair using a 273 base-pair fragment of the pathogen-inducible promoter Gst1 from potato (Martini et al., 1993). Although preliminary results showed enhanced resistance of tomato against two fungal pathogens, further optimization of the tight promoter regulation was suggested since promoter specificity was not fully restricted to the infection sites (Strittmatter at al., 1996; Joosten and de Wit, 1999).

Some transgenic plants which expressed the p50 avirulence gene under control of the 2xS-2xD pathogen-inducible promoter showed resistance to the tumor-inducing bacterium *Agrobacterium tumefaciens.* The bacteria resistant plants developed minor phenotypes under sterile conditions. However, after transfer to soil, all lines showed spontaneous necrosis of stems and leaves. The necrosis appeared mainly on stems and leaves of older plants. Leaf necrosis started at the tip of the leaf, spread concentrically and finally covered the whole leaf. Stem necrosis started near to soil and the attachment sites of the leaves (Niemeyer 2012).

Another scenario discussed was the dissection of cell death reaction and disease resistance (Niemeyer et al., 2013).

However, until today no agronomically attractive approach solving the underlying problem is available. Therefore, there is still a demand for plants with increased resistance or immunity (especially towards fungal pathogens) which do not show abundant cell death or necrosis.

### Summary of the Invention

The present invention was made in view of the prior art described above, and the object of the present invention is to provide plants having resistance to one or more pathogens while avoiding undesired necrosis or growth retardations as well as to provide a method for the production of these plants and the agriculture application of these plants.

The present invention provides a nucleic acid molecule for increasing the resistance of a plant or a part thereof towards at least one plant pathogen, wherein the nucleic acid molecule comprises a nucleotide sequence selected from the group consisting of:
(a) a nucleotide sequence encoding i) a plant resistance protein of the resistance protein class CC-NBS-LRR and ii) an avirulence protein;
(b) a nucleotide sequence encoding a plant resistance protein of the resistance protein class CC-NBS-LRR;
(c) a nucleotide sequence encoding an avirulence protein;
(d) a nucleotide sequence hybridizing under stringent conditions to a sequence which is complementary to a nucleotide sequence according to (a), (b) or (c); and
(e) a nucleotide sequence encoding a polypeptide comprising an amino acid sequence which is at least 70% identical to an amino acid sequence according to SEQ ID NO: 93;
wherein the encoded resistance protein, the encoded avirulence protein, or both, include at least one amino acid substitution in comparison to the wild type amino acid sequence of the resistance and/or avirulence protein, said substitution leading to a decrease of cell death during a hypersensitive reaction.

A nucleic acid encoding a resistance protein as well as an avirulence protein is an artificial sequence which is manmade and which does not occur in nature. Resistance proteins are of plant origin and avirulence proteins are of plant pathogens origin. Therefore, a nucleic acid sequence encoding a resistance protein and a nucleic acid sequence encoding an avirulence protein are heterologous to each other.

A resistance protein or an avirulence protein comprising one or more amino acid substitutions as defined herein are artificial products which do not occur in nature. Also, the nucleic acids encoding these proteins are artificial and are not found in nature.

The present invention also provides a nucleic acid molecule for increasing the resistance of a plant or a part thereof towards at least one plant pathogen, wherein the nucleic acid molecule comprises a nucleotide sequence selected from the group consisting of:
(a) a nucleotide sequence encoding i) a plant resistance protein of the resistance protein class CC-NBS-LRR and ii) an avirulence protein;
(b) a nucleotide sequence encoding a plant resistance protein of the resistance protein class CC-NBS-LRR;
(c) a nucleotide sequence encoding an avirulence protein;
(d) a nucleotide sequence hybridizing under stringent conditions to a sequence which is complementary to a nucleotide sequence according to (a), (b) or (c); and
(e) a nucleotide sequence encoding a polypeptide comprising an amino acid sequence which is at least 70% identical to an amino acid sequence according to SEQ ID NO: 93;
wherein the encoded resistance protein, the encoded avirulence protein, or both, include at least one amino acid substitution wherein the substitution leads to a decrease of cell death during a hypersensitive reaction in comparison to a resistance protein, avirulence protein or both lacking the amino acid substitution. The at least one amino acid substitution can be a substitution in comparison to the wild type amino acid sequence of the resistance and/or avirulence protein.

In one embodiment, the plant resistance protein is selected from the group consisting of: R3a according to SEQ ID NO 94 as exemplarily encoded by the cDNA of SEQ ID NO 93 or the genomic DNA of SEQ ID NO 268, Rpi-blb3 according to SEQ ID NO 45 as exemplarily encoded by SEQ ID NO 44, R2 according to SEQ ID NO 49 as exemplarily encoded by SEQ ID NO 48, Rpi-abpt according to SEQ ID NO 51 as exemplarily encoded by SEQ ID NO 50, Rpi-edn1 according to SEQ ID NO 53 as exemplarily encoded by SEQ ID NO 52, R1 according to SEQ ID NO 55 as exemplarily encoded by SEQ ID NO 54, Rpi-blb2 according to SEQ ID NO 60 as exemplarily encoded by SEQ ID NO 59, Rpi-blb1 according to SEQ ID NO 65 as exemplarily encoded by SEQ ID NO 64, Rpi-pta1 according to SEQ ID NO 70 as exemplarily encoded by SEQ ID NO 69, Rpi-sto1 according to SEQ ID NO 73 as exemplarily encoded by SEQ ID NO 72, Rpi-vnt1.1 according to SEQ ID NO 76 as exemplarily encoded by SEQ ID NO 75, Rpi-edn2 according to SEQ ID NO 80 as exemplarily encoded by SEQ ID NO 79, R9a according to SEQ ID NO 266, R8 according to SEQ ID NO 86 as exemplarily encoded by SEQ ID NO 85, Rpi-chc1 according to SEQ ID NO 90 as exemplarily encoded by SEQ ID NO 89, R3b according to SEQ ID NO 98 as exemplarily encoded by SEQ ID NO 97, Mla1_barley according to SEQ ID NO 102 as exemplarily encoded by SEQ ID NO 101, Mla13_barley according to SEQ ID NO 106 as exemplarily encoded by SEQ ID NO 105, Rpg1-b_Soybean according to SEQ ID NO 111 as exemplarily encoded by SEQ ID NO 110, RPM1 according to SEQ ID NO 119 as exemplarily encoded by SEQ ID NO 118, RPS2 according to SEQ ID NO 121 as exemplarily encoded by SEQ ID NO 120, RPS5 according to SEQ ID NO 125 as exemplarily encoded by SEQ ID NO 124, BS2 according to SEQ ID NO 129 as exemplarily encoded by SEQ ID NO 128, Rxo1 according to SEQ ID NO 133 as exemplarily encoded by SEQ ID NO 132, I-2_tomato according to SEQ ID NO 137 as exemplarily encoded by SEQ ID NO 136, Pi-ta according to SEQ ID NO 144 as exemplarily encoded by SEQ ID NO 143, Piz-t according to SEQ ID NO 150 as exemplarily encoded by SEQ ID NO 149, Pia Rga4 according to SEQ ID NO 155 as exemplarily encoded by SEQ ID NO 154, Pia Rga5 according to SEQ ID NO 157 as exemplarily encoded by SEQ ID NO 156, Pii-1 according to SEQ ID NO 163 as exemplarily encoded by SEQ ID NO 162, Pik-1 according to SEQ ID NO 167 as exemplarily encoded by SEQ ID NO 166, Pik-2 according to SEQ ID NO 170 as exemplarily encoded by SEQ ID NO 169, Pikh-1 according to SEQ ID NO 173 as exemplarily encoded by SEQ ID NO 172, Pikm1-TS according to SEQ ID NO 179 as exemplarily encoded by SEQ ID NO 178, Pikm2-TS according to SEQ ID NO 182 as exemplarily encoded by SEQ ID NO 181, Pikp-1 according to SEQ ID NO 187 as exemplarily encoded by SEQ ID NO 186, Pikp-2 according to SEQ ID NO 190 as exemplarily encoded by SEQ ID NO 189, Piks-1 according to SEQ ID NO 193 as exemplarily encoded by SEQ ID NO 192, Piks-2 according to SEQ ID NO 196 as exemplarily encoded by SEQ ID NO 195, Rps1k-1_soybean according to SEQ ID NO 199 as exemplarily encoded by SEQ ID NO 198, Rps1k-2_soybean according to SEQ ID NO 205 as exemplarily encoded by SEQ ID NO 204, L3 according to SEQ ID NO 207 as exemplarily encoded by SEQ ID NO 206, N'_tobacco according to SEQ ID NO 211 as exemplarily encoded by SEQ ID NO 210, RX1 according to SEQ ID NO 213 as exemplarily encoded by SEQ ID NO 212, RX2 according to SEQ ID NO 218 as exemplarily encoded by SEQ ID NO 217, Pvr4_pepper according to SEQ ID NO 221, Pvr9_pepper according to SEQ ID NO 225 as exemplarily encoded by SEQ ID NO 224, Tm2_Tomato as exemplarily encoded by SEQ ID NO 228 as exemplarily encoded by SEQ ID NO 227, Tm2^2_Tomato according to SEQ ID NO 232 as exemplarily encoded by SEQ ID NO 231, Tsw_pepper according to SEQ ID NO 234 as exemplarily encoded by SEQ ID NO 233, Sw-5b_tomato according to SEQ ID NO 238 as exemplarily encoded by SEQ ID NO 237, Rsv1_3gG2 according to SEQ ID NO 242 as exemplarily encoded by SEQ ID NO 241, Pm2 according to SEQ ID NO 246 as exemplarily encoded by SEQ ID NO 245, PM3A according to SEQ ID NO 252 as exemplarily encoded by SEQ ID NO 251, and PM3F according to SEQ ID NO 258 as exemplarily encoded by SEQ ID NO 257.

According to one embodiment, the resistance protein is not the potato Rx resistance protein or a mutated form thereof and / or not the tomato Cf-9 resistance protein or a mutated form thereof.

In a specific embodiment, the nucleic acid according to the invention is encoding a resistance protein, wherein the nucleic acid molecule encodes a plant resistance protein having at least one amino acid substitution within the CC-domain in comparison to the amino acid sequence of a wild type CC-domain. Alternatively, the encoded plant resistance protein can include at least one amino acid substitution within the CC-domain wherein the substitution leads to a decrease of cell death during a hypersensitive reaction in comparison to a resistance protein, lacking the amino acid substitution within the CC-domain.

According to a further aspect of the invention the amino acid substitution(s) within the resistance protein or the avirulence protein or both of them is not a substitution / are not substitutions that result(s) in a protein variant which is existing in nature. Therefore, a substitution turning the naturally occuring Avr3a-KI protein (including the signal sequence) to the naturally occuring Avr3a-KM protein (including the signal sequence) or the other way round would be excluded according to this aspect.

In one embodiment, the NBS domain of the resistance protein may comprise an NBS motif.

In one embodiment, the LRR domain of the resistance protein may comprise an Leu-xx-Leu-xx-Leu-x-Leu-xx-Cys/Asn-xx motif according to SEQ ID NO 272 (where x is any amino acid).

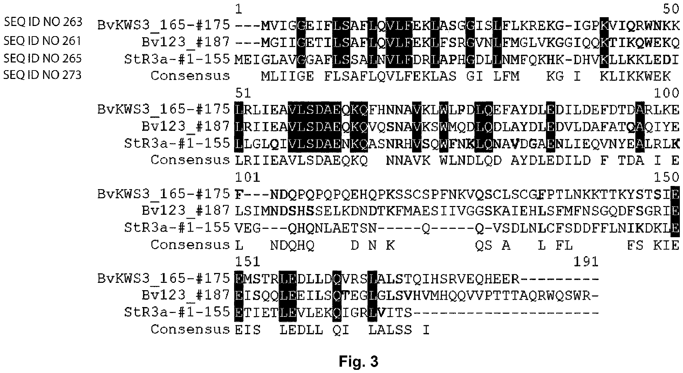

In another embodiment, the resistance protein is a resistance protein which comprises the amino acid motif according to SEQ ID NO 273 as depicted in Fig. 3.

In one embodiment, the avirulence protein is selected from the group consisting of: Avr3a_Phytophthora according to SEQ ID NO 96 as exemplarily encoded by SEQ ID NO 95, Avr2_Phytophthora_infestans according to SEQ ID NO 139 as exemplarily encoded by SEQ ID NO 138, Avr1_Phytophthora_infestans according to SEQ ID NO 58 as exemplarily encoded by SEQ ID NO 57, Avrblb2 according to SEQ ID NO 63 as exemplarily encoded by SEQ ID NO 62, Avr-blb1=Avr-sto1 according to SEQ ID NO 68 as exemplarily encoded by SEQ ID NO 67, Avr-vnt1 according to SEQ ID NO 78 as exemplarily encoded by SEQ ID NO 77, Avrblb2 according to SEQ ID NO 82 as exemplarily encoded by SEQ ID NO 81, Avr8_Phytophthora_infestans according to SEQ ID NO 88 as exemplarily encoded by SEQ ID NO 87, PITG_16245 according to SEQ ID NO 92 as exemplarily encoded by SEQ ID NO 91, Avr3b according to SEQ ID NO 100 as exemplarily encoded by SEQ ID NO 99, Avra1_(AvrMla1)_Blumeria according to SEQ ID NO 104 as exemplarily encoded by SEQ ID NO 103, Avra13_(AvrMla13)_Blumeria according to SEQ ID NO 109 as exemplarily encoded by SEQ ID NO 108, AvrB_Pseudomonas according to SEQ ID NO 113 as exemplarily encoded by SEQ ID NO 112, AvrRpm1 according to SEQ ID NO 115 as exemplarily encoded by SEQ ID NO 114, AvrPpiA1_Pseudomonas according to SEQ ID NO 117 as exemplarily encoded by SEQ ID NO 116, AvrRpt2_Pseudomonas according to SEQ ID NO 123 as exemplarily encoded by SEQ ID NO 122, AvrPphB according to SEQ ID NO 127 as exemplarily encoded by SEQ ID NO 126, AvrBs2_Xanthomonas according to SEQ ID NO 131 as exemplarily encoded by SEQ ID NO 130, AvrRxo1 according to SEQ ID NO 135 as exemplarily encoded by SEQ ID NO 134, Avr2 (SIX3)_Fusarium oxysporum according to SEQ ID NO 139 as exemplarily encoded by SEQ ID NO 138, SIX5_Fusarium oxysporum according to SEQ ID NO 141 as exemplarily encoded by SEQ ID NO 140, AVR Pita according to SEQ ID NO 147 as exemplarily encoded by SEQ ID NO 146, AvrPiz-t according to SEQ ID NO 153 as exemplarily encoded by SEQ ID NO 152, AVR1-CO39 according to SEQ ID NO 159 as exemplarily encoded by SEQ ID NO 158, AVR-Pia according to SEQ ID NO 161 as exemplarily encoded by SEQ ID NO 160, Avr-Pii according to SEQ ID NO 165 as exemplarily encoded by SEQ ID NO 164, Avr Pik_km_kp allele D according to SEQ ID NO 185 as exemplarily encoded by SEQ ID NO 184, Avr1k_Phytophthora sojae according to SEQ ID NO 201 as exemplarily encoded by SEQ ID NO 200, CP_Pepper mild mottle virus according to SEQ ID NO 209 as exemplarily encoded by SEQ ID NO 208, CP_Potato virus X according to SEQ ID NO 216 as exemplarily encoded by SEQ ID NO 215, Nlb_pepper mottle virus according to SEQ ID NO 223 as exemplarily encoded by SEQ ID NO 222, 30kDa MP_Tobacco mosaic virus according to SEQ ID NO 230 as exemplarily encoded by SEQ ID NO 229, NSs_Tomato spotted wilt virus (AvrTsw) according to SEQ ID NO 236 as exemplarily encoded by SEQ ID NO 235, NSm_Tomato spotted wilt virus according to SEQ ID NO 240 as exemplarily encoded by SEQ ID NO 239, P3 cistron_soybean mosaic virus according to SEQ ID NO 244 as exemplarily encoded by SEQ ID NO 243, AvrPm2 according to SEQ ID NO 249 as exemplarily encoded by SEQ ID NO 248, and AvrPm3a2_f2 according to SEQ ID NO 255 as exemplarily encoded by SEQ ID NO 254 wherein preferably at least one of the amino acids of such a protein is substituted.

In a preferred embodiment, the nucleic acid molecule comprises a nucleotide sequence encoding a plant resistance protein of the resistance protein class CC-NBS-LRR and an avirulence protein, wherein the plant resistance protein and the avirulence protein are a combination selected from the following combinations: R3a (CC; AAW48299) and Avr3a_Phytophthora, Rpi-blb3 and Avr2 _Phytophthora_infestans, R2 and Avr2 _Phytophthora_infestans, Rpi-abpt and Avr2 _Phytophthora_infestans, pi-edn1 and Avr2 _Phytophthora_infestans, R1 and Avr1 _Phytophthora_infestans, Rpi-blb2 and Avrblb2, Rpi-blb1 and Avr-blb1=Avr-sto1, Rpi-pta1 and Avr-blb1=Avr-sto1, Rpi-sto1 and Avr-blb1=Avr-sto1, Rpi-vnt1.1 and Avr-vnt1 (PITG_16294)_Phytophthora, Rpi-edn2 and Avrblb2, R9a and Avrblb2, R8 and Avr8_Phytophthora_infestans, Rpi-chc1 and PITG_16245 (Avr-chc1)_Phytophthora, R3b (CC; AEC47890) and Avr3b (PITG_18215)_Phytophthora, Mla1_barley and Avra1_(AvrMla1)_Blumeria, Mla13_barley and Avra13_(AvrMla13)_Blumeria, Rpg1-b_Soybean and AvrB_Pseudomonas, RPM1 (CC; CAA61131) and AvrB_Pseudomonas, RPM1 (CC; CAA61131) and AvrRpm1, RPM1 (CC; CAA61131) and AvrPpiA1_Pseudomonas, RPS2 (CC; AEE85156) and AvrRpt2_Pseudomonas, RPS5 (CC; AEE28852) and AvrPphB (HopAR1)_Pseudomonas, BS2 (CC; AAF09256) and AvrBs2_Xanthomonas, Rxo1 (AAX31149) and AvrRxo1, I-2_tomato and Avr2 (SIX3)_Fusarium oxysporum, I-2_tomato and SIX5_Fusarium oxysporum, Pi-ta (AF207842) and AVR Pita (AF207841), Piz-t (ABC73398) and AvrPiz-t (ACF39937), Pia Rga4 (BAK39922) and AVR1-CO39 (AF463528_3), Pia Rga4 (BAK39922) and AVR-Pia (BAH59484), Pia Rga5 (BAK39930) and AVR1-CO39 (AF463528_3), Pia Rga5 (BAK39930) and AVR-Pia (BAH59484), Pii-1 (BAN59294) and Avr-Pii (BAH59485), Pik-1 (ADZ48537) and Avr Pik_km_kp allele D (allele pex31-D) (BAH59486), Pik-2 (ADZ48538) and Avr Pik_km_kp allele D (allele pex31-D) (BAH59486), Pikh-1 (AET36549) and Avr Pik_km_kp allele D (allele pex31-D) (BAH59486), Pikh-2 (AET36550) and Avr Pik_km_kp allele D (allele pex31-D) (BAH59486), Pikm1-TS (BAG71909) and Avr Pik_km_kp allele D (allele pex31-D) (BAH59486), Pikm2-TS (BAG71908) and Avr Pik_km_kp allele D (allele pex31-D) (BAH59486), Pikp-1 (ADV58352) and Avr Pik_km_kp allele D (allele pex31-D) (BAH59486), Pikp-2 (ADV58351) and Avr Pik_km_kp allele D (allele pex31-D) (BAH59486), Piks-1 (AET36547) and Avr Pik_km_kp allele D (allele pex31-D) (BAH59486), Piks-2 (AET36548) and Avr Pik_km_kp allele D (allele pex31-D) (BAH59486), Rps1k-1_soybean and Avr1k_Phytophthora sojae, Rps1k-2_soybean and Avr1k_Phytophthora sojae, L3 (CC; BAJ33559) and CP_Pepper mild mottle virus (Avr L3&4), N'_tobacco and CP_Pepper mild mottle virus (Avr L3&4), RX1 (CC; CAB50786) and CP_Potato virus X, RX2 (CC; CAB56299) and CP_Potato virus X, Pvr4_pepper and Nlb_pepper mottle virus (Avr Pvr4&9), Pvr9_pepper and Nlb_pepper mottle virus (Avr Pvr4&9), Tm2_Tomato and 30kDa MP_Tobacco mosaic virus (AvrTm2), Tm2^2_Tomato and 30kDa MP_Tobacco mosaic virus (AvrTm2), Tsw_pepper and NSs_Tomato spotted wilt virus (AvrTsw), Sw-5b_tomato and NSm_Tomato spotted wilt virus (AvrSw-5b), Rsv1_3gG2 and P3 cistron_soybean mosaic virus (AvrRsv1_ 3gG2), Pm2 and AvrPm2, PM3A (CC; AAY21626) and AvrPm3a2_f2, and PM3F and AvrPm3a2_f2.

In one embodiment, the nucleic acid molecule comprises a nucleotide sequence encoding the avirulence protein Avr3a lacking the protein signal sequence and having at least one amino acid substitution in the amino acid sequence of SEQ ID NO: 2, wherein the at least one amino acid substitution is selected from the group consisting of: K59/M82, R59/M82, H59/M82, C59/M82, N59/M82, Q59/M82, T59/M82, S59/M82, M59/M82, G59/M82, A59/M82, L59/M82, V59/M82, I59/M82. In this respect an amino acid substitution "R59/M82" means that the amino acid at position 59 is replaced by the amino acid arginine ("R") and the amino acid at position 82 is replaced by methionine ("M"). In this respect the positions 59 and 82 can also correspond to the respective position in a variant of the protein according to SEQ ID NO 2. An example for a sequence encoding a protein according to SEQ ID NO 2 is given by the nucleic acid molecule according to SEQ ID NO 1. Therefore, the following artificial sequences of Avr3a having amino acid substitutions are part of the invention:

| | SEQ ID NO Nucleic acid sequence encoding the protein | SEQ ID NO Protein sequence |
|---|---|---|
| K59/l82 | 1 | 2 |
| K59/M82 | 4 | 5 |
| R59/M82 | 6 | 7 |
| H59/M82 | 8 | 9 |
| D59/M82 | 10 | 11 |
| E59/M82 | 12 | 13 |
| F59/M82 | 14 | 15 |
| Y59/M82 | 16 | 17 |
| W59/M82 | 18 | 19 |
| P59/M82 | 20 | 21 |
| C59/M82 | 22 | 23 |
| N59/M82 | 24 | 25 |
| Q59/M82 | 26 | 27 |
| T59/M82 | 28 | 29 |
| S59/M82 | 30 | 31 |
| M59/M82 | 32 | 33 |
| G59/M82 | 34 | 35 |
| A59/M82 | 36 | 37 |
| L59/M82 | 38 | 39 |
| V59/M82 | 40 | 41 |
| I59/M82 | 42 | 43 |

Avirulence proteins which occur in nature are proteins that are secreted (usually from the pathogen to the infested plant) and therefore naturally occurring avirulence proteins have a signal sequence. Avirulence proteins which lack the signal sequence and the nucleic acids which encode such proteins are manmade and do not occur in nature.

In one embodiment, the nucleic acid molecule comprises a nucleotide sequence encoding the wildtype avirulence protein Avr3a including the signal sequence and having at least one amino acid substitution in the amino acid sequence of SEQ ID NO: 267, wherein the at least one amino acid substitution is selected from the group consisting of: K80/l103 (already included in SEQ ID NO 267), K80/M103, R80/M103, H80/M103, C80/M103, N80/M103, Q80/M103, T80/M103, S80/M103, M80/M103, G80/M103, A80/M103, L80/M103, V80/M103, 180/M103. An example for a sequence encoding a protein according to SEQ ID NO 267 is given by the nucleic acid molecule according to SEQ ID NO 3.

In one embodiment, the nucleic acid molecule comprises a nucleotide sequence encoding a plant resistance protein having at least one amino acid substitution within the CC-domain of the wild type protein. Preferably, the at least one amino acid substitution in the plant resistance protein is selected from the group consisting of: (a) an amino acid substitution within the DAE motif of resistance protein BvKWS3_165 encoded by the nucleic acid sequence according to SEQ ID NO 274 wherein the DAE motif expands from amino acid position 56 to amino acid position 58 in the amino acid sequence according to SEQ ID NO 263,; (b) an amino acid substitution within the DAE motif of resistance protein Bv123 encoded by the nucleic acid sequence according to SEQ ID NO 276 wherein the DAE motif expands from amino acid position 57 to amino acid position 59 of SEQ ID NO: 261, and (c) an amino acid substitution within the DAE motif of resistance protein R3a wherein the DAE motif expands from amino acid position 59 to amino acid position 61 of SEQ ID NO: 265,. The amino acid sequence of SEQ ID NO: 263 is exemplarily encoded by the nucleic acid molecule according to SEQ ID NO: 262. More preferably, the at

least one amino acid substitution is within the D or E of the DAE motif of BvKWS3_165, Bv123, or R3a. The amino acid sequence of SEQ ID NO: 261 is exemplarily encoded by the nucleic acid molecule according to SEQ ID NO: 260. The amino acid sequence of SEQ ID NO: 265 is exemplarily encoded by the nucleic acid molecule according to SEQ ID NO: 264.

In a preferred embodiment, the plant resistance protein is R3a having the wild type amino acid sequence of SEQ ID NO: 269, wherein said wild type amino acid sequence has at least one substitution selected from the group consisting of: K35E, K37M, E61V, E61K, E135N und E136N. In the case of "K35E" this means for example that the amino acid K at position 35 is replaced by E.

Moreover, provided is a cell comprising the nucleic acid molecule, the polypeptide, or the vector or expression cassette of the present invention. Preferably, the cell comprises the nucleic acid molecule of the present invention as a transgene or as an endogene. The cell may be for example an eucaryotic cell like a plant cell or a yeast cell. However, the cell may be for example also a procaryotic cell like a bacterial cell wherein the bacterial cell may be for example a cell of *E.coli* or *Agrobacterium tumefaciens.*

Further provided is a plant or part thereof comprising the nucleic acid molecule of the present invention or comprising the cell of the present invention, and a plant or part thereof regenerated from said cell.

Also provided is a seed or offspring from the plant of the present invention, wherein the seed or the offspring comprises the nucleic acid molecule of the present invention as a transgene or an endogene.

The present invention also provides a method for increasing a plant's resistance towards at least one plant pathogen. In one embodiment, said method comprises the steps of integrating the nucleic acid molecule of the present invention into the genome of at least one cell of a plant and regenerating a plant from that cell. Alternatively, said method comprises the steps of transforming a plant cell with a nucleic acid molecule of the present invention, or the vector or the expression cassette of the present invention, and regenerating a plant from that cell.

Further, the present invention provides a method for the identification of a plant comprising the nucleic acid molecule of the present invention, said method comprising the steps of isolating DNA from the plant of the present invention or part thereof, and performing a polymerase chain reaction using the isolated DNA as a template, thereby amplifying the nucleic acid molecule of the present invention or a part thereof. Preferably the method for identification will detect not only the presence of a nucleic acid molecule according to the present invention but will also detect at least one of the amino acid substitutions encoded by the nucleic acid molecule.

### Brief description of the drawings

Figure 1: Classification of plant resistance genes into five groups (modified after Dangl and Jones, 2001). TIR-NBS-LRR and CC-NBS-LRR proteins are located in the cytoplasm of the plant cells. They have a NBS (nucleotide binding site) and a LRR (leucine rich repeat) domain in common. They differ at the N-terminus by the TIR (Toll/interleukin receptor) and CC (coiled-coil) domain. The CC-NBS-LRR will be subdivided into the CC_{EDVID}-NBS-LRR, CC_{DAE}-NBS-LRR and CC_{R}-NBS-LRR types according this patent disclosure. RLK (receptor-like-kinase) and RLP (receptor-like-proteins) are membrane-bound receptors. A RLK receptor has a LRR at the N-terminus, followed by a transmembrane domain and a kinase domain at the C-terminus. The RLP receptor consists of a LRR domain, followed by a transmembrane domain. The Pto like kinase type consists of a kinase domain.
Figure 2: Detrimental effect of R3a-Avr3a co-expression in adult but not in young plants. The transgenic lines T-047, T-036, T-029 and T-071, which were transformed with the R3a-Avr3a^{KI} genes, showed leaf abortion and stunted growth as adult but not as young plants. Expression analysis by qRT-PCR showed that the Avr3a gene is stronger expressed in young plants than in adult plants. The negative phenotype of adult R3a-Avr3a^{KI} lines correlates with Avr3a^{KI} expression. Avr3a^{KI} expression was normalized against expression of the potato house-keeping gene StMCB75. n=4, ± indicates standard derivation.
Figure 3: Comparison of the CC_{DAE}-domains from the R-proteins BvKWS3_165 according to SEQ ID NO 263 exemplarily encoded by the nucleic acid according to SEQ ID NO 262, Bv123 protein according to SEQ ID NO 261 exemplarily encoded by the nucleic acid according to SEQ ID NO 260 and StR3a protein according to SEQ ID NO 265 exemplarily encoded by the nucleic acid according to SEQ ID NO 264. Identical amino acids of the resistance proteins from sugar beet (BvKWS3_165, Bv123) and potato (StR3a) are highlighted with black background. The DAE motif, which was used for the classification of the CC-type, is highly conserved and located at position 56-58 (BvKWS3_165), 57-59 (Bv123) and 59-61 (StR3a). The EDVID motif is conserved as EDILD consensus sequence and located at position 82-86 (BvKWS3_165), 83-87 (Bv123) and 85-89 (StR3a). The size of the CC-domains is given as numbers, e.g. #175 = 175 amino acids. Also depticed is a consensus motif according to SEQ ID NO 273 deduced from the three sequences.

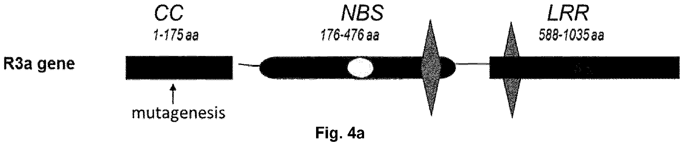

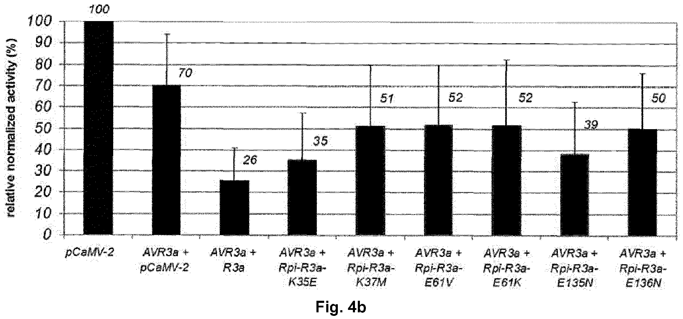

Figure 4a and Figure 4b: Generation of R3a gene variants with reduced cell death activity by single amino acid exchanges of the CC_{DAE}-domain (Fig. 4a). Generated R3a alleles showed reduced cell death after co-expression with the Avr3a^{KI} gene. In each construct (the last six columns), one amino acid of the CC_{DAE}-domain was exchanged in the CC-NBS-LRR protein (e.g. K35E = K at position 35 was replaced by E). The strongest cell death was observed for the native R3a gene (3rd column showing results for "AVR3a + R3a"). The number describe the level of cell death after ballistic transformation of potato leaves (0 = max. cell death, 100 = no cell death detected). pCaMV-2 = empty vector (negative control). The standard derivation of the mean from 3 experiments with 6 replicates is given by error bars (Fig. 4b).

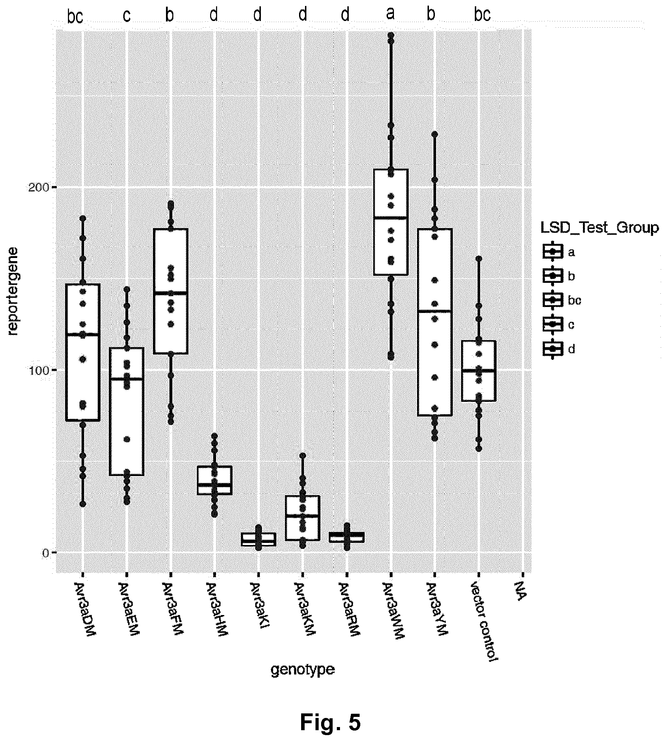

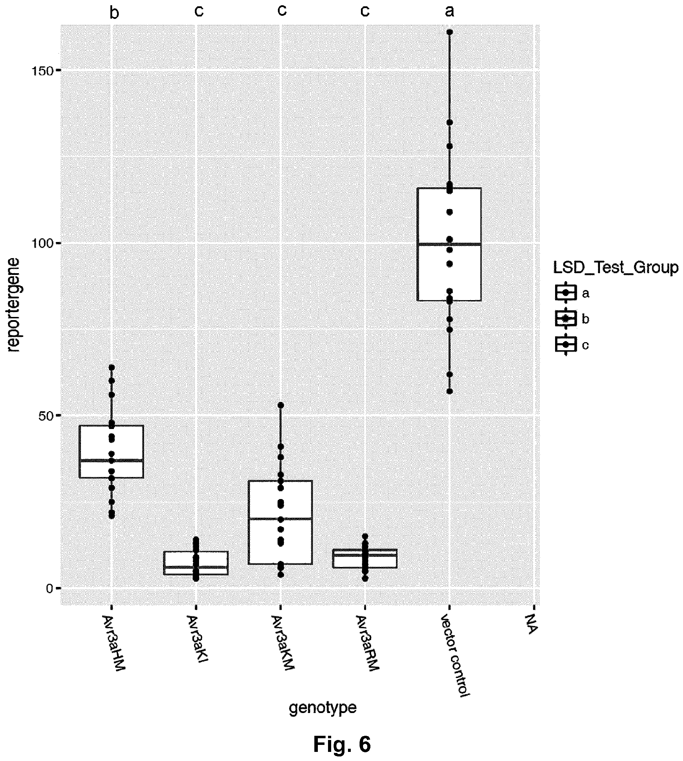

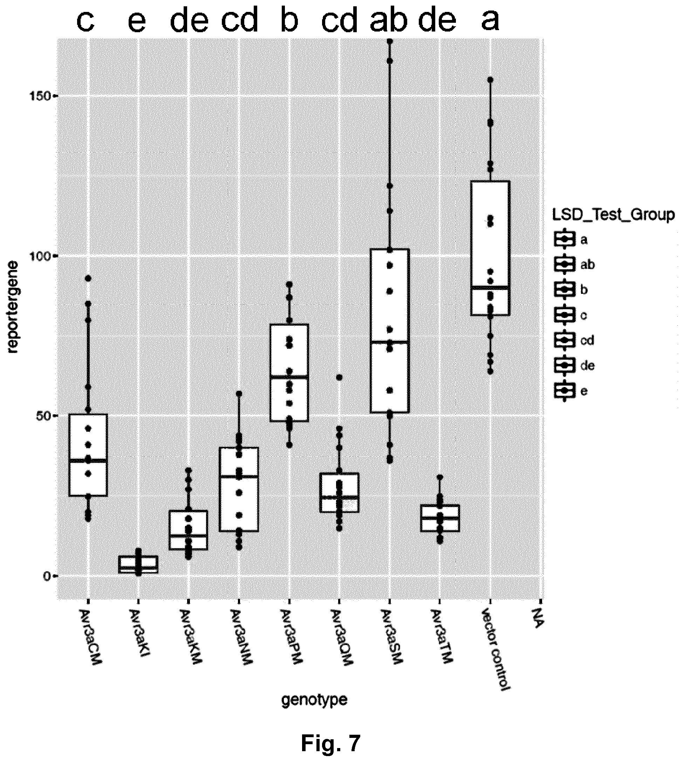

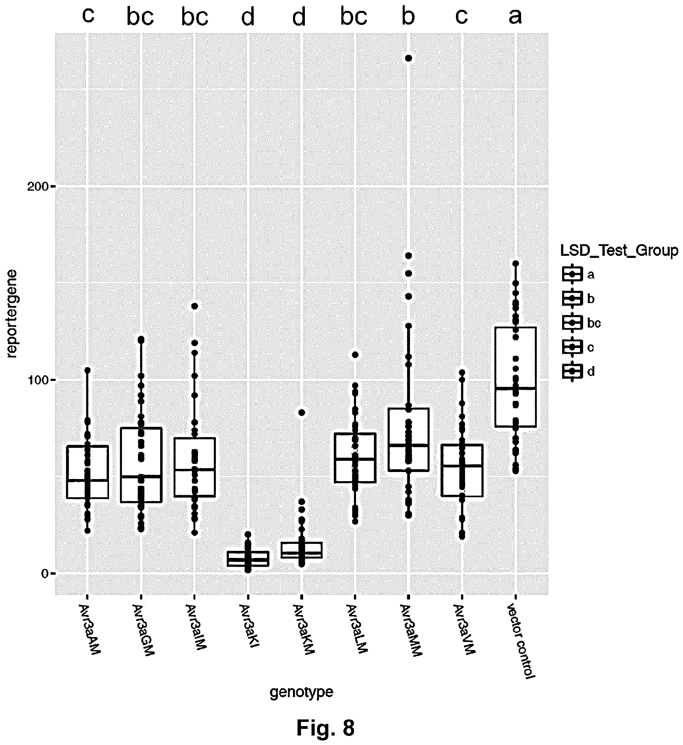

Figure 5 to Figure 8: Allelic variants of the Avr3a gene trigger different levels of cell death in potato leaves. Avr3a variants under the control of the doubled 35S promoter trigger different levels of cell death after transient expression in potato leaves of the R3a cultivar Hermes. The mean of three independent experiments is shown (n=18). Empty vector (expression vector without Avr3a gene) was set to 100, Avr3aKI (native avirulent allele). The Shapiro Wilk test was performed for normality distribution of the data. Data with different letters are significantly different according ANOVA and Tukey test (multiple t-test, p < 0.05).

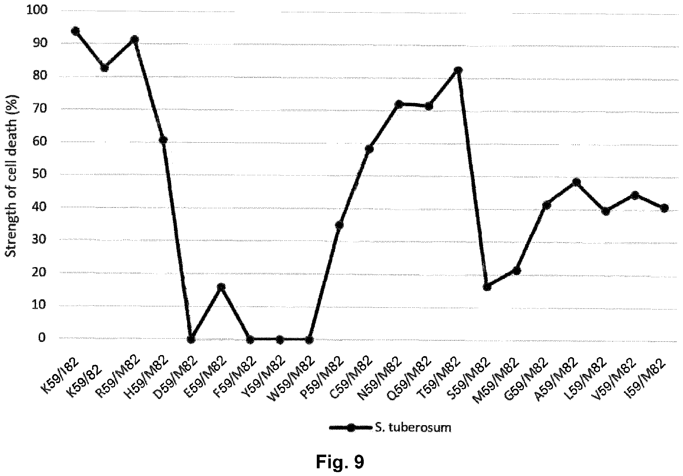

Figure 9: Comparison of cell death strength induced by allelic variants of the Avr3a gene (s. Fig. 5-8) in potato leaves.

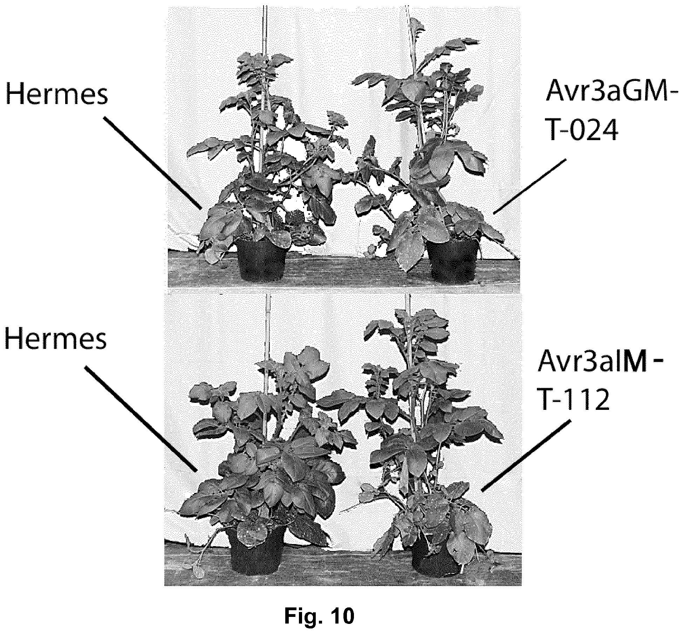

Figure 10: Phenotype of the potato lines Avr3a^{GM-}T-024 and Avr3a^{IM}-T-112 compared to the potato cultivar Hermes. Adult plants of lines transformed with the Avr3a^{GM} and Avr3a^{IM} gene under the control of the synthetic-fungal inducible promoter 2xS-4xD-NpCABE showed the same phenotype as the cultivar Hermes, which was used for transformation.

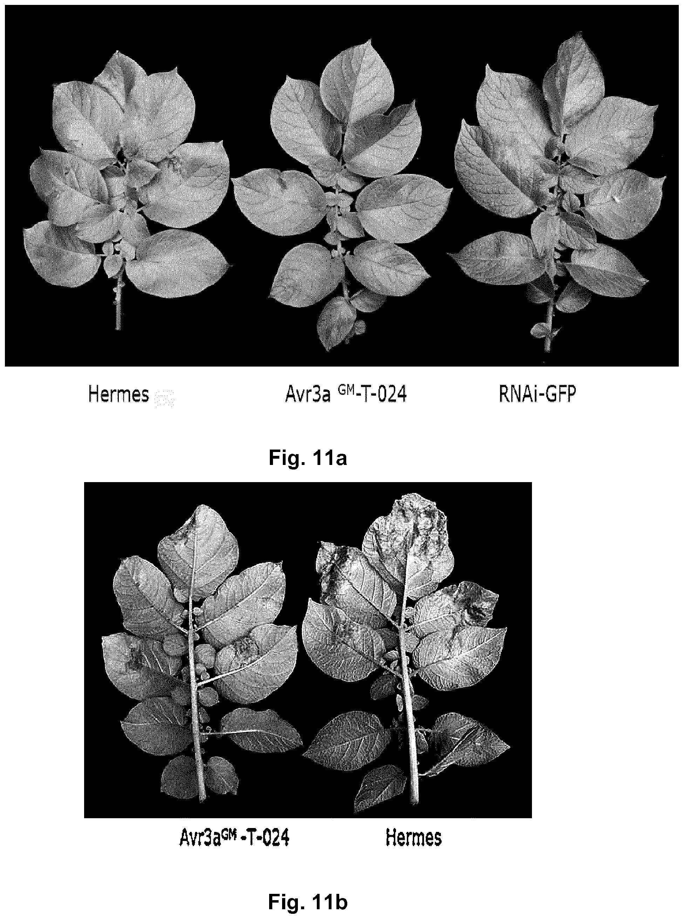

Figure 11a and Figure 11b: Enhanced late blight resistance of line Avr3a^{GM-}T-024 in comparison to Hermes and a transgenic control (RNAi-GFP). The top 5 leaflets of each potato leaf were locally inoculated with one drop of sporangia (20 µl with 600 sporangia). Photos were taken at 5 dpi.

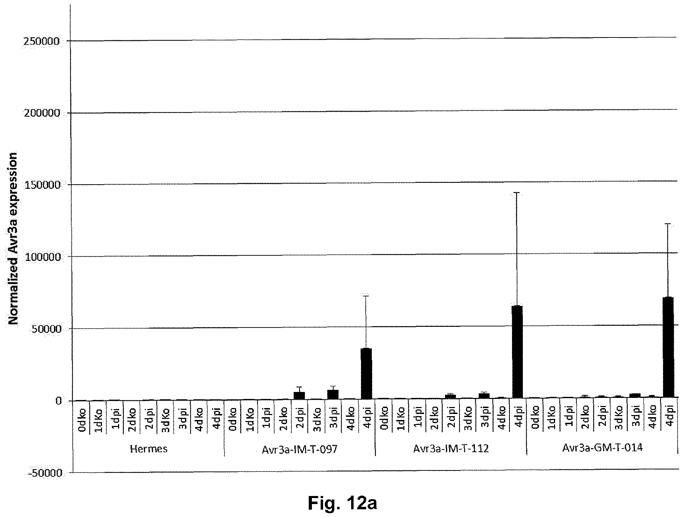

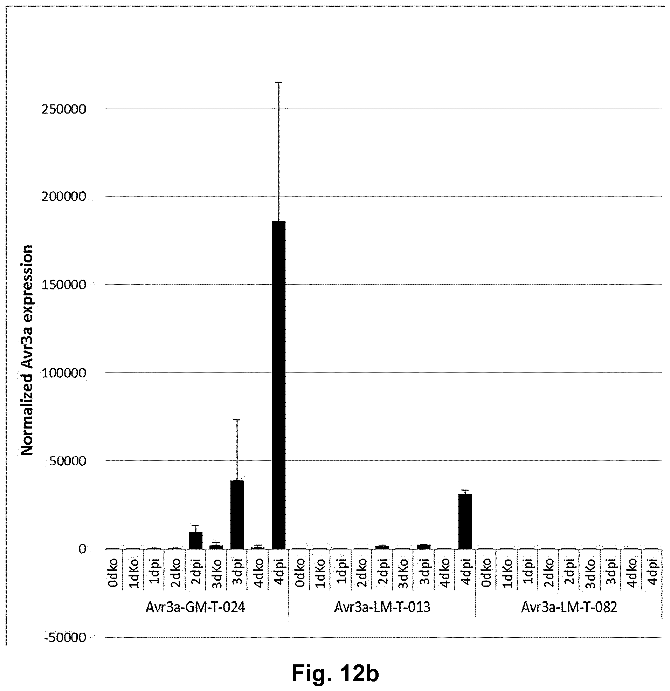

Figure 12a and Figure 12b: Expression analysis of the Avr3a gene in *P. infestans* infected and control leaves of potato by qRT-PCR. The transgenic line with the best late blight resistance, Avr3a^{GM}-T-024, showed the highest expression of the recombinant Avr3a gene after infection at 2, 3 and 4 dpi. Selected primers S3169 and S3170 allowed the specific detection of the recombinant Avr3a gene expression in the presence of P. infestans. Avr3a expression was normalized against the potato gene StMCB75. Black column = infected leaves, grey column = control leaves. n=4. dpi= days after inoculation, ko=non-infected leaves. ± indicates standard derivation.

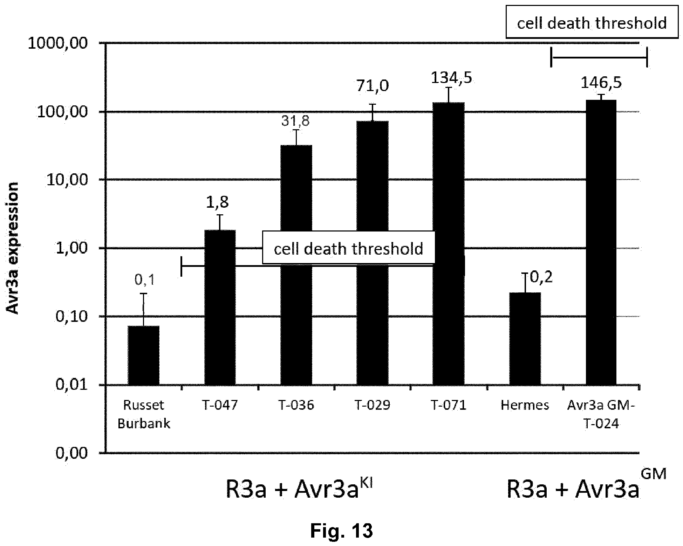

Figure 13: The threshold of cell death induction is increased by application of Avr3a^{GM} in comparison to the native Avr3a^{KI} gene. Expression of the Avr3a^{KI} and Avr3a^{GM} genes in leaves of adult greenhouse plants was determined by qRT-PCR using the gene specific primers S3169 and S3170. The transgenic lines T-047, T-036, T-029 and T-071, which were transformed with the R3a-Avr3a^{KI} genes, showed leaf abortion and stunted growth (see Figure 2). The level of leaf damage correlated with the Avr3a^{KI} expression. Even the Avr3a^{KI} low expressing line T-047 showed visible leaf abortion. This indicated that the threshold for cell death induction is less than 1.8 relative units for the Avr3a^{KI} expressing lines. In contrast, the R3a-Avr3a^{GM} co-expressing line Avr3a^{GM}-T024 did not show a negative phenotype and revealed a Avr3a^{GM} expression of 146.5 ± 30.1 relative units. This result demonstrated that the threshold for cell death induction is greater than 200 relative units for the modified Avr3a gene in line Avr3a^{GM}-T-024. Avr3a expression was normalized against expression of the potato house-keeping gene StMCB75 n=4, ± indicates standard derivation.

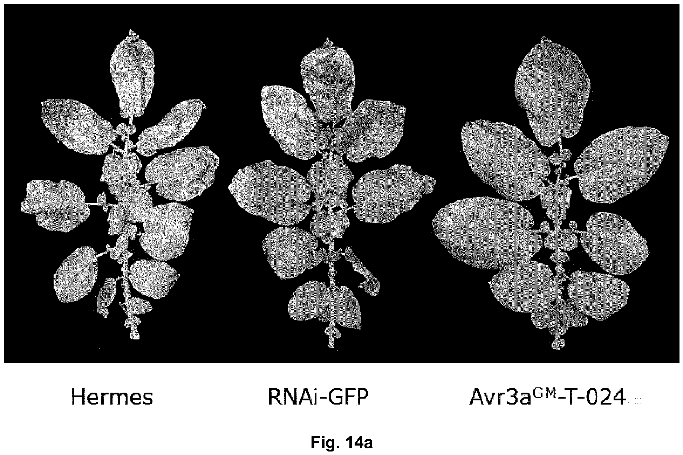

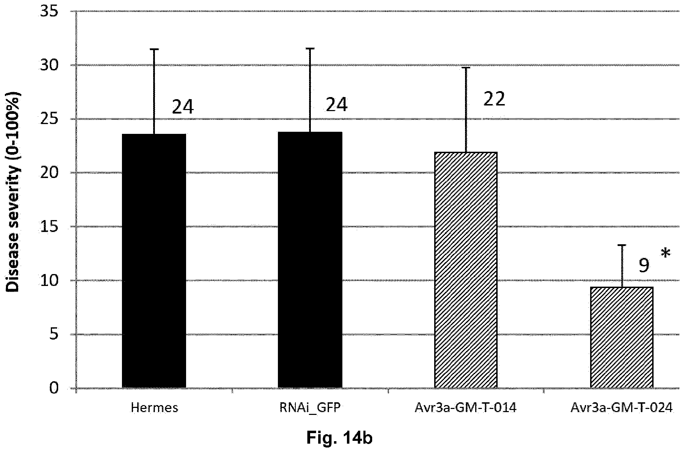

Figure 14a and Figure 14b: Enhanced early blight (*Alternaria solani*) resistance of line Avr3a^{GM-}T-024 in comparison to Hermes and a transgenic control (RNAi-GFP). Greenhouse plants were scored for natural *A. solani* infection (n=8 rows with 3 plants each). 0 = healthy leaves, 100 = completely destroyed leaves. Differences between samples were assessed by using analysis of variance (ANOVA). *Significant different according ANOVA to Hermes, RNAi-GFP and Avr3a^{GM}-T-014, p < 0.05.

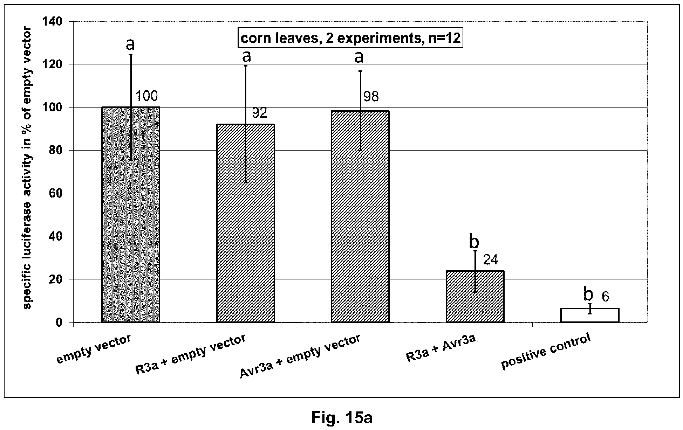

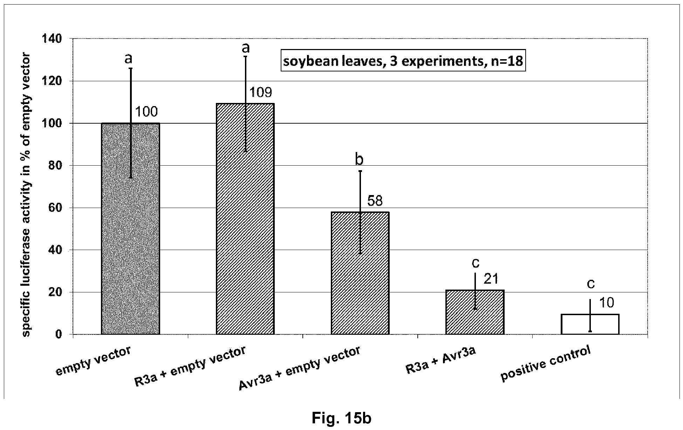

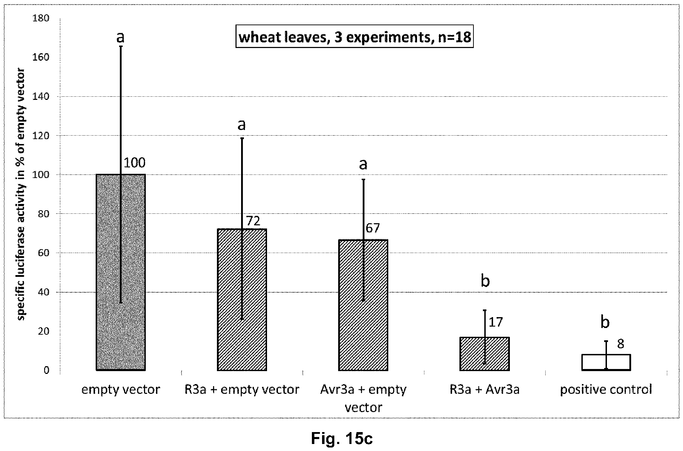

Figure 15a, Figure 15b and Figure 15c: R3a-Avr3a^{KI} co-expression activates cell death, the hallmark of innate immunity, in corn, soybean and wheat leaves. The potato R3a resistance gene and the Avr3a^{KI} gene of *P. infestans* were transiently co-expressed in leaves of corn, soybean and wheat. The induction of cell death was measured by the activity of two co-bombarded luciferase reporter genes 16h after transformation. An auto-activated wheat resistance gene and an auto-activated soybean resistance gene were used as "positive controls" for wheat, corn and soybean.

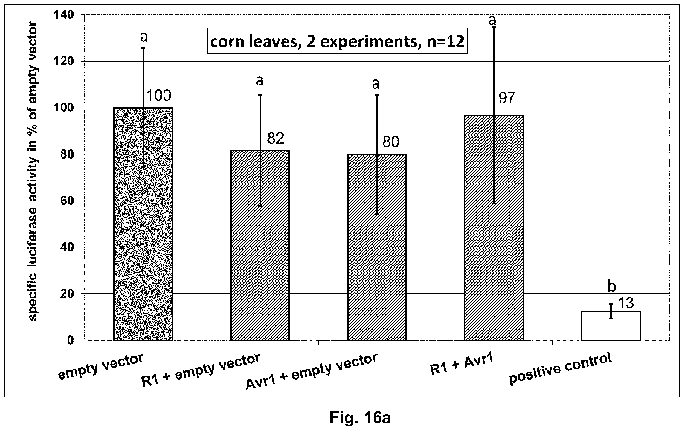

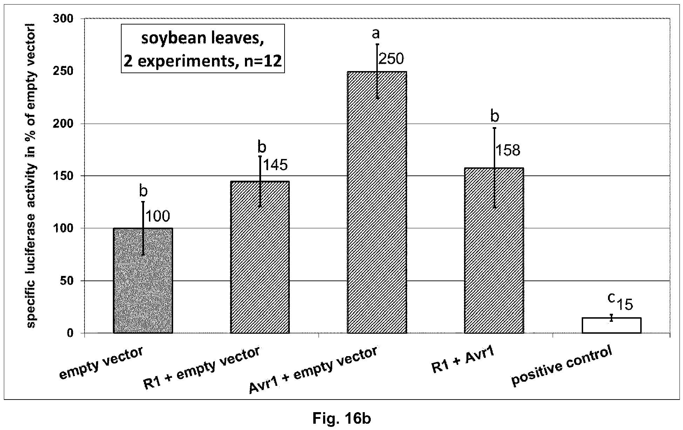

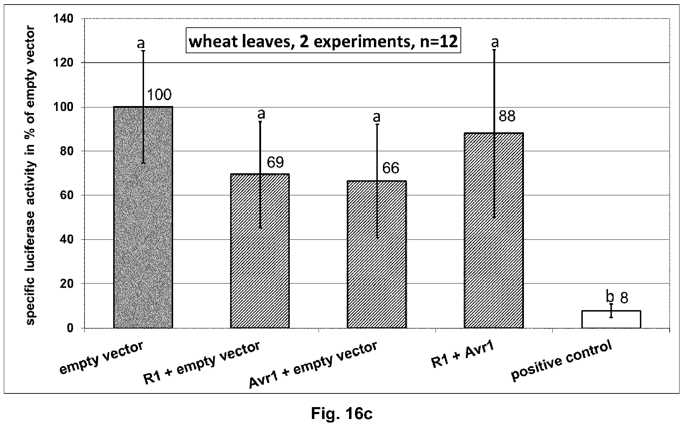

Figure 16a, Figure 16b and Figure 16c: In contrast to R3a-Avr3a^{KI} co-expression, the co-expression of R1-Avr1 does not activate cell death in corn, soybean and wheat leaves. The potato R1 resistance gene and the Avr1 gene of *P. infestans* were transiently co-expressed in leaves of corn, soybean and wheat. The induction of cell death was measured by the activity of two co-bombarded luciferase reporter genes 16h after transformation; an auto-activated wheat resistance gene and an auto-activated soybean resistance gene were used as "positive controls" for wheat, corn and soybean.

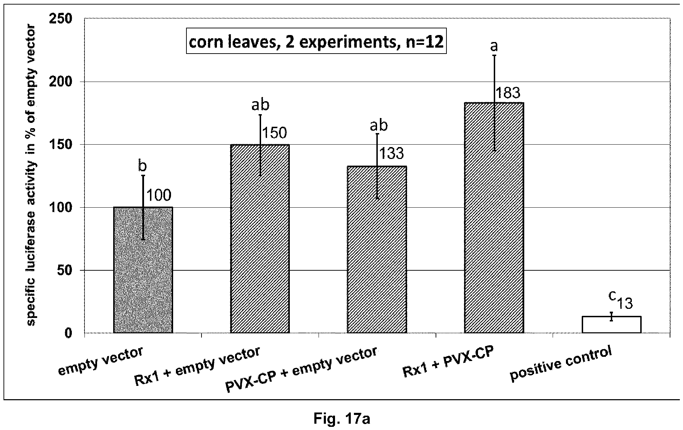

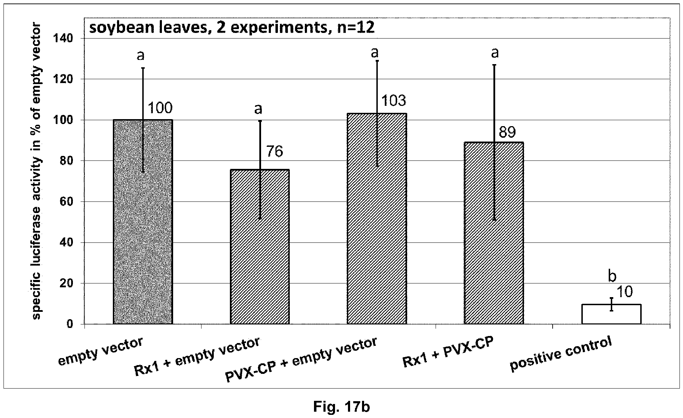

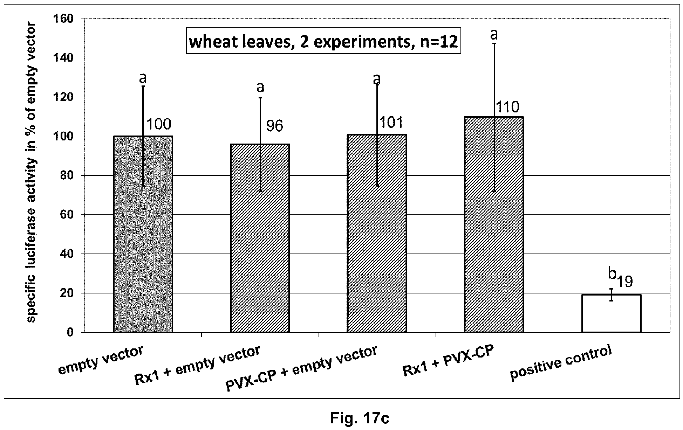

Figure 17a, Figure 17b and Figure 17c: In contrast to R3a-Avr3a^{KI} co-expression, the co-expression of Rx1-PVX-CP does not activate cell death in corn, soybean and wheat leaves. The potato Rx1 resistance gene and the gene encoding the coat protein of Potato virus X (PVX-CP) were transiently co-expressed in leaves of corn, soybean and wheat. The induction of cell death was measured by the activity of two co-bombarded luciferase reporter genes 16h after transformation. An auto-activated wheat resistance gene and an auto-activated soybean resistance gene were used as "positive controls" for wheat, corn and soybean.

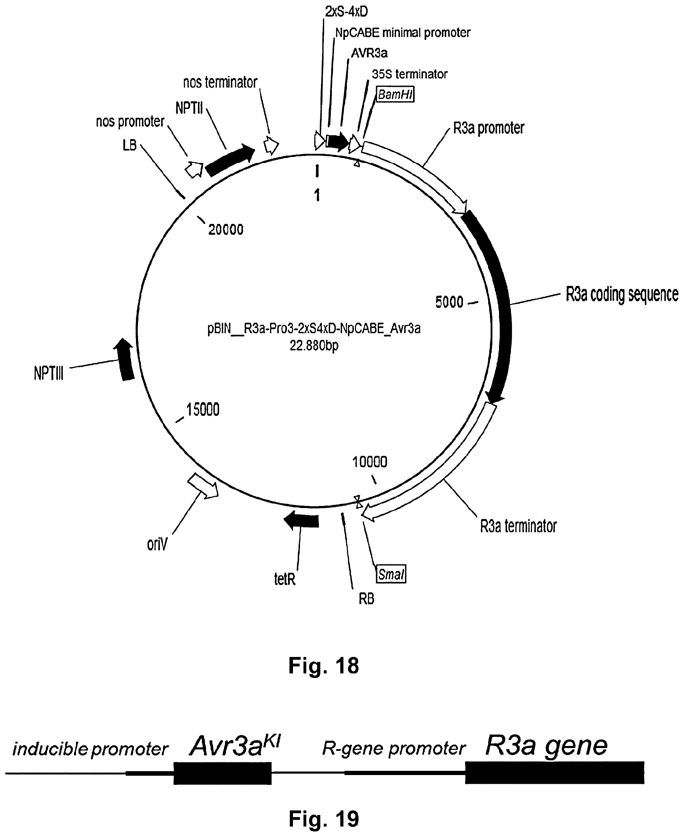

Figure 18: Vectormap of the vector pBIN. The vector comprises the AVR3a gene as avirulence gene and R3a as resistance gene. The avirulence gene is under control of a promoter construct comprising the NpCABE minimal promoter and the 2xS-4xD cis-regulatory elements. The resistance gene is under control of the R3a promoter. The genes NPTII, NPTIII and tetR are included in the vector for conferring resistance to selective agents. LB = left border of the T-DNA (transfer DNA); RB = right border of the T-DNA; the vector is suitable for stable integration of the avirulence gene and the resistance gene into a plant genome and subsequent expression of the genes.
Fig. 19: Schematic construction of an expression cassette including the resistance gene R3a and the avirulence gene Avr3a^{KI}. The expression cassette is usable for expression of the included genes.

### Detailed description of the invention

The present invention is based on the discovery that genetically modified genes involved in the gene-for-gene interaction can be used to decrease cell death and to prevent the undesired activation of defense reactions in the absence of pathogens. Mutagenized versions of native resistance genes or Avr genes having reduced cell death-inducing activity were generated and applied, in order to decrease cell death, e.g. by increasing the threshold for the induction of cell death. Genetic elements of the gene-for-gene interaction having reduced cell death-inducing activity were shown to decrease cell death and to prevent the undesired activation of defense reactions in the absence of pathogens. At the same time, the modified genes were still capable of activating the innate immunity during pathogen infection. The inventive technology was successfully tested for the CC-NBS-LRR resistance gene R3a of potato and the Avr3a gene of *P. infestans.* The same technology may be applied to any CC-NBS-LRR resistance gene of a crop and the matching avirulence gene. In addition, it was shown that the R3a-Avr3a gene combination induces cell death in corn, soybean and wheat. Hence, the findings of the present invention were successfully transferred to these other crops. A list of additional CC-NBS-LRR resistance genes and their corresponding AVR genes which may be used in the methods of the invention is provided.

Accordingly, the present invention provides a nucleic acid molecule for increasing the resistance of a plant or a part thereof towards at least one plant pathogen, wherein the nucleic acid molecule comprises a nucleotide sequence selected from the group consisting of:
(a) a nucleotide sequence encoding i) a plant resistance protein of the resistance protein class CC-NBS-LRR and ii) an avirulence protein;
(b) a nucleotide sequence encoding a plant resistance protein of the resistance protein class CC-NBS-LRR;
(c) a nucleotide sequence encoding an avirulence protein;
(d) a nucleotide sequence hybridizing under stringent conditions to a sequence which is complementary to a nucleotide sequence according to (a), (b) or (c); and
(e) a nucleotide sequence encoding a polypeptide comprising an amino acid sequence which is at least 70% identical to an amino acid sequence according to SEQ ID NO: 93 or 271;
wherein the encoded resistance protein, the encoded avirulence protein, or both, include at least one amino acid substitution in comparison to the wild type amino acid sequence of the resistance and/or avirulence protein, said substitution leading to a decrease of cell death during a hypersensitive reaction.

Preferably, the at least one amino acid substitution is an amino acid substitution outside of the signal sequence of the encoded resistance protein or the encoded avirulence protein. The expression "resistance" or "resistant" as regards a pathogen should be understood to mean the ability of a plant or plant cell to resist the damaging effects of the pathogen and extends from a delay in the development of disease to complete suppression of the development of the disease. The resistance may be complete or partial and may be specific or non-specific to the pathogen race. A conferred resistance may be a newly inherited resistance or an increase in a partial resistance which is already extant.

Resistance may be quantified by methods known in the art. For example, resistance may be quantified by a reduced fungal biomass on the host plant; for this, the fungal DNA may be determined with the aid of quantitative PCR in comparison to the plant DNA in the infested plant tissue. An additional approach to the measurement of resistance is optical rating, wherein rating scores of 1 (not susceptible) to 9 (very susceptible) are awarded. An increased resistance can be measured by for example inoculation of a healthy leaf with an isolate of the pathogen and the determination of the infested surface after determined number of days (for example 15 days). A reduce of 5% of the infested surface corresponds to an increase of the resistance of 5%.

Plant: A plant can be a dicot or a monocot. Plants which can be agronomically exploited are preferred. Examples of plants which can be used in context of the invention are sugar beet or red beet; potato; corn; cotton, rape; Poaceae including cereals like wheat, barley, rye, rice, oat,millet; sugar cane, sunflower; Fabaceae like soybean, beans, peanuts, lentils and lupins.

Part of a plant: In general, every organ or tissue of a plant form a part of a plant. A part of a plant can be leaves, stems, roots, emerged radicles, flowers, flower parts, petals, fruits, pollen, pollen tubes, anther filaments, ovules, embryo sacs, egg cells, ovaries, zygotes, embryos, zygotic embryos, somatic embryos, apical meristems, vascular bundles, pericycles, seeds, roots, and cuttings.

Pathogen: A "pathogen" as used herein refers to an organism which can infect a plant, or which can cause a disease in a plant. Pathogens which can infect a plant, or which can cause a disease in a plant, include fungi, oomycetes, bacteria, viruses, viroids, virus-like organisms, phytoplasmas, protozoa, nematodes and parasitic plants. Plant parasites can cause damage by feeding on a plant and can be selected from ectoparasites like insects, comprising aphids and other sap-sucking insect, mites, and vertebrates. Examples for fungal pathogens which are especially suitable to be combated by the present invention are *Alternaria solani, Phytophthora infestans, Blumeria graminis* or fungi belonging to the genus of *Fusarium.*

Plant resistance protein: The plant resistance proteins are key molecules for the activation of the induced defense mechanisms. According to the gene-for-gene postulate a resistance interacts directly or indirectly with a corresponding protein encoded by pathogen's avirulence gene (Avr-gene) and thereby triggers the induced defensive reaction. CC-NBS-LRR proteins may comprise three domains: A coiled-coil domain (CC), a NBS comain (nucleotide binding site) and a LRR domain (leucin-rich repeat). In the context of this invention CC-NBS-LRR proteins may also be such proteins which comprise the amino acid sequence VLSDAE or the motif VLSDAEXKQ as depticted in Fig. 3 (wherein X can be every genetically encodable amino acid). This motif is also suitable to characterize or identify the CC domain wherein the cc domain my beginn 56 amino acids in n-terminal direction from the position of the amino acid sequence VLSDAE or the motif VLSDAEXKQ (with V being position 0). Furthermore, the CC domain may stretch 129 amino acids towards in c-terminal direction from the position of the amino acid sequence VLSDAE (with E being position 0) or 127 amino acids from the position of the motif motif VLSDAEXKQ (with Q being position 0).

Avirulence protein: An avirulence protein is produced by a plant pathogen and is able for direct or indirect chemical interaction with a corresponding plant resistance protein wherein this interaction triggers a hypersensitive reaction (defense reaction) within a plant cell.

Variant: Variants of proteins or of nucleic acids encompass for example homologs, orthologs, or allelic variants.

Hybridize: The term "hybridize" or "hybridization" should be understood to mean a procedure in which a single stranded nucleic acid molecule agglomerates with a nucleic acid strand which is as complementary as possible, i.e. base-pairs with it. Examples of standard methods for hybridization have been described in 2001 by Sambrook et al. Preferably, this should be understood to mean that at least 60%, more preferably at least 65%, 70%, 75%, 80% or 85%, particularly preferably 90%, 91%, 92%, 93%, 94%, 95%, 96%, 97%, 98% or 99% of the bases of the nucleic acid molecule undergo base pairing with the nucleic acid strand which is as complementary as possible. The possibility of such agglomeration depends on the stringency of the hybridization conditions. The term "stringency" refers to the hybridization conditions. High stringency is when base pairing is more difficult, low stringency is when base pairing is easier. The stringency of the hybridization conditions depends, for example, on the salt concentration or ionic strength and the temperature. In general, the stringency can be increased by raising the temperature and/or by reducing the salt content. The term "stringent hybridization conditions" should be understood to mean those conditions under which a hybridization takes place primarily only between homologous nucleic acid molecules. The term "hybridization conditions" in this respect refers not only to the actual conditions prevailing during actual agglomeration of the nucleic acids, but also to the conditions prevailing during the subsequent washing steps. Examples of high stringent hybridization conditions are conditions under which primarily only those nucleic acid molecules that have at least 90% or at least 95% sequence identity undergo hybridization. Such high stringent hybridization conditions are, for example: 4 x SSC at 65°C and subsequent multiple washes in 0.1 x SSC at 65°C for approximately 1 hour. The term "high stringent hybridization conditions" as used herein may also mean: hybridization at 68°C in 0.25 M sodium phosphate, pH 7.2, 7 % SDS, 1 mM EDTA and 1 % BSA for 16 hours and subsequently washing twice with 2 x SSC and 0.1 % SDS at 68°C. Preferably, hybridization takes place under stringent conditions. Less stringent hybridization conditions are, for example: hybridizing in 4 x SSC at 37 °C and subsequent multiple washing in 1 x SSC at room temperature.

Hypersensitive reaction: A plant defense reaction including the controlled cellular death of the host tissue at the infection site, the strengthening of the plant cell wall by lignification and callose formation, the formation of phytoalexins and the production of PR-(pathogenesis-related) proteins.

Decrease / reduction of cell death and reduced cell death-inducing activity: Characteristic of a protein which comprises at least one amino acid substitution and is suitable to increase the resistance towards at least one pathogen. The decrease or reduction of cell death is evident due to a reduced number of leaf abortion during the development of a plant in comparison to a plant comprising the protein having the corresponding wild type amino acid sequence without substitution of at least one amino acid.

An alternative approach to proof that a resistance inducing protein leads to decreased or reduced cell death is the biolistic transformation of a leaf with an expression cassette comprising a nucleic acid encoding a protein according to the invention and a second leaf treated in the same way using a sequence encoding a protein without amino acid substitution wherein both leaves are co-transformed with a luciferase reporter gene. After 16h the induction of cell death is measured by the activity of the expressed luciferase. A nucleic acid leading to more luciferase activity than the nucleic acid of the comparison-leaf shows reduced induction of cell death.

Signal sequence of a protein: An amino acid sequence within a protein which is usually located in the n-terminal part of a protein and can for example begin after the first n-terminal amino acid. Signal sequences usually consist of 13-36 amino acids. In most cases they possess a central, strong hydrophobic part which extends over 10-15 amino acids along which often can be found: alanine, leucine, valine, isoleucine and phenylalanine. The signal sequence is usually responsible for the transportation of the protein to its final location within the cell. However, the signal sequence is not always necessary for the function of a protein and is often removed after the protein has reached its final localization. For example, a plant's immune system usually can recognize pathogenic Avr proteins even if those Avr proteins lack their naturally occurring signal sequence.

Wild type amino acid sequence or wild type protein: A sequence of amino acids building a protein compound which is existing in nature.

Substitution leading to a decrease of cell death during a hypersensitive reaction: Is a substitution in the amino acid sequence of a resistance protein and / or avirulence protein. The cell death rate during or as consequence of a hypersensitive reaction (wherein the triggering or maintenance of the hypersenstive reaction is affected either by the resistance protein or the avirulence protein or both) is reduced in comparison to a hypersensitive reaction (occuring under comparable circumstances) the triggering or maintenance of which is affected by the resistance protein or avirulence protein or both lacking the substitution. However, the proteins comprsising the substituttion are still able to affect the triggerance or maintenance of a hypersensitive reaction and are therefore functional proteins wherein substitution leading to a complete loss of function are not encompassed.

In one embodiment, the plant resistance protein is selected from the group consisting of: R3a (CC; AAW48299), Rpi-blb3, R2, Rpi-abpt, Rpi-edn1, R1, Rpi-blb2, Rpi-blb1, Rpi-pta1, Rpi-sto1, Rpi-vnt1.1, Rpi-edn2, R9a, R8, Rpi-chc1, R3b (CC; AEC47890), Mla1_barley, Mla13_barley, Rpg1-b_Soybean, RPM1 (CC; CAA61131), RPS2 (CC; AEE85156), RPS5 (CC; AEE28852), BS2 (CC; AAF09256), Rxo1 (AAX31149), I-2_tomato, Pi-ta (AF207842), Piz-t (ABC73398), Pia Rga4 (BAK39922), Pia Rga5 (BAK39930), Pii-1 (BAN59294), Pik-1 (ADZ48537), Pik-2 (ADZ48538), Pikh-1 (AET36549), Pikm1-TS (BAG71909), Pikm2-TS (BAG71908), Pikp-1 (ADV58352), Pikp-2 (ADV58351), Piks-1 (AET36547), Piks-2 (AET36548), Rps1k-1_soybean, Rps1k-2_soybean, L3 (CC; BAJ33559), N'_tobacco, RX1 (CC; CAB50786), RX2 (CC; CAB56299), Pvr4_pepper, Pvr9_pepper, Tm2_Tomato, Tm2^2_Tomato, Tsw_pepper, Sw-5b_tomato, Rsv1_3gG2, Pm2, PM3A (CC; AAY21626), and PM3F.

In another embodiment, the avirulence protein is selected from the group consisting of: Avr3a_Phytophthora, Avr2_Phytophthora_infestans, Avr1 _Phytophthora_infestans, Avrblb2, Avr-blb1=Avr-sto1, Avr-vnt1 (PITG_16294)_Phytophthora, Avrblb2, Avr8_Phytophthora_infestans, PITG_16245 (Avr-chc1)_Phytophthora, Avr3b (PITG_18215)_Phytophthora, Avra1_(AvrMla1)_Blumeria, Avra13_(AvrMla13)_Blumeria, AvrB_Pseudomonas, AvrRpm1, AvrPpiA1_Pseudomonas, AvrRpt2_Pseudomonas, AvrPphB (HopAR1)_Pseudomonas, AvrBs2_Xanthomonas, AvrRxo1, Avr2 (SIX3)_Fusarium oxysporum, SIX5_Fusarium oxysporum, AVR Pita (AF207841), AvrPiz-t (ACF39937), AVR1-CO39 (AF463528_3), AVR-Pia (BAH59484), Avr-Pii (BAH59485), Avr Pik_km_kp allele D (allele pex31-D) (BAH59486), Avr1k_Phytophthora sojae, CP_Pepper mild mottle virus (Avr L3&4), CP_Potato virus X, Nlb_pepper mottle virus (Avr Pvr4&9), 30kDa MP_Tobacco mosaic virus (AvrTm2), NSs_Tomato spotted wilt virus (AvrTsw), NSm_Tomato spotted wilt virus (AvrSw-5b), P3 cistron_soybean mosaic virus (AvrRsv1_ 3gG2), AvrPm2, and AvrPm3a2_f2.

In yet another embodiment, the nucleic acid molecule comprises a nucleotide sequence encoding a plant resistance protein of the resistance protein class CC-NBS-LRR and an avirulence protein, wherein the plant resistance protein and the avirulence protein are a combination selected from the following combinations: R3a (CC; AAW48299) and Avr3a_Phytophthora, Rpi-blb3 and Avr2 _Phytophthora_infestans, R2 and Avr2 _Phytophthora_infestans, Rpi-abpt and Avr2 _Phytophthora_infestans, pi-edn1 and Avr2 _Phytophthora_infestans, R1 and Avr1 _Phytophthora_infestans, Rpi-blb2 and Avrblb2, Rpi-blb1 and Avr-blb1=Avr-sto1, Rpi-pta1 and Avr-blb1=Avr-sto1, Rpi-sto1 and Avrblb1=Avr-sto1, Rpi-vnt1.1 and Avr-vnt1 (PITG_16294)_Phytophthora, Rpi-edn2 and Avrblb2, R9a and Avrblb2, R8 and Avr8_Phytophthora_infestans, Rpi-chc1 and PITG_16245 (Avr-chc1)_Phytophthora, R3b (CC; AEC47890) and Avr3b (PITG_18215)_Phytophthora, Mla1_barley and Avra1_(AvrMla1)_Blumeria, Mla13_barley and Avra13_(AvrMla13)_Blumeria, Rpg1-b_Soybean and AvrB_Pseudomonas, RPM1 (CC; CAA61131) and AvrB_Pseudomonas, RPM1 (CC; CAA61131) and AvrRpm1, RPM1 (CC; CAA61131) and AvrPpiA1_Pseudomonas, RPS2 (CC; AEE85156) and AvrRpt2_Pseudomonas, RPS5 (CC; AEE28852) and AvrPphB (HopAR1)_Pseudomonas, BS2 (CC; AAF09256) and AvrBs2_Xanthomonas, Rxo1 (AAX31149) and AvrRxo1, I-2_tomato and Avr2 (SIX3)_Fusarium oxysporum, I-2_tomato and SIX5_Fusarium oxysporum, Pi-ta (AF207842) and AVR Pita (AF207841), Piz-t (ABC73398) and AvrPiz-t (ACF39937), Pia Rga4 (BAK39922) and AVR1-CO39 (AF463528_3), Pia Rga4 (BAK39922) and AVR-Pia (BAH59484), Pia Rga5 (BAK39930) and AVR1-CO39 (AF463528_3), Pia Rga5 (BAK39930) and AVR-Pia (BAH59484), Pii-1 (BAN59294) and Avr-Pii (BAH59485), Pik-1 (ADZ48537) and Avr Pik_km_kp allele D (allele pex31-D) (BAH59486), Pik-2 (ADZ48538) and Avr Pik_km_kp allele D (allele pex31-D) (BAH59486), Pikh-1 (AET36549) and Avr Pik_km_kp allele D (allele pex31-D) (BAH59486), Pikh-2 (AET36550) and Avr Pik_km_kp allele D (allele pex31-D) (BAH59486), Pikm1-TS (BAG71909) and Avr Pik_km_kp allele D (allele pex31-D) (BAH59486), Pikm2-TS (BAG71908) and Avr Pik_km_kp allele D (allele pex31-D) (BAH59486), Pikp-1 (ADV58352) and Avr Pik_km_kp allele D (allele pex31-D) (BAH59486), Pikp-2 (ADV58351) and Avr Pik_km_kp allele D (allele pex31-D) (BAH59486), Piks-1 (AET36547) and Avr Pik_km_kp allele D (allele pex31-D) (BAH59486), Piks-2 (AET36548) and Avr Pik_km_kp allele D (allele pex31-D) (BAH59486), Rps1k-1_soybean and Avr1k_Phytophthora sojae, Rps1k-2_soybean and Avr1k_Phytophthora sojae, L3 (CC; BAJ33559) and CP_Pepper mild mottle virus (Avr L3&4), N'_tobacco and CP_Pepper mild mottle virus (Avr L3&4), RX1 (CC; CAB50786) and CP_Potato virus X, RX2 (CC; CAB56299) and CP_Potato virus X, Pvr4_pepper and Nlb_pepper mottle virus (Avr Pvr4&9), Pvr9_pepper and Nlb_pepper mottle virus (Avr Pvr4&9), Tm2_Tomato and 30kDa MP_Tobacco mosaic virus (AvrTm2), Tm2^2_Tomato and 30kDa MP_Tobacco mosaic virus (AvrTm2), Tsw_pepper and NSs_Tomato spotted wilt virus (AvrTsw), Sw-5b_tomato and NSm_Tomato spotted wilt virus (AvrSw-5b), Rsv1_3gG2 and P3 cistron_soybean mosaic virus (AvrRsv1_ 3gG2), Pm2 and AvrPm2, PM3A (CC; AAY21626) and AvrPm3a2_f2, and PM3F and AvrPm3a2_f2.

Preferably, the combination of genes is R3a (CC; AAW48299) and Avr3a_Phytophthora.

The following table gives furhter information about CC-NBS-LRR resistance genes from different crops and their corresponding Avr genes suitable to be used in the invention wherein the use as a combination of resistance gene and corresponding Avr gene is preferred:

**Table 1: List of CC-NBS-LRR resistance proteins with matching avirulence proteins.**

| Detailed sequence information is given in an additional listing. Not all known functional protein variants encoded by different alleles are listed. SEQ ID NO = nucleotide sequence as cDNA; (SEQ ID NO in curved brackets = amino acid sequence); [SEQ ID NO in square brackets = genomic sequence] | | | |
|---|---|---|---|
| **Resistance proteins:** | **corresponding Avirulence proteins** | | |
| Rpi-blb3 SEQ ID NO 44 (SEQ ID NO 45) | Avr2 _Phytophthora_infestans SEQ ID NO 46 (SEQ ID NO 47) | | |
| R2 SEQ ID NO 48 (SEQ ID NO 49) | Avr2_Phytophthora_infestans SEQ ID NO 46 (SEQ ID NO 47) | | |
| Rpi-abpt SEQ ID NO 50 (SEQ ID NO 51) | Avr2 _Phytophthora_infestans SEQ ID NO 46 (SEQ ID NO 47) | | |
| Rpi-edn1 SEQ ID NO 52 (SEQ ID NO 53) | Avr2 _Phytophthora_infestans SEQ ID NO 46 (SEQ ID NO 47) | | |
| R1 SEQ ID NO 54 (SEQ ID NO 55) [SEQ ID NO 56] | Avr1_Phytophthora_infestans SEQ ID NO 57 (SEQ ID NO 58) | | |
| Rpi-blb2 SEQ ID NO 59 (SEQ ID NO 60) [SEQ ID NO 61] | Avrblb2 SEQ ID NO 62 (SEQ ID NO 63) | | |
| Rpi-blb1 SEQ ID NO 64 (SEQ ID NO 65) [SEQ ID NO 66] | Avr-blb1=Avr-sto1 SEQ ID NO 67 (SEQ ID NO 68) | | |
| Rpi-pta1 SEQ ID NO 69 (SEQ ID NO 70) [SEQ ID NO 71] | Avr-blb1=Avr-sto1 SEQ ID NO 67 (SEQ ID NO 68) | | |
| Rpi-sto1 SEQ ID NO 72 (SEQ ID NO 73) [SEQ ID NO 74] | Avr-blb1 =Avr-sto1 SEQ ID NO 67 (SEQ ID NO 68) | | |
| Rpi-vnt1.1 SEQ ID NO 75 (SEQ ID NO 76) | Avr-vnt1 (PITG_16294)_Phytophthora SEQ ID NO 77 (SEQ ID NO 78) | | |
| Rpi-edn2 SEQ ID NO 79 (SEQ ID NO 80) | Avrblb2 SEQ ID NO 81 (SEQ ID NO 82) | | |
| R9a (SEQ ID NO 266) | Avrblb2 SEQ ID NO 81 (SEQ ID NO 82) | | |
| R8 SEQ ID NO 85 (SEQ ID NO 86) | Avr8_Phytophthora_infestans SEQ ID NO 87 (SEQ ID NO 88) | | |
| Rpi-chc1 SEQ ID NO 89 (SEQ ID NO 90) | PITG_16245 (Avr-chc1)_Phytophthora SEQ ID NO 91 (SEQ ID NO 92) | | |
| R3a (CC; AAW48299) SEQ ID NO 93 (SEQ ID NO 94) | Avr3a_Phytophthora SEQ ID NO 95 (SEQ ID NO 96) | | |
| R3b (CC; AEC47890) SEQ ID NO 97 (SEQ ID NO 98) | Avr3b (PITG_18215)_Phytophthora SEQ ID NO 99 (SEQ ID NO 100) | | |
| Mla1_barley SEQ ID NO 101 (SEQ ID NO 102) | Avra1_(AvrMla1)_Blumeria SEQ ID NO 103 (SEQ ID NO 104) | | |
| Mla13_barley SEQ ID NO 105 (SEQ ID NO 106) [SEQ ID NO 107] | Avra13_(AvrMla13)_Blumeria SEQ ID NO 108 (SEQ ID NO 109) | | |
| Rpg1-b_Soybean SEQ ID NO 110 (SEQ ID NO 111) | AvrB_Pseudomonas SEQ ID NO 112 (SEQ ID NO 113) | | |
| RPM1 (CC; CAA61131) SEQ ID NO 118 (SEQ ID NO 119) | | AvrRpm1 SEQ ID NO 114 (SEQ ID NO 115) | AvrPpiA1_ Pseudomonas SEQ ID NO 116 (SEQ ID NO 117) |
| RPS2 (CC; AEE85156) SEQ ID NO 120 (SEQ ID NO 121) | AvrRpt2_Pseudomonas SEQ ID NO 122 (SEQ ID NO 123) | | |
| RPS5 (CC; AEE28852) SEQ ID NO 124 (SEQ ID NO 125) | AvrPphB (HopAR1)_Pseudomonas SEQ ID NO 126 (SEQ ID NO 127) | | |
| BS2 (CC; AAF09256) SEQ ID NO 128 (SEQ ID NO 129) | AvrBs2_Xanthomonas SEQ ID NO 130 (SEQ ID NO 131) | | |
| Rxo1 (AAX31149) SEQ ID NO 132 (SEQ ID NO 133) | AvrRxo1 SEQ ID NO 134 (SEQ ID NO 135) | | |
| I-2_tomato SEQ ID NO 136 (SEQ ID NO 137) | Avr2 (SIX3)_Fusarium oxysporum SEQ ID NO 138 (SEQ ID NO 139) | SIX5_Fusarium oxysporum SEQ ID NO 140 (SEQ ID NO 141) [SEQ ID NO 142] | |
| Pi-ta (AF207842) SEQ ID NO 143 (SEQ ID NO 144) [SEQ ID NO 145] | AVR Pita (AF207841) SEQ ID NO 146 (SEQ ID NO 147) [SEQ ID NO 148] | | |
| Piz-t (ABC73398) SEQ ID NO 149 (SEQ ID NO 150) (SEQ ID NO 151) | AvrPiz-t (ACF39937) SEQ ID NO 152 (SEQ ID NO 153) | | |
| Pia Rga4 (BAK39922) SEQ ID NO 154 (SEQ ID NO 155) | AVR1-CO39 (AF463528_3) SEQ ID NO 158 (SEQ ID NO 159) | | |
| | | | |
| Pia Rga5 (BAK39930) SEQ ID NO 156 (SEQ ID NO 157) | AVR-Pia (BAH59484) SEQ ID NO 160 (SEQ ID NO 161) | | |
| Pii-1 (BAN59294) SEQ ID NO 162 (SEQ ID NO 163) | Avr-Pii (BAH59485) SEQ ID NO 164 (SEQ ID NO 165) | | |
| Pik-1 (ADZ48537) SEQ ID NO 166 (SEQ ID NO 167) Pik-2 (ADZ48538) SEQ ID NO 169 (SEQ ID NO 170) [SEQ ID NO 171] | | | |
| | Avr Pik_km_kp allele D (allele pex31-D) (BAH59486) SEQ ID NO 184 (SEQ ID NO 185) | | |
| Pikh-1 (AET36549) SEQ ID NO 172 (SEQ ID NO 173) [SEQ ID NO 174] Pikh-2 (AET36550) SEQ ID NO 175 (SEQ ID NO 176) [SEQ ID NO 177] | | | |
| | | | |
| Pikm1-TS (BAG71909) SEQ ID NO 178 (SEQ ID NO 179) [SEQ ID NO 180] Pikm2-TS (BAG71908) SEQ ID NO 181 (SEQ ID NO 182) [SEQ ID NO 183] | | | |
| | | | |
| Pikp-1 (ADV58352) SEQ ID NO 186 (SEQ ID NO 187) [SEQ ID NO 188] Pikp-2 (ADV58351) SEQ ID NO 189 (SEQ ID NO 190) [SEQ ID NO 191] | Avr Pik_km_kp allele D (allele pex31-D) (BAH59486) SEQ ID NO 184 (SEQ ID NO 185) | | |
| | | | |
| Piks-1 (AET36547) SEQ ID NO 192 (SEQ ID NO 193) [SEQ ID NO 194] Piks-2 (AET36548) SEQ ID NO 195 (SEQ ID NO 196) [SEQ ID NO 197 | | | |
| | | | |
| Rps1 k-1_soybean SEQ ID NO 198 (SEQ ID NO 199) | Avr1k_Phytophthora sojae SEQ ID NO 200 (SEQ ID NO 201) | Avr1b_ Phytophthora sojae SEQ ID NO 202 (SEQ ID NO 203) | |
| Rps1 k-2_soybean SEQ ID NO 204 (SEQ ID NO 205) | | | |
| L3 (CC; BAJ33559) SEQ ID NO 206 (SEQ ID NO 207) | CP_Pepper mild mottle virus (Avr L3&4) SEQ ID NO 208 (SEQ ID NO 209) | | |
| N'_tobacco SEQ ID NO 210 (SEQ ID NO 211) | CP_Pepper mild mottle virus (Avr L3&4) SEQ ID NO 208 (SEQ ID NO 209) | | |
| RX1 (CC; CAB50786) SEQ ID NO 212 (SEQ ID NO 213) [SEQ ID NO 214] | CP_Potato virus X SEQ ID NO 215 (SEQ ID NO 216) | | |
| RX2 (CC; CAB56299) SEQ ID NO 217 (SEQ ID NO 218) [SEQ ID NO 219] | | | |
| Pvr4_pepper SEQ ID NO 220 (SEQ ID NO 221) | Nlb_pepper mottle virus (Avr Pvr4&9) SEQ ID NO 222 (SEQ ID NO 223) | | |
| Pvr9_pepper SEQ ID NO 224 (SEQ ID NO 225) [SEQ ID NO 226] | | | |
| Tm2_Tomato SEQ ID NO 227 (SEQ ID NO 228) | 30kDa MP_Tobacco mosaic virus (AvrTm2) SEQ ID NO 229 (SEQ ID NO 230) | | |
| Tm2^2_Tomato SEQ ID NO 231 (SEQ ID NO 232) | | | |
| Tsw_pepper SEQ ID NO 233 (SEQ ID NO 234) | NSs_Tomato spotted wilt virus (AvrTsw) SEQ ID NO 235 (SEQ ID NO 236) | | |
| Sw-5b_tomato SEQ ID NO 237 (SEQ ID NO 238) | NSm_Tomato spotted wilt virus (AvrSw-5b) SEQ ID NO 239 (SEQ ID NO 240) | | |
| Rsv1_3gG2 SEQ ID NO 241 (SEQ ID NO 242) | P3 cistron_soybean mosaic virus - AvrRsv1_3gG2 SEQ ID NO 243 (SEQ ID NO 244) | | |
| Pm2 SEQ ID NO 245 (SEQ ID NO 246) [SEQ ID NO 247] | AvrPm2 SEQ ID NO 248 (SEQ ID NO 249) [SEQ ID NO 250] | | |
| PM3A (CC; AAY21626) SEQ ID NO 251 (SEQ ID NO 252) [SEQ ID NO 253] | AvrPm3a2_f2 SEQ ID NO 254 (SEQ ID NO 255) [SEQ ID NO 256] | | |
| PM3F SEQ ID NO 257 (SEQ ID NO 258) [SEQ ID NO 259] | | | |

As is apparent from Tab. 1 for example the combination of resistance protein RPM1 (or the gene encoding the protein) with one of the avirulence proteins AvrB_Pseudomonas or AvrRpm1 or AvrPpiA1_Pseudomonas (or with the genes encoding these porteins) within a plant cell can trigger (directly or indirectly) a hyper sensitive immune response. Each of the genes can be modified as described elsewhere herein to reduce or decrease the decrease the cell death during the hypersensitive reaction.

The plant resistance protein and/or the avirulence protein of the present invention comprises at least one amino acid substitution compared to the respective wild type amino acid sequence, resulting in a reduction or decrease of cell death during a hypersensitive reaction.

In one embodiment the nucleic acid molecule according to the invention encodes an avirulence protein comprising at least one amino acid substitution selected from the group consisting of: K59/M82, R59/M82, H59/M82, C59/M82, N59/M82, Q59/M82, T59/M82, S59/M82, M59/M82, G59/M82, A59/M82, L59/M82, V59/M82, l59/M82 within the amino acid sequence of SEQ ID NO: 2. This means that the nucleic acid molecule encodes an avirulence protein comprising essentially the amino acid according to SEQ ID NO: 2 but that this sequence is modified by one or more of the above given amino acid substitutions. This avirulence protein may be the avirulence protein Avr3a wherein the avirulence protein optionally lacks a signal sequence.

It is also possible to use the native avirulence protein Avr3a including the signal sequence and substituting amino acids according to the following table:

**Table 2: Avr3a variants comprising different substitutions according to the invention**

| Twenty Avr3aE59X/M82 variants and the native Avr3aK59/I82 allele are listed together with the corresponding substitution positions and SEQ ID NOs. Furthermore, the chemical proterty of the amino acid at position 59 is given. | | | |
|---|---|---|---|
| | SEQ ID NO Nucleic acid sequence encoding the protein | SEQ ID NO Protein sequence | Chemical property of amino acid at position 59 |
| K59/I82 | 1 | 2 | basic |
| K59/M82 | 4 | 5 | basic |
| R59/M82 | 6 | 7 | basic |
| H59/M82 | 8 | 9 | basic |
| D59/M82 | 10 | 11 | acidic |
| E59/M82 | 12 | 13 | acidic |
| F59/M82 | 14 | 15 | aromatic |
| Y59/M82 | 16 | 17 | aromatic |
| W59/M82 | 18 | 19 | aromatic |
| P59/M82 | 20 | 21 | non-polar |
| C59/M82 | 22 | 23 | polar |
| N59/M82 | 24 | 25 | polar |
| Q59/M82 | 26 | 27 | polar |
| T59/M82 | 28 | 29 | polar |
| S59/M82 | 30 | 31 | polar |
| M59/M82 | 32 | 33 | non-polar |
| G59/M82 | 34 | 35 | non-polar |
| A59/M82 | 36 | 37 | non-polar |
| L59/M82 | 38 | 39 | aliphatic |
| V59/M82 | 40 | 41 | aliphatic |
| I59/M82 | 42 | 43 | aliphatic |

In one embodiment the nucleic acid molecule according to the invention encodes a resistance protein comprising at least one amino acid substitution selected from the group consisting of: K35E, K37M, E61V, E61K, E135N and E136N within the amino acid sequence of SEQ ID NO: 269. This means that the nucleic acid molecule encodes a plant resistance protein comprising essentially the amino acid according to SEQ ID NO: 269 but that this sequence is modified by one or more of the above given amino acid substitutions. This resistance protein may be R3a.

Further provided is a polypeptide encoded by the nucleic acid molecule of the present invention. The polypeptide may be a plant resistance protein or an avirulence protein. The avirulence protein may be lacking the naturally occurring signal sequence which is responsible for the intracellular processing of the avirulence protein in the cell of the pathogen. An avirulence protein lacking the signal sequence is a protein that does not occur in nature. The invention does also encompass a combination of at least one resistance protein and at least one avirulence protein wherein at least one of the proteins comprises at least one amino acid substitution as described elsewhere herein. Potential combinations of resistance genes and avirulence genes are disclosed elsewhere herein. The invention does also encompass a solution comprising at least one protein or at least one combination of proteins according to the invention. The solution may be an aqueous solution and may comprise further compounds like a pH buffer and stabilizers. Part of the invention is also a method for the exterior or interior application of the solution to a plant or a plant part. The method comprises the steps of providing a plant or plant part and application of the solution to the outer or inner tissue of the plant or its part. The solution may be sprayed or injected.

Also provided is a vector or expression cassette comprising the nucleic acid molecule of the present invention. An example of a vector according to the invention is illustrated by Fig. 18. The illustrated vector includes coding sequences for the proteins R3a and Avr3a. The structure of these two proteins can be adapted by including amino acid substitutions as described herein. An example of an expression cassette is given by SEQ ID NO 270. Furthermore Fig. 19 gives an illustration of a suitable expression cassette.

The nucleic acid according to the invention encoding a resistance protein or an avirulence protein or both may be under control of a pathogen-inducible promoter or a promoter heterologous to the resistance protein or the avirulence protein or both. The pathogen-inducible promoter may be a synthetic pathogen-inducible promoter. Synthetic pathogen-inducible promoters are manmade and do not occur in nature.

The vector may be a plasmid, a cosmid, a phage or an expression vector, a transformation vector, shuttle vector or cloning vector, it may be double or single stranded, linear or circular, or it may be a prokaryotic or eukaryotic host, either by integration into its genome or transforming extrachromosomally. The vector may be heterologous to the resistance protein or the avirulence protein or both. Vectors are synthetic constructs which are manmade and which to not occur in nature. Preferably, the nucleotide sequence of the invention is operably linked in an expression vector to one or more regulatory sequences which allow transcription and optionally expression in a prokaryotic or eukaryotic host cell. As an example, the nucleotide sequence may be under the control of a suitable promoter or a terminator. Suitable promoters may be promoters which are constitutively induced (see, for example, the 35S promoter from the "cauliflower mosaic virus" (Odell et al. 1985)); particularly suitable promoters are those promoters which are pathogen-inducible (see, for example, the PR1 promoter from parsley (Rushton et al., 1996)). Particularly suitable pathogen-inducible promoters are synthetic or chimeric promoters which do not occur in nature, are composed of several elements and contain a minimum promoter as well as, upstream of the minimum promoter, at least one cis-regulatory element which act as the binding site for special transcription factors. Chimeric promoters are custom-designed and are induced by various factors or re-primed. Examples of such promoters can be found in WO2000/29592 and WO2007/147395. An example of a suitable terminator is the nos-terminator (Depicker et al., 1982).

Moreover, provided is a cell or host cell comprising the nucleic acid molecule, the polypeptide, or the vector or expression cassette of the present invention. Preferably, the cell comprises the nucleic acid molecule of the present invention as a transgene or as an endogene wherein the nucleic acid molecule being present as endogene is preferably a resistance protein. In this regard the invention also covers cells the genome of which has been modified to encode one or more substitutions according to the inventions in an encogene wherein the endogene encodes a resistance protein. The modifications may be achieved by the use of genome editing technologies as described elsewhere herein. Also encompassed is a plant comprising one or more of such cells or being regenerated from such cell.

The vector or expression cassette may, for example, be introduced into the (host) cell by conjugation, mobilization, biolistic transformation, agrobacterium-conferred transformation, transfection, transduction, vacuum infiltration or electroporation. Methods for vector introduction, as well as methods for the preparation of the vectors and expression cassettes, are familiar to the person skilled in the art (see, for example, Sambrook et al., Molecular Cloning, Cold Spring Harbor Laboratory, 3rd Ed., 2001).

In one embodiment, the host cell may be a prokaryotic cell, e.g., a bacterial cell. In another embodiment, the host cell may be a eukaryotic cell (e.g., a plant cell or a yeast cell). Particularly preferred bacterial host cells are *Agrobacterium tumefaciens, A. rhizogenes,* and *E. coli.*

Further provided is a plant or part thereof comprising the nucleic acid molecule of the present invention or comprising the cell of the present invention, and a plant or part thereof regenerated from said cell.

Also provided is a seed or offspring from the plant of the present invention, wherein the seed or the offspring comprises the nucleic acid molecule of the present invention as a transgene or an endogene.

An additional aspect of the invention is seed stock comprising seeds that contain the nucleic acid or acids according to the invention. The nucleic acid or acids according to the invention may be present transgenically or endogenously. The seed stock and the seeds may be technically treated. The invention thus also comprises technically-treated seed stock and technically-treated seeds. The various embodiments of technically-treated seed stock are explained in detail in the following whereby the term seed stock also includes seeds: Technically-treated seed stock may be present in polished form. The outermost layer of the seed is thereby removed, so that the seed assumes a more rounded form. This is helpful in sowing. An optimally uniform shape leads to a uniform distribution of the seed stock grains. Technically-treated seed stock furthermore encompasses pilled seed stock. The seed stock is thereby placed on a pilling mass that protects the seed stock contained therein and leads to a larger mass, such that the pilled seed stock shows a greater resistance capability with regard to wind drift and is thus less susceptible to being blown away by the wind, and, at the same time, a more precise positioning during sowing is enabled. In a preferred embodiment of the invention, all pilled seed stock grains of a batch or unit designated for sale have essentially the same shape and the same mass. Deviations of 5% in diameter and mass are possible. However, the deviations preferably do not exceed 1%. As one of the main components, the pilling mass may contain for example a mineral compound such as clay and/or peat, for example. Additional possible components are cited in US 4,067,141. Moreover, the pilling mass may contain additional chemical agents that positively influence the cultivation in practice. These may here be substances that are counted among fertilizing agents. Furthermore, these may be fungicides, insecticides, and/or antifeedants. The fungicides may be thiram and/or hymexazol and/or other fungicides. The insecticide may be a substance from the neonicotinoid group. The substance from the neonicotinoid group is preferably imidacloprid (ATC Code: QP53AX17) and/or clothianidin (CAS number 210880-92-5). Furthermore, the insecticide may also be cyfluthrin (CAS number 68359-37-5) or beta-cyfluthrin.

The pilled seed stock is a specific embodiment of dressed seed stock. In this context technically-treated seed stock encompasses also the dressed seed stock. However, the invention is not limited to pilled seed stock, but, rather, may be applied with any form of dressed seed stock. The invention thus also relates to dressed seed stock, which includes pilled seed stock, but is not limited to this. Dry dressing, wet dressing, and suspension dressing are thus also encompassed. The dressing may thereby also contain at least one dye, such that the dressed seed stock may be quickly differentiated from undressed seed stock, and, furthermore, good visibility in the environment is ensured after sowing. The dressing may also contain those agrochemicals which are described in the context of the pilling mass. The invention includes thus such dressed seed stock whereby the dressing contains at least one antifeedant such as an insecticide and / or at least one fungicide. Optionally, so called electonical dressing (dressing by application of electric energy) may be applied. However, electronic dressing is not a dressing in the strict sense of the word.

An additional form of technically-treated seed stock is encrusted seed stock. What is known as coating is also spoken of in this context as well as of seed stock treated with a coating. The difference to pilled seed stock is that the seed grains retain their original shape, wherein this method is especially economical. The method is described in EP 0 334 258 A1, for example. An additional form of technically-treated seed stock is sprouted or primed seed stock. Sprouted seed stock is pretreated via a pre-germination, whereas primed seed stock has been pretreated via a priming ("germination"). Pre-germinated and primed seed stock have the advantage of a shorter emergence time. The point in time of the emergence after sowing is, at the same time, more strongly synchronized. This enables better agrotechnical processing during cultivation and especially during the harvest, and, additionally, increases the yield quantity. In pre-germination, the seed stock is germinated until the radicle exits the seed stock shell, and the process is subsequently stopped. In the priming, the process is stopped before the radicle exits the seed stock shell. Compared to pre-germinated seed stock, seed stock that has been subjected to a priming is insensitive to the stress of a re-drying and, after such a re-drying, has a longer storage life in comparison to pre-germinated seed stock, for which a re-drying is generally not advised. In this context, technically pretreated seed stock also includes primed and re-dried seed stock. The process of pre-germination is explained in US 4,905,411 A. Various embodiments of priming are explained in EP 0 686 340 A1. In addition to this, it is also possible to simultaneously pill and prime seed stock in one process. This method is described in EP 2 002 702 B1. Primed seed stock which is moreover pilled, is encompassed by the present invention.

The technically-treated seed stock may additionally be furnished with one or more of the herbicide resistances explained above. This allows a further-improved agrotechnical cultivation, since the technically-treated seed stock may be deployed on a field that has previously been treated with weed killer, and that therefore is weed-free.

In addition to this, the invention also encompasses a mixture containing the seed stock according to the invention or the seeds according to the invention, and a dressing mass as defined above. The dressing mass is thereby preferably embodied as a pilling mass, as defined above.

With storage of seed stock according to the invention, storage conditions are preferably to be chosen that do not negatively affect the stability or storage life of the seed stock. Fluctuations in humidity may, especially, have a disadvantageous effect here. Part of the invention is a method for the storage of the seed stock in a container that is via simultaneously water-repellent and breathable. Such a container may be designed as a carton. Such a carton may optionally possess an inner vapor barrier. If the carton is designed as a duplex carton, its stability increases. A seed stock according to the invention that includes such a container and such a carton, or technically-treated seed stock according to the invention, is likewise part of the invention. It is likewise part of the invention to contain seed stock according to the invention or technically-treated seed stock according to the invention in such a carton.

In one embodiment, the plant according to the invention is a hybrid plant or a double haploid plant. Hybrid plants and double haploid plants do not occur in nature and cannot be isolated from nature. In a further embodiment of the plant according to the invention, the nucleic acid molecule according to the invention is present in heterozygous or homozygous form. In the case of a hybrid plant, the nucleic acid molecule may also be present in hemizygous form. The invention also encompasses hybrid seeds and double haploid seeds which contain a nucleic acid according to the invention or a polypeptide according to the invention.

The term "transgene" (or "transgenic") is understood to mean that the respective gene is an exogenous gene that was introduced into the plant. The exogenous gene may be derived from a species other than the plant species into which it is introduced. Alternatively, the respective gene may be a gene already present in the plant species into which it is introduced, so that one or more additional copies of said gene are present as a result introducing the transgene.

The present invention also provides a method for increasing a plant's resistance towards at least one plant pathogen. In one embodiment, said method comprises the steps of introducing the nucleic acid molecule of the present invention into the genome of at least one cell of a plant and regenerating a plant from that cell. This may be done via homology-directed repair or homologous recombination for example by the use of gene editing technology as stated elsewhere herein. Alternatively, said method comprises the steps of transforming a plant cell with a nucleic acid molecule of the present invention, or the vector or the expression cassette of the present invention, and regenerating a plant from that cell. The methods for increasing a plant's resistance towards at least one plant pathogen may further comprise the step of causing the expression of the nucleic acid molecule in the plant.

"Introducing" in the meaning of the present invention includes stable integration by means of transformation including Agrobacterium-mediated transformation, transfection, microinjection, biolistic bombardment, insertion using gene editing technology like CRISPR systems, TALENs, zinc finger nucleases or meganucleases, homologous recombination, modification of endogenous gene using random or targeted mutagenesis like TILLING or gene editing, introgression using breeder's methods, etc.

Moreover, the invention comprises a method for the production of a plant comprising a gene encoding a resistance protein wherein the resistance protein has a decreased or reduced induction of cell death or reduced cell death-inducing activity during a hypersensitive response comprising the following steps:
a) Providing a plant or cell thereof comprising an endogenes gene encoding a resistance protein
b) Modifying the coding sequence of the endogenes gene to encode one or two or three or more amino acid substitutions within the amino acid sequence of the resistance protein thereby encoding a resistance protein having a decreased or reduced induction of cell death or reduced cell death-inducing activity during a hypersensitive response
c) Optionally regenerating a plant of the cell

The modification may be achieved by the use of CRISPR systems, TALENs, zinc finger nucleases or meganucleases, homologous recombination, modification of endogenous gene using random or targeted mutagenesis like TILLING. The resistance gene may be one of the resistance genes given elsewhere herein and / or a resistance gene structurally defined by the disclosure of the sequence listing. The encoded amino acid substitution. The modification may encode an amino acid substitution as stated elsewhere herein. Especially the amino acid substitution may encode one of the amino acid substitutions as disclosed herein for the resistance gene R3a. Furthermore, if the resistance genes belong to the class of CC_{DAE}-NBS-LRR resistance genes the amino acid substitution may be a substitution as defined herein in the context of the method for the modification of an R-gene belonging to the CC_{DAE}-NBS-LRR resistance gene class. The invention is also related to a plant obtainable by this method.

The regeneration of organisms out of cells is explained in various standard references of the cell related biology. The regeneration of plants is for example disclosed in the standard reference "Plant biotechnology: comprehensive biotechnology, second supplement" (Michael W. Fowler, Graham Warren, Murray Moo-Young - Pergamon Press - 1992). An example concerning especially the regeneration of Beta vulgaris is given in Lindsey, K., and P. Gallois. "Transformation of sugarbeet (Beta vulgaris) by Agrobacterium tumefaciens." Journal of experimental botany 41.5 (1990): 529-536.

As the invention is related to biological processes the exact effect may be dependend on the avirulence and / or resistance protein chosen, the substitutions included in the protein(s) and the plant species in which the invention is put into practice. For achieving the best possible results, it may be worth to test several avirulence and / or resistance proteins in a specific plant species and to test different substitutions or substitution combinations within the avirulence and/or resistance protein. Therefore, the invention also comprises a method for testing of resistance and / or avirulence having different substitutions in a plant comprising the following steps:
1) Providing a plant
2) Transforming the plant stably or transiently with
   a) a nucleic acid sequences encoding at least two proteins wherein the proteins are resistance and / or avirulence proteins
   b) at least two nucleic acid sequences each encoding a protein wherein the protein is a resistance and / or avirulence protein
   wherein each protein comprises different substitutions according to the invention
3) Assessing the resistance resulting from the transformation of one of the proteins under step 2)
4) Optionally selecting the plant comprising the protein which comprises the substitution(s) which showed the best resistance or optionally selecting the protein which comprises the substitution(s) which showed the best resistance.

The assessment of the resistance can be done by infecting the plant with a pathogen and quantifying the amount of cell death and detecting the presence of a hypersensitive reaction. The pathogen to be used in the test is preferably a fungus and particularly preferably the pathogen from which the avirulence protein (if an avirulence protein has been tested) originates. The invention also relates to the protein which is selected or selectable by the testing method given above.

Further, the present invention provides a method for the identification of a plant comprising the nucleic acid molecule of the present invention, said method comprising the steps of isolating DNA from the plant of the present invention or part thereof, and performing a polymerase chain reaction using the isolated DNA as a template, thereby amplifying the nucleic acid molecule of the present invention or a part thereof. The method may further comprise the step of detecting the presence of the nucleic acid molecule or a part thereof and thereby identifying the plant. For this approach it is suitable to perform the polymerase chain reaction in the presence of at least one molecular marker which allows due to its chemical structure the differentiation of single nucleotide polymorphisms (SNPs).

The present invention further provides an oligonucleotide which may be used, for example, as a primer in the identification method of the present invention, wherein the oligonucleotide is able to hybridize to a nucleic acid molecule of the present invention. Preferably, the nucleotide sequence of the oligonucleotide is identical to a part of the nucleic acid molecule of the present invention and has a length of at least 15 nucleotides.

Furthermore, the oligonucleotide or molecular marker according to the invention may be connected to a fluorescent dye in order to generate a fluorescence signal, e.g., under excitation via light of the corresponding wavelength. The fluorescent dye may be fluorochrome. The oligonucleotides according to the invention may be coupled with other compounds that are suitable for generating a signal. Such oligonucleotides or molecular markers do not occur in nature and cannot be isolated from nature. The following is executed to produce such marked oligonucleotides: DNA may be marked bio-orthogonally. For this, DNA may be marked in vivo or in vitro with nucleoside analogs, which, for example, may subsequently be coupled with a fluorophore per Staudinger reaction. In addition to this, DNA may also be chemically provided with fluorophores. Oligonucleotides may be marked via a phosphoramidite synthesis with fluorophores that, for example, are used in QPCR, DNA sequencing, and in situ hybridization. Furthermore, DNA may be generated enzymatically in the course of a polymerase chain reaction with fluorescent nucleotides, or be marked with a ligase or a terminal deoxynucleotidyl transferase. DNA may also be detected indirectly via a biotinylation and fluorescent avidin. For couplings, fluorescein, fluorescent lanthanides, gold nanoparticles, carbon nanotubes, or quantum dots, among other things, are used as fluorophores. One of the most commonly used fluorescent substances is FAM (carboxyfluorescein). Consequently, oligonucleotides and, in particular, primers that possess a FAM marking are encompassed by the invention. FAM is preferably present as 6-FAM, wherein - depending upon the desired wavelength of the emission and excitation - other FAM variants, e.g., 5-FAM, may, however, also be used. Examples of additional fluorescence markers are AlexaFluor, ATTO, Dabcyl, HEX, Rox, TET, Texas Red, and Yakima Yellow. Depending upon the field of use, the oligonucleotides may be furnished with modifications of the bases or of the sugar phosphate spine. Among these are, among others, amino-dT, azide-dT, 2-aminopurine,5-Br-dC, 2'-deoxyinosine (INO), 3'-deoxy-A, C, G, 5-Met-dC, 5-OH-Met-dCN6-Met-dA, and others.

A kit comprising at least one of the oligonucleotide of the present invention is also provided. The kit may also contain a pair of the oligonucleotides of the present invention.

Furthermore, the present invention also relates to a marker chip ("DNA chip" or microarray) which contains at least one oligonucleotide according to the invention that is suitable for detection. The marker chip is suitable for application in one or more detection methods according to the invention.

Moreover, provided is a method for the reduction of plant pathogen infestation or the formation of necrotic lesions during the cultivation of plants, comprising the steps of (i) planting the plant or part thereof of the present invention, or the seed of the present invention in a cultivation area, and (ii) growing the plant or a germling resulting from the seed.

The nucleic acid according of the present invention may be used for conferring or increasing resistance towards at least one plant pathogen to a plant or a part thereof.

Furthermore, the present invention may be used to engineer any CC-NBS-LRR resistance gene and/or its corresponding avirulence gene using the promoter induced co-expression strategy for the development of pathogen-resistant plants. The technical application of the invention is not restricted to fungal disease but may also be used to develop plants resistant to insects, bacteria, nematodes and/or viruses if an appropriate pathogen-inducible promoter is selected. Suitable resistance proteins and corresponding avirulence proteins are listed in Tab. 1.

The present invention also comprises the use of a nucleic acid according to the invention for conferring or increasing resistance towards at least one plant pathogen to a plant or a part thereof.

The present invention also comprises an oligonucleotide for use as a primer in a method for the identification of a plant according to the present invention as described above, wherein the oligonucleotide is able to hybridize to a nucleic acid molecule according to the present invention. The nucleotide sequence of the oligonucleotide may be identical to a part of the nucleic acid molecule according to the invention and may have a length of at least 15 nucleotides.

Furthermore, the present invention is also related to a method for the modification of an R-gene belonging to the CC_{DAE}-NBS-LRR resistance gene class (Fig. 1). The method is suitable for the production of a gene encoding a modified resistance protein wherein the modified resistance protein leads to a reduced cell death in comparison to the resistance protein lacking the modification. The method comprises the following steps:
a) Providing a gene encoding an R-gene belonging to the CC_{DAE}-NBS-LRR resistance gene class
b) Modifying the nucleic acid sequence to generate an amino acid substitution selected from the group consisting of:
   I) Substitution of a basic, positively charged amino acid by an acidic negatively charged amino acid or an aliphatic amino acid in the CC-domain of the protein
   II) Substitution of D or E in the DAE motif by a basic, positively charged amino acid like K or R or by an aliphatic amino acid like V, I or L
   III) Substitution of one of the two conserved acidic, negatively charged amino acids at the C-terminus of the CC-domain by a polar amino acid like N or Q.

Examples for acidic, negatively charged amino acids are for example aspartic acid (D) or glutamate (E). Examples for basic, positively charged amino acids are for example histidine, lysine or arginine.

In a preferred embodiment of this method the substitution is a substitution according to I) wherein the substitution is selected from: K30E, R31G and / or K119D within SEQ ID NO 263; K35E and / or K37M within SEQ ID NO 269 or the substitution is a substitution according to II) wherein the substitution is selected from: E61V and / or E61K within SEQ ID NO 269 or the substitution is a substitution according to III) wherein the substitution is selected from E135N and / or E136N within SEQ ID NO 269. The present invention is also related to a protein obtainable by this method. The substitution according to step b) may be done in vitro. Furthermore, it is possible to perform this step by gene editing approaches like TALENS, Zinc Finger Nucleases and CRISPR nuclease including Cas9, CasX, CasY or Cpf1 nuclease.

### Examples

The following examples are provided for illustrative purposes only and are not construed as limiting the present invention.

### Example 1: Molecular stack of R3a resistance gene and Avr3a^{KI} avirulence gene

A molecular stack of the native R3a resistance gene of potato and the Avr3a^{KI} gene was constructed (Fig. 19) and transformed into the potato cultivar "Russet Burbank". The expression of the R3a gene was under control of the endogenous R3a promoter, and the Avr3a expression was controlled by the pathogen-inducible synthetic promoter 2xS-4xD-NpCABE which is comprised in SEQ ID NO 270. Transgenic R3a-Avr3a^{KI} lines were generated and transferred into the greenhouse as rooted *in-vitro* plants. First, the plantlets developed well in soil, showing no difference to the non-transgenic Russet Burbank. However, 3-4 weeks after transfer to the greenhouse, spontaneous necrosis of leaf tissue, abortion of leaves and stunted growth of the plants were observed.

**Table 3: Infected potato leaves of T-047, T-036, T-029 and T-071 show reduced sporangia production of P. infestans in comparison to Russet Burbank but severe disturbance of plant development**

| The sporangia were washed off from the infected potato leaves (n=8) at 6 dpi and the sporangia numbers were determined by a particle counter. The number of sporangia of Russet Burbank was set to 100. In total 3 independent resistance assays were performed. Differences between samples were assessed by using analysis of variance (ANOVA). (* = Significant different according ANOVA to Russet Burbank p < 0.05. n.d. = not determined.) | | | | | |
|---|---|---|---|---|---|
| | Sporangia (%) Test: | | | Sporangia (%) Mean of 3 tests | Plant phenotype |
| | 1 | 2 | 3 | | |
| Russet Burbank (control) | 100 | 100 | 100 | 100 ± 0 | normal |
| T-047 | 61 | n.d. | n.d. | 61 | 30 % size reduction Spontaneous leaf necrosis |
| T-036 | n.d. | 16* | 59 | 37 | 50% size reduction single leaf fall |
| T-029 | 21* | 11* | 43* | 25 | 50% size reduction single leaf fall |
| T-071 | n.d. | n.d. | n.d. | n.d. | 80 % size reduction severe leaf fall |

Four R3a-Avr3a^{KI} lines, which were analyzed in detail, showed a reproducible gradient of disintegration (Fig. 2). A qRT-PCR analysis revealed that the level of the negative phenotype of adult plants correlated with the expression of the Avr3a^{KI} gene. Expression of the Avr3a^{KI} gene was also detectable in healthy young plants, and the Avr3a expression was even higher than in adult plants (Fig. 2). This indicated that the resistance gene R3a is developmentally regulated and the resistance mechanism is not active in young plants.

Therefore, on the basis of these results it can be concluded that the direct transfer of the naturally occuring late blight resistance gene R3a from potato (lacking a substitution) and the naturally occuring Avr3aKI gene of Phytophthora infestans (lacking a substitution) under the control of a synthetic pathogen-inducible promoter leads to severe growth retardation and leaf damage of the plant even in the absence of the pathogen. During greenhouse cultivation, the pathogen-inducible promoter is activated by unknown developmental factors and the highly active Avr3aKI protein induces cell death. The results show that the approach of direct transfer is agronomically impractical.

### Example 2: Modification of R3a gene activity

In order to find a balanced level of gene expression, which does not harm the plant in the absence of the pathogen, mutagenized versions of the R3a gene were developed and successfully tested in a transient assay.

A comparison of the CC-domain of R3a with the CC_{DAE}-domains of the sugar beet R-genes BvKWS3_165 and Bv123 revealed that the DAE and EDVID motifs are conserved (Fig. 3). The R3a gene is therefore a member of the CC_{DAE}-NBS-LRR resistance gene class (Fig. 1). A loss-of function analysis of the CC_{DAE}-domain of the R-gene BvKWS3_165 according to SEQ ID NO 263 showed that the K30E, R31G, E96V, K119D, L153P modification, a D and E exchange in the DAE motif and the E143N and E144N modification of two conserved acidic amino acids at the C-terminus impaired the cell death inducing activity. Based on this knowledge, allelic R3a variants with reduced cell death inducing activity were generated in the CC_{DAE}-domain of the CC-NBS-LRR resistance gene.

As a first rule, the replacement of a basic, positively charged amino acid with an acidic, negatively charged amino acid or an aliphatic amino acid in the CC-domain impairs the HR-inducing activity, as shown for K30E, R31G and K119D of BvKWS_165 (compare to SEQ ID NO 263) (Fig. 4a). This rule was applied to reduce R3a activity by a K35E and K37M exchange in the R3a protein (compare to SEQ ID NO 265) (Fig. 4b).

As a second rule, the replacement of D or E in the DAE motif with a basic, positively charged amino acid (K, R) or an aliphatic amino acid (V, I, L) reduces R-gene activity, as shown by an E61V and E61K exchange for R3a (compare to SEQ ID NO 265) (Fig. 4b).

As a third rule, the replacement of one of the two conserved acidic, negatively charged amino acids at the C-terminus of the CC-domain with a polar amino acid (N, Q) reduces cell death, as shown for the E135N and E136N of R3a (compare to SEQ ID NO 265) (Fig. 4b).

These mutagenized versions can be used to generate co-expression lines without a detrimental phenotype.

### Example 3: Modification of Avr3a activity

The Avr3a gene of *P. infestans* is essential for virulence (Bos et al., 2009). Among other existing alleles, two variants seem to have an outstanding function. They are present either as an Avr3a_K59I82 (Avr3a^{KI}) according to SEQ ID NO 1 or as an Avr3a_E59M82 (Avr3a^{EM}) allele according to SEQ ID NO 12 in all strains examined. The Avr3a^{KI} allele activates R3a resistance gene dependent innate immunity, whereas Avr3a^{EM} was not recognized by the R3a gene (Amstrong et al. 2005).

We tested the 20 Avr3a^{XM} (x = each of the 20 natural amino acids) alleles as given in Tab. 2 by ballistic transient assays in leaves of potato expressing the R3a gene. Fourteen alleles - Avr3a^{HM}, Avr3a^{KM}, Avr3a^{RM}, Avr3a^{CM}, Avr3a^{NM}, Avr3a^{PM}, Avr3a^{QM}, Avr3a^{TM}, Avr3a^{AM}, Avr3a^{GM}, Avr3a^{IM}, Avr3a^{LM}, Avr3a^{MM}, Avr3a^{VM} - showed statistically significant cell death induction compared to the vector control (Figs. 5-8). Twelve of these alleles - Avr3a^{HM}, Avr3a^{CM}, Avr3a^{NM}, Avr3a^{PM} Avr3a^{QM}, Avr3a^{TM}, Avr3a^{AM}, Avr3a^{GM}, Avr3a^{IM}, Avr3a^{LM}, Avr3a^{MM}, Avr3a^{VM}-induced statistically significant less cell death compared to the avirulent Avr3a^{KI} allele (Figs. 5-8). This result showed that Avr3a alleles were identified which trigger less cell death in potato than the avirulent allele Avr3a^{KI}, and that these alleles differ in the level of cell death induction.

The sequences given in Table 2 were tested in potato leaves (Fig. 9). The alleles Avr3a^{KI}, Avr3a^{KM} Avr3a^{RM} triggered a strong cell death in potato, whereas the alleles Avr3a^{DM}, Avr3a^{FM}, Avr3a^{YM} and Avr3a^{WM} were not recognized by R3a. However, other alleles (like Avr3a^{HM}, Avr3a^{CM}, Avr3a^{NM}, Avr3a^{PM} Avr3a^{SM}, Avr3a^{TM}, Avr3a^{AM}, Avr3a^{IM}, Avr3a^{LM}, Avr3a^{MM} and Avr3a^{VM}) induced surprisingly low cell death in potato, and the alleles Avr3a^{PM} and Avr3a^{GM} revealed a suprisingly strong cell death induction.

### Example 4: Enhanced late blight resistance

We selected three gain-of-function mutants with moderate level of cell-death inducing activity. Compared to the avirulent allele Avr3a^{KI} (94% cell death), the cell death inducing activity of Avr3a^{IM}, Avr3a^{LM} and Avr3a^{GM} was 41%, 40% and 42%, respectively. The three AVR3a alleles were each combined with the synthetic 2xS-4xD-NpCABE promoter. The promoter-Avr3a cassettes were transformed into the potato cultivar Hermes, which contained the corresponding R3a resistance gene.

Eight independent transgenic potato lines of each modified Avr3a gene were transferred twice to the greenhouse. The lines were analyzed for a change of the phenotype and fungal resistance. With the exception of two lines, all lines showed the same vegetative growth phenotype as Hermes. One Avr3a^{IM} line showed some growth retardation and the leaves of one Avr3a^{LM} line revealed some micro-necrotic lesions. However, also these plants were viable. A negative phenotype was not detectable for lines were Avr3a^{GM} was present (Fig. 10).

Both sets of plants were tested several times for late blight resistance in a detached leaf assay (DLA). The fungal resistance was determined by a visual scoring of symptoms (5 dpi) and the measurement of sporangia produced in the lesions (6 dpi). Especially the sporangia production is important with respect to the epidemiology of *P. infestans.* The highest level of resistance improvement was observed for the line Avr3a^{GM}-T-024. This line showed a visible reduction of disease symptoms (Fig. 11a + 11b) and only 40% sporangia production compared to the non-transgenic cultivar Hermes and a transgenic control in 5 independent resistance assays with two different sets of plants (Table 4). The line Avr3a^{GM}-T-014 and the line Avr3a^{IM}-T-112 also showed enhanced late blight resistance, as determined by 66% and 71% sporangia production (Table 4). No reproducible resistance improvement without a negative phenotype was detected for the Avr3a^{LM} lines. This result indicates that a reduction of cell-death inducing activity of the Avr gene is a promising strategy to circumvent the detrimental effects of uncontrolled R-Avr gene expression. The maximal possible level of resistance improvement is not known since the analysis was confined to 8 randomly selected lines.

**Table 4: Infected potato leaves of Avr3a^{IM}-T-112, Avr3a^{GM}-T-014 and Avr3a^{GM}-T-024 show reduced sporangia production of P. infestans in comparison to Hermes and a transgenic control (RNAi-GFP).**

| The sporangia were washed off from the infected potato leaves (n=8) at 6 dpi and the sporangia numbers were determined by a particle counter. The number of sporangia of Hermes was set to 100. In total 5 independent resistance assays were performed with 2 different sets of plants. The black highlighted line Avr3a^{GM}-T-024 showed the highest level of sporangia reduction in all 5 assays. Differences between samples were assessed by using analysis of variance (ANOVA). (*= Significant different according ANOVA to Hermes p < 0.05. n.d. = not determined.) | | | | | | |
|---|---|---|---|---|---|---|
| | Plant set 1 Sporangia (%) Test: | | | Plant set 1 Sporangia (%) Mean of 3 tests | Plant set 2 Sporangia (%) Test 1 Test 2 | Plant set 2 Sporangia (%) Mean of 2 tests |
| | 1 | 2 | 3 | | | |
| Hermes (control) | 100 | 100 | 100 | 100 ± 0 | 100 100 | 100 ± 0 |
| RNAi-GFP (transgenic control) | n.d. | n.d. | n.d. | n.d. | 102 112 | 107 ± 5 |
| Avr3a^{IM}-T-097 | 22 | 89 | 71 | 61 ± 28 | 105 86 | 95 ± 10 |
| Avr3a^{IM}-T-112 | 30 | 59 | 96 | 62 ± 27 | 91 62 | 76 ± 15 |
| Avr3a^{GM}-T-014 | 26 | 65 | 77 | 56 ± 22 | 87 26* | 76± 31 |
| Avr3a^{GM}-T-024 | 25 | 31* | 63* | 40 ± 17 | 54 28* | 41 ± 13 |
| Avr3a^{LM}-T-013 | 44 | 39 | 81 | 52 ± 19 | 88 130 | 109 ± 21 |
| Avr3a^{LM}-T-082 | 96 | 153 | | 126 ± 29 | 159 82 | 120 ± 39 |

### Example 5: Avr3a gene expression correlates with resistance

The expression of the recombinant Avr3a gene of 6 transgenic lines and Hermes was measured by qRT-PCR analysis using the primers S3169 according to SEQ ID NO 278 and S3170 according to SEQ ID NO 279. Avr3a expression was normalized against the expression of the potato gene StMCB75 using the primers S1494 according to SEQ ID NO 280 and S1495 according to SEQ ID NO 281. Leaves were infected by *P. infestans* as described for the DLA, and RNA was extracted from inoculated and control leaves 0, 1, 2, 3 and 4 days after inoculation (dpi). The expression analysis showed a correlation between Avr3a gene expression and resistance improvement. The strongest Avr3a expression could be detected for the resistant line Avr3a^{GM}-T-024. The absolute expression of the Avr3a^{GM} gene in the resistant line was superior to the other lines at 2, 3 and 4 dpi (Fig. 12a and 12b). Avr3a gene expression was nearly undetectable for line Avr3a^{LM}-T-082, which did not show resistance improvement in any assay.

### Example 6: Modification of Avr3a gene increases threshold for cell death induction

The transgenic lines T-047, T-036, T-029 and T-071, which were transformed with the R3a-Avr3a^{KI} genes, showed leaf abortion and different stunted growth in the greenhouse dependent on the Avr3a^{KI} expression level (Fig. 2). Expression analysis by qRT-PCR showed that even the lowest detected Avr3a^{KI} gene expression of 1.8 relative units in leaves of T-047 triggers spontaneous cell death in the absence of a pathogen.

The late blight resistant line Avr3a^{GM}-T-024 showed a background expression level of 146.5 relative units in the absence of a pathogen without any detrimental effect for the vegetative growth (Figs. 10, 12a, 12b). These results demonstrated that the threshold for cell death induction was at least 100-fold increased by application of the modified Avr3a^{GM} gene (Fig. 13).

### Example 7: Enhanced early blight resistance

The potato cultivar Hermes is very susceptible to *Alternaria solani,* the causal agent of early blight, as observed in field trials in the USA. Adult plants of both plant sets also showed early blight symptoms under greenhouse conditions. In both experiments, the late blight resistant line Avr3a^{GM}-T-024 revealed enhanced early blight resistance. Finally, an early blight disease scoring was made for the 2^{nd} plant set. Avr3a^{GM}-T-024 showed only 40 % disease symptoms compared to Hermes and the transgenic control, which had been transformed with an RNAi construct directed against the GFP gene (Fig. 14a + 14b).

### Example 8: Induction of innate immunity in corn, soybean and wheat by R3a and Avr3a

The activity of resistance genes is confined to the members of a plant family, a phenomenon called "restricted taxonomic functionality". In transient assays, the R3a-Avr3a^{KI} gene combination was tested for cell-death inducing activity in other non-solanaceous crops. After ballistic transformation into leaves of corn, soybean and wheat, a strong induction of cell death was unexpectedly detected as a hallmark of innate immunity (Fig. 15a, 15b, 15c). Other resistance genes of potato and their matching Avr genes (R1-Avr1, Rx1-potato virus X coat protein) did not induce cell death (Figs. 16a, 16b, 16c and 17a, 17b and 17c). This means that the developed technology can also be transferred to other crops for the generation of broad pathogen resistance.

### Cited documents

Armstrong, M. R., Whisson, S. C., Pritchard, L., Bos, J. I., Venter, E., Avrova, A. O., ... & Hamlin, N. (2005). An ancestral oomycete locus contains late blight avirulence gene Avr3a, encoding a protein that is recognized in the host cytoplasm. Proceedings of the National Academy of Sciences of the United States of America, 102(21), 7766-7771.
Ayliffe, M. A., & Lagudah, E. S. (2004). Molecular genetics of disease resistance in cereals. Annals of Botany, 94(6), 765-773.
Bos, J. I., Armstrong, M. R., Gilroy, E. M., Boevink, P. C., Hein, I., Taylor, R. M., ... & Dixelius, C. (2010). Phytophthora infestans effector AVR3a is essential for virulence and manipulates plant immunity by stabilizing host E3 ligase CMPG1. Proceedings of the National Academy of Sciences, 107(21), 9909-9914.
Bourras, S., McNally, K. E., Ben-David, R., Parlange, F., Roffler, S., Praz, C. R., ... & Schaefer, L. K. (2015). Multiple avirulence loci and allele-specific effector recognition control the Pm3 race-specific resistance of wheat to powdery mildew. The Plant Cell, 27(10), 2991-3012.
Brutus, A., Sicilia, F., Macone, A., Cervone, F., & De Lorenzo, G. (2010). A domain swap approach reveals a role of the plant wall-associated kinase 1 (WAK1) as a receptor of oligogalacturonides. Proceedings of the National Academy of Sciences, 107(20), 9452-9457.
Césari, S., Kanzaki, H., Fujiwara, T., Bernoux, M., Chalvon, V., Kawano, Y., ... & Kroj, T. (2014). The NB-LRR proteins RGA4 and RGA5 interact functionally and physically to confer disease resistance. The EMBO journal, 33(17), 1941-1959.
Cesari, S., Moore, J., Chen, C., Webb, D., Periyannan, S., Mago, R., ... & Dodds, P. N. (2016). Cytosolic activation of cell death and stem rust resistance by cereal MLA-family CC-NLR proteins. Proceedings of the National Academy of Sciences, 113(36), 10204-10209.
Chaparro-Garcia, A., Schwizer, S., Sklenar, J., Yoshida, K., Petre, B., Bos, J. I., ... & Kamoun, S. (2015). Phytophthora infestans RXLR-WY effector AVR3a associates with dynamin-related protein 2 required for endocytosis of the plant pattern recognition receptor FLS2. PLoS One, 10(9), e0137071.
Collier, S. M., & Moffett, P. (2009). NB-LRRs work a "bait and switch" on pathogens. Trends in plant science, 14(10), 521-529.
Collier, S. M., Hamel, L. P., & Moffett, P. (2011). Cell death mediated by the N-terminal domains of a unique and highly conserved class of NB-LRR protein. Molecular plant-microbe interactions, 24(8), 918-931.
Dangl, J. L., Horvath, D. M., & Staskawicz, B. J. (2013). Pivoting the plant immune system from dissection to deployment. Science, 341(6147), 746-751.
Dangl, J. L., & Jones, J. D. (2001). Plant pathogens and integrated defence responses to infection. nature, 411(6839), 826.
Depicker, A., Stachel, S., Dhaese, P., Zambryski, P., & Goodman, H. M. (1982). Nopaline synthase: transcript mapping and DNA sequence. Journal of molecular and applied genetics, 1(6), 561-573.
Fradin, E. F., Abd-El-Haliem, A., Masini, L., van den Berg, G. C., Joosten, M. H., & Thomma, B. P. (2011). Interfamily transfer of tomato Ve1 mediates Verticillium resistance in Arabidopsis. Plant physiology, 156(4), 2255-2265.
Gaudet, R. G., & Gray-Owen, S. D. (2016). Heptose sounds the alarm: Innate sensing of a bacterial sugar stimulates immunity. PLoS pathogens, 12(9), e1005807.
Huang, S., Van Der Vossen, E. A., Kuang, H., Vleeshouwers, V. G., Zhang, N., Borm, T. J., ... & Visser, R. G. (2005). Comparative genomics enabled the isolation of the R3a late blight resistance gene in potato. The Plant Journal, 42(2), 251-261.
Jones, J. D., & Dangl, J. L. (2006). The plant immune system. Nature, 444(7117), 323.
Joosten, M. H., & de Wit, P. J. (1999). The Tomato-C ladosporium F ulvum Interaction: A Versatile Experimental System to Study Plant-Pathogen Interactions. Annual review of phytopathology, 37(1), 335-367.
Lindsey, K., & Gallois, P. (1990). Transformation of sugarbeet (Beta vulgaris) by Agrobacterium tumefaciens. Journal of experimental botany, 41(5), 529-536.
Lu, X., Kracher, B., Saur, I. M., Bauer, S., Ellwood, S. R., Wise, R., ... & Schulze-Lefert, P. (2016). Allelic barley MLA immune receptors recognize sequence-unrelated avirulence effectors of the powdery mildew pathogen. Proceedings of the National Academy of Sciences, 113(42), E6486-E6495.
Macho, A. P., & Zipfel, C. (2014). Plant PRRs and the activation of innate immune signaling. Molecular cell, 54(2), 263-272.
   Martini, N., Egen, M., Rüntz, I., & Strittmatter, G. (1993). Promoter sequences of a potato pathogenesis-related gene mediate transcriptional activation selectively upon fungal infection. Molecular and General Genetics MGG, 236(2-3), 179-186.
McHale, L., Tan, X., Koehl, P., & Michelmore, R. W. (2006). Plant NBS-LRR proteins: adaptable guards. Genome biology, 7(4), 212.
   Narusaka, M., Kubo, Y., Hatakeyama, K., Imamura, J., Ezura, H., Nanasato, Y., ... & Narusaka, Y. (2013). Interfamily transfer of dual NB-LRR genes confers resistance to multiple pathogens. PLoS One, 8(2), e55954.
Niemeyer, J., Ruhe, J., Machens, F., & Hehl, R. (2013). Differential expression of the TMV resistance gene N prevents a hypersensitive response in seeds and during germination. Planta, 237(3), 909-915.
Odell, J. T., Nagy, F., & Chua, N. H. (1985). Identification of DNA sequences required for activity of the cauliflower mosaic virus 35S promoter. Nature, 313(6005), 810.
Rairdan, G. J., Collier, S. M., Sacco, M. A., Baldwin, T. T., Boettrich, T., & Moffett, P. (2008). The coiled-coil and nucleotide binding domains of the potato Rx disease resistance protein function in pathogen recognition and signaling. The Plant Cell, 20(3), 739-751.
Rushton, P. J., Torres, J. T., Parniske, M., Wernert, P., Hahlbrock, K., & Somssich, I. E. (1996). Interaction of elicitor-induced DNA-binding proteins with elicitor response elements in the promoters of parsley PR1 genes. The EMBO journal, 15(20), 5690-5700.
Sambrook, J., & Russell, D. W. (2001). Molecular cloning: a laboratory manual 3rd edition. Coldspring-Harbour Laboratory Press, UK.
Strittmatter, G., Gheysen, G., Gianinazzi-Pearson, V., Hahn, K., Niebel, A., Rohde, W., & Tacke, E. (1996). Infections with various types of organisms stimulate transcription from a short promoter fragment of the potato gst1 gene. MPMI-Molecular Plant Microbe Interactions, 9(1), 68-73.
Takken, F. L., Albrecht, M., & Tameling, W. I. (2006). Resistance proteins: molecular switches of plant defence. Current opinion in plant biology, 9(4), 383-390.
Warren, G. S. (1992). Plant biotechnology: comprehensive biotechnology. Second supplement/volume editor, Michael W. Fowler & Graham S. Warren; editor-in-chief, Murray Moo-Young.
de Wit, P. J. (1992). Molecular characterization of gene-for-gene systems in plant-fungus interactions and the application of avirulence genes in control of plant pathogens. Annual review of phytopathology, 30(1), 391-418.

## Claims

**1.** Nucleic acid molecule for increasing the resistance of a plant or a part thereof towards at least one plant pathogen, wherein the nucleic acid molecule comprises a nucleotide sequence selected from the group consisting of:
(a) a nucleotide sequence encoding
i) a plant resistance protein of the resistance protein class CC-NBS-LRR and
ii) an avirulence protein;
(b) a nucleotide sequence encoding a plant resistance protein of the resistance protein class CC-NBS-LRR;
(c) a nucleotide sequence encoding an avirulence protein;
(d) a nucleotide sequence hybridizing under stringent conditions to a sequence which is complementary to a nucleotide sequence according to (a), (b) or (c); and
(e) a nucleotide sequence encoding a polypeptide comprising an amino acid sequence which is at least 70% identical to an amino acid sequence according to SEQ ID NO: 93;
wherein the encoded resistance protein, the encoded avirulence protein, or both, include at least one amino acid substitution wherein the substitution leads to a decrease of cell death during a hypersensitive reaction in comparison to a resistance protein, avirulence protein or both lacking the amino acid substitution.

**2.** A vector or an expression cassette comprising the nucleic acid molecule according to claim 1 preferably operatively linked to a pathogen-inducible promoter.

**3.** The nucleic acid molecule according to claim 1, wherein the plant resistance protein is selected from the group consisting of: SEQ ID NO 94, SEQ ID NO 45, SEQ ID NO 49, SEQ ID NO 51, SEQ ID NO 53, SEQ ID NO 55, SEQ ID NO 60, SEQ ID NO 65, SEQ ID NO 70, SEQ ID NO 73, SEQ ID NO 76, SEQ ID NO 80, SEQ ID NO 266, R8 according to SEQ ID NO 86, SEQ ID NO 90, SEQ ID NO 98, SEQ ID NO 102, SEQ ID NO 106, SEQ ID NO 111, SEQ ID NO 119, SEQ ID NO 121, SEQ ID NO 125, SEQ ID NO 129, SEQ ID NO 133, SEQ ID NO 137, SEQ ID NO 144, SEQ ID NO 150, SEQ ID NO 155, SEQ ID NO 157, SEQ ID NO 163, SEQ ID NO 167, SEQ ID NO 170, SEQ ID NO 173, SEQ ID NO 179, SEQ ID NO 182, SEQ ID NO 187, SEQ ID NO 190, SEQ ID NO 193, SEQ ID NO 196, SEQ ID NO 199, SEQ ID NO 205, SEQ ID NO 207, SEQ ID NO 211, SEQ ID NO 213, SEQ ID NO 218, SEQ ID NO 221, SEQ ID NO 225, SEQ ID NO 228, SEQ ID NO 232, SEQ ID NO 234, SEQ ID NO 238, SEQ ID NO 242, SEQ ID NO 246, SEQ ID NO 252 and SEQ ID NO 258;
and / or the avirulence protein is selected from the group consisting of:
SEQ ID NO 96, SEQ ID NO 139, SEQ ID NO 58, SEQ ID NO 63, SEQ ID NO 68, SEQ ID NO 78, SEQ ID NO 82, SEQ ID NO 88, SEQ ID NO 92, SEQ ID NO 100, SEQ ID NO 104, SEQ ID NO 109, SEQ ID NO 113, SEQ ID NO 115, SEQ ID NO 117, SEQ ID NO 123, SEQ ID NO 127, SEQ ID NO 131, SEQ ID NO 135, SEQ ID NO 139, SEQ ID NO 141, SEQ ID NO 147, SEQ ID NO 153, SEQ ID NO 159, SEQ ID NO 161, SEQ ID NO 165, SEQ ID NO 185, SEQ ID NO 201, SEQ ID NO 209, SEQ ID NO 216, SEQ ID NO 223, SEQ ID NO 230, SEQ ID NO 236, SEQ ID NO 240, SEQ ID NO 244, SEQ ID NO 249, SEQ ID NO 255.

**4.** The nucleic acid molecule according to claim 1, wherein a nucleotide sequence encodes
i) a plant resistance protein of the resistance protein class CC-NBS-LRR and
ii) an avirulence protein;
and wherein the plant resistance protein and the avirulence protein are a combination selected from the group consisting of:
i) SEQ ID NO 94 and SEQ ID NO 96,
ii) SEQ ID NO 45 and SEQ ID NO 47,
iii) SEQ ID NO 49 and SEQ ID NO 47,
iv) SEQ ID NO 51 and SEQ ID NO 47,
v) SEQ ID NO 53 and SEQ ID NO 47,
vi) SEQ ID NO 55 and SEQ ID NO 58,
vii) SEQ ID NO 60 and SEQ ID NO 63,
viii) SEQ ID NO 65 and SEQ ID NO 68,
ix) SEQ ID NO 70 and SEQ ID NO 68,
x) SEQ ID NO 73 and SEQ ID NO 68,
xi) SEQ ID NO 76 and SEQ ID NO 78,
xii) SEQ ID NO 80 and SEQ ID NO 82,
xiii) SEQ ID NO 266 and SEQ ID NO 82,
xiv) SEQ ID NO 86 and SEQ ID NO 88,
xv) SEQ ID NO 90 and SEQ ID NO 92,
xvi) SEQ ID NO 98 and SEQ ID NO 100,
xvii) SEQ ID NO 102 and SEQ ID NO 104,
xviii) SEQ ID NO 106 and SEQ ID NO 109,
xix) SEQ ID NO 111 and SEQ ID NO 113,
xx) SEQ ID NO 119 and SEQ ID NO 113,
xxi) SEQ ID NO 119 and SEQ ID NO 115,
xxii) SEQ ID NO 119 and SEQ ID NO 117,
xxiii) SEQ ID NO 121 and SEQ ID NO 123,
xxiv) SEQ ID NO 125 and SEQ ID NO 127,
xxv) SEQ ID NO 129 and SEQ ID NO 131,
xxvi) SEQ ID NO 133 and SEQ ID NO 135,
xxvii) SEQ ID NO 137 and SEQ ID NO 139,
xxviii) SEQ ID NO 137 and SEQ ID NO 141,
xxix) SEQ ID NO 144 and SEQ ID NO 147,
xxx) SEQ ID NO 150 and SEQ ID NO 153,
xxxi) SEQ ID NO 155 and SEQ ID NO 159,
xxxii) SEQ ID NO 155 and SEQ ID NO 161,
xxxiii) SEQ ID NO 157 and SEQ ID NO 159,
xxxiv) SEQ ID NO 157 and SEQ ID NO 161,
xxxv) SEQ ID NO 163 and SEQ ID NO 165,
xxxvi) SEQ ID NO 167 and SEQ ID NO 185,
xxxvii) SEQ ID NO 176 and SEQ ID NO 185,
xxxviii) SEQ ID NO 173 and SEQ ID NO 185,
xxxix) SEQ ID NO 176 and SEQ ID NO 185,
xl) SEQ ID NO 179 and SEQ ID NO 185,
xli) SEQ ID NO 182 and SEQ ID NO 185,
xlii) SEQ ID NO 187 and SEQ ID NO 185,
xliii) SEQ ID NO 190 and SEQ ID NO 185,
xliv) SEQ ID NO 193 and SEQ ID NO 185,
xlv) SEQ ID NO 196 and SEQ ID NO 185,
xlvi) SEQ ID NO 199 and SEQ ID NO 201,
xlvii) SEQ ID NO 205 and SEQ ID NO 201,
xlviii) SEQ ID NO 207 and SEQ ID NO 209,
xlix) SEQ ID NO 211 and SEQ ID NO 209,
l) SEQ ID NO 213 and SEQ ID NO 216,
li) SEQ ID NO 218 and SEQ ID NO 216,
lii) SEQ ID NO 221 and SEQ ID NO 223,
liii) SEQ ID NO 225 and SEQ ID NO 223,
liv) SEQ ID NO 228 and SEQ ID NO 230,
lv) SEQ ID NO 232 and SEQ ID NO 230,
lvi) SEQ ID NO 234 and SEQ ID NO 236,
lvii) SEQ ID NO 238 and SEQ ID NO 240,
lviii) SEQ ID NO 242 and SEQ ID NO 244,
lix) SEQ ID NO 246 and SEQ ID NO 249,
lx) SEQ ID NO 252 and SEQ ID NO 255, and
lxi) SEQ ID NO 258 and SEQ ID NO 255.

**5.** The nucleic acid molecule according to one of claims 1, 3 and 4 wherein the nucleic acid molecule encodes an avirulence protein comprising at least one amino acid substitution selected from the group consisting of: K59/M82, R59/M82, H59/M82, C59/M82, N59/M82, Q59/M82, T59/M82, S59/M82, M59/M82, G59/M82, A59/M82, L59/M82, V59/M82, I59/M82 within the amino acid sequence of SEQ ID NO: 2.

**6.** The nucleic acid molecule according to one of claims 1 and 3 - 5, wherein the nucleic acid molecule encodes a plant resistance protein having at least one amino acid substitution within the CC-domain leading to a decrease of cell death during a hypersensitive reaction in comparison to a resistance protein which lacks the amino acid substitution within the CC-domain.

**7.** The nucleic acid molecule according to one of claims 1 and 3 - 6, wherein the at least one amino acid substitution is a substitution in a plant resistance protein and wherein the substitution is the exchange of
I) a basic, positively charged amino acid against an acidic, negatively charged or an aliphatic amino acid or
II) D or E in a DAE motif against a basic, positively charged amino acid or an aliphatic amino acid.

**8.** The nucleic acid molecule according to one of claims 1, 3 and 4, wherein the nucleic acid molecule encodes a plant resistance protein comprising at least one amino acid substitution selected from the group consisting of: K35E, K37M, E61V, E61K, E135N and E136N within the amino acid sequence of SEQ ID NO: 269.

**9.** Polypeptide encoded by the nucleic acid molecule according to any one of the preceding claims.

**11.** Cell comprising the nucleic acid molecule according to any one of claims 1 and 3-8, the polypeptide according to claim 9, or the vector or expression cassette according to claim 2.

**12.** Plant or part thereof comprising the cell according to claim 11, or a plant or part thereof regenerated from the cell according to claim 11.

**13.** Seed or offspring from the plant according to claim 12, wherein the seed or the offspring comprises the nucleic acid molecule according to claim 1 and 3-8, the polypeptide according to claim 9, or the vector or expression cassette according to claim 2.

**14.** Method for increasing a plant's resistance towards at least one plant pathogen comprising:
(i) integrating the nucleic acid molecule according to any one of claims 1 and 3-8 into the genome of at least one plant cell and regenerating a plant from that cell; or
(ii) transforming a plant cell with a nucleic acid molecule according to any one of claims 1 and 3-8, or the vector or the expression cassette according to claim 2, and regenerating a plant from that cell.

**15.** The method of claim 14 wherein the nucleic acid molecule according to step (i) or (ii) encodes a resistance protein comprising the amino acid sequence according to SEQ ID NO 269 and / or an avirulence protein comprising the amino acid sequence according to SEQ ID NO 2 or according to SEQ ID NO 267 and wherein the plant cell according to step (i) or (ii) is the cell of a plant belonging to the family of Poaceae or Fabaceae.


SEQUENCE LISTING

<110> KWS SAAT SE

<120> Balanced Avr expression

<130> KWS0273EP

<160> 281

<170> BiSSAP 1.3.6

<210> 1
<211> 381
<212> DNA
<213> Artificial Sequence


<220>
<223> cDNA

<400> 1
atggaccaaa ccaaggtcct ggtgtatggg acgccagctc actacataca cgattcagcc 60

ggcagaagac ttcttcgcaa gaacgaagag aatgaagaaa cgtctgagga gcgagcccca 120

aatttcaatt tggcgaatct aaatgaggag atgtttaatg tggctgcgtt gacgaagaga 180

gcagatgcca aaaagctagc gaaacagctt atgggtaatg ataagctggc ggatgctgca 240

tacatttggt ggcagcacaa cagggttacg ctagaccaga ttgacacgtt cctgaagctt 300

gcaagccgca agacgcaagg cgcaaagtac aatcagatct acaatagcta catgatgcac 360

ctggggctca ctggatatta a 381


<210> 2
<211> 126
<212> PRT
<213> Artificial Sequence


<220>
<223> Avr3a-KI without signal sequence

<400> 2
Met Asp Gln Thr Lys Val Leu Val Tyr Gly Thr Pro Ala His Tyr Ile
1               5                   10                  15
His Asp Ser Ala Gly Arg Arg Leu Leu Arg Lys Asn Glu Glu Asn Glu
            20                  25                  30
Glu Thr Ser Glu Glu Arg Ala Pro Asn Phe Asn Leu Ala Asn Leu Asn
        35                  40                  45
Glu Glu Met Phe Asn Val Ala Ala Leu Thr Lys Arg Ala Asp Ala Lys
    50                  55                  60
Lys Leu Ala Lys Gln Leu Met Gly Asn Asp Lys Leu Ala Asp Ala Ala
65                  70                  75                  80
Tyr Ile Trp Trp Gln His Asn Arg Val Thr Leu Asp Gln Ile Asp Thr
                85                  90                  95
Phe Leu Lys Leu Ala Ser Arg Lys Thr Gln Gly Ala Lys Tyr Asn Gln
            100                 105                 110
Ile Tyr Asn Ser Tyr Met Met His Leu Gly Leu Thr Gly Tyr
        115                 120                 125

<210> 3
<211> 444
<212> DNA
<213> Artificial Sequence

<220>
<223> cDNA

<220>
<221> transit_peptide
<222> 4..66

<400> 3
atgcgtctgg caattatgct gtctgctacg gctgtcgcca taaactttgc aaccagcagt 60

gcaatcgacc aaaccaaggt cctggtgtat gggacgccag ctcactacat acacgattca 120

gccggcagaa gacttcttcg caagaacgaa gagaatgaag aaacgtctga ggagcgagcc 180

ccaaatttca atttggcgaa tctaaatgag gagatgttta atgtggctgc gttgacgaag 240

agagcagatg ccaaaaagct agcgaaacag cttatgggta atgataagct ggcggatgct 300

gcatacattt ggtggcagca caacagggtt acgctagacc agattgacac gttcctgaag 360

cttgcaagcc gcaagacgca aggcgcaaag tacaatcaga tctacaatag ctacatgatg 420

cacctggggc tcactggata ttag 444

<210> 4
<211> 381
<212> DNA
<213> Artificial Sequence

<220>
<223> cDNA

<400> 4
atggaccaaa ccaaggtcct ggtgtatggg acgccagctc actacataca cgattcagcc 60

ggcagaagac ttcttcgcaa gaacgaagag aatgaagaaa cgtctgagga gcgagcccca 120

aatttcaatt tggcgaatct aaatgaggag atgtttaatg tggctgcgtt gacgaagaga 180

gcagatgcca aaaagctagc gaaacagctt atgggtaatg ataagctggc ggatgctgca 240

tacatgtggt ggcagcacaa cagggttacg ctagaccaga ttgacacgtt cctgaagctt 300

gcaagccgca agacgcaagg cgcaaagtac aatcagatct acaatagcta catgatgcac 360

ctggggctca ctggatatta a 381

<210> 5
<211> 126
<212> PRT
<213> Artificial Sequence

<220>
<223> Avr3a-KM without signal sequence

<400> 5
Met Asp Gln Thr Lys Val Leu Val Tyr Gly Thr Pro Ala His Tyr Ile
1               5                   10                  15
His Asp Ser Ala Gly Arg Arg Leu Leu Arg Lys Asn Glu Glu Asn Glu
            20                  25                  30
Glu Thr Ser Glu Glu Arg Ala Pro Asn Phe Asn Leu Ala Asn Leu Asn
        35                  40                  45
Glu Glu Met Phe Asn Val Ala Ala Leu Thr Lys Arg Ala Asp Ala Lys
    50                  55                  60
Lys Leu Ala Lys Gln Leu Met Gly Asn Asp Lys Leu Ala Asp Ala Ala
65                  70                  75                  80
Tyr Met Trp Trp Gln His Asn Arg Val Thr Leu Asp Gln Ile Asp Thr
                85                  90                  95
Phe Leu Lys Leu Ala Ser Arg Lys Thr Gln Gly Ala Lys Tyr Asn Gln
            100                 105                 110
Ile Tyr Asn Ser Tyr Met Met His Leu Gly Leu Thr Gly Tyr
        115                 120                 125

<210> 6
<211> 381
<212> DNA
<213> Artificial Sequence

<220>
<223> cDNA

<400> 6
atggaccaaa ccaaggtcct ggtgtatggg acgccagctc actacataca cgattcagcc      60

ggcagaagac ttcttcgcaa gaacgaagag aatgaagaaa cgtctgagga gcgagcccca     120

aatttcaatt tggcgaatct aaatgaggag atgtttaatg tggctgcgtt gacgagaaga     180

gcagatgcca aaaagctagc gaaacagctt atgggtaatg ataagctggc ggatgctgca     240

tacatgtggt ggcagcacaa cagggttacg ctagaccaga ttgacacgtt cctgaagctt     300

gcaagccgca agacgcaagg cgcaaagtac aatcagatct acaatagcta catgatgcac     360

ctggggctca ctggatatta a                                               381

<210> 7
<211> 126
<212> PRT
<213> Artificial Sequence

<220>
<223> Avr3a-RM without signal sequence

<400> 7
Met Asp Gln Thr Lys Val Leu Val Tyr Gly Thr Pro Ala His Tyr Ile
1               5                   10                  15
His Asp Ser Ala Gly Arg Arg Leu Leu Arg Lys Asn Glu Glu Asn Glu
            20                  25                  30
Glu Thr Ser Glu Glu Arg Ala Pro Asn Phe Asn Leu Ala Asn Leu Asn
        35                  40                  45
Glu Glu Met Phe Asn Val Ala Ala Leu Thr Arg Arg Ala Asp Ala Lys

50                  55                  60
Lys Leu Ala Lys Gln Leu Met Gly Asn Asp Lys Leu Ala Asp Ala Ala
65                  70                  75                  80
Tyr Met Trp Trp Gln His Asn Arg Val Thr Leu Asp Gln Ile Asp Thr
                85                  90                  95
Phe Leu Lys Leu Ala Ser Arg Lys Thr Gln Gly Ala Lys Tyr Asn Gln
            100                 105                 110
Ile Tyr Asn Ser Tyr Met Met His Leu Gly Leu Thr Gly Tyr
        115                 120                 125

<210> 8
<211> 381
<212> DNA
<213> Artificial Sequence

<220>
<223> cDNA

<400> 8
atggaccaaa ccaaggtcct ggtgtatggg acgccagctc actacataca cgattcagcc      60

ggcagaagac ttcttcgcaa gaacgaagag aatgaagaaa cgtctgagga gcgagcccca     120

aatttcaatt tggcgaatct aaatgaggag atgtttaatg tggctgcgtt gacgcacaga     180

gcagatgcca aaaagctagc gaaacagctt atgggtaatg ataagctggc ggatgctgca     240

tacatgtggt ggcagcacaa cagggttacg ctagaccaga ttgacacgtt cctgaagctt     300

gcaagccgca agacgcaagg cgcaaagtac aatcagatct acaatagcta catgatgcac     360

ctggggctca ctggatatta a                                               381

<210> 9
<211> 126
<212> PRT
<213> Artificial Sequence

<220>
<223> Avr3a-HM without signal sequence

<400> 9
Met Asp Gln Thr Lys Val Leu Val Tyr Gly Thr Pro Ala His Tyr Ile
1               5                   10                  15
His Asp Ser Ala Gly Arg Arg Leu Leu Arg Lys Asn Glu Glu Asn Glu
            20                  25                  30
Glu Thr Ser Glu Glu Arg Ala Pro Asn Phe Asn Leu Ala Asn Leu Asn
        35                  40                  45
Glu Glu Met Phe Asn Val Ala Ala Leu Thr His Arg Ala Asp Ala Lys
    50                  55                  60
Lys Leu Ala Lys Gln Leu Met Gly Asn Asp Lys Leu Ala Asp Ala Ala
65                  70                  75                  80
Tyr Met Trp Trp Gln His Asn Arg Val Thr Leu Asp Gln Ile Asp Thr
                85                  90                  95
Phe Leu Lys Leu Ala Ser Arg Lys Thr Gln Gly Ala Lys Tyr Asn Gln
            100                 105                 110
Ile Tyr Asn Ser Tyr Met Met His Leu Gly Leu Thr Gly Tyr
        115                 120                 125

<210> 10

<211> 381
<212> DNA
<213> Artificial Sequence

<220>
<223> cDNA

<400> 10
atggaccaaa ccaaggtcct ggtgtatggg acgccagctc actacataca cgattcagcc 60

ggcagaagac ttcttcgcaa gaacgaagag aatgaagaaa cgtctgagga gcgagcccca 120

aatttcaatt tggcgaatct aaatgaggag atgtttaatg tggctgcgtt gacggataga 180

gcagatgcca aaaagctagc gaaacagctt atgggtaatg ataagctggc ggatgctgca 240

tacatgtggt ggcagcacaa cagggttacg ctagaccaga ttgacacgtt cctgaagctt 300

gcaagccgca agacgcaagg cgcaaagtac aatcagatct acaatagcta catgatgcac 360

ctggggctca ctggatatta a 381

<210> 11
<211> 126
<212> PRT
<213> Artificial Sequence

<220>
<223> Avr3a-DM without signal sequence

<400> 11
Met Asp Gln Thr Lys Val Leu Val Tyr Gly Thr Pro Ala His Tyr Ile
1               5                   10                  15
His Asp Ser Ala Gly Arg Arg Leu Leu Arg Lys Asn Glu Glu Asn Glu
            20                  25                  30
Glu Thr Ser Glu Glu Arg Ala Pro Asn Phe Asn Leu Ala Asn Leu Asn
        35                  40                  45
Glu Glu Met Phe Asn Val Ala Ala Leu Thr Asp Arg Ala Asp Ala Lys
    50                  55                  60
Lys Leu Ala Lys Gln Leu Met Gly Asn Asp Lys Leu Ala Asp Ala Ala
65                  70                  75                  80
Tyr Met Trp Trp Gln His Asn Arg Val Thr Leu Asp Gln Ile Asp Thr
                85                  90                  95
Phe Leu Lys Leu Ala Ser Arg Lys Thr Gln Gly Ala Lys Tyr Asn Gln
            100                 105                 110
Ile Tyr Asn Ser Tyr Met Met His Leu Gly Leu Thr Gly Tyr
        115                 120                 125

<210> 12
<211> 381
<212> DNA
<213> Artificial Sequence

<220>
<223> cDNA

<400> 12
atggaccaaa ccaaggtcct ggtgtatggg acgccagctc actacataca cgattcagcc 60

ggcagaagac ttcttcgcaa gaacgaagag aatgaagaaa cgtctgagga gcgagcccca     120

aatttcaatt tggcgaatct aaatgaggag atgtttaatg tggctgcgtt gacggagaga     180

gcagatgcca aaaagctagc gaaacagctt atgggtaatg ataagctggc ggatgctgca     240

tacatgtggt ggcagcacaa cagggttacg ctagaccaga ttgacacgtt cctgaagctt     300

gcaagccgca agacgcaagg cgcaaagtac aatcagatct acaatagcta catgatgcac     360

ctggggctca ctggatatta a                                               381

<210> 13
<211> 126
<212> PRT
<213> Artificial Sequence

<220>
<223> Avr3a-EM ohne Signalsequenz

<400> 13
Met Asp Gln Thr Lys Val Leu Val Tyr Gly Thr Pro Ala His Tyr Ile
1               5                   10                  15
His Asp Ser Ala Gly Arg Arg Leu Leu Arg Lys Asn Glu Glu Asn Glu
            20                  25                  30
Glu Thr Ser Glu Glu Arg Ala Pro Asn Phe Asn Leu Ala Asn Leu Asn
        35                  40                  45
Glu Glu Met Phe Asn Val Ala Ala Leu Thr Glu Arg Ala Asp Ala Lys
    50                  55                  60
Lys Leu Ala Lys Gln Leu Met Gly Asn Asp Lys Leu Ala Asp Ala Ala
65                  70                  75                  80
Tyr Met Trp Trp Gln His Asn Arg Val Thr Leu Asp Gln Ile Asp Thr
                85                  90                  95
Phe Leu Lys Leu Ala Ser Arg Lys Thr Gln Gly Ala Lys Tyr Asn Gln
            100                 105                 110
Ile Tyr Asn Ser Tyr Met Met His Leu Gly Leu Thr Gly Tyr
        115                 120                 125

<210> 14
<211> 381
<212> DNA
<213> Artificial Sequence

<220>
<223> cDNA

<400> 14
atggaccaaa ccaaggtcct ggtgtatggg acgccagctc actacataca cgattcagcc      60

ggcagaagac ttcttcgcaa gaacgaagag aatgaagaaa cgtctgagga gcgagcccca     120

aatttcaatt tggcgaatct aaatgaggag atgtttaatg tggctgcgtt gacgttcaga     180

gcagatgcca aaaagctagc gaaacagctt atgggtaatg ataagctggc ggatgctgca     240

tacatgtggt ggcagcacaa cagggttacg ctagaccaga ttgacacgtt cctgaagctt     300

gcaagccgca agacgcaagg cgcaaagtac aatcagatct acaatagcta catgatgcac     360

ctggggctca ctggatatta a                                               381

<210> 15
<211> 126
<212> PRT
<213> Artificial Sequence

<220>
<223> Avr3a-FM without signal sequence

<400> 15
Met Asp Gln Thr Lys Val Leu Val Tyr Gly Thr Pro Ala His Tyr Ile
1               5                   10                  15
His Asp Ser Ala Gly Arg Arg Leu Leu Arg Lys Asn Glu Glu Asn Glu
            20                  25                  30
Glu Thr Ser Glu Glu Arg Ala Pro Asn Phe Asn Leu Ala Asn Leu Asn
        35                  40                  45
Glu Glu Met Phe Asn Val Ala Ala Leu Thr Phe Arg Ala Asp Ala Lys
    50                  55                  60
Lys Leu Ala Lys Gln Leu Met Gly Asn Asp Lys Leu Ala Asp Ala Ala
65                  70                  75                  80
Tyr Met Trp Trp Gln His Asn Arg Val Thr Leu Asp Gln Ile Asp Thr
                85                  90                  95
Phe Leu Lys Leu Ala Ser Arg Lys Thr Gln Gly Ala Lys Tyr Asn Gln
            100                 105                 110
Ile Tyr Asn Ser Tyr Met Met His Leu Gly Leu Thr Gly Tyr
        115                 120                 125

<210> 16
<211> 381
<212> DNA
<213> Artificial Sequence

<220>
<223> cDNA

<400> 16
atggaccaaa ccaaggtcct ggtgtatggg acgccagctc actacataca cgattcagcc      60

ggcagaagac ttcttcgcaa gaacgaagag aatgaagaaa cgtctgagga gcgagcccca     120

aatttcaatt tggcgaatct aaatgaggag atgtttaatg tggctgcgtt gacgtacaga     180

gcagatgcca aaaagctagc gaaacagctt atgggtaatg ataagctggc ggatgctgca     240

tacatgtggt ggcagcacaa cagggttacg ctagaccaga ttgacacgtt cctgaagctt     300

gcaagccgca agacgcaagg cgcaaagtac aatcagatct acaatagcta catgatgcac     360

ctggggctca ctggatatta a                                               381

<210> 17
<211> 126
<212> PRT
<213> Artificial Sequence

<220>
<223> Avr3a-YM without signal sequence

<400> 17
Met Asp Gln Thr Lys Val Leu Val Tyr Gly Thr Pro Ala His Tyr Ile
1               5                   10                  15
His Asp Ser Ala Gly Arg Arg Leu Leu Arg Lys Asn Glu Glu Asn Glu
            20                  25                  30
Glu Thr Ser Glu Glu Arg Ala Pro Asn Phe Asn Leu Ala Asn Leu Asn
        35                  40                  45
Glu Glu Met Phe Asn Val Ala Ala Leu Thr Tyr Arg Ala Asp Ala Lys
    50                  55                  60
Lys Leu Ala Lys Gln Leu Met Gly Asn Asp Lys Leu Ala Asp Ala Ala
65                  70                  75                  80
Tyr Met Trp Trp Gln His Asn Arg Val Thr Leu Asp Gln Ile Asp Thr
                85                  90                  95
Phe Leu Lys Leu Ala Ser Arg Lys Thr Gln Gly Ala Lys Tyr Asn Gln
            100                 105                 110
Ile Tyr Asn Ser Tyr Met Met His Leu Gly Leu Thr Gly Tyr
        115                 120                 125

<210> 18
<211> 381
<212> DNA
<213> Artificial Sequence

<220>
<223> cDNA

<400> 18
atggaccaaa ccaaggtcct ggtgtatggg acgccagctc actacataca cgattcagcc      60

ggcagaagac ttcttcgcaa gaacgaagag aatgaagaaa cgtctgagga gcgagcccca     120

aatttcaatt tggcgaatct aaatgaggag atgtttaatg tggctgcgtt gacgtggaga     180

gcagatgcca aaaagctagc gaaacagctt atgggtaatg ataagctggc ggatgctgca     240

tacatgtggt ggcagcacaa cagggttacg ctagaccaga ttgacacgtt cctgaagctt     300

gcaagccgca agacgcaagg cgcaaagtac aatcagatct acaatagcta catgatgcac     360

ctggggctca ctggatatta a                                               381

<210> 19
<211> 126
<212> PRT
<213> Artificial Sequence

<220>
<223> Avr3a-WM without signal sequence

<400> 19
Met Asp Gln Thr Lys Val Leu Val Tyr Gly Thr Pro Ala His Tyr Ile
1               5                   10                  15
His Asp Ser Ala Gly Arg Arg Leu Leu Arg Lys Asn Glu Glu Asn Glu
            20                  25                  30
Glu Thr Ser Glu Glu Arg Ala Pro Asn Phe Asn Leu Ala Asn Leu Asn
        35                  40                  45
Glu Glu Met Phe Asn Val Ala Ala Leu Thr Trp Arg Ala Asp Ala Lys
    50                  55                  60
Lys Leu Ala Lys Gln Leu Met Gly Asn Asp Lys Leu Ala Asp Ala Ala
65                  70                  75                  80

Tyr Met Trp Trp Gln His Asn Arg Val Thr Leu Asp Gln Ile Asp Thr
                85                  90                  95
Phe Leu Lys Leu Ala Ser Arg Lys Thr Gln Gly Ala Lys Tyr Asn Gln
            100                 105                 110
Ile Tyr Asn Ser Tyr Met Met His Leu Gly Leu Thr Gly Tyr
        115                 120                 125

<210> 20
<211> 381
<212> DNA
<213> Artificial Sequence

<220>
<223> cDNA

<400> 20
atggaccaaa ccaaggtcct ggtgtatggg acgccagctc actacataca cgattcagcc      60

ggcagaagac ttcttcgcaa gaacgaagag aatgaagaaa cgtctgagga gcgagcccca     120

aatttcaatt tggcgaatct aaatgaggag atgtttaatg tggctgcgtt gacgccaaga     180

gcagatgcca aaaagctagc gaaacagctt atgggtaatg ataagctggc ggatgctgca     240

tacatgtggt ggcagcacaa cagggttacg ctagaccaga ttgacacgtt cctgaagctt     300

gcaagccgca agacgcaagg cgcaaagtac aatcagatct acaatagcta catgatgcac     360

ctggggctca ctggatatta a                                               381

<210> 21
<211> 126
<212> PRT
<213> Artificial Sequence

<220>
<223> Avr3a-PM without signal sequence

<400> 21
Met Asp Gln Thr Lys Val Leu Val Tyr Gly Thr Pro Ala His Tyr Ile
1               5                   10                  15
His Asp Ser Ala Gly Arg Arg Leu Leu Arg Lys Asn Glu Glu Asn Glu
            20                  25                  30
Glu Thr Ser Glu Glu Arg Ala Pro Asn Phe Asn Leu Ala Asn Leu Asn
        35                  40                  45
Glu Glu Met Phe Asn Val Ala Ala Leu Thr Pro Arg Ala Asp Ala Lys
    50                  55                  60
Lys Leu Ala Lys Gln Leu Met Gly Asn Asp Lys Leu Ala Asp Ala Ala
65                  70                  75                  80
Tyr Met Trp Trp Gln His Asn Arg Val Thr Leu Asp Gln Ile Asp Thr
                85                  90                  95
Phe Leu Lys Leu Ala Ser Arg Lys Thr Gln Gly Ala Lys Tyr Asn Gln
            100                 105                 110
Ile Tyr Asn Ser Tyr Met Met His Leu Gly Leu Thr Gly Tyr
        115                 120                 125

<210> 22
<211> 381
<212> DNA
<213> Artificial Sequence

<220>
<223> cDNA

<400> 22
atggaccaaa ccaaggtcct ggtgtatggg acgccagctc actacataca cgattcagcc 60

ggcagaagac ttcttcgcaa gaacgaagag aatgaagaaa cgtctgagga gcgagcccca 120

aatttcaatt tggcgaatct aaatgaggag atgtttaatg tggctgcgtt gacgtgtaga 180

gcagatgcca aaaagctagc gaaacagctt atgggtaatg ataagctggc ggatgctgca 240

tacatgtggt ggcagcacaa cagggttacg ctagaccaga ttgacacgtt cctgaagctt 300

gcaagccgca agacgcaagg cgcaaagtac aatcagatct acaatagcta catgatgcac 360

ctggggctca ctggatatta a 381


<210> 23
<211> 126
<212> PRT
<213> Artificial Sequence


<220>
<223> Avr3a-CM without signal sequence

<400> 23
Met Asp Gln Thr Lys Val Leu Val Tyr Gly Thr Pro Ala His Tyr Ile
1               5                   10                  15
His Asp Ser Ala Gly Arg Arg Leu Leu Arg Lys Asn Glu Glu Asn Glu
            20                  25                  30
Glu Thr Ser Glu Glu Arg Ala Pro Asn Phe Asn Leu Ala Asn Leu Asn
        35                  40                  45
Glu Glu Met Phe Asn Val Ala Ala Leu Thr Cys Arg Ala Asp Ala Lys
    50                  55                  60
Lys Leu Ala Lys Gln Leu Met Gly Asn Asp Lys Leu Ala Asp Ala Ala
65                  70                  75                  80
Tyr Met Trp Trp Gln His Asn Arg Val Thr Leu Asp Gln Ile Asp Thr
                85                  90                  95
Phe Leu Lys Leu Ala Ser Arg Lys Thr Gln Gly Ala Lys Tyr Asn Gln
            100                 105                 110
Ile Tyr Asn Ser Tyr Met Met His Leu Gly Leu Thr Gly Tyr
        115                 120                 125

<210> 24
<211> 381
<212> DNA
<213> Artificial Sequence


<220>
<223> cDNA

<400> 24
atggaccaaa ccaaggtcct ggtgtatggg acgccagctc actacataca cgattcagcc 60

ggcagaagac ttcttcgcaa gaacgaagag aatgaagaaa cgtctgagga gcgagcccca 120

aatttcaatt tggcgaatct aaatgaggag atgtttaatg tggctgcgtt gacgaacaga 180

gcagatgcca aaaagctagc gaaacagctt atgggtaatg ataagctggc ggatgctgca 240

tacatgtggt ggcagcacaa cagggttacg ctagaccaga ttgacacgtt cctgaagctt 300

gcaagccgca agacgcaagg cgcaaagtac aatcagatct acaatagcta catgatgcac 360

ctggggctca ctggatatta a 381

<210> 25
<211> 126
<212> PRT
<213> Artificial Sequence

<220>
<223> Avr3a-NM without signal sequence

<400> 25
Met Asp Gln Thr Lys Val Leu Val Tyr Gly Thr Pro Ala His Tyr Ile
1               5                   10                  15
His Asp Ser Ala Gly Arg Arg Leu Leu Arg Lys Asn Glu Glu Asn Glu
            20                  25                  30
Glu Thr Ser Glu Glu Arg Ala Pro Asn Phe Asn Leu Ala Asn Leu Asn
        35                  40                  45
Glu Glu Met Phe Asn Val Ala Ala Leu Thr Asn Arg Ala Asp Ala Lys
    50                  55                  60
Lys Leu Ala Lys Gln Leu Met Gly Asn Asp Lys Leu Ala Asp Ala Ala
65                  70                  75                  80
Tyr Met Trp Trp Gln His Asn Arg Val Thr Leu Asp Gln Ile Asp Thr
                85                  90                  95
Phe Leu Lys Leu Ala Ser Arg Lys Thr Gln Gly Ala Lys Tyr Asn Gln
            100                 105                 110
Ile Tyr Asn Ser Tyr Met Met His Leu Gly Leu Thr Gly Tyr
        115                 120                 125

<210> 26
<211> 381
<212> DNA
<213> Artificial Sequence

<220>
<223> cDNA

<400> 26
atggaccaaa ccaaggtcct ggtgtatggg acgccagctc actacataca cgattcagcc 60

ggcagaagac ttcttcgcaa gaacgaagag aatgaagaaa cgtctgagga gcgagcccca 120

aatttcaatt tggcgaatct aaatgaggag atgtttaatg tggctgcgtt gacgcaaaga 180

gcagatgcca aaaagctagc gaaacagctt atgggtaatg ataagctggc ggatgctgca 240

tacatgtggt ggcagcacaa cagggttacg ctagaccaga ttgacacgtt cctgaagctt 300

gcaagccgca agacgcaagg cgcaaagtac aatcagatct acaatagcta catgatgcac 360

ctggggctca ctggatatta a 381

<210> 27

<211> 126
<212> PRT
<213> Artificial Sequence

<220>
<223> Avr3a-QM without signal sequence

<400> 27
Met Asp Gln Thr Lys Val Leu Val Tyr Gly Thr Pro Ala His Tyr Ile
1               5                   10                  15
His Asp Ser Ala Gly Arg Arg Leu Leu Arg Lys Asn Glu Glu Asn Glu
            20                  25                  30
Glu Thr Ser Glu Glu Arg Ala Pro Asn Phe Asn Leu Ala Asn Leu Asn
        35                  40                  45
Glu Glu Met Phe Asn Val Ala Ala Leu Thr Gln Arg Ala Asp Ala Lys
    50                  55                  60
Lys Leu Ala Lys Gln Leu Met Gly Asn Asp Lys Leu Ala Asp Ala Ala
65                  70                  75                  80
Tyr Met Trp Trp Gln His Asn Arg Val Thr Leu Asp Gln Ile Asp Thr
                85                  90                  95
Phe Leu Lys Leu Ala Ser Arg Lys Thr Gln Gly Ala Lys Tyr Asn Gln
            100                 105                 110
Ile Tyr Asn Ser Tyr Met Met His Leu Gly Leu Thr Gly Tyr
        115                 120                 125

<210> 28
<211> 381
<212> DNA
<213> Artificial Sequence

<220>
<223> cDNA

<400> 28
atggaccaaa ccaaggtcct ggtgtatggg acgccagctc actacataca cgattcagcc      60

ggcagaagac ttcttcgcaa gaacgaagag aatgaagaaa cgtctgagga gcgagcccca     120

aatttcaatt tggcgaatct aaatgaggag atgtttaatg tggctgcgtt gacgacgaga     180

gcagatgcca aaaagctagc gaaacagctt atgggtaatg ataagctggc ggatgctgca     240

tacatgtggt ggcagcacaa cagggttacg ctagaccaga ttgacacgtt cctgaagctt     300

gcaagccgca agacgcaagg cgcaaagtac aatcagatct acaatagcta catgatgcac     360

ctggggctca ctggatatta a                                               381

<210> 29
<211> 126
<212> PRT
<213> Artificial Sequence

<220>
<223> Avr3a-TM without signal sequence

<400> 29
Met Asp Gln Thr Lys Val Leu Val Tyr Gly Thr Pro Ala His Tyr Ile
1               5                   10                  15

His Asp Ser Ala Gly Arg Arg Leu Leu Arg Lys Asn Glu Glu Asn Glu
            20                  25                  30
Glu Thr Ser Glu Glu Arg Ala Pro Asn Phe Asn Leu Ala Asn Leu Asn
        35                  40                  45
Glu Glu Met Phe Asn Val Ala Ala Leu Thr Thr Arg Ala Asp Ala Lys
    50                  55                  60
Lys Leu Ala Lys Gln Leu Met Gly Asn Asp Lys Leu Ala Asp Ala Ala
65                  70                  75                  80
Tyr Met Trp Trp Gln His Asn Arg Val Thr Leu Asp Gln Ile Asp Thr
                85                  90                  95
Phe Leu Lys Leu Ala Ser Arg Lys Thr Gln Gly Ala Lys Tyr Asn Gln
            100                 105                 110
Ile Tyr Asn Ser Tyr Met Met His Leu Gly Leu Thr Gly Tyr
        115                 120                 125

<210> 30
<211> 381
<212> DNA
<213> Artificial Sequence

<220>
<223> cDNA

<400> 30
atggaccaaa ccaaggtcct ggtgtatggg acgccagctc actacataca cgattcagcc      60

ggcagaagac ttcttcgcaa gaacgaagag aatgaagaaa cgtctgagga gcgagcccca     120

aatttcaatt tggcgaatct aaatgaggag atgtttaatg tggctgcgtt gacgtctaga     180

gcagatgcca aaaagctagc gaaacagctt atgggtaatg ataagctggc ggatgctgca     240

tacatgtggt ggcagcacaa cagggttacg ctagaccaga ttgacacgtt cctgaagctt     300

gcaagccgca agacgcaagg cgcaaagtac aatcagatct acaatagcta catgatgcac     360

ctggggctca ctggatatta a                                               381

<210> 31
<211> 126
<212> PRT
<213> Artificial Sequence

<220>
<223> Avr3a-SM without signal sequence

<400> 31
Met Asp Gln Thr Lys Val Leu Val Tyr Gly Thr Pro Ala His Tyr Ile
1               5                   10                  15
His Asp Ser Ala Gly Arg Arg Leu Leu Arg Lys Asn Glu Glu Asn Glu
            20                  25                  30
Glu Thr Ser Glu Glu Arg Ala Pro Asn Phe Asn Leu Ala Asn Leu Asn
        35                  40                  45
Glu Glu Met Phe Asn Val Ala Ala Leu Thr Ser Arg Ala Asp Ala Lys
    50                  55                  60
Lys Leu Ala Lys Gln Leu Met Gly Asn Asp Lys Leu Ala Asp Ala Ala
65                  70                  75                  80
Tyr Met Trp Trp Gln His Asn Arg Val Thr Leu Asp Gln Ile Asp Thr
                85                  90                  95
Phe Leu Lys Leu Ala Ser Arg Lys Thr Gln Gly Ala Lys Tyr Asn Gln

                100                 105                 110
Ile Tyr Asn Ser Tyr Met Met His Leu Gly Leu Thr Gly Tyr
        115                 120                 125

<210> 32
<211> 381
<212> DNA
<213> Artificial Sequence


<220>
<223> cDNA

<400> 32
atggaccaaa ccaaggtcct ggtgtatggg acgccagctc actacataca cgattcagcc      60

ggcagaagac ttcttcgcaa gaacgaagag aatgaagaaa cgtctgagga gcgagcccca     120

aatttcaatt tggcgaatct aaatgaggag atgtttaatg tggctgcgtt gacgatgaga     180

gcagatgcca aaaagctagc gaaacagctt atgggtaatg ataagctggc ggatgctgca     240

tacatgtggt ggcagcacaa cagggttacg ctagaccaga ttgacacgtt cctgaagctt     300

gcaagccgca agacgcaagg cgcaaagtac aatcagatct acaatagcta catgatgcac     360

ctggggctca ctggatatta a                                               381


<210> 33
<211> 126
<212> PRT
<213> Artificial Sequence


<220>
<223> Avr3a-MM without signal sequence

<400> 33
Met Asp Gln Thr Lys Val Leu Val Tyr Gly Thr Pro Ala His Tyr Ile
1               5                   10                  15
His Asp Ser Ala Gly Arg Arg Leu Leu Arg Lys Asn Glu Glu Asn Glu
            20                  25                  30
Glu Thr Ser Glu Glu Arg Ala Pro Asn Phe Asn Leu Ala Asn Leu Asn
        35                  40                  45
Glu Glu Met Phe Asn Val Ala Ala Leu Thr Met Arg Ala Asp Ala Lys
    50                  55                  60
Lys Leu Ala Lys Gln Leu Met Gly Asn Asp Lys Leu Ala Asp Ala Ala
65                  70                  75                  80
Tyr Met Trp Trp Gln His Asn Arg Val Thr Leu Asp Gln Ile Asp Thr
                85                  90                  95
Phe Leu Lys Leu Ala Ser Arg Lys Thr Gln Gly Ala Lys Tyr Asn Gln
            100                 105                 110
Ile Tyr Asn Ser Tyr Met Met His Leu Gly Leu Thr Gly Tyr
        115                 120                 125

<210> 34
<211> 381
<212> DNA
<213> Artificial Sequence


<220>

<223> cDNA

<400> 34
atggaccaaa ccaaggtcct ggtgtatggg acgccagctc actacataca cgattcagcc      60

ggcagaagac ttcttcgcaa gaacgaagag aatgaagaaa cgtctgagga gcgagcccca     120

aatttcaatt tggcgaatct aaatgaggag atgtttaatg tggctgcgtt gacgggcaga     180

gcagatgcca aaaagctagc gaaacagctt atgggtaatg ataagctggc ggatgctgca     240

tacatgtggt ggcagcacaa cagggttacg ctagaccaga ttgacacgtt cctgaagctt     300

gcaagccgca agacgcaagg cgcaaagtac aatcagatct acaatagcta catgatgcac     360

ctggggctca ctggatatta a                                               381


<210> 35
<211> 126
<212> PRT
<213> Artificial Sequence


<220>
<223> Avr3a-GM without signal sequence

<400> 35
Met Asp Gln Thr Lys Val Leu Val Tyr Gly Thr Pro Ala His Tyr Ile
1               5                   10                  15
His Asp Ser Ala Gly Arg Arg Leu Leu Arg Lys Asn Glu Glu Asn Glu
            20                  25                  30
Glu Thr Ser Glu Glu Arg Ala Pro Asn Phe Asn Leu Ala Asn Leu Asn
        35                  40                  45
Glu Glu Met Phe Asn Val Ala Ala Leu Thr Gly Arg Ala Asp Ala Lys
    50                  55                  60
Lys Leu Ala Lys Gln Leu Met Gly Asn Asp Lys Leu Ala Asp Ala Ala
65                  70                  75                  80
Tyr Met Trp Trp Gln His Asn Arg Val Thr Leu Asp Gln Ile Asp Thr
                85                  90                  95
Phe Leu Lys Leu Ala Ser Arg Lys Thr Gln Gly Ala Lys Tyr Asn Gln
            100                 105                 110
Ile Tyr Asn Ser Tyr Met Met His Leu Gly Leu Thr Gly Tyr
        115                 120                 125

<210> 36
<211> 381
<212> DNA
<213> Artificial Sequence


<220>
<223> cDNA

<400> 36
atggaccaaa ccaaggtcct ggtgtatggg acgccagctc actacataca cgattcagcc      60

ggcagaagac ttcttcgcaa gaacgaagag aatgaagaaa cgtctgagga gcgagcccca     120

aatttcaatt tggcgaatct aaatgaggag atgtttaatg tggctgcgtt gacggccaga     180

gcagatgcca aaaagctagc gaaacagctt atgggtaatg ataagctggc ggatgctgca     240

tacatgtggt ggcagcacaa cagggttacg ctagaccaga ttgacacgtt cctgaagctt 300

gcaagccgca agacgcaagg cgcaaagtac aatcagatct acaatagcta catgatgcac 360

ctggggctca ctggatatta a 381

<210> 37
<211> 126
<212> PRT
<213> Artificial Sequence

<220>
<223> Avr3a-AM without signal sequence

<400> 37
Met Asp Gln Thr Lys Val Leu Val Tyr Gly Thr Pro Ala His Tyr Ile
1               5                   10                  15
His Asp Ser Ala Gly Arg Arg Leu Leu Arg Lys Asn Glu Glu Asn Glu
            20                  25                  30
Glu Thr Ser Glu Glu Arg Ala Pro Asn Phe Asn Leu Ala Asn Leu Asn
        35                  40                  45
Glu Glu Met Phe Asn Val Ala Ala Leu Thr Ala Arg Ala Asp Ala Lys
    50                  55                  60
Lys Leu Ala Lys Gln Leu Met Gly Asn Asp Lys Leu Ala Asp Ala Ala
65                  70                  75                  80
Tyr Met Trp Trp Gln His Asn Arg Val Thr Leu Asp Gln Ile Asp Thr
                85                  90                  95
Phe Leu Lys Leu Ala Ser Arg Lys Thr Gln Gly Ala Lys Tyr Asn Gln
            100                 105                 110
Ile Tyr Asn Ser Tyr Met Met His Leu Gly Leu Thr Gly Tyr
        115                 120                 125

<210> 38
<211> 381
<212> DNA
<213> Artificial Sequence

<220>
<223> cDNA

<400> 38
atggaccaaa ccaaggtcct ggtgtatggg acgccagctc actacataca cgattcagcc 60

ggcagaagac ttcttcgcaa gaacgaagag aatgaagaaa cgtctgagga gcgagcccca 120

aatttcaatt tggcgaatct aaatgaggag atgtttaatg tggctgcgtt gacgcttaga 180

gcagatgcca aaaagctagc gaaacagctt atgggtaatg ataagctggc ggatgctgca 240

tacatgtggt ggcagcacaa cagggttacg ctagaccaga ttgacacgtt cctgaagctt 300

gcaagccgca agacgcaagg cgcaaagtac aatcagatct acaatagcta catgatgcac 360

ctggggctca ctggatatta a 381

<210> 39
<211> 126
<212> PRT
<213> Artificial Sequence

<220>
<223> Avr3a-LM without signal sequence

<400> 39
Met Asp Gln Thr Lys Val Leu Val Tyr Gly Thr Pro Ala His Tyr Ile
1               5                   10                  15
His Asp Ser Ala Gly Arg Arg Leu Leu Arg Lys Asn Glu Glu Asn Glu
            20                  25                  30
Glu Thr Ser Glu Glu Arg Ala Pro Asn Phe Asn Leu Ala Asn Leu Asn
        35                  40                  45
Glu Glu Met Phe Asn Val Ala Ala Leu Thr Leu Arg Ala Asp Ala Lys
    50                  55                  60
Lys Leu Ala Lys Gln Leu Met Gly Asn Asp Lys Leu Ala Asp Ala Ala
65                  70                  75                  80
Tyr Met Trp Trp Gln His Asn Arg Val Thr Leu Asp Gln Ile Asp Thr
                85                  90                  95
Phe Leu Lys Leu Ala Ser Arg Lys Thr Gln Gly Ala Lys Tyr Asn Gln
            100                 105                 110
Ile Tyr Asn Ser Tyr Met Met His Leu Gly Leu Thr Gly Tyr
        115                 120                 125

<210> 40
<211> 381
<212> DNA
<213> Artificial Sequence


<220>
<223> cDNA

<400> 40
atggaccaaa ccaaggtcct ggtgtatggg acgccagctc actacataca cgattcagcc      60

ggcagaagac ttcttcgcaa gaacgaagag aatgaagaaa cgtctgagga gcgagcccca     120

aatttcaatt tggcgaatct aaatgaggag atgtttaatg tggctgcgtt gacggtgaga     180

gcagatgcca aaaagctagc gaaacagctt atgggtaatg ataagctggc ggatgctgca     240

tacatgtggt ggcagcacaa cagggttacg ctagaccaga ttgacacgtt cctgaagctt     300

gcaagccgca agacgcaagg cgcaaagtac aatcagatct acaatagcta catgatgcac     360

ctggggctca ctggatatta a                                               381


<210> 41
<211> 126
<212> PRT
<213> Artificial Sequence


<220>
<223> Avr3a-VM without signal sequence

<400> 41
Met Asp Gln Thr Lys Val Leu Val Tyr Gly Thr Pro Ala His Tyr Ile
1               5                   10                  15
His Asp Ser Ala Gly Arg Arg Leu Leu Arg Lys Asn Glu Glu Asn Glu
            20                  25                  30
Glu Thr Ser Glu Glu Arg Ala Pro Asn Phe Asn Leu Ala Asn Leu Asn

35                  40                  45
Glu Glu Met Phe Asn Val Ala Ala Leu Thr Val Arg Ala Asp Ala Lys
    50                  55                  60
Lys Leu Ala Lys Gln Leu Met Gly Asn Asp Lys Leu Ala Asp Ala Ala
65                  70                  75                  80
Tyr Met Trp Trp Gln His Asn Arg Val Thr Leu Asp Gln Ile Asp Thr
                85                  90                  95
Phe Leu Lys Leu Ala Ser Arg Lys Thr Gln Gly Ala Lys Tyr Asn Gln
            100                 105                 110
Ile Tyr Asn Ser Tyr Met Met His Leu Gly Leu Thr Gly Tyr
        115                 120                 125

<210> 42
<211> 381
<212> RNA
<213> Artificial Sequence

<220>
<223> cDNA

<400> 42
atggaccaaa ccaaggtcct ggtgtatggg acgccagctc actacataca cgattcagcc      60

ggcagaagac ttcttcgcaa gaacgaagag aatgaagaaa cgtctgagga gcgagcccca     120

aatttcaatt tggcgaatct aaatgaggag atgtttaatg tggctgcgtt gacgattaga     180

gcagatgcca aaaagctagc gaaacagctt atgggtaatg ataagctggc ggatgctgca     240

tacatgtggt ggcagcacaa cagggttacg ctagaccaga ttgacacgtt cctgaagctt     300

gcaagccgca agacgcaagg cgcaaagtac aatcagatct acaatagcta catgatgcac     360

ctggggctca ctggatatta a                                               381

<210> 43
<211> 126
<212> PRT
<213> Artificial Sequence

<220>
<223> Avr3a-IM without signal sequence

<400> 43
Met Asp Gln Thr Lys Val Leu Val Tyr Gly Thr Pro Ala His Tyr Ile
1               5                   10                  15
His Asp Ser Ala Gly Arg Arg Leu Leu Arg Lys Asn Glu Glu Asn Glu
            20                  25                  30
Glu Thr Ser Glu Glu Arg Ala Pro Asn Phe Asn Leu Ala Asn Leu Asn
        35                  40                  45
Glu Glu Met Phe Asn Val Ala Ala Leu Thr Ile Arg Ala Asp Ala Lys
    50                  55                  60
Lys Leu Ala Lys Gln Leu Met Gly Asn Asp Lys Leu Ala Asp Ala Ala
65                  70                  75                  80
Tyr Met Trp Trp Gln His Asn Arg Val Thr Leu Asp Gln Ile Asp Thr
                85                  90                  95
Phe Leu Lys Leu Ala Ser Arg Lys Thr Gln Gly Ala Lys Tyr Asn Gln
            100                 105                 110
Ile Tyr Asn Ser Tyr Met Met His Leu Gly Leu Thr Gly Tyr
        115                 120                 125

<210> 44
<211> 2544
<212> DNA
<213> Artificial Sequence

<220>
<223> cDNA

<400> 44
atggctgatg cctttctatc atttgcagtt caaaaattgg gtgatttcct aatacagaaa 60

gtttccctgc gtaaaagtct cagagatgaa attagatggc tgataaatga gctactcttc 120

atacggtctt tcctcagaga tgcagaacaa aagcagtgcg gagatcaaag agttcaacaa 180

tgggtgtttg agatcaactc tattgctaat gatgctgttg ctatactcga gacttatagc 240

tttgaggctg gtaaaggtgc tagtcgtctc aaggcttgca cttgcatatg taggaaggag 300

aagaaattct acaatgttgc cgaggagatt caatcactca agcaacgaat catggatatc 360

tctcgcaaac gagagactta tggtattaca aatatcaatt ataattcagg agaaaggcca 420

agtaatcagg ttacaacatt gaggagaact acctcatatg tagatgaaca ggattacatt 480

tttgttggct ttcaggatgt tgtacaaaca ttgctagctc aacttctgaa agcagagcct 540

cgtcgaagcg tcctctccat ttatggaatg gggggtttag gcaagaccac tcttgccaga 600

aaactttaca ccagtcctga tatactcaat agctttccta cacgcgcttg gatatgtgtc 660

tctcaagagt acaacacaat ggatcttctt aggactatca taaaatccat ccaaggctgc 720

gccaaggaaa ctctagattt gttggaaaag atggcagaaa tagatctaga aatcaccttt 780

cgtgatctat tgaaagaatg caaatacctt gtggtggttg atgatgtatg gcagagagaa 840

gcatgggaga gtttgaaaag agcattcccg gatggcaaga atggaagcag agtcattatt 900

accacgcgca aagaggatgt cgctgaaaga gtagaccaca gaggttttgt tcataaactt 960

cgtttcctaa gtcaagaaga aagttgggat ctctttcgta ggaaactact tgatgttcga 1020

gcaatggttc cagaaatgga aagtttagct aaggatatgg tggaaaagtg tagaggctta 1080

cctcttgcaa ttgttgtatt gagcggacta ctttcgcata aaaaggggct aaaccaatgg 1140

caaaaggtga aagatcacct ttggaagaac attaaagaag ataaatctat tgaaatctct 1200

aacatactat ccttaagcta caatgatttg tcaactgcgc tcaagcagtg ttttctctac 1260

tttggtattt ttccagaaga tcaagtggta aaggctgatg acataatacg gttgtggatg 1320

gcggagggtt tcatacccag aggagaagaa agaatggagg atgtggctga cggcttcttg 1380

aatgaactga taagacgaag cttggttcaa gtagctaaaa cattttggga aaaagttact 1440

gactgtaggg ttcatgattt acttcgtgat cttgcgatac aaaaggcatt ggaggtaaac 1500

ttctttgacg tttatggtcc aagaagccac tccatatcct ctttatgtat cagacatggc 1560

attcatagtg aaggagaaag gtacctctca tcacttgatc tttctaactt gaagttgagg 1620

tcaattatgt tcttcgatcc agattttcgt aagatgagtc atataaacct caggagtgag 1680

ttccaacatc tgtatgtgtt gtacttggat acgaattttg ggtatgtgtc tatggtacct 1740

gatgccatag gaagtttgta ccacctcaag ttgttaagat tgagaggtat ccatgatatt 1800

ccgtcttcca ttggcaacct caagaattta caaacacttg tcgttgtaaa tggttacaca 1860

tttttttgcc aactaccctg caagacagct gacctaataa atctaagaca tttagttgtt 1920

caatattcag agcctttaaa atgtataaac aaactcacta gtcttcaagt tcttgatggt 1980

gttgcttgtg atcagtggaa agatgttgac cctgttgatt tagtcaatct tcgagaatta 2040

agcatggatc gtatcaggag ctcttactcc ctaaacaaca ttagcagctt gaaaaacctt 2100

agcactctca aattgatttg tggagaacgt caatcatttg catcccttga atttgttaat 2160

tgttgtgaaa agctccagaa attgtggtta caagggagaa tagaggaact gcctcatctg 2220

ttttcaaact ccatcacaat gatggttctg agtttctcag aactgacaga agatccgatg 2280

cctattttgg gaaggtttcc aaacctaagg aatctcaaat tagatggagc ttatgaagga 2340

aaagaaataa tgtgcagtga taacagcttc agtcaactag agttccttca tcttcgtgat 2400

ctttggaagc tagaaagatg ggatttaggc acaagtgcca tgcctctgat taaaggtctt 2460

ggtatccata actgtccaaa tttaaaggag attcctgaga gaatgaaaga catggagctg 2520

ttgaagcgga attatatgtt gtga 2544

<210> 45
<211> 847
<212> PRT
<213> Solanum bulbocastanum

<400> 45
Met Ala Asp Ala Phe Leu Ser Phe Ala Val Gln Lys Leu Gly Asp Phe
1               5                   10                  15
Leu Ile Gln Lys Val Ser Leu Arg Lys Ser Leu Arg Asp Glu Ile Arg
            20                  25                  30
Trp Leu Ile Asn Glu Leu Leu Phe Ile Arg Ser Phe Leu Arg Asp Ala
        35                  40                  45
Glu Gln Lys Gln Cys Gly Asp Gln Arg Val Gln Gln Trp Val Phe Glu
    50                  55                  60
Ile Asn Ser Ile Ala Asn Asp Ala Val Ala Ile Leu Glu Thr Tyr Ser
65                  70                  75                  80
Phe Glu Ala Gly Lys Gly Ala Ser Arg Leu Lys Ala Cys Thr Cys Ile
                85                  90                  95
Cys Arg Lys Glu Lys Lys Phe Tyr Asn Val Ala Glu Glu Ile Gln Ser
            100                 105                 110
Leu Lys Gln Arg Ile Met Asp Ile Ser Arg Lys Arg Glu Thr Tyr Gly
        115                 120                 125
Ile Thr Asn Ile Asn Tyr Asn Ser Gly Glu Arg Pro Ser Asn Gln Val
    130                 135                 140
Thr Thr Leu Arg Arg Thr Thr Ser Tyr Val Asp Glu Gln Asp Tyr Ile
145                 150                 155                 160
Phe Val Gly Phe Gln Asp Val Val Gln Thr Leu Leu Ala Gln Leu Leu

                165                 170                 175
Lys Ala Glu Pro Arg Arg Ser Val Leu Ser Ile Tyr Gly Met Gly Gly
            180                 185                 190
Leu Gly Lys Thr Thr Leu Ala Arg Lys Leu Tyr Thr Ser Pro Asp Ile
        195                 200                 205
Leu Asn Ser Phe Pro Thr Arg Ala Trp Ile Cys Val Ser Gln Glu Tyr
    210                 215                 220
Asn Thr Met Asp Leu Leu Arg Thr Ile Ile Lys Ser Ile Gln Gly Cys
225                 230                 235                 240
Ala Lys Glu Thr Leu Asp Leu Leu Glu Lys Met Ala Glu Ile Asp Leu
                245                 250                 255
Glu Asn His Leu Arg Asp Leu Leu Lys Glu Cys Lys Tyr Leu Val Val
            260                 265                 270
Val Asp Asp Val Trp Gln Arg Glu Ala Trp Glu Ser Leu Lys Arg Ala
        275                 280                 285
Phe Pro Asp Gly Lys Asn Gly Ser Arg Val Ile Ile Thr Thr Arg Lys
    290                 295                 300
Glu Asp Val Ala Glu Arg Val Asp His Arg Gly Phe Val His Lys Leu
305                 310                 315                 320
Arg Phe Leu Ser Gln Glu Glu Ser Trp Asp Leu Phe Arg Arg Lys Leu
                325                 330                 335
Leu Asp Val Arg Ala Met Val Pro Glu Met Glu Ser Leu Ala Lys Asp
            340                 345                 350
Met Val Glu Lys Cys Arg Gly Leu Pro Leu Ala Ile Val Val Leu Ser
        355                 360                 365
Gly Leu Leu Ser His Lys Lys Gly Leu Asn Gln Trp Gln Lys Val Lys
    370                 375                 380
Asp His Leu Trp Lys Asn Ile Lys Glu Asp Lys Ser Ile Glu Ile Ser
385                 390                 395                 400
Asn Ile Leu Ser Leu Ser Tyr Asn Asp Leu Ser Thr Ala Leu Lys Gln
                405                 410                 415
Cys Phe Leu Tyr Phe Gly Ile Phe Pro Glu Asp Gln Val Val Lys Ala
            420                 425                 430
Asp Asp Ile Ile Arg Leu Trp Met Ala Glu Gly Phe Ile Pro Arg Gly
        435                 440                 445
Glu Glu Arg Met Glu Asp Val Ala Asp Gly Phe Leu Asn Glu Leu Ile
    450                 455                 460
Arg Arg Ser Leu Val Gln Val Ala Lys Thr Phe Trp Glu Lys Val Thr
465                 470                 475                 480
Asp Cys Arg Val His Asp Leu Leu Arg Asp Leu Ala Ile Gln Lys Ala
                485                 490                 495
Leu Glu Val Asn Phe Phe Asp Val Tyr Gly Pro Arg Ser His Ser Ile
            500                 505                 510
Ser Ser Leu Cys Ile Arg His Gly Ile His Ser Glu Gly Glu Arg Tyr
        515                 520                 525
Leu Ser Ser Leu Asp Leu Ser Asn Leu Lys Leu Arg Ser Ile Met Phe
    530                 535                 540
Phe Asp Pro Asp Phe Arg Lys Met Ser His Ile Asn Leu Arg Ser Glu
545                 550                 555                 560
Phe Gln His Leu Tyr Val Leu Tyr Leu Asp Thr Asn Phe Gly Tyr Val
                565                 570                 575
Ser Met Val Pro Asp Ala Ile Gly Ser Leu Tyr His Leu Lys Leu Leu
            580                 585                 590
Arg Leu Arg Gly Ile His Asp Ile Pro Ser Ser Ile Gly Asn Leu Lys
        595                 600                 605
Asn Leu Gln Thr Leu Val Val Val Asn Gly Tyr Thr Phe Phe Cys Gln
    610                 615                 620
Leu Pro Cys Lys Thr Ala Asp Leu Ile Asn Leu Arg His Leu Val Val
625                 630                 635                 640
Gln Tyr Ser Glu Pro Leu Lys Cys Ile Asn Lys Leu Thr Ser Leu Gln
                645                 650                 655
Val Leu Asp Gly Val Ala Cys Asp Gln Trp Lys Asp Val Asp Pro Val
            660                 665                 670

Asp Leu Val Asn Leu Arg Glu Leu Ser Met Asp Arg Ile Arg Ser Ser
        675                 680                 685
Tyr Ser Leu Asn Asn Ile Ser Ser Leu Lys Asn Leu Ser Thr Leu Lys
    690                 695                 700
Leu Ile Cys Gly Glu Arg Gln Ser Phe Ala Ser Leu Glu Phe Val Asn
705                 710                 715                 720
Cys Cys Glu Lys Leu Gln Lys Leu Trp Leu Gln Gly Arg Ile Glu Glu
                725                 730                 735
Leu Pro His Leu Phe Ser Asn Ser Ile Thr Met Met Val Leu Ser Phe
            740                 745                 750
Ser Glu Leu Thr Glu Asp Pro Met Pro Ile Leu Gly Arg Phe Pro Asn
        755                 760                 765
Leu Arg Asn Leu Lys Leu Asp Gly Ala Tyr Glu Gly Lys Glu Ile Met
    770                 775                 780
Cys Ser Asp Asn Ser Phe Ser Gln Leu Glu Phe Leu His Leu Arg Asp
785                 790                 795                 800
Leu Trp Lys Leu Glu Arg Trp Asp Leu Gly Thr Ser Ala Met Pro Leu
                805                 810                 815
Ile Lys Gly Leu Gly Ile His Asn Cys Pro Asn Leu Lys Glu Ile Pro
            820                 825                 830
Glu Arg Met Lys Asp Met Glu Leu Leu Lys Arg Asn Tyr Met Leu
        835                 840                 845

<210> 46
<211> 351
<212> DNA
<213> Artificial Sequence

<220>
<223> cDNA

<400> 46
atgcgtctcg cctacatttt cgccgtgatg atggcgggtg ctctacctta ttgcaatgcg      60

ctgcatgcag ctccaggtgc caaggcactg aataaaatca aaactttccc cgattttgcc     120

gccccaagcc ctaaagacgg caaccgtctg ctgcgccgtg tcgataatgg ggaatttgag     180

atcgaggaag agagaggatt cagtctgaag gataccctga agaagctcaa tcccattaaa     240

gctgcggtaa aggcgaagga caaggctaaa gaggtcacgg aaaagatcac ggatgccgac     300

tggaagaagc tagttgagca tttgaaaatt aagggtgaca agaggagtta a              351

<210> 47
<211> 116
<212> PRT
<213> Phytophthora infestans

<400> 47
Met Arg Leu Ala Tyr Ile Phe Ala Val Met Met Ala Gly Ala Leu Pro
1               5                   10                  15
Tyr Cys Asn Ala Leu His Ala Ala Pro Gly Ala Lys Ala Leu Asn Lys
            20                  25                  30
Ile Lys Thr Phe Pro Asp Phe Ala Ala Pro Ser Pro Lys Asp Gly Asn
        35                  40                  45
Arg Leu Leu Arg Arg Val Asp Asn Gly Glu Phe Glu Ile Glu Glu Glu
    50                  55                  60
Arg Gly Phe Ser Leu Lys Asp Thr Leu Lys Lys Leu Asn Pro Ile Lys
65                  70                  75                  80

Ala Ala Val Lys Ala Lys Asp Lys Ala Lys Glu Val Thr Glu Lys Ile
                85                  90                  95
Thr Asp Ala Asp Trp Lys Lys Leu Val Glu His Leu Lys Ile Lys Gly
            100                 105                 110
Asp Lys Arg Ser
        115

<210> 48
<211> 2538
<212> DNA
<213> Artificial Sequence

<220>
<223> cDNA

<400> 48
atggctgatg cctttctatc atttgcagtt caaaaattgg gtgatttcct cattcaacaa      60

gtttctctgc gtaaaaatct gagaaaggaa attgagtggc tgagaaatga gctactcttc     120

atacagtctt tcctcagaga tgcagaacta aagcaatatg gagatcaaag agttcaacaa     180

tgggtgtttg agatcaactc tattgctaat gatgttgttg ctatactcga gacttacacc     240

ttcgaggctg gtaaaggtgc tagtcgtctc aaggcttgcg cttgcatata tacgaaggag     300

aagaaattct acaatgttgc cgaggagatc caatcactca agcaacgaat catggatatc     360

tctcgcaaac gagagactta tggtattaca aatatcaata ataattcagg agaaggccca     420

agtaatcagg ttagaacatt gaggagaact acctcatatg tggatgacca ggattacatt     480

tttgttggac ttcaggatgt tgtacaaaaa ttgctagctc aacttctcaa agcagagccc     540

cgtcgaaccg tcctctccat tcatggcatg ggcggattgg gcaagaccac tcttgcgaga     600

aaactttaca acagttctgc tatactcaat agcttcccta cacgcgcttg gatatgtgtc     660

tctcaagagt acaacacaat ggatcttctt aggaatatca taaaatccgt ccaaggtcgc     720

accaaggaaa ctctagattt gttggaaagg atgacagaag gagatctaga aatctatctt     780

cgtgatctat taaaagaacg caaatacctt gtgatggttg atgatgtatg gcagaaagaa     840

gcatgggata gtttgaagag agcattcccg gatagcaaga atggcagcag agtcattatt     900

accacgcgca aacaggatgt cgctgaaaga gcagacgaca taggttttgt tcataaactt     960

cgtttcctaa gtcaagaaga aagttgggat ctctttcgta agaaactact tgatgttcga    1020

tcaatggttc cagaaatgga aaatctagct aaggatatgg tggaaaagtg tagaggctta    1080

cctcttgcaa ttgttgtatt gagcggacta ctttcgcata aaaaggggct aaaccaatgg    1140

caaaaggtga aagatcacct ttggaagaac attaaagaag ataaatctat tgaaatctct    1200

aacatactat ccttaagcta caatgatttg tcaactgcgc tcaagcagtg ttttctctac    1260

tttggtattt ttccagaaga tcaagtggta aaggctgatg acataatacg gttgtggatg    1320

gcggagggtt tcatacccag aggagaagaa agaatggagg atgtggctga cggcttcttg    1380

aatgaactga taagacgaag cttggttcaa gtagctaaaa cattttggga aaaagttact 1440

gactgtaggg ttcatgattt acttcgtgat cttgcgatac aaaaggtatt ggaggtaaac 1500

ttctttgaca tttatgatcc aagaagccac tccatatcct ctttatgtat cagacatggc 1560

attcatagtg aaggagaaag gtacctctca tcacttgatc tttctaactt gaagttgagg 1620

tcaattatgt tcttcgatcc atatatttgt aatgtgttcc aacatataga tgtgtttcga 1680

catctatatg tgttgtactt ggatacgaat tttgggtatg tgtctatggt acctgatgcc 1740

ataggaagtt tgtaccacct caagttgtta agattgagag gtatccatga tattccgtct 1800

tccattggca acctcaagaa tttacaaaca cttgtcgttg taaatggtta cacatttttt 1860

tgcgaactac cctgcaagac agctgaccta ataaatctaa gacatttagt tgttcaatat 1920

acagagcctt taaaatgtat aaacaaactc actagtcttc aagttcttga tggtgttgct 1980

tgtgatcagt ggaaagatgt tgaccctgtt gatttagtca atcttcgaga attaagcatg 2040

gatcgtatca ggagctctta ctccctaaac aacattagca gcttgaaaaa ccttagcact 2100

ctcaaattga tttgtggaga acgtcaatca tttgcatccc ttgaatttgt taattgttgt 2160

gaaaagctcc agaaattgtg gttacaaggg agaatagagg aactgcctca tctgttttca 2220

aactccatca caatgatggt tctgagtttc tcagaactga cagaagatcc gatgcctatt 2280

ttgggaaggt ttccaaacct aaggaatctc aaattagatg gagcttacga aggaaaagaa 2340

ataatgtgca gtgataacag cttcagtcaa ctagagttcc ttcatcttcg tgatctttgg 2400

aagctagaaa gatgggattt aggcacaagt gccatgcctc tgattaaagg tcttggtatc 2460

cataactgtc caaatttaaa ggagattcct gagagaatga aagacgtgga gctgttgaag 2520

cggaattata tgttgtga 2538

<210> 49
<211> 845
<212> PRT
<213> Solanum demissum

<400> 49
Met Ala Asp Ala Phe Leu Ser Phe Ala Val Gln Lys Leu Gly Asp Phe
1               5                   10                  15
Leu Ile Gln Gln Val Ser Leu Arg Lys Asn Leu Arg Lys Glu Ile Glu
            20                  25                  30
Trp Leu Arg Asn Glu Leu Leu Phe Ile Gln Ser Phe Leu Arg Asp Ala
        35                  40                  45
Glu Leu Lys Gln Tyr Gly Asp Gln Arg Val Gln Gln Trp Val Phe Glu
    50                  55                  60
Ile Asn Ser Ile Ala Asn Asp Val Val Ala Ile Leu Glu Thr Tyr Thr
65                  70                  75                  80
Phe Glu Ala Gly Lys Gly Ala Ser Arg Leu Lys Ala Cys Ala Cys Ile
                85                  90                  95
Tyr Thr Lys Glu Lys Lys Phe Tyr Asn Val Ala Glu Glu Ile Gln Ser
            100                 105                 110
Leu Lys Gln Arg Ile Met Asp Ile Ser Arg Lys Arg Glu Thr Tyr Gly

115                 120                 125
Ile Thr Asn Ile Asn Asn Asn Ser Gly Glu Gly Pro Ser Asn Gln Val
    130                 135                 140
Arg Thr Leu Arg Arg Thr Thr Ser Tyr Val Asp Asp Gln Asp Tyr Ile
145                 150                 155                 160
Phe Val Gly Leu Gln Asp Val Val Gln Lys Leu Leu Ala Gln Leu Leu
                165                 170                 175
Lys Ala Glu Pro Arg Arg Thr Val Leu Ser Ile His Gly Met Gly Gly
            180                 185                 190
Leu Gly Lys Thr Thr Leu Ala Arg Lys Leu Tyr Asn Ser Ser Ala Ile
        195                 200                 205
Leu Asn Ser Phe Pro Thr Arg Ala Trp Ile Cys Val Ser Gln Glu Tyr
    210                 215                 220
Asn Thr Met Asp Leu Leu Arg Asn Ile Ile Lys Ser Val Gln Gly Arg
225                 230                 235                 240
Thr Lys Glu Thr Leu Asp Leu Leu Glu Arg Met Thr Glu Gly Asp Leu
                245                 250                 255
Glu Ile Tyr Leu Arg Asp Leu Leu Lys Glu Arg Lys Tyr Leu Val Met
            260                 265                 270
Val Asp Asp Val Trp Gln Lys Glu Ala Trp Asp Ser Leu Lys Arg Ala
        275                 280                 285
Phe Pro Asp Ser Lys Asn Gly Ser Arg Val Ile Ile Thr Thr Arg Lys
    290                 295                 300
Gln Asp Val Ala Glu Arg Ala Asp Asp Ile Gly Phe Val His Lys Leu
305                 310                 315                 320
Arg Phe Leu Ser Gln Glu Glu Ser Trp Asp Leu Phe Arg Lys Lys Leu
                325                 330                 335
Leu Asp Val Arg Ser Met Val Pro Glu Met Glu Asn Leu Ala Lys Asp
            340                 345                 350
Met Val Glu Lys Cys Arg Gly Leu Pro Leu Ala Ile Val Val Leu Ser
        355                 360                 365
Gly Leu Leu Ser His Lys Lys Gly Leu Asn Gln Trp Gln Lys Val Lys
    370                 375                 380
Asp His Leu Trp Lys Asn Ile Lys Glu Asp Lys Ser Ile Glu Ile Ser
385                 390                 395                 400
Asn Ile Leu Ser Leu Ser Tyr Asn Asp Leu Ser Thr Ala Leu Lys Gln
                405                 410                 415
Cys Phe Leu Tyr Phe Gly Ile Phe Pro Glu Asp Gln Val Val Lys Ala
            420                 425                 430
Asp Asp Ile Ile Arg Leu Trp Met Ala Glu Gly Phe Ile Pro Arg Gly
        435                 440                 445
Glu Glu Arg Met Glu Asp Val Ala Asp Gly Phe Leu Asn Glu Leu Ile
    450                 455                 460
Arg Arg Ser Leu Val Gln Val Ala Lys Thr Phe Trp Glu Lys Val Thr
465                 470                 475                 480
Asp Cys Arg Val His Asp Leu Leu Arg Asp Leu Ala Ile Gln Lys Val
                485                 490                 495
Leu Glu Val Asn Phe Phe Asp Ile Tyr Asp Pro Arg Ser His Ser Ile
            500                 505                 510
Ser Ser Leu Cys Ile Arg His Gly Ile His Ser Glu Gly Glu Arg Tyr
        515                 520                 525
Leu Ser Ser Leu Asp Leu Ser Asn Leu Lys Leu Arg Ser Ile Met Phe
    530                 535                 540
Phe Asp Pro Tyr Ile Cys Asn Val Phe Gln His Ile Asp Val Phe Arg
545                 550                 555                 560
His Leu Tyr Val Leu Tyr Leu Asp Thr Asn Phe Gly Tyr Val Ser Met
                565                 570                 575
Val Pro Asp Ala Ile Gly Ser Leu Tyr His Leu Lys Leu Leu Arg Leu
            580                 585                 590
Arg Gly Ile His Asp Ile Pro Ser Ser Ile Gly Asn Leu Lys Asn Leu
        595                 600                 605
Gln Thr Leu Val Val Val Asn Gly Tyr Thr Phe Phe Cys Glu Leu Pro
    610                 615                 620

Cys Lys Thr Ala Asp Leu Ile Asn Leu Arg His Leu Val Val Gln Tyr
625                 630                 635                 640
Thr Glu Pro Leu Lys Cys Ile Asn Lys Leu Thr Ser Leu Gln Val Leu
                645                 650                 655
Asp Gly Val Ala Cys Asp Gln Trp Lys Asp Val Asp Pro Val Asp Leu
            660                 665                 670
Val Asn Leu Arg Glu Leu Ser Met Asp Arg Ile Arg Ser Ser Tyr Ser
        675                 680                 685
Leu Asn Asn Ile Ser Ser Leu Lys Asn Leu Ser Thr Leu Lys Leu Ile
    690                 695                 700
Cys Gly Glu Arg Gln Ser Phe Ala Ser Leu Glu Phe Val Asn Cys Cys
705                 710                 715                 720
Glu Lys Leu Gln Lys Leu Trp Leu Gln Gly Arg Ile Glu Glu Leu Pro
                725                 730                 735
His Leu Phe Ser Asn Ser Ile Thr Met Met Val Leu Ser Phe Ser Glu
            740                 745                 750
Leu Thr Glu Asp Pro Met Pro Ile Leu Gly Arg Phe Pro Asn Leu Arg
        755                 760                 765
Asn Leu Lys Leu Asp Gly Ala Tyr Glu Gly Lys Glu Ile Met Cys Ser
    770                 775                 780
Asp Asn Ser Phe Ser Gln Leu Glu Phe Leu His Leu Arg Asp Leu Trp
785                 790                 795                 800
Lys Leu Glu Arg Trp Asp Leu Gly Thr Ser Ala Met Pro Leu Ile Lys
                805                 810                 815
Gly Leu Gly Ile His Asn Cys Pro Asn Leu Lys Glu Ile Pro Glu Arg
            820                 825                 830
Met Lys Asp Val Glu Leu Leu Lys Arg Asn Tyr Met Leu
        835                 840                 845

<210> 50
<211> 2538
<212> DNA
<213> Artificial Sequence

<220>
<223> cDNA

<400> 50
atggctgatg cctttctatc atttgcagtt caaaaattgg gtgatttcct aatacagaaa      60

gtttccctgc gtaaaagtct cagagacgaa attagatggc tgatcaatga gctactcttc     120

atacggtctt tcctcagaga tgcagaacaa aagcagtgcg gagatcaaag agttcaacaa     180

tgggtgtttg agatcaactc tattgctaat gatgctgttg ctatactcga gacttatagc     240

tttgaggctg gtaaaggtgc tagtcgtctc aaggcttgca cttgcatatg taggaaggag     300

aagaaattct acaatgttgc cgaggagatt caatcactca agcaacgaat catggatatc     360

tctcgcaaac gagagactta tggtattaca aatatcaata ataatgcagg agaagggcca     420

agtaatcagg ttacaaaatt gaggagaact acctcatatg tagatgaaca ggattacatt     480

tttgttggct ttcaggatgt tgtacaaaca tttctagctc aacttctgaa agcagagcct     540

cgtcgaagcg tcctctccat ttatggaatg gggggtttag gcaagaccac tcttgccaga     600

aaactttaca ccagtcctga tatactcaat agcttccgta cacgcgcttg gatatgtgtc     660

tctcaagagt acaacacaat ggatcttctt aggaatatca taaaatccat ccaaggtcgc     720

accaaggaaa ctctagattt gttggaaagg atgacagaag gagatcttga aatttatctt 780

cgtgatttat tgaaagaacg caaatacctt gtggtggttg atgatgtatg gcagagagaa 840

gcatgggaga gtttgaaaag atcattcccg gatggcaaga atggcagcag agtcattatt 900

accacgcgca aagaggatgt cgctgaaaga gcagacgaca gaggttttgt tcataaactt 960

cgtttcctaa gccaagaaga aagttgggat ctctttcgta ggaaactact tgatgttcga 1020

gcaatggttc cagaaatgga aagtctagct aaggatatgg tggaaaagtg tagaggctta 1080

cctcttgcaa ttgttgtatt gagcggacta ctttcgcata aaaaggggct aaaccaatgg 1140

caaaaggtga aagatcacct ttggaagaac attaaagaag ataaatctat tgaaatctct 1200

aacatactat ccttaagcta caatgatttg tcaactgcgc tcaagcagtg ttttctctac 1260

tttggtattt ttccagaaga tcaagtggta aaggctgatg acataatacg gttgtggatg 1320

gcggagggtt tcatacccag aggagaagaa agaatggagg atgtggctga cggcttcttg 1380

aatgaactga taagacgaag cttggttcaa gtagctaaaa cattttggga aaaagttact 1440

gactgtaggg ttcatgattt acttcgtgat cttgcgatac aaaaggcatt ggaggtaaac 1500

ttctttgaca tttatgatcc aagaagccac tccatatcct ctttatgtat cagacatggc 1560

attcatagtg aaggagaaag gtacctctca tcacttgatc tttctaactt gaagttgagg 1620

tcaattatgt tcttcgatcc atatatttgt aatgtgttcc aacatataga tgtgtttcga 1680

catctatatg tgttgtactt ggatacgaat tttgggtatg tgtctatggt acctgatgcc 1740

ataggaagtt tgtaccacct caagttgtta agattgagag gtatccatga tattccgtct 1800

tccattggca acctcaagaa tttacaaaca cttgtcgttg taaatggtta cacatttttt 1860

tgcgaactac cctgcaagac agctgaccta ataaatctaa gacatttagt tgttcaatat 1920

acagagcctt taaaatgtat aaacaaactc actagtcttc aagttcttga tggtgttgct 1980

tgtgatcagt ggaaagatgt tgaccctgtt gatttagtca atcttcgaga attaagcatg 2040

gatcgtatca ggagctctta ctccctaaac aacattagca gcttgaaaaa ccttagcact 2100

ctcaaattga tttgtggaga acgtcaatca tttgcatccc ttgaatttgt taattgttgt 2160

gaaaagctcc agaaattgtg gttacaaggg agaatagagg aactgcctca tctgttttca 2220

aactccatca caatgatggt tctgagtttc tcagaactga cagaagatcc gatgcctatt 2280

ttgggaaggt ttccaaacct aaggaatctc aaattagatg gagcttacga aggaaaagaa 2340

ataatgtgca gtgataacag cttcagtcaa ctagagttcc ttcatcttcg tgatctttgg 2400

aagctagaaa gatgggattt aggcacaagt gccatgcctc tgattaaagg tcttggtatc 2460

cataactgtc caaatttaaa ggagattcct gagagaatga aagacgtgga gctgttgaag 2520

cggaattata tgttgtga 2538

<210> 51
<211> 845
<212> PRT
<213> Solanum

<400> 51
Met Ala Asp Ala Phe Leu Ser Phe Ala Val Gln Lys Leu Gly Asp Phe
1               5                   10                  15
Leu Ile Gln Lys Val Ser Leu Arg Lys Ser Leu Arg Asp Glu Ile Arg
            20                  25                  30
Trp Leu Ile Asn Glu Leu Leu Phe Ile Arg Ser Phe Leu Arg Asp Ala
        35                  40                  45
Glu Gln Lys Gln Cys Gly Asp Gln Arg Val Gln Gln Trp Val Phe Glu
    50                  55                  60
Ile Asn Ser Ile Ala Asn Asp Ala Val Ala Ile Leu Glu Thr Tyr Ser
65                  70                  75                  80
Phe Glu Ala Gly Lys Gly Ala Ser Arg Leu Lys Ala Cys Thr Cys Ile
                85                  90                  95
Cys Arg Lys Glu Lys Lys Phe Tyr Asn Val Ala Glu Glu Ile Gln Ser
            100                 105                 110
Leu Lys Gln Arg Ile Met Asp Ile Ser Arg Lys Arg Glu Thr Tyr Gly
        115                 120                 125
Ile Thr Asn Ile Asn Asn Asn Ala Gly Glu Gly Pro Ser Asn Gln Val
    130                 135                 140
Thr Lys Leu Arg Arg Thr Thr Ser Tyr Val Asp Glu Gln Asp Tyr Ile
145                 150                 155                 160
Phe Val Gly Phe Gln Asp Val Val Gln Thr Phe Leu Ala Gln Leu Leu
                165                 170                 175
Lys Ala Glu Pro Arg Arg Ser Val Leu Ser Ile Tyr Gly Met Gly Gly
            180                 185                 190
Leu Gly Lys Thr Thr Leu Ala Arg Lys Leu Tyr Thr Ser Pro Asp Ile
        195                 200                 205
Leu Asn Ser Phe Arg Thr Arg Ala Trp Ile Cys Val Ser Gln Glu Tyr
    210                 215                 220
Asn Thr Met Asp Leu Leu Arg Asn Ile Ile Lys Ser Ile Gln Gly Arg
225                 230                 235                 240
Thr Lys Glu Thr Leu Asp Leu Leu Glu Arg Met Thr Glu Gly Asp Leu
                245                 250                 255
Glu Ile Tyr Leu Arg Asp Leu Leu Lys Glu Arg Lys Tyr Leu Val Val
            260                 265                 270
Val Asp Asp Val Trp Gln Arg Glu Ala Trp Glu Ser Leu Lys Arg Ser
        275                 280                 285
Phe Pro Asp Gly Lys Asn Gly Ser Arg Val Ile Ile Thr Thr Arg Lys
    290                 295                 300
Glu Asp Val Ala Glu Arg Ala Asp Asp Arg Gly Phe Val His Lys Leu
305                 310                 315                 320
Arg Phe Leu Ser Gln Glu Glu Ser Trp Asp Leu Phe Arg Arg Lys Leu
                325                 330                 335
Leu Asp Val Arg Ala Met Val Pro Glu Met Glu Ser Leu Ala Lys Asp
            340                 345                 350
Met Val Glu Lys Cys Arg Gly Leu Pro Leu Ala Ile Val Val Leu Ser
        355                 360                 365
Gly Leu Leu Ser His Lys Lys Gly Leu Asn Gln Trp Gln Lys Val Lys
    370                 375                 380
Asp His Leu Trp Lys Asn Ile Lys Glu Asp Lys Ser Ile Glu Ile Ser
385                 390                 395                 400
Asn Ile Leu Ser Leu Ser Tyr Asn Asp Leu Ser Thr Ala Leu Lys Gln
                405                 410                 415
Cys Phe Leu Tyr Phe Gly Ile Phe Pro Glu Asp Gln Val Val Lys Ala
            420                 425                 430
Asp Asp Ile Ile Arg Leu Trp Met Ala Glu Gly Phe Ile Pro Arg Gly
        435                 440                 445

Glu Glu Arg Met Glu Asp Val Ala Asp Gly Phe Leu Asn Glu Leu Ile
    450                 455                 460
Arg Arg Ser Leu Val Gln Val Ala Lys Thr Phe Trp Glu Lys Val Thr
465                 470                 475                 480
Asp Cys Arg Val His Asp Leu Leu Arg Asp Leu Ala Ile Gln Lys Ala
                485                 490                 495
Leu Glu Val Asn Phe Phe Asp Ile Tyr Asp Pro Arg Ser His Ser Ile
            500                 505                 510
Ser Ser Leu Cys Ile Arg His Gly Ile His Ser Glu Gly Glu Arg Tyr
        515                 520                 525
Leu Ser Ser Leu Asp Leu Ser Asn Leu Lys Leu Arg Ser Ile Met Phe
    530                 535                 540
Phe Asp Pro Tyr Ile Cys Asn Val Phe Gln His Ile Asp Val Phe Arg
545                 550                 555                 560
His Leu Tyr Val Leu Tyr Leu Asp Thr Asn Phe Gly Tyr Val Ser Met
                565                 570                 575
Val Pro Asp Ala Ile Gly Ser Leu Tyr His Leu Lys Leu Leu Arg Leu
            580                 585                 590
Arg Gly Ile His Asp Ile Pro Ser Ser Ile Gly Asn Leu Lys Asn Leu
        595                 600                 605
Gln Thr Leu Val Val Val Asn Gly Tyr Thr Phe Phe Cys Glu Leu Pro
    610                 615                 620
Cys Lys Thr Ala Asp Leu Ile Asn Leu Arg His Leu Val Val Gln Tyr
625                 630                 635                 640
Thr Glu Pro Leu Lys Cys Ile Asn Lys Leu Thr Ser Leu Gln Val Leu
                645                 650                 655
Asp Gly Val Ala Cys Asp Gln Trp Lys Asp Val Asp Pro Val Asp Leu
            660                 665                 670
Val Asn Leu Arg Glu Leu Ser Met Asp Arg Ile Arg Ser Ser Tyr Ser
        675                 680                 685
Leu Asn Asn Ile Ser Ser Leu Lys Asn Leu Ser Thr Leu Lys Leu Ile
    690                 695                 700
Cys Gly Glu Arg Gln Ser Phe Ala Ser Leu Glu Phe Val Asn Cys Cys
705                 710                 715                 720
Glu Lys Leu Gln Lys Leu Trp Leu Gln Gly Arg Ile Glu Glu Leu Pro
                725                 730                 735
His Leu Phe Ser Asn Ser Ile Thr Met Met Val Leu Ser Phe Ser Glu
            740                 745                 750
Leu Thr Glu Asp Pro Met Pro Ile Leu Gly Arg Phe Pro Asn Leu Arg
        755                 760                 765
Asn Leu Lys Leu Asp Gly Ala Tyr Glu Gly Lys Glu Ile Met Cys Ser
    770                 775                 780
Asp Asn Ser Phe Ser Gln Leu Glu Phe Leu His Leu Arg Asp Leu Trp
785                 790                 795                 800
Lys Leu Glu Arg Trp Asp Leu Gly Thr Ser Ala Met Pro Leu Ile Lys
                805                 810                 815
Gly Leu Gly Ile His Asn Cys Pro Asn Leu Lys Glu Ile Pro Glu Arg
            820                 825                 830
Met Lys Asp Val Glu Leu Leu Lys Arg Asn Tyr Met Leu
        835                 840                 845

<210> 52
<211> 2544
<212> DNA
<213> Artificial Sequence

<220>
<223> cDNA

<400> 52
atggctgatg cctttctatc atttgcagtt caaaaattgg gtgatttcct aatacagaaa      60

gtttccctgc gtaaaagtct cagagatgaa attagatggc tgatcaatga gctactcttc 120

atacggtctt tcctcagaga tgcagaacaa aagcagtgcg gagatcaaag agttcaacaa 180

tgggtgtttg agatcaactc tattgctaat gatgctgttg ctatactcga gacttatagc 240

tttgaggctg gtaaaggtgc tagtcgtctc aaggcttgca cttgcatatg taggaaggag 300

aagaaattct acaatgttgc cgaggagatt caatcactca agcaacgaat catggatatc 360

tctcgcaaac gagagactta tggtattaca aatatcaata ataatgcagg agaaggccca 420

agtaatcagg ttacaaaatt gaggagaact acctcatatg tagatgaaca ggattacatt 480

tttgttggct ttcaggatgt tgtacaaaca tttctagctc aacttctgaa agcagagcct 540

cgtcgaaccg tcctctccat ttatggaatg gggggtttag gcaagaccac tcttgccaga 600

aaactttaca ccagtcctga tatactcaat agcttccgta cacgcgcttg gatatgtgtc 660

tctcaagagt acaacacaat ggatcttctt aggaatatca taaaatccat ccaaggtcgc 720

accaaggaaa ctctagattt gttggaaagg atgacagaag gagatcttga aatttatctt 780

cgtgatttat tgaaagaacg caaatacctt gtggtggttg atgatgtatg gcagagagaa 840

gcatgggaga gtttgaaaag atcattcccg gatggcaaga atggcagcag agtcattatt 900

accacgcgca aagaggatgt cgctgaaaga gcagacgaca gaggttttgt tcataaactt 960

cgtttcctaa gccaagaaga aagttgggat ctctttcgta ggaaactact tgatgttcga 1020

gcaatggttc cagaaatgga aagtctagct aaggatatgg tggaaaagtg tagaggctta 1080

cctcttgcaa ttgttgtatt gagcggacta ctttcgcata aaaaggggct aaaccaatgg 1140

caaaaggtga aagatcacct ttggaagaac attaaagaag ataaatctat tgaaatctct 1200

aacatactat ccttaagcta caatgatttg tcaactgcgc tcaagcagtg ttttctctac 1260

tttggtattt ttccagaaga tcaagtggta aaggctgatg acataatacg gttgtggatg 1320

gcggagggtt tcatacccag aggagaagaa agaatggagg atgtggctga cggcttcttg 1380

aatgaactga taagacgaag cttggttcaa gtagctaaaa catttgggaa aaaagttact 1440

gactgtaggg ttcatgattt acttcgtgat cttgcgatac aaaaggcatt ggaggtaaac 1500

ttctttgaca tttatgatcc aagaagccac tccatatcct ctttatgtat cagacatggc 1560

attcatagtg aaggagaaag gtacctctca tcacttgatc tttctaactt gaagttgagg 1620

tcaattatgt tcttcgatcc agattttcgt aagatgagtc atataaacct caggagtgag 1680

ttccaacatc tatatgtgtt gtacttggat acgaattttg ggtatgtgtc tatggtacct 1740

gatgccatag gaagtttgta ccacctcaag ttgttaagat tgagaggtat ccatgatatt 1800

ccgtcttcca ttggcaacct caagaattta caaacacttg tcgttgtaaa tggttacaca 1860

tttttttgcg aactaccctg caagacagct gacctaataa atctaagaca tttagttgtt 1920

caatatacag agcctttaaa atgtataaac aaactcacta gtcttcaagt tcttgatggt 1980

gttgcttgtg atcagtggaa agatgttgac cctgttgatt tagtcaatct tcgagaatta 2040

agcatggatc gtatcaggag ctcttactcc ctaaacaaca ttagcagctt gaaaaacctt 2100

agcactctca aattgatttg tggagaacgt caatcatttg catcccttga atttgttaat 2160

tgttgtgaaa agctccagaa attgtggtta caagggagaa tagaggaact gcctcatctg 2220

ttttcaaact ccatcacaat gatggttctg agtttctcag aactgacaga agatccgatg 2280

cctattttgg gaaggtttcc aaacctaagg aatctcaaat tagatggagc ttacgaagga 2340

aaagaaataa tgtgcagtga taacagcttc agtcaactag agttccttca tcttcgtgat 2400

ctttggaagc tagaaagatg ggatttaggc acaagtgcga tgcctctgat taaaggtctt 2460

ggtatccata actgtccaaa tttaaaggag attcctgaga gaatgaaaga cgtggagctg 2520

ttgaagcgga attatatgtt gtga 2544

<210> 53
<211> 847
<212> PRT
<213> Solanum edinense

<400> 53
Met Ala Asp Ala Phe Leu Ser Phe Ala Val Gln Lys Leu Gly Asp Phe
1               5                   10                  15
Leu Ile Gln Lys Val Ser Leu Arg Lys Ser Leu Arg Asp Glu Ile Arg
            20                  25                  30
Trp Leu Ile Asn Glu Leu Leu Phe Ile Arg Ser Phe Leu Arg Asp Ala
        35                  40                  45
Glu Gln Lys Gln Cys Gly Asp Gln Arg Val Gln Gln Trp Val Phe Glu
    50                  55                  60
Ile Asn Ser Ile Ala Asn Asp Ala Val Ala Ile Leu Glu Thr Tyr Ser
65                  70                  75                  80
Phe Glu Ala Gly Lys Gly Ala Ser Arg Leu Lys Ala Cys Thr Cys Ile
                85                  90                  95
Cys Arg Lys Glu Lys Lys Phe Tyr Asn Val Ala Glu Glu Ile Gln Ser
            100                 105                 110
Leu Lys Gln Arg Ile Met Asp Ile Ser Arg Lys Arg Glu Thr Tyr Gly
        115                 120                 125
Ile Thr Asn Ile Asn Asn Asn Ala Gly Glu Gly Pro Ser Asn Gln Val
    130                 135                 140
Thr Lys Leu Arg Arg Thr Thr Ser Tyr Val Asp Glu Gln Asp Tyr Ile
145                 150                 155                 160
Phe Val Gly Phe Gln Asp Val Val Gln Thr Phe Leu Ala Gln Leu Leu
                165                 170                 175
Lys Ala Glu Pro Arg Arg Thr Val Leu Ser Ile Tyr Gly Met Gly Gly
            180                 185                 190
Leu Gly Lys Thr Thr Leu Ala Arg Lys Leu Tyr Thr Ser Pro Asp Ile
        195                 200                 205
Leu Asn Ser Phe Arg Thr Arg Ala Trp Ile Cys Val Ser Gln Glu Tyr
    210                 215                 220
Asn Thr Met Asp Leu Leu Arg Asn Ile Ile Lys Ser Ile Gln Gly Arg
225                 230                 235                 240
Thr Lys Glu Thr Leu Asp Leu Leu Glu Arg Met Thr Glu Gly Asp Leu
                245                 250                 255
Glu Ile Tyr Leu Arg Asp Leu Leu Lys Glu Arg Lys Tyr Leu Val Val
            260                 265                 270

Val Asp Asp Val Trp Gln Arg Glu Ala Trp Glu Ser Leu Lys Arg Ser
            275                 280                 285
Phe Pro Asp Gly Lys Asn Gly Ser Arg Val Ile Ile Thr Thr Arg Lys
    290                 295                 300
Glu Asp Val Ala Glu Arg Ala Asp Asp Arg Gly Phe Val His Lys Leu
305                 310                 315                 320
Arg Phe Leu Ser Gln Glu Glu Ser Trp Asp Leu Phe Arg Arg Lys Leu
                325                 330                 335
Leu Asp Val Arg Ala Met Val Pro Glu Met Glu Ser Leu Ala Lys Asp
            340                 345                 350
Met Val Glu Lys Cys Arg Gly Leu Pro Leu Ala Ile Val Val Leu Ser
        355                 360                 365
Gly Leu Leu Ser His Lys Lys Gly Leu Asn Gln Trp Gln Lys Val Lys
    370                 375                 380
Asp His Leu Trp Lys Asn Ile Lys Glu Asp Lys Ser Ile Glu Ile Ser
385                 390                 395                 400
Asn Ile Leu Ser Leu Ser Tyr Asn Asp Leu Ser Thr Ala Leu Lys Gln
                405                 410                 415
Cys Phe Leu Tyr Phe Gly Ile Phe Pro Glu Asp Gln Val Val Lys Ala
            420                 425                 430
Asp Asp Ile Ile Arg Leu Trp Met Ala Glu Gly Phe Ile Pro Arg Gly
        435                 440                 445
Glu Glu Arg Met Glu Asp Val Ala Asp Gly Phe Leu Asn Glu Leu Ile
    450                 455                 460
Arg Arg Ser Leu Val Gln Val Ala Lys Thr Phe Trp Glu Lys Val Thr
465                 470                 475                 480
Asp Cys Arg Val His Asp Leu Leu Arg Asp Leu Ala Ile Gln Lys Ala
                485                 490                 495
Leu Glu Val Asn Phe Phe Asp Ile Tyr Asp Pro Arg Ser His Ser Ile
            500                 505                 510
Ser Ser Leu Cys Ile Arg His Gly Ile His Ser Glu Gly Glu Arg Tyr
        515                 520                 525
Leu Ser Ser Leu Asp Leu Ser Asn Leu Lys Leu Arg Ser Ile Met Phe
    530                 535                 540
Phe Asp Pro Asp Phe Arg Lys Met Ser His Ile Asn Leu Arg Ser Glu
545                 550                 555                 560
Phe Gln His Leu Tyr Val Leu Tyr Leu Asp Thr Asn Phe Gly Tyr Val
                565                 570                 575
Ser Met Val Pro Asp Ala Ile Gly Ser Leu Tyr His Leu Lys Leu Leu
            580                 585                 590
Arg Leu Arg Gly Ile His Asp Ile Pro Ser Ser Ile Gly Asn Leu Lys
        595                 600                 605
Asn Leu Gln Thr Leu Val Val Val Asn Gly Tyr Thr Phe Phe Cys Glu
    610                 615                 620
Leu Pro Cys Lys Thr Ala Asp Leu Ile Asn Leu Arg His Leu Val Val
625                 630                 635                 640
Gln Tyr Thr Glu Pro Leu Lys Cys Ile Asn Lys Leu Thr Ser Leu Gln
                645                 650                 655
Val Leu Asp Gly Val Ala Cys Asp Gln Trp Lys Asp Val Asp Pro Val
            660                 665                 670
Asp Leu Val Asn Leu Arg Glu Leu Ser Met Asp Arg Ile Arg Ser Ser
        675                 680                 685
Tyr Ser Leu Asn Asn Ile Ser Ser Leu Lys Asn Leu Ser Thr Leu Lys
    690                 695                 700
Leu Ile Cys Gly Glu Arg Gln Ser Phe Ala Ser Leu Glu Phe Val Asn
705                 710                 715                 720
Cys Cys Glu Lys Leu Gln Lys Leu Trp Leu Gln Gly Arg Ile Glu Glu
                725                 730                 735
Leu Pro His Leu Phe Ser Asn Ser Ile Thr Met Met Val Leu Ser Phe
            740                 745                 750
Ser Glu Leu Thr Glu Asp Pro Met Pro Ile Leu Gly Arg Phe Pro Asn
        755                 760                 765
Leu Arg Asn Leu Lys Leu Asp Gly Ala Tyr Glu Gly Lys Glu Ile Met

770                 775                 780
Cys Ser Asp Asn Ser Phe Ser Gln Leu Glu Phe Leu His Leu Arg Asp
785                 790                 795                 800
Leu Trp Lys Leu Glu Arg Trp Asp Leu Gly Thr Ser Ala Met Pro Leu
                805                 810                 815
Ile Lys Gly Leu Gly Ile His Asn Cys Pro Asn Leu Lys Glu Ile Pro
            820                 825                 830
Glu Arg Met Lys Asp Val Glu Leu Leu Lys Arg Asn Tyr Met Leu
        835                 840                 845

<210> 54
<211> 3882
<212> DNA
<213> Artificial Sequence


<220>
<223> cDNA

<400> 54
atgaatttca acaatgaatt gtctgatctg aaaaatcgct tcctatttag gacgctgaga      60

gcccagaaat gctcggatgt tgcaagagat cgaatagatt tctttatatg ggagttaaaa     120

ttccttaatt gttttctcca tttgcagagc ttcgcttttg caagtgaatg tggtatgcta     180

gatatctcac agaaaatgat agaaatttgc aagaggttta atacaccacc tccacataat     240

tcatttgcat actggaagga ggtaatttgc aagaggctgt gcgctattag catccagccg     300

gatgctagtt cagatgatgg atttgcatgc tggaagaaag taatttggaa gactaagcaa     360

gaattcagag ctaaatactc ctttccaaaa acactacttg cagacaacaa ggtatatgat     420

gatgatgata ctaatcccaa atttgtgatg gaattcatcg atgctgttgt ggggaatctc     480

aatgttctag tcaagatcaa tgatccatct tcattgcttt ttgttccagg acccaaggaa     540

caaatagaac aagtgttaaa ggagttgaag ttattgagat tttttgtctg ctttgtttca     600

aacaaatgta tagagcctca ataccaacat actacttttt atactcacgc tttaattgag     660

gctagccaca tcgcaatggt tgtgtggttg aatttgccaa tctatggaaa cagaaatcaa     720

gacttggctt caagtgaagt tagttgtttg ctttctgatt tcatggaaat gaagattaag     780

tccattcagc cagacatcag ccgcaacaat atttatattg atgtcttgag ggcgttgaag     840

tcaaccatac cacaagctca agataagcat gctgctgaga gtggcattgt ggagactcca     900

acacacaatc tgatggttgg tttgagtgat caaatggcca accttcagga gatgctctgc     960

cttctaagag acaatctcat tcatctgcca atactagatc tggaatttca tcttcaagat    1020

atggattctg ttattgttga tgccggactt cttatttact cattatatga tatcaagggg    1080

cagaaggaag acacaacatt ggaggatatc aaccaggcac ttggttttga tcttcccaga    1140

aacattgagc ctatcaaggc aatgatcaac cttgtcatgc aaaaggcatt tcaatgtaac    1200

ttgccaagga ttcatggact aggttatgtc gattttctat tgaaaaacct gaaggatttc    1260

caaggccgtt attcagattc actcgatttc ctcaagaatc aacttcaagt tattcaaact    1320

gaatttgaga gcttgcaacc tttcttgaag gttgtcgtag aagagccaca caataagctc 1380

aagacactga atgaagattg tgctacacag ataattagga aagcatatga ggtggaatat 1440

gtagttgatg cttgtataaa caaagaggtt cctcagtggt gcatcgagcg ttggctcctg 1500

gatatcatag aggagattac ttgtatcaaa gcaaagattc aggaaaagaa cacggttgag 1560

gatacaatga agactgtcat tgctcgtaca tcatcaaaac tggcaaggac tccaaggatg 1620

aatgaagaga ttgttgggtt tgaggatgtc atagaaaatt taagaaaaaa actactgaat 1680

ggaaccaaag ggcaagatgt catttcaatt cacggcatgc caggtttagg taagacgact 1740

ttagccaaca gtctctattc tgacaggtca gttttttctc aatttgatat ttgtgcacaa 1800

tgttgtgtgt ctcaagtata ttcttataag gacttaatat tggccttgct acgtgatgct 1860

attggtgagg gttctgtgcg tagagaactt catgccaatg aattagctga tatgcttcgc 1920

aaaactctat tgccccgaag gtaccttatc cttgttgatg acgtgtggga aaatagtgtt 1980

tgggatgatt taagaggttg ttttccagat gtcaataaca gaagcagaat cattctaaca 2040

acaagacatc atgaagttgc caaatatgct agtgttcata gtgatcccct tcatcttcgt 2100

atgtttgacg aagttgaaag ttggaagttg cttgaaaaga aagtgtttgg tgaagaaagc 2160

tgttcccctc tcctaaaaaa tgttgggcta agaatagcaa aaatgtgtgg acaactacct 2220

ctttcaattg ttctggtggc tggtattctg tcagagatgg aaaaggaagt agaatgttgg 2280

gaacaagtgg ccaacaattt gggttcctac attcacaatg actcaagagc cattgtagac 2340

aaaagttatc atgttttacc ttgtcatctt aagtcttgct tcctttattt tggagcattt 2400

ttagaagata gagtgattga catttcaagg ttaataaggc tatggatatc agaagcattt 2460

ataaaaagta gtgaaggcag gaggttggag gatatagcag aaggttactt ggagaatctt 2520

attggaagaa atctagtaat ggttactcag aggtccattt cagatggtaa ggcgaaagaa 2580

tgtcgccttc atgatgtatt actcgacttc tgcaaggaaa gagcagctga ggagaatttt 2640

ctactatgga taaataggga tcagattacc aaaccttctt cctgtgttta ctctcacaag 2700

cagcatgctc acttggcctt cactgaaatg cataatcttg tagaatggag tgcgtcttgc 2760

tcatttgttg gctcggtagt actttccaat aaatatgact catacttttc cactcgtgac 2820

atatcctcac tacatgattt ttcaatttca cgcattttac caaatttcaa gtttctaaaa 2880

gtgttagatt tggaacaccg ggtttttatt gattttattc caactgagct tgtttacttg 2940

aagtattttt ctgcacacat tgaacagaat tcaattcctt caagcatatc caatctttgg 3000

aaccttgaaa ctcttatatt aaaaagtcca atatatgcgt tacgttgcac gctactacta 3060

cctagtacag tttgggatat ggttaaattg agacatctgt atattcctga cttcagcaca 3120

aggattgaag cagcattact tgagaactct gcaaaacttt ataatttgga aaccctttcc 3180

actctatatt tctctcgtgt tgaggatgca gaattgatgc tgagaaaaac acctaatctt 3240

cgaaaactga tatgtgaagt tgaatgttta gaataccccc ctcagtacca tgtgttgaat 3300

tttccaatac ggcttgaaat actaaagctt tatcgatcaa aatttaaaac catccccttt 3360

tgcatctctg caccaaatct caaatacttg aaactctgtg gcttttccct ggattctcag 3420

tacttatcag aaactgctga tcatctcaag caccttgagg tactcatact gtacaaggtt 3480

gaatttggtg atcataggga atggaaagtg agcaatggca agttccctca actcaaaatc 3540

ttgaaactag aatatttgtc cttggtgaaa tggattgtag ctgatgatgc ctttcctaac 3600

cttgaacaat tggttttgcg tggatgtcaa gatcttatgg agatcccttc ttgtttcatg 3660

gacatccttt ctctcaagta catcggggta gaatactgca atgagtcggt tgtcaagtca 3720

gccttgaata tacaagaaac acaagtcgaa gattatcaaa atactaattt caagctcgtt 3780

ctcatcgagt tttctttgca gaaaaaggcg tggaaattaa atttaactga tgcggaagat 3840

atgcacaatg cagtaaaaaa tattcttgca gaaataagat ag 3882

<210> 55
<211> 1293
<212> PRT
<213> Solanum tuberosum

<400> 55
   Met Asn Phe Asn Asn Glu Leu Ser Asp Leu Lys Asn Arg Phe Leu Phe
   1 5 10 15
   Arg Thr Leu Arg Ala Gln Lys Cys Ser Asp Val Ala Arg Asp Arg Ile
   20 25 30
   Asp Phe Phe Ile Trp Glu Leu Lys Phe Leu Asn Cys Phe Leu His Leu
   35 40 45
   Gln Ser Phe Ala Phe Ala Ser Glu Cys Gly Met Leu Asp Ile Ser Gln
   50 55 60
   Lys Met Ile Glu Ile Cys Lys Arg Phe Asn Thr Pro Pro Pro His Asn
   65 70 75 80
   Ser Phe Ala Tyr Trp Lys Glu Val Ile Cys Lys Arg Leu Cys Ala Ile
   85 90 95
   Ser Ile Gln Pro Asp Ala Ser Ser Asp Asp Gly Phe Ala Cys Trp Lys
   100 105 110
   Lys Val Ile Trp Lys Thr Lys Gln Glu Phe Arg Ala Lys Tyr Ser Phe
   115 120 125
   Pro Lys Thr Leu Leu Ala Asp Asn Lys Val Tyr Asp Asp Asp Asp Thr
   130 135 140
   Asn Pro Lys Phe Val Met Glu Phe Ile Asp Ala Val Val Gly Asn Leu
   145 150 155 160
   Asn Val Leu Val Lys Ile Asn Asp Pro Ser Ser Leu Leu Phe Val Pro
   165 170 175
   Gly Pro Lys Glu Gln Ile Glu Gln Val Leu Lys Glu Leu Lys Leu Leu
   180 185 190
   Arg Phe Phe Val Cys Phe Val Ser Asn Lys Cys Ile Glu Pro Gln Tyr
   195 200 205
   Gln His Thr Thr Phe Tyr Thr His Ala Leu Ile Glu Ala Ser His Ile
   210 215 220
   Ala Met Val Val Trp Leu Asn Leu Pro Ile Tyr Gly Asn Arg Asn Gln
   225 230 235 240
   Asp Leu Ala Ser Ser Glu Val Ser Cys Leu Leu Ser Asp Phe Met Glu
   245 250 255
   Met Lys Ile Lys Ser Ile Gln Pro Asp Ile Ser Arg Asn Asn Ile Tyr
   260 265 270
   Ile Asp Val Leu Arg Ala Leu Lys Ser Thr Ile Pro Gln Ala Gln Asp
   275 280 285
   Lys His Ala Ala Glu Ser Gly Ile Val Glu Thr Pro Thr His Asn Leu
   290 295 300
   Met Val Gly Leu Ser Asp Gln Met Ala Asn Leu Gln Glu Met Leu Cys
   305 310 315 320
   Leu Leu Arg Asp Asn Leu Ile His Leu Pro Ile Leu Asp Leu Glu Phe
   325 330 335
   His Leu Gln Asp Met Asp Ser Val Ile Val Asp Ala Gly Leu Leu Ile
   340 345 350
   Tyr Ser Leu Tyr Asp Ile Lys Gly Gln Lys Glu Asp Thr Thr Leu Glu
   355 360 365
   Asp Ile Asn Gln Ala Leu Gly Phe Asp Leu Pro Arg Asn Ile Glu Pro
   370 375 380
   Ile Lys Ala Met Ile Asn Leu Val Met Gln Lys Ala Phe Gln Cys Asn
   385 390 395 400
   Leu Pro Arg Ile His Gly Leu Gly Tyr Val Asp Phe Leu Leu Lys Asn
   405 410 415
   Leu Lys Asp Phe Gln Gly Arg Tyr Ser Asp Ser Leu Asp Phe Leu Lys
   420 425 430
   Asn Gln Leu Gln Val Ile Gln Thr Glu Phe Glu Ser Leu Gln Pro Phe
   435 440 445
   Leu Lys Val Val Val Glu Glu Pro His Asn Lys Leu Lys Thr Leu Asn
   450 455 460
   Glu Asp Cys Ala Thr Gln Ile Ile Arg Lys Ala Tyr Glu Val Glu Tyr
   465 470 475 480
   Val Val Asp Ala Cys Ile Asn Lys Glu Val Pro Gln Trp Cys Ile Glu
   485 490 495
   Arg Trp Leu Leu Asp Ile Ile Glu Glu Ile Thr Cys Ile Lys Ala Lys
   500 505 510
   Ile Gln Glu Lys Asn Thr Val Glu Asp Thr Met Lys Thr Val Ile Ala
   515 520 525
   Arg Thr Ser Ser Lys Leu Ala Arg Thr Pro Arg Met Asn Glu Glu Ile
   530 535 540
   Val Gly Phe Glu Asp Val Ile Glu Asn Leu Arg Lys Lys Leu Leu Asn
   545 550 555 560
   Gly Thr Lys Gly Gln Asp Val Ile Ser Ile His Gly Met Pro Gly Leu
   565 570 575
   Gly Lys Thr Thr Leu Ala Asn Ser Leu Tyr Ser Asp Arg Ser Val Phe
   580 585 590
   Ser Gln Phe Asp Ile Cys Ala Gln Cys Cys Val Ser Gln Val Tyr Ser
   595 600 605
   Tyr Lys Asp Leu Ile Leu Ala Leu Leu Arg Asp Ala Ile Gly Glu Gly
   610 615 620
   Ser Val Arg Arg Glu Leu His Ala Asn Glu Leu Ala Asp Met Leu Arg
   625 630 635 640
   Lys Thr Leu Leu Pro Arg Arg Tyr Leu Ile Leu Val Asp Asp Val Trp
   645 650 655
   Glu Asn Ser Val Trp Asp Asp Leu Arg Gly Cys Phe Pro Asp Val Asn
   660 665 670
   Asn Arg Ser Arg Ile Ile Leu Thr Thr Arg His His Glu Val Ala Lys
   675 680 685
   Tyr Ala Ser Val His Ser Asp Pro Leu His Leu Arg Met Phe Asp Glu
   690 695 700
   Val Glu Ser Trp Lys Leu Leu Glu Lys Lys Val Phe Gly Glu Glu Ser
   705 710 715 720
   Cys Ser Pro Leu Leu Lys Asn Val Gly Leu Arg Ile Ala Lys Met Cys
   725 730 735
   Gly Gln Leu Pro Leu Ser Ile Val Leu Val Ala Gly Ile Leu Ser Glu
   740 745 750
   Met Glu Lys Glu Val Glu Cys Trp Glu Gln Val Ala Asn Asn Leu Gly
           755                 760                 765
   Ser Tyr Ile His Asn Asp Ser Arg Ala Ile Val Asp Lys Ser Tyr His
       770                 775                 780
   Val Leu Pro Cys His Leu Lys Ser Cys Phe Leu Tyr Phe Gly Ala Phe
   785                 790                 795                 800
   Leu Glu Asp Arg Val Ile Asp Ile Ser Arg Leu Ile Arg Leu Trp Ile
                   805                 810                 815
   Ser Glu Ala Phe Ile Lys Ser Ser Glu Gly Arg Arg Leu Glu Asp Ile
               820                 825                 830
   Ala Glu Gly Tyr Leu Glu Asn Leu Ile Gly Arg Asn Leu Val Met Val
           835                 840                 845
   Thr Gln Arg Ser Ile Ser Asp Gly Lys Ala Lys Glu Cys Arg Leu His
       850                 855                 860
   Asp Val Leu Leu Asp Phe Cys Lys Glu Arg Ala Ala Glu Glu Asn Phe
   865                 870                 875                 880
   Leu Leu Trp Ile Asn Arg Asp Gln Ile Thr Lys Pro Ser Ser Cys Val
                   885                 890                 895
   Tyr Ser His Lys Gln His Ala His Leu Ala Phe Thr Glu Met His Asn
               900                 905                 910
   Leu Val Glu Trp Ser Ala Ser Cys Ser Phe Val Gly Ser Val Val Leu
           915                 920                 925
   Ser Asn Lys Tyr Asp Ser Tyr Phe Ser Thr Arg Asp Ile Ser Ser Leu
       930                 935                 940
   His Asp Phe Ser Ile Ser Arg Ile Leu Pro Asn Phe Lys Phe Leu Lys
   945                 950                 955                 960
   Val Leu Asp Leu Glu His Arg Val Phe Ile Asp Phe Ile Pro Thr Glu
                   965                 970                 975
   Leu Val Tyr Leu Lys Tyr Phe Ser Ala His Ile Glu Gln Asn Ser Ile
               980                 985                 990
   Pro Ser Ser Ile Ser Asn Leu Trp Asn Leu Glu Thr Leu Ile Leu Lys
           995                 1000                1005
   Ser Pro Ile Tyr Ala Leu Arg Cys Thr Leu Leu Leu Pro Ser Thr Val
       1010                1015                1020
   Trp Asp Met Val Lys Leu Arg His Leu Tyr Ile Pro Asp Phe Ser Thr
   1025                1030                1035                1040
   Arg Ile Glu Ala Ala Leu Leu Glu Asn Ser Ala Lys Leu Tyr Asn Leu
                   1045                1050                1055
   Glu Thr Leu Ser Thr Leu Tyr Phe Ser Arg Val Glu Asp Ala Glu Leu
               1060                1065                1070
   Met Leu Arg Lys Thr Pro Asn Leu Arg Lys Leu Ile Cys Glu Val Glu
           1075                1080                1085
   Cys Leu Glu Tyr Pro Pro Gln Tyr His Val Leu Asn Phe Pro Ile Arg
       1090                1095                1100
   Leu Glu Ile Leu Lys Leu Tyr Arg Ser Lys Phe Lys Thr Ile Pro Phe
   1105                1110                1115                1120
   Cys Ile Ser Ala Pro Asn Leu Lys Tyr Leu Lys Leu Cys Gly Phe Ser
                   1125                1130                1135
   Leu Asp Ser Gln Tyr Leu Ser Glu Thr Ala Asp His Leu Lys His Leu
               1140                1145                1150
   Glu Val Leu Ile Leu Tyr Lys Val Glu Phe Gly Asp His Arg Glu Trp
           1155                1160                1165
   Lys Val Ser Asn Gly Lys Phe Pro Gln Leu Lys Ile Leu Lys Leu Glu
       1170                1175                1180
   Tyr Leu Ser Leu Val Lys Trp Ile Val Ala Asp Asp Ala Phe Pro Asn
   1185                1190                1195                1200
   Leu Glu Gln Leu Val Leu Arg Gly Cys Gln Asp Leu Met Glu Ile Pro
                   1205                1210                1215
   Ser Cys Phe Met Asp Ile Leu Ser Leu Lys Tyr Ile Gly Val Glu Tyr
               1220                1225                1230
   Cys Asn Glu Ser Val Val Lys Ser Ala Leu Asn Ile Gln Glu Thr Gln
           1235                1240                1245
   Val Glu Asp Tyr Gln Asn Thr Asn Phe Lys Leu Val Leu Ile Glu Phe
   1250                1255                1260
   Ser Leu Gln Lys Lys Ala Trp Lys Leu Asn Leu Thr Asp Ala Glu Asp
   1265                1270                1275                1280
   Met His Asn Ala Val Lys Asn Ile Leu Ala Glu Ile Arg
                   1285                1290

<210> 56
<211> 4102
<212> DNA
<213> Solanum tuberosum

<400> 56
atgaatttca acaatgaatt gtctgatctg aaaaatcgct tcctatttag gacgctgaga      60

gcccagaaat gctcggatgt tgcaagagat cgaatagatt tctttatatg ggagttaaaa     120

ttccttaatt gttttctcca tttgcagagc ttcgcttttg caagtgaatg tggtatgcta     180

gatatctcac agaaaatgat agaaatttgc aagaggttta atacaccacc tccacataat     240

tcatttgcat actggaagga ggtaatttgc aagaggctgt gcgctattag catccagccg     300

gatgctagtt cagatgatgg atttgcatgc tggaagaaag taatttggaa gactaagcaa     360

gaattcagag ctaaatactc ctttccaaaa acactacttg cagacaacaa ggtatatgat     420

gatgatgata ctaatcccaa atttgtgatg gaattcatcg atgctgttgt ggggaatctc     480

aatgttctag tcaagatcaa tgatccatct tcattgcttt ttgttccagg acccaaggaa     540

caaatagaac aagtgttaaa ggagttgaag ttattgagat tttttgtctg ctttgtttca     600

aacaaatgta tagagcctca ataccaacat actacttttt atactcacgc tttaattgag     660

gctagccaca tcgcaatggt tgtgtggttg aatttgccaa tctatggaaa cagaaatcaa     720

gacttggctt caagtgaagt tagttgtttg ctttctgatt tcatggaaat gaagattaag     780

tccattcagc cagacatcag ccgcaacaat atttatattg atgtcttgag ggcgttgaag     840

tcaaccatac cacaagctca agataagcat gctgctgaga gtggcattgt ggagactcca     900

acacacaatc tgatggttgg tttgagtgat caaatggcca accttcagga gatgctctgc     960

cttctaagag acaatctcat tcatctgcca atactagatc tggaatttca tcttcaagat    1020

atggattctg ttattgttga tgccggactt cttatttact cattatatga tatcaagggg    1080

cagaaggaag acacaacatt ggaggatatc aaccaggcac ttggttttga tcttcccaga    1140

aacattgagc ctatcaaggc aatgatcaac cttgtcatgc aaaaggcatt tcaatgtaac    1200

ttgccaagga ttcatggact aggttatgtc gattttctat tgaaaaacct gaaggatttc    1260

caaggccgtt attcagattc actcgatttc ctcaagaatc aacttcaagt tattcaaact    1320

gaatttgaga gcttgcaacc tttcttgaag gttgtcgtag aagagccaca caataagctc    1380

aagacactga atgaagattg tgctacacag ataattagga aagcatatga ggtggaatat    1440

gtagttgatg cttgtataaa caaagaggtt cctcagtggt gcatcgagcg ttggctcctg    1500

gatatcatag aggagattac ttgtatcaaa gcaaagattc aggaaaagaa cacggttgag 1560
gatacaatga agactgtcat tgctcgtaca tcatcaaaac tggcaaggac tccaaggatg 1620
aatgaagaga ttgttgggtt tgaggatgtc atagaaaatt taagaaaaaa actactgaat 1680
ggaaccaaag ggcaagatgt catttcaatt cacggcatgc caggtttagg taagacgact 1740
ttagccaaca gtctctattc tgacaggtca gttttttctc aatttgatat ttgtgcacaa 1800
tgttgtgtgt ctcaagtata ttcttataag gacttaatat tggccttgct acgtgatgct 1860
attggtgagg gttctgtgcg tagagaactt catgccaatg aattagctga tatgcttcgc 1920
aaaactctat tgccccgaag gtaccttatc cttgttgatg acgtgtggga aaatagtgtt 1980
tgggatgatt taagaggttg ttttccagat gtcaataaca gaagcagaat cattctaaca 2040
acaagacatc atgaagttgc caaatatgct agtgttcata gtgatcccct tcatcttcgt 2100
atgtttgacg aagttgaaag ttggaagttg cttgaaaaga aagtgtttgg tgaagaaagc 2160
tgttcccctc tcctaaaaaa tgttgggcta agaatagcaa aaatgtgtgg acaactacct 2220
ctttcaattg ttctggtggc tggtattctg tcagagatgg aaaaggaagt agaatgttgg 2280
gaacaagtgg ccaacaattt gggttcctac attcacaatg actcaagagc cattgtagac 2340
aaaagttatc atgttttacc ttgtcatctt aagtcttgct tcctttattt tggagcattt 2400
ttagaagata gagtgattga catttcaagg ttaataaggc tatggatatc agaagcattt 2460
ataaaaagta gtgaaggcag gaggttggag gatatagcag aaggttactt ggagaatctt 2520
attggaagaa atctagtaat ggttactcag aggtccattt cagatggtaa ggcgaaagaa 2580
tgtcgccttc atgatgtatt actcgacttc tgcaaggaaa gagcagctga ggagaatttt 2640
ctactatgga taaataggta atatgataag taactgtact ttcaatcaat caagtatttc 2700
aagttatatc tgaaaattaa tgatatgatt ttgctaattg atatattcag ggatcagatt 2760
accaaacctt cttcctgtgt ttactctcac aagcagcatg ctcacttggc cttcactgaa 2820
atgcataatc ttgtagaatg gagtgcgtct tgctcatttg ttggctcggt agtactttcc 2880
aataaatatg actcatactt ttccactcgt gacatatcct cactacatga tttttcaatt 2940
tcacgcattt taccaaattt caagtttcta aaagtgttag atttggaaca ccgggttttt 3000
attgatttta ttccaactga gcttgtttac ttgaagtatt tttctgcaca cattgaacag 3060
aattcaattc cttcaagcat atccaatctt tggaaccttg aaactcttat attaaaaagt 3120
ccaatatatg cgttacgttg cacgctacta ctacctagta cagtttggga tatggttaaa 3180
ttgagacatc tgtatattcc tgacttcagc acaaggattg aagcagcatt acttgagaac 3240
tctgcaaaac tttataattt ggaaaccctt tccactctat atttctctcg tgttgaggat 3300
gcagaattga tgctgagaaa aacacctaat cttcgaaaac tgatatgtga agttgaatgt 3360
ttagaatacc cccctcagta ccatgtgttg aattttccaa tacggcttga aatactaaag 3420

ctttatcgat caaaatttaa aaccatcccc ttttgcatct ctgcaccaaa tctcaaatac 3480

ttgaaactct gtggcttttc cctggattct cagtacttat cagaaactgc tgatcatctc 3540

aagcaccttg aggtactcat actgtacaag gttgaatttg gtgatcatag ggaatggaaa 3600

gtgagcaatg gcaagttccc tcaactcaaa atcttgaaac tagaatattt gtccttggtg 3660

aaatggattg tagctgatga tgcctttcct aaccttgaac aattggtttt gcgtggatgt 3720

caagatctta tggagatccc ttcttgtttc atggacatcc tttctctcaa gtacatcggg 3780

gtagaatact gcaatgagtc ggttgtcaag tcagccttga atatacaaga aacacaagtc 3840

gaagattatc aaaatactaa tttcaagctc gttctcatcg aggtacacta ctgaaaaaag 3900

ctttattctg catgattttg atgaatcaga aatcgcctaa attttacaaa ctgttttctc 3960

agttatcttt acctcgtggc ctcgttttac atttgggttc ttctcttagt tttctttgca 4020

gaaaaaggcg tggaaattaa atttaactga tgcggaagat atgcacaatg cagtaaaaaa 4080

tattcttgca gaaataagat ag 4102

<210> 57
<211> 627
<212> DNA
<213> Artificial Sequence

<220>
<223> cDNA

<400> 57
atgggcttaa tgcaccgcgt attgctgctt gcgacgtttg ccctgttgtg tatgcatgca 60

aaagctgccg gattcgacca cgacaaggtt ccaaggactg ttgaacgagg tggcggtgca 120

agacaactgc gcacggccac gatgagcgac gacgaagcta gagtgtcgaa attgccgtcg 180

tttatcgagt ccttcgttaa aaaccgaaaa atcgagtctt ggattcagaa caaagttact 240

gacgactttg tcctgagcga gctaaaactc gtgagattgc ccggaacgag cctggcggac 300

gacccaaatt tcaagctctt tcaaaagttt aagattggcg gctggctcga ggaaaaggct 360

actacaacga aagcctggga aaaccttggc ttggattcac tcccatttga tcaagtgagc 420

aagatcgacg agttcaagac ttatacgcag tatgtgacgg tgcttaacaa gaaggcaagc 480

aaactcgata ttgatcagtg gcacgggctg ttaagtggag gatcacctga ggaattgatg 540

gccaaagcaa tgatattgag gactttgggc agagatgttc tagagcgcag agtcatgctt 600

ggtggacatg ttgtggtacc attttaa 627

<210> 58
<211> 208
<212> PRT
<213> Phytophthora infestans

<400> 58
   Met Gly Leu Met His Arg Val Leu Leu Leu Ala Thr Phe Ala Leu Leu
   1               5                   10                  15
   Cys Met His Ala Lys Ala Ala Gly Phe Asp His Asp Lys Val Pro Arg
               20                  25                  30
   Thr Val Glu Arg Gly Gly Gly Ala Arg Gln Leu Arg Thr Ala Thr Met
           35                  40                  45
   Ser Asp Asp Glu Ala Arg Val Ser Lys Leu Pro Ser Phe Ile Glu Ser
       50                  55                  60
   Phe Val Lys Asn Arg Lys Ile Glu Ser Trp Ile Gln Asn Lys Val Thr
   65                  70                  75                  80
   Asp Asp Phe Val Leu Ser Glu Leu Lys Leu Val Arg Leu Pro Gly Thr
                   85                  90                  95
   Ser Leu Ala Asp Asp Pro Asn Phe Lys Leu Phe Gln Lys Phe Lys Ile
               100                 105                 110
   Gly Gly Trp Leu Glu Glu Lys Ala Thr Thr Thr Lys Ala Trp Glu Asn
           115                 120                 125
   Leu Gly Leu Asp Ser Leu Pro Phe Asp Gln Val Ser Lys Ile Asp Glu
       130                 135                 140
   Phe Lys Thr Tyr Thr Gln Tyr Val Thr Val Leu Asn Lys Lys Ala Ser
   145                 150                 155                 160
   Lys Leu Asp Ile Asp Gln Trp His Gly Leu Leu Ser Gly Gly Ser Pro
                   165                 170                 175
   Glu Glu Leu Met Ala Lys Ala Met Ile Leu Arg Thr Leu Gly Arg Asp
               180                 185                 190
   Val Leu Glu Arg Arg Val Met Leu Gly Gly His Val Val Val Pro Phe
           195                 200                 205

<210> 59
<211> 3804
<212> DNA
<213> Artificial Sequence

<220>
<223> cDNA

<400> 59
atggaaaaac gaaaagataa tgaagaagca aacaactcat tggagtcatt ttctgctctt      60

cgcaaggatg ctgccaatgt tctggatttc ctagagagat taaagaatga agaagatcaa     120

aaggctgttg atgtggatct gattgaaagc ctgaaattga agctgacatt tatttgtaca     180

tatgtccagc tttcttattc cgatttggag aagtttgaag atataatgac tagaaaaaga     240

caagaggttg agaatctgct tcaaccaatt ttggatgatg atggcaaaga cgtcgggtgt     300

aaatatgtcc ttactagcct cgccggtaat atggatgact gtataagctt gtatcatcgt     360

tctaaatcag atgccaccat gatggatgag caattgggct tcctcctctt gaatctctct     420

catctatcca agcatcgtgc tgaaaagatg tttcctggag tgactcaata tgaggttctt     480

cagaatgtat gtggcaacat aagagatttc catggattga tagtgaattg ttgcattaag     540

catgagatgg ttgagaatgt cttatctctg tttcaactga tggctgagag agtaggacgc     600

ttcctttggg aggatcaggc tgatgaagac tctcaactct ccgagctaga tgaggatgat     660

cagaatgata aagaccctca actcttcaag ctagcacatc tactcttgaa gattgttcca 720

actgaattgg aggttatgca catatgttat aaaactttga aagcttcaac ttcaacagaa 780

attggacgct tcattaagaa gctcctggaa acctctccgg acattctcag agaatatctg 840

attcatctac aagagcatat gataactgtt attaccccta acacttcagg ggctcgaaac 900

attcatgtca tgatggaatt cctattgatt attctttctg atatgccgcc caaggacttt 960

attcatcatg acaaactttt tgatctcttg gctcgtgttg tagcacttac cagggaggta 1020

tcaactcttg tacgcgactt ggaagagaaa ttaaggatta aagagagtac tgacgaaaca 1080

aattgtgcaa ccctaaagtt tctggaaaat attgaactcc ttaaggaaga tctcaaacat 1140

gtttatctga aagtcccgga ttcatctcaa tattgcttcc ccatgagtga tggacctctc 1200

ttcatgcatc tgctacagag acacttagat gatttgctgg attccaatgc ttattcaatt 1260

gctttgataa aggaacaaat tgggctggtg aaagaagact tggaattcat aagatctttt 1320

ttcgcgaata ttgagcaagg attgtataaa gatctctggg aacgtgttct agatgtggca 1380

tatgaggcaa aagatgtcat agattcaatt attgttcgag ataatggtct cttacatctt 1440

attttctcac ttcccattac cagaaagaag atgatgctta tcaaagaaga ggtctctgat 1500

ttacatgaga acatttccaa gaacagaggt ctcatcgttg tgaactctcc caagaaacca 1560

gttgagagca agtcattgac aactgataaa ataattgtag gttttggtga ggagacaaac 1620

ttgatactta gaaagctcac cagtggaccg gcagatctag atgtcatttc gatcattggt 1680

atgccgggtt taggtaaaac tactttggcg tacaaagtat acaatgataa atcagtttct 1740

agccatttcg accttcgtgc atggtgcacg gtcgaccaag tatatgacga gaagaagttg 1800

ttggataaaa ttttcaatca agttagtgac tcaaattcaa aattgagtga gaatattgat 1860

gttgctgata aactacggaa acaattgttt ggaaagaggt atcttattgt cttagatgac 1920

gtgtgggata ctaatacatg ggatgagcta acaagacctt ttcctgatgg tatgaaagga 1980

agtagaatta ttttgacaac tcgagaaaag aaagttgctt tgcatggaaa gctctacact 2040

gatcctctta accttcgatt gctaagatca gaagaaagtt gggagttatt agagaaaagg 2100

gcatttggaa acgagagttg ccctgatgaa ctattggatg ttggtaaaga aatagccgaa 2160

aattgtaaag ggcttccttt ggtggtggat ctgattgctg gaatcattgc tgggagggaa 2220

aagaaaaaga gtgtgtggct tgaagttgta aataatttgc attcctttat tttgaagaat 2280

gaagtggaag tgatgaaagt tatagaaata agttatgacc acttacctga tcacctgaag 2340

ccatgcttgc tgtactttgc aagtgcgccg aaggactggg taacgacaat ccatgagttg 2400

aaacttattt ggggttttga aggatttgtg gaaaagacag atatgaagag tctggaagaa 2460

gtggtgaaaa tttatttgga tgatttaatt tccagtagct tggtaatttg tttcaatgag 2520

ataggtgatt accctacttg ccaacttcat gatcttgtgc atgacttttg tttgataaaa 2580

gcaagaaagg aaaagttgtg tgatcggata agttcaagtg ctccatcaga tttgttgcca 2640

cgtcaaatta gcattgatta tgatgatgat gaagagcact ttgggcttaa ttttgtcctg 2700

ttcggttcaa ataagaaaag gcattccggt aaacacctct attctttgac cataaatgga 2760

gatgagctgg acgaccatct ttctgataca tttcatctaa gacacttgag gcttcttaga 2820

accttgcacc tggaatcctc ttttatcatg gttaaagatt ctttgctgaa tgaaatatgc 2880

atgttgaatc atttgaggta cttaagcatt gggacagaag ttaaatctct gcctttgtct 2940

ttctcaaacc tctggaatct agaaatcttg tttgtggata acaaagaatc aaccttgata 3000

ctattaccga gaatttggga tcttgtaaag ttgcaagtgc tgttcacgac tgcttgttct 3060

ttctttgata tggatgcaga tgaatcaata ctgatagcag aggacacaaa gttagagaac 3120

ttgacagcat taggggaact cgtgctttcc tattggaaag atacagagga tattttcaaa 3180

aggcttccca atcttcaagt gcttcatttc aaactcaagg agtcatggga ttattcaaca 3240

gagcaatatt ggttcccgaa attggatttc ctaactgaac tagaaaaact cactgtagat 3300

tttgaaagat caaacacaaa tgacagtggg tcctctgcag ccataaatcg gccatgggat 3360

tttcactttc cttcgagttt gaaaagattg caattgcatg aatttcctct gacatccgat 3420

tcactatcaa caatagcgag actgctgaac cttgaagagt tgtaccttta tcgtacaatc 3480

atccatgggg aagaatggaa catgggagaa gaagacacct ttgagaatct caaatgtttg 3540

atgttgagtc aagtgattct ttccaagtgg gaggttggag aggaatcttt tcccacgctt 3600

gagaaattag aactgtcgga ctgtcataat cttgaggaga ttccgtctag ttttggggat 3660

atttattcct tgaaaattat cgaacttgta aggagccctc aacttgaaaa ttccgctctc 3720

aagattaagg aatatgctga agatatgagg ggaggggacg agcttcagat ccttggccag 3780

aaggatatcc cgttatttaa gtag 3804

<210> 60
<211> 1267
<212> PRT
<213> Solanum bulbocastanum

<400> 60
Met Glu Lys Arg Lys Asp Asn Glu Glu Ala Asn Asn Ser Leu Glu Ser
1               5                   10                  15
Phe Ser Ala Leu Arg Lys Asp Ala Ala Asn Val Leu Asp Phe Leu Glu
            20                  25                  30
Arg Leu Lys Asn Glu Glu Asp Gln Lys Ala Val Asp Val Asp Leu Ile
        35                  40                  45
Glu Ser Leu Lys Leu Lys Leu Thr Phe Ile Cys Thr Tyr Val Gln Leu
    50                  55                  60
Ser Tyr Ser Asp Leu Glu Lys Phe Glu Asp Ile Met Thr Arg Lys Arg
65                  70                  75                  80
Gln Glu Val Glu Asn Leu Leu Gln Pro Ile Leu Asp Asp Asp Gly Lys

85                  90                  95
Asp Val Gly Cys Lys Tyr Val Leu Thr Ser Leu Ala Gly Asn Met Asp
            100                 105                 110
Asp Cys Ile Ser Leu Tyr His Arg Ser Lys Ser Asp Ala Thr Met Met
        115                 120                 125
Asp Glu Gln Leu Gly Phe Leu Leu Leu Asn Leu Ser His Leu Ser Lys
    130                 135                 140
His Arg Ala Glu Lys Met Phe Pro Gly Val Thr Gln Tyr Glu Val Leu
145                 150                 155                 160
Gln Asn Val Cys Gly Asn Ile Arg Asp Phe His Gly Leu Ile Val Asn
                165                 170                 175
Cys Cys Ile Lys His Glu Met Val Glu Asn Val Leu Ser Leu Phe Gln
            180                 185                 190
Leu Met Ala Glu Arg Val Gly Arg Phe Leu Trp Glu Asp Gln Ala Asp
        195                 200                 205
Glu Asp Ser Gln Leu Ser Glu Leu Asp Glu Asp Asp Gln Asn Asp Lys
    210                 215                 220
Asp Pro Gln Leu Phe Lys Leu Ala His Leu Leu Leu Lys Ile Val Pro
225                 230                 235                 240
Thr Glu Leu Glu Val Met His Ile Cys Tyr Lys Thr Leu Lys Ala Ser
                245                 250                 255
Thr Ser Thr Glu Ile Gly Arg Phe Ile Lys Lys Leu Leu Glu Thr Ser
            260                 265                 270
Pro Asp Ile Leu Arg Glu Tyr Leu Ile His Leu Gln Glu His Met Ile
        275                 280                 285
Thr Val Ile Thr Pro Asn Thr Ser Gly Ala Arg Asn Ile His Val Met
    290                 295                 300
Met Glu Phe Leu Leu Ile Ile Leu Ser Asp Met Pro Pro Lys Asp Phe
305                 310                 315                 320
Ile His His Asp Lys Leu Phe Asp Leu Leu Ala Arg Val Val Ala Leu
                325                 330                 335
Thr Arg Glu Val Ser Thr Leu Val Arg Asp Leu Glu Glu Lys Leu Arg
            340                 345                 350
Ile Lys Glu Ser Thr Asp Glu Thr Asn Cys Ala Thr Leu Lys Phe Leu
        355                 360                 365
Glu Asn Ile Glu Leu Leu Lys Glu Asp Leu Lys His Val Tyr Leu Lys
    370                 375                 380
Val Pro Asp Ser Ser Gln Tyr Cys Phe Pro Met Ser Asp Gly Pro Leu
385                 390                 395                 400
Phe Met His Leu Leu Gln Arg His Leu Asp Asp Leu Leu Asp Ser Asn
                405                 410                 415
Ala Tyr Ser Ile Ala Leu Ile Lys Glu Gln Ile Gly Leu Val Lys Glu
            420                 425                 430
Asp Leu Glu Phe Ile Arg Ser Phe Phe Ala Asn Ile Glu Gln Gly Leu
        435                 440                 445
Tyr Lys Asp Leu Trp Glu Arg Val Leu Asp Val Ala Tyr Glu Ala Lys
    450                 455                 460
Asp Val Ile Asp Ser Ile Ile Val Arg Asp Asn Gly Leu Leu His Leu
465                 470                 475                 480
Ile Phe Ser Leu Pro Ile Thr Arg Lys Lys Met Met Leu Ile Lys Glu
                485                 490                 495
Glu Val Ser Asp Leu His Glu Asn Ile Ser Lys Asn Arg Gly Leu Ile
            500                 505                 510
Val Val Asn Ser Pro Lys Lys Pro Val Glu Ser Lys Ser Leu Thr Thr
        515                 520                 525
Asp Lys Ile Ile Val Gly Phe Gly Glu Glu Thr Asn Leu Ile Leu Arg
    530                 535                 540
Lys Leu Thr Ser Gly Pro Ala Asp Leu Asp Val Ile Ser Ile Ile Gly
545                 550                 555                 560
Met Pro Gly Leu Gly Lys Thr Thr Leu Ala Tyr Lys Val Tyr Asn Asp
                565                 570                 575
Lys Ser Val Ser Ser His Phe Asp Leu Arg Ala Trp Cys Thr Val Asp
            580                 585                 590

Gln Val Tyr Asp Glu Lys Lys Leu Leu Asp Lys Ile Phe Asn Gln Val
            595                 600                 605
Ser Asp Ser Asn Ser Lys Leu Ser Glu Asn Ile Asp Val Ala Asp Lys
        610                 615                 620
Leu Arg Lys Gln Leu Phe Gly Lys Arg Tyr Leu Ile Val Leu Asp Asp
625                 630                 635                 640
Val Trp Asp Thr Asn Thr Trp Asp Glu Leu Thr Arg Pro Phe Pro Asp
                645                 650                 655
Gly Met Lys Gly Ser Arg Ile Ile Leu Thr Thr Arg Glu Lys Lys Val
            660                 665                 670
Ala Leu His Gly Lys Leu Tyr Thr Asp Pro Leu Asn Leu Arg Leu Leu
        675                 680                 685
Arg Ser Glu Glu Ser Trp Glu Leu Leu Glu Lys Arg Ala Phe Gly Asn
    690                 695                 700
Glu Ser Cys Pro Asp Glu Leu Leu Asp Val Gly Lys Glu Ile Ala Glu
705                 710                 715                 720
Asn Cys Lys Gly Leu Pro Leu Val Val Asp Leu Ile Ala Gly Ile Ile
                725                 730                 735
Ala Gly Arg Glu Lys Lys Lys Ser Val Trp Leu Glu Val Val Asn Asn
            740                 745                 750
Leu His Ser Phe Ile Leu Lys Asn Glu Val Glu Val Met Lys Val Ile
        755                 760                 765
Glu Ile Ser Tyr Asp His Leu Pro Asp His Leu Lys Pro Cys Leu Leu
    770                 775                 780
Tyr Phe Ala Ser Ala Pro Lys Asp Trp Val Thr Thr Ile His Glu Leu
785                 790                 795                 800
Lys Leu Ile Trp Gly Phe Glu Gly Phe Val Glu Lys Thr Asp Met Lys
                805                 810                 815
Ser Leu Glu Glu Val Val Lys Ile Tyr Leu Asp Asp Leu Ile Ser Ser
            820                 825                 830
Ser Leu Val Ile Cys Phe Asn Glu Ile Gly Asp Tyr Pro Thr Cys Gln
        835                 840                 845
Leu His Asp Leu Val His Asp Phe Cys Leu Ile Lys Ala Arg Lys Glu
    850                 855                 860
Lys Leu Cys Asp Arg Ile Ser Ser Ser Ala Pro Ser Asp Leu Leu Pro
865                 870                 875                 880
Arg Gln Ile Ser Ile Asp Tyr Asp Asp Asp Glu Glu His Phe Gly Leu
                885                 890                 895
Asn Phe Val Leu Phe Gly Ser Asn Lys Lys Arg His Ser Gly Lys His
            900                 905                 910
Leu Tyr Ser Leu Thr Ile Asn Gly Asp Glu Leu Asp Asp His Leu Ser
        915                 920                 925
Asp Thr Phe His Leu Arg His Leu Arg Leu Leu Arg Thr Leu His Leu
    930                 935                 940
Glu Ser Ser Phe Ile Met Val Lys Asp Ser Leu Leu Asn Glu Ile Cys
945                 950                 955                 960
Met Leu Asn His Leu Arg Tyr Leu Ser Ile Gly Thr Glu Val Lys Ser
                965                 970                 975
Leu Pro Leu Ser Phe Ser Asn Leu Trp Asn Leu Glu Ile Leu Phe Val
            980                 985                 990
Asp Asn Lys Glu Ser Thr Leu Ile Leu Leu Pro Arg Ile Trp Asp Leu
        995                 1000                1005
Val Lys Leu Gln Val Leu Phe Thr Thr Ala Cys Ser Phe Phe Asp Met
    1010                1015                1020
Asp Ala Asp Glu Ser Ile Leu Ile Ala Glu Asp Thr Lys Leu Glu Asn
1025                1030                1035                1040
Leu Thr Ala Leu Gly Glu Leu Val Leu Ser Tyr Trp Lys Asp Thr Glu
                1045                1050                1055
Asp Ile Phe Lys Arg Leu Pro Asn Leu Gln Val Leu His Phe Lys Leu
            1060                1065                1070
Lys Glu Ser Trp Asp Tyr Ser Thr Glu Gln Tyr Trp Phe Pro Lys Leu
        1075                1080                1085
Asp Phe Leu Thr Glu Leu Glu Lys Leu Thr Val Asp Phe Glu Arg Ser

1090                1095                1100
Asn Thr Asn Asp Ser Gly Ser Ser Ala Ala Ile Asn Arg Pro Trp Asp
1105                1110                1115                1120
Phe His Phe Pro Ser Ser Leu Lys Arg Leu Gln Leu His Glu Phe Pro
                1125                1130                1135
Leu Thr Ser Asp Ser Leu Ser Thr Ile Ala Arg Leu Leu Asn Leu Glu
            1140                1145                1150
Glu Leu Tyr Leu Tyr Arg Thr Ile Ile His Gly Glu Glu Trp Asn Met
        1155                1160                1165
Gly Glu Glu Asp Thr Phe Glu Asn Leu Lys Cys Leu Met Leu Ser Gln
    1170                1175                1180
Val Ile Leu Ser Lys Trp Glu Val Gly Glu Glu Ser Phe Pro Thr Leu
1185                1190                1195                1200
Glu Lys Leu Glu Leu Ser Asp Cys His Asn Leu Glu Glu Ile Pro Ser
                1205                1210                1215
Ser Phe Gly Asp Ile Tyr Ser Leu Lys Ile Ile Glu Leu Val Arg Ser
            1220                1225                1230
Pro Gln Leu Glu Asn Ser Ala Leu Lys Ile Lys Glu Tyr Ala Glu Asp
        1235                1240                1245
Met Arg Gly Gly Asp Glu Leu Gln Ile Leu Gly Gln Lys Asp Ile Pro
    1250                1255                1260
Leu Phe Lys
1265

<210> 61
<211> 3890
<212> DNA
<213> Solanum bulbocastanum


<400> 61
atggaaaaac gaaaagataa tgaagaagca aacaactcat tggtatgtta tttgatagag      60

tgaactgtaa agtattgaat tgtagatatc atgtggcttt aaaaatttga tatgtgttat     120

tttggcagga gtcattttct gctcttcgca aggatgctgc caatgttctg gatttcctag     180

agagattaaa gaatgaagaa gatcaaaagg ctgttgatgt ggatctgatt gaaagcctga     240

aattgaagct gacatttatt tgtacatatg tccagctttc ttattccgat ttggagaagt     300

ttgaagatat aatgactaga aaaagacaag aggttgagaa tctgcttcaa ccaattttgg     360

atgatgatgg caaagacgtc gggtgtaaat atgtccttac tagcctcgcc ggtaatatgg     420

atgactgtat aagcttgtat catcgttcta aatcagatgc caccatgatg gatgagcaat     480

tgggcttcct cctcttgaat ctctctcatc tatccaagca tcgtgctgaa aagatgtttc     540

ctggagtgac tcaatatgag gttcttcaga atgtatgtgg caacataaga gatttccatg     600

gattgatagt gaattgttgc attaagcatg agatggttga gaatgtctta tctctgtttc     660

aactgatggc tgagagagta ggacgcttcc tttgggagga tcaggctgat gaagactctc     720

aactctccga gctagatgag gatgatcaga atgataaaga ccctcaactc ttcaagctag     780

cacatctact cttgaagatt gttccaactg aattggaggt tatgcacata tgttataaaa     840

ctttgaaagc ttcaacttca acagaaattg gacgcttcat taagaagctc ctggaaacct     900

ctccggacat tctcagagaa tatctgattc atctacaaga gcatatgata actgttatta     960

cccctaacac ttcaggggct cgaaacattc atgtcatgat ggaattccta ttgattattc 1020

tttctgatat gccgcccaag gactttattc atcatgacaa actttttgat ctcttggctc 1080

gtgttgtagc acttaccagg gaggtatcaa ctcttgtacg cgacttggaa gagaaattaa 1140

ggattaaaga gagtactgac gaaacaaatt gtgcaaccct aaagtttctg gaaaatattg 1200

aactccttaa ggaagatctc aaacatgttt atctgaaagt cccggattca tctcaatatt 1260

gcttccccat gagtgatgga cctctcttca tgcatctgct acagagacac ttagatgatt 1320

tgctggattc caatgcttat tcaattgctt tgataaagga acaaattggg ctggtgaaag 1380

aagacttgga attcataaga tcttttttcg cgaatattga gcaaggattg tataaagatc 1440

tctgggaacg tgttctagat gtggcatatg aggcaaaaga tgtcatagat tcaattattg 1500

ttcgagataa tggtctctta catcttattt tctcacttcc cattaccaga aagaagatga 1560

tgcttatcaa agaagaggtc tctgatttac atgagaacat ttccaagaac agaggtctca 1620

tcgttgtgaa ctctcccaag aaaccagttg agagcaagtc attgacaact gataaaataa 1680

ttgtaggttt tggtgaggag acaaacttga tacttagaaa gctcaccagt ggaccggcag 1740

atctagatgt catttcgatc attggtatgc cgggtttagg taaaactact ttggcgtaca 1800

aagtatacaa tgataaatca gtttctagcc atttcgacct tcgtgcatgg tgcacggtcg 1860

accaagtata tgacgagaag aagttgttgg ataaaatttt caatcaagtt agtgactcaa 1920

attcaaaatt gagtgagaat attgatgttg ctgataaact acggaaacaa ttgtttggaa 1980

agaggtatct tattgtctta gatgacgtgt gggatactaa tacatgggat gagctaacaa 2040

gaccttttcc tgatggtatg aaaggaagta gaattatttt gacaactcga gaaaagaaag 2100

ttgctttgca tggaaagctc tacactgatc ctcttaacct tcgattgcta agatcagaag 2160

aaagttggga gttattagag aaaagggcat ttggaaacga gagttgccct gatgaactat 2220

tggatgttgg taaagaaata gccgaaaatt gtaaagggct tcctttggtg gtggatctga 2280

ttgctggaat cattgctggg agggaaaaga aaaagagtgt gtggcttgaa gttgtaaata 2340

atttgcattc ctttattttg aagaatgaag tggaagtgat gaaagttata gaaataagtt 2400

atgaccactt acctgatcac ctgaagccat gcttgctgta ctttgcaagt gcgccgaagg 2460

actgggtaac gacaatccat gagttgaaac ttatttgggg ttttgaagga tttgtggaaa 2520

agacagatat gaagagtctg gaagaagtgg tgaaatttta tttggatgat ttaatttcca 2580

gtagcttggt aatttgtttc aatgagatag gtgattaccc tacttgccaa cttcatgatc 2640

ttgtgcatga cttttgtttg ataaaagcaa gaaaggaaaa gttgtgtgat cggataagtt 2700

caagtgctcc atcagatttg ttgccacgtc aaattagcat tgattatgat gatgatgaag 2760

agcactttgg gcttaatttt gtcctgttcg gttcaaataa gaaaaggcat tccggtaaac 2820

acctctattc tttgaccata aatggagatg agctggacga ccatctttct gatacatttc 2880

atctaagaca cttgaggctt cttagaacct tgcacctgga atcctctttt atcatggtta 2940

aagattcttt gctgaatgaa atatgcatgt tgaatcattt gaggtactta agcattggga 3000

cagaagttaa atctctgcct ttgtctttct caaacctctg gaatctagaa atcttgtttg 3060

tggataacaa agaatcaacc ttgatactat taccgagaat ttgggatctt gtaaagttgc 3120

aagtgctgtt cacgactgct tgttctttct ttgatatgga tgcagatgaa tcaatactga 3180

tagcagagga cacaaagtta gagaacttga cagcattagg ggaactcgtg ctttcctatt 3240

ggaaagatac agaggatatt ttcaaaaggc ttcccaatct tcaagtgctt catttcaaac 3300

tcaaggagtc atgggattat tcaacagagc aatattggtt cccgaaattg gatttcctaa 3360

ctgaactaga aaaactcact gtagattttg aaagatcaaa cacaaatgac agtgggtcct 3420

ctgcagccat aaatcggcca tgggattttc actttccttc gagtttgaaa agattgcaat 3480

tgcatgaatt tcctctgaca tccgattcac tatcaacaat agcgagactg ctgaaccttg 3540

aagagttgta cctttatcgt acaatcatcc atggggaaga atggaacatg ggagaagaag 3600

acacctttga gaatctcaaa tgtttgatgt tgagtcaagt gattctttcc aagtgggagg 3660

ttggagagga atcttttccc acgcttgaga aattagaact gtcggactgt cataatcttg 3720

aggagattcc gtctagtttt ggggatattt attccttgaa aattatcgaa cttgtaagga 3780

gccctcaact tgaaaattcc gctctcaaga ttaaggaata tgctgaagat atgaggggag 3840

gggacgagct tcagatcctt ggccagaagg atatcccgtt atttaagtag 3890

<210> 62
<211> 279
<212> DNA
<213> Artificial Sequence

<220>
<223> cDNA

<400> 62
cttgcctttg cggttttagc taggagcagc gccgtcgcag cattcccaat ccccgacgag 60

tctcgcccct tgtcgaagac atctcctgac actgtggccc caagatcgct tcggatcgag 120

gcccaagaag ttattcagag cggccgggga gacggatatg gtgggttctg gaaaaacgta 180

gcccagagta ctaacaagat cgtcaagagg ccggatatca agataggcaa acttatcgag 240

gcggccaaga aggcaaaagc aaaaatgacg aagtcctga 279

<210> 63
<211> 92
<212> PRT
<213> Phytophthora infestans

<400> 63
Leu Ala Phe Ala Val Leu Ala Arg Ser Ser Ala Val Ala Ala Phe Pro
1               5                   10                  15
Ile Pro Asp Glu Ser Arg Pro Leu Ser Lys Thr Ser Pro Asp Thr Val
            20                  25                  30
Ala Pro Arg Ser Leu Arg Ile Glu Ala Gln Glu Val Ile Gln Ser Gly
        35                  40                  45
Arg Gly Asp Gly Tyr Gly Gly Phe Trp Lys Asn Val Ala Gln Ser Thr
    50                  55                  60
Asn Lys Ile Val Lys Arg Pro Asp Ile Lys Ile Gly Lys Leu Ile Glu
65                  70                  75                  80
Ala Ala Lys Lys Ala Lys Ala Lys Met Thr Lys Ser
                85                  90

<210> 64
<211> 2913
<212> DNA
<213> Artificial Sequence

<220>
<223> cDNA

<400> 64
atggctgaag ctttcattca agttctgcta gacaatctca cttctttcct caaaggggaa      60

cttgtattgc ttttcggttt tcaagatgag ttccaaaggc tttcaagcat gttttctaca     120

attcaagccg tccttgaaga tgctcaggag aagcaactca acaacaagcc tctagaaaat     180

tggttgcaaa aactcaatgc tgctacatat gaagtcgatg acatcttgga tgaatataaa     240

accaaggcca caagattctc ccagtctgaa tatggccgtt atcatccaaa ggttatccct     300

ttccgtcaca aggtcgggaa aaggatggac caagtgatga aaaaactaaa ggcaattgct     360

gaggaaagaa agaattttca tttgcacgaa aaaattgtag agagacaagc tgttagacgg     420

gaaacaggtt ctgtattaac cgaaccgcag gtttatggaa gagacaaaga gaaagatgag     480

atagtgaaaa tcctaataaa caatgttagt gatgcccaac acctttcagt cctcccaata     540

cttggtatgg ggggattagg aaaaacgact cttgcccaaa tggtcttcaa tgaccagaga     600

gttactgagc atttccattc caaaatatgg atttgtgtct cggaagattt tgatgagaag     660

aggttaataa aggcaattgt agaatctatt gaaggaaggc cactacttgg tgagatggac     720

ttggctccac ttcaaaagaa gcttcaggag ttgctgaatg gaaaaagata cttgcttgtc     780

ttagatgatg tttggaatga agatcaacag aagtgggcta atttaagagc agtcttgaag     840

gttggagcaa gtggtgcttc tgttctaacc actactcgtc ttgaaaaggt tggatcaatt     900

atgggaacat tgcaaccata tgaactgtca aatctgtctc aagaagattg ttggttgttg     960

ttcatgcaac gtgcatttgg acaccaagaa gaaataaatc caaaccttgt ggcaatcgga    1020

aaggagattg tgaaaaaaag tggtggtgtg cctctagcag ccaaaactct tggaggtatt    1080

ttgtgcttca agagagaaga aagagcatgg gaacatgtga gagacagtcc gatttggaat    1140

ttgcctcaag atgaaagttc tattctgcct gccctgaggc ttagttacca tcaacttcca    1200

cttgatttga aacaatgctt tgcgtattgt gcggtgttcc caaaggatgc caaaatggaa 1260

aaagaaaagc taatctctct ctggatggcg catggttttc ttttatcaaa aggaaacatg 1320

gagctagagg atgtgggcga tgaagtatgg aaagaattat acttgaggtc ttttttccaa 1380

gagattgaag ttaaagatgg taaaacttat ttcaagatgc atgatctcat ccatgatttg 1440

gcaacatctc tgttttcagc aaacacatca agcagcaata tccgtgaaat aaataaacac 1500

agttacacac atatgatgtc cattggtttc gccgaagtgg tgttttttta cactcttccc 1560

cccttggaaa agtttatctc gttaagagtg cttaatctag gtgattcgac atttaataag 1620

ttaccatctt ccattggaga tctagtacat ttaagatact tgaacctgta tggcagtggc 1680

atgcgtagtc ttccaaagca gttatgcaag cttcaaaatc tgcaaactct tgatctacaa 1740

tattgcacca agctttgttg tttgccaaaa gaaacaagta aacttggtag tctccgaaat 1800

cttttacttg atggtagcca gtcattgact tgtatgccac caaggatagg atcattgaca 1860

tgccttaaga ctctaggtca atttgttgtt ggaaggaaga aaggttatca acttggtgaa 1920

ctaggaaacc taaatctcta tggctcaatt aaaatctcgc atcttgagag agtgaagaat 1980

gataaggacg caaaagaagc caatttatct gcaaaaggga atctgcattc tttaagcatg 2040

agttggaata actttggacc acatatatat gaatcagaag aagttaaagt gcttgaagcc 2100

ctcaaaccac actccaatct gacttcttta aaaatctatg gcttcagagg aatccatctc 2160

ccagagtgga tgaatcactc agtattgaaa aatattgtct ctattctaat tagcaacttc 2220

agaaactgct catgcttacc accctttggt gatctgcctt gtctagaaag tctagagtta 2280

cactgggggt ctgcggatgt ggagtatgtt gaagaagtgg atattgatgt tcattctgga 2340

ttccccacaa gaataaggtt tccatccttg aggaaacttg atatatggga ctttggtagt 2400

ctgaaaggat tgctgaaaaa ggaaggagaa gagcaattcc ctgtgcttga agagatgata 2460

attcacgagt gcccttttct gaccctttct tctaatctta gggctcttac ttccctcaga 2520

atttgctata ataaagtagc tacttcattc ccagaagaga tgttcaaaaa ccttgcaaat 2580

ctcaaatact tgacaatctc tcggtgcaat aatctcaaag agctgcctac cagcttggct 2640

agtctgaatg ctttgaaaag tctaaaaatt caattgtgtt gcgcactaga gagtctccct 2700

gaggaagggc tggaaggttt atcttcactc acagagttat ttgttgaaca ctgtaacatg 2760

ctaaaatgtt taccagaggg attgcagcac ctaacaaccc tcacaagttt aaaaattcgg 2820

ggatgtccac aactgatcaa gcggtgtgag aagggaatag gagaagactg gcacaaaatt 2880

tctcacattc ctaatgtgaa tatatatatt taa 2913

<210> 65
<211> 970
<212> PRT

<213> Solanum bulbocastanum

<400> 65
Met Ala Glu Ala Phe Ile Gln Val Leu Leu Asp Asn Leu Thr Ser Phe
1               5                   10                  15
Leu Lys Gly Glu Leu Val Leu Leu Phe Gly Phe Gln Asp Glu Phe Gln
            20                  25                  30
Arg Leu Ser Ser Met Phe Ser Thr Ile Gln Ala Val Leu Glu Asp Ala
        35                  40                  45
Gln Glu Lys Gln Leu Asn Asn Lys Pro Leu Glu Asn Trp Leu Gln Lys
    50                  55                  60
Leu Asn Ala Ala Thr Tyr Glu Val Asp Asp Ile Leu Asp Glu Tyr Lys
65                  70                  75                  80
Thr Lys Ala Thr Arg Phe Ser Gln Ser Glu Tyr Gly Arg Tyr His Pro
                85                  90                  95
Lys Val Ile Pro Phe Arg His Lys Val Gly Lys Arg Met Asp Gln Val
            100                 105                 110
Met Lys Lys Leu Lys Ala Ile Ala Glu Glu Arg Lys Asn Phe His Leu
        115                 120                 125
His Glu Lys Ile Val Glu Arg Gln Ala Val Arg Arg Glu Thr Gly Ser
    130                 135                 140
Val Leu Thr Glu Pro Gln Val Tyr Gly Arg Asp Lys Glu Lys Asp Glu
145                 150                 155                 160
Ile Val Lys Ile Leu Ile Asn Asn Val Ser Asp Ala Gln His Leu Ser
                165                 170                 175
Val Leu Pro Ile Leu Gly Met Gly Gly Leu Gly Lys Thr Thr Leu Ala
            180                 185                 190
Gln Met Val Phe Asn Asp Gln Arg Val Thr Glu His Phe His Ser Lys
        195                 200                 205
Ile Trp Ile Cys Val Ser Glu Asp Phe Asp Glu Lys Arg Leu Ile Lys
    210                 215                 220
Ala Ile Val Glu Ser Ile Glu Gly Arg Pro Leu Leu Gly Glu Met Asp
225                 230                 235                 240
Leu Ala Pro Leu Gln Lys Lys Leu Gln Glu Leu Leu Asn Gly Lys Arg
                245                 250                 255
Tyr Leu Leu Val Leu Asp Asp Val Trp Asn Glu Asp Gln Gln Lys Trp
            260                 265                 270
Ala Asn Leu Arg Ala Val Leu Lys Val Gly Ala Ser Gly Ala Ser Val
        275                 280                 285
Leu Thr Thr Thr Arg Leu Glu Lys Val Gly Ser Ile Met Gly Thr Leu
    290                 295                 300
Gln Pro Tyr Glu Leu Ser Asn Leu Ser Gln Glu Asp Cys Trp Leu Leu
305                 310                 315                 320
Phe Met Gln Arg Ala Phe Gly His Gln Glu Glu Ile Asn Pro Asn Leu
                325                 330                 335
Val Ala Ile Gly Lys Glu Ile Val Lys Lys Ser Gly Gly Val Pro Leu
            340                 345                 350
Ala Ala Lys Thr Leu Gly Gly Ile Leu Cys Phe Lys Arg Glu Glu Arg
        355                 360                 365
Ala Trp Glu His Val Arg Asp Ser Pro Ile Trp Asn Leu Pro Gln Asp
    370                 375                 380
Glu Ser Ser Ile Leu Pro Ala Leu Arg Leu Ser Tyr His Gln Leu Pro
385                 390                 395                 400
Leu Asp Leu Lys Gln Cys Phe Ala Tyr Cys Ala Val Phe Pro Lys Asp
                405                 410                 415
Ala Lys Met Glu Lys Glu Lys Leu Ile Ser Leu Trp Met Ala His Gly
            420                 425                 430
Phe Leu Leu Ser Lys Gly Asn Met Glu Leu Glu Asp Val Gly Asp Glu
        435                 440                 445
Val Trp Lys Glu Leu Tyr Leu Arg Ser Phe Phe Gln Glu Ile Glu Val
    450                 455                 460
Lys Asp Gly Lys Thr Tyr Phe Lys Met His Asp Leu Ile His Asp Leu

465                 470                 475                 480
Ala Thr Ser Leu Phe Ser Ala Asn Thr Ser Ser Ser Asn Ile Arg Glu
                485                 490                 495
Ile Asn Lys His Ser Tyr Thr His Met Met Ser Ile Gly Phe Ala Glu
            500                 505                 510
Val Val Phe Phe Tyr Thr Leu Pro Pro Leu Glu Lys Phe Ile Ser Leu
        515                 520                 525
Arg Val Leu Asn Leu Gly Asp Ser Thr Phe Asn Lys Leu Pro Ser Ser
    530                 535                 540
Ile Gly Asp Leu Val His Leu Arg Tyr Leu Asn Leu Tyr Gly Ser Gly
545                 550                 555                 560
Met Arg Ser Leu Pro Lys Gln Leu Cys Lys Leu Gln Asn Leu Gln Thr
                565                 570                 575
Leu Asp Leu Gln Tyr Cys Thr Lys Leu Cys Cys Leu Pro Lys Glu Thr
            580                 585                 590
Ser Lys Leu Gly Ser Leu Arg Asn Leu Leu Leu Asp Gly Ser Gln Ser
        595                 600                 605
Leu Thr Cys Met Pro Pro Arg Ile Gly Ser Leu Thr Cys Leu Lys Thr
    610                 615                 620
Leu Gly Gln Phe Val Val Gly Arg Lys Lys Gly Tyr Gln Leu Gly Glu
625                 630                 635                 640
Leu Gly Asn Leu Asn Leu Tyr Gly Ser Ile Lys Ile Ser His Leu Glu
                645                 650                 655
Arg Val Lys Asn Asp Lys Asp Ala Lys Glu Ala Asn Leu Ser Ala Lys
            660                 665                 670
Gly Asn Leu His Ser Leu Ser Met Ser Trp Asn Asn Phe Gly Pro His
        675                 680                 685
Ile Tyr Glu Ser Glu Glu Val Lys Val Leu Glu Ala Leu Lys Pro His
    690                 695                 700
Ser Asn Leu Thr Ser Leu Lys Ile Tyr Gly Phe Arg Gly Ile His Leu
705                 710                 715                 720
Pro Glu Trp Met Asn His Ser Val Leu Lys Asn Ile Val Ser Ile Leu
                725                 730                 735
Ile Ser Asn Phe Arg Asn Cys Ser Cys Leu Pro Pro Phe Gly Asp Leu
            740                 745                 750
Pro Cys Leu Glu Ser Leu Glu Leu His Trp Gly Ser Ala Asp Val Glu
        755                 760                 765
Tyr Val Glu Glu Val Asp Ile Asp Val His Ser Gly Phe Pro Thr Arg
    770                 775                 780
Ile Arg Phe Pro Ser Leu Arg Lys Leu Asp Ile Trp Asp Phe Gly Ser
785                 790                 795                 800
Leu Lys Gly Leu Leu Lys Lys Glu Gly Glu Glu Gln Phe Pro Val Leu
                805                 810                 815
Glu Glu Met Ile Ile His Glu Cys Pro Phe Leu Thr Leu Ser Ser Asn
            820                 825                 830
Leu Arg Ala Leu Thr Ser Leu Arg Ile Cys Tyr Asn Lys Val Ala Thr
        835                 840                 845
Ser Phe Pro Glu Glu Met Phe Lys Asn Leu Ala Asn Leu Lys Tyr Leu
    850                 855                 860
Thr Ile Ser Arg Cys Asn Asn Leu Lys Glu Leu Pro Thr Ser Leu Ala
865                 870                 875                 880
Ser Leu Asn Ala Leu Lys Ser Leu Lys Ile Gln Leu Cys Cys Ala Leu
                885                 890                 895
Glu Ser Leu Pro Glu Glu Gly Leu Glu Gly Leu Ser Ser Leu Thr Glu
            900                 905                 910
Leu Phe Val Glu His Cys Asn Met Leu Lys Cys Leu Pro Glu Gly Leu
        915                 920                 925
Gln His Leu Thr Thr Leu Thr Ser Leu Lys Ile Arg Gly Cys Pro Gln
    930                 935                 940
Leu Ile Lys Arg Cys Glu Lys Gly Ile Gly Glu Asp Trp His Lys Ile
945                 950                 955                 960
Ser His Ile Pro Asn Val Asn Ile Tyr Ile
                965                 970

<210> 66
<211> 3592
<212> DNA
<213> Solanum bulbocastanum

<400> 66
atggctgaag ctttcattca agttctgcta gacaatctca cttctttcct caaaggggaa 60
cttgtattgc ttttcggttt tcaagatgag ttccaaaggc tttcaagcat gttttctaca 120
attcaagccg tccttgaaga tgctcaggag aagcaactca acaacaagcc tctagaaaat 180
tggttgcaaa aactcaatgc tgctacatat gaagtcgatg acatcttgga tgaatataaa 240
accaaggcca caagattctc ccagtctgaa tatggccgtt atcatccaaa ggttatccct 300
ttccgtcaca aggtcgggaa aaggatggac caagtgatga aaaaactaaa ggcaattgct 360
gaggaaagaa agaattttca tttgcacgaa aaaattgtag agagacaagc tgttagacgg 420
gaaacaggta ctcatcttaa attagtatta caacaactaa gtttatattc atttttttgg 480
caattatcaa attcagaaaa gggttaaata tactcatgtc ctatcgtaaa tagtgtatat 540
atacctctcg ttgtactttc gatctgaata tacttgtcaa atctggcaag ctcagaatca 600
aattatccac cccaactttt aaatactcga tatctttaga aatccacctg tctaactcat 660
ccactaccca ttccctttgc tttgaattct tttctttacc tataaacttg gaacactcga 720
tccgttttgc ttttcttaac aaagcagctc agagaaaaga ggttttcttc tattctgttt 780
ctctgtgtgc tgcacttggg tccttaatcc cattaaaaac agggcatgtt aatcccaacg 840
acggtagcct ttcctgacag ctgactgtaa attttgtcta acaaagaaaa aaaaagatta 900
gacatgtttt tccttgtcat tgattaggct ggatttcttt cagagtggaa cataggggat 960
atattggacc aaaagtagaa tgggtatata tttaaagtat ttctgataga acaggagtat 1020
attgtgcgaa aatatcctct attttctgtt gtctcctaat gagtttgaat gtaataatat 1080
tctcatgtgg acattgcttg caccaggttc tgtattaacc gaaccgcagg tttatggaag 1140
agacaaagag aaagatgaga tagtgaaaat cctaataaac aatgttagtg atgcccaaca 1200
cctttcagtc ctcccaatac ttggtatggg gggattagga aaaacgactc ttgcccaaat 1260
ggtcttcaat gaccagagag ttactgagca tttccattcc aaaatatgga tttgtgtctc 1320
ggaagatttt gatgagaaga ggttaataaa ggcaattgta gaatctattg aaggaaggcc 1380
actacttggt gagatggact tggctccact tcaaaagaag cttcaggagt tgctgaatgg 1440
aaaaagatac ttgcttgtct tagatgatgt ttggaatgaa gatcaacaga agtgggctaa 1500
tttaagagca gtcttgaagg ttggagcaag tggtgcttct gttctaacca ctactcgtct 1560
tgaaaaggtt ggatcaatta tgggaacatt gcaaccatat gaactgtcaa atctgtctca 1620
agaagattgt tggttgttgt tcatgcaacg tgcatttgga caccaagaag aaataaatcc 1680

aaaccttgtg gcaatcggaa aggagattgt gaaaaaaagt ggtggtgtgc ctctagcagc 1740

caaaactctt ggaggtattt tgtgcttcaa gagagaagaa agagcatggg aacatgtgag 1800

agacagtccg atttggaatt tgcctcaaga tgaaagttct attctgcctg ccctgaggct 1860

tagttaccat caacttccac ttgatttgaa acaatgcttt gcgtattgtg cggtgttccc 1920

aaaggatgcc aaaatggaaa aagaaaagct aatctctctc tggatggcgc atggtttctt 1980

tttatcaaaa ggaaacatgg agctagagga tgtgggcgat gaagtatgga aagaattata 2040

cttgaggtct tttttccaag agattgaagt taaagatggt aaaacttatt tcaagatgca 2100

tgatctcatc catgatttgg caacatctct gttttcagca aacacatcaa gcagcaatat 2160

ccgtgaaata aataaacaca gttacacaca tatgatgtcc attggtttcg ccgaagtggt 2220

gtttttttac actcttcccc ccttggaaaa gtttatctcg ttaagagtgc ttaatctagg 2280

tgattcgaca tttaataagt taccatcttc cattggagat ctagtacatt taagatactt 2340

gaacctgtat ggcagtggca tgcgtagtct tccaaagcag ttatgcaagc ttcaaaatct 2400

gcaaactctt gatctacaat attgcaccaa gctttgttgt ttgccaaaag aaacaagtaa 2460

acttggtagt ctccgaaatc ttttacttga tggtagccag tcattgactt gtatgccacc 2520

aaggatagga tcattgacat gccttaagac tctaggtcaa tttgttgttg gaaggaagaa 2580

aggttatcaa cttggtgaac taggaaacct aaatctctat ggctcaatta aaatctcgca 2640

tcttgagaga gtgaagaatg ataaggacgc aaaagaagcc aatttatctg caaaagggaa 2700

tctgcattct ttaagcatga gttggaataa ctttggacca catatatatg aatcagaaga 2760

agttaaagtg cttgaagccc tcaaaccaca ctccaatctg acttctttaa aaatctatgg 2820

cttcagagga atccatctcc cagagtggat gaatcactca gtattgaaaa atattgtctc 2880

tattctaatt agcaacttca gaaactgctc atgcttacca ccctttggtg atctgccttg 2940

tctagaaagt ctagagttac actgggggtc tgcggatgtg gagtatgttg aagaagtgga 3000

tattgatgtt cattctggat tccccacaag aataaggttt ccatccttga ggaaacttga 3060

tatatgggac tttggtagtc tgaaaggatt gctgaaaaag gaaggagaag agcaattccc 3120

tgtgcttgaa gagatgataa ttcacgagtg cccttttctg accctttctt ctaatcttag 3180

ggctcttact tccctcagaa tttgctataa taaagtagct acttcattcc cagaagagat 3240

gttcaaaaac cttgcaaatc tcaaatactt gacaatctct cggtgcaata atctcaaaga 3300

gctgcctacc agcttggcta gtctgaatgc tttgaaaagt ctaaaaattc aattgtgttg 3360

cgcactagag agtctccctg aggaagggct ggaaggttta tcttcactca cagagttatt 3420

tgttgaacac tgtaacatgc taaaatgttt accagaggga ttgcagcacc taacaaccct 3480

cacaagttta aaaattcggg gatgtccaca actgatcaag cggtgtgaga agggaatagg 3540

agaagactgg cacaaaattt ctcacattcc taatgtgaat atatatattt aa          3592

<210> 67
<211> 459
<212> DNA
<213> Artificial Sequence

<220>
<223> cDNA

<400> 67
atgcgttcgc tcctgttgac cgtgcttttg aacctggtcg tgcttctagc aaccactggg      60

gcagtttcat ccaatctcaa caccgccgtg aattacgctt ccacatccaa gattcgcttt     120

ctgtcgactg agtacaacgc cgatgaaaaa agaagcttgc gaggtgacta caacaatgag     180

gtcacaaaag agcccaacac gtctgacgaa gagcgggcgt tttctatctc aaagtctgcg     240

gaatacgtga agatggtact ttatggattc aaacttggat tttctcctcg cactcagtcc     300

aagacggtgt tgcgatacga agataaactg tttacggctc tctataaatc cggagagacg     360

ccgagaagcc taaggaccaa gcatctcgat aaggcttccg ctagcgtatt tttcaacaga     420

ttcaaaaaat ggtacgataa aaacgttggc cctagctag                            459

<210> 68
<211> 152
<212> PRT
<213> Phytophthora infestans

<400> 68
Met Arg Ser Leu Leu Leu Thr Val Leu Leu Asn Leu Val Val Leu Leu
1               5                   10                  15
Ala Thr Thr Gly Ala Val Ser Ser Asn Leu Asn Thr Ala Val Asn Tyr
            20                  25                  30
Ala Ser Thr Ser Lys Ile Arg Phe Leu Ser Thr Glu Tyr Asn Ala Asp
        35                  40                  45
Glu Lys Arg Ser Leu Arg Gly Asp Tyr Asn Asn Glu Val Thr Lys Glu
    50                  55                  60
Pro Asn Thr Ser Asp Glu Glu Arg Ala Phe Ser Ile Ser Lys Ser Ala
65                  70                  75                  80
Glu Tyr Val Lys Met Val Leu Tyr Gly Phe Lys Leu Gly Phe Ser Pro
                85                  90                  95
Arg Thr Gln Ser Lys Thr Val Leu Arg Tyr Glu Asp Lys Leu Phe Thr
            100                 105                 110
Ala Leu Tyr Lys Ser Gly Glu Thr Pro Arg Ser Leu Arg Thr Lys His
        115                 120                 125
Leu Asp Lys Ala Ser Ala Ser Val Phe Phe Asn Arg Phe Lys Lys Trp
    130                 135                 140
Tyr Asp Lys Asn Val Gly Pro Ser
145                 150

<210> 69
<211> 2913
<212> DNA
<213> Artificial Sequence

<220>
<223> cDNA

<400> 69
atggctgaag ctttcattca agttctgtta gacaatctca cttctttcct caaaggggaa 60

cttacattgc ttttcggttt tcaagatgag ttccaaaggc tttcaagcat gttttctaca 120

atccaagccg tccttgaaga tgctcaggag aagcaactca acaacaagcc tctagaaaat 180

tggttgcaaa aactcaatgc tgctacatac gaagtcgatg acatcttgga tgaatataaa 240

accaaggcca caagattctc ccagtctgaa tatggccgtt atcatccaaa ggttatccct 300

ttccgtcaca aggtcgggaa aaggatggac caagtgatga aaaaactaaa ggcaattgct 360

gaggaaagaa agaattttca tttgcacgaa aaaattgtag agagacaagc tgttagacgg 420

gaaacaggtt ctgtattaac cgaaccgcag gtttatggaa gagacaaaga gaaagatgag 480

atagtgaaaa tcctaataaa caatgttagt gatgcccaac acctttcagt cctcccaata 540

cttggtatgg ggggattagg aaaaacgact cttgcccaaa tggtcttcaa tgaccagaga 600

gttactgagc atttccattc caaaatatgg atttgtgtct cggaagattt tgatgagaag 660

aggttaataa aggcaattgt agaatctatt gaaggaaggc cactacttgg tgagatggac 720

ttggctccac ttcaaaagaa gcttcaggag ttgctgaatg gaaaaagata cttgcttgtc 780

ttagatgatg tttggaatga agatcaacag aagtgggcta atttaagagc agtcttgaag 840

gttggagcaa gtggtgcttc tgttctaacc actactcgtc ttgaaaaggt tggatcaatt 900

atgggaacat tgcaaccata tgaactgtca aatctgtctc aagaagattg ttggttgttg 960

ttcatgcaac gtgcatttgg acaccaagaa gaaataaatc caaaccttgt ggcaatcgga 1020

aaggagattg tgaaaaaaag tggtggtgtg cctctagcag ccaaaactct tggaggtatt 1080

ttgtgcttca agagagaaga aagagcatgg gaacatgtga gagacagtcc gatttggaat 1140

ttgcctcaag atgaaagttc tattctgcct gccctgaggc ttagttacca tcaacttcca 1200

cttgatttga aacaatgctt tgcgtattgt gcggtgttcc caaaggatgc caaaatggaa 1260

aaagaaaagc taatctctct ctggatggcg catggttttc ttttatcaaa aggaaacatg 1320

gagctagagg atgtgggcga tgaagtatgg aaagaattat acttgaggtc tttttttcaa 1380

gagattgaag ttaaagatgg taaaacttat ttcaagatgc atgatctcat ccatgatttg 1440

gcaacatctc tgttttcagc aaacacatca agcagcaata tccgtgaaat aaataaacac 1500

agttacacac atatgatgtc cattggtttc gccgaagtgg tgttttttta cactcttccc 1560

cccttggaaa agtttatctc gttaagagtg cttaatctag gtgattcgac atttaataag 1620

ttaccatctt ccattggaga tctagtacat ttaagatact tgaacctgta tggcagtggc 1680

atgcgtagtc ttccaaagca gttatgcaag cttcaaaatc tgcaaactct tgatctacaa 1740

tattgcacca agctttgttg tttgccaaaa gaaacaagta aacttggtag tctccgaaat 1800

cttttacttg atggtagcca gtcattgact tgtatgccac caaggatagg atcattgaca 1860

tgccttaaga ctctaggtca atttgttgtt ggaaggaaga aaggttatca acttggtgaa 1920

ctaggaaacc taaatctcta tggctcaatt aaaatctcgc atcttgagag agtgaagaat 1980

gatagggacg caaaagaagc caatttatct gcaaaaggga atctgcattc tttaagcatg 2040

agttggaata actttggacc acatatatat gaatcagaag aagttaaagt gcttgaagcc 2100

ctcaaaccac actccaatct gacttcttta aaaatctatg gcttcagagg aatccatctc 2160

ccagagtgga tgaatcactc agtattgaaa aatattgtct ctattctaat tagcaacttc 2220

agaaactgct catgcttacc accctttggt gatctgcctt gtctagaaag tctagagtta 2280

cactgggggt ctgcggatgt ggagtatgtt gaagaagtgg atattgatgt tcattctgga 2340

ttccccacaa gaataaggtt tccatccttg aggaaacttg atatatggga ctttggtagt 2400

ctgaaaggat tgctgaaaaa ggaaggagaa gagcaattcc ctgtgcttga agagctgata 2460

attcacgagt gcccttttct gaccctttct tctaatctta gggctcttac ttccctcaga 2520

atttgctata ataaagtagc tacttcattc ccagaagaga tgttcaaaaa ccttgcaaat 2580

ctcaaatact tgacaatctc tcggtgcaat aatctcaaag agctgcctac cagcttggct 2640

agtctgaatg ctttgaaaag tctaaaaatt caattgtgtt gcgcactaga gagtctccct 2700

gaggaagggc tggaaggttt atcttcactc acagagttat ttgttgaaca ctgtaacatg 2760

ctaaaatgtt taccagaggg attgcagcac ctaacaaccc tcacaagttt aaaaattcgg 2820

ggatgtccac aactgatcaa gcggtgtgag aagggaatag gagaagactg gcacaaaatt 2880

tctcacattc ctaatgtgaa tatatataat taa 2913

<210> 70
<211> 970
<212> PRT
<213> Solanum stoloniferum

<400> 70
Met Ala Glu Ala Phe Ile Gln Val Leu Leu Asp Asn Leu Thr Ser Phe
1               5                   10                  15
Leu Lys Gly Glu Leu Thr Leu Leu Phe Gly Phe Gln Asp Glu Phe Gln
            20                  25                  30
Arg Leu Ser Ser Met Phe Ser Thr Ile Gln Ala Val Leu Glu Asp Ala
        35                  40                  45
Gln Glu Lys Gln Leu Asn Asn Lys Pro Leu Glu Asn Trp Leu Gln Lys
    50                  55                  60
Leu Asn Ala Ala Thr Tyr Glu Val Asp Asp Ile Leu Asp Glu Tyr Lys
65                  70                  75                  80
Thr Lys Ala Thr Arg Phe Ser Gln Ser Glu Tyr Gly Arg Tyr His Pro
                85                  90                  95
Lys Val Ile Pro Phe Arg His Lys Val Gly Lys Arg Met Asp Gln Val
            100                 105                 110
Met Lys Lys Leu Lys Ala Ile Ala Glu Glu Arg Lys Asn Phe His Leu

115                 120                 125
His Glu Lys Ile Val Glu Arg Gln Ala Val Arg Arg Glu Thr Gly Ser
    130                 135                 140
Val Leu Thr Glu Pro Gln Val Tyr Gly Arg Asp Lys Glu Lys Asp Glu
145                 150                 155                 160
Ile Val Lys Ile Leu Ile Asn Asn Val Ser Asp Ala Gln His Leu Ser
                165                 170                 175
Val Leu Pro Ile Leu Gly Met Gly Gly Leu Gly Lys Thr Thr Leu Ala
            180                 185                 190
Gln Met Val Phe Asn Asp Gln Arg Val Thr Glu His Phe His Ser Lys
        195                 200                 205
Ile Trp Ile Cys Val Ser Glu Asp Phe Asp Glu Lys Arg Leu Ile Lys
    210                 215                 220
Ala Ile Val Glu Ser Ile Glu Gly Arg Pro Leu Leu Gly Glu Met Asp
225                 230                 235                 240
Leu Ala Pro Leu Gln Lys Lys Leu Gln Glu Leu Leu Asn Gly Lys Arg
                245                 250                 255
Tyr Leu Leu Val Leu Asp Asp Val Trp Asn Glu Asp Gln Gln Lys Trp
            260                 265                 270
Ala Asn Leu Arg Ala Val Leu Lys Val Gly Ala Ser Gly Ala Ser Val
        275                 280                 285
Leu Thr Thr Thr Arg Leu Glu Lys Val Gly Ser Ile Met Gly Thr Leu
    290                 295                 300
Gln Pro Tyr Glu Leu Ser Asn Leu Ser Gln Glu Asp Cys Trp Leu Leu
305                 310                 315                 320
Phe Met Gln Arg Ala Phe Gly His Gln Glu Glu Ile Asn Pro Asn Leu
                325                 330                 335
Val Ala Ile Gly Lys Glu Ile Val Lys Lys Ser Gly Gly Val Pro Leu
            340                 345                 350
Ala Ala Lys Thr Leu Gly Gly Ile Leu Cys Phe Lys Arg Glu Glu Arg
        355                 360                 365
Ala Trp Glu His Val Arg Asp Ser Pro Ile Trp Asn Leu Pro Gln Asp
    370                 375                 380
Glu Ser Ser Ile Leu Pro Ala Leu Arg Leu Ser Tyr His Gln Leu Pro
385                 390                 395                 400
Leu Asp Leu Lys Gln Cys Phe Ala Tyr Cys Ala Val Phe Pro Lys Asp
                405                 410                 415
Ala Lys Met Glu Lys Glu Lys Leu Ile Ser Leu Trp Met Ala His Gly
            420                 425                 430
Phe Leu Leu Ser Lys Gly Asn Met Glu Leu Glu Asp Val Gly Asp Glu
        435                 440                 445
Val Trp Lys Glu Leu Tyr Leu Arg Ser Phe Phe Gln Glu Ile Glu Val
    450                 455                 460
Lys Asp Gly Lys Thr Tyr Phe Lys Met His Asp Leu Ile His Asp Leu
465                 470                 475                 480
Ala Thr Ser Leu Phe Ser Ala Asn Thr Ser Ser Ser Asn Ile Arg Glu
                485                 490                 495
Ile Asn Lys His Ser Tyr Thr His Met Met Ser Ile Gly Phe Ala Glu
            500                 505                 510
Val Val Phe Phe Tyr Thr Leu Pro Pro Leu Glu Lys Phe Ile Ser Leu
        515                 520                 525
Arg Val Leu Asn Leu Gly Asp Ser Thr Phe Asn Lys Leu Pro Ser Ser
    530                 535                 540
Ile Gly Asp Leu Val His Leu Arg Tyr Leu Asn Leu Tyr Gly Ser Gly
545                 550                 555                 560
Met Arg Ser Leu Pro Lys Gln Leu Cys Lys Leu Gln Asn Leu Gln Thr
                565                 570                 575
Leu Asp Leu Gln Tyr Cys Thr Lys Leu Cys Cys Leu Pro Lys Glu Thr
            580                 585                 590
Ser Lys Leu Gly Ser Leu Arg Asn Leu Leu Leu Asp Gly Ser Gln Ser
        595                 600                 605
Leu Thr Cys Met Pro Pro Arg Ile Gly Ser Leu Thr Cys Leu Lys Thr
    610                 615                 620

Leu Gly Gln Phe Val Val Gly Arg Lys Lys Gly Tyr Gln Leu Gly Glu
625                 630                 635                 640
Leu Gly Asn Leu Asn Leu Tyr Gly Ser Ile Lys Ile Ser His Leu Glu
                645                 650                 655
Arg Val Lys Asn Asp Arg Asp Ala Lys Glu Ala Asn Leu Ser Ala Lys
            660                 665                 670
Gly Asn Leu His Ser Leu Ser Met Ser Trp Asn Asn Phe Gly Pro His
        675                 680                 685
Ile Tyr Glu Ser Glu Glu Val Lys Val Leu Glu Ala Leu Lys Pro His
    690                 695                 700
Ser Asn Leu Thr Ser Leu Lys Ile Tyr Gly Phe Arg Gly Ile His Leu
705                 710                 715                 720
Pro Glu Trp Met Asn His Ser Val Leu Lys Asn Ile Val Ser Ile Leu
                725                 730                 735
Ile Ser Asn Phe Arg Asn Cys Ser Cys Leu Pro Pro Phe Gly Asp Leu
            740                 745                 750
Pro Cys Leu Glu Ser Leu Glu Leu His Trp Gly Ser Ala Asp Val Glu
        755                 760                 765
Tyr Val Glu Glu Val Asp Ile Asp Val His Ser Gly Phe Pro Thr Arg
    770                 775                 780
Ile Arg Phe Pro Ser Leu Arg Lys Leu Asp Ile Trp Asp Phe Gly Ser
785                 790                 795                 800
Leu Lys Gly Leu Leu Lys Lys Glu Gly Glu Glu Gln Phe Pro Val Leu
                805                 810                 815
Glu Glu Leu Ile Ile His Glu Cys Pro Phe Leu Thr Leu Ser Ser Asn
            820                 825                 830
Leu Arg Ala Leu Thr Ser Leu Arg Ile Cys Tyr Asn Lys Val Ala Thr
        835                 840                 845
Ser Phe Pro Glu Glu Met Phe Lys Asn Leu Ala Asn Leu Lys Tyr Leu
    850                 855                 860
Thr Ile Ser Arg Cys Asn Asn Leu Lys Glu Leu Pro Thr Ser Leu Ala
865                 870                 875                 880
Ser Leu Asn Ala Leu Lys Ser Leu Lys Ile Gln Leu Cys Cys Ala Leu
                885                 890                 895
Glu Ser Leu Pro Glu Glu Gly Leu Glu Gly Leu Ser Ser Leu Thr Glu
            900                 905                 910
Leu Phe Val Glu His Cys Asn Met Leu Lys Cys Leu Pro Glu Gly Leu
        915                 920                 925
Gln His Leu Thr Thr Leu Thr Ser Leu Lys Ile Arg Gly Cys Pro Gln
    930                 935                 940
Leu Ile Lys Arg Cys Glu Lys Gly Ile Gly Glu Asp Trp His Lys Ile
945                 950                 955                 960
Ser His Ile Pro Asn Val Asn Ile Tyr Asn
                965                 970

<210> 71
<211> 3592
<212> DNA
<213> Solanum stoloniferum

<400> 71
atggctgaag ctttcattca agttctgtta gacaatctca cttctttcct caaaggggaa      60

cttacattgc ttttcggttt tcaagatgag ttccaaaggc tttcaagcat gttttctaca     120

atccaagccg tccttgaaga tgctcaggag aagcaactca acaacaagcc tctagaaaat     180

tggttgcaaa aactcaatgc tgctacatac gaagtcgatg acatcttgga tgaatataaa     240

accaaggcca caagattctc ccagtctgaa tatggccgtt atcatccaaa ggttatccct     300

ttccgtcaca aggtcgggaa aaggatggac caagtgatga aaaaactaaa ggcaattgct     360

gaggaaagaa agaattttca tttgcacgaa aaaattgtag agagacaagc tgttagacgg 420

gaaacaggta ctcatcttaa attagtatta caacaactaa gtttatattc atttttttgg 480

caattatcaa attcagaaaa gggttaaata tactcatgtc ctatcgtaaa tagtgtaaat 540

atacctctcg ttgtactttc gatctgaata tacttgtcaa atctggcaag ctcagaatca 600

aattatccac cccaactttt aaatactcga catctttaga aatccacctg tctaactcat 660

ccactaccca ttccctttgc tttgaattct tttctttacc tataaacttg gaacactcga 720

tccgttttgc ttttcttaac aaagcagctc agagaaaaga ggttttcttc tattctgttt 780

ctctgtgtgc tgcacttggg tccttaatcc cattaaaaac agggcatgtt aatcccaacg 840

acggtagcct ttcctgacag ctgactgtaa attttgtcta acaaagaaaa aaaaagatta 900

gacatgtttt tccttgtcat tgattaggct ggatttcttt cagagtggaa cataggggat 960

atattggacc aaaaatagaa tgggtatata tttaaagtat ttctgataga acaggagtat 1020

attgtgcgaa aatatcctct attttctgtt gtctcctaat gagtttgaat gtaataatat 1080

tctcatgtgg acattgcttg caccaggttc tgtattaacc gaaccgcagg tttatggaag 1140

agacaaagag aaagatgaga tagtgaaaat cctaataaac aatgttagtg atgcccaaca 1200

cctttcagtc ctcccaatac ttggtatggg gggattagga aaaacgactc ttgcccaaat 1260

ggtcttcaat gaccagagag ttactgagca tttccattcc aaaatatgga tttgtgtctc 1320

ggaagatttt gatgagaaga ggttaataaa ggcaattgta gaatctattg aaggaaggcc 1380

actacttggt gagatggact tggctccact tcaaaagaag cttcaggagt tgctgaatgg 1440

aaaaagatac ttgcttgtct tagatgatgt ttggaatgaa gatcaacaga agtgggctaa 1500

tttaagagca gtcttgaagg ttggagcaag tggtgcttct gttctaacca ctactcgtct 1560

tgaaaaggtt ggatcaatta tgggaacatt gcaaccatat gaactgtcaa atctgtctca 1620

agaagattgt tggttgttgt tcatgcaacg tgcatttgga caccaagaag aaataaatcc 1680

aaaccttgtg gcaatcggaa aggagattgt gaaaaaaagt ggtggtgtgc ctctagcagc 1740

caaaactctt ggaggtattt tgtgcttcaa gagagaagaa agagcatggg aacatgtgag 1800

agacagtccg atttggaatt tgcctcaaga tgaaagttct attctgcctg ccctgaggct 1860

tagttaccat caacttccac ttgatttgaa acaatgcttt gcgtattgtg cggtgttccc 1920

aaaggatgcc aaaatggaaa aagaaaagct aatctctctc tggatggcgc atggttttct 1980

tttatcaaaa ggaaacatgg agctagagga tgtgggcgat gaagtatgga aagaattata 2040

cttgaggtct tttttccaag agattgaagt taaagatggt aaaacttatt tcaagatgca 2100

tgatctcatc catgatttgg caacatctct gttttcagca aacacatcaa gcagcaatat 2160

ccgtgaaata aataaacaca gttacacaca tatgatgtcc attggtttcg ccgaagtggt 2220

gtttttttac actcttcccc ccttggaaaa gtttatctcg ttaagagtgc ttaatctagg 2280

tgattcgaca tttaataagt taccatcttc cattggagat ctagtacatt taagatactt 2340

gaacctgtat ggcagtggca tgcgtagtct tccaaagcag ttatgcaagc ttcaaaatct 2400

gcaaactctt gatctacaat attgcaccaa gctttgttgt ttgccaaaag aaacaagtaa 2460

acttggtagt ctccgaaatc ttttacttga tggtagccag tcattgactt gtatgccacc 2520

aaggatagga tcattgacat gccttaagac tctaggtcaa tttgttgttg gaaggaagaa 2580

aggttatcaa cttggtgaac taggaaacct aaatctctat ggctcaatta aaatctcgca 2640

tcttgagaga gtgaagaatg atagggacgc aaaagaagcc aatttatctg caaaagggaa 2700

tctgcattct ttaagcatga gttggaataa ctttggacca catatatatg aatcagaaga 2760

agttaaagtg cttgaagccc tcaaaccaca ctccaatctg acttctttaa aaatctatgg 2820

cttcagagga atccatctcc cagagtggat gaatcactca gtattgaaaa atattgtctc 2880

tattctaatt agcaacttca gaaactgctc atgcttacca ccctttggtg atctgccttg 2940

tctagaaagt ctagagttac actgggggtc tgcggatgtg gagtatgttg aagaagtgga 3000

tattgatgtt cattctggat tccccacaag aataaggttt ccatccttga ggaaacttga 3060

tatatgggac tttggtagtc tgaaaggatt gctgaaaaag gaaggagaag agcaattccc 3120

tgtgcttgaa gagctgataa ttcacgagtg cccttttctg accctttctt ctaatcttag 3180

ggctcttact tccctcagaa tttgctataa taaagtagct acttcattcc cagaagagat 3240

gttcaaaaac cttgcaaatc tcaaatactt gacaatctct cggtgcaata atctcaaaga 3300

gctgcctacc agcttggcta gtctgaatgc tttgaaaagt ctaaaaattc aattgtgttg 3360

cgcactagag agtctccctg aggaagggct ggaaggttta tcttcactca cagagttatt 3420

tgttgaacac tgtaacatgc taaaatgttt accagaggga ttgcagcacc taacaaccct 3480

cacaagttta aaaattcggg gatgtccaca actgatcaag cggtgtgaga agggaatagg 3540

agaagactgg cacaaaattt ctcacattcc taatgtgaat atatataatt aa 3592

<210> 72
<211> 2913
<212> DNA
<213> Artificial Sequence

<220>
<223> cDNA

<400> 72
atggctgaag ctttcattca agttctgtta gacaatctca cttctttcct caaaggggaa 60

cttacattgc ttttcggttt tcaagatgag ttccaaaggc tttcaagcat gttttctaca 120

atccaagccg tccttgaaga tgctcaggag aagcaactca acaacaagcc tctagaaaat 180

tggttgcaaa aactcaatgc tgctacatac gaagtcgatg acatcttgga tgaatataaa 240

accaaggcca caagattctc ccagtctgaa tatggccgtt atcatccaaa ggttatccct 300

ttccgtcaca aggtcgggaa aaggatggac caagtgatga aaaaactaaa ggcaattgct 360

gaggaaagaa agaattttca tttgcacgaa aaaattgtag agagacaagc tgttagacgg 420

gaaacaggtt ctgtattaac cgaaccgcag gtttatggaa gagacaaaga gaaagatgag 480

atagtgaaaa tcctaataaa caatgttagt gatgcccaac acctttcagt cctcccaata 540

cttggtatgg ggggattagg aaaaacgact cttgcccaaa tggtcttcaa tgaccagaga 600

gttactgagc atttccattc caaaatatgg atttgtgtct cggaagattt tgatgagaag 660

aggttaataa aggcaattgt agaatctatt gaaggaaggc cactacttgg tgagatggac 720

ttggctccac ttcaaaagaa gcttcaggag ttgctgaatg gaaaaagata cttgcttgtc 780

ttagatgatg tttggaatga agatcaacag aagtgggcaa atttaagagc agtcttgaag 840

gttggagcaa gtggtgcttc tgttctaacc actactcgtc ttgaaaaggt tggatcaatt 900

atgggaacat tgcaaccata tgaactgtca aatctgtctc aagaagattg ttggttgttg 960

ttcatgcaac gtgcatttgg acaccaagaa gaaataaatc caaaccttgt ggcaatcgga 1020

aaggagattg tgaaaaaaag tggtggtgtg cctctagcag ccaaaactct tggaggtatt 1080

ttgtgcttca agagagaaga aagagcatgg gaacatgtga gagacagtcc gatttggaat 1140

ttgcctcaag atgaaagttc tattctgcct gccctgaggc ttagttacca tcaacttcca 1200

cttgatttga aacaatgctt tgcgtattgt gcggtgttcc caaaggatgc caaaatggaa 1260

aaagaaaagc taatctctct ctggatggcg catggttttc ttttatcaaa aggaaacatg 1320

gagctagagg atgtgggtga tgaagtatgg aaagaattat acttgaggtc ttttttccaa 1380

gagattgaag ttaaagatgg taaaacttat ttcaagatgc atgatctcat ccatgatttg 1440

gcaacatctc tgttttcagc aaacacatca agcagcaata tccgtgaaat aaataaacac 1500

agttacacac atatgatgtc cattggtttc gccgaagtgg tgttttttta cactcttccc 1560

cccttggaaa agtttatctc gttaagagtg cttaatctag gtgattcgac atttaataag 1620

ttaccatctt ccattggaga tctagtacat ttaagatact tgaacctgta tggcagtggc 1680

atgcgtagtc ttccaaagca gttatgcaag cttcaaaatc tgcaaactct tgatctacaa 1740

tattgcacca agctttgttg tttgccaaaa gaaacaagta aacttggtag tctccgaaat 1800

cttttacttg atggtagcca gtcattgact tgtatgccac caaggatagg atcattgaca 1860

tgccttaaga ctctaggtca atttgttgtt ggaaggaaga aaggttatca acttggtgaa 1920

ctaggaaacc taaatctcta tggctcaatt aaaatctcgc atcttgagag agtgaagaat 1980

gataaggacg caaaagaagc caatttatct gcaaaaggga atctgcattc tttaagcatg 2040

agttggaata actttggacc acatatatat gaatcagaag aagttaaagt gcttgaagcc 2100

ctcaaaccac actccaatct gacttcttta aaaatctatg gcttcagagg aatccatctc 2160

ccagagtgga tgaatcactc agtattgaaa aatattgtct ctattctaat tagcaacttc 2220

agaaactgct catgcttacc accctttggt gatctgcctt gtctagaaag tctagagtta 2280

cactgggggt ctgcggatgt ggagtatgtt gaagaagtgg atattgatgt tcattctgga 2340

ttccccacaa gaataaggtt tccatccttg aggaaacttg atatatggga ctttggtagt 2400

ctgaaaggat tgctgaaaaa ggaaggagaa gagcaattcc ctgtgcttga agagatgata 2460

attcacgagt gcccttttct gaccctttct tctaatctta gggctcttac ttccctcaga 2520

atttgctata ataaagtagc tacttcattc ccagaagaga tgttcaaaaa ccttgcaaat 2580

ctcaaatact tgacaatctc tcggtgcaat aatctcaaag agctgcctac cagcttggct 2640

agtctgaatg ctttgaaaag tctaaaaatt caattgtgtt gcgcactaga gagtctccct 2700

gaggaagggc tggaaggttt atcttcactc acagagttat ttgttgaaca ctgtaacatg 2760

ctaaaatgtt taccagaggg attgcagcac ctaacaaccc tcacaagttt aaaaattcgg 2820

ggatgtccac aactgatcaa gcggtgtgag aagggaatag gagaagactg gcacaaaatt 2880

tctcacattc ctaatgtgaa tatatataat taa 2913

<210> 73
<211> 970
<212> PRT
<213> Solanum stoloniferum

<400> 73
Met Ala Glu Ala Phe Ile Gln Val Leu Leu Asp Asn Leu Thr Ser Phe
1               5                   10                  15
Leu Lys Gly Glu Leu Thr Leu Leu Phe Gly Phe Gln Asp Glu Phe Gln
            20                  25                  30
Arg Leu Ser Ser Met Phe Ser Thr Ile Gln Ala Val Leu Glu Asp Ala
        35                  40                  45
Gln Glu Lys Gln Leu Asn Asn Lys Pro Leu Glu Asn Trp Leu Gln Lys
    50                  55                  60
Leu Asn Ala Ala Thr Tyr Glu Val Asp Asp Ile Leu Asp Glu Tyr Lys
65                  70                  75                  80
Thr Lys Ala Thr Arg Phe Ser Gln Ser Glu Tyr Gly Arg Tyr His Pro
                85                  90                  95
Lys Val Ile Pro Phe Arg His Lys Val Gly Lys Arg Met Asp Gln Val
            100                 105                 110
Met Lys Lys Leu Lys Ala Ile Ala Glu Glu Arg Lys Asn Phe His Leu
        115                 120                 125
His Glu Lys Ile Val Glu Arg Gln Ala Val Arg Arg Glu Thr Gly Ser
    130                 135                 140
Val Leu Thr Glu Pro Gln Val Tyr Gly Arg Asp Lys Glu Lys Asp Glu
145                 150                 155                 160
Ile Val Lys Ile Leu Ile Asn Asn Val Ser Asp Ala Gln His Leu Ser
                165                 170                 175
Val Leu Pro Ile Leu Gly Met Gly Gly Leu Gly Lys Thr Thr Leu Ala
            180                 185                 190
Gln Met Val Phe Asn Asp Gln Arg Val Thr Glu His Phe His Ser Lys
        195                 200                 205

Ile Trp Ile Cys Val Ser Glu Asp Phe Asp Glu Lys Arg Leu Ile Lys
    210                 215                 220
Ala Ile Val Glu Ser Ile Glu Gly Arg Pro Leu Leu Gly Glu Met Asp
225                 230                 235                 240
Leu Ala Pro Leu Gln Lys Lys Leu Gln Glu Leu Leu Asn Gly Lys Arg
                245                 250                 255
Tyr Leu Leu Val Leu Asp Asp Val Trp Asn Glu Asp Gln Gln Lys Trp
            260                 265                 270
Ala Asn Leu Arg Ala Val Leu Lys Val Gly Ala Ser Gly Ala Ser Val
        275                 280                 285
Leu Thr Thr Thr Arg Leu Glu Lys Val Gly Ser Ile Met Gly Thr Leu
    290                 295                 300
Gln Pro Tyr Glu Leu Ser Asn Leu Ser Gln Glu Asp Cys Trp Leu Leu
305                 310                 315                 320
Phe Met Gln Arg Ala Phe Gly His Gln Glu Glu Ile Asn Pro Asn Leu
                325                 330                 335
Val Ala Ile Gly Lys Glu Ile Val Lys Lys Ser Gly Gly Val Pro Leu
            340                 345                 350
Ala Ala Lys Thr Leu Gly Gly Ile Leu Cys Phe Lys Arg Glu Glu Arg
        355                 360                 365
Ala Trp Glu His Val Arg Asp Ser Pro Ile Trp Asn Leu Pro Gln Asp
    370                 375                 380
Glu Ser Ser Ile Leu Pro Ala Leu Arg Leu Ser Tyr His Gln Leu Pro
385                 390                 395                 400
Leu Asp Leu Lys Gln Cys Phe Ala Tyr Cys Ala Val Phe Pro Lys Asp
                405                 410                 415
Ala Lys Met Glu Lys Glu Lys Leu Ile Ser Leu Trp Met Ala His Gly
            420                 425                 430
Phe Leu Leu Ser Lys Gly Asn Met Glu Leu Glu Asp Val Gly Asp Glu
        435                 440                 445
Val Trp Lys Glu Leu Tyr Leu Arg Ser Phe Phe Gln Glu Ile Glu Val
    450                 455                 460
Lys Asp Gly Lys Thr Tyr Phe Lys Met His Asp Leu Ile His Asp Leu
465                 470                 475                 480
Ala Thr Ser Leu Phe Ser Ala Asn Thr Ser Ser Ser Asn Ile Arg Glu
                485                 490                 495
Ile Asn Lys His Ser Tyr Thr His Met Met Ser Ile Gly Phe Ala Glu
            500                 505                 510
Val Val Phe Phe Tyr Thr Leu Pro Pro Leu Glu Lys Phe Ile Ser Leu
        515                 520                 525
Arg Val Leu Asn Leu Gly Asp Ser Thr Phe Asn Lys Leu Pro Ser Ser
    530                 535                 540
Ile Gly Asp Leu Val His Leu Arg Tyr Leu Asn Leu Tyr Gly Ser Gly
545                 550                 555                 560
Met Arg Ser Leu Pro Lys Gln Leu Cys Lys Leu Gln Asn Leu Gln Thr
                565                 570                 575
Leu Asp Leu Gln Tyr Cys Thr Lys Leu Cys Cys Leu Pro Lys Glu Thr
            580                 585                 590
Ser Lys Leu Gly Ser Leu Arg Asn Leu Leu Leu Asp Gly Ser Gln Ser
        595                 600                 605
Leu Thr Cys Met Pro Pro Arg Ile Gly Ser Leu Thr Cys Leu Lys Thr
    610                 615                 620
Leu Gly Gln Phe Val Val Gly Arg Lys Lys Gly Tyr Gln Leu Gly Glu
625                 630                 635                 640
Leu Gly Asn Leu Asn Leu Tyr Gly Ser Ile Lys Ile Ser His Leu Glu
                645                 650                 655
Arg Val Lys Asn Asp Lys Asp Ala Lys Glu Ala Asn Leu Ser Ala Lys
            660                 665                 670
Gly Asn Leu His Ser Leu Ser Met Ser Trp Asn Asn Phe Gly Pro His
        675                 680                 685
Ile Tyr Glu Ser Glu Glu Val Lys Val Leu Glu Ala Leu Lys Pro His
    690                 695                 700
Ser Asn Leu Thr Ser Leu Lys Ile Tyr Gly Phe Arg Gly Ile His Leu

705                 710                 715                 720
Pro Glu Trp Met Asn His Ser Val Leu Lys Asn Ile Val Ser Ile Leu
                725                 730                 735
Ile Ser Asn Phe Arg Asn Cys Ser Cys Leu Pro Pro Phe Gly Asp Leu
            740                 745                 750
Pro Cys Leu Glu Ser Leu Glu Leu His Trp Gly Ser Ala Asp Val Glu
        755                 760                 765
Tyr Val Glu Glu Val Asp Ile Asp Val His Ser Gly Phe Pro Thr Arg
    770                 775                 780
Ile Arg Phe Pro Ser Leu Arg Lys Leu Asp Ile Trp Asp Phe Gly Ser
785                 790                 795                 800
Leu Lys Gly Leu Leu Lys Lys Glu Gly Glu Glu Gln Phe Pro Val Leu
                805                 810                 815
Glu Glu Met Ile Ile His Glu Cys Pro Phe Leu Thr Leu Ser Ser Asn
            820                 825                 830
Leu Arg Ala Leu Thr Ser Leu Arg Ile Cys Tyr Asn Lys Val Ala Thr
        835                 840                 845
Ser Phe Pro Glu Glu Met Phe Lys Asn Leu Ala Asn Leu Lys Tyr Leu
    850                 855                 860
Thr Ile Ser Arg Cys Asn Asn Leu Lys Glu Leu Pro Thr Ser Leu Ala
865                 870                 875                 880
Ser Leu Asn Ala Leu Lys Ser Leu Lys Ile Gln Leu Cys Cys Ala Leu
                885                 890                 895
Glu Ser Leu Pro Glu Glu Gly Leu Glu Gly Leu Ser Ser Leu Thr Glu
            900                 905                 910
Leu Phe Val Glu His Cys Asn Met Leu Lys Cys Leu Pro Glu Gly Leu
        915                 920                 925
Gln His Leu Thr Thr Leu Thr Ser Leu Lys Ile Arg Gly Cys Pro Gln
    930                 935                 940
Leu Ile Lys Arg Cys Glu Lys Gly Ile Gly Glu Asp Trp His Lys Ile
945                 950                 955                 960
Ser His Ile Pro Asn Val Asn Ile Tyr Asn
                965                 970

<210> 74
<211> 3592
<212> DNA
<213> Solanum stoloniferum

<400> 74
   atggctgaag ctttcattca agttctgtta gacaatctca cttctttcct caaaggggaa      60
   cttacattgc ttttcggttt tcaagatgag ttccaaaggc tttcaagcat gttttctaca     120
   atccaagccg tccttgaaga tgctcaggag aagcaactca acaacaagcc tctagaaaat     180
   tggttgcaaa aactcaatgc tgctacatac gaagtcgatg acatcttgga tgaatataaa     240
   accaaggcca caagattctc ccagtctgaa tatggccgtt atcatccaaa ggttatccct     300
   ttccgtcaca aggtcgggaa aaggatggac caagtgatga aaaaactaaa ggcaattgct     360
   gaggaaagaa agaattttca tttgcacgaa aaaattgtag agagacaagc tgttagacgg     420
   gaaacaggta ctcatcttaa attagtatta caacaactaa gtttatattc atttttttgg     480
   caattatcaa attcagaaaa gggttaaata tactcatgtc ctatcgtaaa tagtgtaaat     540
   atacctctcg ttgtactttc gatctgaata tacttgtcaa atctggcaag ctcagaatca     600
   aattatccac cccaactttt aaatactcga catctttaga aatccacctg tctaactcat     660
   ccactaccca ttccctttgc tttgaattct tttctttacc tataaacttg gaacactcga 720
   tccgttttgc ttttcttaac aaagcagctc agagaaaaga ggttttcttc tattctgttt 780
   ctctgtgtgc tgcacttggg tccttaatcc cattaaaaac agggcatgtt aatcccaacg 840
   acggtagcct ttcctgacag ctgactgtaa attttgtcta acaaagaaaa aaaaagatta 900
   gacatgtttt tccttgtcat tgattaggct ggatttcttt cagagtggaa cataggggat 960
   atattggacc aaaaatagaa tgggtatata tttaaagtat ttctgataga acaggagtat 1020
   attgtgcgaa aatatcctct attttctgtt gtctcctaat gagtttgaat gtaataatat 1080
   tctcatgtgg acattgcttg caccaggttc tgtattaacc gaaccgcagg tttatggaag 1140
   agacaaagag aaagatgaga tagtgaaaat cctaataaac aatgttagtg atgcccaaca 1200
   cctttcagtc ctcccaatac ttggtatggg gggattagga aaaacgactc ttgcccaaat 1260
   ggtcttcaat gaccagagag ttactgagca tttccattcc aaaatatgga tttgtgtctc 1320
   ggaagatttt gatgagaaga ggttaataaa ggcaattgta gaatctattg aaggaaggcc 1380
   actacttggt gagatggact tggctccact tcaaaagaag cttcaggagt tgctgaatgg 1440
   aaaaagatac ttgcttgtct tagatgatgt ttggaatgaa gatcaacaga agtgggcaaa 1500
   tttaagagca gtcttgaagg ttggagcaag tggtgcttct gttctaacca ctactcgtct 1560
   tgaaaaggtt ggatcaatta tgggaacatt gcaaccatat gaactgtcaa atctgtctca 1620
   agaagattgt tggttgttgt tcatgcaacg tgcatttgga caccaagaag aaataaatcc 1680
   aaaccttgtg gcaatcggaa aggagattgt gaaaaaaagt ggtggtgtgc ctctagcagc 1740
   caaaactctt ggaggtattt tgtgcttcaa gagagaagaa agagcatggg aacatgtgag 1800
   agacagtccg atttggaatt tgcctcaaga tgaaagttct attctgcctg ccctgaggct 1860
   tagttaccat caacttccac ttgatttgaa acaatgcttt gcgtattgtg cggtgttccc 1920
   aaaggatgcc aaaatggaaa aagaaaagct aatctctctc tggatggcgc atggttttct 1980
   tttatcaaaa ggaaacatgg agctagagga tgtgggtgat gaagtatgga aagaattata 2040
   cttgaggtct tttttccaag agattgaagt taaagatggt aaaacttatt tcaagatgca 2100
   tgatctcatc catgatttgg caacatctct gttttcagca aacacatcaa gcagcaatat 2160
   ccgtgaaata aataaacaca gttacacaca tatgatgtcc attggtttcg ccgaagtggt 2220
   gtttttttac actcttcccc ccttggaaaa gtttatctcg ttaagagtgc ttaatctagg 2280
   tgattcgaca tttaataagt taccatcttc cattggagat ctagtacatt taagatactt 2340
   gaacctgtat ggcagtggca tgcgtagtct tccaaagcag ttatgcaagc ttcaaaatct 2400
   gcaaactctt gatctacaat attgcaccaa gctttgttgt ttgccaaaag aaacaagtaa 2460
   acttggtagt ctccgaaatc ttttacttga tggtagccag tcattgactt gtatgccacc 2520
   aaggatagga tcattgacat gccttaagac tctaggtcaa tttgttgttg gaaggaagaa 2580
   aggttatcaa cttggtgaac taggaaacct aaatctctat ggctcaatta aaatctcgca 2640
   tcttgagaga gtgaagaatg ataaggacgc aaaagaagcc aatttatctg caaaagggaa 2700
   tctgcattct ttaagcatga gttggaataa ctttggacca catatatatg aatcagaaga 2760
   agttaaagtg cttgaagccc tcaaaccaca ctccaatctg acttctttaa aaatctatgg 2820
   cttcagagga atccatctcc cagagtggat gaatcactca gtattgaaaa atattgtctc 2880
   tattctaatt agcaacttca gaaactgctc atgcttacca ccctttggtg atctgccttg 2940
   tctagaaagt ctagagttac actgggggtc tgcggatgtg gagtatgttg aagaagtgga 3000
   tattgatgtt cattctggat tccccacaag aataaggttt ccatccttga ggaaacttga 3060
   tatatgggac tttggtagtc tgaaaggatt gctgaaaaag gaaggagaag agcaattccc 3120
   tgtgcttgaa gagatgataa ttcacgagtg cccttttctg accctttctt ctaatcttag 3180
   ggctcttact tccctcagaa tttgctataa taaagtagct acttcattcc cagaagagat 3240
   gttcaaaaac cttgcaaatc tcaaatactt gacaatctct cggtgcaata atctcaaaga 3300
   gctgcctacc agcttggcta gtctgaatgc tttgaaaagt ctaaaaattc aattgtgttg 3360
   cgcactagag agtctccctg aggaagggct ggaaggttta tcttcactca cagagttatt 3420
   tgttgaacac tgtaacatgc taaaatgttt accagaggga ttgcagcacc taacaaccct 3480
   cacaagttta aaaattcggg gatgtccaca actgatcaag cggtgtgaga agggaatagg 3540
   agaagactgg cacaaaattt ctcacattcc taatgtgaat atatataatt aa 3592

<210> 75
<211> 2676
<212> DNA
<213> Artificial Sequence

<220>
<223> cDNA

<400> 75
atgaattatt gtgtttacaa gacttgggcc gttgactctt actttccctt cctcatcctc 60

acatttagaa aaaagaaatt taacgaaaaa ttaaaggaga tggctgaaat tcttctcaca 120

gcagtcatca ataaatcaat agaaatagct ggaaatgtac tctttcaaga aggtacgcgt 180

ttatattggt tgaaagagga catcgattgg ctccagagag aaatgagaca cattcgatca 240

tatgtagaca atgcaaaggc aaaggaagtt ggaggcgatt caagggtgaa aaacttatta 300

aaagatattc aacaactggc aggtgatgtg gaggatctat tagatgagtt tcttccaaaa 360

attcaacaat ccaataagtt catttgttgc cttaagacgg tttcttttgc cgatgagttt 420

gctatggaga ttgagaagat aaaaagaaga gttgctgata ttgaccgtgt aaggacaact 480

tacagcatca cagatacaag taacaataat gatgattgca ttccattgga ccggagaaga 540

ttgttccttc atgctgatga aacagaggtc atcggtctgg aagatgactt caatacacta 600

caagccaaat tacttgatca tgatttgcct tatggagttg tttcaatagt tggcatgccc 660

ggtttgggaa aaacaactct tgccaagaaa ctttataggc atgtctgtca tcaatttgag 720

tgttcgggac tggtctatgt ttcacaacag ccaagggcgg gagaaatctt acatgacata 780

gccaaacaag ttggactgac ggaagaggaa aggaaagaaa acttggagaa caacctacga 840

tcactcttga aaataaaaag gtatgttatt ctcttagatg acatttggga tgttgaaatt 900

tgggatgatc taaaacttgt ccttcctgaa tgtgattcaa aaattggcag taggataatt 960

ataacctctc gaaatagtaa tgtaggcaga tacataggag gggatttctc aatccacgtg 1020

ttgcaacccc tagattcaga gaaaagcttt gaactcttta ccaagaaaat ctttaatttt 1080

gttaatgata attgggccaa tgcttcacca gacttggtaa atattggtag atgtatagtt 1140

gagagatgtg gaggtatacc gctagcaatt gtggtgactg caggcatgtt aagggcaaga 1200

ggaagaacag aacatgcatg gaacagagta cttgagagta tggctcataa aattcaagat 1260

ggatgtggta aggtattggc tctgagttac aatgatttgc ccattgcatt aaggccatgt 1320

ttcttgtact ttggtcttta ccccgaggac catgaaattc gtgcttttga tttgacaaat 1380

atgtggattg ctgagaagct gatagttgta aatactggca atgggcgaga ggctgaaagt 1440

ttggcggatg atgtcctaaa tgatttggtt tcaagaaact tgattcaagt tgccaaaagg 1500

acatatgatg gaagaatttc aagttgtcgc atacatgact tgttacatag tttgtgtgtg 1560

gacttggcta aggaaagtaa cttctttcac acggagcaca atgcatttgg tgatcctagc 1620

aatgttgcta gggtgcgaag gattacattc tactctgatg ataatgccat gaatgagttc 1680

ttccatttaa atcctaagcc tatgaagctt cgttcacttt tctgtttcac aaaagaccgt 1740

tgcatatttt ctcaaatggc tcatcttaac ttcaaattat tgcaagtgtt ggttgtagtc 1800

atgtctcaaa agggttatca gcatgttact ttccccaaaa aaattgggaa catgagttgc 1860

ctacgttatg tgcgattgga gggggcaatt agagtaaaat tgccaaatag tattgtcaag 1920

ctcaaatgtc tagagaccct ggatatattt catagctcta gtaaacttcc ttttggtgtt 1980

tgggagtcta aaatattgag acatctttgt tacacagaag aatgttactg tgtctctttt 2040

gcaagtccat tttgccgaat catgcctcct aataatctac aaactttgat gtgggtggat 2100

gataaatttt gtgaaccaag attgttgcac cgattgataa atttaagaac attgtgtata 2160

atggatgtat ccggttctac cattaagata ttatcagcat tgagccctgt gcctagagcg 2220

ttggaggttc tgaagctcag atttttcaag aacacgagtg agcaaataaa cttgtcgtcc 2280

catccaaata ttgtcgagtt gggtttggtt ggtttctcag caatgctctt gaacattgaa 2340

gcattccctc caaatcttgt caagcttaat cttgtcggct tgatggtaga cggtcatcta 2400

ttggcagtgc ttaagaaatt gcccaaatta aggatactta tattgctttg gtgcagacat 2460

gatgcagaaa aaatggatct ctctggtgat agctttccgc aacttgaagt tttgtatatt 2520

gaggatgcac aagggttgtc tgaagtaacg tgcatggatg atatgagtat gcctaaattg 2580

aaaaagctat ttcttgtaca aggcccaaac atttccccaa ttagtctcag ggtctcggaa 2640

cggcttgcaa agttgagaat atcacaggta ctataa 2676

<210> 76
<211> 891
<212> PRT
<213> Solanum venturii

<400> 76
Met Asn Tyr Cys Val Tyr Lys Thr Trp Ala Val Asp Ser Tyr Phe Pro
1               5                   10                  15
Phe Leu Ile Leu Thr Phe Arg Lys Lys Lys Phe Asn Glu Lys Leu Lys
            20                  25                  30
Glu Met Ala Glu Ile Leu Leu Thr Ala Val Ile Asn Lys Ser Ile Glu
        35                  40                  45
Ile Ala Gly Asn Val Leu Phe Gln Glu Gly Thr Arg Leu Tyr Trp Leu
    50                  55                  60
Lys Glu Asp Ile Asp Trp Leu Gln Arg Glu Met Arg His Ile Arg Ser
65                  70                  75                  80
Tyr Val Asp Asn Ala Lys Ala Lys Glu Val Gly Gly Asp Ser Arg Val
                85                  90                  95
Lys Asn Leu Leu Lys Asp Ile Gln Gln Leu Ala Gly Asp Val Glu Asp
            100                 105                 110
Leu Leu Asp Glu Phe Leu Pro Lys Ile Gln Gln Ser Asn Lys Phe Ile
        115                 120                 125
Cys Cys Leu Lys Thr Val Ser Phe Ala Asp Glu Phe Ala Met Glu Ile
    130                 135                 140
Glu Lys Ile Lys Arg Arg Val Ala Asp Ile Asp Arg Val Arg Thr Thr
145                 150                 155                 160
Tyr Ser Ile Thr Asp Thr Ser Asn Asn Asn Asp Asp Cys Ile Pro Leu
                165                 170                 175
Asp Arg Arg Arg Leu Phe Leu His Ala Asp Glu Thr Glu Val Ile Gly
            180                 185                 190
Leu Glu Asp Asp Phe Asn Thr Leu Gln Ala Lys Leu Leu Asp His Asp
        195                 200                 205
Leu Pro Tyr Gly Val Val Ser Ile Val Gly Met Pro Gly Leu Gly Lys
    210                 215                 220
Thr Thr Leu Ala Lys Lys Leu Tyr Arg His Val Cys His Gln Phe Glu
225                 230                 235                 240
Cys Ser Gly Leu Val Tyr Val Ser Gln Gln Pro Arg Ala Gly Glu Ile
                245                 250                 255
Leu His Asp Ile Ala Lys Gln Val Gly Leu Thr Glu Glu Glu Arg Lys
            260                 265                 270
Glu Asn Leu Glu Asn Asn Leu Arg Ser Leu Leu Lys Ile Lys Arg Tyr
        275                 280                 285
Val Ile Leu Leu Asp Asp Ile Trp Asp Val Glu Ile Trp Asp Asp Leu
    290                 295                 300
Lys Leu Val Leu Pro Glu Cys Asp Ser Lys Ile Gly Ser Arg Ile Ile
305                 310                 315                 320
Ile Thr Ser Arg Asn Ser Asn Val Gly Arg Tyr Ile Gly Gly Asp Phe
                325                 330                 335
Ser Ile His Val Leu Gln Pro Leu Asp Ser Glu Lys Ser Phe Glu Leu
            340                 345                 350
Phe Thr Lys Lys Ile Phe Asn Phe Val Asn Asp Asn Trp Ala Asn Ala

355                 360                 365
Ser Pro Asp Leu Val Asn Ile Gly Arg Cys Ile Val Glu Arg Cys Gly
    370                 375                 380
Gly Ile Pro Leu Ala Ile Val Val Thr Ala Gly Met Leu Arg Ala Arg
385                 390                 395                 400
Gly Arg Thr Glu His Ala Trp Asn Arg Val Leu Glu Ser Met Ala His
                405                 410                 415
Lys Ile Gln Asp Gly Cys Gly Lys Val Leu Ala Leu Ser Tyr Asn Asp
            420                 425                 430
Leu Pro Ile Ala Leu Arg Pro Cys Phe Leu Tyr Phe Gly Leu Tyr Pro
        435                 440                 445
Glu Asp His Glu Ile Arg Ala Phe Asp Leu Thr Asn Met Trp Ile Ala
    450                 455                 460
Glu Lys Leu Ile Val Val Asn Thr Gly Asn Gly Arg Glu Ala Glu Ser
465                 470                 475                 480
Leu Ala Asp Asp Val Leu Asn Asp Leu Val Ser Arg Asn Leu Ile Gln
                485                 490                 495
Val Ala Lys Arg Thr Tyr Asp Gly Arg Ile Ser Ser Cys Arg Ile His
            500                 505                 510
Asp Leu Leu His Ser Leu Cys Val Asp Leu Ala Lys Glu Ser Asn Phe
        515                 520                 525
Phe His Thr Glu His Asn Ala Phe Gly Asp Pro Ser Asn Val Ala Arg
    530                 535                 540
Val Arg Arg Ile Thr Phe Tyr Ser Asp Asp Asn Ala Met Asn Glu Phe
545                 550                 555                 560
Phe His Leu Asn Pro Lys Pro Met Lys Leu Arg Ser Leu Phe Cys Phe
                565                 570                 575
Thr Lys Asp Arg Cys Ile Phe Ser Gln Met Ala His Leu Asn Phe Lys
            580                 585                 590
Leu Leu Gln Val Leu Val Val Val Met Ser Gln Lys Gly Tyr Gln His
        595                 600                 605
Val Thr Phe Pro Lys Lys Ile Gly Asn Met Ser Cys Leu Arg Tyr Val
    610                 615                 620
Arg Leu Glu Gly Ala Ile Arg Val Lys Leu Pro Asn Ser Ile Val Lys
625                 630                 635                 640
Leu Lys Cys Leu Glu Thr Leu Asp Ile Phe His Ser Ser Ser Lys Leu
                645                 650                 655
Pro Phe Gly Val Trp Glu Ser Lys Ile Leu Arg His Leu Cys Tyr Thr
            660                 665                 670
Glu Glu Cys Tyr Cys Val Ser Phe Ala Ser Pro Phe Cys Arg Ile Met
        675                 680                 685
Pro Pro Asn Asn Leu Gln Thr Leu Met Trp Val Asp Asp Lys Phe Cys
    690                 695                 700
Glu Pro Arg Leu Leu His Arg Leu Ile Asn Leu Arg Thr Leu Cys Ile
705                 710                 715                 720
Met Asp Val Ser Gly Ser Thr Ile Lys Ile Leu Ser Ala Leu Ser Pro
                725                 730                 735
Val Pro Arg Ala Leu Glu Val Leu Lys Leu Arg Phe Phe Lys Asn Thr
            740                 745                 750
Ser Glu Gln Ile Asn Leu Ser Ser His Pro Asn Ile Val Glu Leu Gly
        755                 760                 765
Leu Val Gly Phe Ser Ala Met Leu Leu Asn Ile Glu Ala Phe Pro Pro
    770                 775                 780
Asn Leu Val Lys Leu Asn Leu Val Gly Leu Met Val Asp Gly His Leu
785                 790                 795                 800
Leu Ala Val Leu Lys Lys Leu Pro Lys Leu Arg Ile Leu Ile Leu Leu
                805                 810                 815
Trp Cys Arg His Asp Ala Glu Lys Met Asp Leu Ser Gly Asp Ser Phe
            820                 825                 830
Pro Gln Leu Glu Val Leu Tyr Ile Glu Asp Ala Gln Gly Leu Ser Glu
        835                 840                 845
Val Thr Cys Met Asp Asp Met Ser Met Pro Lys Leu Lys Lys Leu Phe
    850                 855                 860

Leu Val Gln Gly Pro Asn Ile Ser Pro Ile Ser Leu Arg Val Ser Glu
865                 870                 875                 880
Arg Leu Ala Lys Leu Arg Ile Ser Gln Val Leu
                885                 890

<210> 77
<211> 462
<212> DNA
<213> Artificial Sequence

<220>
<223> cDNA

<400> 77
atgcgagtgt acgccgcctt cgtcgtggcg ctggtctccc ttctagctac caacagtgct      60

gttctagcag taacgacccc gaccaagtta gcgaagttga cggctcctga attcacagac     120

aacgagaatc gtcagcgtcg cttgctaaga aaacaagaag gtgccgagct gggtgacgaa     180

gaacgaacga gcacccaaaa ccttgggaac tctttgatga gggtgttctc gaaagaggcc     240

acaaggaaat actatttgga tttgttcaag cgagccgact ttacagcgaa tctcccaaaa     300

cttgcgaaaa aaggtgggcc tgacaggctg aacgatgcct taaaaaaact acgaaaggct     360

ggcatctctg aggagaagtt tgcggagctc aaaggtgcgg cggcgaagta cgcagatgac     420

tggtatagaa tatatggcaa gcttgatcct attagagctt ga                        462

<210> 78
<211> 153
<212> PRT
<213> Phytophthora infestans

<400> 78
Met Arg Val Tyr Ala Ala Phe Val Val Ala Leu Val Ser Leu Leu Ala
1               5                   10                  15
Thr Asn Ser Ala Val Leu Ala Val Thr Thr Pro Thr Lys Leu Ala Lys
            20                  25                  30
Leu Thr Ala Pro Glu Phe Thr Asp Asn Glu Asn Arg Gln Arg Arg Leu
        35                  40                  45
Leu Arg Lys Gln Glu Gly Ala Glu Leu Gly Asp Glu Glu Arg Thr Ser
    50                  55                  60
Thr Gln Asn Leu Gly Asn Ser Leu Met Arg Val Phe Ser Lys Glu Ala
65                  70                  75                  80
Thr Arg Lys Tyr Tyr Leu Asp Leu Phe Lys Arg Ala Asp Phe Thr Ala
                85                  90                  95
Asn Leu Pro Lys Leu Ala Lys Lys Gly Gly Pro Asp Arg Leu Asn Asp
            100                 105                 110
Ala Leu Lys Lys Leu Arg Lys Ala Gly Ile Ser Glu Glu Lys Phe Ala
        115                 120                 125
Glu Leu Lys Gly Ala Ala Ala Lys Tyr Ala Asp Asp Trp Tyr Arg Ile
    130                 135                 140
Tyr Gly Lys Leu Asp Pro Ile Arg Ala
145                 150

<210> 79
<211> 2592
<212> DNA

<213> Artificial Sequence

<220>
<223> cDNA

<400> 79
atggctgaaa ttcttcttac agcagtcatc aataaatctg tagaaatagc tgcaaatgta 60

ctctttcaac aagggagccg cttgaatttt ttgaaagagg acatcgattg gctccagaga 120

gtactgagac acattcgatc atatgtagac gatgcaaagg ccaaggaagt tggaggcgat 180

tcaagggtca aaaacttatt aaaagatatt caagaattgg caggtgatgt ggaggatctc 240

ttagatgagt ttcttccaaa aatccaacaa tccagtaagt tcaaaggcgc aatttgttgc 300

cttaagacgg tttcttttgc cgatgagttt gctgtggaga ttgagaagat aagaagaagg 360

gttgctgaca ttgatagttt aaggacaact ttcaacatca cagatacaag taacaacaat 420

aatgattgca ttccaatgga acagagaaga aaattccttc atgctgatga aacagaggtc 480

atcggtttgg atgatgactt caacaagctc caagacaaat tgcttgttca agatttgtgt 540

aatggagttg tttcaatagt tggcatgcct ggtctaggaa aaacaactct tgccaagaaa 600

ctttataggc atgtccgtca tcaatttgag tgttctgcac tggtctacgt ttcacaacag 660

ccaagagcag gagaaatctt acttgacata gccaagcaag ttggactgac ggacgaggga 720

aggaaagaac acttggagga caatctaaga tcacttttgg aaacaaaaag gtatgttatt 780

ctcttagatg acatttggga tactaaaatc tgggatgctc tgaaccgtgt ccttcgtcct 840

gaatgtgatt caaaaattgg cagtaggata attatcactt ctcgatatca tcatgtaggc 900

agatacatag gagaggattt ctcgctccac gagttgcaac ccttagattc agagaaaagt 960

tttgaactct ttaccaagaa aatctttatt tttgataata ataataattg ggctaatgct 1020

tcacctgtct tggtagatat tggtaaaagt atagttcgga gatgtggagg tattccatta 1080

gccattgtgg tgacggcagg catgttaagg gcaagagaaa gaacggaaca tgcatggaat 1140

agagtgcttg agcgtatagg tcataatatt caggatggat gtgctaaggc attggctctg 1200

agttacaatg atttgcccat tgcattaagg ccatgtttct tgtactttgg tctttacccc 1260

gaggaccatg aaattcgtgc ttttgatttg acaaatatgt ggattgctga gaagctgata 1320

gttgtaaata gtggcaatag gcgagaggct gaaagtttgg cggatgatgt cctaaatgat 1380

ttggtttcaa gaaacttgat tcaagttgcc aagaggacat atgatggagg aatttcaagt 1440

tgtcgcatac atgacttgtt acatagtttg tgtgttgact tggctaagga aagcaacttc 1500

tttcacaccg agcacaatgc atttggtgat cccggcaatg tttctaggct gcgaaggatt 1560

acattctact ctgataataa tgccatgaat gagttcttcc gttcaaatcc taagcttgag 1620

aagcttcgtg cacttttctg tttcacaaaa ggagactctt gcatattttc tcatttggct 1680

catcatgact tcaaattatt acaagtgttg gttgtagtcc agcctcgaaa aaattatgat 1740

ttcagcatta gccaaatcaa aattgggaac atgagttgct tacgctatct gcgattcgag 1800

ggggatattt atggggaact gccaaattgt atggtgaagc tcaaacactt agagacccta 1860

gatattagta aaagcttcat tattaaactt cctactggtg tttggaagac tacacaattg 1920

agacatcttc gttctaatgg ttataatcta gcaccttaca gttacttttg tataagccca 1980

ttttttccaa acgtgcctcc taataatgta caaactttga tgtggatgga tggtgaattt 2040

tttgaaccga gatggttgca ccgatttatc aatttaagaa aactgggttt acaggaagta 2100

tccgattcta ccattaagaa attatcaaca ttgagccctg tgccaacgac actggaggtt 2160

ctaaagctca gctcattttt cagtgaattg agagagcaaa taaacttgtc gtcgtatcca 2220

aatattgtta agttgcattt gaacggaaga attcccttga acgtctctga atcattccct 2280

ccaaatcttg tcaagcttac tctttgcaac ttgatggtag acggtcatgt agtggcagtg 2340

cttaagaaat tacccaaatt aaagatactt acattgcata ggtgcagaca tgatgcagaa 2400

aaaatggatc tctctggtga tggtgatagc tttccgcaac ttgaagtttt gcatattaaa 2460

gatccagtct gcttgtctga agtaacgtgc acggatgatg tcggtatgcc taaattgaaa 2520

aagttattac ttatagaaag aactgattcc aacgttaggc tctcggaaag acttgcaaaa 2580

ctgagagtat ga 2592

<210> 80
<211> 863
<212> PRT
<213> solanum edinense

<400> 80
Met Ala Glu Ile Leu Leu Thr Ala Val Ile Asn Lys Ser Val Glu Ile
1               5                   10                  15
Ala Ala Asn Val Leu Phe Gln Gln Gly Ser Arg Leu Asn Phe Leu Lys
            20                  25                  30
Glu Asp Ile Asp Trp Leu Gln Arg Val Leu Arg His Ile Arg Ser Tyr
        35                  40                  45
Val Asp Asp Ala Lys Ala Lys Glu Val Gly Gly Asp Ser Arg Val Lys
    50                  55                  60
Asn Leu Leu Lys Asp Ile Gln Glu Leu Ala Gly Asp Val Glu Asp Leu
65                  70                  75                  80
Leu Asp Glu Phe Leu Pro Lys Ile Gln Gln Ser Ser Lys Phe Lys Gly
                85                  90                  95
Ala Ile Cys Cys Leu Lys Thr Val Ser Phe Ala Asp Glu Phe Ala Val
            100                 105                 110
Glu Ile Glu Lys Ile Arg Arg Arg Val Ala Asp Ile Asp Ser Leu Arg
        115                 120                 125
Thr Thr Phe Asn Ile Thr Asp Thr Ser Asn Asn Asn Asn Asp Cys Ile
    130                 135                 140
Pro Met Glu Gln Arg Arg Lys Phe Leu His Ala Asp Glu Thr Glu Val
145                 150                 155                 160
Ile Gly Leu Asp Asp Asp Phe Asn Lys Leu Gln Asp Lys Leu Leu Val
                165                 170                 175
Gln Asp Leu Cys Asn Gly Val Val Ser Ile Val Gly Met Pro Gly Leu

            180                 185                 190
Gly Lys Thr Thr Leu Ala Lys Lys Leu Tyr Arg His Val Arg His Gln
        195                 200                 205
Phe Glu Cys Ser Ala Leu Val Tyr Val Ser Gln Gln Pro Arg Ala Gly
    210                 215                 220
Glu Ile Leu Leu Asp Ile Ala Lys Gln Val Gly Leu Thr Asp Glu Gly
225                 230                 235                 240
Arg Lys Glu His Leu Glu Asp Asn Leu Arg Ser Leu Leu Glu Thr Lys
                245                 250                 255
Arg Tyr Val Ile Leu Leu Asp Asp Ile Trp Asp Thr Lys Ile Trp Asp
            260                 265                 270
Ala Leu Asn Arg Val Leu Arg Pro Glu Cys Asp Ser Lys Ile Gly Ser
        275                 280                 285
Arg Ile Ile Ile Thr Ser Arg Tyr His His Val Gly Arg Tyr Ile Gly
    290                 295                 300
Glu Asp Phe Ser Leu His Glu Leu Gln Pro Leu Asp Ser Glu Lys Ser
305                 310                 315                 320
Phe Glu Leu Phe Thr Lys Lys Ile Phe Ile Phe Asp Asn Asn Asn Asn
                325                 330                 335
Trp Ala Asn Ala Ser Pro Val Leu Val Asp Ile Gly Lys Ser Ile Val
            340                 345                 350
Arg Arg Cys Gly Gly Ile Pro Leu Ala Ile Val Val Thr Ala Gly Met
        355                 360                 365
Leu Arg Ala Arg Glu Arg Thr Glu His Ala Trp Asn Arg Val Leu Glu
    370                 375                 380
Arg Ile Gly His Asn Ile Gln Asp Gly Cys Ala Lys Ala Leu Ala Leu
385                 390                 395                 400
Ser Tyr Asn Asp Leu Pro Ile Ala Leu Arg Pro Cys Phe Leu Tyr Phe
                405                 410                 415
Gly Leu Tyr Pro Glu Asp His Glu Ile Arg Ala Phe Asp Leu Thr Asn
            420                 425                 430
Met Trp Ile Ala Glu Lys Leu Ile Val Val Asn Ser Gly Asn Arg Arg
        435                 440                 445
Glu Ala Glu Ser Leu Ala Asp Asp Val Leu Asn Asp Leu Val Ser Arg
    450                 455                 460
Asn Leu Ile Gln Val Ala Lys Arg Thr Tyr Asp Gly Gly Ile Ser Ser
465                 470                 475                 480
Cys Arg Ile His Asp Leu Leu His Ser Leu Cys Val Asp Leu Ala Lys
                485                 490                 495
Glu Ser Asn Phe Phe His Thr Glu His Asn Ala Phe Gly Asp Pro Gly
            500                 505                 510
Asn Val Ser Arg Leu Arg Arg Ile Thr Phe Tyr Ser Asp Asn Asn Ala
        515                 520                 525
Met Asn Glu Phe Phe Arg Ser Asn Pro Lys Leu Glu Lys Leu Arg Ala
    530                 535                 540
Leu Phe Cys Phe Thr Lys Gly Asp Ser Cys Ile Phe Ser His Leu Ala
545                 550                 555                 560
His His Asp Phe Lys Leu Leu Gln Val Leu Val Val Val Gln Pro Arg
                565                 570                 575
Lys Asn Tyr Asp Phe Ser Ile Ser Gln Ile Lys Ile Gly Asn Met Ser
            580                 585                 590
Cys Leu Arg Tyr Leu Arg Phe Glu Gly Asp Ile Tyr Gly Glu Leu Pro
        595                 600                 605
Asn Cys Met Val Lys Leu Lys His Leu Glu Thr Leu Asp Ile Ser Lys
    610                 615                 620
Ser Phe Ile Ile Lys Leu Pro Thr Gly Val Trp Lys Thr Thr Gln Leu
625                 630                 635                 640
Arg His Leu Arg Ser Asn Gly Tyr Asn Leu Ala Pro Tyr Ser Tyr Phe
                645                 650                 655
Cys Ile Ser Pro Phe Phe Pro Asn Val Pro Pro Asn Asn Val Gln Thr
            660                 665                 670
Leu Met Trp Met Asp Gly Glu Phe Phe Glu Pro Arg Trp Leu His Arg
        675                 680                 685

Phe Ile Asn Leu Arg Lys Leu Gly Leu Gln Glu Val Ser Asp Ser Thr
    690                 695                 700
Ile Lys Lys Leu Ser Thr Leu Ser Pro Val Pro Thr Thr Leu Glu Val
705                 710                 715                 720
Leu Lys Leu Ser Ser Phe Phe Ser Glu Leu Arg Glu Gln Ile Asn Leu
                725                 730                 735
Ser Ser Tyr Pro Asn Ile Val Lys Leu His Leu Asn Gly Arg Ile Pro
            740                 745                 750
Leu Asn Val Ser Glu Ser Phe Pro Pro Asn Leu Val Lys Leu Thr Leu
        755                 760                 765
Cys Asn Leu Met Val Asp Gly His Val Val Ala Val Leu Lys Lys Leu
    770                 775                 780
Pro Lys Leu Lys Ile Leu Thr Leu His Arg Cys Arg His Asp Ala Glu
785                 790                 795                 800
Lys Met Asp Leu Ser Gly Asp Gly Asp Ser Phe Pro Gln Leu Glu Val
                805                 810                 815
Leu His Ile Lys Asp Pro Val Cys Leu Ser Glu Val Thr Cys Thr Asp
            820                 825                 830
Asp Val Gly Met Pro Lys Leu Lys Lys Leu Leu Leu Ile Glu Arg Thr
        835                 840                 845
Asp Ser Asn Val Arg Leu Ser Glu Arg Leu Ala Lys Leu Arg Val
    850                 855                 860

<210> 81
<211> 279
<212> DNA
<213> Artificial Sequence

<220>
<223> cDNA

<400> 81
cttgcctttg cggttttagc taggagcagc gccgtcgcag cattcccaat ccccgacgag      60

tctcgcccct tgtcgaagac atctcctgac actgtggccc caagatcgct tcggatcgag     120

gcccaagaag ttattcagag cggccgggga gacggatatg gtgggttctg gaaaaacgta     180

gcccagagta ctaacaagat cgtcaagagg ccggatatca agataggcaa acttatcgag     240

gcggccaaga aggcaaaagc aaaaatgacg aagtcctga                            279

<210> 82
<211> 92
<212> PRT
<213> Phytophthora infestans

<400> 82
Leu Ala Phe Ala Val Leu Ala Arg Ser Ser Ala Val Ala Ala Phe Pro
1               5                   10                  15
Ile Pro Asp Glu Ser Arg Pro Leu Ser Lys Thr Ser Pro Asp Thr Val
            20                  25                  30
Ala Pro Arg Ser Leu Arg Ile Glu Ala Gln Glu Val Ile Gln Ser Gly
        35                  40                  45
Arg Gly Asp Gly Tyr Gly Gly Phe Trp Lys Asn Val Ala Gln Ser Thr
    50                  55                  60
Asn Lys Ile Val Lys Arg Pro Asp Ile Lys Ile Gly Lys Leu Ile Glu
65                  70                  75                  80
Ala Ala Lys Lys Ala Lys Ala Lys Met Thr Lys Ser
                85                  90

<210> 83
<400> 83
000

<210> 84
<400> 84
000

<210> 85
<211> 3738
<212> DNA
<213> Artificial Sequence

<220>
<223> cDNA

<400> 85
atgaatgaaa atgaaattga ggaaatgtta gatcacctaa gaaggatcaa aattgaaggt 60

aacctggatt tcttcaagat tcgtcgtatt ggggatcttg atattgtgct aagagttttt 120

agaaccttta taaagtatca tgttctttta cctgattgtt ttgtcaaact cacaatgaat 180

gccgaatgga ctgtggaaat gcttcaccgg gtatttgatg ggatatcaga tgaatgtaaa 240

actaacctta atttggaaag gctagaatca catttgttgg aattctttga aggtaactcc 300

agtttaagtt acaattatga gttgaatgat tttgatctgt cgaaatatat ggattgtctg 360

gaaaaaattc taaatgatgt actaatgatg ttcctggaaa agggtaggtc ctgttatccc 420

atagaaaaac ttgcaataca gctatctata aagaaactga aaattgttca aaagaaaatg 480

atatttttga gatacatata taccacagag ataaatggta acgtcaacta tgagaagctg 540

gaatgtttgg agactcgaat tcagttcatt gctaacactg tgggacaatt ttgtttggcc 600

gtattagatt atgttgctga tattgaattt agtgataata atgatatctt taatatacct 660

ccgtatctat tatcattgat tgtgtttgtg gagctggaaa tgaagaagat ttttcatggt 720

gaactaaagg tgtcaaagtt tactcaatca aaaactttca aggacaagaa attaccaaaa 780

gaattttcag atcttctcca atatctgttg atgtatctca gaaacgaaaa actcgagaac 840

tttcctaata atatctctgc tcaaaatatt gatgtggcaa tagaattctt gttggttttc 900

cttgatgctg atgtgtcaaa tcacgttatt aatggtaact ggttgaatga ggtcttgtta 960

aaggttggag ctatagcggg tgatattcta tatgtaattc aaaagcttct tcctagatct 1020

ataaacaaag atgacactag caaaataagt ttttgctcga tacagatatt ggagaagact 1080

aaagatctga aggcacaagt tgagacgtac tacaaatcct taaaatttac tccatctcag 1140

ttccccacct ttggtggatt gagctttctg gattctcttt taaggaaact gaatgagatg 1200

tcgaaatcta agtctggttt agatttcctg atgaaacctc ttttagggaa tttggagaaa 1260

gagttatcat ctcttacatc cattttagag aaggagctat catccatttt cagagatgtc 1320

gtgcaccacg aacataaaat tcctaaagat cttcagagac gtaccatcaa tttggcatat 1380

gaggctgagg ttgccattga ctctattctt gctcagtata atgctttttt gcatattttt 1440

tgctcacttc ctacaatttt aaaagagatc aagcaaatta atgcagaggt gactgagatg 1500

tggtcagcaa acattcctct taatcctcgc tacgtggctg ctccatttaa acatctgcca 1560

gctcgacata gcaatcttgt gactgatgag gaggtagtgg gttttgagaa taaagcagaa 1620

aaactaattg gttatctgat tagaggtaca aatgagctag acgtcatccc aattgtaggc 1680

atggggggac aagggaaaac gacaattgct agaaagttgt acaataatga catcattgtt 1740

tctcgcttta atgttcgagc atggtgcatc atttctcaaa catatagccg aagagagcta 1800

ttacaagaga ttttcagtca agttacgggc tccaaggaca aggaagatga ggtaggcaaa 1860

cttgctgaca ggttgaggaa aagcctaatg ggaaagagat atctcattgt attggatgat 1920

atgtgggatt gtatggtatg ggatgactta aggctttctt ttccagatga tggaatcaga 1980

agtagaatag tcgtaacaac tcgacttgaa gaagtgggta agcaagtcaa gaaccatact 2040

gatccttatt ctcttccatt cctcacaaca aaagagagtt gccaattgct gcagaaaaaa 2100

gtgtttcaaa aggaagattg cccgcctgaa ctacaatatg tgagtcaagc agttgcagaa 2160

aaatgcaaag gactgcccct agtggttgtc ttggtagctg gaataatcaa aaaaaggaaa 2220

atggaagaat cttggtggaa tgaggtgaaa gatgctttat ttgactatct tgacagtgag 2280

ttcgaagaat acagtctagc gactatgcag ttgagttttg ataacctagc tgattgttta 2340

aagccttgtc ttctttatat ggggatgttt ctggaggacg caagaattcc agtgtctaaa 2400

ttgataagct tatggattgc tgaaggattc gtggagaaca ctgaatctgg gagattaatg 2460

gaagaggaag ctgaaggtta cttgatggat ctcattagca gtaacgtggt aattgtttca 2520

aagaaaggtt ataatggtaa agtcaaatgc tgccaggttc atgatgttgt gcatcacttt 2580

tgcttgaaga agagtagaga agaaaagttt atgcttgcag tgaagggtca atatatccag 2640

tttcaaccgt tggattggaa gggaagtcga gtgagcttca gtttcagtga agagctttcc 2700

aagtttgcat ctctggtttc caaaacacag aagcctttcc accaacactt gaggtctctg 2760

ataacggcta atggtggaga atctattgat gtgattcccg tctgtcagat taatgaattg 2820

cgacttctta aggtcttgga tttgagttct tattatgtgg agtctttgtg gttagctaga 2880

ttaaacccac ttaatcagct gaagtacctc gcagtttggg caggtacttt ctattttgat 2940

ccacaatcac atctgcccca tatagaaact ttaattgtga cgagttgttt ttatggtgta 3000

cggttaccag tgtctttttg ggaaatggaa aaattaaggc atgttcattt tgctggcgct 3060

ggttttgcta tgcagggact ctttgaagga tcctctaaat tggaaaattt gaggatatta 3120

aagaaaattg aggaatttcc aattgatagg ctggatgtgt tatcaaggag gtgtcctaat 3180

cttcaacaac ttcaaatcac atttgaggat gatgtagagc ctttttgtcc caaattggag 3240

agtcttaccc agcttcaaga acttcaactt tcctttgtgc atccccgcat tctatccggg 3300

ttacagttgc cttcaaattt aaacaaattg gtacttaaag gaattcatat ggaaagtgct 3360

atttccttca ttgcggaact accaagcctg gagtatctcc aattactaga tgtgtgtttt 3420

cctcaatcag aagagtggtg ccttggagat atcacgttcc ataaacttaa gttgttgaaa 3480

ctggtgcagt taaatatctc aaagtgggat gcctcggagg aatcatttcc cttgcttgaa 3540

acacttgtta taaaaaagtg tgatgacctt gaggagatcc cacttagctt tgctgatatt 3600

ccatcattga aacagattaa gttgattggg tcttggaaag tatctatgga ggcttcagct 3660

gtgagaatta aggaagaagt cgaagagatt gaaggatgtg accgtataga cctcgtaaga 3720

agaagtcgaa gagattga 3738

<210> 86
<211> 1245
<212> PRT
<213> Solanum demissum

<400> 86
Met Asn Glu Asn Glu Ile Glu Glu Met Leu Asp His Leu Arg Arg Ile
1               5                   10                  15
Lys Ile Glu Gly Asn Leu Asp Phe Phe Lys Ile Arg Arg Ile Gly Asp
            20                  25                  30
Leu Asp Ile Val Leu Arg Val Phe Arg Thr Phe Ile Lys Tyr His Val
        35                  40                  45
Leu Leu Pro Asp Cys Phe Val Lys Leu Thr Met Asn Ala Glu Trp Thr
    50                  55                  60
Val Glu Met Leu His Arg Val Phe Asp Gly Ile Ser Asp Glu Cys Lys
65                  70                  75                  80
Thr Asn Leu Asn Leu Glu Arg Leu Glu Ser His Leu Leu Glu Phe Phe
                85                  90                  95
Glu Gly Asn Ser Ser Leu Ser Tyr Asn Tyr Glu Leu Asn Asp Phe Asp
            100                 105                 110
Leu Ser Lys Tyr Met Asp Cys Leu Glu Lys Ile Leu Asn Asp Val Leu
        115                 120                 125
Met Met Phe Leu Glu Lys Gly Arg Ser Cys Tyr Pro Ile Glu Lys Leu
    130                 135                 140
Ala Ile Gln Leu Ser Ile Lys Lys Leu Lys Ile Val Gln Lys Lys Met
145                 150                 155                 160
Ile Phe Leu Arg Tyr Ile Tyr Thr Thr Glu Ile Asn Gly Asn Val Asn
                165                 170                 175
Tyr Glu Lys Leu Glu Cys Leu Glu Thr Arg Ile Gln Phe Ile Ala Asn
            180                 185                 190
Thr Val Gly Gln Phe Cys Leu Ala Val Leu Asp Tyr Val Ala Asp Ile
        195                 200                 205
Glu Phe Ser Asp Asn Asn Asp Ile Phe Asn Ile Pro Pro Tyr Leu Leu
    210                 215                 220
Ser Leu Ile Val Phe Val Glu Leu Glu Met Lys Lys Ile Phe His Gly
225                 230                 235                 240
Glu Leu Lys Val Ser Lys Phe Thr Gln Ser Lys Thr Phe Lys Asp Lys
                245                 250                 255
Lys Leu Pro Lys Glu Phe Ser Asp Leu Leu Gln Tyr Leu Leu Met Tyr
            260                 265                 270
Leu Arg Asn Glu Lys Leu Glu Asn Phe Pro Asn Asn Ile Ser Ala Gln
        275                 280                 285

Asn Ile Asp Val Ala Ile Glu Phe Leu Leu Val Phe Leu Asp Ala Asp
    290                 295                 300
Val Ser Asn His Val Ile Asn Gly Asn Trp Leu Asn Glu Val Leu Leu
305                 310                 315                 320
Lys Val Gly Ala Ile Ala Gly Asp Ile Leu Tyr Val Ile Gln Lys Leu
                325                 330                 335
Leu Pro Arg Ser Ile Asn Lys Asp Asp Thr Ser Lys Ile Ser Phe Cys
            340                 345                 350
Ser Ile Gln Ile Leu Glu Lys Thr Lys Asp Leu Lys Ala Gln Val Glu
        355                 360                 365
Thr Tyr Tyr Lys Ser Leu Lys Phe Thr Pro Ser Gln Phe Pro Thr Phe
    370                 375                 380
Gly Gly Leu Ser Phe Leu Asp Ser Leu Leu Arg Lys Leu Asn Glu Met
385                 390                 395                 400
Ser Lys Ser Lys Ser Gly Leu Asp Phe Leu Met Lys Pro Leu Leu Gly
                405                 410                 415
Asn Leu Glu Lys Glu Leu Ser Ser Leu Thr Ser Ile Leu Glu Lys Glu
            420                 425                 430
Leu Ser Ser Ile Phe Arg Asp Val Val His His Glu His Lys Ile Pro
        435                 440                 445
Lys Asp Leu Gln Arg Arg Thr Ile Asn Leu Ala Tyr Glu Ala Glu Val
    450                 455                 460
Ala Ile Asp Ser Ile Leu Ala Gln Tyr Asn Ala Phe Leu His Ile Phe
465                 470                 475                 480
Cys Ser Leu Pro Thr Ile Leu Lys Glu Ile Lys Gln Ile Asn Ala Glu
                485                 490                 495
Val Thr Glu Met Trp Ser Ala Asn Ile Pro Leu Asn Pro Arg Tyr Val
            500                 505                 510
Ala Ala Pro Phe Lys His Leu Pro Ala Arg His Ser Asn Leu Val Thr
        515                 520                 525
Asp Glu Glu Val Val Gly Phe Glu Asn Lys Ala Glu Lys Leu Ile Gly
    530                 535                 540
Tyr Leu Ile Arg Gly Thr Asn Glu Leu Asp Val Ile Pro Ile Val Gly
545                 550                 555                 560
Met Gly Gly Gln Gly Lys Thr Thr Ile Ala Arg Lys Leu Tyr Asn Asn
                565                 570                 575
Asp Ile Ile Val Ser Arg Phe Asn Val Arg Ala Trp Cys Ile Ile Ser
            580                 585                 590
Gln Thr Tyr Ser Arg Arg Glu Leu Leu Gln Glu Ile Phe Ser Gln Val
        595                 600                 605
Thr Gly Ser Lys Asp Lys Glu Asp Glu Val Gly Lys Leu Ala Asp Arg
    610                 615                 620
Leu Arg Lys Ser Leu Met Gly Lys Arg Tyr Leu Ile Val Leu Asp Asp
625                 630                 635                 640
Met Trp Asp Cys Met Val Trp Asp Asp Leu Arg Leu Ser Phe Pro Asp
                645                 650                 655
Asp Gly Ile Arg Ser Arg Ile Val Val Thr Thr Arg Leu Glu Glu Val
            660                 665                 670
Gly Lys Gln Val Lys Asn His Thr Asp Pro Tyr Ser Leu Pro Phe Leu
        675                 680                 685
Thr Thr Lys Glu Ser Cys Gln Leu Leu Gln Lys Lys Val Phe Gln Lys
    690                 695                 700
Glu Asp Cys Pro Pro Glu Leu Gln Tyr Val Ser Gln Ala Val Ala Glu
705                 710                 715                 720
Lys Cys Lys Gly Leu Pro Leu Val Val Val Leu Val Ala Gly Ile Ile
                725                 730                 735
Lys Lys Arg Lys Met Glu Glu Ser Trp Trp Asn Glu Val Lys Asp Ala
            740                 745                 750
Leu Phe Asp Tyr Leu Asp Ser Glu Phe Glu Glu Tyr Ser Leu Ala Thr
        755                 760                 765
Met Gln Leu Ser Phe Asp Asn Leu Ala Asp Cys Leu Lys Pro Cys Leu
    770                 775                 780
Leu Tyr Met Gly Met Phe Leu Glu Asp Ala Arg Ile Pro Val Ser Lys

785                 790                 795                 800
Leu Ile Ser Leu Trp Ile Ala Glu Gly Phe Val Glu Asn Thr Glu Ser
                805                 810                 815
Gly Arg Leu Met Glu Glu Glu Ala Glu Gly Tyr Leu Met Asp Leu Ile
            820                 825                 830
Ser Ser Asn Val Val Ile Val Ser Lys Lys Gly Tyr Asn Gly Lys Val
        835                 840                 845
Lys Cys Cys Gln Val His Asp Val Val His His Phe Cys Leu Lys Lys
    850                 855                 860
Ser Arg Glu Glu Lys Phe Met Leu Ala Val Lys Gly Gln Tyr Ile Gln
865                 870                 875                 880
Phe Gln Pro Leu Asp Trp Lys Gly Ser Arg Val Ser Phe Ser Phe Ser
                885                 890                 895
Glu Glu Leu Ser Lys Phe Ala Ser Leu Val Ser Lys Thr Gln Lys Pro
            900                 905                 910
Phe His Gln His Leu Arg Ser Leu Ile Thr Ala Asn Gly Gly Glu Ser
        915                 920                 925
Ile Asp Val Ile Pro Val Cys Gln Ile Asn Glu Leu Arg Leu Leu Lys
    930                 935                 940
Val Leu Asp Leu Ser Ser Tyr Tyr Val Glu Ser Leu Trp Leu Ala Arg
945                 950                 955                 960
Leu Asn Pro Leu Asn Gln Leu Lys Tyr Leu Ala Val Trp Ala Gly Thr
                965                 970                 975
Phe Tyr Phe Asp Pro Gln Ser His Leu Pro His Ile Glu Thr Leu Ile
            980                 985                 990
Val Thr Ser Cys Phe Tyr Gly Val Arg Leu Pro Val Ser Phe Trp Glu
        995                 1000                1005
Met Glu Lys Leu Arg His Val His Phe Ala Gly Ala Gly Phe Ala Met
    1010                1015                1020
Gln Gly Leu Phe Glu Gly Ser Ser Lys Leu Glu Asn Leu Arg Ile Leu
1025                1030                1035                1040
Lys Lys Ile Glu Glu Phe Pro Ile Asp Arg Leu Asp Val Leu Ser Arg
                1045                1050                1055
Arg Cys Pro Asn Leu Gln Gln Leu Gln Ile Thr Phe Glu Asp Asp Val
            1060                1065                1070
Glu Pro Phe Cys Pro Lys Leu Glu Ser Leu Thr Gln Leu Gln Glu Leu
        1075                1080                1085
Gln Leu Ser Phe Val His Pro Arg Ile Leu Ser Gly Leu Gln Leu Pro
    1090                1095                1100
Ser Asn Leu Asn Lys Leu Val Leu Lys Gly Ile His Met Glu Ser Ala
1105                1110                1115                1120
Ile Ser Phe Ile Ala Glu Leu Pro Ser Leu Glu Tyr Leu Gln Leu Leu
                1125                1130                1135
Asp Val Cys Phe Pro Gln Ser Glu Glu Trp Cys Leu Gly Asp Ile Thr
            1140                1145                1150
Phe His Lys Leu Lys Leu Leu Lys Leu Val Gln Leu Asn Ile Ser Lys
        1155                1160                1165
Trp Asp Ala Ser Glu Glu Ser Phe Pro Leu Leu Glu Thr Leu Val Ile
    1170                1175                1180
Lys Lys Cys Asp Asp Leu Glu Glu Ile Pro Leu Ser Phe Ala Asp Ile
1185                1190                1195                1200
Pro Ser Leu Lys Gln Ile Lys Leu Ile Gly Ser Trp Lys Val Ser Met
                1205                1210                1215
Glu Ala Ser Ala Val Arg Ile Lys Glu Glu Val Glu Glu Ile Glu Gly
            1220                1225                1230
Cys Asp Arg Ile Asp Leu Val Arg Arg Ser Arg Arg Asp
        1235                1240                1245

<210> 87
<211> 735
<212> DNA
<213> Artificial Sequence

<220>
<223> cDNA

<400> 87
atgcgctcaa tccaacttct gatcatcgcg ctggttgcgt tcctcgcctg ttgtagtgca 60

acaccagcac cgccacaagt atccctctcg ttccttccag tccagagacg gtcactgcgc 120

actgacacaa ctcttgacag tgaagacaac aacgaagact cgggggagag aagcgtctgg 180

aaacatgtca aagtgaggtg gtggttagag accgaaaagt ctgacgactt tgtccggaag 240

gcgctcaaac taaacggact tgacgataca gctatgaagg cccacaagaa ctacaaatat 300

tatgcctatt ttgccaaaaa ggctgaagac tatcttttca acaagtggct acggaatcat 360

gtacccacat ttgaagcctg gaagagtctc aacctgggca aaatcaccaa ggctgaccag 420

ctgaaggaga tagcaaacac taagaacttc attagctaca gtcgcttcgt gaagcagtat 480

gacgacaacg tagtttcgac gctgaacgcc gggtataacc cccctgttgt ggcggttgct 540

aggggtgcct cagaagctga gataacagcg cgaacgatga tcatggctag tgcacgtagg 600

gacgacgatg tcgccaaagt gttgttgggc ttgactaaac cgggctatcc gcggcgagtg 660

ctagacggca atgcgctcac gcagcatgat gagtacaagt actatcagct ttttaaagaa 720

gcgaaaacat cgtaa 735

<210> 88
<211> 244
<212> PRT
<213> Phytophthora infestans

<400> 88
Met Arg Ser Ile Gln Leu Leu Ile Ile Ala Leu Val Ala Phe Leu Ala
1               5                   10                  15
Cys Cys Ser Ala Thr Pro Ala Pro Pro Gln Val Ser Leu Ser Phe Leu
            20                  25                  30
Pro Val Gln Arg Arg Ser Leu Arg Thr Asp Thr Thr Leu Asp Ser Glu
        35                  40                  45
Asp Asn Asn Glu Asp Ser Gly Glu Arg Ser Val Trp Lys His Val Lys
    50                  55                  60
Val Arg Trp Trp Leu Glu Thr Glu Lys Ser Asp Asp Phe Val Arg Lys
65                  70                  75                  80
Ala Leu Lys Leu Asn Gly Leu Asp Asp Thr Ala Met Lys Ala His Lys
                85                  90                  95
Asn Tyr Lys Tyr Tyr Ala Tyr Phe Ala Lys Lys Ala Glu Asp Tyr Leu
            100                 105                 110
Phe Asn Lys Trp Leu Arg Asn His Val Pro Thr Phe Glu Ala Trp Lys
        115                 120                 125
Ser Leu Asn Leu Gly Lys Ile Thr Lys Ala Asp Gln Leu Lys Glu Ile
    130                 135                 140
Ala Asn Thr Lys Asn Phe Ile Ser Tyr Ser Arg Phe Val Lys Gln Tyr
145                 150                 155                 160
Asp Asp Asn Val Val Ser Thr Leu Asn Ala Gly Tyr Asn Pro Pro Val
                165                 170                 175
Val Ala Val Ala Arg Gly Ala Ser Glu Ala Glu Ile Thr Ala Arg Thr
            180                 185                 190

Met Ile Met Ala Ser Ala Arg Arg Asp Asp Asp Val Ala Lys Val Leu
        195                 200                 205
Leu Gly Leu Thr Lys Pro Gly Tyr Pro Arg Arg Val Leu Asp Gly Asn
    210                 215                 220
Ala Leu Thr Gln His Asp Glu Tyr Lys Tyr Tyr Gln Leu Phe Lys Glu
225                 230                 235                 240
Ala Lys Thr Ser

<210> 89
<211> 3909
<212> DNA
<213> Artificial Sequence

<220>
<223> cDNA

<400> 89
atgaattatt gtcttccttc gagtactcta caaacaacta ccaaacgaag acttactttg      60

agaagacttt gttgcaaaca gctcaggaag aagactttgg tgcagactgc agaggaagag     120

gaagcaaaca caacaatggc cgatcctgta attggtgcta ctgttcaagt tttgcttgaa     180

aagttgattt ctctcactat cgaggaagtc aacagctcaa gggatttcaa caaagatctc     240

gaaatgttga cacaaaatgt atctttaatc caagctttca ttcatgatgt tgaaacacca     300

caagagaaac aacagtctgt ggaacaatgg ctcaacaggc ttgagagagt tgctgaagat     360

gctcaaaatg tgtttgatcg attcatatat gaatctctca aaacaaaagt ggtgaggagc     420

ccattgaaaa aggtcagtgg tttcttttct catactgctt ttaagagaaa aatgtctcaa     480

aaaatcaaca acatcaataa agagttgacg gctatcaata aggtagccaa agacctcggt     540

ctacaatcac tcatggttcc ttctcggaaa atactaccaa ttcgagaaac agattccttt     600

gtagttgctt ctgatattgt cggtagagat ttggatattg ctgagataaa ggagaagatt     660

ttgaacatga gagaggagga tattgttctg tccaccattc ccatagtagg tatgggaggt     720

ttagggaaaa caactgtggc taagaggatt tacaatgatg aacacatgaa gcaaatcttt     780

gaaaagagaa tttggttgtg tctacctgaa atgtctgaaa cgaagagctt tcttgaacaa     840

atcctcgaat cgttgataga gaggaaaatt gaggttgaaa ggagagatat aatagtcaag     900

aagctacaag atgaattggg aggaaaaaaa tatttgctag tcctggatga tttgtggtgt     960

gttgactcta catcgtggca tgagttcatt gacaccttga gaggaataaa tacatccaga    1020

ggaaactgca ttcttgtgac tactcgtagg aagcaggtgg catccacagt agcaacagat    1080

cttcatatct tggggaagtt aacagaagat cattgttggt ctattttcaa acaaaaagca    1140

tttgttgatg gcagggttcc agaggaatta gcgagcatgg gcaacaagat tgttaaaatg    1200

tgccaaggtc taccgttggc tgcaagtgtg ttgggagggc tcttacacaa caaagaaaaa    1260

catgaatggc aagcaattct tgatggcaac ctccttgttg caggtgaaga tgataatgga    1320

gaaaatagca taaagaaaat cctaaaactc agctatgatt atctaccatc tccacatctg 1380

aaaaaatgct ttgcttactt tgcaatgttt ccaaaagatt atatgtttga aaaggaccaa 1440

ctaatccaac tctggatggc agaaggtttt cttcgtccaa gtcaagagat ccctgtgatg 1500

gaagacgttg ggcacaggtt ttttcaaatc ttgttgcaga attccttgct acaagatgtt 1560

ctgttagatg aacacaacaa tataacacac tgcaagatgc acgatcttgt gcatgatttg 1620

gctggagata tcttaaaatc tagactattt gatccgaagg gcgacaatgg agaaaaactt 1680

tctcaagttc gatactttgg atgtgagtca ccaacggatc aaatagataa gatatatgag 1740

ccagaacgtt tgtgcacact gttttggaga agcaattata catctaaaga tatgctgttg 1800

aactttaagt tcttgagagt tttagatttg tccagttcag gaatcaagga gttgtcagcc 1860

aaaatcggga agctgatata cttgagatat cttgatctct cgaacactga gatcacagcc 1920

ttgcccaact ccatttgcaa gctctataat ttgcaaacat ttagagtcat caactgcttt 1980

tcactccagg aacttccata tgagatgaga aatatgataa gtttgagaca catatattac 2040

acttctgttg acgaaacaag tgggcattgg ggaggatggt gtcttcacaa tgaacatttt 2100

cagattccac ttaatatggg gcaattgact agtcttcaaa ccctcaagtt tttcaaggta 2160

ggtttagaga aaggtcgtca aatagaagaa ttaggtcatt tgaaaaacct aagaggtgaa 2220

ttgacgatca atggtctcca attggtctgt gataaagaag aggctcaaac agcatattta 2280

cacgataaac caaacatctg caagctggca tatttatggt cccatgatga atcagaaggc 2340

tgtgagatca atgatgagca tgtgttggat ggtcttcaac cgcatcctaa cttgaaaacc 2400

ttagcagtag tggactattt agggactaaa tttccttcat ggttcagtga agagtcgcta 2460

ccaaatttgg tcaagttgaa attaagtggt agcaaaaggt gcaaagaaat tccatccctt 2520

ggccaactga aattccttcg gcatcttgag ctgataggat tccatgagtt ggaatgcatt 2580

ggacctgctt tttatggtgt tgagatgaga aatattggat caaacagcat tatccaagtg 2640

ttcccgtcat tgaaaaaact agtattgaag gatatgcgta gccttattga gtggaaggga 2700

gatgaagttg gagtaagaat gtctcccggt cttgagaagt tgcggattac agactgtcca 2760

ttgttaaaaa gtattccgaa tcaatttgaa atcctccgtc aattaaaaat tacaggagtt 2820

gacagtgaaa tgccattgtt gaacttgtgc agcaacttga catctctcgt aaagcttaga 2880

gtctatgata tgaaagagct cacttgtctt ccagatgaga tgctacgtaa caacgtttct 2940

cttcaacaga taataatttt caactgcgga gagtttcgtg aattgccaca aagcttgtac 3000

aatctccatt ctcttaggag attagacatt tacaactgca ccaatttcag ttctcttcct 3060

gttcccaatg gagacaacta tttgacttcc ctcgaattct tttgcttaca taattgtaat 3120

ggattgatca gtataccaat tggaatgcta gatcaatgcc ggctagtgtt tttgaatgtc 3180

agctgctgta acaacttggt ttcattccct gtacatgtgt gggaaatgcc ttcactttca 3240

tatttgctta tatcagaatg tcccaaattg attagtgtac ccaaagtggg ccttcaccat 3300

ctcaccgggt tagtgagatt gggaattggt cctttctcag agatggtgga ttttgatgca 3360

ttccaattga tttttaatgg cattcagcag ttgttgtccc ttcgtgatct ggaggtgtac 3420

ggacgtgggc actgggattc tctgccctat cagcttatgc aactctctga cctaagagag 3480

atcacaatag ctgatttcgg aattgaggct cttcctccta ctcttgacaa ccttacttct 3540

cttgaaagtt tgacgctagt gaggtgcaaa cagctacaac atctgaactt ctcagatgcc 3600

atgcccaaat tacggctcct gtggatacgt gattgtccat tgttagaagc tctgtcggat 3660

gggctcggca accttgtttc tttggaagaa ttatatttac atgactgcga aaaactagag 3720

catctaccgt cccgagatgc catgcgacgc ctcactaaat tatggaacat gagaattaaa 3780

ggatgcccaa agttagaaga aagtttcacc aactactccc agtggtccaa aatttcccat 3840

atttcaaata ttgaattagg tgggtggaga aggacagcca taagtctcgg tttctctttc 3900

actttctga 3909

<210> 90
<211> 1302
<212> PRT
<213> Solanum chacoense

<400> 90
Met Asn Tyr Cys Leu Pro Ser Ser Thr Leu Gln Thr Thr Thr Lys Arg
1               5                   10                  15
Arg Leu Thr Leu Arg Arg Leu Cys Cys Lys Gln Leu Arg Lys Lys Thr
            20                  25                  30
Leu Val Gln Thr Ala Glu Glu Glu Glu Ala Asn Thr Thr Met Ala Asp
        35                  40                  45
Pro Val Ile Gly Ala Thr Val Gln Val Leu Leu Glu Lys Leu Ile Ser
    50                  55                  60
Leu Thr Ile Glu Glu Val Asn Ser Ser Arg Asp Phe Asn Lys Asp Leu
65                  70                  75                  80
Glu Met Leu Thr Gln Asn Val Ser Leu Ile Gln Ala Phe Ile His Asp
                85                  90                  95
Val Glu Thr Pro Gln Glu Lys Gln Gln Ser Val Glu Gln Trp Leu Asn
            100                 105                 110
Arg Leu Glu Arg Val Ala Glu Asp Ala Gln Asn Val Phe Asp Arg Phe
        115                 120                 125
Ile Tyr Glu Ser Leu Lys Thr Lys Val Val Arg Ser Pro Leu Lys Lys
    130                 135                 140
Val Ser Gly Phe Phe Ser His Thr Ala Phe Lys Arg Lys Met Ser Gln
145                 150                 155                 160
Lys Ile Asn Asn Ile Asn Lys Glu Leu Thr Ala Ile Asn Lys Val Ala
                165                 170                 175
Lys Asp Leu Gly Leu Gln Ser Leu Met Val Pro Ser Arg Lys Ile Leu
            180                 185                 190
Pro Ile Arg Glu Thr Asp Ser Phe Val Val Ala Ser Asp Ile Val Gly
        195                 200                 205
Arg Asp Leu Asp Ile Ala Glu Ile Lys Glu Lys Ile Leu Asn Met Arg
    210                 215                 220
Glu Glu Asp Ile Val Leu Ser Thr Ile Pro Ile Val Gly Met Gly Gly
225                 230                 235                 240

Leu Gly Lys Thr Thr Val Ala Lys Arg Ile Tyr Asn Asp Glu His Met
                245                 250                 255
Lys Gln Ile Phe Glu Lys Arg Ile Trp Leu Cys Leu Pro Glu Met Ser
            260                 265                 270
Glu Thr Lys Ser Phe Leu Glu Gln Ile Leu Glu Ser Leu Ile Glu Arg
        275                 280                 285
Lys Ile Glu Val Glu Arg Arg Asp Ile Ile Val Lys Lys Leu Gln Asp
    290                 295                 300
Glu Leu Gly Gly Lys Lys Tyr Leu Leu Val Leu Asp Asp Leu Trp Cys
305                 310                 315                 320
Val Asp Ser Thr Ser Trp His Glu Phe Ile Asp Thr Leu Arg Gly Ile
                325                 330                 335
Asn Thr Ser Arg Gly Asn Cys Ile Leu Val Thr Thr Arg Arg Lys Gln
            340                 345                 350
Val Ala Ser Thr Val Ala Thr Asp Leu His Ile Leu Gly Lys Leu Thr
        355                 360                 365
Glu Asp His Cys Trp Ser Ile Phe Lys Gln Lys Ala Phe Val Asp Gly
    370                 375                 380
Arg Val Pro Glu Glu Leu Ala Ser Met Gly Asn Lys Ile Val Lys Met
385                 390                 395                 400
Cys Gln Gly Leu Pro Leu Ala Ala Ser Val Leu Gly Gly Leu Leu His
                405                 410                 415
Asn Lys Glu Lys His Glu Trp Gln Ala Ile Leu Asp Gly Asn Leu Leu
            420                 425                 430
Val Ala Gly Glu Asp Asp Asn Gly Glu Asn Ser Ile Lys Lys Ile Leu
        435                 440                 445
Lys Leu Ser Tyr Asp Tyr Leu Pro Ser Pro His Leu Lys Lys Cys Phe
    450                 455                 460
Ala Tyr Phe Ala Met Phe Pro Lys Asp Tyr Met Phe Glu Lys Asp Gln
465                 470                 475                 480
Leu Ile Gln Leu Trp Met Ala Glu Gly Phe Leu Arg Pro Ser Gln Glu
                485                 490                 495
Ile Pro Val Met Glu Asp Val Gly His Arg Phe Phe Gln Ile Leu Leu
            500                 505                 510
Gln Asn Ser Leu Leu Gln Asp Val Leu Leu Asp Glu His Asn Asn Ile
        515                 520                 525
Thr His Cys Lys Met His Asp Leu Val His Asp Leu Ala Gly Asp Ile
    530                 535                 540
Leu Lys Ser Arg Leu Phe Asp Pro Lys Gly Asp Asn Gly Glu Lys Leu
545                 550                 555                 560
Ser Gln Val Arg Tyr Phe Gly Cys Glu Ser Pro Thr Asp Gln Ile Asp
                565                 570                 575
Lys Ile Tyr Glu Pro Glu Arg Leu Cys Thr Leu Phe Trp Arg Ser Asn
            580                 585                 590
Tyr Thr Ser Lys Asp Met Leu Leu Asn Phe Lys Phe Leu Arg Val Leu
        595                 600                 605
Asp Leu Ser Ser Ser Gly Ile Lys Glu Leu Ser Ala Lys Ile Gly Lys
    610                 615                 620
Leu Ile Tyr Leu Arg Tyr Leu Asp Leu Ser Asn Thr Glu Ile Thr Ala
625                 630                 635                 640
Leu Pro Asn Ser Ile Cys Lys Leu Tyr Asn Leu Gln Thr Phe Arg Val
                645                 650                 655
Ile Asn Cys Phe Ser Leu Gln Glu Leu Pro Tyr Glu Met Arg Asn Met
            660                 665                 670
Ile Ser Leu Arg His Ile Tyr Tyr Thr Ser Val Asp Glu Thr Ser Gly
        675                 680                 685
His Trp Gly Gly Trp Cys Leu His Asn Glu His Phe Gln Ile Pro Leu
    690                 695                 700
Asn Met Gly Gln Leu Thr Ser Leu Gln Thr Leu Lys Phe Phe Lys Val
705                 710                 715                 720
Gly Leu Glu Lys Gly Arg Gln Ile Glu Glu Leu Gly His Leu Lys Asn
                725                 730                 735
Leu Arg Gly Glu Leu Thr Ile Asn Gly Leu Gln Leu Val Cys Asp Lys

740                 745                 750
Glu Glu Ala Gln Thr Ala Tyr Leu His Asp Lys Pro Asn Ile Cys Lys
        755                 760                 765
Leu Ala Tyr Leu Trp Ser His Asp Glu Ser Glu Gly Cys Glu Ile Asn
    770                 775                 780
Asp Glu His Val Leu Asp Gly Leu Gln Pro His Pro Asn Leu Lys Thr
785                 790                 795                 800
Leu Ala Val Val Asp Tyr Leu Gly Thr Lys Phe Pro Ser Trp Phe Ser
                805                 810                 815
Glu Glu Ser Leu Pro Asn Leu Val Lys Leu Lys Leu Ser Gly Ser Lys
            820                 825                 830
Arg Cys Lys Glu Ile Pro Ser Leu Gly Gln Leu Lys Phe Leu Arg His
        835                 840                 845
Leu Glu Leu Ile Gly Phe His Glu Leu Glu Cys Ile Gly Pro Ala Phe
    850                 855                 860
Tyr Gly Val Glu Met Arg Asn Ile Gly Ser Asn Ser Ile Ile Gln Val
865                 870                 875                 880
Phe Pro Ser Leu Lys Lys Leu Val Leu Lys Asp Met Arg Ser Leu Ile
                885                 890                 895
Glu Trp Lys Gly Asp Glu Val Gly Val Arg Met Ser Pro Gly Leu Glu
            900                 905                 910
Lys Leu Arg Ile Thr Asp Cys Pro Leu Leu Lys Ser Ile Pro Asn Gln
        915                 920                 925
Phe Glu Ile Leu Arg Gln Leu Lys Ile Thr Gly Val Asp Ser Glu Met
    930                 935                 940
Pro Leu Leu Asn Leu Cys Ser Asn Leu Thr Ser Leu Val Lys Leu Arg
945                 950                 955                 960
Val Tyr Asp Met Lys Glu Leu Thr Cys Leu Pro Asp Glu Met Leu Arg
                965                 970                 975
Asn Asn Val Ser Leu Gln Gln Ile Ile Ile Phe Asn Cys Gly Glu Phe
            980                 985                 990
Arg Glu Leu Pro Gln Ser Leu Tyr Asn Leu His Ser Leu Arg Arg Leu
        995                 1000                1005
Asp Ile Tyr Asn Cys Thr Asn Phe Ser Ser Leu Pro Val Pro Asn Gly
    1010                1015                1020
Asp Asn Tyr Leu Thr Ser Leu Glu Phe Phe Cys Leu His Asn Cys Asn
1025                1030                1035                1040
Gly Leu Ile Ser Ile Pro Ile Gly Met Leu Asp Gln Cys Arg Leu Val
                1045                1050                1055
Phe Leu Asn Val Ser Cys Cys Asn Asn Leu Val Ser Phe Pro Val His
            1060                1065                1070
Val Trp Glu Met Pro Ser Leu Ser Tyr Leu Leu Ile Ser Glu Cys Pro
        1075                1080                1085
Lys Leu Ile Ser Val Pro Lys Val Gly Leu His His Leu Thr Gly Leu
    1090                1095                1100
Val Arg Leu Gly Ile Gly Pro Phe Ser Glu Met Val Asp Phe Asp Ala
1105                1110                1115                1120
Phe Gln Leu Ile Phe Asn Gly Ile Gln Gln Leu Leu Ser Leu Arg Asp
                1125                1130                1135
Leu Glu Val Tyr Gly Arg Gly His Trp Asp Ser Leu Pro Tyr Gln Leu
            1140                1145                1150
Met Gln Leu Ser Asp Leu Arg Glu Ile Thr Ile Ala Asp Phe Gly Ile
        1155                1160                1165
Glu Ala Leu Pro Pro Thr Leu Asp Asn Leu Thr Ser Leu Glu Ser Leu
    1170                1175                1180
Thr Leu Val Arg Cys Lys Gln Leu Gln His Leu Asn Phe Ser Asp Ala
1185                1190                1195                1200
Met Pro Lys Leu Arg Leu Leu Trp Ile Arg Asp Cys Pro Leu Leu Glu
                1205                1210                1215
Ala Leu Ser Asp Gly Leu Gly Asn Leu Val Ser Leu Glu Glu Leu Tyr
            1220                1225                1230
Leu His Asp Cys Glu Lys Leu Glu His Leu Pro Ser Arg Asp Ala Met
        1235                1240                1245

Arg Arg Leu Thr Lys Leu Trp Asn Met Arg Ile Lys Gly Cys Pro Lys
    1250                1255                1260
Leu Glu Glu Ser Phe Thr Asn Tyr Ser Gln Trp Ser Lys Ile Ser His
1265                1270                1275                1280
Ile Ser Asn Ile Glu Leu Gly Gly Trp Arg Arg Thr Ala Ile Ser Leu
                1285                1290                1295
Gly Phe Ser Phe Thr Phe
            1300

<210> 91
<211> 348
<212> DNA
<213> Artificial Sequence

<220>
<223> cDNA

<400> 91
atggcgacgg ccactgttct agtacagagt cctgcgtccg gtttgaccac gactgtggct      60

gacacggccc agacggcaac cagcattcta actcctgttc tagctgggga gccgaacaaa     120

cacgttacaa cgcgatcttt gagaacgcat ccgatagccg acagcgacga cggcgaagag     180

cgactgctta atggtatgac agattttgtc aagtaccacg ctggaaagat gaatcccgag     240

cagctttaca agtacttaaa gttacaagga cgtggtcaag aagcctacaa acacaagaac     300

tacgctagtt acattaagaa gtcgaagaag tggtggaaga accagtaa                  348

<210> 92
<211> 115
<212> PRT
<213> Phytophthora infestans

<400> 92
Met Ala Thr Ala Thr Val Leu Val Gln Ser Pro Ala Ser Gly Leu Thr
1               5                   10                  15
Thr Thr Val Ala Asp Thr Ala Gln Thr Ala Thr Ser Ile Leu Thr Pro
            20                  25                  30
Val Leu Ala Gly Glu Pro Asn Lys His Val Thr Thr Arg Ser Leu Arg
        35                  40                  45
Thr His Pro Ile Ala Asp Ser Asp Asp Gly Glu Glu Arg Leu Leu Asn
    50                  55                  60
Gly Met Thr Asp Phe Val Lys Tyr His Ala Gly Lys Met Asn Pro Glu
65                  70                  75                  80
Gln Leu Tyr Lys Tyr Leu Lys Leu Gln Gly Arg Gly Gln Glu Ala Tyr
                85                  90                  95
Lys His Lys Asn Tyr Ala Ser Tyr Ile Lys Lys Ser Lys Lys Trp Trp
            100                 105                 110
Lys Asn Gln
        115

<210> 93
<211> 3849
<212> DNA
<213> Artificial Sequence

<220>

<223> cDNA

<400> 93
atggagattg gcttagcagt tggtggtgca tttctctctt cagctttgaa tgttctcttt 60

gataggcttg ctcctcacgg tgatctgctc aacatgtttc agaagcataa ggatcatgtt 120

aagctcttaa agaagctgga ggacattttg ctcggtcttc agattgtgct aagtgatgca 180

gagaataaac aagcatcaaa tcgacatgtg agccagtggt tcaataagct tcagaatgct 240

gtggacggtg ctgagaactt gatagaacaa gtcaattatg aagctttgag gcttaaggtg 300

gaaggccagc atcaaaatct tgcagaaaca agcaaccagc aagtaagtga ccttaacctg 360

tgcttcagtg atgatttctt tcttaacata aaggataagt tggaagaaac cattgaaaca 420

ttggaggtgt tggaaaagca aattggtcgc cttggcttaa aggaacattt tggttcgact 480

aaacaagaaa ctagaacacc ttcaacttct ttagttgatg actctgatat ctttggaagg 540

cagaatgata tagaggattt gattgaccgt ttattatctg aagatgcaag tggaaaaaag 600

cggactgtag ttcctattgt tggaatgggt ggtctgggta agacaacact tgctaaagcg 660

gtttacaatg atgagagagt gcagatacat tttggtttga aagcttggtt ttgtgtttct 720

gaggcatttg atgctttcag aataacaaaa gggttacttc aagaaattgg ctcatttgac 780

ttgaaggctg atgacaatct taatcagcta caagtcaaat tgaaggaaag attaaaggga 840

aagaagtttc ttatagtttt ggatgatgtg tggaatgaca actacaacaa gtgggatgaa 900

ttgagaaatg tttttgtaca aggagatata ggaagtaaga tcattgtgac gacacgtaaa 960

gagagtgttg ccttgatgat gggaaatgag caaattagca tggacaattt gtctactgaa 1020

tcctcttggt ctttatttaa aacacatgca tttgaaaaca tgggtcctat gggacatccg 1080

gaacttgaag aggtcgggaa acaaattgca gctaagtgca aaggactgcc cttagctctg 1140

aagacgctcg ctggcatgtt acgctccaaa tcagaggttg aagagtggaa acgtattttg 1200

agaagtgaaa tatgggagct gccacacaat gacatattac cagcgttgat gttgagctac 1260

aatgatcttc ccgcacattt aaagcgatgt tttccttttt gtgcaatatt tcctaaagat 1320

tatcccttta ggaaagaaca agttattcat ctgtggattg ccaatggtct cgtaccacag 1380

gaagatgtaa taattgaaga ttcaggcaac caatactttc tcgagttgag gtcaagatca 1440

ttattcgaaa gggtcccaaa tccttctcaa gggaacacag agaatttatt cttaatgcat 1500

gaccttgtca atgatttagc gcaaattgca tcttcaaaac tttgtatcag gttggaagag 1560

agccaaggat ctcatatgtt ggaacaaagt caacacttat catattcaat gggatatggt 1620

ggtgagtttg agaaattgac acccctctac aaattggagc agctgaggac attgcttccg 1680

acatgtattg atctccctga ttgttgtcac catctaagca agagggtgct acataacata 1740

ctgccaagac taacatcctt aagggcatta tcgttgtcat gctatgagat tgttgagttg 1800

ccaaatgact tgtttatcaa attaaagctc ctcagatttt tggatatttc tcggacagag 1860
attaaaaggt tgccagattc catttgtgca ttgtataact tagagacact tctcctgtca 1920
tcttgttatg atcttgagga gctaccgctg cagatggaga agctgattaa cttgcgtcat 1980
cttgacataa gcaacactcg tctcttgaag atgccgctac atctgagcaa gttgaaaagc 2040
ctccaagtgt tagtgggagc caaatttctt ataggtggtt tgagaatgga agatttgggt 2100
gaagtacata acttgtacgg atctctatca gttgtagagt tgcaaaatgt ggttgataga 2160
agggaagctg tgaaggcaaa gatgagggag aagaatcacg tcgacaggtt atatttggag 2220
tggagtggaa gtagtagtgc cgacaattca caaacagaaa gagacatact tgatgagcta 2280
cgcccccata aaaacataaa agtagtcaaa atcactggat atagagggac aaactttccc 2340
aattggctag ctgatccttt gtttcttaag ctggtaaaat tgtctcttag aaactgcaag 2400
aactgttatt cattgccagc actaggacaa ctcccttttc tgaaattcct ttcgattaga 2460
gagatgcatg gaataacaga ggtgacggaa gaattctatg gcagttggtc ctccaaaaag 2520
ccttttaact gtcttgagaa gcttgaattt aaagatatgc cggagtggaa gcaatgggac 2580
ctactaggaa gtggagagtt ccctatactt gagaagcttt tgattgaaaa ttgccctgaa 2640
ctcagtttgg agacggtacc catccaactt tcaagtttaa aaagttttga cgtaattggt 2700
tctcccttgg ttataaactt tcctttaagc atactgccca ctaccttgaa gagaataaag 2760
atatctgatt gccagaaatt gaaattggaa cagccaactg gtgagattag tatgtttctg 2820
gaggaattga cactgattaa atgtgattgt atagatgata tatcacctga gttactccca 2880
agagcacgca aattgtgggt acaggattgg cacaacctta ctaggttttt gattcctact 2940
gccactgaaa ctctcgatat ttggaattgt gagaatgttg aaatactttc ggtggcatgt 3000
ggggggaccc agatgacgtc actgactatt gcctactgta agaagctgaa gtggctgcca 3060
gaacgtatgc aggaactcct tccatctctt aaggaactgc atctgtctaa ttgtccagaa 3120
atagagtcct ttcctgaagg tggattgccc ttcaatttac aacaacttgc gatcagatat 3180
tgcaagaaac tggtgaacgg ccgaaaggag tggcatttac agagacgact ctgtctcaca 3240
gcgttaatca tctaccatga tggcagtgac gaagagattg ttggtggtga gaattgggag 3300
ttgccttcct ctattcaaag gcttaccata gtgaatctga aaacattaag cagccaacat 3360
ctcaaaaacc tcacctctct tcaatatcta tttattaggg gtaatttacc tcagattcag 3420
ccaatgctgg aacaaggcca gtgttcgcac ctcacttcgc ttcaaagtct acaaatctcc 3480
tccctccaat cacttcctga atcggcactg ccctcctccc tctctcacct ggagatctcc 3540
cattgcccta atctccaatc acttcctgaa tcggcactgc cctcctccct ctctcagctg 3600
accatcaata attgccctaa tctccaatca ctttccgaat caacactgcc ctcctccctc 3660
tctcagctgg agatctcctt ttgccctaat ctccaatatc ttccactaaa agggatgccc 3720

tcttccctct ctgaactatc tatttacaaa tgcccattgc tcaaaccaca actagaattt     3780

gacaaggggg aatactggcc aaatattgct caatttccca ccataaagat cgatagggaa     3840

tgcatgtga                                                             3849

<210> 94
<211> 1282
<212> PRT
<213> Solanum tuberosum

<400> 94
Met Glu Ile Gly Leu Ala Val Gly Gly Ala Phe Leu Ser Ser Ala Leu
1               5                   10                  15
Asn Val Leu Phe Asp Arg Leu Ala Pro His Gly Asp Leu Leu Asn Met
            20                  25                  30
Phe Gln Lys His Lys Asp His Val Lys Leu Leu Lys Lys Leu Glu Asp
        35                  40                  45
Ile Leu Leu Gly Leu Gln Ile Val Leu Ser Asp Ala Glu Asn Lys Gln
    50                  55                  60
Ala Ser Asn Arg His Val Ser Gln Trp Phe Asn Lys Leu Gln Asn Ala
65                  70                  75                  80
Val Asp Gly Ala Glu Asn Leu Ile Glu Gln Val Asn Tyr Glu Ala Leu
                85                  90                  95
Arg Leu Lys Val Glu Gly Gln His Gln Asn Leu Ala Glu Thr Ser Asn
            100                 105                 110
Gln Gln Val Ser Asp Leu Asn Leu Cys Phe Ser Asp Asp Phe Phe Leu
        115                 120                 125
Asn Ile Lys Asp Lys Leu Glu Glu Thr Ile Glu Thr Leu Glu Val Leu
    130                 135                 140
Glu Lys Gln Ile Gly Arg Leu Gly Leu Lys Glu His Phe Gly Ser Thr
145                 150                 155                 160
Lys Gln Glu Thr Arg Thr Pro Ser Thr Ser Leu Val Asp Asp Ser Asp
                165                 170                 175
Ile Phe Gly Arg Gln Asn Asp Ile Glu Asp Leu Ile Asp Arg Leu Leu
            180                 185                 190
Ser Glu Asp Ala Ser Gly Lys Lys Arg Thr Val Val Pro Ile Val Gly
        195                 200                 205
Met Gly Gly Leu Gly Lys Thr Thr Leu Ala Lys Ala Val Tyr Asn Asp
    210                 215                 220
Glu Arg Val Gln Ile His Phe Gly Leu Lys Ala Trp Phe Cys Val Ser
225                 230                 235                 240
Glu Ala Phe Asp Ala Phe Arg Ile Thr Lys Gly Leu Leu Gln Glu Ile
                245                 250                 255
Gly Ser Phe Asp Leu Lys Ala Asp Asp Asn Leu Asn Gln Leu Gln Val
            260                 265                 270
Lys Leu Lys Glu Arg Leu Lys Gly Lys Lys Phe Leu Ile Val Leu Asp
        275                 280                 285
Asp Val Trp Asn Asp Asn Tyr Asn Lys Trp Asp Glu Leu Arg Asn Val
    290                 295                 300
Phe Val Gln Gly Asp Ile Gly Ser Lys Ile Ile Val Thr Thr Arg Lys
305                 310                 315                 320
Glu Ser Val Ala Leu Met Met Gly Asn Glu Gln Ile Ser Met Asp Asn
                325                 330                 335
Leu Ser Thr Glu Ser Ser Trp Ser Leu Phe Lys Thr His Ala Phe Glu
            340                 345                 350
Asn Met Gly Pro Met Gly His Pro Glu Leu Glu Glu Val Gly Lys Gln
        355                 360                 365
Ile Ala Ala Lys Cys Lys Gly Leu Pro Leu Ala Leu Lys Thr Leu Ala
    370                 375                 380

Gly Met Leu Arg Ser Lys Ser Glu Val Glu Glu Trp Lys Arg Ile Leu
385                 390                 395                 400
Arg Ser Glu Ile Trp Glu Leu Pro His Asn Asp Ile Leu Pro Ala Leu
                405                 410                 415
Met Leu Ser Tyr Asn Asp Leu Pro Ala His Leu Lys Arg Cys Phe Ser
            420                 425                 430
Phe Cys Ala Ile Phe Pro Lys Asp Tyr Pro Phe Arg Lys Glu Gln Val
        435                 440                 445
Ile His Leu Trp Ile Ala Asn Gly Leu Val Pro Gln Glu Asp Val Ile
    450                 455                 460
Ile Glu Asp Ser Gly Asn Gln Tyr Phe Leu Glu Leu Arg Ser Arg Ser
465                 470                 475                 480
Leu Phe Glu Arg Val Pro Asn Pro Ser Gln Gly Asn Thr Glu Asn Leu
                485                 490                 495
Phe Leu Met His Asp Leu Val Asn Asp Leu Ala Gln Ile Ala Ser Ser
            500                 505                 510
Lys Leu Cys Ile Arg Leu Glu Glu Ser Gln Gly Ser His Met Leu Glu
        515                 520                 525
Gln Ser Gln His Leu Ser Tyr Ser Met Gly Tyr Gly Gly Glu Phe Glu
    530                 535                 540
Lys Leu Thr Pro Leu Tyr Lys Leu Glu Gln Leu Arg Thr Leu Leu Pro
545                 550                 555                 560
Thr Cys Ile Asp Leu Pro Asp Cys Cys His His Leu Ser Lys Arg Val
                565                 570                 575
Leu His Asn Ile Leu Pro Arg Leu Thr Ser Leu Arg Ala Leu Ser Leu
            580                 585                 590
Ser Cys Tyr Glu Ile Val Glu Leu Pro Asn Asp Leu Phe Ile Lys Leu
        595                 600                 605
Lys Leu Leu Arg Phe Leu Asp Ile Ser Arg Thr Glu Ile Lys Arg Leu
    610                 615                 620
Pro Asp Ser Ile Cys Ala Leu Tyr Asn Leu Glu Thr Leu Leu Leu Ser
625                 630                 635                 640
Ser Cys Tyr Asp Leu Glu Glu Leu Pro Leu Gln Met Glu Lys Leu Ile
                645                 650                 655
Asn Leu Arg His Leu Asp Ile Ser Asn Thr Arg Leu Leu Lys Met Pro
            660                 665                 670
Leu His Leu Ser Lys Leu Lys Ser Leu Gln Val Leu Val Gly Ala Lys
        675                 680                 685
Phe Leu Ile Gly Gly Leu Arg Met Glu Asp Leu Gly Glu Val His Asn
    690                 695                 700
Leu Tyr Gly Ser Leu Ser Val Val Glu Leu Gln Asn Val Val Asp Arg
705                 710                 715                 720
Arg Glu Ala Val Lys Ala Lys Met Arg Glu Lys Asn His Val Asp Arg
                725                 730                 735
Leu Tyr Leu Glu Trp Ser Gly Ser Ser Ser Ala Asp Asn Ser Gln Thr
            740                 745                 750
Glu Arg Asp Ile Leu Asp Glu Leu Arg Pro His Lys Asn Ile Lys Val
        755                 760                 765
Val Lys Ile Thr Gly Tyr Arg Gly Thr Asn Phe Pro Asn Trp Leu Ala
    770                 775                 780
Asp Pro Leu Phe Leu Lys Leu Val Lys Leu Ser Leu Arg Asn Cys Lys
785                 790                 795                 800
Asn Cys Tyr Ser Leu Pro Ala Leu Gly Gln Leu Pro Phe Leu Lys Phe
                805                 810                 815
Leu Ser Ile Arg Glu Met His Gly Ile Thr Glu Val Thr Glu Glu Phe
            820                 825                 830
Tyr Gly Ser Trp Ser Ser Lys Lys Pro Phe Asn Cys Leu Glu Lys Leu
        835                 840                 845
Glu Phe Lys Asp Met Pro Glu Trp Lys Gln Trp Asp Leu Leu Gly Ser
    850                 855                 860
Gly Glu Phe Pro Ile Leu Glu Lys Leu Leu Ile Glu Asn Cys Pro Glu
865                 870                 875                 880
Leu Ser Leu Glu Thr Val Pro Ile Gln Leu Ser Ser Leu Lys Ser Phe

885                 890                 895
Asp Val Ile Gly Ser Pro Leu Val Ile Asn Phe Pro Leu Ser Ile Leu
            900                 905                 910
Pro Thr Thr Leu Lys Arg Ile Lys Ile Ser Asp Cys Gln Lys Leu Lys
        915                 920                 925
Leu Glu Gln Pro Thr Gly Glu Ile Ser Met Phe Leu Glu Glu Leu Thr
    930                 935                 940
Leu Ile Lys Cys Asp Cys Ile Asp Asp Ile Ser Pro Glu Leu Leu Pro
945                 950                 955                 960
Arg Ala Arg Lys Leu Trp Val Gln Asp Trp His Asn Leu Thr Arg Phe
                965                 970                 975
Leu Ile Pro Thr Ala Thr Glu Thr Leu Asp Ile Trp Asn Cys Glu Asn
            980                 985                 990
Val Glu Ile Leu Ser Val Ala Cys Gly Gly Thr Gln Met Thr Ser Leu
        995                 1000                1005
Thr Ile Ala Tyr Cys Lys Lys Leu Lys Trp Leu Pro Glu Arg Met Gln
    1010                1015                1020
Glu Leu Leu Pro Ser Leu Lys Glu Leu His Leu Ser Asn Cys Pro Glu
1025                1030                1035                1040
Ile Glu Ser Phe Pro Glu Gly Gly Leu Pro Phe Asn Leu Gln Gln Leu
                1045                1050                1055
Ala Ile Arg Tyr Cys Lys Lys Leu Val Asn Gly Arg Lys Glu Trp His
            1060                1065                1070
Leu Gln Arg Arg Leu Cys Leu Thr Ala Leu Ile Ile Tyr His Asp Gly
        1075                1080                1085
Ser Asp Glu Glu Ile Val Gly Gly Glu Asn Trp Glu Leu Pro Ser Ser
    1090                1095                1100
Ile Gln Arg Leu Thr Ile Val Asn Leu Lys Thr Leu Ser Ser Gln His
1105                1110                1115                1120
Leu Lys Asn Leu Thr Ser Leu Gln Tyr Leu Phe Ile Arg Gly Asn Leu
                1125                1130                1135
Pro Gln Ile Gln Pro Met Leu Glu Gln Gly Gln Cys Ser His Leu Thr
            1140                1145                1150
Ser Leu Gln Ser Leu Gln Ile Ser Ser Leu Gln Ser Leu Pro Glu Ser
        1155                1160                1165
Ala Leu Pro Ser Ser Leu Ser His Leu Glu Ile Ser His Cys Pro Asn
    1170                1175                1180
Leu Gln Ser Leu Pro Glu Ser Ala Leu Pro Ser Ser Leu Ser Gln Leu
1185                1190                1195                1200
Thr Ile Asn Asn Cys Pro Asn Leu Gln Ser Leu Ser Glu Ser Thr Leu
                1205                1210                1215
Pro Ser Ser Leu Ser Gln Leu Glu Ile Ser Phe Cys Pro Asn Leu Gln
            1220                1225                1230
Tyr Leu Pro Leu Lys Gly Met Pro Ser Ser Leu Ser Glu Leu Ser Ile
        1235                1240                1245
Tyr Lys Cys Pro Leu Leu Lys Pro Gln Leu Glu Phe Asp Lys Gly Glu
    1250                1255                1260
Tyr Trp Pro Asn Ile Ala Gln Phe Pro Thr Ile Lys Ile Asp Arg Glu
1265                1270                1275                1280
Cys Met


<210> 95
<211> 445
<212> DNA
<213> Artificial Sequence


<220>
<223> cDNA

<400> 95
atgcgtctgg caattatgct gtctgctacg gctgtcgcca aaaactttgc aacctgcagt        60

gcaatcgacc aaaccaaggt cctggtgtat gggacgccag ctcactacat acacgattca     120

gccggcagaa gacttcttcg caagaacgaa gagaatgaag aaacgtctga ggagcgagcc     180

ccaaatttca atttggcgaa tctaaatgag gagatgttta atgtggctgc gttgacgaag     240

agagcagatg ccaaaaagct agcgaaacag cttatgggta atgataagct ggcggatgct     300

gcatacattt ggtggcagca caacagggtt acgctagacc agattgacac gttcctgaag     360

cttgcaagcc gcaagacgca aggcgcaaag tacaatcaga tctacaatag ccacatgatg     420

cacctggggc tcactggata ttagt                                           445

<210> 96
<211> 147
<212> PRT
<213> Phytophthora infestans

<400> 96
Met Arg Leu Ala Ile Met Leu Ser Ala Thr Ala Val Ala Lys Asn Phe
1               5                   10                  15
Ala Thr Cys Ser Ala Ile Asp Gln Thr Lys Val Leu Val Tyr Gly Thr
            20                  25                  30
Pro Ala His Tyr Ile His Asp Ser Ala Gly Arg Arg Leu Leu Arg Lys
        35                  40                  45
Asn Glu Glu Asn Glu Glu Thr Ser Glu Glu Arg Ala Pro Asn Phe Asn
    50                  55                  60
Leu Ala Asn Leu Asn Glu Glu Met Phe Asn Val Ala Ala Leu Thr Lys
65                  70                  75                  80
Arg Ala Asp Ala Lys Lys Leu Ala Lys Gln Leu Met Gly Asn Asp Lys
                85                  90                  95
Leu Ala Asp Ala Ala Tyr Ile Trp Trp Gln His Asn Arg Val Thr Leu
            100                 105                 110
Asp Gln Ile Asp Thr Phe Leu Lys Leu Ala Ser Arg Lys Thr Gln Gly
        115                 120                 125
Ala Lys Tyr Asn Gln Ile Tyr Asn Ser His Met Met His Leu Gly Leu
    130                 135                 140
Thr Gly Tyr
145

<210> 97
<211> 3852
<212> DNA
<213> Artificial Sequence

<220>
<223> cDNA

<400> 97
atggaaattg gcttagcagt tggtggtgca tttctctctt cagctttgaa tgttctcttt      60

gacaggcttg ctcctaatag tgatctgttg aagatgttta agagggacaa gcgtgatgtt     120

cggctcttaa agaagctgag gatgactttg cttggccttc aggctgtgtt aagtgatgcg     180

gagaataagc aagcatcaaa tccatacgtg agccagtggc ttaatgagct tcaagatgct     240

gtggacggtg ctgaaaactt aattgaagaa gtcaattatg aagttttgag actaaaggtg     300

gaaggtcagt gtcaaaatct tggagaaaca agcaatcaac aggtaagtga ctgcaacctg 360

tgcttgagtg atgatttttt tcttaacata aaggagaagt tggaagagac cattgaaaca 420

ttggaagagt tggaaaagca aattggtcgc cttgatctaa caaagtatct tgattcgggt 480

aaacaagaaa caagggaatc ttcaacttct gttgttgatg aatctgatat cttaggtagg 540

cagaacgaaa tagagggatt gattgaccgt ttgttgtctg aggatggaaa gaatctgact 600

gtagttcctg ttgttggaat ggggggcgtg ggcaagacaa cacttgctaa agctgtttac 660

aatgatgaga aagtaaaaaa ccattttggt ttcaaagctt ggatctgtgt gtctgaacca 720

tatgatattc tcagaataac aaaggagtta cttcaagaat ttggcttaat ggttgataac 780

aatctgaatc aacttcaagt caaattgaag gagagcttaa agggaaaaaa gtttcttatt 840

gtcctagatg atgtatggaa tgaaaactat aaagagtggg atgacttgag aaatcttttt 900

gtacaaggag atgtaggaag taagatcatt gtgacgacac gtaaggagag tgttgccttg 960

atgatgggtt gtggggcaat caacgtgggg actctatcta gtgaagtctc ttgggatctt 1020

ttcaagcggc attcatttga aaatagggat cctaaggaac atccagaact tgaagagatt 1080

ggaatacaaa ttgcatacaa gtgcaaaggt ttgcctttag ctctaaaggc acttgctggt 1140

attttacgct ccaaatcaga ggtggatgag tggagacaca ttttaagaag tgaaatatgg 1200

gagctgcaaa gtcgttcgaa tggaatctta ccagcgttga tgttgagcta taatgatctt 1260

cctccacaat tgaagcggtg ttttgctttt tgtgcaatat atccgaaaga ttatctattt 1320

tgcaaagaac aagttgttca cctgtggatt gctaatggtc ttgtacagca gttgcattca 1380

gctaaccaat actttctcga gttgagatcg cgatcattgt ttgaaaaggt ccgagagtct 1440

tctaaatgga attcggggga attcttaatg catgaccttg tcaacgattt ggcccaaatt 1500

gcatcttcaa atctgtgtat gaggttggaa gagaaccaag gatctcatat gttggaacga 1560

actcgacatt tgtcgtattc aatgggtgat ggtgatttcg gtaaactgaa aaccctcaac 1620

aaattggagc aattgaggac attgcttccc atcaatatcc agcggcgtcc atgccatctt 1680

aagaagagga tgcttcatga catatttcca agactaatat ccctaagggc actatcactg 1740

tctccttatg atattgagga gttgccgaat gacttgttta tcaaattgaa gcacctaaaa 1800

tttttggacc tttcttggac acagataaaa aagttgccag attcaatttg tgaactgtac 1860

agcttagaga tacttatctt gtcacattgt agtcatctta atgagccacc gctgcagatg 1920

gagaagttga tcaacttgca tcacctcgac gttagcgacg cttatttctt gaagacgccg 1980

ctacatgtga gcaagttgaa aaatctccat gtgctagtgg gagctaaatt ttttcttact 2040

ggttccagtg gtttgagaat tgaagatttg ggtgaactac ataacttgta tggatctcta 2100

tcaattctag agttgcaaca tgtggtagat agaagggaat ctctgaaggc aaatatgagg 2160

gaaaagaaac atgttgaaag gttatctttg gagtgggggg gaagttttgc tgacaattca 2220

caaactgaaa gagacatact tgatgagcta caaccaaata caaacataaa agaactccga 2280

atcactggct atagaggaac aaaatttcca aattggctag ctgatcattc atttcataag 2340

ctaatagaaa tgtctcttag ctactgcaag gactgtgatt ccttgccagc actaggacag 2400

cttccttgtt taaaatccct taccattaga gggatgcatc aaataacaga ggtgagtgaa 2460

gagttctatg gtcgtttttc ctccacaaag ccatttaact ctcttgagaa acttgaattt 2520

gcagagatgc cggagtggaa gcagtggcat gtactgggga agggagagtt ccctgtacta 2580

gaggaacttt tgatttatcg ttgcccaaag ttgattggga agttgcctga aaatgtttct 2640

tcgctgagaa gattgagaat tttaaaatgc cctgaactca gtttggagac acctatccaa 2700

ctttcaaatt taaaagagtt tgaagttgct gatgctcaac tgtttacatc tcaacttgaa 2760

ggaatgaagc agattgttaa attagatatt actgattgta agtctcttac ctccttacct 2820

attagcattc tgccgagtac cttgaagaga ataagaatag ctttttgtgg ggagctgaaa 2880

ttggaggcgt cgatgaatgc tatgtttctc gagaaattgt ctctagtaaa atgtgattct 2940

cctgagttgg tcccaagagc acgcaatttg agtgtaagaa gttgcaacaa ccttactagg 3000

cttttgattc ctactgccac tgaaagactc agtattagag attatgataa tcttgaaata 3060

ctttcagtgg cacgtgggac tcagatgaca tcattgaata tttacgactg caagaagctg 3120

aagtcgctgc cagaacatat gcaggaactc cttccatctc ttaagaaact ggttgtgcaa 3180

gcttgtccag aaatagagtc ctttcctgaa ggaggattgc ccttcaattt acaagccctt 3240

tcaatctgga attgcaagaa actggtgaat ggccgaaaag agtggcattt acagagactc 3300

ccctctctca tagatttaac catctaccat gatggtagcg atgaagaggt tcttgctggt 3360

gaaaaatggg agttgccttg ctctattcga aggcttacta tatccaatct gaaaacatta 3420

agcagccaac ttctcaaaag cctcacctcc cttgagtacc tagatgctag agagttgcct 3480

caaattcagt cactgctgga agaaggcctt cctttctctc tttctgagct aatattattt 3540

agtaatcatg atcttcattc actaccgaca gaaggtcttc agcatctcac gtggcttcga 3600

cgtctagaga ttgtgggttg ccctagtctc caatctcttc ccgaatcggg gttgccctcc 3660

tccctctctg agctgggcat ttggaattgc tctaatcttc aatctcttcc cgaatcaggg 3720

atgccccctt ccatctctaa actacgcatt tccgaatgcc cattgctcaa accactccta 3780

gaatttaaca agggggatta ctggccaaaa attgctcata ttcccaccat atatattgat 3840

aaggaatact ag 3852

<210> 98
<211> 1283
<212> PRT
<213> Solanum demissum

<400> 98
Met Glu Ile Gly Leu Ala Val Gly Gly Ala Phe Leu Ser Ser Ala Leu
1               5                   10                  15
Asn Val Leu Phe Asp Arg Leu Ala Pro Asn Ser Asp Leu Leu Lys Met
            20                  25                  30
Phe Lys Arg Asp Lys Arg Asp Val Arg Leu Leu Lys Lys Leu Arg Met
        35                  40                  45
Thr Leu Leu Gly Leu Gln Ala Val Leu Ser Asp Ala Glu Asn Lys Gln
    50                  55                  60
Ala Ser Asn Pro Tyr Val Ser Gln Trp Leu Asn Glu Leu Gln Asp Ala
65                  70                  75                  80
Val Asp Gly Ala Glu Asn Leu Ile Glu Glu Val Asn Tyr Glu Val Leu
                85                  90                  95
Arg Leu Lys Val Glu Gly Gln Cys Gln Asn Leu Gly Glu Thr Ser Asn
            100                 105                 110
Gln Gln Val Ser Asp Cys Asn Leu Cys Leu Ser Asp Asp Phe Phe Leu
        115                 120                 125
Asn Ile Lys Glu Lys Leu Glu Glu Thr Ile Glu Thr Leu Glu Glu Leu
    130                 135                 140
Glu Lys Gln Ile Gly Arg Leu Asp Leu Thr Lys Tyr Leu Asp Ser Gly
145                 150                 155                 160
Lys Gln Glu Thr Arg Glu Ser Ser Thr Ser Val Val Asp Glu Ser Asp
                165                 170                 175
Ile Leu Gly Arg Gln Asn Glu Ile Glu Gly Leu Ile Asp Arg Leu Leu
            180                 185                 190
Ser Glu Asp Gly Lys Asn Leu Thr Val Val Pro Val Val Gly Met Gly
        195                 200                 205
Gly Val Gly Lys Thr Thr Leu Ala Lys Ala Val Tyr Asn Asp Glu Lys
    210                 215                 220
Val Lys Asn His Phe Gly Phe Lys Ala Trp Ile Cys Val Ser Glu Pro
225                 230                 235                 240
Tyr Asp Ile Leu Arg Ile Thr Lys Glu Leu Leu Gln Glu Phe Gly Leu
                245                 250                 255
Met Val Asp Asn Asn Leu Asn Gln Leu Gln Val Lys Leu Lys Glu Ser
            260                 265                 270
Leu Lys Gly Lys Lys Phe Leu Ile Val Leu Asp Asp Val Trp Asn Glu
        275                 280                 285
Asn Tyr Lys Glu Trp Asp Asp Leu Arg Asn Leu Phe Val Gln Gly Asp
    290                 295                 300
Val Gly Ser Lys Ile Ile Val Thr Thr Arg Lys Glu Ser Val Ala Leu
305                 310                 315                 320
Met Met Gly Cys Gly Ala Ile Asn Val Gly Thr Leu Ser Ser Glu Val
                325                 330                 335
Ser Trp Asp Leu Phe Lys Arg His Ser Phe Glu Asn Arg Asp Pro Lys
            340                 345                 350
Glu His Pro Glu Leu Glu Glu Ile Gly Ile Gln Ile Ala Tyr Lys Cys
        355                 360                 365
Lys Gly Leu Pro Leu Ala Leu Lys Ala Leu Ala Gly Ile Leu Arg Ser
    370                 375                 380
Lys Ser Glu Val Asp Glu Trp Arg His Ile Leu Arg Ser Glu Ile Trp
385                 390                 395                 400
Glu Leu Gln Ser Arg Ser Asn Gly Ile Leu Pro Ala Leu Met Leu Ser
                405                 410                 415
Tyr Asn Asp Leu Pro Pro Gln Leu Lys Arg Cys Phe Ala Phe Cys Ala
            420                 425                 430
Ile Tyr Pro Lys Asp Tyr Leu Phe Cys Lys Glu Gln Val Val His Leu
        435                 440                 445
Trp Ile Ala Asn Gly Leu Val Gln Gln Leu His Ser Ala Asn Gln Tyr
    450                 455                 460
Phe Leu Glu Leu Arg Ser Arg Ser Leu Phe Glu Lys Val Arg Glu Ser
465                 470                 475                 480

Ser Lys Trp Asn Ser Gly Glu Phe Leu Met His Asp Leu Val Asn Asp
                485                 490                 495
Leu Ala Gln Ile Ala Ser Ser Asn Leu Cys Met Arg Leu Glu Glu Asn
            500                 505                 510
Gln Gly Ser His Met Leu Glu Arg Thr Arg His Leu Ser Tyr Ser Met
        515                 520                 525
Gly Asp Gly Asp Phe Gly Lys Leu Lys Thr Leu Asn Lys Leu Glu Gln
    530                 535                 540
Leu Arg Thr Leu Leu Pro Ile Asn Ile Gln Arg Arg Pro Cys His Leu
545                 550                 555                 560
Lys Lys Arg Met Leu His Asp Ile Phe Pro Arg Leu Ile Ser Leu Arg
                565                 570                 575
Ala Leu Ser Leu Ser Pro Tyr Asp Ile Glu Glu Leu Pro Asn Asp Leu
            580                 585                 590
Phe Ile Lys Leu Lys His Leu Lys Phe Leu Asp Leu Ser Trp Thr Gln
        595                 600                 605
Ile Lys Lys Leu Pro Asp Ser Ile Cys Glu Leu Tyr Ser Leu Glu Ile
    610                 615                 620
Leu Ile Leu Ser His Cys Ser His Leu Asn Glu Pro Pro Leu Gln Met
625                 630                 635                 640
Glu Lys Leu Ile Asn Leu His His Leu Asp Val Ser Asp Ala Tyr Phe
                645                 650                 655
Leu Lys Thr Pro Leu His Val Ser Lys Leu Lys Asn Leu His Val Leu
            660                 665                 670
Val Gly Ala Lys Phe Phe Leu Thr Gly Ser Ser Gly Leu Arg Ile Glu
        675                 680                 685
Asp Leu Gly Glu Leu His Asn Leu Tyr Gly Ser Leu Ser Ile Leu Glu
    690                 695                 700
Leu Gln His Val Val Asp Arg Arg Glu Ser Leu Lys Ala Asn Met Arg
705                 710                 715                 720
Glu Lys Lys His Val Glu Arg Leu Ser Leu Glu Trp Gly Gly Ser Phe
                725                 730                 735
Ala Asp Asn Ser Gln Thr Glu Arg Asp Ile Leu Asp Glu Leu Gln Pro
            740                 745                 750
Asn Thr Asn Ile Lys Glu Leu Arg Ile Thr Gly Tyr Arg Gly Thr Lys
        755                 760                 765
Phe Pro Asn Trp Leu Ala Asp His Ser Phe His Lys Leu Ile Glu Met
    770                 775                 780
Ser Leu Ser Tyr Cys Lys Asp Cys Asp Ser Leu Pro Ala Leu Gly Gln
785                 790                 795                 800
Leu Pro Cys Leu Lys Ser Leu Thr Ile Arg Gly Met His Gln Ile Thr
                805                 810                 815
Glu Val Ser Glu Glu Phe Tyr Gly Arg Phe Ser Ser Thr Lys Pro Phe
            820                 825                 830
Asn Ser Leu Glu Lys Leu Glu Phe Ala Glu Met Pro Glu Trp Lys Gln
        835                 840                 845
Trp His Val Leu Gly Lys Gly Glu Phe Pro Val Leu Glu Glu Leu Leu
    850                 855                 860
Ile Tyr Arg Cys Pro Lys Leu Ile Gly Lys Leu Pro Glu Asn Val Ser
865                 870                 875                 880
Ser Leu Arg Arg Leu Arg Ile Leu Lys Cys Pro Glu Leu Ser Leu Glu
                885                 890                 895
Thr Pro Ile Gln Leu Ser Asn Leu Lys Glu Phe Glu Val Ala Asp Ala
            900                 905                 910
Gln Leu Phe Thr Ser Gln Leu Glu Gly Met Lys Gln Ile Val Lys Leu
        915                 920                 925
Asp Ile Thr Asp Cys Lys Ser Leu Thr Ser Leu Pro Ile Ser Ile Leu
    930                 935                 940
Pro Ser Thr Leu Lys Arg Ile Arg Ile Ala Phe Cys Gly Glu Leu Lys
945                 950                 955                 960
Leu Glu Ala Ser Met Asn Ala Met Phe Leu Glu Lys Leu Ser Leu Val
                965                 970                 975
Lys Cys Asp Ser Pro Glu Leu Val Pro Arg Ala Arg Asn Leu Ser Val

980                 985                 990
Arg Ser Cys Asn Asn Leu Thr Arg Leu Leu Ile Pro Thr Ala Thr Glu
        995                 1000                1005
Arg Leu Ser Ile Arg Asp Tyr Asp Asn Leu Glu Ile Leu Ser Val Ala
    1010                1015                1020
Arg Gly Thr Gln Met Thr Ser Leu Asn Ile Tyr Asp Cys Lys Lys Leu
1025                1030                1035                1040
Lys Ser Leu Pro Glu His Met Gln Glu Leu Leu Pro Ser Leu Lys Lys
                1045                1050                1055
Leu Val Val Gln Ala Cys Pro Glu Ile Glu Ser Phe Pro Glu Gly Gly
            1060                1065                1070
Leu Pro Phe Asn Leu Gln Ala Leu Ser Ile Trp Asn Cys Lys Lys Leu
        1075                1080                1085
Val Asn Gly Arg Lys Glu Trp His Leu Gln Arg Leu Pro Ser Leu Ile
    1090                1095                1100
Asp Leu Thr Ile Tyr His Asp Gly Ser Asp Glu Glu Val Leu Ala Gly
1105                1110                1115                1120
Glu Lys Trp Glu Leu Pro Cys Ser Ile Arg Arg Leu Thr Ile Ser Asn
                1125                1130                1135
Leu Lys Thr Leu Ser Ser Gln Leu Leu Lys Ser Leu Thr Ser Leu Glu
            1140                1145                1150
Tyr Leu Asp Ala Arg Glu Leu Pro Gln Ile Gln Ser Leu Leu Glu Glu
        1155                1160                1165
Gly Leu Pro Phe Ser Leu Ser Glu Leu Ile Leu Phe Ser Asn His Asp
    1170                1175                1180
Leu His Ser Leu Pro Thr Glu Gly Leu Gln His Leu Thr Trp Leu Arg
1185                1190                1195                1200
Arg Leu Glu Ile Val Gly Cys Pro Ser Leu Gln Ser Leu Pro Glu Ser
                1205                1210                1215
Gly Leu Pro Ser Ser Leu Ser Glu Leu Gly Ile Trp Asn Cys Ser Asn
            1220                1225                1230
Leu Gln Ser Leu Pro Glu Ser Gly Met Pro Pro Ser Ile Ser Lys Leu
        1235                1240                1245
Arg Ile Ser Glu Cys Pro Leu Leu Lys Pro Leu Leu Glu Phe Asn Lys
    1250                1255                1260
Gly Asp Tyr Trp Pro Lys Ile Ala His Ile Pro Thr Ile Tyr Ile Asp
1265                1270                1275                1280
Lys Glu Tyr


<210> 99
<211> 447
<212> DNA
<213> Artificial Sequence


<220>
<223> cDNA

<400> 99
atgcgagcct actttgtcct actggttgct gcaactgcga tcctgaccta tggaggagcg      60

acggccacgt actcgacttc aaagggggag atgaatttaa ccggaactgt cgaaaataac     120

cgcccgactc gctctcttcg tgttgcaccc agtggcggca atggtgaaga aagatcgtgg     180

tcaaccattt acggaatttc tcgatcgaag gcggagactg tgagagattg gttgatgccc     240

cggcttaacc aaggaatgga cgtacaggcg ctcgccagag aaatggggat tacttcgaga     300

caggcggcta cgcagcatca gaactgggac gctctcgtga agtacctgaa gatgtataat     360

tacgctgtaa ggggcgaaaa gatgtcgaaa tcgatggctg agagcgtact cctccataac     420

gtcctgaccg caaagaacaa tttctaa                                          447

<210> 100
<211> 148
<212> PRT
<213> Phytophthora infestans

<400> 100
Met Arg Ala Tyr Phe Val Leu Leu Val Ala Ala Thr Ala Ile Leu Thr
1               5                   10                  15
Tyr Gly Gly Ala Thr Ala Thr Tyr Ser Thr Ser Lys Gly Glu Met Asn
            20                  25                  30
Leu Thr Gly Thr Val Glu Asn Asn Arg Pro Thr Arg Ser Leu Arg Val
        35                  40                  45
Ala Pro Ser Gly Gly Asn Gly Glu Glu Arg Ser Trp Ser Thr Ile Tyr
    50                  55                  60
Gly Ile Ser Arg Ser Lys Ala Glu Thr Val Arg Asp Trp Leu Met Pro
65                  70                  75                  80
Arg Leu Asn Gln Gly Met Asp Val Gln Ala Leu Ala Arg Glu Met Gly
                85                  90                  95
Ile Thr Ser Arg Gln Ala Ala Thr Gln His Gln Asn Trp Asp Ala Leu
            100                 105                 110
Val Lys Tyr Leu Lys Met Tyr Asn Tyr Ala Val Arg Gly Glu Lys Met
        115                 120                 125
Ser Lys Ser Met Ala Glu Ser Val Leu Leu His Asn Val Leu Thr Ala
    130                 135                 140
Lys Asn Asn Phe
145

<210> 101
<211> 2877
<212> DNA
<213> Artificial Sequence

<220>
<223> cDNA

<400> 101
atggatattg tcaccggtgc catttccaac ctgattccca agttggggga gctgctcacg      60

gaggagttca agctgcacaa gggtgtcaag aaaaatattg aggacctcgg gaaggagctt     120

gagagcatga acgctgccct catcaagatt ggtgaggtgc cgagggagca gctcgacagc     180

caagacaagc tctgggccga tgaagtcaga gagctctcct acgtcattga ggatgtcgtc     240

gacaagttcc tcgtacaggt tgatggcatt cagtttgatg ataacaacaa caaatttaag     300

gggttcatga agaggacgac cgagttgttg aagaaagtca agcataagca tgggatagct     360

cacgcgatca aggacatcca agagcaactc caaaaggtgg ctgataggcg tgacaggaac     420

aaggtatttg ttcctcatcc tacgagaaca attgctattg acccttgcct tcgagctttg     480

tatgctgaag cgacagagct agttggcata tatggaaaga gggatcaaga cctcatgagg     540

ttgctttcca tggagggcga tgatgcctct aataagagac tgaagaaggt ctccattgtt     600

ggatttggag ggttgggcaa gaccactctt gctagagcgg tatacgagaa gattaaaggt     660

gatttcgatt gtcgggcttt tgttccggtc ggtcagaacc ctcacatgaa gaaggtttta 720

agggatatcc tcattgatct cggaaatcct cactcagatc ttgcgatgct ggatgccaat 780

cagcttatta aaaaacttcg tgaatttcta gagaacaaaa ggtatcttgt cataattgat 840

gatatatggg atgaaaaatt atgggaaggc atcaactttg ctttctccaa taggaataat 900

ctaggcagtc ggctaatcac cacaacccgc attgtcagtg tctctaattc atgttgctca 960

tcacatggtg attcggttta tcaaatggaa ccactttctg ttgatgactc cagaatactc 1020

ttctggaaaa gaatatttcc agatgagaat ggatgtctaa atgaatttga acaagtgtcg 1080

agagatattc taaagaaatg tggtggggta ccactagcca taattaccat agctagtgct 1140

ttggccggtg accagaagat gaaaccaaag tgtgagtggg atattctcct tcagtccctt 1200

ggctctggac taacagaaga taacagttta gaggagatgc ggagaatact ctctttcagc 1260

tattctaatc taccttctca tctgaaaact tgtctactgt atctatgtat atatccagaa 1320

gatagcaaga ttcatagaga tgaactgata tggaagtggg tggccgaagg atttgtccac 1380

catgaaaacc aaggaaatag cttgtatttg ctcggattaa attacttcaa ccagctcatt 1440

aatagaagta tgatccagcc catatatggt tttaatgacg aggtatatgt atgtcgtgta 1500

catgatatgg ttctggacct tatctgcaac ttgtcacgtg aagcaaaatt tgtgaatcta 1560

ttggatggca gtgggaatag catgtcttca cagggtaatt gtcgccgtct gtcccttcaa 1620

aaaagaaatg aagatcatca agccaaacct atcacagata tcaagagtat gtcacgagtg 1680

aggtcaatta ctatctttcc acctgctatt gaagtcatgc catctctttc aaggtttgac 1740

gttttacgtg tacttgatct gtcacgatgt aatcttgggg agaatagcag cctgcagctt 1800

aacctgaagg atgttggaca tttaactcac ctaaggtacc ttggtctaga aggtaccaac 1860

atcagtaagc tccctgctga gataggaaaa ctgcagtttt tggaggtgtt ggatcttgga 1920

aacaatcata atctaaagga attgccgtcc actgtttgta atttcagaag attaatctac 1980

ctaaatttat ttgggtgtcc ggtggttcct ccagttggtg tgttgcaaaa tctgacatcc 2040

atagaagtgt tgagggggat cttggtctct gtgaacatta ttgcacaaga gcttggcaac 2100

ctggaaaggc tgagggtgct tgatatttgc ttcagggatg gtagtttgga tttgtataaa 2160

gatttcgtga agtctctgtg caacctacat cacatcgaaa gtctacgtat tgagtgcaat 2220

tccagagaaa catcatcttt tgaactggtg gatctcttgg gagaacgctg ggtgcctcct 2280

gtacatttcc gtgaatttgt gtcatccatg cctagccaac tctctgcact gcgagggtgg 2340

ataaagagag acccctccca tctctcgaac ctctccgagt taatcctctc gtcagtgaag 2400

gacgtgcagc aggatgacgt ggaaatcatt ggggggttgt tgtgccttcg tcgtctcttt 2460

ataataacga gcaccgacca aacgcaacgg ctgctagtca tccgtgcaga tgggttccgc 2520

tgtacggttg actttcgatt ggattgtgga tctgccacgc agatattgtt tgaaccagga 2580

gctttgccaa gggcggtaag agtttggttc agccttggcg tgcgggtgac gaaagaggat 2640

ggtaaccgtg gcttcgactt gggcctgcag gggaacctgt tctcccttcg agagtttgtc 2700

tctgtttata tgtattgtgg tggagcgagg gttggggagg caaaggaagc ggaggctgcg 2760

gtgaggcgtg ccctggaagc tcatcccagc catccccgga tttatattca gatgaggccg 2820

catatagcaa aaggtgctca tgatgacgat ttgtgtgagg acgaggagga gaactga 2877

<210> 102
<211> 958
<212> PRT
<213> Hordeum vulgare

<400> 102
Met Asp Ile Val Thr Gly Ala Ile Ser Asn Leu Ile Pro Lys Leu Gly
1               5                   10                  15
Glu Leu Leu Thr Glu Glu Phe Lys Leu His Lys Gly Val Lys Lys Asn
            20                  25                  30
Ile Glu Asp Leu Gly Lys Glu Leu Glu Ser Met Asn Ala Ala Leu Ile
        35                  40                  45
Lys Ile Gly Glu Val Pro Arg Glu Gln Leu Asp Ser Gln Asp Lys Leu
    50                  55                  60
Trp Ala Asp Glu Val Arg Glu Leu Ser Tyr Val Ile Glu Asp Val Val
65                  70                  75                  80
Asp Lys Phe Leu Val Gln Val Asp Gly Ile Gln Phe Asp Asp Asn Asn
                85                  90                  95
Asn Lys Phe Lys Gly Phe Met Lys Arg Thr Thr Glu Leu Leu Lys Lys
            100                 105                 110
Val Lys His Lys His Gly Ile Ala His Ala Ile Lys Asp Ile Gln Glu
        115                 120                 125
Gln Leu Gln Lys Val Ala Asp Arg Arg Asp Arg Asn Lys Val Phe Val
    130                 135                 140
Pro His Pro Thr Arg Thr Ile Ala Ile Asp Pro Cys Leu Arg Ala Leu
145                 150                 155                 160
Tyr Ala Glu Ala Thr Glu Leu Val Gly Ile Tyr Gly Lys Arg Asp Gln
                165                 170                 175
Asp Leu Met Arg Leu Leu Ser Met Glu Gly Asp Asp Ala Ser Asn Lys
            180                 185                 190
Arg Leu Lys Lys Val Ser Ile Val Gly Phe Gly Gly Leu Gly Lys Thr
        195                 200                 205
Thr Leu Ala Arg Ala Val Tyr Glu Lys Ile Lys Gly Asp Phe Asp Cys
    210                 215                 220
Arg Ala Phe Val Pro Val Gly Gln Asn Pro His Met Lys Lys Val Leu
225                 230                 235                 240
Arg Asp Ile Leu Ile Asp Leu Gly Asn Pro His Ser Asp Leu Ala Met
                245                 250                 255
Leu Asp Ala Asn Gln Leu Ile Lys Lys Leu Arg Glu Phe Leu Glu Asn
            260                 265                 270
Lys Arg Tyr Leu Val Ile Ile Asp Asp Ile Trp Asp Glu Lys Leu Trp
        275                 280                 285
Glu Gly Ile Asn Phe Ala Phe Ser Asn Arg Asn Asn Leu Gly Ser Arg
    290                 295                 300
Leu Ile Thr Thr Thr Arg Ile Val Ser Val Ser Asn Ser Cys Cys Ser
305                 310                 315                 320
Ser His Gly Asp Ser Val Tyr Gln Met Glu Pro Leu Ser Val Asp Asp
                325                 330                 335
Ser Arg Ile Leu Phe Trp Lys Arg Ile Phe Pro Asp Glu Asn Gly Cys

            340                 345                 350
Leu Asn Glu Phe Glu Gln Val Ser Arg Asp Ile Leu Lys Lys Cys Gly
        355                 360                 365
Gly Val Pro Leu Ala Ile Ile Thr Ile Ala Ser Ala Leu Ala Gly Asp
    370                 375                 380
Gln Lys Met Lys Pro Lys Cys Glu Trp Asp Ile Leu Leu Gln Ser Leu
385                 390                 395                 400
Gly Ser Gly Leu Thr Glu Asp Asn Ser Leu Glu Glu Met Arg Arg Ile
                405                 410                 415
Leu Ser Phe Ser Tyr Ser Asn Leu Pro Ser His Leu Lys Thr Cys Leu
            420                 425                 430
Leu Tyr Leu Cys Ile Tyr Pro Glu Asp Ser Lys Ile His Arg Asp Glu
        435                 440                 445
Leu Ile Trp Lys Trp Val Ala Glu Gly Phe Val His His Glu Asn Gln
    450                 455                 460
Gly Asn Ser Leu Tyr Leu Leu Gly Leu Asn Tyr Phe Asn Gln Leu Ile
465                 470                 475                 480
Asn Arg Ser Met Ile Gln Pro Ile Tyr Gly Phe Asn Asp Glu Val Tyr
                485                 490                 495
Val Cys Arg Val His Asp Met Val Leu Asp Leu Ile Cys Asn Leu Ser
            500                 505                 510
Arg Glu Ala Lys Phe Val Asn Leu Leu Asp Gly Ser Gly Asn Ser Met
        515                 520                 525
Ser Ser Gln Gly Asn Cys Arg Arg Leu Ser Leu Gln Lys Arg Asn Glu
    530                 535                 540
Asp His Gln Ala Lys Pro Ile Thr Asp Ile Lys Ser Met Ser Arg Val
545                 550                 555                 560
Arg Ser Ile Thr Ile Phe Pro Pro Ala Ile Glu Val Met Pro Ser Leu
                565                 570                 575
Ser Arg Phe Asp Val Leu Arg Val Leu Asp Leu Ser Arg Cys Asn Leu
            580                 585                 590
Gly Glu Asn Ser Ser Leu Gln Leu Asn Leu Lys Asp Val Gly His Leu
        595                 600                 605
Thr His Leu Arg Tyr Leu Gly Leu Glu Gly Thr Asn Ile Ser Lys Leu
    610                 615                 620
Pro Ala Glu Ile Gly Lys Leu Gln Phe Leu Glu Val Leu Asp Leu Gly
625                 630                 635                 640
Asn Asn His Asn Leu Lys Glu Leu Pro Ser Thr Val Cys Asn Phe Arg
                645                 650                 655
Arg Leu Ile Tyr Leu Asn Leu Phe Gly Cys Pro Val Val Pro Pro Val
            660                 665                 670
Gly Val Leu Gln Asn Leu Thr Ser Ile Glu Val Leu Arg Gly Ile Leu
        675                 680                 685
Val Ser Val Asn Ile Ile Ala Gln Glu Leu Gly Asn Leu Glu Arg Leu
    690                 695                 700
Arg Val Leu Asp Ile Cys Phe Arg Asp Gly Ser Leu Asp Leu Tyr Lys
705                 710                 715                 720
Asp Phe Val Lys Ser Leu Cys Asn Leu His His Ile Glu Ser Leu Arg
                725                 730                 735
Ile Glu Cys Asn Ser Arg Glu Thr Ser Ser Phe Glu Leu Val Asp Leu
            740                 745                 750
Leu Gly Glu Arg Trp Val Pro Pro Val His Phe Arg Glu Phe Val Ser
        755                 760                 765
Ser Met Pro Ser Gln Leu Ser Ala Leu Arg Gly Trp Ile Lys Arg Asp
    770                 775                 780
Pro Ser His Leu Ser Asn Leu Ser Glu Leu Ile Leu Ser Ser Val Lys
785                 790                 795                 800
Asp Val Gln Gln Asp Asp Val Glu Ile Ile Gly Gly Leu Leu Cys Leu
                805                 810                 815
Arg Arg Leu Phe Ile Ile Thr Ser Thr Asp Gln Thr Gln Arg Leu Leu
            820                 825                 830
Val Ile Arg Ala Asp Gly Phe Arg Cys Thr Val Asp Phe Arg Leu Asp
        835                 840                 845

Cys Gly Ser Ala Thr Gln Ile Leu Phe Glu Pro Gly Ala Leu Pro Arg
    850                 855                 860
Ala Val Arg Val Trp Phe Ser Leu Gly Val Arg Val Thr Lys Glu Asp
865                 870                 875                 880
Gly Asn Arg Gly Phe Asp Leu Gly Leu Gln Gly Asn Leu Phe Ser Leu
                885                 890                 895
Arg Glu Phe Val Ser Val Tyr Met Tyr Cys Gly Gly Ala Arg Val Gly
            900                 905                 910
Glu Ala Lys Glu Ala Glu Ala Ala Val Arg Arg Ala Leu Glu Ala His
        915                 920                 925
Pro Ser His Pro Arg Ile Tyr Ile Gln Met Arg Pro His Ile Ala Lys
    930                 935                 940
Gly Ala His Asp Asp Asp Leu Cys Glu Asp Glu Glu Glu Asn
945                 950                 955

<210> 103
<211> 357
<212> DNA
<213> Artificial Sequence

<220>
<223> cDNA

<400> 103
atgaaatttt atcactcatc atttgtttca aacttgacaa tactatcttt gttcttgaac      60

attaatggcg ctttttcacg aacgtggcaa tgcccaagcg gtgacatact ttatgaagaa     120

gatgtttatt cacatacaaa tatgaattcc tttgaacact ttccgtttaa aactatagag     180

gatgaagtct atgaaaacac tagtttgagt cgcggtgtcg tttacaaggt attggatgtg     240

catccttcgt catctagcgc aacaactttt gctgtcttta cttcaaataa acgagtggca     300

gtagtatacc aacagggcta tcgaaataac cgaaaattca ttgaagaatg cacctag       357

<210> 104
<211> 118
<212> PRT
<213> Blumeria graminis

<400> 104
Met Lys Phe Tyr His Ser Ser Phe Val Ser Asn Leu Thr Ile Leu Ser
1               5                   10                  15
Leu Phe Leu Asn Ile Asn Gly Ala Phe Ser Arg Thr Trp Gln Cys Pro
            20                  25                  30
Ser Gly Asp Ile Leu Tyr Glu Glu Asp Val Tyr Ser His Thr Asn Met
        35                  40                  45
Asn Ser Phe Glu His Phe Pro Phe Lys Thr Ile Glu Asp Glu Val Tyr
    50                  55                  60
Glu Asn Thr Ser Leu Ser Arg Gly Val Val Tyr Lys Val Leu Asp Val
65                  70                  75                  80
His Pro Ser Ser Ser Ser Ala Thr Thr Phe Ala Val Phe Thr Ser Asn
                85                  90                  95
Lys Arg Val Ala Val Val Tyr Gln Gln Gly Tyr Arg Asn Asn Arg Lys
            100                 105                 110
Phe Ile Glu Glu Cys Thr
        115

<210> 105

<211> 2880
<212> DNA
<213> Artificial Sequence

<220>
<223> cDNA

<400> 105
atggatattg tcaccggtgc catttccaac ctgattccca agttggggga gctactcacg 60

gaggagttca agctgcacaa gggtgtcaag aaaaatattg aggacctcgg gaaggagctt 120

gagagcatga acgctgccct catcaagatt ggtgaggtgc cgagggagca gctcgacagc 180

caagacaagc tctgggccga tgaggtcaga gagctctcct acgtcattga ggatgtcgtc 240

gacaagttcc tcgtacaggt tgatggcatt aagtctgatg ataacaacaa caaatttaag 300

gggctcatga agaggactac cgagttgttg aagaaagtca agcataagca tgggatagct 360

cacgcgatca aggacatcca agagcaactc caaaaggtgg ctgataggcg tgacaggaac 420

aaggtatttg ttcctcatcc tacgagaaca attgctattg acccttgcct tcgagctttg 480

tatgctgaag cgacagagct agttggcata tatggaaaga gggatcaagg cctcatgagg 540

ttgctttcca tggagggcga tgatgcctct aataagagac tgaagaaggt ctccattgtt 600

ggatttggag ggttgggcaa gaccactctt gctagagcgg tatacgagaa gattaaaggt 660

gatttcgatt gtcgggcatt tgttccggtc ggtcagaacc ctgacatgaa gaaggtttta 720

agggatatcc tcattgatct cggaaatcct cactcagatc ttgcgatgct ggatgccaat 780

cagcttatta aaaagcttca tgaatttcta gagaacaaaa ggtatcttgt cataattgat 840

gatatatggg atgaaaaatt gtgggaaggc atcaactttg ctttctccaa taggaataat 900

ctaggcagtc ggctaatcac cacaacccgc attgtcagtg tctctaattc atgttgctca 960

tcagatggtg attctgttta tcaaatggaa ccgctttctg ttgatgactc cagaatgctc 1020

ttctacaaaa gaatatttcc tgatgagaat gcatgtataa atgaatttga acaagtatcc 1080

agagatattc taaagaaatg tggtggggta ccactagcca taattactat agctagtgct 1140

ttggctggtg accagaagat gaaaccaaaa tgtgagtggg atattctcct tcggtccctt 1200

ggctctggac taacagaaga taacagttta gaggagatgc ggagaatact ctctttcagc 1260

tattctaatc taccttcgaa tctgaaaact tgtctactgt atctatgtgt atatccagaa 1320

gatagtatga tttctagaga taaactgata tggaagtggg tggccgaagg atttgtccac 1380

catgaaaatc aaggaaatag cctgtatttg ctcggattaa attacttcaa ccagctcatt 1440

aatagaagta tgatccagcc aatatataat tatagcggcg aggcatatgc ttgccgtgta 1500

catgatatgg ttctggacct tatctgcaac ttgtcaaatg aagcaaagtt tgtgaatcta 1560

ttggatggca ctgggaatag catgtcttca cagagtaatt gtcgccgttt gtcccttcag 1620

aaaagaaatg aagatcatca agccaggcct ttcacagata tcaagagtat gtcacgagtg 1680

aggtcaatta ctatctttcc atctgctatt gaagtcatgc catctctttc aaggtttgac 1740

gttttacgtg tacttgatct gtcacgatgt aatcttgggg agaatagcag catgcagctt 1800

aacctcaagg gtgttggaca tttaactcac ctaaggtacc ttggtctaga aggtaccaac 1860

atcagtaagc tccctgctga gataggaaaa ctgcagtttt tggaggtgtt ggatcttgaa 1920

aacaatcata atctaaagga attgccgtcc acagtttgta atttcagaag attaatctac 1980

ctaaatttag ttggctgtca ggtggttcct ccagttggtg tgttgcaaaa tctgacatcc 2040

atagaagtgt tgagtggtat cttggtctct ctgaacatta ttgcacaaga gcttggcaac 2100

ttgaaaaggc tgagggagct taacattctc ttcaatgatg gtagtttgga tttgtatgaa 2160

ggttttgtga agtctctttg caacctacat cacatcgaaa gcctaatcat tggttgcaat 2220

tctagagaaa catcatcttt tgaactgatg gatctcctgg gggaacgctg ggtgcctcct 2280

gtacatttcc gtgaatttgt gtcatccatg cctagccaac tctctgcact gcgagggtgg 2340

ataaagagag acccctccca tctctcgaac ctctccgagt taatcctcac atcagtgaag 2400

gaggtgcagc aggatgacgt ggtaatcatt ggggccttgt cgtcccttcg ccgtctctgc 2460

ataaagagca cctaccaaac acaacggctg ctagtcatcc ctgcagatgg gttccgctgt 2520

attgttggct ttcatttgga ttgtggatct gccacacaga tattgtttga gcctggagct 2580

ttgccgaggg cagaatcagt tgtgatcagt ctcggcgtgc gggtggcgaa agaggatggt 2640

aaccgtggct tcgacttggg cctgcagggg aacctgctct cccttcggcg ggatgtcttt 2700

gtttctatct attgtggtgg agcgagggtt ggggaggcaa aggaagcgga ggctgcggtg 2760

aggcgtgccc tcgacgccca tcccagccat cccccgattt attttgagat gaggccgcat 2820

atagcaaaag gtgctcatga tgatgatttg tgtgaggaga ggaggagaac tgatttctga 2880

<210> 106
<211> 959
<212> PRT
<213> Hordeum vulgare

<400> 106
   Met Asp Ile Val Thr Gly Ala Ile Ser Asn Leu Ile Pro Lys Leu Gly
   1 5 10 15
   Glu Leu Leu Thr Glu Glu Phe Lys Leu His Lys Gly Val Lys Lys Asn
   20 25 30
   Ile Glu Asp Leu Gly Lys Glu Leu Glu Ser Met Asn Ala Ala Leu Ile
   35 40 45
   Lys Ile Gly Glu Val Pro Arg Glu Gln Leu Asp Ser Gln Asp Lys Leu
   50 55 60
   Trp Ala Asp Glu Val Arg Glu Leu Ser Tyr Val Ile Glu Asp Val Val
   65 70 75 80
   Asp Lys Phe Leu Val Gln Val Asp Gly Ile Lys Ser Asp Asp Asn Asn
   85 90 95
   Asn Lys Phe Lys Gly Leu Met Lys Arg Thr Thr Glu Leu Leu Lys Lys
                   100                 105                 110
   Val Lys His Lys His Gly Ile Ala His Ala Ile Lys Asp Ile Gln Glu
           115                 120                 125
   Gln Leu Gln Lys Val Ala Asp Arg Arg Asp Arg Asn Lys Val Phe Val
       130                 135                 140
   Pro His Pro Thr Arg Thr Ile Ala Ile Asp Pro Cys Leu Arg Ala Leu
   145                 150                 155                 160
   Tyr Ala Glu Ala Thr Glu Leu Val Gly Ile Tyr Gly Lys Arg Asp Gln
                   165                 170                 175
   Gly Leu Met Arg Leu Leu Ser Met Glu Gly Asp Asp Ala Ser Asn Lys
               180                 185                 190
   Arg Leu Lys Lys Val Ser Ile Val Gly Phe Gly Gly Leu Gly Lys Thr
           195                 200                 205
   Thr Leu Ala Arg Ala Val Tyr Glu Lys Ile Lys Gly Asp Phe Asp Cys
       210                 215                 220
   Arg Ala Phe Val Pro Val Gly Gln Asn Pro Asp Met Lys Lys Val Leu
   225                 230                 235                 240
   Arg Asp Ile Leu Ile Asp Leu Gly Asn Pro His Ser Asp Leu Ala Met
                   245                 250                 255
   Leu Asp Ala Asn Gln Leu Ile Lys Lys Leu His Glu Phe Leu Glu Asn
               260                 265                 270
   Lys Arg Tyr Leu Val Ile Ile Asp Asp Ile Trp Asp Glu Lys Leu Trp
           275                 280                 285
   Glu Gly Ile Asn Phe Ala Phe Ser Asn Arg Asn Asn Leu Gly Ser Arg
       290                 295                 300
   Leu Ile Thr Thr Thr Arg Ile Val Ser Val Ser Asn Ser Cys Cys Ser
   305                 310                 315                 320
   Ser Asp Gly Asp Ser Val Tyr Gln Met Glu Pro Leu Ser Val Asp Asp
                   325                 330                 335
   Ser Arg Met Leu Phe Tyr Lys Arg Ile Phe Pro Asp Glu Asn Ala Cys
               340                 345                 350
   Ile Asn Glu Phe Glu Gln Val Ser Arg Asp Ile Leu Lys Lys Cys Gly
           355                 360                 365
   Gly Val Pro Leu Ala Ile Ile Thr Ile Ala Ser Ala Leu Ala Gly Asp
       370                 375                 380
   Gln Lys Met Lys Pro Lys Cys Glu Trp Asp Ile Leu Leu Arg Ser Leu
   385                 390                 395                 400
   Gly Ser Gly Leu Thr Glu Asp Asn Ser Leu Glu Glu Met Arg Arg Ile
                   405                 410                 415
   Leu Ser Phe Ser Tyr Ser Asn Leu Pro Ser Asn Leu Lys Thr Cys Leu
               420                 425                 430
   Leu Tyr Leu Cys Val Tyr Pro Glu Asp Ser Met Ile Ser Arg Asp Lys
           435                 440                 445
   Leu Ile Trp Lys Trp Val Ala Glu Gly Phe Val His His Glu Asn Gln
       450                 455                 460
   Gly Asn Ser Leu Tyr Leu Leu Gly Leu Asn Tyr Phe Asn Gln Leu Ile
   465                 470                 475                 480
   Asn Arg Ser Met Ile Gln Pro Ile Tyr Asn Tyr Ser Gly Glu Ala Tyr
                   485                 490                 495
   Ala Cys Arg Val His Asp Met Val Leu Asp Leu Ile Cys Asn Leu Ser
               500                 505                 510
   Asn Glu Ala Lys Phe Val Asn Leu Leu Asp Gly Thr Gly Asn Ser Met
           515                 520                 525
   Ser Ser Gln Ser Asn Cys Arg Arg Leu Ser Leu Gln Lys Arg Asn Glu
       530                 535                 540
   Asp His Gln Ala Arg Pro Phe Thr Asp Ile Lys Ser Met Ser Arg Val
   545                 550                 555                 560
   Arg Ser Ile Thr Ile Phe Pro Ser Ala Ile Glu Val Met Pro Ser Leu
                   565                 570                 575
   Ser Arg Phe Asp Val Leu Arg Val Leu Asp Leu Ser Arg Cys Asn Leu
               580                 585                 590
   Gly Glu Asn Ser Ser Met Gln Leu Asn Leu Lys Gly Val Gly His Leu
           595                 600                 605
   Thr His Leu Arg Tyr Leu Gly Leu Glu Gly Thr Asn Ile Ser Lys Leu
       610                 615                 620
   Pro Ala Glu Ile Gly Lys Leu Gln Phe Leu Glu Val Leu Asp Leu Glu
   625                 630                 635                 640
   Asn Asn His Asn Leu Lys Glu Leu Pro Ser Thr Val Cys Asn Phe Arg
                   645                 650                 655
   Arg Leu Ile Tyr Leu Asn Leu Val Gly Cys Gln Val Val Pro Pro Val
               660                 665                 670
   Gly Val Leu Gln Asn Leu Thr Ser Ile Glu Val Leu Ser Gly Ile Leu
           675                 680                 685
   Val Ser Leu Asn Ile Ile Ala Gln Glu Leu Gly Asn Leu Lys Arg Leu
       690                 695                 700
   Arg Glu Leu Asn Ile Leu Phe Asn Asp Gly Ser Leu Asp Leu Tyr Glu
   705                 710                 715                 720
   Gly Phe Val Lys Ser Leu Cys Asn Leu His His Ile Glu Ser Leu Ile
                   725                 730                 735
   Ile Gly Cys Asn Ser Arg Glu Thr Ser Ser Phe Glu Leu Met Asp Leu
               740                 745                 750
   Leu Gly Glu Arg Trp Val Pro Pro Val His Phe Arg Glu Phe Val Ser
           755                 760                 765
   Ser Met Pro Ser Gln Leu Ser Ala Leu Arg Gly Trp Ile Lys Arg Asp
       770                 775                 780
   Pro Ser His Leu Ser Asn Leu Ser Glu Leu Ile Leu Thr Ser Val Lys
   785                 790                 795                 800
   Glu Val Gln Gln Asp Asp Val Val Ile Ile Gly Ala Leu Ser Ser Leu
                   805                 810                 815
   Arg Arg Leu Cys Ile Lys Ser Thr Tyr Gln Thr Gln Arg Leu Leu Val
               820                 825                 830
   Ile Pro Ala Asp Gly Phe Arg Cys Ile Val Gly Phe His Leu Asp Cys
           835                 840                 845
   Gly Ser Ala Thr Gln Ile Leu Phe Glu Pro Gly Ala Leu Pro Arg Ala
       850                 855                 860
   Glu Ser Val Val Ile Ser Leu Gly Val Arg Val Ala Lys Glu Asp Gly
   865                 870                 875                 880
   Asn Arg Gly Phe Asp Leu Gly Leu Gln Gly Asn Leu Leu Ser Leu Arg
                   885                 890                 895
   Arg Asp Val Phe Val Ser Ile Tyr Cys Gly Gly Ala Arg Val Gly Glu
               900                 905                 910
   Ala Lys Glu Ala Glu Ala Ala Val Arg Arg Ala Leu Asp Ala His Pro
           915                 920                 925
   Ser His Pro Pro Ile Tyr Phe Glu Met Arg Pro His Ile Ala Lys Gly
       930                 935                 940
   Ala His Asp Asp Asp Leu Cys Glu Glu Arg Arg Arg Thr Asp Phe
   945                 950                 955

<210> 107
<211> 4022
<212> DNA
<213> Hordeum vulgare

<400> 107
atggatattg tcaccggtgc catttccaac ctgattccca agttggggga gctactcacg      60

gaggagttca agctgcacaa gggtgtcaag aaaaatattg aggacctcgg gaaggagctt     120

gagagcatga acgctgccct catcaagatt ggtgaggtgc cgagggagca gctcgacagc     180

caagacaagc tctgggccga tgaggtcaga gagctctcct acgtcattga ggatgtcgtc     240

gacaagttcc tcgtacaggt tgatggcatt aagtctgatg ataacaacaa caaatttaag     300

gggctcatga agaggactac cgagttgttg aagaaagtca agcataagca tgggatagct     360

cacgcgatca aggacatcca agagcaactc caaaaggtgg ctgataggcg tgacaggaac 420

aaggtatttg ttcctcatcc tacgagaaca attgctattg acccttgcct tcgagctttg 480

tatgctgaag cgacagagct agttggcata tatggaaaga gggatcaagg cctcatgagg 540

ttgctttcca tggagggcga tgatgcctct aataagagac tgaagaaggt ctccattgtt 600

ggatttggag ggttgggcaa gaccactctt gctagagcgg tatacgagaa gattaaaggt 660

gatttcgatt gtcgggcatt tgttccggtc ggtcagaacc ctgacatgaa gaaggtttta 720

agggatatcc tcattgatct cggaaatcct cactcagatc ttgcgatgct ggatgccaat 780

cagcttatta aaaagcttca tgaatttcta gagaacaaaa ggtatgcatc aatttagaaa 840

aaagtacact attatgtgat gtttgtttcc tatgctagtg gaacggatta gaatattttt 900

tttcattaag gtcaccttta ctggcataag cagttcacac taaacagtaa accttatagg 960

tgaaaatttt caggcatata tatatatata tatatatata tatatatata tatatatata 1020

tatatatata tgtttgattc tttccggctt aacaaaataa ttagcaagta cttcttgttg 1080

catttgttcc aacggctgaa tttattggca tcagtccaag aaatccatct aaacgtttta 1140

catttcacca aagtgtgtgt catgacagat gtaacaaata ataaaccaaa aggagaggaa 1200

ggaaagaggg agataaatgt tacaaaaatt taaatcaaac ttatttctac ctttctcctt 1260

acctacccag ttgtaaaaca catatatatt ttaaagagag gcaacatgcg ccaaaggctg 1320

cccttgaaaa ttcctaaaat attgtacatt tgactcatga ccaaacaaaa aggtaaattg 1380

tctcttcctt atcgcattat atttccatgc atgccttttt ctggaaactt actatcagca 1440

aaatttagac gaaaggatga tgccacataa tttcagtctc caagagattt gttagttgcc 1500

atatattaaa ttggtgtgcc aacctatacc tgggcctttt ttatgtatct acttgatcat 1560

ttgaacttct gtagttaatt gtattctatg aatgatcact catccaaaaa cttgctattt 1620

gtgtttcact ttcttgagtc ttgaatattt attcattttg ttcatcatac gattggaggc 1680

ccataatgga tgcttaatga gagtaagatt atcgagctcc aaacacatgc ttcttactag 1740

tgtttgaata tatagcctta tagatgtata gttcaaccca tagattcata tgaccctcag 1800

ctttctgatg tgtatatata accttacact gacactccga aataatgtag gtatcttgtc 1860

ataattgatg atatatggga tgaaaaattg tgggaaggca tcaactttgc tttctccaat 1920

aggaataatc taggcagtcg gctaatcacc acaacccgca ttgtcagtgt ctctaattca 1980

tgttgctcat cagatggtga ttctgtttat caaatggaac cgctttctgt tgatgactcc 2040

agaatgctct tctacaaaag aatatttcct gatgagaatg catgtataaa tgaatttgaa 2100

caagtatcca gagatattct aaagaaatgt ggtggggtac cactagccat aattactata 2160

gctagtgctt tggctggtga ccagaagatg aaaccaaaat gtgagtggga tattctcctt 2220

cggtcccttg gctctggact aacagaagat aacagtttag aggagatgcg gagaatactc 2280

tctttcagct attctaatct accttcgaat ctgaaaactt gtctactgta tctatgtgta 2340

tatccagaag atagtatgat ttctagagat aaactgatat ggaagtgggt ggccgaagga 2400

tttgtccacc atgaaaatca aggaaatagc ctgtatttgc tcggattaaa ttacttcaac 2460

cagctcatta atagaagtat gatccagcca atatataatt atagcggcga ggcatatgct 2520

tgccgtgtac atgatatggt tctggacctt atctgcaact tgtcaaatga agcaaagttt 2580

gtgaatctat tggatggcac tgggaatagc atgtcttcac agagtaattg tcgccgtttg 2640

tcccttcaga aaagaaatga agatcatcaa gccaggcctt tcacagatat caagagtatg 2700

tcacgagtga ggtcaattac tatctttcca tctgctattg aagtcatgcc atctctttca 2760

aggtttgacg ttttacgtgt acttgatctg tcacgatgta atcttgggga gaatagcagc 2820

atgcagctta acctcaaggg tgttggacat ttaactcacc taaggtacct tggtctagaa 2880

ggtaccaaca tcagtaagct ccctgctgag ataggaaaac tgcagttttt ggaggtgttg 2940

gatcttgaaa acaatcataa tctaaaggaa ttgccgtcca cagtttgtaa tttcagaaga 3000

ttaatctacc taaatttagt tggctgtcag gtggttcctc cagttggtgt gttgcaaaat 3060

ctgacatcca tagaagtgtt gagtggtatc ttggtctctc tgaacattat tgcacaagag 3120

cttggcaact tgaaaaggct gagggagctt aacattctct tcaatgatgg tagtttggat 3180

ttgtatgaag gttttgtgaa gtctctttgc aacctacatc acatcgaaag cctaatcatt 3240

ggttgcaatt ctagagaaac atcatctttt gaactgatgg atctcctggg ggaacgctgg 3300

gtgcctcctg tacatttccg tgaatttgtg tcatccatgc ctagccaact ctctgcactg 3360

cgagggtgga taaagagaga cccctcccat ctctcgaacc tctccgagtt aatcctcaca 3420

tcagtgaagg aggtgcagca ggatgacgtg gtaatcattg gggccttgtc gtcccttcgc 3480

cgtctctgca taaagagcac ctaccaaaca caacggctgc tagtcatccc tgcagatggg 3540

ttccgctgta ttgttggctt tcatttggat tgtggatctg ccacacagat attgtttgag 3600

cctggagctt tgccgagggc agaatcagtt gtgatcagtc tcggcgtgcg ggtggcgaaa 3660

gaggatggta accgtggctt cgacttgggc ctgcagggga acctgctctc ccttcggcgg 3720

gatgtctttg tttctatcta ttgtggtgga gcgagggttg gggaggcaaa ggaagcggag 3780

gctgcggtga ggcgtgccct cgacgcccat cccagccatc cccgatttta ttttgagatg 3840

aggccgcata tagcaaaagg tacgcatcct gcacctaact aattacttgt gcacttacac 3900

atgtgttttt ttctcaatga cggactaacc ttattacttt ctgcgtggat tttgatctct 3960

aaatctccca aggtgctcat gatgatgatt tgtgtgagga gaggaggaga actgatttct 4020

ga 4022

<210> 108
<211> 369
<212> DNA
<213> Artificial Sequence

<220>
<223> cDNA

<400> 108
atgaaaactt ttcaatttgc ttctattgtt gcaggactta gttttctgaa gacgacaatt      60

gctgctggcg atggttatat tacccttggt atggggtcaa ttcataaaaa tgatatctat     120

agagtcgccg aacatatgtg gacgatagat gcctacagtg ttccctcgaa taatcatggt     180

agctatccta tttttgggga agaaataaac ggctcggtaa cacggatatt tccaatagtc     240

tataatggcg acgactggcg tagtggagat ttttattatt cggttgaatc aaccgaagat     300

ttgagttata tcaaactccg ttacaatggt gcgagatatg agacctgtat ggtttcaagc     360

cctgaatag                                                             369

<210> 109
<211> 122
<212> PRT
<213> Blumeria graminis

<400> 109
Met Lys Thr Phe Gln Phe Ala Ser Ile Val Ala Gly Leu Ser Phe Leu
1               5                   10                  15
Lys Thr Thr Ile Ala Ala Gly Asp Gly Tyr Ile Thr Leu Gly Met Gly
            20                  25                  30
Ser Ile His Lys Asn Asp Ile Tyr Arg Val Ala Glu His Met Trp Thr
        35                  40                  45
Ile Asp Ala Tyr Ser Val Pro Ser Asn Asn His Gly Ser Tyr Pro Ile
    50                  55                  60
Phe Gly Glu Glu Ile Asn Gly Ser Val Thr Arg Ile Phe Pro Ile Val
65                  70                  75                  80
Tyr Asn Gly Asp Asp Trp Arg Ser Gly Asp Phe Tyr Tyr Ser Val Glu
                85                  90                  95
Ser Thr Glu Asp Leu Ser Tyr Ile Lys Leu Arg Tyr Asn Gly Ala Arg
            100                 105                 110
Tyr Glu Thr Cys Met Val Ser Ser Pro Glu
        115                 120

<210> 110
<211> 3654
<212> DNA
<213> Artificial Sequence

<220>
<223> cDNA

<400> 110
atggcactag aattggttgg tggtgctctt ctttctgctt tccttcaggt tgcgtttgag      60

aagcttgctt ctcctcaagt tctggacttc tttcgtggaa gaaagcttga tcaaaagctg     120

ctcaacaact tggaaatcaa gctgaactcc atacaggctc tggctaatga tgcagaacta 180

aagcagttca gagatccact tgtcagaaac tggcttctta aggtcaaaga tgctgtgttt 240

gacgcagagg atatcttgga tgaaatacaa catgagatct ccaaatgcca ggtcgaagct 300

gaagctgaag ctgagtctca aacctgcact tgcaaggtac caaatttctt caaatcttct 360

cctgctagtt cctttaacag ggaaattaaa tccaggatgg aagaaatcct tgatagatta 420

gatcttctct caagccaaaa ggatgatcta ggcttgaaaa attctagtgg tgttggcgtt 480

ggatcagaat tgggtagtgc agtaccacag atatcacaat caacatcttc agttgttgaa 540

agtgatattt atggcagaga taaagacaaa aaaatgattt ttgattggct gacatctgac 600

aatggcaatc ctaaccagcc atcgatactt tctattgtgg gcatgggtgg gatgggtaag 660

accacacttg ctcaacatgt attcaatgac cctaggatcg aggaggctag atttgatgtc 720

aaagcttggg tctgtgtttc agatgatttt gatgctttca gggtaacaag aacaattctt 780

gaggccatca ctaaatcgac tgatgatagt agagacctag agatggttca cggaagattg 840

aaagaaaaat tgacggggaa gagatttctt cttgttttgg atgacgtttg gaacgaaaac 900

cgacttaaat gggaagctgt gctaaaacat cttggttttg gggctcaggg gagtagaatt 960

attgccacca cacgtagtaa ggaagttgct tctaccatga ggtcaaaaga acacctcctg 1020

gagcaattac aagaagatca ttgctggaag ttgtttgcta aacatgcatt ccaagatgat 1080

aatattcaac caaatccaga ttgcaaggag attggtatga agatagttga aaaatgtaaa 1140

ggacttcctc tggccttgaa aacaatggga agtctattac acaacaagtc atctgttacg 1200

gaatggaaaa gcatattgca gagcgagata tgggaatttt caacagagcg tagtgatatt 1260

gtacctgctt tagcactaag ctatcaccat cttccttctc atctcaagag atgttttgct 1320

tattgtgcct tattccccaa agattatgag tttgataagg agtgcttaat acaattgtgg 1380

atggctgaaa aatttctaca atgttctcaa cagggtaaga gtccaggaga agttggtgaa 1440

caatacttca atgatctatt atcaaggtgc ttctttcaac aatcaagcaa cacagagaga 1500

acagattttg tcatgcatga ccttctcaac gatttggcaa gatttatttg tggggacatt 1560

tgtttcagat tggatgggaa tcaaacaaaa ggtacaccaa aagcaacccg tcatttttta 1620

attgacgtta aatgttttga tgggtttgga actctatgtg acacaaaaaa gttacgtact 1680

tatatgccaa caagttacaa atattgggat tgcgagatgt caatacacga attgttctcc 1740

aagtttaatt acttacgtgt cttatctctg tttgattgtc atgacctaag agaggtgcct 1800

gactctgtgg gtaatctcaa atatctccgt tcgttagact tatccaatac taaaatagaa 1860

aaactacctg aatcaatatg ttcactctac aacttgcaaa tactgaagct aaatggttgt 1920

agacatttga aggagctgcc ctcaaatttg cataaactca ctgatttgca tcggcttgaa 1980

ttaatagaaa ctggagtgag aaaggtacca gcacatttgg gaaaactgga gtatcttcaa 2040

gtattaatga gttcgtttaa tgttggcaaa agtagggagt tcagtatcca gcagttagga 2100

gaactcaatc ttcatggaag tctatcaata aggcaactgc agaatgttga gaatccatca 2160

gatgcattag cggtggattt aaaaaataaa acacaccttg tcgagctaga gttagaatgg 2220

gattcagact ggaaccctga tgattcaaca aaagaaaggg atgtaatcga gaatctacag 2280

ccttccaaac acttggagaa gttgaggatg aggaactatg ggggtactca atttccaagg 2340

tggttattca acaattcatc atgtagtgtg gtgtccttaa ccttgaagaa ctgtaaatat 2400

tgcctatgtt tgcctcccct tggacttttg ccatctctga aggaactttc aattaaaggg 2460

cttgatggga tcgtgagtat caatgctgat tttttcggga gtagctcttg ttcatttaca 2520

tccttgaaat ctttggagtt ctaccatatg aaggaatggg aagaatggga atgtaaaggt 2580

gtgacaggtg cttttccacg tcttcaacgt ctttctatag agcgttgtcc caagctgaaa 2640

gggcacttgc cagagcaact atgtcattta aattctctaa agatttctgg gtgtgaacaa 2700

cttgtacctt ctgctctcag tgcccctgat attcataaat tatacctagg agactgtgga 2760

gagctgcaaa ttgatcatgg gacaactttg aaagagctta ccattgaagg tcacaacgtg 2820

gaggcagcct tattcgaaga gattgggcgc aattactctt gttcaaataa caatattccc 2880

atgcacagtt gctatgattt ccttgtaagc ttgcgcatca aaggtggctg cgactcttta 2940

acgacctttc cgctagatat gttcacaata ctcagggaac tttgtatctg gaagtgtcct 3000

aatctacgga ggatttcaca agggcaggct cataatcatc tccagactct ggatatcaaa 3060

gagtgccccc aattagaatc attgcctgaa ggaatgcatg tcctccttcc atctcttgat 3120

tctctgtgca tagatgattg tccaaaagtt gaaatgtttc ccgaaggagg tttgccatca 3180

aatttaaaag aaatgggtct ctttggtggt agttacaaac ttatgtcctt attgaaaagt 3240

gccttgggag gcaatcactc tctagaacgg ttagttattg gaaaagtgga ttttgagtgt 3300

cttcctgagg aaggtgtact gccacactct cttgtttctc tacagatcaa tagctgtgga 3360

gatcttaaaa gactggacta caaaggtatc tgccacctct cctctctcaa ggaattgagt 3420

cttgaagact gccccaggct ccaatgctta ccagaggagg gtctgcccaa atccatttca 3480

tctttgtgga tttgggggga ctgtcagttg ctcaaagaac gttgccggga acccgaaggc 3540

gaagactggc caaagattgc tcacttttgc ccgttgctca atcaacgttg ccgggaaccc 3600

ggaggcgaag actggccaaa gattgctgac attgaaaacg tgtatataag gtag 3654

<210> 111
<211> 1217
<212> PRT
<213> Glycine max

<400> 111

Met Ala Leu Glu Leu Val Gly Gly Ala Leu Leu Ser Ala Phe Leu Gln
1               5                   10                  15
Val Ala Phe Glu Lys Leu Ala Ser Pro Gln Val Leu Asp Phe Phe Arg
            20                  25                  30
Gly Arg Lys Leu Asp Gln Lys Leu Leu Asn Asn Leu Glu Ile Lys Leu
        35                  40                  45
Asn Ser Ile Gln Ala Leu Ala Asn Asp Ala Glu Leu Lys Gln Phe Arg
    50                  55                  60
Asp Pro Leu Val Arg Asn Trp Leu Leu Lys Val Lys Asp Ala Val Phe
65                  70                  75                  80
Asp Ala Glu Asp Ile Leu Asp Glu Ile Gln His Glu Ile Ser Lys Cys
                85                  90                  95
Gln Val Glu Ala Glu Ala Glu Ala Glu Ser Gln Thr Cys Thr Cys Lys
            100                 105                 110
Val Pro Asn Phe Phe Lys Ser Ser Pro Ala Ser Ser Phe Asn Arg Glu
        115                 120                 125
Ile Lys Ser Arg Met Glu Glu Ile Leu Asp Arg Leu Asp Leu Leu Ser
    130                 135                 140
Ser Gln Lys Asp Asp Leu Gly Leu Lys Asn Ser Ser Gly Val Gly Val
145                 150                 155                 160
Gly Ser Glu Leu Gly Ser Ala Val Pro Gln Ile Ser Gln Ser Thr Ser
                165                 170                 175
Ser Val Val Glu Ser Asp Ile Tyr Gly Arg Asp Lys Asp Lys Lys Met
            180                 185                 190
Ile Phe Asp Trp Leu Thr Ser Asp Asn Gly Asn Pro Asn Gln Pro Ser
        195                 200                 205
Ile Leu Ser Ile Val Gly Met Gly Gly Met Gly Lys Thr Thr Leu Ala
    210                 215                 220
Gln His Val Phe Asn Asp Pro Arg Ile Glu Glu Ala Arg Phe Asp Val
225                 230                 235                 240
Lys Ala Trp Val Cys Val Ser Asp Asp Phe Asp Ala Phe Arg Val Thr
                245                 250                 255
Arg Thr Ile Leu Glu Ala Ile Thr Lys Ser Thr Asp Asp Ser Arg Asp
            260                 265                 270
Leu Glu Met Val His Gly Arg Leu Lys Glu Lys Leu Thr Gly Lys Arg
        275                 280                 285
Phe Leu Leu Val Leu Asp Asp Val Trp Asn Glu Asn Arg Leu Lys Trp
    290                 295                 300
Glu Ala Val Leu Lys His Leu Gly Phe Gly Ala Gln Gly Ser Arg Ile
305                 310                 315                 320
Ile Ala Thr Thr Arg Ser Lys Glu Val Ala Ser Thr Met Arg Ser Lys
                325                 330                 335
Glu His Leu Leu Glu Gln Leu Gln Glu Asp His Cys Trp Lys Leu Phe
            340                 345                 350
Ala Lys His Ala Phe Gln Asp Asp Asn Ile Gln Pro Asn Pro Asp Cys
        355                 360                 365
Lys Glu Ile Gly Met Lys Ile Val Glu Lys Cys Lys Gly Leu Pro Leu
    370                 375                 380
Ala Leu Lys Thr Met Gly Ser Leu Leu His Asn Lys Ser Ser Val Thr
385                 390                 395                 400
Glu Trp Lys Ser Ile Leu Gln Ser Glu Ile Trp Glu Phe Ser Thr Glu
                405                 410                 415
Arg Ser Asp Ile Val Pro Ala Leu Ala Leu Ser Tyr His His Leu Pro
            420                 425                 430
Ser His Leu Lys Arg Cys Phe Ala Tyr Cys Ala Leu Phe Pro Lys Asp
        435                 440                 445
Tyr Glu Phe Asp Lys Glu Cys Leu Ile Gln Leu Trp Met Ala Glu Lys
    450                 455                 460
Phe Leu Gln Cys Ser Gln Gln Gly Lys Ser Pro Gly Glu Val Gly Glu
465                 470                 475                 480
Gln Tyr Phe Asn Asp Leu Leu Ser Arg Cys Phe Phe Gln Gln Ser Ser
                485                 490                 495
Asn Thr Glu Arg Thr Asp Phe Val Met His Asp Leu Leu Asn Asp Leu

            500                 505                 510
Ala Arg Phe Ile Cys Gly Asp Ile Cys Phe Arg Leu Asp Gly Asn Gln
        515                 520                 525
Thr Lys Gly Thr Pro Lys Ala Thr Arg His Phe Leu Ile Asp Val Lys
    530                 535                 540
Cys Phe Asp Gly Phe Gly Thr Leu Cys Asp Thr Lys Lys Leu Arg Thr
545                 550                 555                 560
Tyr Met Pro Thr Ser Tyr Lys Tyr Trp Asp Cys Glu Met Ser Ile His
                565                 570                 575
Glu Leu Phe Ser Lys Phe Asn Tyr Leu Arg Val Leu Ser Leu Phe Asp
            580                 585                 590
Cys His Asp Leu Arg Glu Val Pro Asp Ser Val Gly Asn Leu Lys Tyr
        595                 600                 605
Leu Arg Ser Leu Asp Leu Ser Asn Thr Lys Ile Glu Lys Leu Pro Glu
    610                 615                 620
Ser Ile Cys Ser Leu Tyr Asn Leu Gln Ile Leu Lys Leu Asn Gly Cys
625                 630                 635                 640
Arg His Leu Lys Glu Leu Pro Ser Asn Leu His Lys Leu Thr Asp Leu
                645                 650                 655
His Arg Leu Glu Leu Ile Glu Thr Gly Val Arg Lys Val Pro Ala His
            660                 665                 670
Leu Gly Lys Leu Glu Tyr Leu Gln Val Leu Met Ser Ser Phe Asn Val
        675                 680                 685
Gly Lys Ser Arg Glu Phe Ser Ile Gln Gln Leu Gly Glu Leu Asn Leu
    690                 695                 700
His Gly Ser Leu Ser Ile Arg Gln Leu Gln Asn Val Glu Asn Pro Ser
705                 710                 715                 720
Asp Ala Leu Ala Val Asp Leu Lys Asn Lys Thr His Leu Val Glu Leu
                725                 730                 735
Glu Leu Glu Trp Asp Ser Asp Trp Asn Pro Asp Asp Ser Thr Lys Glu
            740                 745                 750
Arg Asp Val Ile Glu Asn Leu Gln Pro Ser Lys His Leu Glu Lys Leu
        755                 760                 765
Arg Met Arg Asn Tyr Gly Gly Thr Gln Phe Pro Arg Trp Leu Phe Asn
    770                 775                 780
Asn Ser Ser Cys Ser Val Val Ser Leu Thr Leu Lys Asn Cys Lys Tyr
785                 790                 795                 800
Cys Leu Cys Leu Pro Pro Leu Gly Leu Leu Pro Ser Leu Lys Glu Leu
                805                 810                 815
Ser Ile Lys Gly Leu Asp Gly Ile Val Ser Ile Asn Ala Asp Phe Phe
            820                 825                 830
Gly Ser Ser Ser Cys Ser Phe Thr Ser Leu Lys Ser Leu Glu Phe Tyr
        835                 840                 845
His Met Lys Glu Trp Glu Glu Trp Glu Cys Lys Gly Val Thr Gly Ala
    850                 855                 860
Phe Pro Arg Leu Gln Arg Leu Ser Ile Glu Arg Cys Pro Lys Leu Lys
865                 870                 875                 880
Gly His Leu Pro Glu Gln Leu Cys His Leu Asn Ser Leu Lys Ile Ser
                885                 890                 895
Gly Cys Glu Gln Leu Val Pro Ser Ala Leu Ser Ala Pro Asp Ile His
            900                 905                 910
Lys Leu Tyr Leu Gly Asp Cys Gly Glu Leu Gln Ile Asp His Gly Thr
        915                 920                 925
Thr Leu Lys Glu Leu Thr Ile Glu Gly His Asn Val Glu Ala Ala Leu
    930                 935                 940
Phe Glu Glu Ile Gly Arg Asn Tyr Ser Cys Ser Asn Asn Asn Ile Pro
945                 950                 955                 960
Met His Ser Cys Tyr Asp Phe Leu Val Ser Leu Arg Ile Lys Gly Gly
                965                 970                 975
Cys Asp Ser Leu Thr Thr Phe Pro Leu Asp Met Phe Thr Ile Leu Arg
            980                 985                 990
Glu Leu Cys Ile Trp Lys Cys Pro Asn Leu Arg Arg Ile Ser Gln Gly
        995                 1000                1005

Gln Ala His Asn His Leu Gln Thr Leu Asp Ile Lys Glu Cys Pro Gln
    1010                1015                1020
Leu Glu Ser Leu Pro Glu Gly Met His Val Leu Leu Pro Ser Leu Asp
1025                1030                1035                1040
Ser Leu Cys Ile Asp Asp Cys Pro Lys Val Glu Met Phe Pro Glu Gly
                1045                1050                1055
Gly Leu Pro Ser Asn Leu Lys Glu Met Gly Leu Phe Gly Gly Ser Tyr
            1060                1065                1070
Lys Leu Met Ser Leu Leu Lys Ser Ala Leu Gly Gly Asn His Ser Leu
        1075                1080                1085
Glu Arg Leu Val Ile Gly Lys Val Asp Phe Glu Cys Leu Pro Glu Glu
    1090                1095                1100
Gly Val Leu Pro His Ser Leu Val Ser Leu Gln Ile Asn Ser Cys Gly
1105                1110                1115                1120
Asp Leu Lys Arg Leu Asp Tyr Lys Gly Ile Cys His Leu Ser Ser Leu
                1125                1130                1135
Lys Glu Leu Ser Leu Glu Asp Cys Pro Arg Leu Gln Cys Leu Pro Glu
            1140                1145                1150
Glu Gly Leu Pro Lys Ser Ile Ser Ser Leu Trp Ile Trp Gly Asp Cys
        1155                1160                1165
Gln Leu Leu Lys Glu Arg Cys Arg Glu Pro Glu Gly Glu Asp Trp Pro
    1170                1175                1180
Lys Ile Ala His Phe Cys Pro Leu Leu Asn Gln Arg Cys Arg Glu Pro
1185                1190                1195                1200
Gly Gly Glu Asp Trp Pro Lys Ile Ala Asp Ile Glu Asn Val Tyr Ile
                1205                1210                1215
Arg

<210> 112
<211> 966
<212> DNA
<213> Artificial Sequence

<220>
<223> cDNA

<400> 112
atgggctgcg tctcgtcaaa aagcaccaca gtgctttctc cacagacatc ttttaatgaa      60

gcctcccgta cgtctttcag agcactcccc ggcccatcgc aaagacaatt ggaggtctat     120

gatcaatgct taattggtgc agcgcgctgg cctgacgatt ccagtaagtc gaatacgcct     180

gaaaacaggg catattgtca gagcatgtac aactcaattc gctctgctgg agatgaaatt     240

tccagaggtg gaatcacatc ttttgaggaa ctatgggggc gcgcaactga atggcgactt     300

tcaaagttgc agagaggaga gccgttatac tctgcattcg cctccgaaag gacgtccgat     360

acagacgcag taacccctct ggtcaaacct tacaagtctg tccttgccag agtcgttgat     420

cacgaggatg cccacgatga aataatgcag gacaatttgt ttggcgatct gaatgttaaa     480

gtatatcgcc aaacagcata cctccatgga aatgttattc cacttaacac ttttcgcgtc     540

gcgacagata ctgaatatct gagagatcga gtagcgcatc tgagaacaga gttgggcgca     600

aaagctctta aacagcattt gcagagatac aatcctgata gaatagacca tacgaacgct     660

tcctatttac ccataataaa agatcattta aatgatcttt acagacaagc aatatcttcc     720

gatttaagcc aagccgaatt gataagcctg atagcccgta cccattggtg ggctgcgagt 780

gcaatgcctg accaaagagg tagtgctgct aaggccgagt ttgcagctag agccatagct 840

agcgcacatg gtatagagct cccgcctttt cgaaatggta acgtttcgga tatagaagcg 900

atgctcagcg gagaggagga attcgtagaa aaatacagaa gcttgctaga ttctgattgc 960

ttttaa 966

<210> 113
<211> 321
<212> PRT
<213> Pseudomonas syringae

<400> 113
Met Gly Cys Val Ser Ser Lys Ser Thr Thr Val Leu Ser Pro Gln Thr
1               5                   10                  15
Ser Phe Asn Glu Ala Ser Arg Thr Ser Phe Arg Ala Leu Pro Gly Pro
            20                  25                  30
Ser Gln Arg Gln Leu Glu Val Tyr Asp Gln Cys Leu Ile Gly Ala Ala
        35                  40                  45
Arg Trp Pro Asp Asp Ser Ser Lys Ser Asn Thr Pro Glu Asn Arg Ala
    50                  55                  60
Tyr Cys Gln Ser Met Tyr Asn Ser Ile Arg Ser Ala Gly Asp Glu Ile
65                  70                  75                  80
Ser Arg Gly Gly Ile Thr Ser Phe Glu Glu Leu Trp Gly Arg Ala Thr
                85                  90                  95
Glu Trp Arg Leu Ser Lys Leu Gln Arg Gly Glu Pro Leu Tyr Ser Ala
            100                 105                 110
Phe Ala Ser Glu Arg Thr Ser Asp Thr Asp Ala Val Thr Pro Leu Val
        115                 120                 125
Lys Pro Tyr Lys Ser Val Leu Ala Arg Val Val Asp His Glu Asp Ala
    130                 135                 140
His Asp Glu Ile Met Gln Asp Asn Leu Phe Gly Asp Leu Asn Val Lys
145                 150                 155                 160
Val Tyr Arg Gln Thr Ala Tyr Leu His Gly Asn Val Ile Pro Leu Asn
                165                 170                 175
Thr Phe Arg Val Ala Thr Asp Thr Glu Tyr Leu Arg Asp Arg Val Ala
            180                 185                 190
His Leu Arg Thr Glu Leu Gly Ala Lys Ala Leu Lys Gln His Leu Gln
        195                 200                 205
Arg Tyr Asn Pro Asp Arg Ile Asp His Thr Asn Ala Ser Tyr Leu Pro
    210                 215                 220
Ile Ile Lys Asp His Leu Asn Asp Leu Tyr Arg Gln Ala Ile Ser Ser
225                 230                 235                 240
Asp Leu Ser Gln Ala Glu Leu Ile Ser Leu Ile Ala Arg Thr His Trp
                245                 250                 255
Trp Ala Ala Ser Ala Met Pro Asp Gln Arg Gly Ser Ala Ala Lys Ala
            260                 265                 270
Glu Phe Ala Ala Arg Ala Ile Ala Ser Ala His Gly Ile Glu Leu Pro
        275                 280                 285
Pro Phe Arg Asn Gly Asn Val Ser Asp Ile Glu Ala Met Leu Ser Gly
    290                 295                 300
Glu Glu Glu Phe Val Glu Lys Tyr Arg Ser Leu Leu Asp Ser Asp Cys
305                 310                 315                 320
Phe

<210> 114
<211> 663

<212> DNA
<213> Artificial Sequence

<220>
<223> cDNA

<400> 114
atgggctgtg tatcgagcac ttcaagaagt actggatatt attccggtta cgaaaatcat 60

gaggagccaa gggtggcaag tagcccgacg aacaggcatg atcgcggcta cgaaaccgat 120

tccgagcatt ccaatgttaa cttaataccc aatgcgagga gagtgtataa agagtctgtt 180

ttgtggcatg gtacatctat gcaatcaaaa atagatttga gaaggcaagg gtttgatgtg 240

aatagaaaaa ctgatggtgc aaccgaggga tctagaacat cgaactcagc ccctacagac 300

acgctcatca gaaatgctag acgtcacaac tacttaacag cgtatgatgt aacagctaag 360

aattatgctc gaagagcaga ccccgaatcc ccagccctag ttagaactat aggtgtcaag 420

agcaatttta atattgagct agatcctgaa tcgaaagacg agaatggtga aatatcccaa 480

ttcagctgca gaacgacaga ttcaatacct aaaaaataca ttgttggaag caagagcagc 540

cagccgggag cagatgcaaa ggtttttaaa aaagaactct ccaatgcggg ctttgaagtt 600

tccacaagcc gtgcaggcga gttattaaga gctgttcagt ctgactcaga agatgacttt 660

taa 663

<210> 115
<211> 220
<212> PRT
<213> Pseudomonas syringae

<400> 115
Met Gly Cys Val Ser Ser Thr Ser Arg Ser Thr Gly Tyr Tyr Ser Gly
1               5                   10                  15
Tyr Glu Asn His Glu Glu Pro Arg Val Ala Ser Ser Pro Thr Asn Arg
            20                  25                  30
His Asp Arg Gly Tyr Glu Thr Asp Ser Glu His Ser Asn Val Asn Leu
        35                  40                  45
Ile Pro Asn Ala Arg Arg Val Tyr Lys Glu Ser Val Leu Trp His Gly
    50                  55                  60
Thr Ser Met Gln Ser Lys Ile Asp Leu Arg Arg Gln Gly Phe Asp Val
65                  70                  75                  80
Asn Arg Lys Thr Asp Gly Ala Thr Glu Gly Ser Arg Thr Ser Asn Ser
                85                  90                  95
Ala Pro Thr Asp Thr Leu Ile Arg Asn Ala Arg Arg His Asn Tyr Leu
            100                 105                 110
Thr Ala Tyr Asp Val Thr Ala Lys Asn Tyr Ala Arg Arg Ala Asp Pro
        115                 120                 125
Glu Ser Pro Ala Leu Val Arg Thr Ile Gly Val Lys Ser Asn Phe Asn
    130                 135                 140
Ile Glu Leu Asp Pro Glu Ser Lys Asp Glu Asn Gly Glu Ile Ser Gln
145                 150                 155                 160
Phe Ser Cys Arg Thr Thr Asp Ser Ile Pro Lys Lys Tyr Ile Val Gly
                165                 170                 175
Ser Lys Ser Ser Gln Pro Gly Ala Asp Ala Lys Val Phe Lys Lys Glu

180                 185                 190
Leu Ser Asn Ala Gly Phe Glu Val Ser Thr Ser Arg Ala Gly Glu Leu
        195                 200                 205
Leu Arg Ala Val Gln Ser Asp Ser Glu Asp Asp Phe
    210                 215                 220

<210> 116
<211> 663
<212> DNA
<213> Artificial Sequence

<220>
<223> cDNA

<400> 116
atgggctgtg tatcgagcac ttcaagaagc actggatatt attccggtta cgaaagtcat      60

gaggagccaa gggtggcaag tagcccggca aacaggcatg atcgcggcta cgaaactgat     120

tccgaacatt ccaatgacaa cttaattccc aatgcgagga gagtgtatag agagtctgtt     180

ttgtggcatg gtacatctat gcaatcaaaa atagatttga gaaggcaagg gtttgatgtg     240

aatagaaaaa ctgatggtgc aaccgaggga tctagaacat cgagctcagc cccgacagac     300

atgctcgtca gaaatgctag acttcacaac tacttaacag cgtatgatgt aactgctaag     360

aattatgctc gaagagcaga ccccgaatcc ccagccctag ttagaactat aggtgtcagg     420

agcaatttta atattgagct agatcctgaa tcgaaagacg agaatggtga aatatcccaa     480

ttcagctgca gaacgacaga ttcaatacct aaaaaataca ttgttggaag caagagcagc     540

cagccgggag cggatgcaaa ggtttttaaa acagaactct ccaatgcggg ttttgaagtt     600

tccacaagcc gtgcaggcga gttattaaga gctgttcagt ctgactcaga agatgacttt     660

taa                                                                   663

<210> 117
<211> 220
<212> PRT
<213> Pseudomonas syringae

<400> 117
Met Gly Cys Val Ser Ser Thr Ser Arg Ser Thr Gly Tyr Tyr Ser Gly
1               5                   10                  15
Tyr Glu Ser His Glu Glu Pro Arg Val Ala Ser Ser Pro Ala Asn Arg
            20                  25                  30
His Asp Arg Gly Tyr Glu Thr Asp Ser Glu His Ser Asn Asp Asn Leu
        35                  40                  45
Ile Pro Asn Ala Arg Arg Val Tyr Arg Glu Ser Val Leu Trp His Gly
    50                  55                  60
Thr Ser Met Gln Ser Lys Ile Asp Leu Arg Arg Gln Gly Phe Asp Val
65                  70                  75                  80
Asn Arg Lys Thr Asp Gly Ala Thr Glu Gly Ser Arg Thr Ser Ser Ser
                85                  90                  95
Ala Pro Thr Asp Met Leu Val Arg Asn Ala Arg Leu His Asn Tyr Leu
            100                 105                 110
Thr Ala Tyr Asp Val Thr Ala Lys Asn Tyr Ala Arg Arg Ala Asp Pro

115                 120                 125
Glu Ser Pro Ala Leu Val Arg Thr Ile Gly Val Arg Ser Asn Phe Asn
    130                 135                 140
Ile Glu Leu Asp Pro Glu Ser Lys Asp Glu Asn Gly Glu Ile Ser Gln
145                 150                 155                 160
Phe Ser Cys Arg Thr Thr Asp Ser Ile Pro Lys Lys Tyr Ile Val Gly
                165                 170                 175
Ser Lys Ser Ser Gln Pro Gly Ala Asp Ala Lys Val Phe Lys Thr Glu
            180                 185                 190
Leu Ser Asn Ala Gly Phe Glu Val Ser Thr Ser Arg Ala Gly Glu Leu
        195                 200                 205
Leu Arg Ala Val Gln Ser Asp Ser Glu Asp Asp Phe
    210                 215                 220

<210> 118
<211> 2781
<212> DNA
<213> Artificial Sequence

<220>
<223> cDNA

<400> 118
atggcttcgg ctactgttga ttttgggatc ggacggattc ttagtgtcct ggaaaacgag      60

accttgttat tgtcaggagt ccatggtgag attgataaaa tgaagaagga gttgctgatc     120

atgaagtcct ttcttgagga tactcataaa catggtggaa atggatccac aacaacaaca     180

actcaactct tccaaacttt tgtggcaaac acgagagatt tggcttacca aatcgaagac     240

attctcgacg agtttggcta tcacatccac ggttatcgta gctgcgccaa aatttggcgt     300

gcgtttcatt tcccgaggta tatgtgggcg aggcactcga tagctcaaaa gctaggaatg     360

gttaatgtca tgattcaatc catttctgat tccatgaaaa ggtactatca ttcagaaaac     420

taccaagcag cattattacc gcctattgat gatggcgatg caaagtgggt gaacaacatc     480

agtgagtcat ctcttttctt tagtgaaaat agtcttgtag ggattgatgc acccaaggga     540

aagctcatcg gacggcttct aagtcccgaa cctcagcgga ttgttgtcgc ggtggttggg     600

atgggcggtt cagggaaaac tacactctca gcgaatatct tcaagtctca aagtgtgcgg     660

aggcattttg agtcttacgc ttgggttaca atctcgaaat cttatgtgat tgaagatgtg     720

tttaggacca tgatcaagga gttttacaaa gaagcagata cacagattcc agctgaatta     780

tactccttag gctacagaga gttggtggaa aaacttgtgg agtatttgca gtcgaagagg     840

tacattgttg tgcttgatga tgtatggacc actggtctat ggagggagat aagtattgct     900

ttaccagatg gtatttatgg aagtcgtgtt atgatgacta ctcgggacat gaacgtggct     960

tctttcccat atggaattgg gagcacaaag cacgagattg agctcttaaa agaagacgaa    1020

gcttgggtgc ttttctccaa caaagctttt ccggcaagtc ttgagcaatg cagaacgcag    1080

aatttggagc cgatagcacg aaaattagtc gagagatgtc agggtttgcc attagctatt    1140

gcttctttgg gaagcatgat gtcaaccaag aagtttgaat cagaatggaa gaaagtctac    1200

agtactttaa actgggaact gaacaacaat catgaactca agattgttcg aagcattatg 1260

ttccttagct tcaatgattt gccatacccg cttaaacgtt gtttcttata ttgttctctt 1320

ttccctgtga actatcggat gaaaagaaag aggctcatta ggatgtggat ggcacaaagg 1380

tttgtggaac cgatcagagg agtgaaagca gaagaagtgg ctgatagtta cctcaatgaa 1440

cttgtttaca gaaatatgct tcaagttatc ttgtggaatc cttttggacg acccaaggcg 1500

ttcaagatgc atgacgtgat atgggagatt gctctatctg tatccaaact tgagaggttc 1560

tgtgatgtat ataatgatga cagtgatggt gatgatgctg cagaaacaat ggagaattac 1620

ggtagtcgcc atttatgcat tcagaaagag atgacacctg atagtatacg tgcaacaaac 1680

cttcactctc ttttggtttg ctcttctgct aaacacaaga tggaattact tccaagtctg 1740

aaccttctga gagccttaga ccttgaagac tcttccatca gcaagttacc agattgttta 1800

gtaactatgt tcaacttaaa gtacttgaat ttgtccaaga cacaggtgaa agaactgcca 1860

aaaaacttcc ataaactcgt caatttggag acgttgaaca caaagcactc caagattgag 1920

gaacttcctc tagggatgtg gaaattgaag aagttgcgat atctcattac ttttcgccgc 1980

aatgaaggac atgattccaa ttggaattat gttttaggta caagggttgt tcctaaaatc 2040

tggcagttga aggatttgca agtcatggac tgcttcaacg cagaagatga gcttatcaaa 2100

aatctcgggt gtatgactca gctcacaaga ataagtcttg tcatggttag gagagaacat 2160

ggaagagact tgtgcgattc actgaataag attaaaagaa ttcgattctt atctttaacg 2220

tcgattgatg aagaggaacc gttagagata gatgacctga tcgcaactgc aagcattgag 2280

aagctctttc ttgctgggaa actggagaga gtaccaagtt ggttcaacac tcttcaaaac 2340

cttacctatt tgggtttgcg tggatctcag ctacaggaaa acgccattct ctctatccaa 2400

acgcttccta gattggtatg gctttcattt tacaatgcat acatgggacc taggttgcgt 2460

tttgcacaag gatttcagaa tctgaagatt ctagagatag ttcagatgaa acatctgaca 2520

gaagttgtta tagaagacgg tgcaatgttt gagcttcaga agctttatgt cagggcttgt 2580

agagggttag agtacgtacc aagaggaatt gagaatctta taaatcttca agaactgcat 2640

ttgattcatg tttctaatca gttagtagaa cgcattcgcg gagaagggag tgtggaccgg 2700

tcaagggtta aacacattcc tgcgattaag cattatttca gaactgataa tggctctttc 2760

tatgtgagcc tctcatctta g 2781

<210> 119
<211> 926
<212> PRT
<213> Arabidopsis thaliana

<400> 119

Met Ala Ser Ala Thr Val Asp Phe Gly Ile Gly Arg Ile Leu Ser Val
1               5                   10                  15
Leu Glu Asn Glu Thr Leu Leu Leu Ser Gly Val His Gly Glu Ile Asp
            20                  25                  30
Lys Met Lys Lys Glu Leu Leu Ile Met Lys Ser Phe Leu Glu Asp Thr
        35                  40                  45
His Lys His Gly Gly Asn Gly Ser Thr Thr Thr Thr Thr Gln Leu Phe
    50                  55                  60
Gln Thr Phe Val Ala Asn Thr Arg Asp Leu Ala Tyr Gln Ile Glu Asp
65                  70                  75                  80
Ile Leu Asp Glu Phe Gly Tyr His Ile His Gly Tyr Arg Ser Cys Ala
                85                  90                  95
Lys Ile Trp Arg Ala Phe His Phe Pro Arg Tyr Met Trp Ala Arg His
            100                 105                 110
Ser Ile Ala Gln Lys Leu Gly Met Val Asn Val Met Ile Gln Ser Ile
        115                 120                 125
Ser Asp Ser Met Lys Arg Tyr Tyr His Ser Glu Asn Tyr Gln Ala Ala
    130                 135                 140
Leu Leu Pro Pro Ile Asp Asp Gly Asp Ala Lys Trp Val Asn Asn Ile
145                 150                 155                 160
Ser Glu Ser Ser Leu Phe Phe Ser Glu Asn Ser Leu Val Gly Ile Asp
                165                 170                 175
Ala Pro Lys Gly Lys Leu Ile Gly Arg Leu Leu Ser Pro Glu Pro Gln
            180                 185                 190
Arg Ile Val Val Ala Val Val Gly Met Gly Gly Ser Gly Lys Thr Thr
        195                 200                 205
Leu Ser Ala Asn Ile Phe Lys Ser Gln Ser Val Arg Arg His Phe Glu
    210                 215                 220
Ser Tyr Ala Trp Val Thr Ile Ser Lys Ser Tyr Val Ile Glu Asp Val
225                 230                 235                 240
Phe Arg Thr Met Ile Lys Glu Phe Tyr Lys Glu Ala Asp Thr Gln Ile
                245                 250                 255
Pro Ala Glu Leu Tyr Ser Leu Gly Tyr Arg Glu Leu Val Glu Lys Leu
            260                 265                 270
Val Glu Tyr Leu Gln Ser Lys Arg Tyr Ile Val Val Leu Asp Asp Val
        275                 280                 285
Trp Thr Thr Gly Leu Trp Arg Glu Ile Ser Ile Ala Leu Pro Asp Gly
    290                 295                 300
Ile Tyr Gly Ser Arg Val Met Met Thr Thr Arg Asp Met Asn Val Ala
305                 310                 315                 320
Ser Phe Pro Tyr Gly Ile Gly Ser Thr Lys His Glu Ile Glu Leu Leu
                325                 330                 335
Lys Glu Asp Glu Ala Trp Val Leu Phe Ser Asn Lys Ala Phe Pro Ala
            340                 345                 350
Ser Leu Glu Gln Cys Arg Thr Gln Asn Leu Glu Pro Ile Ala Arg Lys
        355                 360                 365
Leu Val Glu Arg Cys Gln Gly Leu Pro Leu Ala Ile Ala Ser Leu Gly
    370                 375                 380
Ser Met Met Ser Thr Lys Lys Phe Glu Ser Glu Trp Lys Lys Val Tyr
385                 390                 395                 400
Ser Thr Leu Asn Trp Glu Leu Asn Asn Asn His Glu Leu Lys Ile Val
                405                 410                 415
Arg Ser Ile Met Phe Leu Ser Phe Asn Asp Leu Pro Tyr Pro Leu Lys
            420                 425                 430
Arg Cys Phe Leu Tyr Cys Ser Leu Phe Pro Val Asn Tyr Arg Met Lys
        435                 440                 445
Arg Lys Arg Leu Ile Arg Met Trp Met Ala Gln Arg Phe Val Glu Pro
    450                 455                 460
Ile Arg Gly Val Lys Ala Glu Glu Val Ala Asp Ser Tyr Leu Asn Glu
465                 470                 475                 480
Leu Val Tyr Arg Asn Met Leu Gln Val Ile Leu Trp Asn Pro Phe Gly
                485                 490                 495
Arg Pro Lys Ala Phe Lys Met His Asp Val Ile Trp Glu Ile Ala Leu

                500                 505                 510
Ser Val Ser Lys Leu Glu Arg Phe Cys Asp Val Tyr Asn Asp Asp Ser
            515                 520                 525
Asp Gly Asp Asp Ala Ala Glu Thr Met Glu Asn Tyr Gly Ser Arg His
    530                 535                 540
Leu Cys Ile Gln Lys Glu Met Thr Pro Asp Ser Ile Arg Ala Thr Asn
545                 550                 555                 560
Leu His Ser Leu Leu Val Cys Ser Ser Ala Lys His Lys Met Glu Leu
                565                 570                 575
Leu Pro Ser Leu Asn Leu Leu Arg Ala Leu Asp Leu Glu Asp Ser Ser
            580                 585                 590
Ile Ser Lys Leu Pro Asp Cys Leu Val Thr Met Phe Asn Leu Lys Tyr
        595                 600                 605
Leu Asn Leu Ser Lys Thr Gln Val Lys Glu Leu Pro Lys Asn Phe His
    610                 615                 620
Lys Leu Val Asn Leu Glu Thr Leu Asn Thr Lys His Ser Lys Ile Glu
625                 630                 635                 640
Glu Leu Pro Leu Gly Met Trp Lys Leu Lys Lys Leu Arg Tyr Leu Ile
                645                 650                 655
Thr Phe Arg Arg Asn Glu Gly His Asp Ser Asn Trp Asn Tyr Val Leu
            660                 665                 670
Gly Thr Arg Val Val Pro Lys Ile Trp Gln Leu Lys Asp Leu Gln Val
        675                 680                 685
Met Asp Cys Phe Asn Ala Glu Asp Glu Leu Ile Lys Asn Leu Gly Cys
    690                 695                 700
Met Thr Gln Leu Thr Arg Ile Ser Leu Val Met Val Arg Arg Glu His
705                 710                 715                 720
Gly Arg Asp Leu Cys Asp Ser Leu Asn Lys Ile Lys Arg Ile Arg Phe
                725                 730                 735
Leu Ser Leu Thr Ser Ile Asp Glu Glu Glu Pro Leu Glu Ile Asp Asp
            740                 745                 750
Leu Ile Ala Thr Ala Ser Ile Glu Lys Leu Phe Leu Ala Gly Lys Leu
        755                 760                 765
Glu Arg Val Pro Ser Trp Phe Asn Thr Leu Gln Asn Leu Thr Tyr Leu
    770                 775                 780
Gly Leu Arg Gly Ser Gln Leu Gln Glu Asn Ala Ile Leu Ser Ile Gln
785                 790                 795                 800
Thr Leu Pro Arg Leu Val Trp Leu Ser Phe Tyr Asn Ala Tyr Met Gly
                805                 810                 815
Pro Arg Leu Arg Phe Ala Gln Gly Phe Gln Asn Leu Lys Ile Leu Glu
            820                 825                 830
Ile Val Gln Met Lys His Leu Thr Glu Val Val Ile Glu Asp Gly Ala
        835                 840                 845
Met Phe Glu Leu Gln Lys Leu Tyr Val Arg Ala Cys Arg Gly Leu Glu
    850                 855                 860
Tyr Val Pro Arg Gly Ile Glu Asn Leu Ile Asn Leu Gln Glu Leu His
865                 870                 875                 880
Leu Ile His Val Ser Asn Gln Leu Val Glu Arg Ile Arg Gly Glu Gly
                885                 890                 895
Ser Val Asp Arg Ser Arg Val Lys His Ile Pro Ala Ile Lys His Tyr
            900                 905                 910
Phe Arg Thr Asp Asn Gly Ser Phe Tyr Val Ser Leu Ser Ser
        915                 920                 925

<210> 120
<211> 2730
<212> DNA
<213> Artificial Sequence

<220>
<223> cDNA

<400> 120
atggatttca tctcatctct tatcgttggc tgtgctcagg tgttgtgtga atctatgaat 60

atggcggaga gaagaggaca taagactgat cttagacaag ccatcactga tcttgaaaca 120

gccatcggtg acttgaaggc catacgtgat gacctgactt tacggatcca acaagacggt 180

ctagagggac gaagctgctc aaatcgtgcc agagagtggc ttagtgcggt gcaagtaacg 240

gagactaaaa cagccctact tttagtgagg tttaggcgtc gggaacagag gacgcgaatg 300

aggaggagat acctcagttg tttcggttgt gccgactaca aactgtgcaa gaaggtttct 360

gccatattga agagcattgg tgagctgaga gaacgctctg aagctatcaa aacagatggc 420

gggtcaattc aagtaacttg tagagagata cccatcaagt ccgttgtcgg aaataccacg 480

atgatggaac aggttttgga atttctcagt gaagaagaag aaagaggaat cattggtgtt 540

tatggacctg gtggggttgg gaagacaacg ttaatgcaga gcattaacaa cgagctgatc 600

acaaaaggac atcagtatga tgtactgatt tgggttcaaa tgtccagaga attcggcgag 660

tgtacaattc agcaagccgt tggagcacgg ttgggtttat cttgggacga gaaggagacc 720

ggcgaaaaca gagctttgaa gatatacaga gctttgagac agaaacgttt cttgttgttg 780

ctagatgatg tctgggaaga gatagacttg gagaaaactg gagttcctcg acctgacagg 840

gaaaacaaat gcaaggtgat gttcacgaca cggtctatag cattatgcaa caatatgggt 900

gcggaataca agttgagagt ggagtttctg gagaagaaac acgcgtggga gctgttctgt 960

agtaaggtat ggagaaaaga tcttttagag tcatcatcaa ttcgccggct cgcggagatt 1020

atagtgagta aatgtggagg attgccacta gcgttgatca ctttaggagg agccatggct 1080

catagagaga cagaagaaga gtggatccat gctagtgaag ttctgactag atttccagca 1140

gagatgaagg gtatgaacta tgtatttgcc cttttgaaat tcagctacga caacctcgag 1200

agtgatctgc ttcggtcttg tttcttgtac tgcgctttat tcccagaaga acattctata 1260

gagatcgagc agcttgttga gtactgggtc ggcgaagggt ttctcaccag ctcccatggc 1320

gttaacacca tttacaaggg atattttctc attggggatc tgaaagcggc atgtttgttg 1380

gaaaccggag atgagaaaac acaggtgaag atgcataatg tggtcagaag ctttgcattg 1440

tggatggcat ctgaacaggg gacttataag gagctgatcc tagttgagcc tagcatggga 1500

catactgaag ctcctaaagc agaaaactgg cgacaagcgt tggtgatctc attgttagat 1560

aacagaatcc agaccttgcc tgaaaaactc atatgcccga aactgacaac actgatgctc 1620

caacagaaca gctctttgaa gaagattcca acagggtttt tcatgcatat gcctgttctc 1680

agagtcttgg acttgtcgtt cacaagtatc actgagattc cgttgtctat caagtatttg 1740

gtggagttgt atcatctgtc tatgtcagga acaaagataa gtgtattgcc acaggagctt 1800

gggaatctta gaaaactgaa gcatctggac ctacaaagaa ctcagtttct tcagacgatc 1860

ccacgagatg ccatatgttg gctgagcaag ctcgaggttc tgaacttgta ctacagttac 1920

gccggttggg aactgcagag ctttggagaa gatgaagcag aagaactcgg attcgctgac 1980

ttggaatact tggaaaacct aaccacactc ggtatcactg ttctctcatt ggagacccta 2040

aaaactctct tcgagttcgg tgctttgcat aaacatatac agcatctcca cgttgaagag 2100

tgcaatgaac tcctctactt caatctccca tcactcacta accatggcag gaacctgaga 2160

agacttagca ttaaaagttg ccatgacttg gagtacctgg tcacacccgc agattttgaa 2220

aatgattggc ttccgagtct agaggttctg acgttacaca gccttcacaa cttaaccaga 2280

gtgtggggaa attctgtaag ccaagattgt ctgcggaata tccgttgcat aaacatttca 2340

cactgcaaca agctgaagaa tgtctcatgg gttcagaaac tcccaaagct agaggtgatt 2400

gaactgttcg actgcagaga gatagaggaa ttgataagcg aacacgagag tccatccgtc 2460

gaagatccaa cattgttccc aagcctgaag accttgagaa ctagggatct gccagaacta 2520

aacagcatcc tcccatctcg attttcattc caaaaagttg aaacattagt catcacaaat 2580

tgccccagag ttaagaaact gccgtttcag gagaggagga cccagatgaa cttgccaaca 2640

gtttattgtg aggagaaatg gtggaaagca ctggaaaaag atcaaccaaa cgaagagctt 2700

tgttatttac cgcgctttgt tccaaattga 2730

<210> 121
<211> 909
<212> PRT
<213> Arabidopsis thaliana

<400> 121
Met Asp Phe Ile Ser Ser Leu Ile Val Gly Cys Ala Gln Val Leu Cys
1               5                   10                  15
Glu Ser Met Asn Met Ala Glu Arg Arg Gly His Lys Thr Asp Leu Arg
            20                  25                  30
Gln Ala Ile Thr Asp Leu Glu Thr Ala Ile Gly Asp Leu Lys Ala Ile
        35                  40                  45
Arg Asp Asp Leu Thr Leu Arg Ile Gln Gln Asp Gly Leu Glu Gly Arg
    50                  55                  60
Ser Cys Ser Asn Arg Ala Arg Glu Trp Leu Ser Ala Val Gln Val Thr
65                  70                  75                  80
Glu Thr Lys Thr Ala Leu Leu Leu Val Arg Phe Arg Arg Arg Glu Gln
                85                  90                  95
Arg Thr Arg Met Arg Arg Arg Tyr Leu Ser Cys Phe Gly Cys Ala Asp
            100                 105                 110
Tyr Lys Leu Cys Lys Lys Val Ser Ala Ile Leu Lys Ser Ile Gly Glu
        115                 120                 125
Leu Arg Glu Arg Ser Glu Ala Ile Lys Thr Asp Gly Gly Ser Ile Gln
    130                 135                 140
Val Thr Cys Arg Glu Ile Pro Ile Lys Ser Val Val Gly Asn Thr Thr
145                 150                 155                 160
Met Met Glu Gln Val Leu Glu Phe Leu Ser Glu Glu Glu Glu Arg Gly
                165                 170                 175
Ile Ile Gly Val Tyr Gly Pro Gly Gly Val Gly Lys Thr Thr Leu Met
            180                 185                 190
Gln Ser Ile Asn Asn Glu Leu Ile Thr Lys Gly His Gln Tyr Asp Val

        195                 200                 205
Leu Ile Trp Val Gln Met Ser Arg Glu Phe Gly Glu Cys Thr Ile Gln
    210                 215                 220
Gln Ala Val Gly Ala Arg Leu Gly Leu Ser Trp Asp Glu Lys Glu Thr
225                 230                 235                 240
Gly Glu Asn Arg Ala Leu Lys Ile Tyr Arg Ala Leu Arg Gln Lys Arg
                245                 250                 255
Phe Leu Leu Leu Leu Asp Asp Val Trp Glu Glu Ile Asp Leu Glu Lys
            260                 265                 270
Thr Gly Val Pro Arg Pro Asp Arg Glu Asn Lys Cys Lys Val Met Phe
        275                 280                 285
Thr Thr Arg Ser Ile Ala Leu Cys Asn Asn Met Gly Ala Glu Tyr Lys
    290                 295                 300
Leu Arg Val Glu Phe Leu Glu Lys Lys His Ala Trp Glu Leu Phe Cys
305                 310                 315                 320
Ser Lys Val Trp Arg Lys Asp Leu Leu Glu Ser Ser Ser Ile Arg Arg
                325                 330                 335
Leu Ala Glu Ile Ile Val Ser Lys Cys Gly Gly Leu Pro Leu Ala Leu
            340                 345                 350
Ile Thr Leu Gly Gly Ala Met Ala His Arg Glu Thr Glu Glu Glu Trp
        355                 360                 365
Ile His Ala Ser Glu Val Leu Thr Arg Phe Pro Ala Glu Met Lys Gly
    370                 375                 380
Met Asn Tyr Val Phe Ala Leu Leu Lys Phe Ser Tyr Asp Asn Leu Glu
385                 390                 395                 400
Ser Asp Leu Leu Arg Ser Cys Phe Leu Tyr Cys Ala Leu Phe Pro Glu
                405                 410                 415
Glu His Ser Ile Glu Ile Glu Gln Leu Val Glu Tyr Trp Val Gly Glu
            420                 425                 430
Gly Phe Leu Thr Ser Ser His Gly Val Asn Thr Ile Tyr Lys Gly Tyr
        435                 440                 445
Phe Leu Ile Gly Asp Leu Lys Ala Ala Cys Leu Leu Glu Thr Gly Asp
    450                 455                 460
Glu Lys Thr Gln Val Lys Met His Asn Val Val Arg Ser Phe Ala Leu
465                 470                 475                 480
Trp Met Ala Ser Glu Gln Gly Thr Tyr Lys Glu Leu Ile Leu Val Glu
                485                 490                 495
Pro Ser Met Gly His Thr Glu Ala Pro Lys Ala Glu Asn Trp Arg Gln
            500                 505                 510
Ala Leu Val Ile Ser Leu Leu Asp Asn Arg Ile Gln Thr Leu Pro Glu
        515                 520                 525
Lys Leu Ile Cys Pro Lys Leu Thr Thr Leu Met Leu Gln Gln Asn Ser
    530                 535                 540
Ser Leu Lys Lys Ile Pro Thr Gly Phe Phe Met His Met Pro Val Leu
545                 550                 555                 560
Arg Val Leu Asp Leu Ser Phe Thr Ser Ile Thr Glu Ile Pro Leu Ser
                565                 570                 575
Ile Lys Tyr Leu Val Glu Leu Tyr His Leu Ser Met Ser Gly Thr Lys
            580                 585                 590
Ile Ser Val Leu Pro Gln Glu Leu Gly Asn Leu Arg Lys Leu Lys His
        595                 600                 605
Leu Asp Leu Gln Arg Thr Gln Phe Leu Gln Thr Ile Pro Arg Asp Ala
    610                 615                 620
Ile Cys Trp Leu Ser Lys Leu Glu Val Leu Asn Leu Tyr Tyr Ser Tyr
625                 630                 635                 640
Ala Gly Trp Glu Leu Gln Ser Phe Gly Glu Asp Glu Ala Glu Glu Leu
                645                 650                 655
Gly Phe Ala Asp Leu Glu Tyr Leu Glu Asn Leu Thr Thr Leu Gly Ile
            660                 665                 670
Thr Val Leu Ser Leu Glu Thr Leu Lys Thr Leu Phe Glu Phe Gly Ala
        675                 680                 685
Leu His Lys His Ile Gln His Leu His Val Glu Glu Cys Asn Glu Leu
    690                 695                 700

Leu Tyr Phe Asn Leu Pro Ser Leu Thr Asn His Gly Arg Asn Leu Arg
705                 710                 715                 720
Arg Leu Ser Ile Lys Ser Cys His Asp Leu Glu Tyr Leu Val Thr Pro
                725                 730                 735
Ala Asp Phe Glu Asn Asp Trp Leu Pro Ser Leu Glu Val Leu Thr Leu
            740                 745                 750
His Ser Leu His Asn Leu Thr Arg Val Trp Gly Asn Ser Val Ser Gln
        755                 760                 765
Asp Cys Leu Arg Asn Ile Arg Cys Ile Asn Ile Ser His Cys Asn Lys
    770                 775                 780
Leu Lys Asn Val Ser Trp Val Gln Lys Leu Pro Lys Leu Glu Val Ile
785                 790                 795                 800
Glu Leu Phe Asp Cys Arg Glu Ile Glu Glu Leu Ile Ser Glu His Glu
                805                 810                 815
Ser Pro Ser Val Glu Asp Pro Thr Leu Phe Pro Ser Leu Lys Thr Leu
            820                 825                 830
Arg Thr Arg Asp Leu Pro Glu Leu Asn Ser Ile Leu Pro Ser Arg Phe
        835                 840                 845
Ser Phe Gln Lys Val Glu Thr Leu Val Ile Thr Asn Cys Pro Arg Val
    850                 855                 860
Lys Lys Leu Pro Phe Gln Glu Arg Arg Thr Gln Met Asn Leu Pro Thr
865                 870                 875                 880
Val Tyr Cys Glu Glu Lys Trp Trp Lys Ala Leu Glu Lys Asp Gln Pro
                885                 890                 895
Asn Glu Glu Leu Cys Tyr Leu Pro Arg Phe Val Pro Asn
            900                 905

<210> 122
<211> 768
<212> DNA
<213> Artificial Sequence

<220>
<223> cDNA

<400> 122
atgaaaattg ctccagttgc cataaatcac agcccgctca gcagggaggt cccgtcacac      60

gcggcaacca ctcaggcaaa gcaaaccaac cttcaatctg aagctggcga tttagatgca     120

agaaaaagta gcgcttcaag cccggaaacc cgcgcattac tcgctactaa gacagtactc     180

gggagacaca agatagaggt tccagccttt ggagggtggt tcaaaaagaa atcatctaag     240

cacgagacgg gcggttcaag tgccaacgca gatagttcga gcgtggcttc cgattccacc     300

gaaaaacctt tgttccgtct cacgcacgtt ccttacgtat cccaaggtaa tgagcgaatg     360

ggatgttggt atgcctgcgc aagaatggtt ggccattctg tcgaagctgg gcctcgccta     420

gggctgccgg agctctatga gggaagggag ggcccagctg ggctacaaga tttttcagat     480

gtagaaaggt ttattcacaa tgaaggatta actcgggtag accttccaga caatgagaga     540

tttacacacg aagagttggg tgcactgttg tataagcacg ggccgattat atttgggtgg     600

aaaactccga atgacagctg gcacatgtcg gtcctcactg gtgtcgataa agagacgtcg     660

tccattactt ttcacgatcc tcgacagggg ccggacctag caatgccgct cgattacttt     720

aatcagcgat tggcatggca ggttccacac gcaatgctct accgctaa                  768

<210> 123
<211> 255
<212> PRT
<213> Pseudomonas syringae

<400> 123
Met Lys Ile Ala Pro Val Ala Ile Asn His Ser Pro Leu Ser Arg Glu
1               5                   10                  15
Val Pro Ser His Ala Ala Thr Thr Gln Ala Lys Gln Thr Asn Leu Gln
            20                  25                  30
Ser Glu Ala Gly Asp Leu Asp Ala Arg Lys Ser Ser Ala Ser Ser Pro
        35                  40                  45
Glu Thr Arg Ala Leu Leu Ala Thr Lys Thr Val Leu Gly Arg His Lys
    50                  55                  60
Ile Glu Val Pro Ala Phe Gly Gly Trp Phe Lys Lys Lys Ser Ser Lys
65                  70                  75                  80
His Glu Thr Gly Gly Ser Ser Ala Asn Ala Asp Ser Ser Ser Val Ala
                85                  90                  95
Ser Asp Ser Thr Glu Lys Pro Leu Phe Arg Leu Thr His Val Pro Tyr
            100                 105                 110
Val Ser Gln Gly Asn Glu Arg Met Gly Cys Trp Tyr Ala Cys Ala Arg
        115                 120                 125
Met Val Gly His Ser Val Glu Ala Gly Pro Arg Leu Gly Leu Pro Glu
    130                 135                 140
Leu Tyr Glu Gly Arg Glu Gly Pro Ala Gly Leu Gln Asp Phe Ser Asp
145                 150                 155                 160
Val Glu Arg Phe Ile His Asn Glu Gly Leu Thr Arg Val Asp Leu Pro
                165                 170                 175
Asp Asn Glu Arg Phe Thr His Glu Glu Leu Gly Ala Leu Leu Tyr Lys
            180                 185                 190
His Gly Pro Ile Ile Phe Gly Trp Lys Thr Pro Asn Asp Ser Trp His
        195                 200                 205
Met Ser Val Leu Thr Gly Val Asp Lys Glu Thr Ser Ser Ile Thr Phe
    210                 215                 220
His Asp Pro Arg Gln Gly Pro Asp Leu Ala Met Pro Leu Asp Tyr Phe
225                 230                 235                 240
Asn Gln Arg Leu Ala Trp Gln Val Pro His Ala Met Leu Tyr Arg
                245                 250                 255

<210> 124
<211> 2670
<212> DNA
<213> Artificial Sequence

<220>
<223> cDNA

<400> 124
atgggaggtt gtttctctgt ttcattgcca tgtgatcaag tagtgagtca gttctctcag      60

ttgttatgtg tcaggggaag ttatattcac aatctctcca agaatctggc ttctctgcag     120

aaggccatgc gaatgctcaa ggctcggcaa tatgatgtga taagaaggtt ggaaacagaa     180

gagtttacag ggcgtcaaca aaggctttcc caagttcagg tatggcttac tagtgtccta     240

atcattcaaa accagtttaa tgatctgctt cgtagtaatg aagttgagct tcaaaggttg     300

tgtctttgtg gtttctgctc caaagatttg aaattgagct atcgttatgg gaaaagagtt     360

atcatgatgt tgaaggaagt tgagagtctc agttctcaag gattctttga tgtggtttct 420

gaggcaactc cgtttgctga tgtggatgag atcccttttc aacccacaat tgttggtcag 480

gagataatgc ttgaaaaggc atggaaccgt ctcatggaag atggatccgg gattttgggt 540

ctgtacggta tggggggagt aggcaaaacg acactactca cgaagatcaa caataagttt 600

tctaaaatag atgacagatt tgatgttgtg atatgggttg ttgtgtctag aagttcaaca 660

gtccgtaaga tacaaagaga catagcagaa aaggtaggcc ttgggggaat ggagtggagc 720

gagaaaaacg ataaccagat agccgttgac atccacaatg tccttaggag acgaaagttt 780

gttttattgt tggatgacat atgggagaaa gtgaatttaa aagcggttgg agtcccgtat 840

ccaagcaaag ataacggatg caaggtagca ttcactactc gttctcgaga tgtgtgtggg 900

cgcatggggg ttgatgatcc gatggaagtt agctgtctac aacccgaaga atcttgggat 960

ttgtttcaaa tgaaggttgg gaaaaataca ctgggaagtc acccagacat tcccggactt 1020

gctagaaaag tcgctaggaa atgtcgtggc ctaccattag cactcaatgt tatcggtgaa 1080

gctatggcat gcaaaagaac agtacatgaa tggtgtcatg ccattgacgt tttaacatca 1140

tctgccatag atttttcagg tatggaagat gaaattctcc atgttttgaa gtatagctat 1200

gataatttaa atggggagct gatgaaatcc tgcttcctct attgctctct gtttcctgaa 1260

gactatctta ttgataaaga ggggttggta gactactgga taagtgaggg attcataaat 1320

gaaaaagaag gcagagagag gaatattaac caaggttatg agataattgg tacccttgtc 1380

cgtgcatgtt tgttgttgga ggaagaaagg aacaaatcaa acgtgaaaat gcatgatgtg 1440

gttcgtgaga tggctctatg gatatcatcc gatcttggga agcaaaagga gaaatgtatt 1500

gtgcgagctg gtgttgggtt acgtgaagta ccaaaagtca aggattggaa cactgtgaga 1560

aagatttctt tgatgaataa tgagattgaa gagatatttg acagtcacga gtgtgctgca 1620

cttacaactc tatttctcca aaagaacgac gtggtaaaga tctcggctga attctttcga 1680

tgtatgcctc atctggtggt tttagatcta tcagagaatc agagtcttaa tgaattacca 1740

gaagaaatat cagagctggc ctctttgaga tatttcaact tgtcatatac atgtatacat 1800

caactacccg ttggtttatg gacattgaaa aagctaatac atctgaatct cgaacacatg 1860

agcagtcttg ggagtatatt agggatatca aatttgtgga atttgaggac attgggacta 1920

cgagattcca gactgttgct agatatgagt ttagtgaagg agctgcagct cttagaacat 1980

ctagaagtta taaccctaga tatatcatca agtttggttg cagagccatt gttatgctct 2040

caaaggttgg tggaatgtat aaaagaggta gattttaagt accttaagga agaatcagtg 2100

agagtattga ctttgccaac catgggtaat ctccgcaagc tcgggataaa aagatgtgga 2160

atgagggaga taaagataga gaggacaact tcatcatcgt ctcggaacaa aagtcccaca 2220

actccatgct tctcaaacct ctccagagtc tttatagcta aatgccatgg tcttaaggat 2280

ttgacgtggc ttttgtttgc tcctaatctt acttttcttg aagttgggtt ctcaaaggaa 2340

gtagaggata taattagtga agagaaagct gaggagcatt cagctacgat tgttcctttt 2400

aggaaattgg agacactaca cctgtttgaa ttacgcgggc tcaagagaat ctatgcaaag 2460

gctctgcatt ttccgtgtct gaaagttatc cacgtagaaa aatgtgaaaa gctgagaaag 2520

cttccattgg attctaaaag tgggatcgcg ggtgaagaac ttgtcatata ttatggagag 2580

agagaatgga tagaaagggt tgaatgggag gaccaagcaa ctcaactccg tttcttacct 2640

tcatccaggt ggcggtggag agaaacataa 2670

<210> 125
<211> 889
<212> PRT
<213> Arabidopsis thaliana

<400> 125
Met Gly Gly Cys Phe Ser Val Ser Leu Pro Cys Asp Gln Val Val Ser
1               5                   10                  15
Gln Phe Ser Gln Leu Leu Cys Val Arg Gly Ser Tyr Ile His Asn Leu
            20                  25                  30
Ser Lys Asn Leu Ala Ser Leu Gln Lys Ala Met Arg Met Leu Lys Ala
        35                  40                  45
Arg Gln Tyr Asp Val Ile Arg Arg Leu Glu Thr Glu Glu Phe Thr Gly
    50                  55                  60
Arg Gln Gln Arg Leu Ser Gln Val Gln Val Trp Leu Thr Ser Val Leu
65                  70                  75                  80
Ile Ile Gln Asn Gln Phe Asn Asp Leu Leu Arg Ser Asn Glu Val Glu
                85                  90                  95
Leu Gln Arg Leu Cys Leu Cys Gly Phe Cys Ser Lys Asp Leu Lys Leu
            100                 105                 110
Ser Tyr Arg Tyr Gly Lys Arg Val Ile Met Met Leu Lys Glu Val Glu
        115                 120                 125
Ser Leu Ser Ser Gln Gly Phe Phe Asp Val Val Ser Glu Ala Thr Pro
    130                 135                 140
Phe Ala Asp Val Asp Glu Ile Pro Phe Gln Pro Thr Ile Val Gly Gln
145                 150                 155                 160
Glu Ile Met Leu Glu Lys Ala Trp Asn Arg Leu Met Glu Asp Gly Ser
                165                 170                 175
Gly Ile Leu Gly Leu Tyr Gly Met Gly Gly Val Gly Lys Thr Thr Leu
            180                 185                 190
Leu Thr Lys Ile Asn Asn Lys Phe Ser Lys Ile Asp Asp Arg Phe Asp
        195                 200                 205
Val Val Ile Trp Val Val Val Ser Arg Ser Ser Thr Val Arg Lys Ile
    210                 215                 220
Gln Arg Asp Ile Ala Glu Lys Val Gly Leu Gly Gly Met Glu Trp Ser
225                 230                 235                 240
Glu Lys Asn Asp Asn Gln Ile Ala Val Asp Ile His Asn Val Leu Arg
                245                 250                 255
Arg Arg Lys Phe Val Leu Leu Leu Asp Asp Ile Trp Glu Lys Val Asn
            260                 265                 270
Leu Lys Ala Val Gly Val Pro Tyr Pro Ser Lys Asp Asn Gly Cys Lys
        275                 280                 285
Val Ala Phe Thr Thr Arg Ser Arg Asp Val Cys Gly Arg Met Gly Val
    290                 295                 300
Asp Asp Pro Met Glu Val Ser Cys Leu Gln Pro Glu Glu Ser Trp Asp
305                 310                 315                 320

Leu Phe Gln Met Lys Val Gly Lys Asn Thr Leu Gly Ser His Pro Asp
                325                 330                 335
Ile Pro Gly Leu Ala Arg Lys Val Ala Arg Lys Cys Arg Gly Leu Pro
            340                 345                 350
Leu Ala Leu Asn Val Ile Gly Glu Ala Met Ala Cys Lys Arg Thr Val
        355                 360                 365
His Glu Trp Cys His Ala Ile Asp Val Leu Thr Ser Ser Ala Ile Asp
    370                 375                 380
Phe Ser Gly Met Glu Asp Glu Ile Leu His Val Leu Lys Tyr Ser Tyr
385                 390                 395                 400
Asp Asn Leu Asn Gly Glu Leu Met Lys Ser Cys Phe Leu Tyr Cys Ser
                405                 410                 415
Leu Phe Pro Glu Asp Tyr Leu Ile Asp Lys Glu Gly Leu Val Asp Tyr
            420                 425                 430
Trp Ile Ser Glu Gly Phe Ile Asn Glu Lys Glu Gly Arg Glu Arg Asn
        435                 440                 445
Ile Asn Gln Gly Tyr Glu Ile Ile Gly Thr Leu Val Arg Ala Cys Leu
    450                 455                 460
Leu Leu Glu Glu Glu Arg Asn Lys Ser Asn Val Lys Met His Asp Val
465                 470                 475                 480
Val Arg Glu Met Ala Leu Trp Ile Ser Ser Asp Leu Gly Lys Gln Lys
                485                 490                 495
Glu Lys Cys Ile Val Arg Ala Gly Val Gly Leu Arg Glu Val Pro Lys
            500                 505                 510
Val Lys Asp Trp Asn Thr Val Arg Lys Ile Ser Leu Met Asn Asn Glu
        515                 520                 525
Ile Glu Glu Ile Phe Asp Ser His Glu Cys Ala Ala Leu Thr Thr Leu
    530                 535                 540
Phe Leu Gln Lys Asn Asp Val Val Lys Ile Ser Ala Glu Phe Phe Arg
545                 550                 555                 560
Cys Met Pro His Leu Val Val Leu Asp Leu Ser Glu Asn Gln Ser Leu
                565                 570                 575
Asn Glu Leu Pro Glu Glu Ile Ser Glu Leu Ala Ser Leu Arg Tyr Phe
            580                 585                 590
Asn Leu Ser Tyr Thr Cys Ile His Gln Leu Pro Val Gly Leu Trp Thr
        595                 600                 605
Leu Lys Lys Leu Ile His Leu Asn Leu Glu His Met Ser Ser Leu Gly
    610                 615                 620
Ser Ile Leu Gly Ile Ser Asn Leu Trp Asn Leu Arg Thr Leu Gly Leu
625                 630                 635                 640
Arg Asp Ser Arg Leu Leu Leu Asp Met Ser Leu Val Lys Glu Leu Gln
                645                 650                 655
Leu Leu Glu His Leu Glu Val Ile Thr Leu Asp Ile Ser Ser Ser Leu
            660                 665                 670
Val Ala Glu Pro Leu Leu Cys Ser Gln Arg Leu Val Glu Cys Ile Lys
        675                 680                 685
Glu Val Asp Phe Lys Tyr Leu Lys Glu Glu Ser Val Arg Val Leu Thr
    690                 695                 700
Leu Pro Thr Met Gly Asn Leu Arg Lys Leu Gly Ile Lys Arg Cys Gly
705                 710                 715                 720
Met Arg Glu Ile Lys Ile Glu Arg Thr Thr Ser Ser Ser Ser Arg Asn
                725                 730                 735
Lys Ser Pro Thr Thr Pro Cys Phe Ser Asn Leu Ser Arg Val Phe Ile
            740                 745                 750
Ala Lys Cys His Gly Leu Lys Asp Leu Thr Trp Leu Leu Phe Ala Pro
        755                 760                 765
Asn Leu Thr Phe Leu Glu Val Gly Phe Ser Lys Glu Val Glu Asp Ile
    770                 775                 780
Ile Ser Glu Glu Lys Ala Glu Glu His Ser Ala Thr Ile Val Pro Phe
785                 790                 795                 800
Arg Lys Leu Glu Thr Leu His Leu Phe Glu Leu Arg Gly Leu Lys Arg
                805                 810                 815
Ile Tyr Ala Lys Ala Leu His Phe Pro Cys Leu Lys Val Ile His Val

                820                 825                 830
Glu Lys Cys Glu Lys Leu Arg Lys Leu Pro Leu Asp Ser Lys Ser Gly
        835                 840                 845
Ile Ala Gly Glu Glu Leu Val Ile Tyr Tyr Gly Glu Arg Glu Trp Ile
    850                 855                 860
Glu Arg Val Glu Trp Glu Asp Gln Ala Thr Gln Leu Arg Phe Leu Pro
865                 870                 875                 880
Ser Ser Arg Trp Arg Trp Arg Glu Thr
                885

<210> 126
<211> 804
<212> DNA
<213> Artificial Sequence


<220>
<223> cDNA

<400> 126
atgaaaatag gtacgcaggc cacctcgctg gccgttttgc acaatcagga gtcgcatgct      60

ccacaggcac caattgctgt aagacctgag cctgctcacg caatcccaga aattccttta     120

gacctcgcca ttaggcctag aacacgtggc atacacccat tcctggcaat gacactgggc     180

gataaagggt gtgcatcctc ttcaggcgta agtctagaag atgattctca cacgcaagta     240

agcttgagtg atttttctgt ggcttcaaga gatgttaatc ataacaatat ttgtgccggc     300

ctctccacgg agtggcttgt aatgagtagt gacggagatg cagaatcacg catggaccat     360

ctcgactata acggagaggg acaaagcagg ggaagcgaaa ggcatcaggt ttataatgat     420

gcgttacgtg ccgcactatc aaatgatgat gaagcacctt ttttcacagc cagtaccgct     480

gttatcgaag atgctggttt ctctctacgc agggaaccaa aaactgttca tgcgtcaggt     540

ggcagtgcgc aattgggcca gacagtagcc catgatgtag ctcaatctgg tagaaaacat     600

ttgctgagct tacgttttgc aaacgttcaa ggacacgcaa tcgcgtgctc ctgtgaggga     660

agtcagttca agctgtttga ccctaacctc ggtgagtttc aatcatcccg cagcgcagca     720

ccgcagctca ttaagggctt aatagatcac tataatagct taaactacga cgtagcttgc     780

gtaaacgagt ttagagtttc gtaa                                            804


<210> 127
<211> 267
<212> PRT
<213> Pseudomonas syringae


<400> 127
Met Lys Ile Gly Thr Gln Ala Thr Ser Leu Ala Val Leu His Asn Gln
1               5                   10                  15
Glu Ser His Ala Pro Gln Ala Pro Ile Ala Val Arg Pro Glu Pro Ala
            20                  25                  30
His Ala Ile Pro Glu Ile Pro Leu Asp Leu Ala Ile Arg Pro Arg Thr
        35                  40                  45
Arg Gly Ile His Pro Phe Leu Ala Met Thr Leu Gly Asp Lys Gly Cys

50                  55                  60
Ala Ser Ser Ser Gly Val Ser Leu Glu Asp Asp Ser His Thr Gln Val
65                  70                  75                  80
Ser Leu Ser Asp Phe Ser Val Ala Ser Arg Asp Val Asn His Asn Asn
                85                  90                  95
Ile Cys Ala Gly Leu Ser Thr Glu Trp Leu Val Met Ser Ser Asp Gly
            100                 105                 110
Asp Ala Glu Ser Arg Met Asp His Leu Asp Tyr Asn Gly Glu Gly Gln
        115                 120                 125
Ser Arg Gly Ser Glu Arg His Gln Val Tyr Asn Asp Ala Leu Arg Ala
    130                 135                 140
Ala Leu Ser Asn Asp Asp Glu Ala Pro Phe Phe Thr Ala Ser Thr Ala
145                 150                 155                 160
Val Ile Glu Asp Ala Gly Phe Ser Leu Arg Arg Glu Pro Lys Thr Val
                165                 170                 175
His Ala Ser Gly Gly Ser Ala Gln Leu Gly Gln Thr Val Ala His Asp
            180                 185                 190
Val Ala Gln Ser Gly Arg Lys His Leu Leu Ser Leu Arg Phe Ala Asn
        195                 200                 205
Val Gln Gly His Ala Ile Ala Cys Ser Cys Glu Gly Ser Gln Phe Lys
    210                 215                 220
Leu Phe Asp Pro Asn Leu Gly Glu Phe Gln Ser Ser Arg Ser Ala Ala
225                 230                 235                 240
Pro Gln Leu Ile Lys Gly Leu Ile Asp His Tyr Asn Ser Leu Asn Tyr
                245                 250                 255
Asp Val Ala Cys Val Asn Glu Phe Arg Val Ser
            260                 265

<210> 128
<211> 2718
<212> DNA
<213> Artificial Sequence

<220>
<223> cDNA

<400> 128
atggctcatg caagtgtggc ttctcttatg agaacaatag aatctctctt gacattcaat      60

tcgccgatgc aatctctatc ctgtgatcac agagaagaac tttgcgctct tcgtgaaaaa     120

gttagttccc tggaagtatt tgtcaagaac tttgagaaaa acaatgtttt tggggaaatg     180

acggattttg aagtagaggt aagagaagtt gcaagtgctg ctgaatacac aattcaactg     240

agactaacag gaactgtact gggagaaaat aaaagccaga aaaaaaaggc gcgtcgaagg     300

tttcgtcaaa gcctgcaaca agtagcagag gacatggatc atatctggaa agagtcgaca     360

aagatccaag ataaaggaaa acaagtatca aaggaatcat tggttcatga tttttcaagt     420

tcaacaaacg atattttgaa ggttaagaac aatatggttg gacgtgatga tcaaaggaaa     480

cagttgttag aagatctgac tagaagctac tctggggaac ccaaagtcat cccgattgtc     540

gggatgggag gcataggtaa aacaacctta gcaaaagaag tttacaatga tgaatcaatt     600

ctatgccgtt ttgatgttca tgcctgggct accatatctc aacagcacaa caaaaaggaa     660

attttgctgg gccttctgca ttccacaatc aaaatggatg acagggttaa gatgattggt     720

gaagcagagc tagcagacat gttacagaaa agtttaaaga gaaagaggta cttaattgtc     780

ttggatgata tctggagttg tgaagtgtgg gatggcgtga gacgatgctt tccaactgaa 840

gacaatgcag ggagtcgaat actgttgact acccgtaatg atgaagtagc ttgttatgct 900

ggtgtagaga atttttcttt gcggatgagc ttcatggatc aagatgagag ttggagtctt 960

ttcaaaagtg cagcattttc aagtgaagca ttaccatatg agttcgagac tgttggaaag 1020

caaatcgcag atgaatgtca cgggttacca ctaactattg tcgtggttgc agggcttctc 1080

aaatctaaaa ggacaataga agattggaaa actgttgcta aagatgtcaa gtcattcgtc 1140

acaaatgatc ctgatgaacg atgttcacgt gtgcttgggt tgagttacga tcacttgaca 1200

agcgatctaa aaacatgtct tctgcatttc ggaatttttc cagaagacag tgatattcca 1260

gtgaagaatt tgatgagatc atggatggct gaggggttcc tgaagttgga aaatgatttg 1320

gaaggagagg ttgagaagtg tttgcaagag cttgtcgata gatgtctagt cctcgtcagc 1380

aagagaagtc gagatggaac aaaaattaga tcatgtaagg ttcatgatct aatatatgac 1440

ctgtgcgtga gagaagttca aagggagaac atttttatca tgaacgacat tgttcttgac 1500

gtatcatatc cagaatgttc atatctctgt atgtataaaa tgcagccctt taagcgcgtg 1560

actggtgatg aaattaatta ttgtccctat ggtctttata gggctcttct tacccctgta 1620

aatcgtcagt tgagagatca tgacaacaac aatcttttga aacgaaccca ttctgttttc 1680

tcttttcatc ttgagccttt atattatgtt ctcaaatcag aggttgttca tttcaaatta 1740

ctcaaagtct tggagctgag acacagacag attgatggtt tccctcgaga gatactaagc 1800

ctcatctggt tgaggtacct atcattgttc agctatggga atttcgatgt acctccagaa 1860

atttgcaggt tatggaatct gcagacattc attgttcaac ggtttcgatc agatataata 1920

atttttgctg aggaaatttg ggaactaatg caattaaggc atcttaaact gcccagattt 1980

tatttgccag attgcccaag tggatctgtt gacaaaggaa ggcacttgga tttttcaaac 2040

ttacaaacta tttcttactt gtctccacgt tgttgcacga aggaggttat tatggggatt 2100

cagaatgtca aaaaattagg aatcagtgga aataaggatg actataaaag ttttcgggac 2160

tctgggcttc ccaacaatct tgtctatctg cagcaacttg aaatattgag tcttatatct 2220

gttgattata gccttttgcc agtgatcatt tcaagtgcaa aagcttttcc agcaacgctc 2280

aagaagttga agttggaaag aacttatcta agctggtcat acttggacat catagctgag 2340

ttgcctaacc ttgaggtgct gaagctgatg gatgacgctt gttgtggtga agaatggcat 2400

ccaattgtta tgggatttaa tcgattgaag cttttgctaa ttaaatatag ttttctcaag 2460

ttctggaaag ccacaaatga caattttcct gtccttgagc gcctcatgat tagaagttgc 2520

aaaaatttga aagagatacc cattgagttt gcagatatac acacactaca gctgattgag 2580

ttaagagagt gtcctcccaa acttggggaa tctgctgcac gaattcagaa agaacaagaa 2640

gacctcggaa acaaccctgt ggatgttcgt atctcaaatc cattgaagga gagtgattct 2700

gattcagaag aacattag 2718

<210> 129
<211> 905
<212> PRT
<213> Capsicum chacoense

<400> 129
Met Ala His Ala Ser Val Ala Ser Leu Met Arg Thr Ile Glu Ser Leu
1               5                   10                  15
Leu Thr Phe Asn Ser Pro Met Gln Ser Leu Ser Cys Asp His Arg Glu
            20                  25                  30
Glu Leu Cys Ala Leu Arg Glu Lys Val Ser Ser Leu Glu Val Phe Val
        35                  40                  45
Lys Asn Phe Glu Lys Asn Asn Val Phe Gly Glu Met Thr Asp Phe Glu
    50                  55                  60
Val Glu Val Arg Glu Val Ala Ser Ala Ala Glu Tyr Thr Ile Gln Leu
65                  70                  75                  80
Arg Leu Thr Gly Thr Val Leu Gly Glu Asn Lys Ser Gln Lys Lys Lys
                85                  90                  95
Ala Arg Arg Arg Phe Arg Gln Ser Leu Gln Gln Val Ala Glu Asp Met
            100                 105                 110
Asp His Ile Trp Lys Glu Ser Thr Lys Ile Gln Asp Lys Gly Lys Gln
        115                 120                 125
Val Ser Lys Glu Ser Leu Val His Asp Phe Ser Ser Ser Thr Asn Asp
    130                 135                 140
Ile Leu Lys Val Lys Asn Asn Met Val Gly Arg Asp Asp Gln Arg Lys
145                 150                 155                 160
Gln Leu Leu Glu Asp Leu Thr Arg Ser Tyr Ser Gly Glu Pro Lys Val
                165                 170                 175
Ile Pro Ile Val Gly Met Gly Gly Ile Gly Lys Thr Thr Leu Ala Lys
            180                 185                 190
Glu Val Tyr Asn Asp Glu Ser Ile Leu Cys Arg Phe Asp Val His Ala
        195                 200                 205
Trp Ala Thr Ile Ser Gln Gln His Asn Lys Lys Glu Ile Leu Leu Gly
    210                 215                 220
Leu Leu His Ser Thr Ile Lys Met Asp Asp Arg Val Lys Met Ile Gly
225                 230                 235                 240
Glu Ala Glu Leu Ala Asp Met Leu Gln Lys Ser Leu Lys Arg Lys Arg
                245                 250                 255
Tyr Leu Ile Val Leu Asp Asp Ile Trp Ser Cys Glu Val Trp Asp Gly
            260                 265                 270
Val Arg Arg Cys Phe Pro Thr Glu Asp Asn Ala Gly Ser Arg Ile Leu
        275                 280                 285
Leu Thr Thr Arg Asn Asp Glu Val Ala Cys Tyr Ala Gly Val Glu Asn
    290                 295                 300
Phe Ser Leu Arg Met Ser Phe Met Asp Gln Asp Glu Ser Trp Ser Leu
305                 310                 315                 320
Phe Lys Ser Ala Ala Phe Ser Ser Glu Ala Leu Pro Tyr Glu Phe Glu
                325                 330                 335
Thr Val Gly Lys Gln Ile Ala Asp Glu Cys His Gly Leu Pro Leu Thr
            340                 345                 350
Ile Val Val Val Ala Gly Leu Leu Lys Ser Lys Arg Thr Ile Glu Asp
        355                 360                 365
Trp Lys Thr Val Ala Lys Asp Val Lys Ser Phe Val Thr Asn Asp Pro
    370                 375                 380
Asp Glu Arg Cys Ser Arg Val Leu Gly Leu Ser Tyr Asp His Leu Thr
385                 390                 395                 400
Ser Asp Leu Lys Thr Cys Leu Leu His Phe Gly Ile Phe Pro Glu Asp

                405                 410                 415
Ser Asp Ile Pro Val Lys Asn Leu Met Arg Ser Trp Met Ala Glu Gly
            420                 425                 430
Phe Leu Lys Leu Glu Asn Asp Leu Glu Gly Glu Val Glu Lys Cys Leu
        435                 440                 445
Gln Glu Leu Val Asp Arg Cys Leu Val Leu Val Ser Lys Arg Ser Arg
    450                 455                 460
Asp Gly Thr Lys Ile Arg Ser Cys Lys Val His Asp Leu Ile Tyr Asp
465                 470                 475                 480
Leu Cys Val Arg Glu Val Gln Arg Glu Asn Ile Phe Ile Met Asn Asp
                485                 490                 495
Ile Val Leu Asp Val Ser Tyr Pro Glu Cys Ser Tyr Leu Cys Met Tyr
            500                 505                 510
Lys Met Gln Pro Phe Lys Arg Val Thr Gly Asp Glu Ile Asn Tyr Cys
        515                 520                 525
Pro Tyr Gly Leu Tyr Arg Ala Leu Leu Thr Pro Val Asn Arg Gln Leu
    530                 535                 540
Arg Asp His Asp Asn Asn Asn Leu Leu Lys Arg Thr His Ser Val Phe
545                 550                 555                 560
Ser Phe His Leu Glu Pro Leu Tyr Tyr Val Leu Lys Ser Glu Val Val
                565                 570                 575
His Phe Lys Leu Leu Lys Val Leu Glu Leu Arg His Arg Gln Ile Asp
            580                 585                 590
Gly Phe Pro Arg Glu Ile Leu Ser Leu Ile Trp Leu Arg Tyr Leu Ser
        595                 600                 605
Leu Phe Ser Tyr Gly Asn Phe Asp Val Pro Pro Glu Ile Cys Arg Leu
    610                 615                 620
Trp Asn Leu Gln Thr Phe Ile Val Gln Arg Phe Arg Ser Asp Ile Ile
625                 630                 635                 640
Ile Phe Ala Glu Glu Ile Trp Glu Leu Met Gln Leu Arg His Leu Lys
                645                 650                 655
Leu Pro Arg Phe Tyr Leu Pro Asp Cys Pro Ser Gly Ser Val Asp Lys
            660                 665                 670
Gly Arg His Leu Asp Phe Ser Asn Leu Gln Thr Ile Ser Tyr Leu Ser
        675                 680                 685
Pro Arg Cys Cys Thr Lys Glu Val Ile Met Gly Ile Gln Asn Val Lys
    690                 695                 700
Lys Leu Gly Ile Ser Gly Asn Lys Asp Asp Tyr Lys Ser Phe Arg Asp
705                 710                 715                 720
Ser Gly Leu Pro Asn Asn Leu Val Tyr Leu Gln Gln Leu Glu Ile Leu
                725                 730                 735
Ser Leu Ile Ser Val Asp Tyr Ser Leu Leu Pro Val Ile Ile Ser Ser
            740                 745                 750
Ala Lys Ala Phe Pro Ala Thr Leu Lys Lys Leu Lys Leu Glu Arg Thr
        755                 760                 765
Tyr Leu Ser Trp Ser Tyr Leu Asp Ile Ile Ala Glu Leu Pro Asn Leu
    770                 775                 780
Glu Val Leu Lys Leu Met Asp Asp Ala Cys Cys Gly Glu Glu Trp His
785                 790                 795                 800
Pro Ile Val Met Gly Phe Asn Arg Leu Lys Leu Leu Leu Ile Lys Tyr
                805                 810                 815
Ser Phe Leu Lys Phe Trp Lys Ala Thr Asn Asp Asn Phe Pro Val Leu
            820                 825                 830
Glu Arg Leu Met Ile Arg Ser Cys Lys Asn Leu Lys Glu Ile Pro Ile
        835                 840                 845
Glu Phe Ala Asp Ile His Thr Leu Gln Leu Ile Glu Leu Arg Glu Cys
    850                 855                 860
Pro Pro Lys Leu Gly Glu Ser Ala Ala Arg Ile Gln Lys Glu Gln Glu
865                 870                 875                 880
Asp Leu Gly Asn Asn Pro Val Asp Val Arg Ile Ser Asn Pro Leu Lys
                885                 890                 895
Glu Ser Asp Ser Asp Ser Glu Glu His
            900                 905

<210> 130
<211> 2145
<212> DNA
<213> Artificial Sequence

<220>
<223> cDNA

<400> 130
atgcgtatcg gtcctctgca accttctatc gcgcacactg ccgcgccggc actaccgacg 60

cacaccagtg cgatcagccc gacgcaggtg ccgcacatgc cgggcaacac cccgccactg 120

cgagaaaggc cgcgtcgccg cgccggcaat atgcctccgc tggtgccgtt gaacgacagc 180

gcgatgaccg gcaagccggc cctggtggcg ctcgacagcg aattctccga gcagcgtctg 240

gccgaagtgc aggcgcgcca gatcaccgtg cagaccctgc aaggcaagct tgccacgcat 300

cttgcgcagg ccggcacggc gctcaaaccc gacagcattg cggcacgctt tgctgccggc 360

acactggagc cggtgtatct ggataccgcc gccttcaatg ccatgtcgcg cgggctgccc 420

gcacgcgcac gtgcggccgc aggcccggtg ctgatcgatg cacaacaagg tcgcatcatc 480

ttcaatctgc agcgcgcgtt tgcgcctggc gacaccttca gcgacgcggc gcttgccgcg 540

cttggcaagc agttgaatct ttccggccac gggctggcaa cgccgaactg gctgcagcct 600

gccgcaggca cgccggggcg gcgcaagctg cagcaagccg cgcgctatca cggccacgag 660

gtgccggccc gcgacggtgg cgccgggttc ttcaaggcca acgaccatcg cctgctggaa 720

ggcaagcaag tgctgttgcg caatcatcaa aagtcgctcg tgcacaacca ctacttcgaa 780

gcacccagca cgcgtgcgtt cggaaaggac gtcatggtgc atcgcgggct gttcgataat 840

cacgccggca ttccggaaaa ctcgctggcg tccatcgatc atgcctacga gcagggctac 900

cgcaatctgg agctggacgt cgaagtcagt tccgatggcg tgccggtgtt gatgcacgat 960

ttcagcatcg gccgcatggc aggcgacccg cagaaccggt tggtgtcgca ggtgccgttt 1020

gccgagctgc gtgaaatgcc gttggtgatt cgcaacccgt ctgacggaaa ctacgtcaag 1080

accgaccaga ccatcgccgg tgtggagcag atgctggagc acgtgctcaa aaagcccgag 1140

ccgatgtcgg tggcgctgga ctgcaaggaa aacaccggcg aagcagtggc gatgctgctg 1200

atgcgccggc cggacctgcg caaggctgcg gcgatcaagg tctatgccaa gtactacacg 1260

ggcggctttg accaattcct gtccaatttg tacaagcact accagatcaa cccgttgcac 1320

tcgcaggatg cgccgcgccg cgccgcgctg gatcgcttgc tggccaagat caacgtggtg 1380

ccggtcttga gccagggcat gttgaacgac gagcgcttgc gcggcttctt tcgaagcaat 1440

gagcagggcg ccgcggggct cgcagacacc gcaatgcagt ggctggacag ctggaccaag 1500

atgcgcccgg tgatcgtgga ggcggtggcc accgacgaca gcgatgccgg caaggccatg 1560

gaaatggctc ggacgcggat gcgccagccg gactcggcct acgcgaaggc cgcgtattcg 1620

gtgagctacc ggtatgagga cttttccgtg ccgcgcgcca atcacgacaa ggactactac 1680

gtttaccgca acttcggtga actgcaaaag ctcaccaacg aggccttcgg cgtcaagcgc 1740

accacggccg gcgcgtttcg cgacgatggc gaaagcctgt tgaccgatca gcccgaggcc 1800

gaattgctcg ccatcctgga aaaccgcgcg ctggcccggg gccataccgg caatgaactc 1860

gacgtgccgc cggaaacgcc catcgatatc aaccgggatg ccgagatcgt gaagcagcga 1920

acccagcaat ttcaggccag ctccatcccc gctgacccga accacatctc cgcggttcgc 1980

gaaggcaagc aacacgatca caccgcagac atggtcaatg accctgcggc aacgcgtgcg 2040

ctggacaagc gcgccaaagc gcttggtttg ctgaccgaca aataccgtgg cgcgcctgtg 2100

acccactatc tcaatgagca ggccaggcag acggagacgg attga 2145

<210> 131
<211> 714
<212> PRT
<213> Xanthomonas campestris

<400> 131
Met Arg Ile Gly Pro Leu Gln Pro Ser Ile Ala His Thr Ala Ala Pro
1               5                   10                  15
Ala Leu Pro Thr His Thr Ser Ala Ile Ser Pro Thr Gln Val Pro His
            20                  25                  30
Met Pro Gly Asn Thr Pro Pro Leu Arg Glu Arg Pro Arg Arg Arg Ala
        35                  40                  45
Gly Asn Met Pro Pro Leu Val Pro Leu Asn Asp Ser Ala Met Thr Gly
    50                  55                  60
Lys Pro Ala Leu Val Ala Leu Asp Ser Glu Phe Ser Glu Gln Arg Leu
65                  70                  75                  80
Ala Glu Val Gln Ala Arg Gln Ile Thr Val Gln Thr Leu Gln Gly Lys
                85                  90                  95
Leu Ala Thr His Leu Ala Gln Ala Gly Thr Ala Leu Lys Pro Asp Ser
            100                 105                 110
Ile Ala Ala Arg Phe Ala Ala Gly Thr Leu Glu Pro Val Tyr Leu Asp
        115                 120                 125
Thr Ala Ala Phe Asn Ala Met Ser Arg Gly Leu Pro Ala Arg Ala Arg
    130                 135                 140
Ala Ala Ala Gly Pro Val Leu Ile Asp Ala Gln Gln Gly Arg Ile Ile
145                 150                 155                 160
Phe Asn Leu Gln Arg Ala Phe Ala Pro Gly Asp Thr Phe Ser Asp Ala
                165                 170                 175
Ala Leu Ala Ala Leu Gly Lys Gln Leu Asn Leu Ser Gly His Gly Leu
            180                 185                 190
Ala Thr Pro Asn Trp Leu Gln Pro Ala Ala Gly Thr Pro Gly Arg Arg
        195                 200                 205
Lys Leu Gln Gln Ala Ala Arg Tyr His Gly His Glu Val Pro Ala Arg
    210                 215                 220
Asp Gly Gly Ala Gly Phe Phe Lys Ala Asn Asp His Arg Leu Leu Glu
225                 230                 235                 240
Gly Lys Gln Val Leu Leu Arg Asn His Gln Lys Ser Leu Val His Asn
                245                 250                 255
His Tyr Phe Glu Ala Pro Ser Thr Arg Ala Phe Gly Lys Asp Val Met
            260                 265                 270
Val His Arg Gly Leu Phe Asp Asn His Ala Gly Ile Pro Glu Asn Ser

        275                 280                 285
Leu Ala Ser Ile Asp His Ala Tyr Glu Gln Gly Tyr Arg Asn Leu Glu
    290                 295                 300
Leu Asp Val Glu Val Ser Ser Asp Gly Val Pro Val Leu Met His Asp
305                 310                 315                 320
Phe Ser Ile Gly Arg Met Ala Gly Asp Pro Gln Asn Arg Leu Val Ser
                325                 330                 335
Gln Val Pro Phe Ala Glu Leu Arg Glu Met Pro Leu Val Ile Arg Asn
            340                 345                 350
Pro Ser Asp Gly Asn Tyr Val Lys Thr Asp Gln Thr Ile Ala Gly Val
        355                 360                 365
Glu Gln Met Leu Glu His Val Leu Lys Lys Pro Glu Pro Met Ser Val
    370                 375                 380
Ala Leu Asp Cys Lys Glu Asn Thr Gly Glu Ala Val Ala Met Leu Leu
385                 390                 395                 400
Met Arg Arg Pro Asp Leu Arg Lys Ala Ala Ala Ile Lys Val Tyr Ala
                405                 410                 415
Lys Tyr Tyr Thr Gly Gly Phe Asp Gln Phe Leu Ser Asn Leu Tyr Lys
            420                 425                 430
His Tyr Gln Ile Asn Pro Leu His Ser Gln Asp Ala Pro Arg Arg Ala
        435                 440                 445
Ala Leu Asp Arg Leu Leu Ala Lys Ile Asn Val Val Pro Val Leu Ser
    450                 455                 460
Gln Gly Met Leu Asn Asp Glu Arg Leu Arg Gly Phe Phe Arg Ser Asn
465                 470                 475                 480
Glu Gln Gly Ala Ala Gly Leu Ala Asp Thr Ala Met Gln Trp Leu Asp
                485                 490                 495
Ser Trp Thr Lys Met Arg Pro Val Ile Val Glu Ala Val Ala Thr Asp
            500                 505                 510
Asp Ser Asp Ala Gly Lys Ala Met Glu Met Ala Arg Thr Arg Met Arg
        515                 520                 525
Gln Pro Asp Ser Ala Tyr Ala Lys Ala Ala Tyr Ser Val Ser Tyr Arg
    530                 535                 540
Tyr Glu Asp Phe Ser Val Pro Arg Ala Asn His Asp Lys Asp Tyr Tyr
545                 550                 555                 560
Val Tyr Arg Asn Phe Gly Glu Leu Gln Lys Leu Thr Asn Glu Ala Phe
                565                 570                 575
Gly Val Lys Arg Thr Thr Ala Gly Ala Phe Arg Asp Asp Gly Glu Ser
            580                 585                 590
Leu Leu Thr Asp Gln Pro Glu Ala Glu Leu Leu Ala Ile Leu Glu Asn
        595                 600                 605
Arg Ala Leu Ala Arg Gly His Thr Gly Asn Glu Leu Asp Val Pro Pro
    610                 615                 620
Glu Thr Pro Ile Asp Ile Asn Arg Asp Ala Glu Ile Val Lys Gln Arg
625                 630                 635                 640
Thr Gln Gln Phe Gln Ala Ser Ser Ile Pro Ala Asp Pro Asn His Ile
                645                 650                 655
Ser Ala Val Arg Glu Gly Lys Gln His Asp His Thr Ala Asp Met Val
            660                 665                 670
Asn Asp Pro Ala Ala Thr Arg Ala Leu Asp Lys Arg Ala Lys Ala Leu
        675                 680                 685
Gly Leu Leu Thr Asp Lys Tyr Arg Gly Ala Pro Val Thr His Tyr Leu
    690                 695                 700
Asn Glu Gln Ala Arg Gln Thr Glu Thr Asp
705                 710

<210> 132
<211> 2718
<212> DNA
<213> Artificial Sequence

<220>

<223> cDNA

<400> 132
atggcagaga ttgctgttct tctcgtcctg aagaagattg ccatagctct ggcaggagag 60

accctaagtt ttgctaaacc attgcttgca aagaagtctg agtcggtggc agcactccca 120

gatgacatga aactgattag taatgagctc gagcttatcc gggcgtttct caaggaaatc 180

ggcaggaaag gctggaaaag cgaagtgata gaaacatgga tagggcaggt ccgaagactg 240

gcttatgata tggaagacac tgtagaccat tttatttacg ttgttggtac acacgatcag 300

atgggatcat gctgggatta catgaagaag atagccaaga agcctcggcg tctggtttca 360

ctagatgaaa ttgctagtga gattaagaag ataaagcaag agcttaaaca actctcggaa 420

agtagagacc gctggactaa acccttggat ggcgggagtg gtatacctgc aggaagctat 480

gaaactgaaa aggaaatgta tcttcctgga catgattaca caatcagcga cgaggagctt 540

gcaggaattg atgaaaataa gcaaaccttg attagttcat taaaatttga agatccatca 600

cttcggatca ttgctgtctg gggtatgggt ggcgttggaa aaagcactct tgtaaataat 660

gtgtacaaaa atgaaggatc caactttgac tgccgtgcat gggtttctat ctctcagtca 720

tatagactag aagatatatg gaagaaaatg ctgacagatc tcatcgggaa agataagata 780

gaatttgatc ttggaacaat ggatagtgca gaactaagag agcaattgac aaaaactcta 840

gacaaaaggc agtacctgat catactggat gatgtctgga tggctaatgt tttttttaaa 900

attaaagaag ttcttgtaga taatggcctt ggaagcagag taataatcac aacaagaatt 960

gaggaggtag cttcactagc taagggtagt tgtaagatca aagtagaacc tctgggtgtc 1020

gatgattcct ggcatgtatt ttgtaggaag gcatttctga aagatgaaaa ccatatctgc 1080

cctccagagt tgcgtcagtg tggtataaac attgtggaga aatgtgatgg tttgccatta 1140

gcccttgtgg caataggaag catattgtcg ctgagaccga agaatgttga cgagtggaag 1200

ctattttatg accagcttat ctgggagcta cacaacaatg agaaccttaa tcgcgtggag 1260

aaaattatga atctaagtta taaatactta ccagactatt tgaagaactg cttcctgtat 1320

tgtgctatgt ttccagaaga ctatctaata cacagaaaga gattgattag attgtggata 1380

gctgaaggat ttattgaaca aaaaggggca tgcagcttag aagacactgc tgaaagttat 1440

cttaaagaac ttatacgacg gagcatgctt cacgttgcag agaggaactg ctttggtagg 1500

attaaatgta ttcgaatgca tgatcttgtg cgtgaacttg ccattttcca atctaaaaga 1560

gagggtttca gtacaactta tggtggaaat aatgaagcag tgctagtggg atcatattct 1620

cgacgagtgg ctgtgctcca atgcagcaag ggcattccat caaccattga tccatccagg 1680

cttcgcacct taattacatt cgacaccagc agagcattat ctgtgtggta ttcttctatt 1740

tcctccaaac caaagtacct tgcagtatta gacttatcaa gcttgcctat tgagactatt 1800

ccaaattcaa ttggagagct tttcaacctt aggcttttat gcctcaataa aaccaaagtg 1860

aaagagctcc ctaaatctat tacaaaactt caaaacctac agacaatgag tcttgagaat 1920

ggggagttag tgaagttccc acaagggttt tcaaaactga agaaattgcg acatcttatg 1980

gtttcacggt tgcaagatgt aactttcagc ggtttcaaaa gttgggaagc tgtggagcca 2040

tttaagggct tgtggacttt gattgaactg caaactctgt atgccattac agcaagtgaa 2100

gtattagttg caaaacttgg aaatttatcc cagctgaggc ggcttataat ttgtgatgta 2160

aggagtaact tatgtgcaca gttgtgtggc tctttgtcaa agttgtgcca gctgtcacga 2220

ttaacgataa gagcatgcaa tgaagatgaa gtgctacagc tggatcattt gacatttcca 2280

aatcctcttc aaacccttag tttagatgga cgactgtcag aaggaacttt caaatctccc 2340

tttttcttaa atcatggaaa tgggcttctt aggctaatgt tgttttacag tcagctttca 2400

gaaaatccag taccacacct ctctgaattg tcaaacttga ctaggttatc ccttataaag 2460

gcatacaccg gccaagaatt atacttccaa gcaggttggt tcctgaattt aaaagaactt 2520

tacttgaaga atttgtcccg gctcaatcaa atagacatac aggagggagc tttggccagc 2580

cttgaacgta taacaatgaa acacctcccg gagctacggg aggttccagt cggtttcaga 2640

tttctcaagt ccctaaaaac aatatttttt tccgatatgc atcctgagtt tgaaagcagc 2700

tttcaaaagg aaatgtag 2718

<210> 133
<211> 905
<212> PRT
<213> Zea mays

<400> 133
Met Ala Glu Ile Ala Val Leu Leu Val Leu Lys Lys Ile Ala Ile Ala
1               5                   10                  15
Leu Ala Gly Glu Thr Leu Ser Phe Ala Lys Pro Leu Leu Ala Lys Lys
            20                  25                  30
Ser Glu Ser Val Ala Ala Leu Pro Asp Asp Met Lys Leu Ile Ser Asn
        35                  40                  45
Glu Leu Glu Leu Ile Arg Ala Phe Leu Lys Glu Ile Gly Arg Lys Gly
    50                  55                  60
Trp Lys Ser Glu Val Ile Glu Thr Trp Ile Gly Gln Val Arg Arg Leu
65                  70                  75                  80
Ala Tyr Asp Met Glu Asp Thr Val Asp His Phe Ile Tyr Val Val Gly
                85                  90                  95
Thr His Asp Gln Met Gly Ser Cys Trp Asp Tyr Met Lys Lys Ile Ala
            100                 105                 110
Lys Lys Pro Arg Arg Leu Val Ser Leu Asp Glu Ile Ala Ser Glu Ile
        115                 120                 125
Lys Lys Ile Lys Gln Glu Leu Lys Gln Leu Ser Glu Ser Arg Asp Arg
    130                 135                 140
Trp Thr Lys Pro Leu Asp Gly Gly Ser Gly Ile Pro Ala Gly Ser Tyr
145                 150                 155                 160
Glu Thr Glu Lys Glu Met Tyr Leu Pro Gly His Asp Tyr Thr Ile Ser
                165                 170                 175
Asp Glu Glu Leu Ala Gly Ile Asp Glu Asn Lys Gln Thr Leu Ile Ser

            180                 185                 190
Ser Leu Lys Phe Glu Asp Pro Ser Leu Arg Ile Ile Ala Val Trp Gly
        195                 200                 205
Met Gly Gly Val Gly Lys Ser Thr Leu Val Asn Asn Val Tyr Lys Asn
    210                 215                 220
Glu Gly Ser Asn Phe Asp Cys Arg Ala Trp Val Ser Ile Ser Gln Ser
225                 230                 235                 240
Tyr Arg Leu Glu Asp Ile Trp Lys Lys Met Leu Thr Asp Leu Ile Gly
                245                 250                 255
Lys Asp Lys Ile Glu Phe Asp Leu Gly Thr Met Asp Ser Ala Glu Leu
            260                 265                 270
Arg Glu Gln Leu Thr Lys Thr Leu Asp Lys Arg Gln Tyr Leu Ile Ile
        275                 280                 285
Leu Asp Asp Val Trp Met Ala Asn Val Phe Phe Lys Ile Lys Glu Val
    290                 295                 300
Leu Val Asp Asn Gly Leu Gly Ser Arg Val Ile Ile Thr Thr Arg Ile
305                 310                 315                 320
Glu Glu Val Ala Ser Leu Ala Lys Gly Ser Cys Lys Ile Lys Val Glu
                325                 330                 335
Pro Leu Gly Val Asp Asp Ser Trp His Val Phe Cys Arg Lys Ala Phe
            340                 345                 350
Leu Lys Asp Glu Asn His Ile Cys Pro Pro Glu Leu Arg Gln Cys Gly
        355                 360                 365
Ile Asn Ile Val Glu Lys Cys Asp Gly Leu Pro Leu Ala Leu Val Ala
    370                 375                 380
Ile Gly Ser Ile Leu Ser Leu Arg Pro Lys Asn Val Asp Glu Trp Lys
385                 390                 395                 400
Leu Phe Tyr Asp Gln Leu Ile Trp Glu Leu His Asn Asn Glu Asn Leu
                405                 410                 415
Asn Arg Val Glu Lys Ile Met Asn Leu Ser Tyr Lys Tyr Leu Pro Asp
            420                 425                 430
Tyr Leu Lys Asn Cys Phe Leu Tyr Cys Ala Met Phe Pro Glu Asp Tyr
        435                 440                 445
Leu Ile His Arg Lys Arg Leu Ile Arg Leu Trp Ile Ala Glu Gly Phe
    450                 455                 460
Ile Glu Gln Lys Gly Ala Cys Ser Leu Glu Asp Thr Ala Glu Ser Tyr
465                 470                 475                 480
Leu Lys Glu Leu Ile Arg Arg Ser Met Leu His Val Ala Glu Arg Asn
                485                 490                 495
Cys Phe Gly Arg Ile Lys Cys Ile Arg Met His Asp Leu Val Arg Glu
            500                 505                 510
Leu Ala Ile Phe Gln Ser Lys Arg Glu Gly Phe Ser Thr Thr Tyr Gly
        515                 520                 525
Gly Asn Asn Glu Ala Val Leu Val Gly Ser Tyr Ser Arg Arg Val Ala
    530                 535                 540
Val Leu Gln Cys Ser Lys Gly Ile Pro Ser Thr Ile Asp Pro Ser Arg
545                 550                 555                 560
Leu Arg Thr Leu Ile Thr Phe Asp Thr Ser Arg Ala Leu Ser Val Trp
                565                 570                 575
Tyr Ser Ser Ile Ser Ser Lys Pro Lys Tyr Leu Ala Val Leu Asp Leu
            580                 585                 590
Ser Ser Leu Pro Ile Glu Thr Ile Pro Asn Ser Ile Gly Glu Leu Phe
        595                 600                 605
Asn Leu Arg Leu Leu Cys Leu Asn Lys Thr Lys Val Lys Glu Leu Pro
    610                 615                 620
Lys Ser Ile Thr Lys Leu Gln Asn Leu Gln Thr Met Ser Leu Glu Asn
625                 630                 635                 640
Gly Glu Leu Val Lys Phe Pro Gln Gly Phe Ser Lys Leu Lys Lys Leu
                645                 650                 655
Arg His Leu Met Val Ser Arg Leu Gln Asp Val Thr Phe Ser Gly Phe
            660                 665                 670
Lys Ser Trp Glu Ala Val Glu Pro Phe Lys Gly Leu Trp Thr Leu Ile
        675                 680                 685

Glu Leu Gln Thr Leu Tyr Ala Ile Thr Ala Ser Glu Val Leu Val Ala
    690                 695                 700
Lys Leu Gly Asn Leu Ser Gln Leu Arg Arg Leu Ile Ile Cys Asp Val
705                 710                 715                 720
Arg Ser Asn Leu Cys Ala Gln Leu Cys Gly Ser Leu Ser Lys Leu Cys
                725                 730                 735
Gln Leu Ser Arg Leu Thr Ile Arg Ala Cys Asn Glu Asp Glu Val Leu
            740                 745                 750
Gln Leu Asp His Leu Thr Phe Pro Asn Pro Leu Gln Thr Leu Ser Leu
        755                 760                 765
Asp Gly Arg Leu Ser Glu Gly Thr Phe Lys Ser Pro Phe Phe Leu Asn
    770                 775                 780
His Gly Asn Gly Leu Leu Arg Leu Met Leu Phe Tyr Ser Gln Leu Ser
785                 790                 795                 800
Glu Asn Pro Val Pro His Leu Ser Glu Leu Ser Asn Leu Thr Arg Leu
                805                 810                 815
Ser Leu Ile Lys Ala Tyr Thr Gly Gln Glu Leu Tyr Phe Gln Ala Gly
            820                 825                 830
Trp Phe Leu Asn Leu Lys Glu Leu Tyr Leu Lys Asn Leu Ser Arg Leu
        835                 840                 845
Asn Gln Ile Asp Ile Gln Glu Gly Ala Leu Ala Ser Leu Glu Arg Ile
    850                 855                 860
Thr Met Lys His Leu Pro Glu Leu Arg Glu Val Pro Val Gly Phe Arg
865                 870                 875                 880
Phe Leu Lys Ser Leu Lys Thr Ile Phe Phe Ser Asp Met His Pro Glu
                885                 890                 895
Phe Glu Ser Ser Phe Gln Lys Glu Met
            900                 905

<210> 134
<211> 1266
<212> DNA
<213> Artificial Sequence

<220>
<223> cDNA

<400> 134
atgaaaaaca agacagacat tgctggcata gccggacttt ctacgtctca tgtttccttt      60

gatgacgcag aatccaaaag cgtcctagat ggatcaacac agtctacgcc ggagtacaaa     120

ttccagaccc cgcttgatca gttgccggct aggcccgctg gtccgaagag cagtaacaca     180

actgatgctt acattcatga atcactgccg cattcggttc caatcgcagg tgcatcgacc     240

tccgcggtga cacatcaaca ggctactcag cgtaaaggca ccgatgagat ctatggtttg     300

ggcagcctcc catctgctgg tccagggcga tgggagtatc ttgcgaatcc ggggaactgg     360

catcccgagc gccgtaagtt acacgaaaag ctattagatc aagcgcgatc ttctgccttg     420

accctggcag aatcccttga gagtgatggc tgccaaccga cattgtttgc gctgcgcgga     480

aataccgcaa cgggtaaaac acggattgcg acaaaaaaga tcccggtgct cgcagcagct     540

ctgaagaaga cagctgggaa aggttgcgtg aatccagatg tattcaagag ttcgcttgcg     600

aaatccgaga caggcgccaa gatatttagt tcggcacaag tgcatagcga gtcatctttt     660

cttgccgacc ggtttgaagg cggacttcgc tctcaaaaga cgggcagcgg tgcaatcgct     720

agcattgttg tcgacaagcg cttgtcaaga gagtacgaaa ttgatagcta catacaacta 780

gcgaaagaaa ccggtagaaa agtggaattg tgcgatatcg acgcgcccct agagaattct 840

ttggtgggag ttttgcagcg aaaaccggaa ggtgaagatc ctaggcctcc gtaccctgta 900

gtctcgagtg gatttgtagc cgtacggagc aatagaatgt acgtgataga caggttcatt 960

gccgatccga gcctgggaaa ctaccgacta ttcggaacag ctgaagatgg tgagaaggtc 1020

atggttgcta gcgtgattgg cggtgagttt agtgtagaga atgctgagtt gtatgaaaaa 1080

atcacgtccc cacagctctc ggttacgggc gatttggctg ataaggtaat cgacaaggaa 1140

ttgattgatc gtctggaaaa taatatagct gaccccgagc gggcggccaa gacgcgagcc 1200

gccctggaaa aatacagcgg taagtcatgg tcggccgcgc tagctgctca cagcgagcta 1260

atttga 1266

<210> 135
<211> 421
<212> PRT
<213> Xanthomonas oryzae

<400> 135
Met Lys Asn Lys Thr Asp Ile Ala Gly Ile Ala Gly Leu Ser Thr Ser
1               5                   10                  15
His Val Ser Phe Asp Asp Ala Glu Ser Lys Ser Val Leu Asp Gly Ser
            20                  25                  30
Thr Gln Ser Thr Pro Glu Tyr Lys Phe Gln Thr Pro Leu Asp Gln Leu
        35                  40                  45
Pro Ala Arg Pro Ala Gly Pro Lys Ser Ser Asn Thr Thr Asp Ala Tyr
    50                  55                  60
Ile His Glu Ser Leu Pro His Ser Val Pro Ile Ala Gly Ala Ser Thr
65                  70                  75                  80
Ser Ala Val Thr His Gln Gln Ala Thr Gln Arg Lys Gly Thr Asp Glu
                85                  90                  95
Ile Tyr Gly Leu Gly Ser Leu Pro Ser Ala Gly Pro Gly Arg Trp Glu
            100                 105                 110
Tyr Leu Ala Asn Pro Gly Asn Trp His Pro Glu Arg Arg Lys Leu His
        115                 120                 125
Glu Lys Leu Leu Asp Gln Ala Arg Ser Ser Ala Leu Thr Leu Ala Glu
    130                 135                 140
Ser Leu Glu Ser Asp Gly Cys Gln Pro Thr Leu Phe Ala Leu Arg Gly
145                 150                 155                 160
Asn Thr Ala Thr Gly Lys Thr Arg Ile Ala Thr Lys Lys Ile Pro Val
                165                 170                 175
Leu Ala Ala Ala Leu Lys Lys Thr Ala Gly Lys Gly Cys Val Asn Pro
            180                 185                 190
Asp Val Phe Lys Ser Ser Leu Ala Lys Ser Glu Thr Gly Ala Lys Ile
        195                 200                 205
Phe Ser Ser Ala Gln Val His Ser Glu Ser Ser Phe Leu Ala Asp Arg
    210                 215                 220
Phe Glu Gly Gly Leu Arg Ser Gln Lys Thr Gly Ser Gly Ala Ile Ala
225                 230                 235                 240
Ser Ile Val Val Asp Lys Arg Leu Ser Arg Glu Tyr Glu Ile Asp Ser
                245                 250                 255
Tyr Ile Gln Leu Ala Lys Glu Thr Gly Arg Lys Val Glu Leu Cys Asp
            260                 265                 270
Ile Asp Ala Pro Leu Glu Asn Ser Leu Val Gly Val Leu Gln Arg Lys

275                 280                 285
Pro Glu Gly Glu Asp Pro Arg Pro Pro Tyr Pro Val Val Ser Ser Gly
    290                 295                 300
Phe Val Ala Val Arg Ser Asn Arg Met Tyr Val Ile Asp Arg Phe Ile
305                 310                 315                 320
Ala Asp Pro Ser Leu Gly Asn Tyr Arg Leu Phe Gly Thr Ala Glu Asp
                325                 330                 335
Gly Glu Lys Val Met Val Ala Ser Val Ile Gly Gly Glu Phe Ser Val
            340                 345                 350
Glu Asn Ala Glu Leu Tyr Glu Lys Ile Thr Ser Pro Gln Leu Ser Val
        355                 360                 365
Thr Gly Asp Leu Ala Asp Lys Val Ile Asp Lys Glu Leu Ile Asp Arg
    370                 375                 380
Leu Glu Asn Asn Ile Ala Asp Pro Glu Arg Ala Ala Lys Thr Arg Ala
385                 390                 395                 400
Ala Leu Glu Lys Tyr Ser Gly Lys Ser Trp Ser Ala Ala Leu Ala Ala
                405                 410                 415
His Ser Glu Leu Ile
            420

<210> 136
<211> 3801
<212> DNA
<213> Artificial Sequence

<220>
<223> cDNA

<400> 136
atggagattg gcttagcagt tggtggtgca tttctctcct cagctttgaa tgttctgttt      60

gataggcttg ctcctaacgg tgatctgctc aacatgtttc ggaagcataa ggatcatgtt     120

aagctcttaa agaagctgaa aatgactttg cgtggtattc agattgtgct aagtgatgca     180

gagaataagc aagcatcaaa tccatctgtg agagactggc ttaatgagct tcgagatgct     240

gtcgactctg ctgaaaattt aatagaagaa gtcaattatg aagctttgag gcttaaggtg     300

gaaggtcagc atcagaattt ttcagaaaca agcaaccagc aagtaagtga tgattttttc      360

cttaacataa aggacaagct ggaagacact attgaaacat taaaggattt gcaagagcaa     420

attggtctcc ttggcttaaa ggagtatttt gattccacga aactagaaac tagaagacct     480

tcaacttctg tggatgatga atctgatatc tttggtaggc agagcgaaat agaggatttg     540

attgaccgtc tattgtctga aggtgcaagt gggaaaaagc tgacagtagt tcctatcgtt     600

ggaatgggcg gccagggcaa gacaacactt gctaaagccg tatacaatga tgagagggtg     660

aagaatcatt ttgatttgaa agcgtggtat tgcgtttctg aaggatttga tgctttgaga     720

ataacaaaag aattactcca agaaattggc aaatttgact cgaaggatgt ccacaacaat     780

cttaaccagc ttcaagtcaa attgaaggaa agtttgaagg gaaagaagtt ccttattgtt     840

ttggatgatg tgtggaatga aaattacaac gagtggaatg acttgagaaa tatttttgca      900

caaggagata taggaagtaa gatcattgtg acgacacgca aagacagtgt tgccttgatg     960

atgggaaatg agcaaattcg catgggcaat ttgtctaccg aagcctcttg gtctttattt    1020

caaagacatg catttgaaaa catggatcct atgggacatc cggaacttga agaggtcgga 1080

agacaaattg cagccaagtg caaaggactg cccttagctc tgaagacgct cgctggcatg 1140

ttacgctcca aatcagaggt tgaagagtgg aaacgtattt tgagaagtga aatatgggag 1200

ctgccacaca atgacatatt accagcgttg atgttgagct acaatgatct tcccgcacat 1260

ttaaagcgat gcttttcttt ttgtgcaata tttcctaaag attatccatt taggaaagaa 1320

caagttattc atctatggat tgccaatggt ctcgtaccag tgaaagatga aataaatcaa 1380

gatttaggca accaatactt tctagagttg agatcaagat cattatttga aaaggtccca 1440

aatccttcta aaaggaacat agaggaatta ttccttatgc atgaccttgt caatgattta 1500

gcccaacttg catcttcaaa actttgtatc aggttagaag agagccaagg atctcatatg 1560

ttggaacaat gtcggcactt atcttattca ataggattta atggtgagtt taagaaattg 1620

acacccctct acaaattgga gcagttgagg acattgcttc cgatacgtat tgaattcaga 1680

ttgcacaatc taagcaagag ggtgttgcat aacatactgc ctacactaag atccttgagg 1740

gccctatcat tctctcaata caagattaag gagttgccaa atgacttgtt taccaaatta 1800

aagctcctca gatttttgga tatttctcgg acatggatta caaagttgcc ggattccatt 1860

tgtggattat ataacttgga gacacttctc ctgtcatctt gtgctgatct tgaggagcta 1920

ccgctgcaga tggagaagtt gattaacttg cgtcatcttg acgtaagcaa cactcggcgc 1980

ttgaagatgc cactacatct gagcaggttg aaaagcctcc aagtgttggt gggacccaag 2040

ttttttgtag atggttggag aatggaagat ttgggtgaag cacaaaactt acatggatct 2100

ctatcagttg tgaagttgga aaatgtggtt gatagaaggg aagctgtgaa ggcaaagatg 2160

agggagaaga atcatgttga gcaattatca ttggagtgga gtgaaagtag tattgccgac 2220

aattcacaaa cagaaagtga catacttgat gagctatgcc cacataaaaa catcaaaaaa 2280

gtcgaaatca gtggatatag agggacaaac tttcccaatt gggtagctga tcctttgttt 2340

cttaagctgg tgaatttgtc tctaagaaac tgcaaggact gttactcctt gccagcacta 2400

ggacaactcc cttgtttgaa attcctttcc gttaaaggga tgcatggaat aagagtggtg 2460

acggaagaat tctatggcag attgtcctcc aaaaagcctt ttaactctct agagaagctt 2520

gaatttgaag atatgacgga gtggaagcaa tggcacgcac taggaattgg agagttccct 2580

acacttgaga acctttcaat taaaaattgc cctgagctca gtttggagat acccatccaa 2640

ttttcaagtt taaaaaggtt agaagttagt gattgtccag ttgtttttga tgatgcccaa 2700

ctgtttagat cccaacttga ggcaatgaag cagattgagg aaatagatat atgtgattgt 2760

aactctgtta cctcctttcc ttttagcata ctgccaacta ccttgaagag aatacagata 2820

tctcgttgcc caaaattgaa attggaggcg ccagttggtg agatgtttgt ggagtatttg 2880

agagtgaatg attgtggttg tgtagatgat atatcacctg agtttctccc aacagcacgt 2940

caattgagta ttgaaaattg ccagaacgtt actaggtttt tgattcctac tgccactgaa 3000

actctccgta tttcgaattg tgagaatgtt gaaaaactat cggtggcatg tggaggagcg 3060

gcccagatga cgtcactgaa tatttgggga tgtaagaagc tcaagtgtct tccagaactc 3120

cttccatctc tcaaggaact gcgtctgtct gattgtccag aaatagaagg agaattgccc 3180

ttcaatttag aaatactccg tatcatatat tgcaagaaac tggtgaatgg ccgaaaggag 3240

tggcatttac agagactcac agagttatgg atcgatcatg atgggagtga cgaagatatt 3300

gaacattggg agttgccttg ttctattcag agacttacca taaagaatct taaaacatta 3360

agcagccaac atctcaaaag cctcacctct cttcaatatc tatgtattga gggttattta 3420

tctcagattc agtcacaagg ccagctttcc tccttttctc acctcacttc gcttcaaact 3480

ctacaaatct ggaatttcct taatctccaa tcacttgctg aatcagcact gccctcctcc 3540

ctctctcacc tggagataga tgattgccct aatctccaat cactcttcga atcagcactg 3600

ccctcctccc tctctcagct gttcatccag gattgcccta atctccaatc ccttccattt 3660

aaagggatgc cctcttccct ctctaaacta tctattttca attgcccatt gctcacacca 3720

ctactagaat ttgacaaggg ggaatactgg ccacaaattg ctcatattcc catcataaat 3780

atcgattgga aatatattta a 3801

<210> 137
<211> 1266
<212> PRT
<213> Solanum lycopersicum

<400> 137
Met Glu Ile Gly Leu Ala Val Gly Gly Ala Phe Leu Ser Ser Ala Leu
1               5                   10                  15
Asn Val Leu Phe Asp Arg Leu Ala Pro Asn Gly Asp Leu Leu Asn Met
            20                  25                  30
Phe Arg Lys His Lys Asp His Val Lys Leu Leu Lys Lys Leu Lys Met
        35                  40                  45
Thr Leu Arg Gly Ile Gln Ile Val Leu Ser Asp Ala Glu Asn Lys Gln
    50                  55                  60
Ala Ser Asn Pro Ser Val Arg Asp Trp Leu Asn Glu Leu Arg Asp Ala
65                  70                  75                  80
Val Asp Ser Ala Glu Asn Leu Ile Glu Glu Val Asn Tyr Glu Ala Leu
                85                  90                  95
Arg Leu Lys Val Glu Gly Gln His Gln Asn Phe Ser Glu Thr Ser Asn
            100                 105                 110
Gln Gln Val Ser Asp Asp Phe Phe Leu Asn Ile Lys Asp Lys Leu Glu
        115                 120                 125
Asp Thr Ile Glu Thr Leu Lys Asp Leu Gln Glu Gln Ile Gly Leu Leu
    130                 135                 140
Gly Leu Lys Glu Tyr Phe Asp Ser Thr Lys Leu Glu Thr Arg Arg Pro
145                 150                 155                 160
Ser Thr Ser Val Asp Asp Glu Ser Asp Ile Phe Gly Arg Gln Ser Glu
                165                 170                 175
Ile Glu Asp Leu Ile Asp Arg Leu Leu Ser Glu Gly Ala Ser Gly Lys

            180                 185                 190
Lys Leu Thr Val Val Pro Ile Val Gly Met Gly Gly Gln Gly Lys Thr
        195                 200                 205
Thr Leu Ala Lys Ala Val Tyr Asn Asp Glu Arg Val Lys Asn His Phe
    210                 215                 220
Asp Leu Lys Ala Trp Tyr Cys Val Ser Glu Gly Phe Asp Ala Leu Arg
225                 230                 235                 240
Ile Thr Lys Glu Leu Leu Gln Glu Ile Gly Lys Phe Asp Ser Lys Asp
                245                 250                 255
Val His Asn Asn Leu Asn Gln Leu Gln Val Lys Leu Lys Glu Ser Leu
            260                 265                 270
Lys Gly Lys Lys Phe Leu Ile Val Leu Asp Asp Val Trp Asn Glu Asn
        275                 280                 285
Tyr Asn Glu Trp Asn Asp Leu Arg Asn Ile Phe Ala Gln Gly Asp Ile
    290                 295                 300
Gly Ser Lys Ile Ile Val Thr Thr Arg Lys Asp Ser Val Ala Leu Met
305                 310                 315                 320
Met Gly Asn Glu Gln Ile Arg Met Gly Asn Leu Ser Thr Glu Ala Ser
                325                 330                 335
Trp Ser Leu Phe Gln Arg His Ala Phe Glu Asn Met Asp Pro Met Gly
            340                 345                 350
His Pro Glu Leu Glu Glu Val Gly Arg Gln Ile Ala Ala Lys Cys Lys
        355                 360                 365
Gly Leu Pro Leu Ala Leu Lys Thr Leu Ala Gly Met Leu Arg Ser Lys
    370                 375                 380
Ser Glu Val Glu Glu Trp Lys Arg Ile Leu Arg Ser Glu Ile Trp Glu
385                 390                 395                 400
Leu Pro His Asn Asp Ile Leu Pro Ala Leu Met Leu Ser Tyr Asn Asp
                405                 410                 415
Leu Pro Ala His Leu Lys Arg Cys Phe Ser Phe Cys Ala Ile Phe Pro
            420                 425                 430
Lys Asp Tyr Pro Phe Arg Lys Glu Gln Val Ile His Leu Trp Ile Ala
        435                 440                 445
Asn Gly Leu Val Pro Val Lys Asp Glu Ile Asn Gln Asp Leu Gly Asn
    450                 455                 460
Gln Tyr Phe Leu Glu Leu Arg Ser Arg Ser Leu Phe Glu Lys Val Pro
465                 470                 475                 480
Asn Pro Ser Lys Arg Asn Ile Glu Glu Leu Phe Leu Met His Asp Leu
                485                 490                 495
Val Asn Asp Leu Ala Gln Leu Ala Ser Ser Lys Leu Cys Ile Arg Leu
            500                 505                 510
Glu Glu Ser Gln Gly Ser His Met Leu Glu Gln Cys Arg His Leu Ser
        515                 520                 525
Tyr Ser Ile Gly Phe Asn Gly Glu Phe Lys Lys Leu Thr Pro Leu Tyr
    530                 535                 540
Lys Leu Glu Gln Leu Arg Thr Leu Leu Pro Ile Arg Ile Glu Phe Arg
545                 550                 555                 560
Leu His Asn Leu Ser Lys Arg Val Leu His Asn Ile Leu Pro Thr Leu
                565                 570                 575
Arg Ser Leu Arg Ala Leu Ser Phe Ser Gln Tyr Lys Ile Lys Glu Leu
            580                 585                 590
Pro Asn Asp Leu Phe Thr Lys Leu Lys Leu Leu Arg Phe Leu Asp Ile
        595                 600                 605
Ser Arg Thr Trp Ile Thr Lys Leu Pro Asp Ser Ile Cys Gly Leu Tyr
    610                 615                 620
Asn Leu Glu Thr Leu Leu Leu Ser Ser Cys Ala Asp Leu Glu Glu Leu
625                 630                 635                 640
Pro Leu Gln Met Glu Lys Leu Ile Asn Leu Arg His Leu Asp Val Ser
                645                 650                 655
Asn Thr Arg Arg Leu Lys Met Pro Leu His Leu Ser Arg Leu Lys Ser
            660                 665                 670
Leu Gln Val Leu Val Gly Pro Lys Phe Phe Val Asp Gly Trp Arg Met
        675                 680                 685

Glu Asp Leu Gly Glu Ala Gln Asn Leu His Gly Ser Leu Ser Val Val
    690                 695                 700
Lys Leu Glu Asn Val Val Asp Arg Arg Glu Ala Val Lys Ala Lys Met
705                 710                 715                 720
Arg Glu Lys Asn His Val Glu Gln Leu Ser Leu Glu Trp Ser Glu Ser
                725                 730                 735
Ser Ile Ala Asp Asn Ser Gln Thr Glu Ser Asp Ile Leu Asp Glu Leu
            740                 745                 750
Cys Pro His Lys Asn Ile Lys Lys Val Glu Ile Ser Gly Tyr Arg Gly
        755                 760                 765
Thr Asn Phe Pro Asn Trp Val Ala Asp Pro Leu Phe Leu Lys Leu Val
    770                 775                 780
Asn Leu Ser Leu Arg Asn Cys Lys Asp Cys Tyr Ser Leu Pro Ala Leu
785                 790                 795                 800
Gly Gln Leu Pro Cys Leu Lys Phe Leu Ser Val Lys Gly Met His Gly
                805                 810                 815
Ile Arg Val Val Thr Glu Glu Phe Tyr Gly Arg Leu Ser Ser Lys Lys
            820                 825                 830
Pro Phe Asn Ser Leu Glu Lys Leu Glu Phe Glu Asp Met Thr Glu Trp
        835                 840                 845
Lys Gln Trp His Ala Leu Gly Ile Gly Glu Phe Pro Thr Leu Glu Asn
    850                 855                 860
Leu Ser Ile Lys Asn Cys Pro Glu Leu Ser Leu Glu Ile Pro Ile Gln
865                 870                 875                 880
Phe Ser Ser Leu Lys Arg Leu Glu Val Ser Asp Cys Pro Val Val Phe
                885                 890                 895
Asp Asp Ala Gln Leu Phe Arg Ser Gln Leu Glu Ala Met Lys Gln Ile
            900                 905                 910
Glu Glu Ile Asp Ile Cys Asp Cys Asn Ser Val Thr Ser Phe Pro Phe
        915                 920                 925
Ser Ile Leu Pro Thr Thr Leu Lys Arg Ile Gln Ile Ser Arg Cys Pro
    930                 935                 940
Lys Leu Lys Leu Glu Ala Pro Val Gly Glu Met Phe Val Glu Tyr Leu
945                 950                 955                 960
Arg Val Asn Asp Cys Gly Cys Val Asp Asp Ile Ser Pro Glu Phe Leu
                965                 970                 975
Pro Thr Ala Arg Gln Leu Ser Ile Glu Asn Cys Gln Asn Val Thr Arg
            980                 985                 990
Phe Leu Ile Pro Thr Ala Thr Glu Thr Leu Arg Ile Ser Asn Cys Glu
        995                 1000                1005
Asn Val Glu Lys Leu Ser Val Ala Cys Gly Gly Ala Ala Gln Met Thr
    1010                1015                1020
Ser Leu Asn Ile Trp Gly Cys Lys Lys Leu Lys Cys Leu Pro Glu Leu
1025                1030                1035                1040
Leu Pro Ser Leu Lys Glu Leu Arg Leu Ser Asp Cys Pro Glu Ile Glu
                1045                1050                1055
Gly Glu Leu Pro Phe Asn Leu Glu Ile Leu Arg Ile Ile Tyr Cys Lys
            1060                1065                1070
Lys Leu Val Asn Gly Arg Lys Glu Trp His Leu Gln Arg Leu Thr Glu
        1075                1080                1085
Leu Trp Ile Asp His Asp Gly Ser Asp Glu Asp Ile Glu His Trp Glu
    1090                1095                1100
Leu Pro Cys Ser Ile Gln Arg Leu Thr Ile Lys Asn Leu Lys Thr Leu
1105                1110                1115                1120
Ser Ser Gln His Leu Lys Ser Leu Thr Ser Leu Gln Tyr Leu Cys Ile
                1125                1130                1135
Glu Gly Tyr Leu Ser Gln Ile Gln Ser Gln Gly Gln Leu Ser Ser Phe
            1140                1145                1150
Ser His Leu Thr Ser Leu Gln Thr Leu Gln Ile Trp Asn Phe Leu Asn
        1155                1160                1165
Leu Gln Ser Leu Ala Glu Ser Ala Leu Pro Ser Ser Leu Ser His Leu
    1170                1175                1180
Glu Ile Asp Asp Cys Pro Asn Leu Gln Ser Leu Phe Glu Ser Ala Leu

1185                1190                1195                1200
Pro Ser Ser Leu Ser Gln Leu Phe Ile Gln Asp Cys Pro Asn Leu Gln
                1205                1210                1215
Ser Leu Pro Phe Lys Gly Met Pro Ser Ser Leu Ser Lys Leu Ser Ile
            1220                1225                1230
Phe Asn Cys Pro Leu Leu Thr Pro Leu Leu Glu Phe Asp Lys Gly Glu
        1235                1240                1245
Tyr Trp Pro Gln Ile Ala His Ile Pro Ile Ile Asn Ile Asp Trp Lys
    1250                1255                1260
Tyr Ile
1265

<210> 138
<211> 492
<212> DNA
<213> Artificial Sequence

<220>
<223> cDNA

<400> 138
atgcgtttcc ttctgcttat agccatgtcc atgacatggg tttgctctat tgctgggcta      60

cctgtggaag atgccgattc atctgtcggt cagctccaag gacggggtaa cccatattgc     120

gtgtttcccg gccgccgcac gtcttctact tcatttacta cgagcttcag caccgaacct     180

ctgggttatg ctagaatgtt acacagagac cctccatatg agcgggctgg caattcggga     240

ctgaatcacc gcatttacga acgaagtcgc gttggaggcc tccgcactgt cattgatgtc     300

gcccccccgg acggacacca agccatcgcc aactacgaga tcgaagtccg tcgtattcct     360

gttgcaacac ctaatgcggc tggagattgc tttcacactg cacgccttag taccggctct     420

cgaggtccag ccacaatcag ttgggatgcc gacgctagtt atacctacta tcttactatc     480

tcagaggatt ga                                                         492

<210> 139
<211> 163
<212> PRT
<213> Fusarium oxysporum

<400> 139
Met Arg Phe Leu Leu Leu Ile Ala Met Ser Met Thr Trp Val Cys Ser
1               5                   10                  15
Ile Ala Gly Leu Pro Val Glu Asp Ala Asp Ser Ser Val Gly Gln Leu
            20                  25                  30
Gln Gly Arg Gly Asn Pro Tyr Cys Val Phe Pro Gly Arg Arg Thr Ser
        35                  40                  45
Ser Thr Ser Phe Thr Thr Ser Phe Ser Thr Glu Pro Leu Gly Tyr Ala
    50                  55                  60
Arg Met Leu His Arg Asp Pro Pro Tyr Glu Arg Ala Gly Asn Ser Gly
65                  70                  75                  80
Leu Asn His Arg Ile Tyr Glu Arg Ser Arg Val Gly Gly Leu Arg Thr
                85                  90                  95
Val Ile Asp Val Ala Pro Pro Asp Gly His Gln Ala Ile Ala Asn Tyr
            100                 105                 110
Glu Ile Glu Val Arg Arg Ile Pro Val Ala Thr Pro Asn Ala Ala Gly

115                 120                 125
Asp Cys Phe His Thr Ala Arg Leu Ser Thr Gly Ser Arg Gly Pro Ala
    130                 135                 140
Thr Ile Ser Trp Asp Ala Asp Ala Ser Tyr Thr Tyr Tyr Leu Thr Ile
145                 150                 155                 160
Ser Glu Asp

<210> 140
<211> 357
<212> DNA
<213> Artificial Sequence

<220>
<223> cDNA

<400> 140
atgcgcttcg agtacatctc tgttctagcc ctttgcggcg cgagtctcgc aagggatcat      60

cagtactgtg cttgccagtc cggttctggt gacagcattg atatagatgc caccactcag     120

cttcagaacg ataatagtaa atcatatttg tgggctcaga cctctccagc atattggttt     180

gctgaccgtc ataaacctgg tccacgattc gctggcattt atctgaaagc tgcaaatggc     240

aaaattgatg gcgatacatt ctataatctt tgcattaata atggaggagc agactcaaca     300

tgctttgact gctctaaatc tcaccaggtt agaaacgtta tctattgtga tgcggcc       357

<210> 141
<211> 119
<212> PRT
<213> Fusarium oxysporum

<400> 141
Met Arg Phe Glu Tyr Ile Ser Val Leu Ala Leu Cys Gly Ala Ser Leu
1               5                   10                  15
Ala Arg Asp His Gln Tyr Cys Ala Cys Gln Ser Gly Ser Gly Asp Ser
            20                  25                  30
Ile Asp Ile Asp Ala Thr Thr Gln Leu Gln Asn Asp Asn Ser Lys Ser
        35                  40                  45
Tyr Leu Trp Ala Gln Thr Ser Pro Ala Tyr Trp Phe Ala Asp Arg His
    50                  55                  60
Lys Pro Gly Pro Arg Phe Ala Gly Ile Tyr Leu Lys Ala Ala Asn Gly
65                  70                  75                  80
Lys Ile Asp Gly Asp Thr Phe Tyr Asn Leu Cys Ile Asn Asn Gly Gly
                85                  90                  95
Ala Asp Ser Thr Cys Phe Asp Cys Ser Lys Ser His Gln Val Arg Asn
            100                 105                 110
Val Ile Tyr Cys Asp Ala Ala
        115

<210> 142
<211> 523
<212> DNA
<213> Fusarium oxysporum

<400> 142
atgcgcttcg agtacatctc tgttctagcc ctttgcggcg cgaggtaatc ttagcccttt      60

gttatcctac acgcctgtgt agttcgtgtg tttgctaaca tggtcttggt ctcgcaaggg 120

atcatcagta ctgtgcttgc cagtccggtt ctggtgacag cattgatata gatgccacca 180

ctcagcttca gaacgataat agtaaatcat atttgtgggg tatgtatacc tcatagtgat 240

tgggcaattg ggcaagctta caattctata gctcagacct ctccagcata ttggtttgct 300

gaccgtcata aacctggtcc acgattcgct ggcatttatg taagtctaca gcttaggatg 360

attaagtgcc tgaactaacc ttgactagct gaaagctgca aatggcaaaa ttgatggcga 420

tacattctat aatctttgca ttaataatgg aggagcagac tcaacatgct ttgactgctc 480

taaatctcac caggttagaa acgttatcta ttgtgatgcg gcc 523

<210> 143
<211> 2787
<212> DNA
<213> Artificial Sequence

<220>
<223> cDNA

<400> 143
atggcgccgg cggtcattgc atcgcagggt gtcatcatgc ggtccctgac gagcaagctc 60

gactcgctgc tgctgcagcc gccggagccg ccgccgcctg cgcaaccgtc gtcgctgcgg 120

aagggggaga ggaagaagat cctcctcctc agaggcgatc tccgacacct gctagatgac 180

tactacctcc tcgtggagcc gccgtcagac accgcgccac cgccagactc gacggcggcg 240

tgctgggcta aggaggttcg cgagctctcc tacgacgtcg acgacttcct cgacgagcta 300

acgacccagc tcctccacca ccgcggcggc ggcgatggca gtagcactgc tggtgccaag 360

aagatgatca gcagcatgat cgcgcggctt cgaggggagc ttaaccggcg gcggtggatc 420

gccgacgagg tcaccctgtt cagggcccgc gtgaaggagg ccattcgccg ccacgagagc 480

taccatcttg gcaggcgcac ctcgagctcg aggccgagag aagaagacga cgacgacgat 540

cgcgaggact ccgccggcaa cgaacgccgc cggtttctgt cgctgacgtt cgggatggac 600

gacgctgctg tgcacggcca gctcgttggt agggatattt cgatgcaaaa gctcgtccgg 660

tggctggccg acggcgagcc gaagctcaag gtggcttcca ttgttggatc cggaggtgtt 720

ggcaagacga cgctggccac agaattctat cgtctgcatg gccggcggtt ggatgcgccg 780

ttcgactgcc gggctttcgt gcggacgccc cggaagcctg acatgacgaa gatcctcacc 840

gacatgctgt cacagctgcg gccacaacat cagcatcagt cttcggatgt ttgggaggtt 900

gatcgactcc ttgaaactat ccggacgcat ttgcaagata aaaggtactt catcataatt 960

gaagatttat gggcttcatc aatgtgggat attgttagcc gtggtttgcc tgataataat 1020

agttgcagta gaatactaat aacaacagaa attgaacctg tagctttggc atgctgtgga 1080

tataactcag agcacattat taagattgat ccactgggtg atgatgtctc aagtcaattg 1140

tttttcagtg gagttgttgg ccaaggaaat gaatttcctg gacatcttac tgaagtttct 1200

catgacatga taaaaaaatg tggtggcttg ccactagcaa taactataac agccagacat 1260

tttaaaagcc agctgttaga tggaatgcag caatggaatc acatacaaaa atcattgact 1320

acttccaatt tgaagaaaaa tcctactttg caggggatga ggcaagtact caaccttatt 1380

tacaataatc ttcctcattg tttgaaagca tgtctgttat accttagcat ctacaaagag 1440

gactacataa ttaggaaggc caacttggtg aggcaatgga tggctgaagg tttcatcaat 1500

tccatagaaa ataaagtcat ggaagaagtt gcagggaact attttgatga acttgttggt 1560

aggggcctgg tccaaccagt agatgttaac tgcaaaaatg aggtattgtc atgtgtagtg 1620

caccacatgg tattaaattt catcaggtgt aagtcaatag aggagaattt cagcattaca 1680

ttggatcatt ctcagacgac agtaagacat gctgacaagg ttcgccgact ctcgcttcac 1740

ttcagcaatg cacatgatac aacaccacta gcaggtttga gactctcaca agttcgatcg 1800

atggcatttt tcggacaagt caagtgtatg ccttccattg cagattatag gcttcttcga 1860

gttctgattc tttgtttttg ggctgatcaa gagaaaacaa gctatgacct cacaagcatt 1920

tttgaactgt tacaactgag atatctgaag ataacaggta atatcacagt taaacttcca 1980

gagaagatcc aaggactaca acacttgcag acactggaag cagatgcaag agcaactgct 2040

gtcctattgg atattgttca tacacagtgt ttgttgcacc ttcgtcttgt actacttgat 2100

ctgctccctc actgtcacag gtacatcttc accagcatcc ccaaatggac tggaaagctc 2160

aacaatctcc gcattttaaa cattgcagtc atgcaaattt cccaggatga ccttgacact 2220

ctcaaaggac tgggatctct cactgctctt tcgctgcttg ttcgaacagc gcctgcgcaa 2280

agaatcgtcg ctgcgaatga ggggttcggg tctctcaagt acttcatgtt tgtctgtaca 2340

gcaccatgca tgacttttgt ggaaggagca atgccgagtg tgcaaaggtt aaatctaagg 2400

ttcaatgcca acgagttcaa gcagtatgac tctaaggaga cagggttgga acacttggtc 2460

gcccttgcag agatctctgc aagaattggg ggcactgatg atgatgaatc aaacaaaact 2520

gaagtggagt ctgccttgag gactgcaatt cgcaagcatc cgacgccgag cactcttatg 2580

gttgatatac aatgggtgga ttggatcttt ggtgctgaag ggagagactt ggatgaagat 2640

ttggcacaac aagatgatca cgggtatgga tttttcattc tattcccagg ttacaactta 2700

caaggattat tgagcttctt tctttctctg ccgtggcttc tatctttacc tgctatgcat 2760

cttcaacctg acttgatgat tgtttga 2787

<210> 144
<211> 928
<212> PRT

<213> Oryza sativa

<400> 144
Met Ala Pro Ala Val Ile Ala Ser Gln Gly Val Ile Met Arg Ser Leu
1               5                   10                  15
Thr Ser Lys Leu Asp Ser Leu Leu Leu Gln Pro Pro Glu Pro Pro Pro
            20                  25                  30
Pro Ala Gln Pro Ser Ser Leu Arg Lys Gly Glu Arg Lys Lys Ile Leu
        35                  40                  45
Leu Leu Arg Gly Asp Leu Arg His Leu Leu Asp Asp Tyr Tyr Leu Leu
    50                  55                  60
Val Glu Pro Pro Ser Asp Thr Ala Pro Pro Pro Asp Ser Thr Ala Ala
65                  70                  75                  80
Cys Trp Ala Lys Glu Val Arg Glu Leu Ser Tyr Asp Val Asp Asp Phe
                85                  90                  95
Leu Asp Glu Leu Thr Thr Gln Leu Leu His His Arg Gly Gly Gly Asp
            100                 105                 110
Gly Ser Ser Thr Ala Gly Ala Lys Lys Met Ile Ser Ser Met Ile Ala
        115                 120                 125
Arg Leu Arg Gly Glu Leu Asn Arg Arg Arg Trp Ile Ala Asp Glu Val
    130                 135                 140
Thr Leu Phe Arg Ala Arg Val Lys Glu Ala Ile Arg Arg His Glu Ser
145                 150                 155                 160
Tyr His Leu Gly Arg Arg Thr Ser Ser Ser Arg Pro Arg Glu Glu Asp
                165                 170                 175
Asp Asp Asp Asp Arg Glu Asp Ser Ala Gly Asn Glu Arg Arg Arg Phe
            180                 185                 190
Leu Ser Leu Thr Phe Gly Met Asp Asp Ala Ala Val His Gly Gln Leu
        195                 200                 205
Val Gly Arg Asp Ile Ser Met Gln Lys Leu Val Arg Trp Leu Ala Asp
    210                 215                 220
Gly Glu Pro Lys Leu Lys Val Ala Ser Ile Val Gly Ser Gly Gly Val
225                 230                 235                 240
Gly Lys Thr Thr Leu Ala Thr Glu Phe Tyr Arg Leu His Gly Arg Arg
                245                 250                 255
Leu Asp Ala Pro Phe Asp Cys Arg Ala Phe Val Arg Thr Pro Arg Lys
            260                 265                 270
Pro Asp Met Thr Lys Ile Leu Thr Asp Met Leu Ser Gln Leu Arg Pro
        275                 280                 285
Gln His Gln His Gln Ser Ser Asp Val Trp Glu Val Asp Arg Leu Leu
    290                 295                 300
Glu Thr Ile Arg Thr His Leu Gln Asp Lys Arg Tyr Phe Ile Ile Ile
305                 310                 315                 320
Glu Asp Leu Trp Ala Ser Ser Met Trp Asp Ile Val Ser Arg Gly Leu
                325                 330                 335
Pro Asp Asn Asn Ser Cys Ser Arg Ile Leu Ile Thr Thr Glu Ile Glu
            340                 345                 350
Pro Val Ala Leu Ala Cys Cys Gly Tyr Asn Ser Glu His Ile Ile Lys
        355                 360                 365
Ile Asp Pro Leu Gly Asp Asp Val Ser Ser Gln Leu Phe Phe Ser Gly
    370                 375                 380
Val Val Gly Gln Gly Asn Glu Phe Pro Gly His Leu Thr Glu Val Ser
385                 390                 395                 400
His Asp Met Ile Lys Lys Cys Gly Gly Leu Pro Leu Ala Ile Thr Ile
                405                 410                 415
Thr Ala Arg His Phe Lys Ser Gln Leu Leu Asp Gly Met Gln Gln Trp
            420                 425                 430
Asn His Ile Gln Lys Ser Leu Thr Thr Ser Asn Leu Lys Lys Asn Pro
        435                 440                 445
Thr Leu Gln Gly Met Arg Gln Val Leu Asn Leu Ile Tyr Asn Asn Leu
    450                 455                 460
Pro His Cys Leu Lys Ala Cys Leu Leu Tyr Leu Ser Ile Tyr Lys Glu

465                 470                 475                 480
Asp Tyr Ile Ile Arg Lys Ala Asn Leu Val Arg Gln Trp Met Ala Glu
                485                 490                 495
Gly Phe Ile Asn Ser Ile Glu Asn Lys Val Met Glu Glu Val Ala Gly
            500                 505                 510
Asn Tyr Phe Asp Glu Leu Val Gly Arg Gly Leu Val Gln Pro Val Asp
        515                 520                 525
Val Asn Cys Lys Asn Glu Val Leu Ser Cys Val Val His His Met Val
    530                 535                 540
Leu Asn Phe Ile Arg Cys Lys Ser Ile Glu Glu Asn Phe Ser Ile Thr
545                 550                 555                 560
Leu Asp His Ser Gln Thr Thr Val Arg His Ala Asp Lys Val Arg Arg
                565                 570                 575
Leu Ser Leu His Phe Ser Asn Ala His Asp Thr Thr Pro Leu Ala Gly
            580                 585                 590
Leu Arg Leu Ser Gln Val Arg Ser Met Ala Phe Phe Gly Gln Val Lys
        595                 600                 605
Cys Met Pro Ser Ile Ala Asp Tyr Arg Leu Leu Arg Val Leu Ile Leu
    610                 615                 620
Cys Phe Trp Ala Asp Gln Glu Lys Thr Ser Tyr Asp Leu Thr Ser Ile
625                 630                 635                 640
Phe Glu Leu Leu Gln Leu Arg Tyr Leu Lys Ile Thr Gly Asn Ile Thr
                645                 650                 655
Val Lys Leu Pro Glu Lys Ile Gln Gly Leu Gln His Leu Gln Thr Leu
            660                 665                 670
Glu Ala Asp Ala Arg Ala Thr Ala Val Leu Leu Asp Ile Val His Thr
        675                 680                 685
Gln Cys Leu Leu His Leu Arg Leu Val Leu Leu Asp Leu Leu Pro His
    690                 695                 700
Cys His Arg Tyr Ile Phe Thr Ser Ile Pro Lys Trp Thr Gly Lys Leu
705                 710                 715                 720
Asn Asn Leu Arg Ile Leu Asn Ile Ala Val Met Gln Ile Ser Gln Asp
                725                 730                 735
Asp Leu Asp Thr Leu Lys Gly Leu Gly Ser Leu Thr Ala Leu Ser Leu
            740                 745                 750
Leu Val Arg Thr Ala Pro Ala Gln Arg Ile Val Ala Ala Asn Glu Gly
        755                 760                 765
Phe Gly Ser Leu Lys Tyr Phe Met Phe Val Cys Thr Ala Pro Cys Met
    770                 775                 780
Thr Phe Val Glu Gly Ala Met Pro Ser Val Gln Arg Leu Asn Leu Arg
785                 790                 795                 800
Phe Asn Ala Asn Glu Phe Lys Gln Tyr Asp Ser Lys Glu Thr Gly Leu
                805                 810                 815
Glu His Leu Val Ala Leu Ala Glu Ile Ser Ala Arg Ile Gly Gly Thr
            820                 825                 830
Asp Asp Asp Glu Ser Asn Lys Thr Glu Val Glu Ser Ala Leu Arg Thr
        835                 840                 845
Ala Ile Arg Lys His Pro Thr Pro Ser Thr Leu Met Val Asp Ile Gln
    850                 855                 860
Trp Val Asp Trp Ile Phe Gly Ala Glu Gly Arg Asp Leu Asp Glu Asp
865                 870                 875                 880
Leu Ala Gln Gln Asp Asp His Gly Tyr Gly Phe Phe Ile Leu Phe Pro
                885                 890                 895
Gly Tyr Asn Leu Gln Gly Leu Leu Ser Phe Phe Leu Ser Leu Pro Trp
            900                 905                 910
Leu Leu Ser Leu Pro Ala Met His Leu Gln Pro Asp Leu Met Ile Val
        915                 920                 925

<210> 145
<211> 4250
<212> DNA

<213> Oryza sativa

<400> 145
atggcgccgg cggtcattgc atcgcagggt gtcatcatgc ggtccctgac gagcaagctc 60
gactcgctgc tgctgcagcc gccggagccg ccgccgcctg cgcaaccgtc gtcgctgcgg 120
aagggggaga ggaagaagat cctcctcctc agaggcgatc tccgacacct gctagatgac 180
tactacctcc tcgtggagcc gccgtcagac accgcgccac cgccagactc gacggcggcg 240
tgctgggcta aggaggttcg cgagctctcc tacgacgtcg acgacttcct cgacgagcta 300
acgacccagc tcctccacca ccgcggcggc ggcgatggca gtagcactgc tggtgccaag 360
aagatgatca gcagcatgat cgcgcggctt cgaggggagc ttaaccggcg gcggtggatc 420
gccgacgagg tcaccctgtt cagggcccgc gtgaaggagg ccattcgccg ccacgagagc 480
taccatcttg gcaggcgcac ctcgagctcg aggccgagag aagaagacga cgacgacgat 540
cgcgaggact ccgccggcaa cgaacgccgc cggtttctgt cgctgacgtt cgggatggac 600
gacgctgctg tgcacggcca gctcgttggt agggatattt cgatgcaaaa gctcgtccgg 660
tggctggccg acggcgagcc gaagctcaag gtggcttcca ttgttggatc cggaggtgtt 720
ggcaagacga cgctggccac agaattctat cgtctgcatg gccggcggtt ggatgcgccg 780
ttcgactgcc gggctttcgt gcggacgccc cggaagcctg acatgacgaa gatcctcacc 840
gacatgctgt cacagctgcg gccacaacat cagcatcagt cttcggatgt ttgggaggtt 900
gatcgactcc ttgaaactat ccggacgcat ttgcaagata aaaggtaatt catgtctaca 960
tctatctcta gtattttttt catgaattta caaactattt tctcaaattt cccctttttt 1020
atccttcata tagtaattaa gttagtaact atacatatga atttaattta ctcgacaatg 1080
ccaataatat tttaaattac ttttatagtg tcctctatta ttaaatacaa ttcgatcgag 1140
ccatgaatca tactggtaat ctaaacaatt attataccca cctcattcta ctaacagcaa 1200
ttgagttgtt aatataaata gatatggtct attgcatgaa aaaataaagg taaagtgttt 1260
acattatttc tactactagt agcagaacta caaagttgtt tgtattttta attattaaat 1320
gtaaatgaaa gtacatgttt catacccttg ttattttttt aggcgactaa actactagta 1380
cattatttct actactagta gaacaactac aaagttgttt gtatttttaa ttactaaatg 1440
taaatgaaag tacatgcttc atacccttgt tataatattt tagactaaac taccacaatg 1500
gcatgttaac ttatagcagt aagttgtaat ttattttctt ttcattttct cagttgttac 1560
aagaactttt ttttacttaa taaaaaatag tcgtaagggc ctcccttgtt cggtcaaaaa 1620
agaattagac taaactcaat cgtatttgta cataaacaaa atttaaaata taactaaaac 1680
gaaacagtga taaaaatggt gcaatttaac tgctgcctct tgttttaatg tctgacaatg 1740
ttgattttgt atatatgttt ggccatttat tttattcaaa ttttttacaa atatgaaaaa 1800

tataatatgt gcttaaatta cttttaatga taaaataaat ttaagaaaat gataattata 1860

tttttaataa gatggatgat ctaacatata tatgtccaaa agtttgacat caaacattaa 1920

aaacaagaga gagtatttgc ttaggagtac gtgtcttttt ccatgcctta gaacaggtac 1980

aatagcaggt tataagctag gccaaacata ttttaaagag atataggaag agagagaaga 2040

gcagcagcct acagatctgt agccagctgc agcacggact ctaagacgta atgtgtgtat 2100

gacaggtagg accaagtatt aatagtatag taagcaacta ttgtatgaat tggctatttg 2160

gctctagatg atttggagct agtagtcggc tatactatta aacttgctct tagatatgtg 2220

gttaaattta atttctgaaa tttacaagat atagaacatg tcttcaccat gtttgcaaag 2280

ttgataaaat gaaattgcac tcaacttttt aaattcagca tgctatccca cgtatagctt 2340

tttaaatatg atttattttt tcgtacatta gaagtttaat gtttacgaag tactaaatgt 2400

tctgcaggta cttcatcata attgaagatt tatgggcttc atcaatgtgg gatattgtta 2460

gccgtggttt gcctgataat aatagttgca gtagaatact aataacaaca gaaattgaac 2520

ctgtagcttt ggcatgctgt ggatataact cagagcacat tattaagatt gatccactgg 2580

gtgatgatgt ctcaagtcaa ttgtttttca gtggagttgt tggccaagga aatgaatttc 2640

ctggacatct tactgaagtt tctcatgaca tgataaaaaa atgtggtggc ttgccactag 2700

caataactat aacagccaga cattttaaaa gccagctgtt agatggaatg cagcaatgga 2760

atcacataca aaaatcattg actacttcca atttgaagaa aaatcctact ttgcagggga 2820

tgaggcaagt actcaacctt atttacaata atcttcctca ttgtttgaaa gcatgtctgt 2880

tataccttag catctacaaa gaggactaca taattaggaa ggccaacttg gtgaggcaat 2940

ggatggctga aggtttcatc aattccatag aaaataaagt catggaagaa gttgcaggga 3000

actattttga tgaacttgtt ggtaggggcc tggtccaacc agtagatgtt aactgcaaaa 3060

atgaggtatt gtcatgtgta gtgcaccaca tggtattaaa tttcatcagg tgtaagtcaa 3120

tagaggagaa tttcagcatt acattggatc attctcagac gacagtaaga catgctgaca 3180

aggttcgccg actctcgctt cacttcagca atgcacatga tacaacacca ctagcaggtt 3240

tgagactctc acaagttcga tcgatggcat ttttcggaca agtcaagtgt atgccttcca 3300

ttgcagatta taggcttctt cgagttctga ttctttgttt ttgggctgat caagagaaaa 3360

caagctatga cctcacaagc atttttgaac tgttacaact gagatatctg aagataacag 3420

gtaatatcac agttaaactt ccagagaaga tccaaggact acaacacttg cagacactgg 3480

aagcagatgc aagagcaact gctgtcctat tggatattgt tcatacacag tgtttgttgc 3540

accttcgtct tgtactactt gatctgctcc ctcactgtca caggtacatc ttcaccagca 3600

tccccaaatg gactggaaag ctcaacaatc tccgcatttt aaacattgca gtcatgcaaa 3660

tttcccagga tgaccttgac actctcaaag gactgggatc tctcactgct ctttcgctgc 3720

ttgttcgaac agcgcctgcg caaagaatcg tcgctgcgaa tgaggggttc gggtctctca 3780

agtacttcat gtttgtctgt acagcaccat gcatgacttt tgtggaagga gcaatgccga 3840

gtgtgcaaag gttaaatcta aggttcaatg ccaacgagtt caagcagtat gactctaagg 3900

agacagggtt ggaacacttg gtcgcccttg cagagatctc tgcaagaatt gggggcactg 3960

atgatgatga atcaaacaaa actgaagtgg agtctgcctt gaggactgca attcgcaagc 4020

atccgacgcc gagcactctt atggttgata tacaatgggt ggattggatc tttggtgctg 4080

aagggagaga cttggatgaa gatttggcac aacaagatga tcacgggtat ggatttttca 4140

ttctattccc aggttacaac ttacaaggat tattgagctt ctttctttct ctgccgtggc 4200

ttctatcttt acctgctatg catcttcaac ctgacttgat gattgtttga 4250

<210> 146
<211> 672
<212> DNA
<213> Artificial Sequence

<220>
<223> cDNA

<400> 146
atgctttttt attcattatt tttttttcac accgttgcga tttcggcctt caccaacatt 60

ggcacctttt cacacccagt ttacgattac aatccaattc caaaccatat ccacggagat 120

ttaaaaaggc gggcttatat tgaacgctat tcccaatgtt cagattcgca ggcctccgaa 180

attcgtgccg cgctaaaaag ttgtgccgag ctcgcctcgt ggggctatca cgccgttaaa 240

aatgacaatc ggttatttag attaatcttt aaaactgaca gcacagatat tcaaaactgg 300

gttcaaaaga attttaacga aatttacaag gaatgtaaca gggacgcgga cgaaatttct 360

ctaacctgcc acgataaaaa tgtttatacg tgcgtccgag aaggagttca taatttggcg 420

tatgcactta ttaacgaaaa agaaattgtt atatgccctc ctttcttcaa caaccccgta 480

aacagcaggg aaattactgc cggtaaccaa gatacagtta tattacatga aatggtgcat 540

ataattttaa aagagtggaa agattatggt tacgaatggg atgggattca caaattggat 600

agtacagaaa gtattaaaaa ccccgacagt tatgctattt ttgcacaatg tgcacgttat 660

aaatattgtt aa 672

<210> 147
<211> 222
<212> PRT
<213> Magnaporthe grisea

<400> 147

Met Leu Phe Tyr Ser Leu Phe Phe Phe His Thr Val Ala Ile Ser Ala
1               5                   10                  15
Phe Thr Asn Ile Gly Thr Phe Ser His Pro Val Tyr Asp Tyr Asn Pro
            20                  25                  30
Ile Pro Asn His Ile His Gly Asp Leu Lys Arg Arg Ala Tyr Ile Glu
        35                  40                  45
Arg Tyr Ser Gln Cys Ser Asp Ser Gln Ala Ser Glu Ile Arg Ala Ala
    50                  55                  60
Leu Lys Ser Cys Ala Glu Leu Ala Ser Trp Gly Tyr His Ala Val Lys
65                  70                  75                  80
Asn Asp Asn Arg Leu Phe Arg Leu Ile Phe Lys Thr Asp Ser Thr Asp
                85                  90                  95
Ile Gln Asn Trp Val Gln Lys Asn Phe Asn Glu Ile Tyr Lys Glu Cys
            100                 105                 110
Asn Arg Asp Ala Asp Glu Ile Ser Leu Thr Cys His Asp Lys Asn Val
        115                 120                 125
Tyr Thr Cys Val Arg Glu Gly Val His Asn Leu Ala Tyr Ala Leu Ile
    130                 135                 140
Asn Glu Lys Glu Ile Val Ile Cys Pro Pro Phe Phe Asn Asn Pro Val
145                 150                 155                 160
Asn Ser Arg Glu Ile Thr Ala Gly Asn Gln Asp Thr Val Ile Leu His
                165                 170                 175
Glu Met Val His Ile Ile Leu Lys Glu Trp Lys Asp Tyr Gly Tyr Glu
            180                 185                 190
Trp Asp Gly Ile His Lys Leu Asp Ser Thr Glu Ser Ile Lys Asn Pro
        195                 200                 205
Asp Ser Tyr Ala Ile Phe Ala Gln Cys Ala Arg Tyr Lys Tyr
    210                 215                 220

<210> 148
<211> 882
<212> DNA
<213> Magnaporthe grisea

<400> 148
atgctttttt attcattatt tttttttcac accgttgcga tttcggcctt caccaacatt      60

ggcacctttt cacacccagt ttacgattac aatccaattc caaaccatat ccacggagat     120

ttaaaaaggc gggcttatat tgaacgctat tcccaatgtt cagattcgca ggcctccgaa     180

attcgtgccg cgctaaaaag gtaaattgaa actctttaaa acaaattcgg aaaacaatgt     240

taaatatttt gtttagttgt gccgagctcg cctcgtgggg ctatcacgcc gttaaaaatg     300

acaatcggtt atttagatta atctttaaaa ctgacagcac agatattcaa aactgggttc     360

aaaagaattt taacgaaatt tacaaggaat gtaacaggga cgcggacgaa atttctctaa     420

cctgccacga taaaaatgtt tatacgtgcg tccgagaagg agttcataat ttggcgtatg     480

cacttattaa cgaaaaagaa attgttatat gccctccttt cttcaacaac cccgtaaaca     540

gcagggaaat tactgccggt aaccaagata cagttatatt acatgaaatg gtgcatataa     600

ttttaagtaa gtttgctttt acaaattgat aaaacattta caaaagttta tttataaaaa     660

ttttcaaaaa ctaaaaattc aaatttttat ttagaagagt ggaaagatta tggttacgaa     720

tgggatggga ttcacaagta agttgtcgaa aaacaaattt gctgaatgtt gttttatgtt     780

gataaattct aattaatatt aagattggat agtacagaaa gtattaaaaa ccccgacagt     840

tatgctattt ttgcacaatg tgcacgttat aaatattgtt aa 882

<210> 149
<211> 3102
<212> DNA
<213> Artificial Sequence

<220>
<223> cDNA

<400> 149
atggcggaga cggtgctgag catggcgagg tcgctggtgg gcagcgccat cagcaaggcc 60

gcctccgctg ctgccgacga gaccagcctc ctgctgggcg tcgagaaaga catctggtat 120

atcaaagatg agctaaaaac gatgcaagca ttccttagag ctgctgaact tatgaaaaag 180

aaagatgaac tattaaaggt ttgggcagag caaatacgtg acctgtcata tgacattgaa 240

gattcccttg atgaatttaa ggtccatatt gaaagccaaa ccctatttcg tcagttggtg 300

aaactcagag aacgccaccg aattgctatc cgtatccaca accttaaatc aagagttgaa 360

gaagtgagta gcaggaacac acgctacagt ttagtcaagc ctatttcctc tggcacagag 420

attgacatgg attcctatgc agaagacatt cgtaatcagt cagctcgcaa tgtggatgaa 480

gctgagcttg ttgggttttc tgactccaag aaaaggctgc ttgaaatgat cgataccaat 540

gctaatgatg gtccggccaa ggtaatctgt gttgttggga tgggtggttt aggcaagaca 600

gctctttcga ggaagatctt tgaaagcgaa gaagacatta ggaagaactt cccttgcaat 660

gcttggatta cagtgtcaca atcatttcac aggattgagc tacttaaaga tatgatacgc 720

caacttcttg gccccagttc tctggatcaa ctcttgcaag aattgcaagg gaaggtggtg 780

gtgcaagtac atcatctttc tgagtacctg atagaagagc tcaaggagaa gaggtacttt 840

gttgttctag atgatctatg gattttacat gattggaatt ggataaatga aattgcattt 900

cctaagaaca ataagaaggg cagtcgaata gtaataacca ctcggaatgt tgatcttgcg 960

gagaagtgtg ccacagcctc actggtgtac caccttgatt tcttgcagat gaacgatgcc 1020

ataacattgc tactgagaaa aacaaataaa aatcatgaag acatggaatc aaataaaaat 1080

atgcaaaaga tggttgaacg aattgtaaat aaatgtggtc gtctaccatt agcaatactt 1140

acaataggag ctgtgcttgc aactaaacag gtgtcagaat gggagaaatt ctatgaacac 1200

cttccttcag aactagaaat aaacccaagc ctggaagctt tgaggagaat ggtgacccta 1260

ggttacaacc acctaccatc ccatttgaaa ccatgctttt tgtatctaag tatctttcct 1320

gaggattttg aaatcaaaag gaatcgtcta gtaggtagat ggatagcaga agggtttgtt 1380

agaccaaagg ttgggatgac gactaaggat gtcggagaaa gttactttaa tgagctaatc 1440

aaccgaagta tgattcaacg atcaagagtg ggcatagcag gaaaaattaa gacttgtcga 1500

attcatgata tcatccgtga tatcacagtt tcaatctcga gacaggaaaa ttttgtatta 1560

ttaccaatgg gagatggctc tgatttagtt caggaaaaca ctcgccacat agcattccat 1620

gggagtatgt cctgcaaaac tggattggat tggagcatta ttcgatcatt agctattttt 1680

ggtgacagac ccaagagtct agcacatgca gtttgtccag atcaattgag gatgttacgg 1740

gtcttggatc ttgaagatgt gacattctta atcactcaaa aagatttcga ccgtattgca 1800

ttgttgtgcc acttgaaata cttgagtatt ggatattcgt catccatata ttcacttccc 1860

agatccattg gtaaactaca gggcctacaa actttgaaca tgccgagcac atacattgca 1920

gcactaccaa gtgagatcag taaactccaa tgtctgcata ctcttcgttg tataggacag 1980

tttcattatg acaactttag tctaaaccac ccaatgaagt gcataactaa cacaatatgc 2040

ctgcctaaag tattcacacc tttagttagt cgcgatgatc gtgcaaaaca aattgctgaa 2100

ttgcacatgg ccaccaaaag ttgctggtct gaatcaatcg gtgtgaaggt acccaaagga 2160

ataggtaagt tgcgagactt gcaggttcta gagtatgtag atatcaggcg gaccagtagt 2220

agagcaatca aagagctggg gcagttaagc aagctgagga aattaggtgt gacaacaaac 2280

gggtcgacaa aggaaaaatg taagatactt tatgcagcca ttgagaagct ctcttccctc 2340

caatctctcc atgtggatgc tgtgttattc tcaggtatta ttggaacact tgagtgccta 2400

gattctattt catctcctcc tcccctactg aggacactcg tgttggatgg aattcttgag 2460

gagatgccta actggattga gcagctcatg cacctgaaga agatctactt attgagcagc 2520

aaactaaagg aaggtaaaac catgctgata cttggggcac tgcccaacct catggtcctt 2580

catctttatc ggaatgctta ccttggggag aagctagtat tcaaaacagg agcattccca 2640

aatcttagaa cactttggat ttatgaattg gatcagctaa gagagatcag atttgaggac 2700

ggcagctcac ccctgttgga aaagatagaa ataggcgagt gcaggttgga atctgggatt 2760

actggtatca ttcaccttcc aaagctcaag gagattccaa ttagatacgg aagtaaagtg 2820

gctgggcttg gtcagctgga gggagaagtg aacgcacacc caaatcgccc cgtgctgcta 2880

atgtacagtg accgaaggta tcacgacctg ggggctgaag ccgaaggatc ttctatagaa 2940

gtgcaaacag cagatcctgt tcctgatgcc gaaggatcag tcactgtagc agtggaagca 3000

acggatcccc ttcccgagca ggagggagag agctcgcagt cgcaggtgat cacgttgacg 3060

acgaatgata gcgaagagat aggcacagct caagctggct ga 3102

<210> 150
<211> 1033
<212> PRT
<213> Oryza sativa

<400> 150

Met Ala Glu Thr Val Leu Ser Met Ala Arg Ser Leu Val Gly Ser Ala
1               5                   10                  15
Ile Ser Lys Ala Ala Ser Ala Ala Ala Asp Glu Thr Ser Leu Leu Leu
            20                  25                  30
Gly Val Glu Lys Asp Ile Trp Tyr Ile Lys Asp Glu Leu Lys Thr Met
        35                  40                  45
Gln Ala Phe Leu Arg Ala Ala Glu Leu Met Lys Lys Lys Asp Glu Leu
    50                  55                  60
Leu Lys Val Trp Ala Glu Gln Ile Arg Asp Leu Ser Tyr Asp Ile Glu
65                  70                  75                  80
Asp Ser Leu Asp Glu Phe Lys Val His Ile Glu Ser Gln Thr Leu Phe
                85                  90                  95
Arg Gln Leu Val Lys Leu Arg Glu Arg His Arg Ile Ala Ile Arg Ile
            100                 105                 110
His Asn Leu Lys Ser Arg Val Glu Glu Val Ser Ser Arg Asn Thr Arg
        115                 120                 125
Tyr Ser Leu Val Lys Pro Ile Ser Ser Gly Thr Glu Ile Asp Met Asp
    130                 135                 140
Ser Tyr Ala Glu Asp Ile Arg Asn Gln Ser Ala Arg Asn Val Asp Glu
145                 150                 155                 160
Ala Glu Leu Val Gly Phe Ser Asp Ser Lys Lys Arg Leu Leu Glu Met
                165                 170                 175
Ile Asp Thr Asn Ala Asn Asp Gly Pro Ala Lys Val Ile Cys Val Val
            180                 185                 190
Gly Met Gly Gly Leu Gly Lys Thr Ala Leu Ser Arg Lys Ile Phe Glu
        195                 200                 205
Ser Glu Glu Asp Ile Arg Lys Asn Phe Pro Cys Asn Ala Trp Ile Thr
    210                 215                 220
Val Ser Gln Ser Phe His Arg Ile Glu Leu Leu Lys Asp Met Ile Arg
225                 230                 235                 240
Gln Leu Leu Gly Pro Ser Ser Leu Asp Gln Leu Leu Gln Glu Leu Gln
                245                 250                 255
Gly Lys Val Val Val Gln Val His His Leu Ser Glu Tyr Leu Ile Glu
            260                 265                 270
Glu Leu Lys Glu Lys Arg Tyr Phe Val Val Leu Asp Asp Leu Trp Ile
        275                 280                 285
Leu His Asp Trp Asn Trp Ile Asn Glu Ile Ala Phe Pro Lys Asn Asn
    290                 295                 300
Lys Lys Gly Ser Arg Ile Val Ile Thr Thr Arg Asn Val Asp Leu Ala
305                 310                 315                 320
Glu Lys Cys Ala Thr Ala Ser Leu Val Tyr His Leu Asp Phe Leu Gln
                325                 330                 335
Met Asn Asp Ala Ile Thr Leu Leu Leu Arg Lys Thr Asn Lys Asn His
            340                 345                 350
Glu Asp Met Glu Ser Asn Lys Asn Met Gln Lys Met Val Glu Arg Ile
        355                 360                 365
Val Asn Lys Cys Gly Arg Leu Pro Leu Ala Ile Leu Thr Ile Gly Ala
    370                 375                 380
Val Leu Ala Thr Lys Gln Val Ser Glu Trp Glu Lys Phe Tyr Glu His
385                 390                 395                 400
Leu Pro Ser Glu Leu Glu Ile Asn Pro Ser Leu Glu Ala Leu Arg Arg
                405                 410                 415
Met Val Thr Leu Gly Tyr Asn His Leu Pro Ser His Leu Lys Pro Cys
            420                 425                 430
Phe Leu Tyr Leu Ser Ile Phe Pro Glu Asp Phe Glu Ile Lys Arg Asn
        435                 440                 445
Arg Leu Val Gly Arg Trp Ile Ala Glu Gly Phe Val Arg Pro Lys Val
    450                 455                 460
Gly Met Thr Thr Lys Asp Val Gly Glu Ser Tyr Phe Asn Glu Leu Ile
465                 470                 475                 480
Asn Arg Ser Met Ile Gln Arg Ser Arg Val Gly Ile Ala Gly Lys Ile
                485                 490                 495
Lys Thr Cys Arg Ile His Asp Ile Ile Arg Asp Ile Thr Val Ser Ile

500                 505                 510
Ser Arg Gln Glu Asn Phe Val Leu Leu Pro Met Gly Asp Gly Ser Asp
        515                 520                 525
Leu Val Gln Glu Asn Thr Arg His Ile Ala Phe His Gly Ser Met Ser
    530                 535                 540
Cys Lys Thr Gly Leu Asp Trp Ser Ile Ile Arg Ser Leu Ala Ile Phe
545                 550                 555                 560
Gly Asp Arg Pro Lys Ser Leu Ala His Ala Val Cys Pro Asp Gln Leu
                565                 570                 575
Arg Met Leu Arg Val Leu Asp Leu Glu Asp Val Thr Phe Leu Ile Thr
            580                 585                 590
Gln Lys Asp Phe Asp Arg Ile Ala Leu Leu Cys His Leu Lys Tyr Leu
        595                 600                 605
Ser Ile Gly Tyr Ser Ser Ser Ile Tyr Ser Leu Pro Arg Ser Ile Gly
    610                 615                 620
Lys Leu Gln Gly Leu Gln Thr Leu Asn Met Pro Ser Thr Tyr Ile Ala
625                 630                 635                 640
Ala Leu Pro Ser Glu Ile Ser Lys Leu Gln Cys Leu His Thr Leu Arg
                645                 650                 655
Cys Ile Gly Gln Phe His Tyr Asp Asn Phe Ser Leu Asn His Pro Met
            660                 665                 670
Lys Cys Ile Thr Asn Thr Ile Cys Leu Pro Lys Val Phe Thr Pro Leu
        675                 680                 685
Val Ser Arg Asp Asp Arg Ala Lys Gln Ile Ala Glu Leu His Met Ala
    690                 695                 700
Thr Lys Ser Cys Trp Ser Glu Ser Ile Gly Val Lys Val Pro Lys Gly
705                 710                 715                 720
Ile Gly Lys Leu Arg Asp Leu Gln Val Leu Glu Tyr Val Asp Ile Arg
                725                 730                 735
Arg Thr Ser Ser Arg Ala Ile Lys Glu Leu Gly Gln Leu Ser Lys Leu
            740                 745                 750
Arg Lys Leu Gly Val Thr Thr Asn Gly Ser Thr Lys Glu Lys Cys Lys
        755                 760                 765
Ile Leu Tyr Ala Ala Ile Glu Lys Leu Ser Ser Leu Gln Ser Leu His
    770                 775                 780
Val Asp Ala Val Leu Phe Ser Gly Ile Ile Gly Thr Leu Glu Cys Leu
785                 790                 795                 800
Asp Ser Ile Ser Ser Pro Pro Pro Leu Leu Arg Thr Leu Val Leu Asp
                805                 810                 815
Gly Ile Leu Glu Glu Met Pro Asn Trp Ile Glu Gln Leu Met His Leu
            820                 825                 830
Lys Lys Ile Tyr Leu Leu Ser Ser Lys Leu Lys Glu Gly Lys Thr Met
        835                 840                 845
Leu Ile Leu Gly Ala Leu Pro Asn Leu Met Val Leu His Leu Tyr Arg
    850                 855                 860
Asn Ala Tyr Leu Gly Glu Lys Leu Val Phe Lys Thr Gly Ala Phe Pro
865                 870                 875                 880
Asn Leu Arg Thr Leu Trp Ile Tyr Glu Leu Asp Gln Leu Arg Glu Ile
                885                 890                 895
Arg Phe Glu Asp Gly Ser Ser Pro Leu Leu Glu Lys Ile Glu Ile Gly
            900                 905                 910
Glu Cys Arg Leu Glu Ser Gly Ile Thr Gly Ile Ile His Leu Pro Lys
        915                 920                 925
Leu Lys Glu Ile Pro Ile Arg Tyr Gly Ser Lys Val Ala Gly Leu Gly
    930                 935                 940
Gln Leu Glu Gly Glu Val Asn Ala His Pro Asn Arg Pro Val Leu Leu
945                 950                 955                 960
Met Tyr Ser Asp Arg Arg Tyr His Asp Leu Gly Ala Glu Ala Glu Gly
                965                 970                 975
Ser Ser Ile Glu Val Gln Thr Ala Asp Pro Val Pro Asp Ala Glu Gly
            980                 985                 990
Ser Val Thr Val Ala Val Glu Ala Thr Asp Pro Leu Pro Glu Gln Glu
        995                 1000                1005

Gly Glu Ser Ser Gln Ser Gln Val Ile Thr Leu Thr Thr Asn Asp Ser
    1010                1015                1020
Glu Glu Ile Gly Thr Ala Gln Ala Gly
1025                1030

<210> 151
<211> 7067
<212> DNA
<213> Oryza sativa

<400> 151
atggcggaga cggtgctgag catggcgagg tcgctggtgg gcagcgccat cagcaaggcc      60

gcctccgctg ctgccgacga gaccagcctc ctgctgggcg tcgagaaaga catctggtac     120

gtactgcgtg actctcgtta atttattctg tagatgctca ggaatcagca actattgtgt     180

tgatttccat cgtagcatat cgattttgtt ggccaccaat tctaatcggc cggaacaagc     240

tagtcactaa atctggcaaa tcgatcagct gctgagtgca caaacatgca tgttattctt     300

tttttttttg ggttatatgt taagcaacaa agccccttgg taagatatgc atggcaaatg     360

aactaatatc gacatacgta aagcggagga cccctcgttc catgcgtggg tgactcgagc     420

ggtgacaaat cctagcacct ccacctcctt ggatggcctg tggtgacgct ttcggccccg     480

agtttccctt gaatacatca tctacaaggt gctattaatg tctagtcaca tcatttacga     540

ggtgctatta ttgcctagtc tgcccgaaga tagtttagac aacactcttg gatggcggtg     600

tccttcgccc agtgatgtcc aagagcccgt ggatgtttag ttgtttagac atggtgttgg     660

gtggtgcact agtgggcctg atgggccagt tgtaggtcca gtggtaacca atcatgctta     720

gcaatagccg gatgcccgga ttggtgcttg ttcttttttc ggtgtcgacg catggtagta     780

tttacttttc ctgtttttcc tgattatagc atcctaggct atactcttct aatttattca     840

tgctatatta atattaaaac ttggtatggt ttgtttcatt caagaccctt ggtggtcaaa     900

ggcttgtttg gttcaagttc attcctagcc ttaccaactt tttggcaata gcaagaaatg     960

gtcattgaaa aaaaaaggca aaaattggct aggcctacag tttatttcct agcaaagtta    1020

tactttagca ttccactaag ccaaataatt cggcaatgcc attttcttat ctacatgcca    1080

aatatatggc taatattttg gcattaatta ctcttatttt ttttggcaaa attgatcaaa    1140

agttcacatt tttagctcta tagtattaaa agttatctat tcactttaat agaccgaaag    1200

tttactcggt tccgttttta gcactaccgt ctcttttctc ttgatttgcc gtcaattttg    1260

accggcagtc ctacccccag gagacattga gcagcagccc gtgatccccc tctctcgccg    1320

ccggtgacgc tgtggtggca tcgttcctgc tgcgggcaga ataagtctgg cgtcatcgcc    1380

ctatcgcctg gagctgcaac caccactacc gggcccatcg atcgtctaga gcgttatcca    1440

ccctgcctgc cccattactt gcagctccgg ctgggtcaga acctctccat gcctgataaa    1500

ttggttcaag attgtcgctg tccggccagc gcttgaattt tcagaatatg ccatcgaata    1560

cgcgctgctt ttaagatatg ctacccgatt catgctattt ttagaatacg ccatcagaac 1620

acgaattttc ttcgttccgt gccactccgt ctctcggagt cagtcgtgcc gtcgtcatcc 1680

gtccgcccag cactgtcgtc gtcagtccgc cacccgtgcc tgactgtccg ttcagctgcg 1740

ccgtcgtccg tccgtcgccg ccatcgccgt cgtccactgt cgcgcccgca cctgcacccg 1800

tgtcaggcgc gccgtcgtcc gtccgctgtc accatcatcg tcgtccactg cggcgagcgc 1860

agacggctgt ggacggatga ttgcgcggca agcgcaagcg gcggtggacg gacgacgggg 1920

cagtgcacgc gagcacggta gccgatggac tgacgttggc ggcattggga gatggacgac 1980

gacggcatga ttgacgtggg gaacggaatg tcacggaacg gagaaaattc gcactccggt 2040

ggcatatatt ccgaaaatag cacgaattgg gtggcatatc ttaaaaacag tgcctatttg 2100

gtggcatatt ctaaaaattc tcggtcagca taatccccat caatccccaa tccctcaaca 2160

gttgggttaa tattcctgga gatgtgttcg gttgtttagg ttgaagttct ccacttcacc 2220

tccatgagta catgcacctc tacacgtacg ttcttaatgt gtttgtttgt tctatcctcc 2280

gcttgggttc tattttgttg gttccgatct gatttgatct ggagcggggt cgatcttcca 2340

cgacggcgag agagacgttg ttcgggctgc tcgattaggt tcaactgttt aggtcgaagg 2400

gaggggtaga attgcaattc aagtgcatgg tcagtcaatt tgggtcaaaa ttaacatcaa 2460

actgggataa agagacgaca gtgccaaaat tggtaacggg aaaactttga gttctattaa 2520

agtgaaccag taactttcgt tgctatagaa taaaaacgta aacttttaat gctccttggt 2580

tgagcttggt acaaaccaaa cagacgtaaa ataaacacta tcctgaatca agtctactaa 2640

gttccattga actcaaccag gatacgtaca cttcctctta gaagatgtct tgttttcact 2700

ttgtacaatt ttttctattg taaatttggt acctcgttgt acctaggtac aagaggtacc 2760

atgagatacc aaattttaca ctaaaatttt ggtacctcat ggtacctcct caacgaccgt 2820

agaattgctc ttaatttaat ttaaaaaaaa cataatattt ttaaagcata ttatggaaat 2880

tttagtaatt attacttttg taatatatga gttacggtta tactcgagat atcctaaatt 2940

gcttggagat gaataattac aaggtatatc aaagatgagt tgaaaataat gcaggcattc 3000

cttagagctg cagaagttat gaaaaagaaa gacgaactat taaaggtttg ggcagagcaa 3060

atacgtgacc tgtcatatca cattgaagat tcccttgatg aatttaaggt ccatgttgaa 3120

agtcaaaccc tatttcgtca gttggtgaaa cttagatagc gtcaccggat cgctagcccg 3180

tggatgttta gttgtttgca catggtgctg gatggtgcgc tcatggtctt gttgtaggtc 3240

tggtaccaac cagtcatgct tagaaatagc cggatcagtg cacggtgcta ggactttact 3300

tggtggtctg tgcagcgcta tcgacatgtg gtggtgtgct tttttttttt ccggattaca 3360

atctcatagg gctacactct agttattttg ctgctatatt aatatgaaaa cttggtatgg 3420

ttcgtttctt ttagaaaaaa acctagttga tcaagggcta gttttcttca agtgcattcc 3480

taatcttagc ttcttttttt tttgcaatgg caagaattgt tcattaaaaa aattgataaa 3540

aattggctag gcctacgttt tgtttcttac caaagttgta ctttaacaat aaactaaggc 3600

aaatatttcg gcaatgccat tttcttgtct acagaccaaa tatatggcta aattttggca 3660

taaccatttt tttgtttgct tggttgagct tggtacaaac caaacagacc caaaataaac 3720

agtgtcatga atcacgtcta ctaaattcct ttgaactgaa ctagaatata gttgctctta 3780

aaagatttct tgatttcact cggtaccatt tactagtaca aacttagatt taatttttaa 3840

aaataaaatc ataatattgt tattatggaa aatttagtca tagtactttt gtaatatatg 3900

agatgggtta tacttgagat atcctaaatt gctttaagat gaataattgc taggtatatc 3960

aaagatgagc taaaaacgat gcaagcattc cttagagctg ctgaacttat gaaaaagaaa 4020

gatgaactat taaaggtttg ggcagagcaa atacgtgacc tgtcatatga cattgaagat 4080

tcccttgatg aatttaaggt ccatattgaa agccaaaccc tatttcgtca gttggtgaaa 4140

ctcagagaac gccaccgaat tgctatccgt atccacaacc ttaaatcaag agttgaagaa 4200

gtgagtagca ggaacacacg ctacagttta gtcaagccta tttcctctgg cacagagatt 4260

gacatggatt cctatgcaga agacattcgt aatcagtcag ctcgcaatgt ggatgaagct 4320

gagcttgttg ggttttctga ctccaagaaa aggctgcttg aaatgatcga taccaatgct 4380

aatgatggtc cggccaaggt aatctgtgtt gttgggatgg gtggtttagg caagacagct 4440

ctttcgagga agatctttga aagcgaagaa gacattagga agaacttccc ttgcaatgct 4500

tggattacag tgtcacaatc atttcacagg attgagctac ttaaagatat gatacgccaa 4560

cttcttggcc ccagttctct ggatcaactc ttgcaagaat tgcaaggaaa ggtggtggtg 4620

caagtacatc atctttctga gtacctgata gaagagctca aggagaagag gtactttgtt 4680

gttctagatg atctatggat tttacatgat tggaattgga taaatgaaat tgcatttcct 4740

aagaacaata agaagggcag tcgaatagta ataaccactc ggaatgttga tcttgcggag 4800

aagtgtgcca cagcctcact ggtgtaccac cttgatttct tgcagatgaa cgatgccata 4860

acattgctac tgagaaaaac aaataaaaat catgaagaca tggaatcaaa taaaaatatg 4920

caaaagatgg ttgaacgaat tgtaaataaa tgtggtcgtc taccattagc aatacttaca 4980

ataggagctg tgcttgcaac taaacaggtg tcagaatggg agaaattcta tgaacacctt 5040

ccttcagaac tagaaataaa cccaagcctg gaagctttga ggagaatggt gaccctaggt 5100

tacaaccacc taccatccca tttgaaacca tgctttttgt atctaagtat ctttcctgag 5160

gattttgaaa tcaaaaggaa tcgtctagta ggtagatgga tagcagaagg gtttgttaga 5220

ccaaaggttg ggatgacgac taaggatgtc ggagaaagtt actttaatga gctaatcaac 5280

cgaagtatga ttcaacgatc aagagtgggc atagcaggaa aaattaagac ttgtcgaatt 5340

catgatatca tccgtgatat cacagtttca atctcgagac aggaaaattt tgtattatta 5400

ccaatgggag atggctctga tttagttcag gaaaacactc gccacatagc attccatggg 5460

agtatgtcct gcaaaactgg attggattgg agcattattc gatcattagc tatttttggt 5520

gacagaccca agagtctagc acatgcagtt tgtccagatc aattgaggat gttacgggtc 5580

ttggatcttg aagatgtgac attcttaatc actcaaaaag atttcgaccg tattgcattg 5640

ttgtgccact tgaaatactt gagtattgga tattcgtcat ccatatattc acttcccaga 5700

tccattggta aactacaggg cctacaaact ttgaacatgc cgagcacata cattgcagca 5760

ctaccaagtg agatcagtaa actccaatgt ctgcatactc ttcgttgtat aggacagttt 5820

cattatgaca actttagtct aaaccaccca atgaagtgca taactaacac aatatgcctg 5880

cctaaagtat tcacaccttt agttagtcgc gatgatcgtg caaaacaaat tgctgaattg 5940

cacatggcca ccaaaagttg ctggtctgaa tcaatcggtg tgaaggtacc caaaggaata 6000

ggtaagttgc gagacttgca ggttctagag tatgtagata tcaggcggac cagtagtaga 6060

gcaatcaaag agctggggca gttaagcaag ctgaggaaat taggtgtgac aacaaacggg 6120

tcgacaaagg aaaaatgtaa gatactttat gcagccattg agaagctctc ttccctccaa 6180

tctctccatg tggatgctgt gttattctca ggtattattg gaacacttga gtgcctagat 6240

tctatttcat ctcctcctcc cctactgagg acactcgtgt tggatggaat tcttgaggag 6300

atgcctaact ggattgagca gctcatgcac ctgaagaaga tctacttatt gagcagcaaa 6360

ctaaaggaag gtaaaaccat gctgatactt ggggcactgc ccaacctcat ggtccttcat 6420

ctttatcgga atgcttacct tggggagaag ctagtattca aaacaggagc attcccaaat 6480

cttagaacac tttggattta tgaattggat cagctaagag agatcagatt tgaggacggc 6540

agctcacccc tgttggaaaa gatagaaata ggcgagtgca ggttggaatc tgggattact 6600

ggtatcattc accttccaaa gctcaaggag attccaatta gatacggaag taaagtggct 6660

gggcttggtc agctggaggg agaagtgaac gcacacccaa atcgccccgt gctgctaatg 6720

tacagtgacc gaaggtatca cgacctgggg gctgaagccg aaggatcttc tatagaagtg 6780

caaacagcag atcctgttcc tgatgccgaa ggatcagtca ctgtagcagt ggaagcaacg 6840

gatccccttc ccgagcagga gggagagagc tcgcagtcgc aggtgatcac gttgacgacg 6900

aatgataggt cagtcactcc ctacatggca gcttaattaa cttgtttcta attctcttct 6960

tgttcagtat tagccatcag gtgagggcga tgatttcaac tcacttttca tctctctcgt 7020

tttcttaacc tgacagcgaa gagataggca cagctcaagc tggctga 7067

<210> 152
<211> 327
<212> DNA

<213> Artificial Sequence

<220>
<223> cDNA

<400> 152
atgcagttct caaccatcat caccgtgtgc ctcttcaccg ggctcgcctc cgccagcttc 60

gtacaatgca atcatcatct cctgtacaat ggcagacact ggggcacgat aaggaagaag 120

gcgggttggg ccgttagatt ttacgaagaa aaaccagggc agccaaagag gctggtcgcg 180

atttgcaaaa acgcgtcacc cgtacactgc aactatctga aatgcaccaa tttggcagca 240

ggcttctcgg cagggacgtc cactgatgtt ctctcctccg gcaccgttgg ctcgattggg 300

aatgaccctc aggctcagcg ccaatag 327

<210> 153
<211> 108
<212> PRT
<213> Magnaporthe grisea

<400> 153
Met Gln Phe Ser Thr Ile Ile Thr Val Cys Leu Phe Thr Gly Leu Ala
1               5                   10                  15
Ser Ala Ser Phe Val Gln Cys Asn His His Leu Leu Tyr Asn Gly Arg
            20                  25                  30
His Trp Gly Thr Ile Arg Lys Lys Ala Gly Trp Ala Val Arg Phe Tyr
        35                  40                  45
Glu Glu Lys Pro Gly Gln Pro Lys Arg Leu Val Ala Ile Cys Lys Asn
    50                  55                  60
Ala Ser Pro Val His Cys Asn Tyr Leu Lys Cys Thr Asn Leu Ala Ala
65                  70                  75                  80
Gly Phe Ser Ala Gly Thr Ser Thr Asp Val Leu Ser Ser Gly Thr Val
                85                  90                  95
Gly Ser Ile Gly Asn Asp Pro Gln Ala Gln Arg Gln
            100                 105

<210> 154
<211> 2991
<212> DNA
<213> Artificial Sequence

<220>
<223> cDNA

<400> 154
atggaggccg cgcttttgag cggattcatc aaggccatcc tgccgaggct cttctccctc 60

gtcgacgata agcataagct ccacaagggc gtcaagggcg acatcgattt cctcatcaag 120

gagctccgca tgatcgtcgg cgccatcgac gacgacctct ccctcgacca ccccgccgcc 180

gccgccgtac agacgctatg catggaggac ctacgcgagc tcgcccacgg catcgaggac 240

tgcatcgacg gcgtcctgta ccgcgccgcc agggaccagc agcagtcacc ggtccgccgc 300

gccgtccagg cccccaagaa gctccagaga aacctgcagc ttgcccagca gctgcagcgg 360

ctgaagagga tggcggcgga ggcgaaccag cggaagcaga ggtacacggc ggcggcgccc 420

ggtcaacacg gtcaggtcta ctcatcggct gcggcgcagg tggatgagcc atggccgtcg 480

tgctcatctg cctcagatcc gcgcatccac gaggcggacc tggtcggcgt cgacgcggac 540

cgggaggagc tcctggagca gctggcggag cggcagccgg agcagctcaa ggtgatcgcc 600

atcgtcgggt tctgcgggct ggggaagacc gccctcgccg cggaggcgta caaccgagag 660

accggcggcg ggagattcga gaggcacgcg tgggtttgcg ccgggcaccg gagtgcacgg 720

gaggtgctcg gcgaattgct ccgcaggctt gacgccgacg gccgttcttt ccatggggac 780

tccgatgctg gtcagctgtg tgtagatatc agacagcagt tggagaagaa caggtacttc 840

attgttatcg atgacattca aacagaagat cagtggaaaa gcatcaaatc ggcttttccg 900

actgataagg atattggcag cagaatagtg gtcacgacga ccatccagtc agtagctaat 960

gcctgctgtt ctgcgaatgg gtatttgcac aaaatgagca ggcttgataa gaactgctca 1020

aagcaattgt tgtccaagaa agcttgccca gagaggtatt cacattacaa gcagccagat 1080

tcagcagcaa ttttgaagaa atgtgatggc cagccacttg ctcttgttac cataggtgaa 1140

ttcctgcaag ctaatggctg gccaacgggg cctaactgtg aagatctctg caaccggcta 1200

cattatcacc tagagaatga taaaacactt gaaaggatgt ggcgagtgct ggtccgcaac 1260

tacaccagtc tacctggcca tgctctcaag gcctgcttac tatattttgg catgttccca 1320

tctgatcatc caatcaggag gaaaagtctg ctgaggcgat ggttggccga gggatttgta 1380

gaaccactgt cttcatcgag taacatagat tccacggctg ctttcaatgt gctaatggac 1440

cggaacatca ttgagcccat caatgtaagc aataatgata aagtcaagac atgccaaacg 1500

tacggtatga tgcgtgaatt catatcgcat atgtcaatct ctcagaactt tgtcaccttt 1560

ttctgtgatg acaagttcgt gcccaaatat gttcgtcggc tttctctcca tggtgacact 1620

gttgtgaatg gtgataactt caacggtatt gatttatcac ttgtacggtc tctggcagtc 1680

tttggggagg caggtacaac cgtattagac ttcagcaagt atcaattgct gcgagtttta 1740

gatcttgaaa aatgtgatga tttgaaggat gatcatctca aagaaatatg caatctggtg 1800

cttctgaaat atctgagcct tgggggtaat atctccaaac ttccaaagga tattgccaaa 1860

ttgaaagatt tggaggcact cgatgtaagg agatcgaaag taaagataat gccggtagaa 1920

gtcttcgggt tgccatgcct aattcatctg cttggaaaat ttaagctctc agataaagtc 1980

aagcagaaga ctgaagtgca agagtttctc ttgaaaggaa aaagcaactt gcagacgcta 2040

gcaggatttg ctagcaacgg aagcgaaggg tttctgcatc ttatgaggta catgaataag 2100

ttgagaaagt tgaagatttg gtgtacgtcg tctgcaggta gcaccgattg gactgatctt 2160

agggaggcca ttcaacagtt cattctggat gaaaaggaag caaatattgg tacccgttct 2220

ttatcgcttc atttcagtgg atgctctgaa gatgcgataa attccttaaa agaaccctgc 2280

tacctcagct cgttgaaatt acatggcaac ttcccccaat tgcctcagtt tgttacatca 2340

cttcgtggtc tcaaggagct ttgcctttca tctactaaat ttacaacagg ccttcttgaa 2400

gccttgagta atttgagcta cttgcagtat ctcaaactgg ttgccgatga acttgagaag 2460

ttcatcataa aagttcaggg gttccccagg ctgctacgcc tttgtattgt gctacaatat 2520

ccaacattcc cagtaattga agaaggagct ctgccatttc ttgtcacact tcagctgcta 2580

tgcaaagatc tacatggcct ttctgacatc caaattgaat gttttaaaca tcttcaggag 2640

gtaactcttc attctggagt cactccagct acaagacagg aatgggtaaa ggctgctaag 2700

gagcatccga ataggccaaa agtattgctt ctcaaatctg ttgatacagc agaaagtgaa 2760

catacagacg ttgatagtgt aatggaggca gtcaaaagtg agactacaga atattctatt 2820

gcaccagagg gaccagaaca agtgaacaac aaaatgcaac ttgaccatgg attggaatcc 2880

tcttctgtgc tgaacaaaca aaacaacttt gcagaccaat caagctcaaa agatcaactg 2940

cattattctt tcaacaatat ggggctttca gatgtctcct gctgtgagtg a 2991

<210> 155
<211> 996
<212> PRT
<213> Oryza sativa

<400> 155
Met Glu Ala Ala Leu Leu Ser Gly Phe Ile Lys Ala Ile Leu Pro Arg
1               5                   10                  15
Leu Phe Ser Leu Val Asp Asp Lys His Lys Leu His Lys Gly Val Lys
            20                  25                  30
Gly Asp Ile Asp Phe Leu Ile Lys Glu Leu Arg Met Ile Val Gly Ala
        35                  40                  45
Ile Asp Asp Asp Leu Ser Leu Asp His Pro Ala Ala Ala Ala Val Gln
    50                  55                  60
Thr Leu Cys Met Glu Asp Leu Arg Glu Leu Ala His Gly Ile Glu Asp
65                  70                  75                  80
Cys Ile Asp Gly Val Leu Tyr Arg Ala Ala Arg Asp Gln Gln Gln Ser
                85                  90                  95
Pro Val Arg Arg Ala Val Gln Ala Pro Lys Lys Leu Gln Arg Asn Leu
            100                 105                 110
Gln Leu Ala Gln Gln Leu Gln Arg Leu Lys Arg Met Ala Ala Glu Ala
        115                 120                 125
Asn Gln Arg Lys Gln Arg Tyr Thr Ala Ala Ala Pro Gly Gln His Gly
    130                 135                 140
Gln Val Tyr Ser Ser Ala Ala Ala Gln Val Asp Glu Pro Trp Pro Ser
145                 150                 155                 160
Cys Ser Ser Ala Ser Asp Pro Arg Ile His Glu Ala Asp Leu Val Gly
                165                 170                 175
Val Asp Ala Asp Arg Glu Glu Leu Leu Glu Gln Leu Ala Glu Arg Gln
            180                 185                 190
Pro Glu Gln Leu Lys Val Ile Ala Ile Val Gly Phe Cys Gly Leu Gly
        195                 200                 205
Lys Thr Ala Leu Ala Ala Glu Ala Tyr Asn Arg Glu Thr Gly Gly Gly
    210                 215                 220
Arg Phe Glu Arg His Ala Trp Val Cys Ala Gly His Arg Ser Ala Arg

225                 230                 235                 240
Glu Val Leu Gly Glu Leu Leu Arg Arg Leu Asp Ala Asp Gly Arg Ser
                245                 250                 255
Phe His Gly Asp Ser Asp Ala Gly Gln Leu Cys Val Asp Ile Arg Gln
            260                 265                 270
Gln Leu Glu Lys Asn Arg Tyr Phe Ile Val Ile Asp Asp Ile Gln Thr
        275                 280                 285
Glu Asp Gln Trp Lys Ser Ile Lys Ser Ala Phe Pro Thr Asp Lys Asp
    290                 295                 300
Ile Gly Ser Arg Ile Val Val Thr Thr Thr Ile Gln Ser Val Ala Asn
305                 310                 315                 320
Ala Cys Cys Ser Ala Asn Gly Tyr Leu His Lys Met Ser Arg Leu Asp
                325                 330                 335
Lys Asn Cys Ser Lys Gln Leu Leu Ser Lys Lys Ala Cys Pro Glu Arg
            340                 345                 350
Tyr Ser His Tyr Lys Gln Pro Asp Ser Ala Ala Ile Leu Lys Lys Cys
        355                 360                 365
Asp Gly Gln Pro Leu Ala Leu Val Thr Ile Gly Glu Phe Leu Gln Ala
    370                 375                 380
Asn Gly Trp Pro Thr Gly Pro Asn Cys Glu Asp Leu Cys Asn Arg Leu
385                 390                 395                 400
His Tyr His Leu Glu Asn Asp Lys Thr Leu Glu Arg Met Trp Arg Val
                405                 410                 415
Leu Val Arg Asn Tyr Thr Ser Leu Pro Gly His Ala Leu Lys Ala Cys
            420                 425                 430
Leu Leu Tyr Phe Gly Met Phe Pro Ser Asp His Pro Ile Arg Arg Lys
        435                 440                 445
Ser Leu Leu Arg Arg Trp Leu Ala Glu Gly Phe Val Glu Pro Leu Ser
    450                 455                 460
Ser Ser Ser Asn Ile Asp Ser Thr Ala Ala Phe Asn Val Leu Met Asp
465                 470                 475                 480
Arg Asn Ile Ile Glu Pro Ile Asn Val Ser Asn Asn Asp Lys Val Lys
                485                 490                 495
Thr Cys Gln Thr Tyr Gly Met Met Arg Glu Phe Ile Ser His Met Ser
            500                 505                 510
Ile Ser Gln Asn Phe Val Thr Phe Phe Cys Asp Asp Lys Phe Val Pro
        515                 520                 525
Lys Tyr Val Arg Arg Leu Ser Leu His Gly Asp Thr Val Val Asn Gly
    530                 535                 540
Asp Asn Phe Asn Gly Ile Asp Leu Ser Leu Val Arg Ser Leu Ala Val
545                 550                 555                 560
Phe Gly Glu Ala Gly Thr Thr Val Leu Asp Phe Ser Lys Tyr Gln Leu
                565                 570                 575
Leu Arg Val Leu Asp Leu Glu Lys Cys Asp Asp Leu Lys Asp Asp His
            580                 585                 590
Leu Lys Glu Ile Cys Asn Leu Val Leu Leu Lys Tyr Leu Ser Leu Gly
        595                 600                 605
Gly Asn Ile Ser Lys Leu Pro Lys Asp Ile Ala Lys Leu Lys Asp Leu
    610                 615                 620
Glu Ala Leu Asp Val Arg Arg Ser Lys Val Lys Ile Met Pro Val Glu
625                 630                 635                 640
Val Phe Gly Leu Pro Cys Leu Ile His Leu Leu Gly Lys Phe Lys Leu
                645                 650                 655
Ser Asp Lys Val Lys Gln Lys Thr Glu Val Gln Glu Phe Leu Leu Lys
            660                 665                 670
Gly Lys Ser Asn Leu Gln Thr Leu Ala Gly Phe Ala Ser Asn Gly Ser
        675                 680                 685
Glu Gly Phe Leu His Leu Met Arg Tyr Met Asn Lys Leu Arg Lys Leu
    690                 695                 700
Lys Ile Trp Cys Thr Ser Ser Ala Gly Ser Thr Asp Trp Thr Asp Leu
705                 710                 715                 720
Arg Glu Ala Ile Gln Gln Phe Ile Leu Asp Glu Lys Glu Ala Asn Ile
                725                 730                 735

Gly Thr Arg Ser Leu Ser Leu His Phe Ser Gly Cys Ser Glu Asp Ala
            740                 745                 750
Ile Asn Ser Leu Lys Glu Pro Cys Tyr Leu Ser Ser Leu Lys Leu His
        755                 760                 765
Gly Asn Phe Pro Gln Leu Pro Gln Phe Val Thr Ser Leu Arg Gly Leu
    770                 775                 780
Lys Glu Leu Cys Leu Ser Ser Thr Lys Phe Thr Thr Gly Leu Leu Glu
785                 790                 795                 800
Ala Leu Ser Asn Leu Ser Tyr Leu Gln Tyr Leu Lys Leu Val Ala Asp
                805                 810                 815
Glu Leu Glu Lys Phe Ile Ile Lys Val Gln Gly Phe Pro Arg Leu Leu
            820                 825                 830
Arg Leu Cys Ile Val Leu Gln Tyr Pro Thr Phe Pro Val Ile Glu Glu
        835                 840                 845
Gly Ala Leu Pro Phe Leu Val Thr Leu Gln Leu Leu Cys Lys Asp Leu
    850                 855                 860
His Gly Leu Ser Asp Ile Gln Ile Glu Cys Phe Lys His Leu Gln Glu
865                 870                 875                 880
Val Thr Leu His Ser Gly Val Thr Pro Ala Thr Arg Gln Glu Trp Val
                885                 890                 895
Lys Ala Ala Lys Glu His Pro Asn Arg Pro Lys Val Leu Leu Leu Lys
            900                 905                 910
Ser Val Asp Thr Ala Glu Ser Glu His Thr Asp Val Asp Ser Val Met
        915                 920                 925
Glu Ala Val Lys Ser Glu Thr Thr Glu Tyr Ser Ile Ala Pro Glu Gly
    930                 935                 940
Pro Glu Gln Val Asn Asn Lys Met Gln Leu Asp His Gly Leu Glu Ser
945                 950                 955                 960
Ser Ser Val Leu Asn Lys Gln Asn Asn Phe Ala Asp Gln Ser Ser Ser
                965                 970                 975
Lys Asp Gln Leu His Tyr Ser Phe Asn Asn Met Gly Leu Ser Asp Val
            980                 985                 990
Ser Cys Cys Glu
        995

<210> 156
<211> 3351
<212> DNA
<213> Artificial Sequence

<220>
<223> cDNA

<400> 156
atggatgctc cggcgagctt ttccctaggt gccatgggcc ctctcctcag gaagctcgat      60

tcgcttctgg tggctcccga aatccggctg cctaagccat tgaaggaggg aatcgagctg     120

ctcaaggaag atctagaaga gataggcgtc tccctggtgg agcactccgt ggtcgattca     180

ccaacccaca aggcaagatt ctggatggat gaggtgcgtg acctctccta ccacatcgag     240

gactgcatcg acaccatgtt ttcgatgcgc tccggcggcg acgacggcaa gcccagatcc     300

gagcgccgcc acaaggttgg ccgtgcgaag atcgacggtt tctccaagaa gcccaagccg     360

tgcacaagga tggctaggat cgcggagctc agggctctgg tgcgggaggc gagcgagcgg     420

ctcgaaaggt accagcttgg tgacgtctgc ggctcgagct ctcccgttgt gttcaccgcg     480

gatggtcggg ctcgacctct ccaccatggg gtttctgcta atcttgttgg cgtcgatgag     540

ttcaagacga agctgaacag gtggctcagc gacgaggagg ggccgcacct gaaggttgct 600

gccattgttg gacctgcagg gattggtaag acggcacttg ctacagagtt gtaccgtgac 660

catagatggc aattcgagtg ccgagctttc gtccgggcgt cgcgaaagcc tgacatgcag 720

cggcttcttg ggggtattct ctctcaagtt cagcgtcgcc aacgatcgtc tgatgcctac 780

gctgacagca cggtgcagag cctcattgat aatctccggg agcatctcca agataggagg 840

taccttatta taattgatgg tttgtgggag acggcagtat ggaatattgc taacagtgct 900

tttccagatg ttaacagttt cagtagaata ttaataactg cggatatcga acaggtagct 960

ctggaatgct gcggttacaa gtatgattat attatgagga tggaacctct tggcagcctt 1020

gattccaaaa aagttttctt caataaagtt tttggctccg aagatcaatg tcctcctgag 1080

ttgaaggagg tgtcaaacac aatcctagaa aaatgtggtg gtttgccact tgcaatcatc 1140

agcatagcag gtcttctagg aagccaaccg gaaaatccag tgctatggga ctatgtaacg 1200

aaatatctat gttccagttt gggtacaaat cctactttga aagacgtggt gaaagaaacg 1260

ctaaacctta gctacaacag tcttcctcat ccgttcaaaa catgtctgct atatcttggt 1320

atgtatcctg atgggcacat aatgttgaag gctgatttga tgaagcaatg gtcagctgaa 1380

ggttttgtct ctgccaatga agcaaaagac acagaagaaa ttgtagataa atactttgat 1440

gagcttgtca acaggggaat tctagaacct gttgagatta acaagaatgg caaggtgttg 1500

tcctgtaccc tgcaccacgc tgtacatgac cttgtcatgc ccaaattcaa cgatgacaaa 1560

tttactatgt cagtagatta ctctcagact attacaggac cttctaccat ggttcgtcgt 1620

ttgtcccttc atttcagcag taccagatac gcgacaaaac ctgcaggaat aatattgtca 1680

cgagttcgat cacttgcctt ttttggcctc ctcaattgta tgccttgtat tggggaattt 1740

aagttactgc gagttctaat cctagaattt tggggcagtc atggtgagca aaggagttta 1800

aacctaatac cagtatgtag attatttcag ctgagatatt tgaaaacttc aggtgatgta 1860

gttgttcagt taccagctca gataagtggg ctacagtact tggaaacact ggaaatagac 1920

gcaagagtgt cagctgttcc atttgatctt gttcatctcc cgaacctact gcatctccaa 1980

cttcaagatg agacaaaatt acctgatggg attggctgca tgagatcttt gcgtacacta 2040

cagtactttg accttggtaa caactctgta gacaacttac ggggtcttgg agagctgaca 2100

aacctgcagg atcttcatct cagctattct gcaccgtcat ctaatgaggg tttgatgata 2160

aatctgaatg ctataacctc ttcacttagc agacttagta acctcaaatc tctgattcta 2220

tcaccaggtg ctataagcat ggttattttc tttgatatat caagcatcat atctgtggtt 2280

cctgtatttc ttcagagact tgagctgttg ccacccatct gcatcttttg cagacttccc 2340

aagtcgattg gacaacttca caaactctgc attttgaaag tttccgttag agaacttctg 2400

acaactgata ttgataacct cacaggatta ccttccctca cagttctctc attatatgct 2460

cagacagctc cagagggaag gtttatcttc aaagacggga ctctcccagt tctcaagtat 2520

ttcaaatttg gatgtggtga actctgcctt gcttttatgg ctggagcaat gcccaatctc 2580

cagaggctca aattagtttt caacatccgt aaaagtgaga agtaccgcca tacgcttttt 2640

gggattgagc accttgtaag cctccaggat attgccacac gaattggggt tgacaccagc 2700

accggcgaat ctgatagaag ggctgcagag tcagcattca aggaaactgt caacaagcat 2760

ccaaggtgtc ttaggtccag cttgcagtgg gtggtttcca ctgaggaaga aagtcatcct 2820

ttggagaaac aacatcataa gagagaaaaa ggctcatcag ctggacatgg ggttttagag 2880

aaagaatcag tggaagatag cgagaagaac actgatagag tgcaaacatt attgtcaccc 2940

caactgtcaa acatggagag tgtggtggaa agtgcattaa cggggcaacg aactaagata 3000

gttgttaagg tgcacatgcc atgcggaaaa tcccgagcaa aagccatggc gctggctgcg 3060

tcagtgaacg gggtggacag cgtggagata acgggggagg acaaagaccg gctggtggtg 3120

gtcggccgtg gcattgaccc tgttcgcctg gtggctctcc tgcgcgagaa atgtggcctc 3180

gccgagctct tgatggtgga gttagttgag aaagagaaga cacagctggc tggaggaaag 3240

aaaggtgcat acaagaagca ccctacctac aatttatcgc cgtttgacta tgtagagtat 3300

ccaccttcag caccaataat gcaagatatt aacccttgct caaccatgtg a 3351

<210> 157
<211> 1116
<212> PRT
<213> Oryza sativa

<400> 157
Met Asp Ala Pro Ala Ser Phe Ser Leu Gly Ala Met Gly Pro Leu Leu
1               5                   10                  15
Arg Lys Leu Asp Ser Leu Leu Val Ala Pro Glu Ile Arg Leu Pro Lys
            20                  25                  30
Pro Leu Lys Glu Gly Ile Glu Leu Leu Lys Glu Asp Leu Glu Glu Ile
        35                  40                  45
Gly Val Ser Leu Val Glu His Ser Val Val Asp Ser Pro Thr His Lys
    50                  55                  60
Ala Arg Phe Trp Met Asp Glu Val Arg Asp Leu Ser Tyr His Ile Glu
65                  70                  75                  80
Asp Cys Ile Asp Thr Met Phe Ser Met Arg Ser Gly Gly Asp Asp Gly
                85                  90                  95
Lys Pro Arg Ser Glu Arg Arg His Lys Val Gly Arg Ala Lys Ile Asp
            100                 105                 110
Gly Phe Ser Lys Lys Pro Lys Pro Cys Thr Arg Met Ala Arg Ile Ala
        115                 120                 125
Glu Leu Arg Ala Leu Val Arg Glu Ala Ser Glu Arg Leu Glu Arg Tyr
    130                 135                 140
Gln Leu Gly Asp Val Cys Gly Ser Ser Ser Pro Val Val Phe Thr Ala
145                 150                 155                 160
Asp Gly Arg Ala Arg Pro Leu His His Gly Val Ser Ala Asn Leu Val
                165                 170                 175
Gly Val Asp Glu Phe Lys Thr Lys Leu Asn Arg Trp Leu Ser Asp Glu
            180                 185                 190

Glu Gly Pro His Leu Lys Val Ala Ala Ile Val Gly Pro Ala Gly Ile
        195                 200                 205
Gly Lys Thr Ala Leu Ala Thr Glu Leu Tyr Arg Asp His Arg Trp Gln
    210                 215                 220
Phe Glu Cys Arg Ala Phe Val Arg Ala Ser Arg Lys Pro Asp Met Gln
225                 230                 235                 240
Arg Leu Leu Gly Gly Ile Leu Ser Gln Val Gln Arg Arg Gln Arg Ser
                245                 250                 255
Ser Asp Ala Tyr Ala Asp Ser Thr Val Gln Ser Leu Ile Asp Asn Leu
            260                 265                 270
Arg Glu His Leu Gln Asp Arg Arg Tyr Leu Ile Ile Ile Asp Gly Leu
        275                 280                 285
Trp Glu Thr Ala Val Trp Asn Ile Ala Asn Ser Ala Phe Pro Asp Val
    290                 295                 300
Asn Ser Phe Ser Arg Ile Leu Ile Thr Ala Asp Ile Glu Gln Val Ala
305                 310                 315                 320
Leu Glu Cys Cys Gly Tyr Lys Tyr Asp Tyr Ile Met Arg Met Glu Pro
                325                 330                 335
Leu Gly Ser Leu Asp Ser Lys Lys Val Phe Phe Asn Lys Val Phe Gly
            340                 345                 350
Ser Glu Asp Gln Cys Pro Pro Glu Leu Lys Glu Val Ser Asn Thr Ile
        355                 360                 365
Leu Glu Lys Cys Gly Gly Leu Pro Leu Ala Ile Ile Ser Ile Ala Gly
    370                 375                 380
Leu Leu Gly Ser Gln Pro Glu Asn Pro Val Leu Trp Asp Tyr Val Thr
385                 390                 395                 400
Lys Tyr Leu Cys Ser Ser Leu Gly Thr Asn Pro Thr Leu Lys Asp Val
                405                 410                 415
Val Lys Glu Thr Leu Asn Leu Ser Tyr Asn Ser Leu Pro His Pro Phe
            420                 425                 430
Lys Thr Cys Leu Leu Tyr Leu Gly Met Tyr Pro Asp Gly His Ile Met
        435                 440                 445
Leu Lys Ala Asp Leu Met Lys Gln Trp Ser Ala Glu Gly Phe Val Ser
    450                 455                 460
Ala Asn Glu Ala Lys Asp Thr Glu Glu Ile Val Asp Lys Tyr Phe Asp
465                 470                 475                 480
Glu Leu Val Asn Arg Gly Ile Leu Glu Pro Val Glu Ile Asn Lys Asn
                485                 490                 495
Gly Lys Val Leu Ser Cys Thr Leu His His Ala Val His Asp Leu Val
            500                 505                 510
Met Pro Lys Phe Asn Asp Asp Lys Phe Thr Met Ser Val Asp Tyr Ser
        515                 520                 525
Gln Thr Ile Thr Gly Pro Ser Thr Met Val Arg Arg Leu Ser Leu His
    530                 535                 540
Phe Ser Ser Thr Arg Tyr Ala Thr Lys Pro Ala Gly Ile Ile Leu Ser
545                 550                 555                 560
Arg Val Arg Ser Leu Ala Phe Phe Gly Leu Leu Asn Cys Met Pro Cys
                565                 570                 575
Ile Gly Glu Phe Lys Leu Leu Arg Val Leu Ile Leu Glu Phe Trp Gly
            580                 585                 590
Ser His Gly Glu Gln Arg Ser Leu Asn Leu Ile Pro Val Cys Arg Leu
        595                 600                 605
Phe Gln Leu Arg Tyr Leu Lys Thr Ser Gly Asp Val Val Val Gln Leu
    610                 615                 620
Pro Ala Gln Ile Ser Gly Leu Gln Tyr Leu Glu Thr Leu Glu Ile Asp
625                 630                 635                 640
Ala Arg Val Ser Ala Val Pro Phe Asp Leu Val His Leu Pro Asn Leu
                645                 650                 655
Leu His Leu Gln Leu Gln Asp Glu Thr Lys Leu Pro Asp Gly Ile Gly
            660                 665                 670
Cys Met Arg Ser Leu Arg Thr Leu Gln Tyr Phe Asp Leu Gly Asn Asn
        675                 680                 685
Ser Val Asp Asn Leu Arg Gly Leu Gly Glu Leu Thr Asn Leu Gln Asp

690                 695                 700
Leu His Leu Ser Tyr Ser Ala Pro Ser Ser Asn Glu Gly Leu Met Ile
705                 710                 715                 720
Asn Leu Asn Ala Ile Thr Ser Ser Leu Ser Arg Leu Ser Asn Leu Lys
                725                 730                 735
Ser Leu Ile Leu Ser Pro Gly Ala Ile Ser Met Val Ile Phe Phe Asp
            740                 745                 750
Ile Ser Ser Ile Ile Ser Val Val Pro Val Phe Leu Gln Arg Leu Glu
        755                 760                 765
Leu Leu Pro Pro Ile Cys Ile Phe Cys Arg Leu Pro Lys Ser Ile Gly
    770                 775                 780
Gln Leu His Lys Leu Cys Ile Leu Lys Val Ser Val Arg Glu Leu Leu
785                 790                 795                 800
Thr Thr Asp Ile Asp Asn Leu Thr Gly Leu Pro Ser Leu Thr Val Leu
                805                 810                 815
Ser Leu Tyr Ala Gln Thr Ala Pro Glu Gly Arg Phe Ile Phe Lys Asp
            820                 825                 830
Gly Thr Leu Pro Val Leu Lys Tyr Phe Lys Phe Gly Cys Gly Glu Leu
        835                 840                 845
Cys Leu Ala Phe Met Ala Gly Ala Met Pro Asn Leu Gln Arg Leu Lys
    850                 855                 860
Leu Val Phe Asn Ile Arg Lys Ser Glu Lys Tyr Arg His Thr Leu Phe
865                 870                 875                 880
Gly Ile Glu His Leu Val Ser Leu Gln Asp Ile Ala Thr Arg Ile Gly
                885                 890                 895
Val Asp Thr Ser Thr Gly Glu Ser Asp Arg Arg Ala Ala Glu Ser Ala
            900                 905                 910
Phe Lys Glu Thr Val Asn Lys His Pro Arg Cys Leu Arg Ser Ser Leu
        915                 920                 925
Gln Trp Val Val Ser Thr Glu Glu Glu Ser His Pro Leu Glu Lys Gln
    930                 935                 940
His His Lys Arg Glu Lys Gly Ser Ser Ala Gly His Gly Val Leu Glu
945                 950                 955                 960
Lys Glu Ser Val Glu Asp Ser Glu Lys Asn Thr Asp Arg Val Gln Thr
                965                 970                 975
Leu Leu Ser Pro Gln Leu Ser Asn Met Glu Ser Val Val Glu Ser Ala
            980                 985                 990
Leu Thr Gly Gln Arg Thr Lys Ile Val Val Lys Val His Met Pro Cys
        995                 1000                1005
Gly Lys Ser Arg Ala Lys Ala Met Ala Leu Ala Ala Ser Val Asn Gly
    1010                1015                1020
Val Asp Ser Val Glu Ile Thr Gly Glu Asp Lys Asp Arg Leu Val Val
1025                1030                1035                1040
Val Gly Arg Gly Ile Asp Pro Val Arg Leu Val Ala Leu Leu Arg Glu
                1045                1050                1055
Lys Cys Gly Leu Ala Glu Leu Leu Met Val Glu Leu Val Glu Lys Glu
            1060                1065                1070
Lys Thr Gln Leu Ala Gly Gly Lys Lys Gly Ala Tyr Lys Lys His Pro
        1075                1080                1085
Thr Tyr Asn Leu Ser Pro Phe Asp Tyr Val Glu Tyr Pro Pro Ser Ala
    1090                1095                1100
Pro Ile Met Gln Asp Ile Asn Pro Cys Ser Thr Met
1105                1110                1115

<210> 158
<211> 270
<212> DNA
<213> Artificial Sequence

<220>
<223> cDNA

<400> 158
atgaaagtcc aagctacatt cgccaccctt atcgcccttg cggcttactt tccagcagcc      60

aatgcttgga aagattgcat catccaacgt tataaagacg gcgatgtcaa caacatatat     120

actgccaata ggaacgaaga gataactatt gaggaatata aagtcttcgt taatgaggcc     180

tgccatccct acccagttat acttcccgac agatcggtcc tttctggcga ttttacatca     240

gcttacgctg acgacgatga gtcttgttga                                      270

<210> 159
<211> 89
<212> PRT
<213> Magnaporthe grisea

<400> 159
Met Lys Val Gln Ala Thr Phe Ala Thr Leu Ile Ala Leu Ala Ala Tyr
1               5                   10                  15
Phe Pro Ala Ala Asn Ala Trp Lys Asp Cys Ile Ile Gln Arg Tyr Lys
            20                  25                  30
Asp Gly Asp Val Asn Asn Ile Tyr Thr Ala Asn Arg Asn Glu Glu Ile
        35                  40                  45
Thr Ile Glu Glu Tyr Lys Val Phe Val Asn Glu Ala Cys His Pro Tyr
    50                  55                  60
Pro Val Ile Leu Pro Asp Arg Ser Val Leu Ser Gly Asp Phe Thr Ser
65                  70                  75                  80
Ala Tyr Ala Asp Asp Asp Glu Ser Cys
                85

<210> 160
<211> 258
<212> DNA
<213> Artificial Sequence

<220>
<223> cDNA

<400> 160
atgcattttt cgacaatttt catccccttt gccttagctg ctctaaaagt aagcgctgcg      60

ccagctagat tttgcgtcta ttacgacggc caccttcccg cgacacgtgt cctgcttatg     120

tacgttagaa tcggcactac agcgactatt acggcccgtg ggcacgaatt cgaagttgaa     180

gcaaaagacc agaattgcaa agttattctc accaatggca aacaagcacc ggattggctt     240

gctgccgagc cttactag                                                   258

<210> 161
<211> 85
<212> PRT
<213> Magnaporthe oryzae

<400> 161
Met His Phe Ser Thr Ile Phe Ile Pro Phe Ala Leu Ala Ala Leu Lys
1               5                   10                  15
Val Ser Ala Ala Pro Ala Arg Phe Cys Val Tyr Tyr Asp Gly His Leu

20                  25                  30
Pro Ala Thr Arg Val Leu Leu Met Tyr Val Arg Ile Gly Thr Thr Ala
        35                  40                  45
Thr Ile Thr Ala Arg Gly His Glu Phe Glu Val Glu Ala Lys Asp Gln
    50                  55                  60
Asn Cys Lys Val Ile Leu Thr Asn Gly Lys Gln Ala Pro Asp Trp Leu
65                  70                  75                  80
Ala Ala Glu Pro Tyr
                85

<210> 162
<211> 3078
<212> DNA
<213> Artificial Sequence


<220>
<223> cDNA

<400> 162
atggttggcg ccgagatgct tgtggccgcg gcggtgagcc aggtcgcccg gaagatcaac      60

gacatcgtgg gggtcgcgca gggcgaggtg aagctgtgct gcaatttcag cgacgatttg     120

gagggcatca aggataccct tgtgtacctg gaaaccttgc tgaaaaatgc ggagaataac     180

tccttcggaa gcgacagggc caacctgcgc cactggcttg gccagatcaa gtccctggct     240

tacgatatcg aagatatcgt tgatgggtac tactcttcca aggagcagtt cgatgggggc     300

agctatgcac agaaggggtc attattctgc tcgctatcca atccaatgct tctgaaaggt     360

agcatggttt ataagatgaa atccaagaga gagatgctac agcaaagcca acagttgccc     420

aatcagtatc atttcctttc atatatcaat tcagctgtgc attattttga ggagaagcaa     480

acaacatcat acagaaatac tgacattgca attgtcggga gggatgctga tttggatcat     540

ctcatggatc ttttaatgca aaacagcgct gaagagcttt gtattatacc catagttggg     600

cctgtaggtt ttggaaagac aagccttgca cagttagttt tcaatgatac aagaacagag     660

gtattcagct ttaggatatg ggttcatgtt tccatgggta atatcaacct tgaaaaaatt     720

gggagagata tagtttcaca aactacagaa aaaattgagg gaaatatgca gctgcagtca     780

atcaagaatg ctgttcagcg tgtgctaaat aaatatagtt gcttgatcat aatagacagc     840

ctttggggaa aggatgaaga agtgaatgaa ttgaagcaga tgttgcttac aggtagacac     900

acagaaagca agatcatagt gaccactcat agcaataaag tagctaagct gatttccacc     960

gttccactgt acaagttggc agctttatct gaggatgatt gtttaaaaat attctctcaa    1020

agggcaatga caggtccggg tgacccgttg ttcagggaat atggagaaga aatcgttaga    1080

aggtgtgaag gcacaccctt ggtagccaat tttctcggtt ctgtggtgaa tgctcaacga    1140

caaaggcgtg agatttggca agctgcaaag gataaagaaa tgtggaagat agaggaagat    1200

tatccccaag acaaaacttc accactattt ccatcattca agataatata ttataatatg    1260

ccccatgagc taagattatg ctttgtatat tgttcaatct tccctaaagg aactgttata    1320

gaaaagaaga aacttattca gcaatggatt gcacttgaca tgattgagtc caaacatgga 1380

accttgccac ttgatgtaac tgcggagaaa tatattgatg aacttaaagc aatctatttc 1440

cttcaagttt tagagcggtc tcagaatgat gcagaaagat ccagtgcttc tgaggaaatg 1500

cttcgcatgc ataacttggc tcatgatctt gctagatcgg ttgctggtga agatatcctt 1560

gttattttag atgccgagaa tgagcgcaat gccagatatt gcgattaccg ttatgcacag 1620

gtgtctgctt ctagtttaga gtcaatcgat cgcaaggcat ggccttccaa ggcaaggtca 1680

ctaattttca agaatagtgg tgcggacttt gagcgtgtca gtgaagttct ttcagtgaac 1740

aaatacctgc gtgttttgga tctcagtgga tgttgtgttc aagatattcc atctcctatc 1800

tttcagctga aacaattgag atacctcgac gtttcatctt tatctattac agcactccct 1860

ctgcaaatta gtagctttca taagttacaa atgttggatc tttcagaaac tgaactaaca 1920

gagttgccac cctttataag caacttaaaa ggactgaatt atttgaatct ccaaggttgc 1980

cagaaacttc aacgattgaa tagccttcat ttgttgcatg atctacatta cctaaacttg 2040

tcatgctgcc ctgaagttac tagttttcct gaatctattg aaaatctgac caaactccgt 2100

ttcttgaatc tttctggatg ctctaagctt tcaacattac ctatcagatt tttggaatca 2160

tttgctagcc tctgttcttt ggtagatctt aacttaagtg gctttgaatt ccaaatgttg 2220

cccgactttt ttggcaacat atattcactt cagtatttaa atctgtcaaa atgtttgaaa 2280

cttgaggtat taccacaatc ttttggccaa cttgcatatc tgaaaagcct aaatctttca 2340

tattgttctg atcttaaact gctggaatcc tttgaatgcc ttacctctct tcggtttttg 2400

aatctctcga actgctctag gcttgaatat ttgccatcgt gctttgacaa gcttaataat 2460

ttagagtctc tgaatttatc acaatgtctt ggacttaaag cactacctga atcacttcaa 2520

aaccttaaaa atcttcagct tgatgtttct gggtgtcagg attgtatagt acaatccttt 2580

tctctaagta ccagaagttc ccagtcctgc caacggtcgg agaaagctga gcaggtcaga 2640

tcaagaaaca gtgaaatttc agagatcact tatgaggaac ctgctgagat tgaactttta 2700

aagaataatc caagtaaaga tttggcctcc atctcacacc taaatgagga tagaattgag 2760

gagcctgaag ttgtcactga gccaagtgca actagaggta tggtacaaca gattccagga 2820

aaccagctct catcgccttc atctcatctt tcttcctttg catcaagctc agcgccattt 2880

gcatcctcct cttcggacac ctcaacaagt gagcatccag tgcctaatga agaggcggca 2940

gctttgacag ttcctcggtc caacgagaaa tgcgacaaca ctcccatgcc ggtaaaagat 3000

ggcctgatat ctgaagatga tgcaccggta catctgcatc agaagcccct gcaggcgaca 3060

gccatggcag ccatatga 3078

<210> 163

<211> 1025
<212> PRT
<213> Oryza sativa

<220>
<223> cDNA

<400> 163
Met Val Gly Ala Glu Met Leu Val Ala Ala Ala Val Ser Gln Val Ala
1               5                   10                  15
Arg Lys Ile Asn Asp Ile Val Gly Val Ala Gln Gly Glu Val Lys Leu
            20                  25                  30
Cys Cys Asn Phe Ser Asp Asp Leu Glu Gly Ile Lys Asp Thr Leu Val
        35                  40                  45
Tyr Leu Glu Thr Leu Leu Lys Asn Ala Glu Asn Asn Ser Phe Gly Ser
    50                  55                  60
Asp Arg Ala Asn Leu Arg His Trp Leu Gly Gln Ile Lys Ser Leu Ala
65                  70                  75                  80
Tyr Asp Ile Glu Asp Ile Val Asp Gly Tyr Tyr Ser Ser Lys Glu Gln
                85                  90                  95
Phe Asp Gly Gly Ser Tyr Ala Gln Lys Gly Ser Leu Phe Cys Ser Leu
            100                 105                 110
Ser Asn Pro Met Leu Leu Lys Gly Ser Met Val Tyr Lys Met Lys Ser
        115                 120                 125
Lys Arg Glu Met Leu Gln Gln Ser Gln Gln Leu Pro Asn Gln Tyr His
    130                 135                 140
Phe Leu Ser Tyr Ile Asn Ser Ala Val His Tyr Phe Glu Glu Lys Gln
145                 150                 155                 160
Thr Thr Ser Tyr Arg Asn Thr Asp Ile Ala Ile Val Gly Arg Asp Ala
                165                 170                 175
Asp Leu Asp His Leu Met Asp Leu Leu Met Gln Asn Ser Ala Glu Glu
            180                 185                 190
Leu Cys Ile Ile Pro Ile Val Gly Pro Val Gly Phe Gly Lys Thr Ser
        195                 200                 205
Leu Ala Gln Leu Val Phe Asn Asp Thr Arg Thr Glu Val Phe Ser Phe
    210                 215                 220
Arg Ile Trp Val His Val Ser Met Gly Asn Ile Asn Leu Glu Lys Ile
225                 230                 235                 240
Gly Arg Asp Ile Val Ser Gln Thr Thr Glu Lys Ile Glu Gly Asn Met
                245                 250                 255
Gln Leu Gln Ser Ile Lys Asn Ala Val Gln Arg Val Leu Asn Lys Tyr
            260                 265                 270
Ser Cys Leu Ile Ile Ile Asp Ser Leu Trp Gly Lys Asp Glu Glu Val
        275                 280                 285
Asn Glu Leu Lys Gln Met Leu Leu Thr Gly Arg His Thr Glu Ser Lys
    290                 295                 300
Ile Ile Val Thr Thr His Ser Asn Lys Val Ala Lys Leu Ile Ser Thr
305                 310                 315                 320
Val Pro Leu Tyr Lys Leu Ala Ala Leu Ser Glu Asp Asp Cys Leu Lys
                325                 330                 335
Ile Phe Ser Gln Arg Ala Met Thr Gly Pro Gly Asp Pro Leu Phe Arg
            340                 345                 350
Glu Tyr Gly Glu Glu Ile Val Arg Arg Cys Glu Gly Thr Pro Leu Val
        355                 360                 365
Ala Asn Phe Leu Gly Ser Val Val Asn Ala Gln Arg Gln Arg Arg Glu
    370                 375                 380
Ile Trp Gln Ala Ala Lys Asp Lys Glu Met Trp Lys Ile Glu Glu Asp
385                 390                 395                 400
Tyr Pro Gln Asp Lys Thr Ser Pro Leu Phe Pro Ser Phe Lys Ile Ile
                405                 410                 415
Tyr Tyr Asn Met Pro His Glu Leu Arg Leu Cys Phe Val Tyr Cys Ser
            420                 425                 430

Ile Phe Pro Lys Gly Thr Val Ile Glu Lys Lys Lys Leu Ile Gln Gln
        435                 440                 445
Trp Ile Ala Leu Asp Met Ile Glu Ser Lys His Gly Thr Leu Pro Leu
    450                 455                 460
Asp Val Thr Ala Glu Lys Tyr Ile Asp Glu Leu Lys Ala Ile Tyr Phe
465                 470                 475                 480
Leu Gln Val Leu Glu Arg Ser Gln Asn Asp Ala Glu Arg Ser Ser Ala
                485                 490                 495
Ser Glu Glu Met Leu Arg Met His Asn Leu Ala His Asp Leu Ala Arg
            500                 505                 510
Ser Val Ala Gly Glu Asp Ile Leu Val Ile Leu Asp Ala Glu Asn Glu
        515                 520                 525
Arg Asn Ala Arg Tyr Cys Asp Tyr Arg Tyr Ala Gln Val Ser Ala Ser
    530                 535                 540
Ser Leu Glu Ser Ile Asp Arg Lys Ala Trp Pro Ser Lys Ala Arg Ser
545                 550                 555                 560
Leu Ile Phe Lys Asn Ser Gly Ala Asp Phe Glu Arg Val Ser Glu Val
                565                 570                 575
Leu Ser Val Asn Lys Tyr Leu Arg Val Leu Asp Leu Ser Gly Cys Cys
            580                 585                 590
Val Gln Asp Ile Pro Ser Pro Ile Phe Gln Leu Lys Gln Leu Arg Tyr
        595                 600                 605
Leu Asp Val Ser Ser Leu Ser Ile Thr Ala Leu Pro Leu Gln Ile Ser
    610                 615                 620
Ser Phe His Lys Leu Gln Met Leu Asp Leu Ser Glu Thr Glu Leu Thr
625                 630                 635                 640
Glu Leu Pro Pro Phe Ile Ser Asn Leu Lys Gly Leu Asn Tyr Leu Asn
                645                 650                 655
Leu Gln Gly Cys Gln Lys Leu Gln Arg Leu Asn Ser Leu His Leu Leu
            660                 665                 670
His Asp Leu His Tyr Leu Asn Leu Ser Cys Cys Pro Glu Val Thr Ser
        675                 680                 685
Phe Pro Glu Ser Ile Glu Asn Leu Thr Lys Leu Arg Phe Leu Asn Leu
    690                 695                 700
Ser Gly Cys Ser Lys Leu Ser Thr Leu Pro Ile Arg Phe Leu Glu Ser
705                 710                 715                 720
Phe Ala Ser Leu Cys Ser Leu Val Asp Leu Asn Leu Ser Gly Phe Glu
                725                 730                 735
Phe Gln Met Leu Pro Asp Phe Phe Gly Asn Ile Tyr Ser Leu Gln Tyr
            740                 745                 750
Leu Asn Leu Ser Lys Cys Leu Lys Leu Glu Val Leu Pro Gln Ser Phe
        755                 760                 765
Gly Gln Leu Ala Tyr Leu Lys Ser Leu Asn Leu Ser Tyr Cys Ser Asp
    770                 775                 780
Leu Lys Leu Leu Glu Ser Phe Glu Cys Leu Thr Ser Leu Arg Phe Leu
785                 790                 795                 800
Asn Leu Ser Asn Cys Ser Arg Leu Glu Tyr Leu Pro Ser Cys Phe Asp
                805                 810                 815
Lys Leu Asn Asn Leu Glu Ser Leu Asn Leu Ser Gln Cys Leu Gly Leu
            820                 825                 830
Lys Ala Leu Pro Glu Ser Leu Gln Asn Leu Lys Asn Leu Gln Leu Asp
        835                 840                 845
Val Ser Gly Cys Gln Asp Cys Ile Val Gln Ser Phe Ser Leu Ser Thr
    850                 855                 860
Arg Ser Ser Gln Ser Cys Gln Arg Ser Glu Lys Ala Glu Gln Val Arg
865                 870                 875                 880
Ser Arg Asn Ser Glu Ile Ser Glu Ile Thr Tyr Glu Glu Pro Ala Glu
                885                 890                 895
Ile Glu Leu Leu Lys Asn Asn Pro Ser Lys Asp Leu Ala Ser Ile Ser
            900                 905                 910
His Leu Asn Glu Asp Arg Ile Glu Glu Pro Glu Val Val Thr Glu Pro
        915                 920                 925
Ser Ala Thr Arg Gly Met Val Gln Gln Ile Pro Gly Asn Gln Leu Ser

930                 935                 940
Ser Pro Ser Ser His Leu Ser Ser Phe Ala Ser Ser Ser Ala Pro Phe
945                 950                 955                 960
Ala Ser Ser Ser Ser Asp Thr Ser Thr Ser Glu His Pro Val Pro Asn
                965                 970                 975
Glu Glu Ala Ala Ala Leu Thr Val Pro Arg Ser Asn Glu Lys Cys Asp
            980                 985                 990
Asn Thr Pro Met Pro Val Lys Asp Gly Leu Ile Ser Glu Asp Asp Ala
        995                 1000                1005
Pro Val His Leu His Gln Lys Pro Leu Gln Ala Thr Ala Met Ala Ala
    1010                1015                1020
Ile
1025

<210> 164
<211> 213
<212> DNA
<213> Artificial Sequence

<220>
<223> cDNA

<400> 164
atgcaacttt ccaaaattac tttcgctatt gcattatatg caatcggaat cgcagcactt      60

cccactccgg ccagcctgaa tggcaacact gaggtcgcaa ccatctccga cgttaaactt     120

gaggcccgca gcgacaccac ttatcataaa tgctccaaat gcggttatgg cagcgatgat     180

tccgacgcgt attttaatca taaatgcaac taa                                  213

<210> 165
<211> 70
<212> PRT
<213> Magnaporthe oryzae

<400> 165
Met Gln Leu Ser Lys Ile Thr Phe Ala Ile Ala Leu Tyr Ala Ile Gly
1               5                   10                  15
Ile Ala Ala Leu Pro Thr Pro Ala Ser Leu Asn Gly Asn Thr Glu Val
            20                  25                  30
Ala Thr Ile Ser Asp Val Lys Leu Glu Ala Arg Ser Asp Thr Thr Tyr
        35                  40                  45
His Lys Cys Ser Lys Cys Gly Tyr Gly Ser Asp Asp Ser Asp Ala Tyr
    50                  55                  60
Phe Asn His Lys Cys Asn
65                  70

<210> 166
<211> 3432
<212> DNA
<213> Oryza sativa

<220>
<223> cDNA

<400> 166
atggaggcgg ctgccatggc cgtaaccgca gccacggggg ccttggcgcc cgtgctagtg      60

aagctggccg ctttgctgga cgacggggag tgcaatcttc tggaggggag ccggagcgac 120

gcagagttca tcagatccga gctggaggcc gtgcactctc tcctcacccc aaatatcttg 180

gggaggatgg gggatgacga tgcggcgtgc aaggatggct tgattgcgga ggtccgggag 240

ctgtcctacg acctggatga tgccgtcgac gacttcttgg agctcaattt cgagcagcga 300

agaagcgcaa gccctttcgg tgagctcaag gcaagagttg aggagcgtgt ctccaatcgc 360

ttctctgact ggaagctacc ggcggcgagc cttccgccgt cgtcggtaca ccgtcgagct 420

ggcttgccgc caccagatgc agggctggtg ggatgcacaa aacgtaagga agagctcatc 480

gagttgctgg aacaagggag cagtgatgct tcacgatggc gcaagcgaaa acctcatgtc 540

cccctcagga taatggaggg ggaaatgcaa aaatcgtgtt caagattccc atggtggacg 600

gataagagcc gtacaaaagc aatgtcattg gttgcaagta cggttggagt gcactcggtt 660

gcaatcgccg gtgacctaag agacgaggtt gtggtggtcg gtgatggcat tgactccatc 720

aatctggtct ctgcgctccg gaagaaggtg ggccatgcgg agttgctgca ggtcagccaa 780

gtaaaggaag atgtgaagga gataacggcg atgcttgcgc cggtgaaatc catatgtgaa 840

tttcacgagg tcaaaacaat ttgcatcctt ggattgccag gggaggcaaa aacaacgatt 900

gcccgagtac tatatcatgc attgggaacg cagttccaat gccgggtttt cgcatcaatc 960

tctccaagtt ccagccccag tcccaatcta acagagactc ttgcagacat tttcgctcaa 1020

gcacaactag gagtaactga tacacttagc acaccatatg gtgggagtgg gaccgggaga 1080

gctcttcaac aacatctcat cgacaacata tcagctttcc tcctcaacaa aaagtatctc 1140

attgtaatcg atgacatttg gcattgggaa gaatgggaag tcatcagaaa gtccattccc 1200

aagaatgatc tgggtggtag aataatcatg actactcgtc ttaattcaat agctgagaag 1260

tgccacactg atgacaatga tgtttttgtc tacgaagttg gggatctaga taataatgat 1320

gctttgtcgt tgtcttgggg gatagcaaca aagtctgggg caggcaacag gatcggaact 1380

ggagaggata attcatgcta tgatattgtg aacatgtgtt atggtatgcc tttagcactt 1440

atttggctgt cgtcagcatt ggttggagag atagaagaat taggtggtgc tgaagtgaaa 1500

aaatgtaggg atttgagaca catagaggat ggtattttgg acatcccatc cttacaacca 1560

ttggcggaga gtttatgcct tggttataac catcttcctc tttatctgag gactttgttg 1620

ttgtactgta gtgcatacca ttggtctaac agaatcgaaa ggggtcgtct ggtcaggagg 1680

tggattgcgg aaggatttgt gtcggaagag aaagaagcag aaggttactt tggcgagctt 1740

attaacagag gatggattac gcagcacgga gacaacaaca gttataatta ctatgagatc 1800

caccccgtga tgctggcctt cctgagatgc aagtccaagg agtacaattt tttaacatgc 1860

ttgggtctgg gatctgatac tagtactagt gcatcctccc caaggttgat tcgccggctg 1920

tctcttcagg gggggtatcc agtggactgc ttgtcaagca tgagtatgga tgtgtcacac 1980

acttgcagcc ttgtcgtcct tggtgacgtg gcgcgaccca agggaatccc cttctatatg 2040

tttaagcgct tgcgagtgtt ggaccttgaa gataataagg atatacagga ttctcatctg 2100

cagggcatat gtgaacagtt aagcctcaga gtgaggtacc ttggtctcaa gggaacgcgg 2160

atccgaaagc tccctcagga gatgaggaag ctgaagcatt tggagatttt gtatgtgggg 2220

agcactcgga tcagtgaact tccgcaagag attggagagc tgaagcatct gcggattctg 2280

gacgtgagaa acacggacat cactgagctc ccactgcaga tacgggagct gcagcatctg 2340

cacactctgg acgtgaggaa cactccaatc agtgagctcc cgccgcaggt tggcaagctg 2400

cagaatctca agattatgtg cgtgaggagc actggggtta gggagctccc aaaggagatt 2460

ggggagctga atcatctaca gactctggac gtgagaaaca cgagggtgag agagctgcca 2520

tggcaagctg gccagatctc ccaatcgttg cgcgtgcttg ccggtgacag tggcgatggc 2580

gtgcggttgc ccgaaggcgt ctgcgaagct ctgatcaacg gtattccagg ggctacgcgt 2640

gcaaaatgca gggaggttct gtccatcgcg atcatcgatc gtttcggacc tccccttgtt 2700

gggatattca aagttcccgg cagtcatatg cgtatcccga agatgatcaa agaccacttc 2760

cgcgttcttt cttgcctaga catcaggctc tgccacaagc ttgaggatga tgaccaaaag 2820

ttcctcgccg agatgcccaa cctgcagacg ctcgtgctga ggttcgaggc cctaccaaga 2880

caacccataa ccatcaacgg cacaggcttc cagatgctgg agagcttccg tgtcgacagc 2940

cggttgccaa ggatagcctt ccatgaagac gccatgccca acctcaagct tctcgagttc 3000

aagttctacg ccggcccagc aagcaacgat gccatcggca tcaccaacct gaagagcctc 3060

caaaaggtgg tctttcggtg ctcgccatgg tacaagagcg acgcccctgg catcagcgcc 3120

accattgacg tcgtgaagaa agaagccgag gagcatccca accggccgat caccctcctc 3180

atcaatgctg ggtataagga gatatcaact gagtcacacg ggagcagtga aaacattgcg 3240

ggcagcagtg ggatcgatac tgagcctgca caggcacagc atgataatct ccctgctgtt 3300

cgagatgact acaagggaaa agggattctt cttgatggca ggtgtcctac ctgcggccga 3360

gcgactaaaa ttgaagagga aacccaagat cgagtagcag atattgaaat tcaaacagaa 3420

actactagct ag 3432

<210> 167
<211> 1143
<212> PRT
<213> Oryza sativa

<400> 167
   Met Glu Ala Ala Ala Met Ala Val Thr Ala Ala Thr Gly Ala Leu Ala
   1 5 10 15
   Pro Val Leu Val Lys Leu Ala Ala Leu Leu Asp Asp Gly Glu Cys Asn
   20 25 30
   Leu Leu Glu Gly Ser Arg Ser Asp Ala Glu Phe Ile Arg Ser Glu Leu
           35                  40                  45
   Glu Ala Val His Ser Leu Leu Thr Pro Asn Ile Leu Gly Arg Met Gly
       50                  55                  60
   Asp Asp Asp Ala Ala Cys Lys Asp Gly Leu Ile Ala Glu Val Arg Glu
   65                  70                  75                  80
   Leu Ser Tyr Asp Leu Asp Asp Ala Val Asp Asp Phe Leu Glu Leu Asn
                   85                  90                  95
   Phe Glu Gln Arg Arg Ser Ala Ser Pro Phe Gly Glu Leu Lys Ala Arg
               100                 105                 110
   Val Glu Glu Arg Val Ser Asn Arg Phe Ser Asp Trp Lys Leu Pro Ala
           115                 120                 125
   Ala Ser Leu Pro Pro Ser Ser Val His Arg Arg Ala Gly Leu Pro Pro
       130                 135                 140
   Pro Asp Ala Gly Leu Val Gly Met His Lys Arg Lys Glu Glu Leu Ile
   145                 150                 155                 160
   Glu Leu Leu Glu Gln Gly Ser Ser Asp Ala Ser Arg Trp Arg Lys Arg
                   165                 170                 175
   Lys Pro His Val Pro Leu Arg Ile Met Gly Gly Glu Met Gln Lys Ile
               180                 185                 190
   Val Phe Lys Ile Pro Met Val Asp Asp Lys Ser Arg Thr Lys Ala Met
           195                 200                 205
   Ser Leu Val Ala Ser Thr Val Gly Val His Ser Val Ala Ile Ala Gly
       210                 215                 220
   Asp Leu Arg Asp Glu Val Val Val Val Gly Asp Gly Ile Asp Ser Ile
   225                 230                 235                 240
   Asn Leu Val Ser Ala Leu Arg Lys Lys Val Gly His Ala Glu Leu Leu
                   245                 250                 255
   Gln Val Ser Gln Val Lys Glu Asp Val Lys Glu Ile Thr Ala Met Leu
               260                 265                 270
   Ala Pro Val Lys Ser Ile Cys Glu Phe His Glu Val Lys Thr Ile Cys
           275                 280                 285
   Ile Leu Gly Leu Pro Gly Gly Gly Lys Thr Thr Ile Ala Arg Val Leu
       290                 295                 300
   Tyr His Ala Leu Gly Thr Gln Phe Gln Cys Arg Val Phe Ala Ser Ile
   305                 310                 315                 320
   Ser Pro Ser Ser Ser Pro Ser Pro Asn Leu Thr Glu Thr Leu Ala Asp
                   325                 330                 335
   Ile Phe Ala Gln Ala Gln Leu Gly Val Thr Asp Thr Leu Ser Thr Pro
               340                 345                 350
   Tyr Gly Gly Ser Gly Thr Gly Arg Ala Leu Gln Gln His Leu Ile Asp
           355                 360                 365
   Asn Ile Ser Ala Phe Leu Leu Asn Lys Lys Tyr Leu Ile Val Ile Asp
       370                 375                 380
   Asp Ile Trp His Trp Glu Glu Trp Glu Val Ile Arg Lys Ser Ile Pro
   385                 390                 395                 400
   Lys Asn Asp Leu Gly Gly Arg Ile Ile Met Thr Thr Arg Leu Asn Ser
                   405                 410                 415
   Ile Ala Glu Lys Cys His Thr Asp Asp Asn Asp Val Phe Val Tyr Glu
               420                 425                 430
   Val Gly Asp Leu Asp Asn Asn Asp Ala Leu Ser Leu Ser Trp Gly Ile
           435                 440                 445
   Ala Thr Lys Ser Gly Ala Gly Asn Arg Ile Gly Thr Gly Glu Asp Asn
       450                 455                 460
   Ser Cys Tyr Asp Ile Val Asn Met Cys Tyr Gly Met Pro Leu Ala Leu
   465                 470                 475                 480
   Ile Trp Leu Ser Ser Ala Leu Val Gly Glu Ile Glu Glu Leu Gly Gly
                   485                 490                 495
   Ala Glu Val Lys Lys Cys Arg Asp Leu Arg His Ile Glu Asp Gly Ile
               500                 505                 510
   Leu Asp Ile Pro Ser Leu Gln Pro Leu Ala Glu Ser Leu Cys Leu Gly
           515                 520                 525
   Tyr Asn His Leu Pro Leu Tyr Leu Arg Thr Leu Leu Leu Tyr Cys Ser
   530 535 540
   Ala Tyr His Trp Ser Asn Arg Ile Glu Arg Gly Arg Leu Val Arg Arg
   545 550 555 560
   Trp Ile Ala Glu Gly Phe Val Ser Glu Glu Lys Glu Ala Glu Gly Tyr
   565 570 575
   Phe Gly Glu Leu Ile Asn Arg Gly Trp Ile Thr Gln His Gly Asp Asn
   580 585 590
   Asn Ser Tyr Asn Tyr Tyr Glu Ile His Pro Val Met Leu Ala Phe Leu
   595 600 605
   Arg Cys Lys Ser Lys Glu Tyr Asn Phe Leu Thr Cys Leu Gly Leu Gly
   610 615 620
   Ser Asp Thr Ser Thr Ser Ala Ser Ser Pro Arg Leu Ile Arg Arg Leu
   625 630 635 640
   Ser Leu Gln Gly Gly Tyr Pro Val Asp Cys Leu Ser Ser Met Ser Met
   645 650 655
   Asp Val Ser His Thr Cys Ser Leu Val Val Leu Gly Asp Val Ala Arg
   660 665 670
   Pro Lys Gly Ile Pro Phe Tyr Met Phe Lys Arg Leu Arg Val Leu Asp
   675 680 685
   Leu Glu Asp Asn Lys Asp Ile Gln Asp Ser His Leu Gln Gly Ile Cys
   690 695 700
   Glu Gln Leu Ser Leu Arg Val Arg Tyr Leu Gly Leu Lys Gly Thr Arg
   705 710 715 720
   Ile Arg Lys Leu Pro Gln Glu Met Arg Lys Leu Lys His Leu Glu Ile
   725 730 735
   Leu Tyr Val Gly Ser Thr Arg Ile Ser Glu Leu Pro Gln Glu Ile Gly
   740 745 750
   Glu Leu Lys His Leu Arg Ile Leu Asp Val Arg Asn Thr Asp Ile Thr
   755 760 765
   Glu Leu Pro Leu Gln Ile Arg Glu Leu Gln His Leu His Thr Leu Asp
   770 775 780
   Val Arg Asn Thr Pro Ile Ser Glu Leu Pro Pro Gln Val Gly Lys Leu
   785 790 795 800
   Gln Asn Leu Lys Ile Met Cys Val Arg Ser Thr Gly Val Arg Glu Leu
   805 810 815
   Pro Lys Glu Ile Gly Glu Leu Asn His Leu Gln Thr Leu Asp Val Arg
   820 825 830
   Asn Thr Arg Val Arg Glu Leu Pro Trp Gln Ala Gly Gln Ile Ser Gln
   835 840 845
   Ser Leu Arg Val Leu Ala Gly Asp Ser Gly Asp Gly Val Arg Leu Pro
   850 855 860
   Glu Gly Val Cys Glu Ala Leu Ile Asn Gly Ile Pro Gly Ala Thr Arg
   865 870 875 880
   Ala Lys Cys Arg Glu Val Leu Ser Ile Ala Ile Ile Asp Arg Phe Gly
   885 890 895
   Pro Pro Leu Val Gly Ile Phe Lys Val Pro Gly Ser His Met Arg Ile
   900 905 910
   Pro Lys Met Ile Lys Asp His Phe Arg Val Leu Ser Cys Leu Asp Ile
   915 920 925
   Arg Leu Cys His Lys Leu Glu Asp Asp Asp Gln Lys Phe Leu Ala Glu
   930 935 940
   Met Pro Asn Leu Gln Thr Leu Val Leu Arg Phe Glu Ala Leu Pro Arg
   945 950 955 960
   Gln Pro Ile Thr Ile Asn Gly Thr Gly Phe Gln Met Leu Glu Ser Phe
   965 970 975
   Arg Val Asp Ser Arg Leu Pro Arg Ile Ala Phe His Glu Asp Ala Met
   980 985 990
   Pro Asn Leu Lys Leu Leu Glu Phe Lys Phe Tyr Ala Gly Pro Ala Ser
   995 1000 1005
   Asn Asp Ala Ile Gly Ile Thr Asn Leu Lys Ser Leu Gln Lys Val Val
   1010 1015 1020
   Phe Arg Cys Ser Pro Trp Tyr Lys Ser Asp Ala Pro Gly Ile Ser Ala
   1025 1030 1035 1040
   Thr Ile Asp Val Val Lys Lys Glu Ala Glu Glu His Pro Asn Arg Pro
                   1045                1050                1055
   Ile Thr Leu Leu Ile Asn Ala Gly Tyr Lys Glu Ile Ser Thr Glu Ser
               1060                1065                1070
   His Gly Ser Ser Glu Asn Ile Ala Gly Ser Ser Gly Ile Asp Thr Glu
           1075                1080                1085
   Pro Ala Gln Ala Gln His Asp Asn Leu Pro Ala Val Arg Asp Asp Tyr
       1090                1095                1100
   Lys Gly Lys Gly Ile Leu Leu Asp Gly Arg Cys Pro Thr Cys Gly Arg
   1105                1110                1115                1120
   Ala Thr Lys Ile Glu Glu Glu Thr Gln Asp Arg Val Ala Asp Ile Glu
                   1125                1130                1135
   Ile Gln Thr Glu Thr Thr Ser
               1140

<210> 168
<211> 6317
<212> DNA
<213> Oryza sativa

<400> 168
atggaggcgg ctgccatggc cgtaaccgca gccacggggg ccttggcgcc cgtgctagtg      60
aagctggccg ctttgctgga cgacggggag tgcaatcttc tggaggggag ccggagcgac     120
gcagagttca tcagatccga gctggaggcc gtgcactctc tcctcacccc aaatatcttg     180
gggaggatgg gggatgacga tgcggcgtgc aaggatggct tgattgcgga ggtccgggag     240
ctgtcctacg acctggatga tgccgtcgac gacttcttgg agctcaattt cgagcagcga     300
agaagcgcaa gccctttcgg tgagctcaag gcaagagttg aggagcgtgt ctccaatcgc     360
ttctctgact ggaagctacc ggcggcgagc cttccgccgt cgtcggtaca ccgtcgagct     420
ggcttgccgc caccagatgc agggctggtg gggatgcaca aacgtaagga agagctcatc     480
gagttgctgg aacaagggag cagtgatgct tcacgatggc gcaagcgaaa acctcatgtc     540
cccctcagga taatggaggg ggaaatggta ccggtggatc tcgattcctc acaaatagct     600
tcgcttcctt ggatggcgct gccaataaat ttgtttactt tttctcttga ttcttcaact     660
tggaagtact tcattttttg ttgcagcaaa aaatcgtgtt caagattccc atggtggacg     720
ataagagccg tacaaaagca atgtcattgg ttgcaagtac ggttggagtg cactcggttg     780
caatcgccgg tgacctaaga gacgaggttg tggtggtcgg tgatggcatt gactccatca     840
atctggtctc tgcgctccgg aagaaggtgg gccatgcgga gttgctgcag gtcagccaag     900
taaaggaaga tgtgaaggag ataacggcga tgcttgcgcc ggtgaaatcc atatgtgaat     960
ttcacgaggt caaaacaatt tgcatccttg gattgccagg gggaggcaaa acaacgattg    1020
cccgagtact atatcatgca ttgggaacgc agttccaatg ccgggttttc gcatcaatct    1080
ctccaagttc cagccccagt cccaatctaa cagagactct tgcagacatt ttcgctcaag    1140
cacaactagg agtaactgat acacttagca caccatatgg tgggagtggg accgggagag    1200
ctcttcaaca acatctcatc gacaacatat cagctttcct cctcaacaaa aagtaagcaa    1260

tatcttatat ctcttatcta gcttgtcctt aatccggctt cccaaattaa agtgaacagg 1320

ctaaatgtcc atataatgtt gggttttttt tttctgtcct cgatgaggta tatatcaaga 1380

tatgccaaac tttttttaat tagcgttact ttattctacc tagtaaatag atacagaaat 1440

ggggcaggcc cctcctttgt gacaggccaa tggttttatg atgtggaaac aactccctta 1500

tcactgaagc tcttgctatt actcgtgatg gtactgatct tgcaaaggct tgcagtattt 1560

gtaaatccac agggagtggt catctttttt ttttaagata atgaatagaa atctggcctc 1620

tatatagaaa gccaaaggtt acacaagtgc atacactcca aatctcaaag ctgagaaaca 1680

cgaaaaacaa acgaccaaaa agaaaagacg ataagattaa gtttcaagct tttcctcctt 1740

gcttcagaaa acagggaaat gcagagctag cccgctcttc ccctgccatg ccctctcact 1800

gccattgcaa ctaccacaaa agctcccctg tttcgccgtg agcgaaggga ggggatggaa 1860

ctagtctgct gctttgttgt cgccaagcac tggtttcatg ctgcccaaac aacctatgcc 1920

ccgctagcga taacgagtct gttgccgcca tgctgttgtc gctggagagc acttgtcgct 1980

gctcagctat gtgttaagta agctactaac aatctctcca ttctcttgta gactgcttat 2040

aaaagattgt acttcaactt tttattaatg aaatagagtt cctgccttac aacaacaaga 2100

acgagagaga gagagagagc caatatcatg agttagcact gtgaagactt cctgactgtt 2160

atgtcttcaa gaacagtata aaagcatttg ccactataaa aatatgatag ccctcaagat 2220

cattaaaaat atgtcatgtt cttttacctt cataacaacc agtatcacaa atatgaaaac 2280

atttaaagga aaatcagtat ttgccattta tgtaaagaaa aactagacta gaactctggg 2340

cttttatttt agtgcacact gtatacatgt gtgttctttg acgtatatac ttgatatttt 2400

ttcaaaaact tttgggggca tatctgtaac ccatgcccca tgcatccaaa gtagtagtga 2460

gacaactttc gtatcaatgg acatgtggca ttaaatttaa tcccaatatt tgttctctcg 2520

aagtcggaac atataattgg acaaaattat ataatttaat tagccaattg agccatatat 2580

acacacacta taagaaactt ctacaaaaca aacattcgct ccttttaggt tttgaccagt 2640

ctatacatga accagccaac ggtgtgctgg tctgactata aatgtttttt tagagagtaa 2700

aggcaccttc cagtacctct ctcgaggata gtaaaaacac ccatgcttta aggttatgaa 2760

caacaaagca tagaacaacc acaagaaaaa aaaaacaact agaacaagtt ctggaactag 2820

ttagggattc gggaagccat tgcttgctgg cttgggacaa gctatgcctg ccattggact 2880

atggtggaga ctgggcataa aggatctcaa tctcttggag attggcctat gtcctaggtg 2940

gccatggctg actgcaagga gatatatttc ttgcaacgga aattttcatt gcaaaagctg 3000

aaaatcgttg cgatatgtac ctattgtgac ataattgaat ccattgggag ccgtagcaat 3060

aaatcccgtg gtaataggct attgcaatga ttttccgatg gttgcataaa aaggtgctat 3120

tacaatggtt ttgcatgcta ttgcaacaac ttgatatgtc ataggtattt attgcaacag 3180

tttgtaccat gctgcaataa gatttcgcaa cacagaccta ttcattaaat agcctatttc 3240

cacacattca tttttgtggc aataattcag tgcgacaagc atgttctata gctataacct 3300

gatgcaacgg gttagacttg ttgcagtaac ttattagcac atttaatttt ttactaaatt 3360

tatattgatt caactatttc aacatcattc aatacagtaa atactggaaa atgcaagcaa 3420

tctccaaaaa ttcaccaata agagaaacga attaaatcat atacacaatt atttcacacc 3480

gtacagtgca cttaagtgta ggaaatataa tgctatatac atttccaacc ctcaatacag 3540

gttctgtaaa taatgaaaag aaacatcaat ttgatttctt gaccggtccc tctgagacac 3600

acagtgcttt cctcagcaac atcgttgccg atctatgtaa cttgtaaact ccttgtgctt 3660

aataaattct ggccagggtt acttcagcct gcatggcgaa aaaaaagaac ttgttaggga 3720

gatcgcaaac aaatgttagc tgccccattt cttctctact gaccataaaa gctctaggat 3780

caggtcatga aaacaatgcc gtcagaccgg aaatactcaa gattcagaac acgactccta 3840

ggaactgcaa gtttaaattt caacaaaagc aaaatacaac cagatgacaa actaatatat 3900

atcagctgac tgttcatctc aagtggactc acatctgtga aattgttagt tctatgcata 3960

tatgctaatg taatactaat atgctattga ggcttattac tcttctcgac ttttataggt 4020

atctcattgt aatcgatgac atttggcatt gggaagaatg ggaagtcatc agaaagtcca 4080

ttcccaagaa tgatctgggt ggtagaataa tcatgactac tcgtcttaat tcaatagctg 4140

agaagtgcca cactgatgac aatgatgttt ttgtctacga agttggggat ctagataata 4200

atgatgcttt gtcgttgtct tgggggatag caacaaagtc tggggcaggc aacaggatcg 4260

gaactggaga ggataattca tgctatgata ttgtgaacat gtgttatggt atgcctttag 4320

cacttatttg gctgtcgtca gcattggttg gagagataga agaattaggt ggtgctgaag 4380

tgaaaaaatg tagggatttg agacacatag aggatggtat tttggacatc ccatccttac 4440

aaccattggc ggagagttta tgccttggtt ataaccatct tcctctttat ctgaggactt 4500

tgttgttgta ctgtagtgca taccattggt ctaacagaat cgaaaggggt cgtctggtca 4560

ggaggtggat tgcggaagga tttgtgtcgg aagagaaaga agcagaaggt tactttggcg 4620

agcttattaa cagaggatgg attacgcagc acggagacaa caacagttat aattactatg 4680

agatccaccc cgtgatgctg gccttcctga gatgcaagtc caaggagtac aattttttaa 4740

catgcttggg tctgggatct gatactagta ctagtgcatc ctccccaagg ttgattcgcc 4800

ggctgtctct tcaggggggg tatccagtgg actgcttgtc aagcatgagt atggatgtgt 4860

cacacacttg cagccttgtc gtccttggtg acgtggcgcg acccaaggga atccccttct 4920

atatgtttaa gcgcttgcga gtgttggacc ttgaagataa taaggatata caggattctc 4980

atctgcaggg catatgtgaa cagttaagcc tcagagtgag gtaccttggt ctcaagggaa 5040

cgcggatccg aaagctccct caggagatga ggaagctgaa gcatttggag attttgtatg 5100

tggggagcac tcggatcagt gaacttccgc aagagattgg agagctgaag catctgcgga 5160

ttctggacgt gagaaacacg gacatcactg agctcccact gcagatacgg gagctgcagc 5220

atctgcacac tctggacgtg aggaacactc caatcagtga gctcccgccg caggttggca 5280

agctgcagaa tctcaagatt atgtgcgtga ggagcactgg ggttagggag ctcccaaagg 5340

agattgggga gctgaatcat ctacagactc tggacgtgag aaacacgagg gtgagagagc 5400

tgccatggca agctggccag atctcccaat cgttgcgcgt gcttgccggt gacagtggcg 5460

atggcgtgcg gttgcccgaa ggcgtctgcg aagctctgat caacggtatt ccaggggcta 5520

cgcgtgcaaa atgcagggag gttctgtcca tcgcgatcat cgatcgtttc ggacctcccc 5580

ttgttgggat attcaaagtt cccggcagtc atatgcgtat cccgaagatg atcaaagacc 5640

acttccgcgt tctttcttgc ctagacatca ggctctgcca caagcttgag gatgatgacc 5700

aaaagttcct cgccgagatg cccaacctgc agacgctcgt gctgaggttc gaggccctac 5760

caagacaacc cataaccatc aacggcacag gcttccagat gctggagagc ttccgtgtcg 5820

acagccggtt gccaaggata gccttccatg aagacgccat gcccaacctc aagcttctcg 5880

agttcaagtt ctacgccggc ccagcaagca acgatgccat cggcatcacc aacctgaaga 5940

gcctccaaaa ggtggtcttt cggtgctcgc catggtacaa gagcgacgcc cctggcatca 6000

gcgccaccat tgacgtcgtg aagaaagaag ccgaggagca tcccaaccgg ccgatcaccc 6060

tcctcatcaa tgctgggtat aaggagatat caactgagtc acacgggagc agtgaaaaca 6120

ttgcgggcag cagtgggatc gatactgagc ctgcacaggc acagcatgat aatctccctg 6180

ctgttcgaga tgactacaag ggaaaaggga ttcttcttga tggcaggtgt cctacctgcg 6240

gccgagcgac taaaattgaa gaggaaaccc aagatcgagt agcagatatt gaaattcaaa 6300

cagaaactac tagctag 6317

<210> 169
<211> 3066
<212> DNA
<213> Artificial Sequence

<220>
<223> cDNA

<400> 169
atggagttgg tggtaggtgc ttccgaagcc accatgaaat ctctcttggg caagctgggc 60

aatcttctag cccaggagta tgctctcatc agcggtatcc gtggtgacat ccagtacatc 120

aatgacgagc ttgccagcat gcaggccttc ctccgtgatc tcagcaacgt gccagagggt 180

cacagtcatg gccaccggat gaaggactgg atgaagcaga tccgagacat cgcctatgat 240

gttgaggact gtatcgatga ctttgcccac cgcctccctc aggattccat cagcgatgcc 300

aaatggtcct tcctactcac aaaaatctat gaactatgga catggtggcc acgtcgtgtg 360

attgcttcca acattgccca actcaaggta cgggcacaac agatcgcaga tcgacgtagt 420

agatacggag tgaacaaccc agaacacctt gacagtagca gcagtgccag gacccgtgct 480

gtcaattacg aaattgctga gtatcaggtc acaagccctc agatcattgg tataaaggag 540

cctgtgggga tgaagacggt catggaggag cttgaggttt ggttaactaa tcctcaagct 600

gaaaatgggc aagctgttct gtccatagtc ggttttggag gtgtgggaaa gactaccatt 660

gccacagcat tgtacagaaa agtcagtgaa aaatttcagt gccgggcatc agtagctgtg 720

tctcagaact atgaccaagg caaagtcctc aatagtattc tgagtcaagt cagcaatcag 780

gagcagggca gcagcacaac aattagtgag aaaaagaacc tcacctcagg cgctaagagc 840

atgttgaaga cagccctgtc actgctcaga ggtaattgta tatgtcagcc agaaaatgat 900

ggaaaccctg ataatacacc aatcaggctg caggaaacaa cggacgatga tcaaaacccc 960

agaaaactgg aacagctcct ggccgaaaag agttatatcc tcttgattga tgacatttgg 1020

tctgccgaaa catgggagag tatcagatcg attttgccta aaaataataa aggcggtaga 1080

ataatagtga ctacaagatt tcaagctgtt ggttcaacat gctcccctct tgaaactgat 1140

cgtttgcata cagttgattt tctcaccgat gacgagtccc aaaacttatt caatacaagt 1200

atttgtgaat caaagataag aaaagatagc aacaaagtag acgagcaagt ccctgaggaa 1260

atatggaaaa tatgtggggg attgcctttg gccatagtca gcatggctgg tcttgtcgcc 1320

tgcaacccaa ggaaagcctg ctgcgattgg agtaaacttt gcaaatcatt atttccagag 1380

caagaaactc ctcttaccct cgatggtgtt acaaggatac tggattgttg ttacaatgat 1440

ttgcctgcgg atctgaagac ttgcttattg tacttgagta tatttccgaa gggttggaaa 1500

attagtagga aacgtttgtc ccggcgatgg atagctgaag gttttgctaa tgagaagcaa 1560

gggttaaccc aggaaagagt tgcagaggca tactttaatc aactcacaag aaggaactta 1620

gtacgtccca tggagcatgg cagcaatggg aaggtaaaaa cgtttcaagt tcatgacatg 1680

gttcttgaat acatcatgtc caaatcaatc gaagagaatt ttattactgt ggttggtgga 1740

cactggcaga tgactgcacc aagcaataaa gtccgtcgac tgtcgatgca aagcagtgga 1800

tccaatcgtg gaagttcaac aaaaggcctg aacttggctc aagtgagatc actgacggtg 1860

tttgggaacc tgaaccatgt gccattccat tcattcaact atgggataat acaggtgctg 1920

gatcttgagg actggaaggg tttgaaagag agacatatga cggagatatg tcaaatgctt 1980

ttactcaagt atttgagcat ccgacgaaca gaaatttcca aaattccctc caagattcag 2040

aaacttgagt acttggaaac tcttgacata agggagacat atgtcaggga cctgcctaag 2100

tcaatagtcc agctaaaacg gatcattagc atacttggag ggaataaaaa cacacggaag 2160

gggctgaggt tgcctcaaga aaaaagtaag aagccaatta aaaacccgtc gcctcaagga 2220

aaaacaaagg agcccgcaaa gaaaggattc ttatcccaag aaaaaggtaa aggcgcaatg 2280

aaagcactcc gtgtactgtc agggattgag attgttgagg aatcatcaga agtagctgca 2340

ggccttcatc agttgacagg gctaaggaag cttgccatat acaagctcaa tataacaaag 2400

ggtggtgata ccttcaaaca attacagtcc tccattgagt accttggcag ctgtggtctg 2460

cagactctgg ccatcaatga tgagaattct gaatttatca actcactggg cgacatgccc 2520

gcgcctccaa gatatcttgt cgcccttgag ctgtctggca agttggagaa gctacccaag 2580

tggatcacca gcatcactac tctcaacaag ctaaccatat ctgtaacagt tcttaggact 2640

gaaactttgg agatcctcca cattttacct tcattgtttt ccctcacctt cgccttttca 2700

cttagtgcag cgaagcagga tcaggacata ataaaggaca tccttgagaa taataaattg 2760

gacagtgatg gggaaatcgt cattccagct gaaggattca agagtcttaa gctgcttcgc 2820

ttctttgcac ctttagtgcc gaagctcagc tttttggaca agaatgcaat gccagcactc 2880

gaaatcattg aaatgcggtt taaagacttc gaaggtctat ttggcatcga aatccttgaa 2940

aatctccgtg aggtgcatct caaagttagt gatggggcag aagcaataac caagttcctt 3000

gtaaatgatt tgaaggttaa tactgagaaa ccaaaagtat ttgttgatgg catcgtcact 3060

gcatga 3066

<210> 170
<211> 1021
<212> PRT
<213> Oryza sativa

<400> 170
Met Glu Leu Val Val Gly Ala Ser Glu Ala Thr Met Lys Ser Leu Leu
1               5                   10                  15
Gly Lys Leu Gly Asn Leu Leu Ala Gln Glu Tyr Ala Leu Ile Ser Gly
            20                  25                  30
Ile Arg Gly Asp Ile Gln Tyr Ile Asn Asp Glu Leu Ala Ser Met Gln
        35                  40                  45
Ala Phe Leu Arg Asp Leu Ser Asn Val Pro Glu Gly His Ser His Gly
    50                  55                  60
His Arg Met Lys Asp Trp Met Lys Gln Ile Arg Asp Ile Ala Tyr Asp
65                  70                  75                  80
Val Glu Asp Cys Ile Asp Asp Phe Ala His Arg Leu Pro Gln Asp Ser
                85                  90                  95
Ile Ser Asp Ala Lys Trp Ser Phe Leu Leu Thr Lys Ile Tyr Glu Leu
            100                 105                 110
Trp Thr Trp Trp Pro Arg Arg Val Ile Ala Ser Asn Ile Ala Gln Leu
        115                 120                 125
Lys Val Arg Ala Gln Gln Ile Ala Asp Arg Arg Ser Arg Tyr Gly Val
    130                 135                 140
Asn Asn Pro Glu His Leu Asp Ser Ser Ser Ser Ala Arg Thr Arg Ala
145                 150                 155                 160
Val Asn Tyr Glu Ile Ala Glu Tyr Gln Val Thr Ser Pro Gln Ile Ile

                165                 170                 175
Gly Ile Lys Glu Pro Val Gly Met Lys Thr Val Met Glu Glu Leu Glu
            180                 185                 190
Val Trp Leu Thr Asn Pro Gln Ala Glu Asn Gly Gln Ala Val Leu Ser
        195                 200                 205
Ile Val Gly Phe Gly Gly Val Gly Lys Thr Thr Ile Ala Thr Ala Leu
    210                 215                 220
Tyr Arg Lys Val Ser Glu Lys Phe Gln Cys Arg Ala Ser Val Ala Val
225                 230                 235                 240
Ser Gln Asn Tyr Asp Gln Gly Lys Val Leu Asn Ser Ile Leu Ser Gln
                245                 250                 255
Val Ser Asn Gln Glu Gln Gly Ser Ser Thr Thr Ile Ser Glu Lys Lys
            260                 265                 270
Asn Leu Thr Ser Gly Ala Lys Ser Met Leu Lys Thr Ala Leu Ser Leu
        275                 280                 285
Leu Arg Gly Asn Cys Ile Cys Gln Pro Glu Asn Asp Gly Asn Pro Asp
    290                 295                 300
Asn Thr Pro Ile Arg Leu Gln Glu Thr Thr Asp Asp Asp Gln Asn Pro
305                 310                 315                 320
Arg Lys Leu Glu Gln Leu Leu Ala Glu Lys Ser Tyr Ile Leu Leu Ile
                325                 330                 335
Asp Asp Ile Trp Ser Ala Glu Thr Trp Glu Ser Ile Arg Ser Ile Leu
            340                 345                 350
Pro Lys Asn Asn Lys Gly Gly Arg Ile Ile Val Thr Thr Arg Phe Gln
        355                 360                 365
Ala Val Gly Ser Thr Cys Ser Pro Leu Glu Thr Asp Arg Leu His Thr
    370                 375                 380
Val Asp Phe Leu Thr Asp Asp Glu Ser Gln Asn Leu Phe Asn Thr Ser
385                 390                 395                 400
Ile Cys Glu Ser Lys Ile Arg Lys Asp Ser Asn Lys Val Asp Glu Gln
                405                 410                 415
Val Pro Glu Glu Ile Trp Lys Ile Cys Gly Gly Leu Pro Leu Ala Ile
            420                 425                 430
Val Ser Met Ala Gly Leu Val Ala Cys Asn Pro Arg Lys Ala Cys Cys
        435                 440                 445
Asp Trp Ser Lys Leu Cys Lys Ser Leu Phe Pro Glu Gln Glu Thr Pro
    450                 455                 460
Leu Thr Leu Asp Gly Val Thr Arg Ile Leu Asp Cys Cys Tyr Asn Asp
465                 470                 475                 480
Leu Pro Ala Asp Leu Lys Thr Cys Leu Leu Tyr Leu Ser Ile Phe Pro
                485                 490                 495
Lys Gly Trp Lys Ile Ser Arg Lys Arg Leu Ser Arg Arg Trp Ile Ala
            500                 505                 510
Glu Gly Phe Ala Asn Glu Lys Gln Gly Leu Thr Gln Glu Arg Val Ala
        515                 520                 525
Glu Ala Tyr Phe Asn Gln Leu Thr Arg Arg Asn Leu Val Arg Pro Met
    530                 535                 540
Glu His Gly Ser Asn Gly Lys Val Lys Thr Phe Gln Val His Asp Met
545                 550                 555                 560
Val Leu Glu Tyr Ile Met Ser Lys Ser Ile Glu Glu Asn Phe Ile Thr
                565                 570                 575
Val Val Gly Gly His Trp Gln Met Thr Ala Pro Ser Asn Lys Val Arg
            580                 585                 590
Arg Leu Ser Met Gln Ser Ser Gly Ser Asn Arg Gly Ser Ser Thr Lys
        595                 600                 605
Gly Leu Asn Leu Ala Gln Val Arg Ser Leu Thr Val Phe Gly Asn Leu
    610                 615                 620
Asn His Val Pro Phe His Ser Phe Asn Tyr Gly Ile Ile Gln Val Leu
625                 630                 635                 640
Asp Leu Glu Asp Trp Lys Gly Leu Lys Glu Arg His Met Thr Glu Ile
                645                 650                 655
Cys Gln Met Leu Leu Leu Lys Tyr Leu Ser Ile Arg Arg Thr Glu Ile
            660                 665                 670

Ser Lys Ile Pro Ser Lys Ile Gln Lys Leu Glu Tyr Leu Glu Thr Leu
        675                 680                 685
Asp Ile Arg Glu Thr Tyr Val Arg Asp Leu Pro Lys Ser Ile Val Gln
    690                 695                 700
Leu Lys Arg Ile Ile Ser Ile Leu Gly Gly Asn Lys Asn Thr Arg Lys
705                 710                 715                 720
Gly Leu Arg Leu Pro Gln Glu Lys Ser Lys Lys Pro Ile Lys Asn Pro
                725                 730                 735
Ser Pro Gln Gly Lys Thr Lys Glu Pro Ala Lys Lys Gly Phe Leu Ser
            740                 745                 750
Gln Glu Lys Gly Lys Gly Ala Met Lys Ala Leu Arg Val Leu Ser Gly
        755                 760                 765
Ile Glu Ile Val Glu Glu Ser Ser Glu Val Ala Ala Gly Leu His Gln
    770                 775                 780
Leu Thr Gly Leu Arg Lys Leu Ala Ile Tyr Lys Leu Asn Ile Thr Lys
785                 790                 795                 800
Gly Gly Asp Thr Phe Lys Gln Leu Gln Ser Ser Ile Glu Tyr Leu Gly
                805                 810                 815
Ser Cys Gly Leu Gln Thr Leu Ala Ile Asn Asp Glu Asn Ser Glu Phe
            820                 825                 830
Ile Asn Ser Leu Gly Asp Met Pro Ala Pro Pro Arg Tyr Leu Val Ala
        835                 840                 845
Leu Glu Leu Ser Gly Lys Leu Glu Lys Leu Pro Lys Trp Ile Thr Ser
    850                 855                 860
Ile Thr Thr Leu Asn Lys Leu Thr Ile Ser Val Thr Val Leu Arg Thr
865                 870                 875                 880
Glu Thr Leu Glu Ile Leu His Ile Leu Pro Ser Leu Phe Ser Leu Thr
                885                 890                 895
Phe Ala Phe Ser Leu Ser Ala Ala Lys Gln Asp Gln Asp Ile Ile Lys
            900                 905                 910
Asp Ile Leu Glu Asn Asn Lys Leu Asp Ser Asp Gly Glu Ile Val Ile
        915                 920                 925
Pro Ala Glu Gly Phe Lys Ser Leu Lys Leu Leu Arg Phe Phe Ala Pro
    930                 935                 940
Leu Val Pro Lys Leu Ser Phe Leu Asp Lys Asn Ala Met Pro Ala Leu
945                 950                 955                 960
Glu Ile Ile Glu Met Arg Phe Lys Asp Phe Glu Gly Leu Phe Gly Ile
                965                 970                 975
Glu Ile Leu Glu Asn Leu Arg Glu Val His Leu Lys Val Ser Asp Gly
            980                 985                 990
Ala Glu Ala Ile Thr Lys Phe Leu Val Asn Asp Leu Lys Val Asn Thr
        995                 1000                1005
Glu Lys Pro Lys Val Phe Val Asp Gly Ile Val Thr Ala
    1010                1015                1020

<210> 171
<211> 3229
<212> DNA
<213> Oryza sativa

<400> 171
atggagttgg tggtaggtgc ttccgaagcc accatgaaat ctctcttggg caagctgggc      60

aatcttctag cccaggagta tgctctcatc agcggtatcc gtggtgacat ccagtacatc     120

aatgacgagc ttgccagcat gcaggccttc ctccgtgatc tcagcaacgt gccagagggt     180

cacagtcatg gccaccggat gaaggactgg atgaagcaga tccgagacat cgcctatgat     240

gttgaggact gtatcgatga ctttgcccac cgcctccctc aggattccat cagcgatgcc     300

aaatggtcct tcctactcac aaaaatctat gaactatgga catggtggcc acgtcgtgtg     360

attgcttcca acattgccca actcaaggta cgggcacaac agatcgcaga tcgacgtagt 420

agatacggag tgaacaaccc agaacacctt gacagtagca gcagtgccag gacccgtgct 480

gtcaattacg aaattgctga gtatcaggtc acaagccctc agatcattgg tataaaggag 540

cctgtgggga tgaagacggt catggaggag cttgaggttt ggttaactaa tcctcaagct 600

gaaaatgggc aagctgttct gtccatagtc ggttttggag gtgtgggaaa gactaccatt 660

gccacagcat tgtacagaaa agtcagtgaa aaatttcagt gccgggcatc agtagctgtg 720

tctcagaact atgaccaagg caaagtcctc aatagtattc tgagtcaagt cagcaatcag 780

gagcagggca gcagcacaac aattagtgag aaaaagaacc tcacctcagg cgctaagagc 840

atgttgaaga cagccctgtc actgctcaga ggtaattgta tatgtcagcc agaaaatgat 900

ggaaaccctg ataatacacc aatcaggctg caggaaacaa cggacgatga tcaaaacccc 960

agaaaactgg aacagctcct ggccgaaaag aggtaccttt ttttgtaaat aaaattgctt 1020

tgcttatctg taaattaact tactcatccc actctaaatc taatgtttat ttccttctat 1080

acacagcaca actccatctt ttgaatgggt tttatttttc tcacttgtgc tcattttttt 1140

ttatcatctc tgcagttata tcctcttgat tgatgacatt tggtctgccg aaacatggga 1200

gagtatcaga tcgattttgc ctaaaaataa taaaggcggt agaataatag tgactacaag 1260

atttcaagct gttggttcaa catgctcccc tcttgaaact gatcgtttgc atacagttga 1320

ttttctcacc gatgacgagt cccaaaactt attcaataca agtatttgtg aatcaaagat 1380

aagaaaagat agcaacaaag tagacgagca agtccctgag gaaatatgga aaatatgtgg 1440

gggattgcct ttggccatag tcagcatggc tggtcttgtc gcctgcaacc caaggaaagc 1500

ctgctgcgat tggagtaaac tttgcaaatc attatttcca gagcaagaaa ctcctcttac 1560

cctcgatggt gttacaagga tactggattg ttgttacaat gatttgcctg cggatctgaa 1620

gacttgctta ttgtacttga gtatatttcc gaagggttgg aaaattagta ggaaacgttt 1680

gtcccggcga tggatagctg aaggttttgc taatgagaag caagggttaa cccaggaaag 1740

agttgcagag gcatacttta atcaactcac aagaaggaac ttagtacgtc ccatggagca 1800

tggcagcaat gggaaggtaa aaacgtttca agttcatgac atggttcttg aatacatcat 1860

gtccaaatca atcgaagaga attttattac tgtggttggt ggacactggc agatgactgc 1920

accaagcaat aaagtccgtc gactgtcgat gcaaagcagt ggatccaatc gtggaagttc 1980

aacaaaaggc ctgaacttgg ctcaagtgag atcactgacg gtgtttggga acctgaacca 2040

tgtgccattc cattcattca actatgggat aatacaggtg ctggatcttg aggactggaa 2100

gggtttgaaa gagagacata tgacggagat atgtcaaatg cttttactca agtatttgag 2160

catccgacga acagaaattt ccaaaattcc ctccaagatt cagaaacttg agtacttgga 2220

aactcttgac ataagggaga catatgtcag ggacctgcct aagtcaatag tccagctaaa 2280

acggatcatt agcatacttg gagggaataa aaacacacgg aaggggctga ggttgcctca 2340

agaaaaaagt aagaagccaa ttaaaaaccc gtcgcctcaa ggaaaaacaa aggagcccgc 2400

aaagaaagga ttcttatccc aagaaaaagg taaaggcgca atgaaagcac tccgtgtact 2460

gtcagggatt gagattgttg aggaatcatc agaagtagct gcaggccttc atcagttgac 2520

agggctaagg aagcttgcca tatacaagct caatataaca aagggtggtg ataccttcaa 2580

acaattacag tcctccattg agtaccttgg cagctgtggt ctgcagactc tggccatcaa 2640

tgatgagaat tctgaattta tcaactcact gggcgacatg cccgcgcctc caagatatct 2700

tgtcgccctt gagctgtctg gcaagttgga gaagctaccc aagtggatca ccagcatcac 2760

tactctcaac aagctaacca tatctgtaac agttcttagg actgaaactt tggagatcct 2820

ccacatttta ccttcattgt tttccctcac cttcgccttt tcacttagtg cagcgaagca 2880

ggatcaggac ataataaagg acatccttga gaataataaa ttggacagtg atggggaaat 2940

cgtcattcca gctgaaggat tcaagagtct taagctgctt cgcttctttg cacctttagt 3000

gccgaagctc agctttttgg acaagaatgc aatgccagca ctcgaaatca ttgaaatgcg 3060

gtttaaagac ttcgaaggtc tatttggcat cgaaatcctt gaaaatctcc gtgaggtgca 3120

tctcaaagtt agtgatgggg cagaagcaat aaccaagttc cttgtaaatg atttgaaggt 3180

taatactgag aaaccaaaag tatttgttga tggcatcgtc actgcatga 3229

<210> 172
<211> 3429
<212> DNA
<213> Artificial Sequence

<220>
<223> cDNA

<400> 172
atggaggcgg ctgccatggc cgtaaccgca gccacggggg ccttggcgcc cgtgctagtg 60

aagctggccg ctttgctgga cgacggggag tgcaatcttc tggaggggag ccggagcgac 120

gcagagttca tcagatccga gctggaggcc gttcattctc tcctcacccc aaatatcttg 180

gggaggatgg gggatgacga tgcggcgtgc aaggatggct tgattgcgga ggtccgggag 240

ctgtcctacg acctggatga tgccgtcgac gacttcttgg agctcaattt cgagcagcga 300

agaagcgcaa gccctttcgg tgagctcaag gcaagagttg aggagcatgt ctccaatcgc 360

ttctctgact ggaagctacc ggcggcgagc cttccgccgt cgtcggtaca ccgccgagct 420

ggcttgccgc caccagatgc agagctggtg gggatggaca aacgtatgga agagctcacc 480

aaattgctgg aacaaggagg caatgatgct tcacgatggc gcaagcgaaa accgcatttc 540

ccgctcagaa aaacagggct aaagcaaaaa atcgtgatca aggttgccat ggagggcaat 600

aattgccgtt caaaagcaat ggctttagtt gcgagcactg gaggagtgga ctcggttgcg 660

ctcgtaggtg atctaagaga caagatagag gtggtcggtt atggcattga ccccatcaag 720

ctgatctccg cgctccggaa gaaggtgggc gatgcggagt tgctgcaggt cagccaagca 780

aagaaagatg tgaaggagac gacgccgatg cttgcgccgg tgaaatccat atgtgaattt 840

cacaaggtca aaacagtttg catccttgga ttgccaggtg gaggcaaaac aacggttgcc 900

agagaattat atgacgcctt gggaacgcac ttcccatgcc gggttttcgt gtcagtctct 960

ccaagttcta gtcccagtcc caatctcaca aagactcttg cagacatttt cgctcaagca 1020

caactaggag taactgatac acttagcaca ccatatggtg ggagtgggac cgggagagct 1080

cttcaacaac atctcatcga caacatatca gctttcctcc tcaacaaaaa gtatctcatt 1140

gtaatcgatg acatttggca ttgggaagaa tgggaagtca tcagaaagtc cattcccaag 1200

aatgatctgg gtggtagaat aatcatgact actcgtctta attcaatagc tgagaagtgc 1260

cacactgatg acaatgatgt ttttgtctac gaagttgggg atctagataa taatgatgct 1320

ttgtcgttgt cttgggggat agcaacaaag tctggggcag gcaacaggat cggaactgga 1380

gaggataatc catgctatga tattgtgaac atgtgttatg gtatgccttt agcacttatt 1440

tggctgtcgt cagcattggt tggagagata gaagaattag gtggtgctga agtgaaaaaa 1500

tgtagggatt tgagacacat agaggatggt attttggaca tcccatcctt acaaccattg 1560

gcggagagtt tatgccttgg ttataaccat cttcctcttt atctgaggac tttgttgttg 1620

tactgtagtg cataccattg gtctaacaga atcgaaaggg gtcgtctggt caggaggtgg 1680

attgcggaag gatttgtgtc ggaagagaaa gaagcagaag gttactttgg cgagcttatt 1740

aacagaggat ggattacgca gcacggagac aacaacagtt ataattacta tgagatccac 1800

cccgtgatgc tggccttcct gagatgcaag tccaaggagt acaatttttt aacatgcttg 1860

ggtctgggat ctgatactag tactagtgca tcctccccaa ggttgattcg ccggctgtct 1920

cttcaggggg ggtatccagt ggactgcttg tcaagcatga gtatggatgt gtcacacact 1980

tgcagccttg tcgtccttgg tgacgtggcg cgacccaagg gaatcccctt ctatatgttt 2040

aagcgcttgc gagtgttgga ccttgaagat aataaggata tacaggattc tcatctgcag 2100

ggcatatgtg aacagttaag cctcagagtg aggtaccttg gtctcaaggg aacgcggatc 2160

cgaaagctcc ctcaggagat gaggaagctg aagcatttgg agattttgta tgtggggagc 2220

actcggatca gtgaacttcc gcaagagatt ggagagctga agcatctgcg gattctggac 2280

gtgagaaaca cggacatcac tgagctccca ctgcagatac gggagctgca gcatctgcac 2340

actctggacg tgaggaacac tccaatcagt gagctcccgc cgcaggttgg caagctgcag 2400

aatctcaaga ttatgtgcgt gaggagcact ggggttaggg agctcccaaa ggagattggg 2460

gagctgaatc atctacagac tctggacgtg agaaacacga gggtgagaga gctgccatgg 2520

caagctggcc agatctccca atcgttgcgc gtgcttgccg gtgacagtgg cgatggcgtg 2580

cggttgcccg aaggcgtctg cgaagctctg atcaacggta ttccaggggc tacgcgtgca 2640

aaatgcaggg aggttctgtc catcgcgatc atcgatcgtt tcggacctcc ccttgttggg 2700

atattcaaag ttcccggcag tcatatgcgt atcccgaaga tgatcaaaga ccacttccgc 2760

gttctttctt gcctagacat caggctctgc cacaagcttg aggatgatga ccaaaagttc 2820

ctcgccgaga tgcccaacct gcagacgctc gtgctgaggt tcgaggccct accaagacaa 2880

cccataacca tcaacggcac aggcttccag atgctggaga gcttccgtgt cgacagccgg 2940

gtgccaagga tagccttcca tgaagacgcc atgcccaacc tcaagcttct cgagttcaag 3000

ttctacgccg gcccagcaag caacgatgcc atcggcatca ccaacctgaa gagcctccaa 3060

aaggtggtct ttcggtgctc gccatggtac aagagcgacg cccctggcat cagcgccacc 3120

attgacgtcg tgaagaaaga agccgaggag catcccaacc ggccgatcac cctcctcatc 3180

aatgctgggt ataaggagat atcaactgag tcacacggga gcagtgaaaa cattgcgggc 3240

agcagtggga tcgatactga gcctgcacag gcacagcatg ataatctccc tgctgttcga 3300

gatgactaca agggaaaagg gattcttctt gatggcaggt gtcctacctg cggccgagcg 3360

actaaaattg aagaggaaac ccaagatcga gtagcagata ttgaaattca aacagaaact 3420

actagctag 3429

<210> 173
<211> 1142
<212> PRT
<213> Oryza sativa

<400> 173
Met Glu Ala Ala Ala Met Ala Val Thr Ala Ala Thr Gly Ala Leu Ala
1               5                   10                  15
Pro Val Leu Val Lys Leu Ala Ala Leu Leu Asp Asp Gly Glu Cys Asn
            20                  25                  30
Leu Leu Glu Gly Ser Arg Ser Asp Ala Glu Phe Ile Arg Ser Glu Leu
        35                  40                  45
Glu Ala Val His Ser Leu Leu Thr Pro Asn Ile Leu Gly Arg Met Gly
    50                  55                  60
Asp Asp Asp Ala Ala Cys Lys Asp Gly Leu Ile Ala Glu Val Arg Glu
65                  70                  75                  80
Leu Ser Tyr Asp Leu Asp Asp Ala Val Asp Asp Phe Leu Glu Leu Asn
                85                  90                  95
Phe Glu Gln Arg Arg Ser Ala Ser Pro Phe Gly Glu Leu Lys Ala Arg
            100                 105                 110
Val Glu Glu His Val Ser Asn Arg Phe Ser Asp Trp Lys Leu Pro Ala
        115                 120                 125
Ala Ser Leu Pro Pro Ser Ser Val His Arg Arg Ala Gly Leu Pro Pro
    130                 135                 140
Pro Asp Ala Glu Leu Val Gly Met Asp Lys Arg Met Glu Glu Leu Thr
145                 150                 155                 160

Lys Leu Leu Glu Gln Gly Ser Asn Asp Ala Ser Arg Trp Arg Lys Arg
                165                 170                 175
Lys Pro His Phe Pro Leu Arg Lys Thr Gly Leu Lys Gln Lys Ile Val
            180                 185                 190
Ile Lys Val Ala Met Glu Gly Asn Asn Cys Arg Ser Lys Ala Met Ala
        195                 200                 205
Leu Val Ala Ser Thr Gly Gly Val Asp Ser Val Ala Leu Val Gly Asp
    210                 215                 220
Leu Arg Asp Lys Ile Glu Val Val Gly Tyr Gly Ile Asp Pro Ile Lys
225                 230                 235                 240
Leu Ile Ser Ala Leu Arg Lys Lys Val Gly Asp Ala Glu Leu Leu Gln
                245                 250                 255
Val Ser Gln Ala Lys Lys Asp Val Lys Glu Thr Thr Pro Met Leu Ala
            260                 265                 270
Pro Val Lys Ser Ile Cys Glu Phe His Lys Val Lys Thr Val Cys Ile
        275                 280                 285
Leu Gly Leu Pro Gly Gly Gly Lys Thr Thr Val Ala Arg Glu Leu Tyr
    290                 295                 300
Asp Ala Leu Gly Thr His Phe Pro Cys Arg Val Phe Val Ser Val Ser
305                 310                 315                 320
Pro Ser Ser Ser Pro Ser Pro Asn Leu Thr Lys Thr Leu Ala Asp Ile
                325                 330                 335
Phe Ala Gln Ala Gln Leu Gly Val Thr Asp Thr Leu Ser Thr Pro Tyr
            340                 345                 350
Gly Gly Ser Gly Thr Gly Arg Ala Leu Gln Gln His Leu Ile Asp Asn
        355                 360                 365
Ile Ser Ala Phe Leu Leu Asn Lys Lys Tyr Leu Ile Val Ile Asp Asp
    370                 375                 380
Ile Trp His Trp Glu Glu Trp Glu Val Ile Arg Lys Ser Ile Pro Lys
385                 390                 395                 400
Asn Asp Leu Gly Gly Arg Ile Ile Met Thr Thr Arg Leu Asn Ser Ile
                405                 410                 415
Ala Glu Lys Cys His Thr Asp Asp Asn Asp Val Phe Val Tyr Glu Val
            420                 425                 430
Gly Asp Leu Asp Asn Asn Asp Ala Leu Ser Leu Ser Trp Gly Ile Ala
        435                 440                 445
Thr Lys Ser Gly Ala Gly Asn Arg Ile Gly Thr Gly Glu Asp Asn Pro
    450                 455                 460
Cys Tyr Asp Ile Val Asn Met Cys Tyr Gly Met Pro Leu Ala Leu Ile
465                 470                 475                 480
Trp Leu Ser Ser Ala Leu Val Gly Glu Ile Glu Glu Leu Gly Gly Ala
                485                 490                 495
Glu Val Lys Lys Cys Arg Asp Leu Arg His Ile Glu Asp Gly Ile Leu
            500                 505                 510
Asp Ile Pro Ser Leu Gln Pro Leu Ala Glu Ser Leu Cys Leu Gly Tyr
        515                 520                 525
Asn His Leu Pro Leu Tyr Leu Arg Thr Leu Leu Leu Tyr Cys Ser Ala
    530                 535                 540
Tyr His Trp Ser Asn Arg Ile Glu Arg Gly Arg Leu Val Arg Arg Trp
545                 550                 555                 560
Ile Ala Glu Gly Phe Val Ser Glu Glu Lys Glu Ala Glu Gly Tyr Phe
                565                 570                 575
Gly Glu Leu Ile Asn Arg Gly Trp Ile Thr Gln His Gly Asp Asn Asn
            580                 585                 590
Ser Tyr Asn Tyr Tyr Glu Ile His Pro Val Met Leu Ala Phe Leu Arg
        595                 600                 605
Cys Lys Ser Lys Glu Tyr Asn Phe Leu Thr Cys Leu Gly Leu Gly Ser
    610                 615                 620
Asp Thr Ser Thr Ser Ala Ser Ser Pro Arg Leu Ile Arg Arg Leu Ser
625                 630                 635                 640
Leu Gln Gly Gly Tyr Pro Val Asp Cys Leu Ser Ser Met Ser Met Asp
                645                 650                 655
Val Ser His Thr Cys Ser Leu Val Val Leu Gly Asp Val Ala Arg Pro

660                 665                 670
Lys Gly Ile Pro Phe Tyr Met Phe Lys Arg Leu Arg Val Leu Asp Leu
        675                 680                 685
Glu Asp Asn Lys Asp Ile Gln Asp Ser His Leu Gln Gly Ile Cys Glu
    690                 695                 700
Gln Leu Ser Leu Arg Val Arg Tyr Leu Gly Leu Lys Gly Thr Arg Ile
705                 710                 715                 720
Arg Lys Leu Pro Gln Glu Met Arg Lys Leu Lys His Leu Glu Ile Leu
                725                 730                 735
Tyr Val Gly Ser Thr Arg Ile Ser Glu Leu Pro Gln Glu Ile Gly Glu
            740                 745                 750
Leu Lys His Leu Arg Ile Leu Asp Val Arg Asn Thr Asp Ile Thr Glu
        755                 760                 765
Leu Pro Leu Gln Ile Arg Glu Leu Gln His Leu His Thr Leu Asp Val
    770                 775                 780
Arg Asn Thr Pro Ile Ser Glu Leu Pro Pro Gln Val Gly Lys Leu Gln
785                 790                 795                 800
Asn Leu Lys Ile Met Cys Val Arg Ser Thr Gly Val Arg Glu Leu Pro
                805                 810                 815
Lys Glu Ile Gly Glu Leu Asn His Leu Gln Thr Leu Asp Val Arg Asn
            820                 825                 830
Thr Arg Val Arg Glu Leu Pro Trp Gln Ala Gly Gln Ile Ser Gln Ser
        835                 840                 845
Leu Arg Val Leu Ala Gly Asp Ser Gly Asp Gly Val Arg Leu Pro Glu
    850                 855                 860
Gly Val Cys Glu Ala Leu Ile Asn Gly Ile Pro Gly Ala Thr Arg Ala
865                 870                 875                 880
Lys Cys Arg Glu Val Leu Ser Ile Ala Ile Ile Asp Arg Phe Gly Pro
                885                 890                 895
Pro Leu Val Gly Ile Phe Lys Val Pro Gly Ser His Met Arg Ile Pro
            900                 905                 910
Lys Met Ile Lys Asp His Phe Arg Val Leu Ser Cys Leu Asp Ile Arg
        915                 920                 925
Leu Cys His Lys Leu Glu Asp Asp Asp Gln Lys Phe Leu Ala Glu Met
    930                 935                 940
Pro Asn Leu Gln Thr Leu Val Leu Arg Phe Glu Ala Leu Pro Arg Gln
945                 950                 955                 960
Pro Ile Thr Ile Asn Gly Thr Gly Phe Gln Met Leu Glu Ser Phe Arg
                965                 970                 975
Val Asp Ser Arg Val Pro Arg Ile Ala Phe His Glu Asp Ala Met Pro
            980                 985                 990
Asn Leu Lys Leu Leu Glu Phe Lys Phe Tyr Ala Gly Pro Ala Ser Asn
        995                 1000                1005
Asp Ala Ile Gly Ile Thr Asn Leu Lys Ser Leu Gln Lys Val Val Phe
    1010                1015                1020
Arg Cys Ser Pro Trp Tyr Lys Ser Asp Ala Pro Gly Ile Ser Ala Thr
1025                1030                1035                1040
Ile Asp Val Val Lys Lys Glu Ala Glu Glu His Pro Asn Arg Pro Ile
                1045                1050                1055
Thr Leu Leu Ile Asn Ala Gly Tyr Lys Glu Ile Ser Thr Glu Ser His
            1060                1065                1070
Gly Ser Ser Glu Asn Ile Ala Gly Ser Ser Gly Ile Asp Thr Glu Pro
        1075                1080                1085
Ala Gln Ala Gln His Asp Asn Leu Pro Ala Val Arg Asp Asp Tyr Lys
    1090                1095                1100
Gly Lys Gly Ile Leu Leu Asp Gly Arg Cys Pro Thr Cys Gly Arg Ala
1105                1110                1115                1120
Thr Lys Ile Glu Glu Glu Thr Gln Asp Arg Val Ala Asp Ile Glu Ile
                1125                1130                1135
Gln Thr Glu Thr Thr Ser
            1140

<210> 174

<211> 6348
<212> DNA
<213> Oryza sativa

<400> 174
atggaggcgg ctgccatggc cgtaaccgca gccacggggg ccttggcgcc cgtgctagtg 60
aagctggccg ctttgctgga cgacggggag tgcaatcttc tggaggggag ccggagcgac 120
gcagagttca tcagatccga gctggaggcc gttcattctc tcctcacccc aaatatcttg 180
gggaggatgg gggatgacga tgcggcgtgc aaggatggct tgattgcgga ggtccgggag 240
ctgtcctacg acctggatga tgccgtcgac gacttcttgg agctcaattt cgagcagcga 300
agaagcgcaa gccctttcgg tgagctcaag gcaagagttg aggagcatgt ctccaatcgc 360
ttctctgact ggaagctacc ggcggcgagc cttccgccgt cgtcggtaca ccgccgagct 420
ggcttgccgc caccagatgc agagctggtg ggatggacaa aacgtatgga agagctcacc 480
aaattgctgg aacaagggag caatgatgct tcacgatggc gcaagcgaaa accgcatttc 540
ccgctcagaa aaacagggct aaaggtacgg ttggatctcg attcttcaca aatatagctt 600
cctccgatag cagtgccaat gaatttgttt acttctctct tgattcttta atttggaagt 660
actgtataac aaacatggag ggatcgtcat ttaacttaat tttttttgtt gcagcaaaaa 720
atcgtgatca aggttgccat ggagggcaat aattgccgtt caaaagcaat ggctttagtt 780
gcgagcactg gaggagtgga ctcggttgcg ctcgtaggtg atctaagaga caagatagag 840
gtggtcggtt atggcattga ccccatcaag ctgatctccg cgctccggaa gaaggtgggc 900
gatgcggagt tgctgcaggt cagccaagca aagaaagatg tgaaggagac gacgccgatg 960
cttgcgccgg tgaaatccat atgtgaattt cacaaggtca aaacagtttg catccttgga 1020
ttgccaggtg gaggcaaaac aacggttgcc agagaattat atgacgcctt gggaacgcac 1080
ttcccatgcc gggttttcgt gtcagtctct ccaagttcta gtcccagtcc caatctcaca 1140
aagactcttg cagacatttt cgctcaagca caactaggag taactgatac acttagcaca 1200
ccatatggtg ggagtgggac cgggagagct cttcaacaac atctcatcga caacatatca 1260
gctttcctcc tcaacaaaaa gtaagcaata tcttatatct cttatctagc ttgtccttaa 1320
tccggcttcc caaattaaag tgaacaggct atatgtccat ataatgttgg gttttttttt 1380
tctgtcctcg atgaggtata tatcaagata tgccaaactt tttttaatta gcgttacttt 1440
attctaccta gtaaatagat acagaaatgg ggcaggcccc tcctttgtga caggccaatg 1500
gttttatgat gtggaaacaa ctcccttatc actgaagctc ttgctattac tcgtgatggt 1560
actgatcttg caaaggcttg cagtatttgt aaatccacag ggagtggtca tctttttttt 1620
tttaagataa tgaatagaaa tctggcctct atatagaaag ccaaaggtta cacaagtgca 1680
tacactccaa atctcaaagc tgagaaacac gaaaaacaaa cgaccaaaaa gaaaagacga 1740

taagattaag tttcaagctt ttcctccttg cttcagaaaa cagggaaatg cagagctagc 1800

ccgctcttcc cctgccatgc cctctcactg ccattgcaac taccacaaaa gctcccctgt 1860

ttcgccgtga gcgaagggag gggatggaac tagtctgctg ctttgttgtc gccaagcact 1920

ggtttcatgc tgcccaaaca acctatgccc cgctagcgat aacgagtctg ttgccgccat 1980

gctgttgtcg ctggagagca cttgtcgctg ctcagctatg tgttaagtaa gctactaaca 2040

atctctccat tctcttgtag actgcttata aaagattgta cttcaacttt ttattaatga 2100

aatagagttc ctgccttaca acaacaagaa cgagagagag agagagagcc aatatcacga 2160

gttagcactg tgaagacttc ctgactgtta tgtcttcaag aacagtataa aagcatttgc 2220

cactataaaa atatgatagc cctcaagatc attaaaaata tgtcatgttc ttttaccttc 2280

ataacaacca gtatcacaaa tatgaaaaca tttaaaggaa aatcagtatt tgccatttat 2340

gtaaagaaaa actagactag aactctgggc ttttatttta gtgcacactg tatacatgtg 2400

tgttctttga cgtatatact tgatattttt tcaaaaactt ttgggggcat atctgtaacc 2460

catgccccat gcatccaaag tagtagtgag acaactttcg tatcaatgga catgtggcat 2520

taaatttaat cccaatattt gttctctcga agtcctgaac atataattgg acaaaattat 2580

ataatttaat taggccaatt gagccatata tacacacact ataagaaact tctacaaaac 2640

aaacattcgc tccttttagg ttttgaccag tctatacatg aaccagccaa cggtgtgctg 2700

gtctgactat aaatgttttt ttagagagta aaggcacctt ccagtacctc tctcgaggac 2760

agtaaaaaca cccatgcttt aaggttatga acaacaaagc atagaacaac cacaagaaaa 2820

aaaaacaact agaacaagtt ctggaactag ttagggattc gggaagccat tgcttgctgg 2880

cttgggacaa gctatgcctg ccattggact atggtggaga ctgggcataa aggatctcaa 2940

tctcttggag attggcctat gtcctaggtg gccatggctg actgcaagga gatatatttc 3000

ttgcaacgga aattttcatt gcaaaagctg aaaatcgttg cgatatgtac ctattgtgac 3060

ataattgaat ccattgggag ccgtagcaat aaatcccgtg gtaataggct attgcaatga 3120

ttttccgatg gttgcataaa aaggtgctat tacaatggtt ttgcattgct attgcaacaa 3180

cttgatatgt cataggtatt tattgcaaca gtttgtacca tgctgcaata agatttcgca 3240

acacagacct attcattaaa tagcctattt ccacacattc atttttgtgg caataattca 3300

gtgcgacaag catgttctat agctataacc tgatgcaacg ggttagactt gttgcagtaa 3360

cttattagca catttaattt tttactaaat ttatattgat tcaactattt caacatcatt 3420

caatacagta aatactggaa aatgcaagca atctccaaaa attcaccaat aagagaaacg 3480

aattaaatca tatacacaat tatttcacac cgtacagtgc acttaagtgt aggaaatata 3540

atgctatata catttccaac cctcaataca ggttctgtaa ataatgaaaa gaaacatcaa 3600

tttgatttct tgaccggtcc ctctgagaca cacagtgctt tcctcagcaa catcgttgcc 3660
gatctatgta acttgtaaac tccttgtgct taataaattc tggccagggt tacttcagcc 3720
tgcatggcga aaaaaaagaa cttgttaggg agatggcaaa caaatgttag ctgccccatt 3780
tcttctctac tgaccataaa agctctagga tcaggtcatg aaaacaatgc cgtcagaccg 3840
gaaatactca agattcagaa cacgactcct aggaactgca agtttaaatt tcaacaaaag 3900
caaaatacaa ccagatgaca aactaatata tatcagctga ctgttcatct caagtggact 3960
cacatctgtg aaattgttag ttctatgcat atatgctaat gtaatactaa tatgctattg 4020
aggcttatta ctcttctcga cttttatagg tatctcattg taatcgatga catttggcat 4080
tgggaagaat gggaagtcat cagaaagtcc attcccaaga atgatctggg tggtagaata 4140
atcatgacta ctcgtcttaa ttcaatagct gagaagtgcc acactgatga caatgatgtt 4200
tttgtctacg aagttgggga tctagataat aatgatgctt tgtcgttgtc ttgggggata 4260
gcaacaaagt ctggggcagg caacaggatc ggaactggag aggataatcc atgctatgat 4320
attgtgaaca tgtgttatgg tatgccttta gcacttattt ggctgtcgtc agcattggtt 4380
ggagagatag aagaattagg tggtgctgaa gtgaaaaaat gtagggattt gagacacata 4440
gaggatggta ttttggacat cccatcctta caaccattgg cggagagttt atgccttggt 4500
tataaccatc ttcctcttta tctgaggact ttgttgttgt actgtagtgc ataccattgg 4560
tctaacagaa tcgaaagggg tcgtctggtc aggaggtgga ttgcggaagg atttgtgtcg 4620
gaagagaaag aagcagaagg ttactttggc gagcttatta acagaggatg gattacgcag 4680
cacggagaca acaacagtta taattactat gagatccacc ccgtgatgct ggccttcctg 4740
agatgcaagt ccaaggagta caatttttta acatgcttgg gtctgggatc tgatactagt 4800
actagtgcat cctccccaag gttgattcgc cggctgtctc ttcagggggg gtatccagtg 4860
gactgcttgt caagcatgag tatggatgtg tcacacactt gcagccttgt cgtccttggt 4920
gacgtggcgc gacccaaggg aatccccttc tatatgttta agcgcttgcg agtgttggac 4980
cttgaagata ataaggatat acaggattct catctgcagg gcatatgtga acagttaagc 5040
ctcagagtga ggtaccttgg tctcaaggga acgcggatcc gaaagctccc tcaggagatg 5100
aggaagctga agcatttgga gattttgtat gtggggagca ctcggatcag tgaacttccg 5160
caagagattg gagagctgaa gcatctgcgg attctggacg tgagaaacac ggacatcact 5220
gagctcccac tgcagatacg ggagctgcag catctgcaca ctctggacgt gaggaacact 5280
ccaatcagtg agctcccgcc gcaggttggc aagctgcaga atctcaagat tatgtgcgtg 5340
aggagcactg gggttaggga gctcccaaag gagattgggg agctgaatca tctacagact 5400
ctggacgtga gaaacacgag ggtgagagag ctgccatggc aagctggcca gatctcccaa 5460
tcgttgcgcg tgcttgccgg tgacagtggc gatggcgtgc ggttgcccga aggcgtctgc 5520

gaagctctga tcaacggtat tccaggggct acgcgtgcaa aatgcaggga ggttctgtcc 5580

atcgcgatca tcgatcgttt cggacctccc cttgttggga tattcaaagt tcccggcagt 5640

catatgcgta tcccgaagat gatcaaagac cacttccgcg ttctttcttg cctagacatc 5700

aggctctgcc acaagcttga ggatgatgac caaaagttcc tcgccgagat gcccaacctg 5760

cagacgctcg tgctgaggtt cgaggcccta ccaagacaac ccataaccat caacggcaca 5820

ggcttccaga tgctggagag cttccgtgtc gacagccggg tgccaaggat agccttccat 5880

gaagacgcca tgcccaacct caagcttctc gagttcaagt tctacgccgg cccagcaagc 5940

aacgatgcca tcggcatcac caacctgaag agcctccaaa aggtggtctt tcggtgctcg 6000

ccatggtaca agagcgacgc ccctggcatc agcgccacca ttgacgtcgt gaagaaagaa 6060

gccgaggagc atcccaaccg gccgatcacc ctcctcatca atgctgggta taaggagata 6120

tcaactgagt cacacgggag cagtgaaaac attgcgggca gcagtgggat cgatactgag 6180

cctgcacagg cacagcatga taatctccct gctgttcgag atgactacaa gggaaaaggg 6240

attcttcttg atggcaggtg tcctacctgc ggccgagcga ctaaaattga agaggaaacc 6300

caagatcgag tagcagatat tgaaattcaa acagaaacta ctagctag 6348

<210> 175
<211> 3066
<212> DNA
<213> Artificial Sequence

<220>
<223> cDNA

<400> 175
atggagttgg tggtaggtgc ttccgaagcc accatgaaat ctctcttggg caagctgggc 60

aatcttctag cccaggagta tgctctcatc agcggtatcc gtggtgacat ccagtacatc 120

aatgacgagc ttgccagcat gcaggccttc ctccgtgatc tcagcaacgt gccagagggt 180

cacagtcatg gccaccggat gaaggactgg atgaagcaga tccgagacat cgcctatgat 240

gttgaggact gtatcgatga ctttgcccac cgcctccctc aggattccat cagcgatgcc 300

aaatggtcct tcctactcac aaaaatctat gaactatgga catggtggcc acgtcgtgtg 360

attgcttcca acattgccca actcaaggta cgggcacaac agatcgcaga tcgacgtagt 420

agatacggag tgaacaaccc agaacacctt gacagtagca gcagtgccag gacccgtgct 480

gtcaattacg aaattgctga gtatcaggtc acaagccctc agatcattgg tataaaggag 540

cctgtgggga tgaagacggt catggaggag cttgaggttt ggttaactaa tcctcaagct 600

gaaaatgggc aagctgttct gtccatagtc ggttttggag gtgtgggaaa gactaccatt 660

gccacagcat tgtacagaaa agtcagtgat aaatttcagt gccgggcatc agtagctgtg 720

tctcagaact atgaccaagg caaagtcctc aatagtattc tgagtcaagt cagcaatcag 780

gagcagggca gcagcacaac aattagtgag aaaaagaacc tcacctcagg cgctaagagc 840

atgttgaaga cagccctgtc actgctcaga ggtaattgta tatgtcagcc agaaaatgat 900

ggaaaccctg ataatacacc aatcaggctg caggaaacaa cggacgatga tcaaaacccc 960

agaaaactgg aacagctcct ggccgaaaag agttatatcc tcttgattga tgacatttgg 1020

tctgccgaaa catgggagag tatcagatcg attttgccta aaaataataa aggcggtaga 1080

ataatagtga ctacaagatt tcaagctgtt ggttcaacat gctcccctct tgaaactgat 1140

cgtttgcata cagttgattt tctcaccgat gacgagtccc aaaacttatt caatacaagt 1200

atttgtgaat caaagataag aaaagatagc aacaaagtag acgagcaagt ccctgaggaa 1260

atatggaaaa tatgtggggg attgcctttg gccatagtca ccatggctgg tcttgtcgcc 1320

tgcaacccaa ggaaagcctg ctgcgattgg agtaaacttt gcaaatcatt atttccagag 1380

caagaaactc ctcttaccct cgatggtgtt acaaggatac tggattgttg ttacaatgat 1440

ttgcctgcgg atctgaagac ttgcttattg tacttgagta tatttccgaa gggttggaaa 1500

attagtagga aacgtttgtc ccggcgatgg atagctgaag gttttgctaa tgagaagcaa 1560

gggttaaccc aggaaagagt tgcagaggca tactttaatc aactcacaag aaggaactta 1620

gtacgtccca tggagcatgg cagcaatggg aaggtaaaaa cgtttcaagt tcatgacatg 1680

gttcttgaat acatcatgtc caaatcaatc gaagagaatt ttattactgt ggttggtgga 1740

cactggcaga tgactgcacc aagcaataaa gtccgtcgac tgtcgatgca aagcagtgga 1800

tccaatcgtg gaagttcaac aaaaggcctg aacttggctc aagtgagatc actgacggtg 1860

tttgggaacc tgaaccatat gccattccat tcattcaact atgggataat acaggtgctg 1920

gatcttgagg actggaaggg tttgaaagag agacatatga cggagatatg tcaaatgctt 1980

ttactcaagt atttgagcat ccgacgaaca gaaatttcca aaattccctc caagattcag 2040

aaacttgagt acttggaaac tcttgacata agggagacat atgtcaggga cctgcctaag 2100

tcaatagtcc agctaaaacg gatcattagc atacttggag ggaataaaaa cacacggaag 2160

gggctgaggt tgcctcaaga aaaaagtaag aagccaatta aaaacccgtc gcctcaagga 2220

aaaacaaagg agcccgcaaa gaaaggattc ttatcccaag aaaaaggtaa aggcgcaatg 2280

aaagcactcc gtgtactgtc agggattgag attgttgagg aatcatcaga agtagctgca 2340

ggccttcatc agttgacagg gctaaggaag cttgccatat acaagctcaa tataacaaag 2400

ggtggtgata ccttcaaaca attacagtcc tccattgagt accttggcag ctgtggtctg 2460

cagactctgg ccatcaatga tgagaattct gaatttatca actcactggg cgacatgccc 2520

gcgcctccaa gatatcttgt cgcccttgag ctgtctggca agttggagaa gctacccaag 2580

tggatcacca gcatcactac tctcaacaag ctaaccatat ctgtaacagt tcttaggact 2640

gaaactttgg agatcctcca cattttacct tcattgtttt ccctcacctt cgccttttca 2700

cttagtgcag cgaagcagga tcaggacata ataaaggaca tccttgagaa taataaattg 2760

gacagtgatg gggaaatcgt cattccagct gaaggattca agagtcttaa gctgcttcgc 2820

ttctttgcac ctttagtgcc gaagctcagc tttttggaca agaatgcaat gccagcactc 2880

gaaatcattg aaatgcggtt taaagacttc gaaggtctat ttggcatcga aatccttgaa 2940

aatctccgtg aggtgcatct caaagttagt gatggggcag aagcaataac caagttcctt 3000

gtaaatgatt tgaaggttaa tactgagaaa ccaaaagtat ttgttgatgg catcgtcact 3060

gcatga 3066

<210> 176
<211> 3066
<212> PRT
<213> Oryza sativa

<400> 176
   Ala Thr Gly Gly Ala Gly Thr Thr Gly Gly Thr Gly Gly Thr Ala Gly
   1 5 10 15
   Gly Thr Gly Cys Thr Thr Cys Cys Gly Ala Ala Gly Cys Cys Ala Cys
   20 25 30
   Cys Ala Thr Gly Ala Ala Ala Thr Cys Thr Cys Thr Cys Thr Thr Gly
   35 40 45
   Gly Gly Cys Ala Ala Gly Cys Thr Gly Gly Gly Cys Ala Ala Thr Cys
   50 55 60
   Thr Thr Cys Thr Ala Gly Cys Cys Cys Ala Gly Gly Ala Gly Thr Ala
   65 70 75 80
   Thr Gly Cys Thr Cys Thr Cys Ala Thr Cys Ala Gly Cys Gly Gly Thr
   85 90 95
   Ala Thr Cys Cys Gly Thr Gly Gly Thr Gly Ala Cys Ala Thr Cys Cys
   100 105 110
   Ala Gly Thr Ala Cys Ala Thr Cys Ala Ala Thr Gly Ala Cys Gly Ala
   115 120 125
   Gly Cys Thr Thr Gly Cys Cys Ala Gly Cys Ala Thr Gly Cys Ala Gly
   130 135 140
   Gly Cys Cys Thr Thr Cys Cys Thr Cys Cys Gly Thr Gly Ala Thr Cys
   145 150 155 160
   Thr Cys Ala Gly Cys Ala Ala Cys Gly Thr Gly Cys Cys Ala Gly Ala
   165 170 175
   Gly Gly Gly Thr Cys Ala Cys Ala Gly Thr Cys Ala Thr Gly Gly Cys
   180 185 190
   Cys Ala Cys Cys Gly Gly Ala Thr Gly Ala Ala Gly Gly Ala Cys Thr
   195 200 205
   Gly Gly Ala Thr Gly Ala Ala Gly Cys Ala Gly Ala Thr Cys Cys Gly
   210 215 220
   Ala Gly Ala Cys Ala Thr Cys Gly Cys Cys Thr Ala Thr Gly Ala Thr
   225 230 235 240
   Gly Thr Thr Gly Ala Gly Gly Ala Cys Thr Gly Thr Ala Thr Cys Gly
   245 250 255
   Ala Thr Gly Ala Cys Thr Thr Thr Gly Cys Cys Cys Ala Cys Cys Gly
   260 265 270
   Cys Cys Thr Cys Cys Cys Thr Cys Ala Gly Gly Ala Thr Thr Cys Cys
   275 280 285
   Ala Thr Cys Ala Gly Cys Gly Ala Thr Gly Cys Cys Ala Ala Ala Thr
   290                 295                 300
   Gly Gly Thr Cys Cys Thr Thr Cys Cys Thr Ala Cys Thr Cys Ala Cys
   305                 310                 315                 320
   Ala Ala Ala Ala Ala Thr Cys Thr Ala Thr Gly Ala Ala Cys Thr Ala
                   325                 330                 335
   Thr Gly Gly Ala Cys Ala Thr Gly Gly Thr Gly Gly Cys Cys Ala Cys
               340                 345                 350
   Gly Thr Cys Gly Thr Gly Thr Gly Ala Thr Thr Gly Cys Thr Thr Cys
           355                 360                 365
   Cys Ala Ala Cys Ala Thr Thr Gly Cys Cys Cys Ala Ala Cys Thr Cys
       370                 375                 380
   Ala Ala Gly Gly Thr Ala Cys Gly Gly Gly Cys Ala Cys Ala Ala Cys
   385                 390                 395                 400
   Ala Gly Ala Thr Cys Gly Cys Ala Gly Ala Thr Cys Gly Ala Cys Gly
                   405                 410                 415
   Thr Ala Gly Thr Ala Gly Ala Thr Ala Cys Gly Gly Ala Gly Thr Gly
               420                 425                 430
   Ala Ala Cys Ala Ala Cys Cys Cys Ala Gly Ala Ala Cys Ala Cys Cys
           435                 440                 445
   Thr Thr Gly Ala Cys Ala Gly Thr Ala Gly Cys Ala Gly Cys Ala Gly
       450                 455                 460
   Thr Gly Cys Cys Ala Gly Gly Ala Cys Cys Cys Gly Thr Gly Cys Thr
   465                 470                 475                 480
   Gly Thr Cys Ala Ala Thr Thr Ala Cys Gly Ala Ala Ala Thr Thr Gly
                   485                 490                 495
   Cys Thr Gly Ala Gly Thr Ala Thr Cys Ala Gly Gly Thr Cys Ala Cys
               500                 505                 510
   Ala Ala Gly Cys Cys Cys Thr Cys Ala Gly Ala Thr Cys Ala Thr Thr
           515                 520                 525
   Gly Gly Thr Ala Thr Ala Ala Ala Gly Gly Ala Gly Cys Cys Thr Gly
       530                 535                 540
   Thr Gly Gly Gly Gly Ala Thr Gly Ala Ala Gly Ala Cys Gly Gly Thr
   545                 550                 555                 560
   Cys Ala Thr Gly Gly Ala Gly Gly Ala Gly Cys Thr Thr Gly Ala Gly
                   565                 570                 575
   Gly Thr Thr Thr Gly Gly Thr Thr Ala Ala Cys Thr Ala Ala Thr Cys
               580                 585                 590
   Cys Thr Cys Ala Ala Gly Cys Thr Gly Ala Ala Ala Ala Thr Gly Gly
           595                 600                 605
   Gly Cys Ala Ala Gly Cys Thr Gly Thr Thr Cys Thr Gly Thr Cys Cys
       610                 615                 620
   Ala Thr Ala Gly Thr Cys Gly Gly Thr Thr Thr Thr Gly Gly Ala Gly
   625                 630                 635                 640
   Gly Thr Gly Thr Gly Gly Gly Ala Ala Ala Gly Ala Cys Thr Ala Cys
                   645                 650                 655
   Cys Ala Thr Thr Gly Cys Cys Ala Cys Ala Gly Cys Ala Thr Thr Gly
               660                 665                 670
   Thr Ala Cys Ala Gly Ala Ala Ala Ala Gly Thr Cys Ala Gly Thr Gly
           675                 680                 685
   Ala Thr Ala Ala Ala Thr Thr Thr Cys Ala Gly Thr Gly Cys Cys Gly
       690                 695                 700
   Gly Gly Cys Ala Thr Cys Ala Gly Thr Ala Gly Cys Thr Gly Thr Gly
   705                 710                 715                 720
   Thr Cys Thr Cys Ala Gly Ala Ala Cys Thr Ala Thr Gly Ala Cys Cys
                   725                 730                 735
   Ala Ala Gly Gly Cys Ala Ala Ala Gly Thr Cys Cys Thr Cys Ala Ala
               740                 745                 750
   Thr Ala Gly Thr Ala Thr Thr Cys Thr Gly Ala Gly Thr Cys Ala Ala
           755                 760                 765
   Gly Thr Cys Ala Gly Cys Ala Ala Thr Cys Ala Gly Gly Ala Gly Cys
       770                 775                 780
   Ala Gly Gly Gly Cys Ala Gly Cys Ala Gly Cys Ala Cys Ala Ala Cys
   785                 790                 795                 800
   Ala Ala Thr Thr Ala Gly Thr Gly Ala Gly Ala Ala Ala Ala Ala Gly
                   805                 810                 815
   Ala Ala Cys Cys Thr Cys Ala Cys Cys Thr Cys Ala Gly Gly Cys Gly
               820                 825                 830
   Cys Thr Ala Ala Gly Ala Gly Cys Ala Thr Gly Thr Thr Gly Ala Ala
           835                 840                 845
   Gly Ala Cys Ala Gly Cys Cys Cys Thr Gly Thr Cys Ala Cys Thr Gly
       850                 855                 860
   Cys Thr Cys Ala Gly Ala Gly Gly Thr Ala Ala Thr Thr Gly Thr Ala
   865                 870                 875                 880
   Thr Ala Thr Gly Thr Cys Ala Gly Cys Cys Ala Gly Ala Ala Ala Ala
                   885                 890                 895
   Thr Gly Ala Thr Gly Gly Ala Ala Ala Cys Cys Cys Thr Gly Ala Thr
               900                 905                 910
   Ala Ala Thr Ala Cys Ala Cys Cys Ala Ala Thr Cys Ala Gly Gly Cys
           915                 920                 925
   Thr Gly Cys Ala Gly Gly Ala Ala Ala Cys Ala Ala Cys Gly Gly Ala
       930                 935                 940
   Cys Gly Ala Thr Gly Ala Thr Cys Ala Ala Ala Ala Cys Cys Cys Cys
   945                 950                 955                 960
   Ala Gly Ala Ala Ala Ala Cys Thr Gly Gly Ala Ala Cys Ala Gly Cys
                   965                 970                 975
   Thr Cys Cys Thr Gly Gly Cys Cys Gly Ala Ala Ala Ala Gly Ala Gly
               980                 985                 990
   Thr Thr Ala Thr Ala Thr Cys Cys Thr Cys Thr Thr Gly Ala Thr Thr
           995                 1000                1005
   Gly Ala Thr Gly Ala Cys Ala Thr Thr Thr Gly Gly Thr Cys Thr Gly
       1010                1015                1020
   Cys Cys Gly Ala Ala Ala Cys Ala Thr Gly Gly Gly Ala Gly Ala Gly
   1025                1030                1035                1040
   Thr Ala Thr Cys Ala Gly Ala Thr Cys Gly Ala Thr Thr Thr Thr Gly
                   1045                1050                1055
   Cys Cys Thr Ala Ala Ala Ala Ala Thr Ala Ala Thr Ala Ala Ala Gly
               1060                1065                1070
   Gly Cys Gly Gly Thr Ala Gly Ala Ala Thr Ala Ala Thr Ala Gly Thr
           1075                1080                1085
   Gly Ala Cys Thr Ala Cys Ala Ala Gly Ala Thr Thr Thr Cys Ala Ala
       1090                1095                1100
   Gly Cys Thr Gly Thr Thr Gly Gly Thr Thr Cys Ala Ala Cys Ala Thr
   1105                1110                1115                1120
   Gly Cys Thr Cys Cys Cys Cys Thr Cys Thr Thr Gly Ala Ala Ala Cys
                   1125                1130                1135
   Thr Gly Ala Thr Cys Gly Thr Thr Thr Gly Cys Ala Thr Ala Cys Ala
               1140                1145                1150
   Gly Thr Thr Gly Ala Thr Thr Thr Thr Cys Thr Cys Ala Cys Cys Gly
           1155                1160                1165
   Ala Thr Gly Ala Cys Gly Ala Gly Thr Cys Cys Cys Ala Ala Ala Ala
       1170                1175                1180
   Cys Thr Thr Ala Thr Thr Cys Ala Ala Thr Ala Cys Ala Ala Gly Thr
   1185                1190                1195                1200
   Ala Thr Thr Thr Gly Thr Gly Ala Ala Thr Cys Ala Ala Ala Gly Ala
                   1205                1210                1215
   Thr Ala Ala Gly Ala Ala Ala Ala Gly Ala Thr Ala Gly Cys Ala Ala
               1220                1225                1230
   Cys Ala Ala Ala Gly Thr Ala Gly Ala Cys Gly Ala Gly Cys Ala Ala
           1235                1240                1245
   Gly Thr Cys Cys Cys Thr Gly Ala Gly Gly Ala Ala Ala Thr Ala Thr
       1250                1255                1260
   Gly Gly Ala Ala Ala Ala Thr Ala Thr Gly Thr Gly Gly Gly Gly Gly
   1265                1270                1275                1280
   Ala Thr Thr Gly Cys Cys Thr Thr Thr Gly Gly Cys Cys Ala Thr Ala
                   1285                1290                1295
   Gly Thr Cys Ala Cys Cys Ala Thr Gly Gly Cys Thr Gly Gly Thr Cys
   1300                1305                1310
   Thr Thr Gly Thr Cys Gly Cys Cys Thr Gly Cys Ala Ala Cys Cys Cys
           1315                1320                1325
   Ala Ala Gly Gly Ala Ala Ala Gly Cys Cys Thr Gly Cys Thr Gly Cys
       1330                1335                1340
   Gly Ala Thr Thr Gly Gly Ala Gly Thr Ala Ala Ala Cys Thr Thr Thr
   1345                1350                1355                1360
   Gly Cys Ala Ala Ala Thr Cys Ala Thr Thr Ala Thr Thr Thr Cys Cys
                   1365                1370                1375
   Ala Gly Ala Gly Cys Ala Ala Gly Ala Ala Ala Cys Thr Cys Cys Thr
               1380                1385                1390
   Cys Thr Thr Ala Cys Cys Cys Thr Cys Gly Ala Thr Gly Gly Thr Gly
           1395                1400                1405
   Thr Thr Ala Cys Ala Ala Gly Gly Ala Thr Ala Cys Thr Gly Gly Ala
       1410                1415                1420
   Thr Thr Gly Thr Thr Gly Thr Thr Ala Cys Ala Ala Thr Gly Ala Thr
   1425                1430                1435                1440
   Thr Thr Gly Cys Cys Thr Gly Cys Gly Gly Ala Thr Cys Thr Gly Ala
                   1445                1450                1455
   Ala Gly Ala Cys Thr Thr Gly Cys Thr Thr Ala Thr Thr Gly Thr Ala
               1460                1465                1470
   Cys Thr Thr Gly Ala Gly Thr Ala Thr Ala Thr Thr Thr Cys Cys Gly
           1475                1480                1485
   Ala Ala Gly Gly Gly Thr Thr Gly Gly Ala Ala Ala Ala Thr Thr Ala
       1490                1495                1500
   Gly Thr Ala Gly Gly Ala Ala Ala Cys Gly Thr Thr Thr Gly Thr Cys
   1505                1510                1515                1520
   Cys Cys Gly Gly Cys Gly Ala Thr Gly Gly Ala Thr Ala Gly Cys Thr
                   1525                1530                1535
   Gly Ala Ala Gly Gly Thr Thr Thr Thr Gly Cys Thr Ala Ala Thr Gly
               1540                1545                1550
   Ala Gly Ala Ala Gly Cys Ala Ala Gly Gly Gly Thr Thr Ala Ala Cys
           1555                1560                1565
   Cys Cys Ala Gly Gly Ala Ala Ala Gly Ala Gly Thr Thr Gly Cys Ala
       1570                1575                1580
   Gly Ala Gly Gly Cys Ala Thr Ala Cys Thr Thr Thr Ala Ala Thr Cys
   1585                1590                1595                1600
   Ala Ala Cys Thr Cys Ala Cys Ala Ala Gly Ala Ala Gly Gly Ala Ala
                   1605                1610                1615
   Cys Thr Thr Ala Gly Thr Ala Cys Gly Thr Cys Cys Cys Ala Thr Gly
               1620                1625                1630
   Gly Ala Gly Cys Ala Thr Gly Gly Cys Ala Gly Cys Ala Ala Thr Gly
           1635                1640                1645
   Gly Gly Ala Ala Gly Gly Thr Ala Ala Ala Ala Ala Cys Gly Thr Thr
       1650                1655                1660
   Thr Cys Ala Ala Gly Thr Thr Cys Ala Thr Gly Ala Cys Ala Thr Gly
   1665                1670                1675                1680
   Gly Thr Thr Cys Thr Thr Gly Ala Ala Thr Ala Cys Ala Thr Cys Ala
                   1685                1690                1695
   Thr Gly Thr Cys Cys Ala Ala Ala Thr Cys Ala Ala Thr Cys Gly Ala
               1700                1705                1710
   Ala Gly Ala Gly Ala Ala Thr Thr Thr Thr Ala Thr Thr Ala Cys Thr
           1715                1720                1725
   Gly Thr Gly Gly Thr Thr Gly Gly Thr Gly Gly Ala Cys Ala Cys Thr
       1730                1735                1740
   Gly Gly Cys Ala Gly Ala Thr Gly Ala Cys Thr Gly Cys Ala Cys Cys
   1745                1750                1755                1760
   Ala Ala Gly Cys Ala Ala Thr Ala Ala Ala Gly Thr Cys Cys Gly Thr
                   1765                1770                1775
   Cys Gly Ala Cys Thr Gly Thr Cys Gly Ala Thr Gly Cys Ala Ala Ala
               1780                1785                1790
   Gly Cys Ala Gly Thr Gly Gly Ala Thr Cys Cys Ala Ala Thr Cys Gly
           1795                1800                1805
   Thr Gly Gly Ala Ala Gly Thr Thr Cys Ala Ala Cys Ala Ala Ala Ala
       1810                1815                1820
   Gly Gly Cys Cys Thr Gly Ala Ala Cys Thr Thr Gly Gly Cys Thr Cys
   1825                1830                1835                1840
   Ala Ala Gly Thr Gly Ala Gly Ala Thr Cys Ala Cys Thr Gly Ala Cys
                   1845                1850                1855
   Gly Gly Thr Gly Thr Thr Thr Gly Gly Gly Ala Ala Cys Cys Thr Gly
               1860                1865                1870
   Ala Ala Cys Cys Ala Thr Ala Thr Gly Cys Cys Ala Thr Thr Cys Cys
           1875                1880                1885
   Ala Thr Thr Cys Ala Thr Thr Cys Ala Ala Cys Thr Ala Thr Gly Gly
       1890                1895                1900
   Gly Ala Thr Ala Ala Thr Ala Cys Ala Gly Gly Thr Gly Cys Thr Gly
   1905                1910                1915                1920
   Gly Ala Thr Cys Thr Thr Gly Ala Gly Gly Ala Cys Thr Gly Gly Ala
                   1925                1930                1935
   Ala Gly Gly Gly Thr Thr Thr Gly Ala Ala Ala Gly Ala Gly Ala Gly
               1940                1945                1950
   Ala Cys Ala Thr Ala Thr Gly Ala Cys Gly Gly Ala Gly Ala Thr Ala
           1955                1960                1965
   Thr Gly Thr Cys Ala Ala Ala Thr Gly Cys Thr Thr Thr Thr Ala Cys
       1970                1975                1980
   Thr Cys Ala Ala Gly Thr Ala Thr Thr Thr Gly Ala Gly Cys Ala Thr
   1985                1990                1995                2000
   Cys Cys Gly Ala Cys Gly Ala Ala Cys Ala Gly Ala Ala Ala Thr Thr
                   2005                2010                2015
   Thr Cys Cys Ala Ala Ala Ala Thr Thr Cys Cys Cys Thr Cys Cys Ala
               2020                2025                2030
   Ala Gly Ala Thr Thr Cys Ala Gly Ala Ala Ala Cys Thr Thr Gly Ala
           2035                2040                2045
   Gly Thr Ala Cys Thr Thr Gly Gly Ala Ala Ala Cys Thr Cys Thr Thr
       2050                2055                2060
   Gly Ala Cys Ala Thr Ala Ala Gly Gly Gly Ala Gly Ala Cys Ala Thr
   2065                2070                2075                2080
   Ala Thr Gly Thr Cys Ala Gly Gly Gly Ala Cys Cys Thr Gly Cys Cys
                   2085                2090                2095
   Thr Ala Ala Gly Thr Cys Ala Ala Thr Ala Gly Thr Cys Cys Ala Gly
               2100                2105                2110
   Cys Thr Ala Ala Ala Ala Cys Gly Gly Ala Thr Cys Ala Thr Thr Ala
           2115                2120                2125
   Gly Cys Ala Thr Ala Cys Thr Thr Gly Gly Ala Gly Gly Gly Ala Ala
       2130                2135                2140
   Thr Ala Ala Ala Ala Ala Cys Ala Cys Ala Cys Gly Gly Ala Ala Gly
   2145                2150                2155                2160
   Gly Gly Gly Cys Thr Gly Ala Gly Gly Thr Thr Gly Cys Cys Thr Cys
                   2165                2170                2175
   Ala Ala Gly Ala Ala Ala Ala Ala Ala Gly Thr Ala Ala Gly Ala Ala
               2180                2185                2190
   Gly Cys Cys Ala Ala Thr Thr Ala Ala Ala Ala Ala Cys Cys Cys Gly
           2195                2200                2205
   Thr Cys Gly Cys Cys Thr Cys Ala Ala Gly Gly Ala Ala Ala Ala Ala
       2210                2215                2220
   Cys Ala Ala Ala Gly Gly Ala Gly Cys Cys Cys Gly Cys Ala Ala Ala
   2225                2230                2235                2240
   Gly Ala Ala Ala Gly Gly Ala Thr Thr Cys Thr Thr Ala Thr Cys Cys
                   2245                2250                2255
   Cys Ala Ala Gly Ala Ala Ala Ala Ala Gly Gly Thr Ala Ala Ala Gly
               2260                2265                2270
   Gly Cys Gly Cys Ala Ala Thr Gly Ala Ala Ala Gly Cys Ala Cys Thr
           2275                2280                2285
   Cys Cys Gly Thr Gly Thr Ala Cys Thr Gly Thr Cys Ala Gly Gly Gly
       2290                2295                2300
   Ala Thr Thr Gly Ala Gly Ala Thr Thr Gly Thr Thr Gly Ala Gly Gly
   2305                    2310                    2315                    2320
   Ala Ala Thr Cys Ala Thr Cys Ala Gly Ala Ala Gly Thr Ala Gly Cys
                   2325                    2330                    2335
   Thr Gly Cys Ala Gly Gly Cys Cys Thr Thr Cys Ala Thr Cys Ala Gly
               2340                    2345                    2350
   Thr Thr Gly Ala Cys Ala Gly Gly Gly Cys Thr Ala Ala Gly Gly Ala
           2355                    2360                    2365
   Ala Gly Cys Thr Thr Gly Cys Cys Ala Thr Ala Thr Ala Cys Ala Ala
       2370                    2375                    2380
   Gly Cys Thr Cys Ala Ala Thr Ala Thr Ala Ala Cys Ala Ala Ala Gly
   2385                    2390                    2395                    2400
   Gly Gly Thr Gly Gly Thr Gly Ala Thr Ala Cys Cys Thr Thr Cys Ala
                   2405                    2410                    2415
   Ala Ala Cys Ala Ala Thr Thr Ala Cys Ala Gly Thr Cys Cys Thr Cys
               2420                    2425                    2430
   Cys Ala Thr Thr Gly Ala Gly Thr Ala Cys Cys Thr Thr Gly Gly Cys
           2435                    2440                    2445
   Ala Gly Cys Thr Gly Thr Gly Gly Thr Cys Thr Gly Cys Ala Gly Ala
       2450                    2455                    2460
   Cys Thr Cys Thr Gly Gly Cys Cys Ala Thr Cys Ala Ala Thr Gly Ala
   2465                    2470                    2475                    2480
   Thr Gly Ala Gly Ala Ala Thr Thr Cys Thr Gly Ala Ala Thr Thr Thr
                   2485                    2490                    2495
   Ala Thr Cys Ala Ala Cys Thr Cys Ala Cys Thr Gly Gly Gly Cys Gly
               2500                    2505                    2510
   Ala Cys Ala Thr Gly Cys Cys Cys Gly Cys Gly Cys Cys Thr Cys Cys
           2515                    2520                    2525
   Ala Ala Gly Ala Thr Ala Thr Cys Thr Thr Gly Thr Cys Gly Cys Cys
       2530                    2535                    2540
   Cys Thr Thr Gly Ala Gly Cys Thr Gly Thr Cys Thr Gly Gly Cys Ala
   2545                    2550                    2555                    2560
   Ala Gly Thr Thr Gly Gly Ala Gly Ala Ala Gly Cys Thr Ala Cys Cys
                   2565                    2570                    2575
   Cys Ala Ala Gly Thr Gly Gly Ala Thr Cys Ala Cys Cys Ala Gly Cys
               2580                    2585                    2590
   Ala Thr Cys Ala Cys Thr Ala Cys Thr Cys Thr Cys Ala Ala Cys Ala
           2595                    2600                    2605
   Ala Gly Cys Thr Ala Ala Cys Cys Ala Thr Ala Thr Cys Thr Gly Thr
       2610                    2615                    2620
   Ala Ala Cys Ala Gly Thr Thr Cys Thr Thr Ala Gly Gly Ala Cys Thr
   2625                    2630                    2635                    2640
   Gly Ala Ala Ala Cys Thr Thr Thr Gly Gly Ala Gly Ala Thr Cys Cys
                   2645                    2650                    2655
   Thr Cys Cys Ala Cys Ala Thr Thr Thr Thr Ala Cys Cys Thr Thr Cys
               2660                    2665                    2670
   Ala Thr Thr Gly Thr Thr Thr Thr Cys Cys Cys Thr Cys Ala Cys Cys
           2675                    2680                    2685
   Thr Thr Cys Gly Cys Cys Thr Thr Thr Thr Cys Ala Cys Thr Thr Ala
       2690                    2695                    2700
   Gly Thr Gly Cys Ala Gly Cys Gly Ala Ala Gly Cys Ala Gly Gly Ala
   2705                    2710                    2715                    2720
   Thr Cys Ala Gly Gly Ala Cys Ala Thr Ala Ala Thr Ala Ala Ala Gly
                   2725                    2730                    2735
   Gly Ala Cys Ala Thr Cys Cys Thr Thr Gly Ala Gly Ala Ala Thr Ala
               2740                    2745                    2750
   Ala Thr Ala Ala Ala Thr Thr Gly Gly Ala Cys Ala Gly Thr Gly Ala
           2755                    2760                    2765
   Thr Gly Gly Gly Gly Ala Ala Ala Thr Cys Gly Thr Cys Ala Thr Thr
       2770                    2775                    2780
   Cys Cys Ala Gly Cys Thr Gly Ala Ala Gly Gly Ala Thr Thr Cys Ala
   2785                    2790                    2795                    2800
   Ala Gly Ala Gly Thr Cys Thr Thr Ala Ala Gly Cys Thr Gly Cys Thr
                   2805                    2810                    2815
   Thr Cys Gly Cys Thr Thr Cys Thr Thr Thr Gly Cys Ala Cys Cys Thr
               2820                2825                2830
   Thr Thr Ala Gly Thr Gly Cys Cys Gly Ala Ala Gly Cys Thr Cys Ala
           2835                2840                2845
   Gly Cys Thr Thr Thr Thr Thr Gly Gly Ala Cys Ala Ala Gly Ala Ala
       2850                2855                2860
   Thr Gly Cys Ala Ala Thr Gly Cys Cys Ala Gly Cys Ala Cys Thr Cys
   2865                2870                2875                2880
   Gly Ala Ala Ala Thr Cys Ala Thr Thr Gly Ala Ala Ala Thr Gly Cys
                   2885                2890                2895
   Gly Gly Thr Thr Thr Ala Ala Ala Gly Ala Cys Thr Thr Cys Gly Ala
               2900                2905                2910
   Ala Gly Gly Thr Cys Thr Ala Thr Thr Thr Gly Gly Cys Ala Thr Cys
           2915                2920                2925
   Gly Ala Ala Ala Thr Cys Cys Thr Thr Gly Ala Ala Ala Ala Thr Cys
       2930                2935                2940
   Thr Cys Cys Gly Thr Gly Ala Gly Gly Thr Gly Cys Ala Thr Cys Thr
   2945                2950                2955                2960
   Cys Ala Ala Ala Gly Thr Thr Ala Gly Thr Gly Ala Thr Gly Gly Gly
                   2965                2970                2975
   Gly Cys Ala Gly Ala Ala Gly Cys Ala Ala Thr Ala Ala Cys Cys Ala
               2980                2985                2990
   Ala Gly Thr Thr Cys Cys Thr Thr Gly Thr Ala Ala Ala Thr Gly Ala
           2995                3000                3005
   Thr Thr Thr Gly Ala Ala Gly Gly Thr Thr Ala Ala Thr Ala Cys Thr
       3010                3015                3020
   Gly Ala Gly Ala Ala Ala Cys Cys Ala Ala Ala Ala Gly Thr Ala Thr
   3025                3030                3035                3040
   Thr Thr Gly Thr Thr Gly Ala Thr Gly Gly Cys Ala Thr Cys Gly Thr
                   3045                3050                3055
   Cys Ala Cys Thr Gly Cys Ala Thr Gly Ala
               3060                3065

<210> 177
<211> 3230
<212> DNA
<213> Oryza sativa

<400> 177
atggagttgg tggtaggtgc ttccgaagcc accatgaaat ctctcttggg caagctgggc      60

aatcttctag cccaggagta tgctctcatc agcggtatcc gtggtgacat ccagtacatc     120

aatgacgagc ttgccagcat gcaggccttc ctccgtgatc tcagcaacgt gccagagggt     180

cacagtcatg gccaccggat gaaggactgg atgaagcaga tccgagacat cgcctatgat     240

gttgaggact gtatcgatga ctttgcccac cgcctccctc aggattccat cagcgatgcc     300

aaatggtcct tcctactcac aaaaatctat gaactatgga catggtggcc acgtcgtgtg     360

attgcttcca acattgccca actcaaggta cgggcacaac agatcgcaga tcgacgtagt     420

agatacggag tgaacaaccc agaacacctt gacagtagca gcagtgccag gacccgtgct     480

gtcaattacg aaattgctga gtatcaggtc acaagccctc agatcattgg tataaaggag     540

cctgtgggga tgaagacggt catggaggag cttgaggttt ggttaactaa tcctcaagct     600

gaaaatgggc aagctgttct gtccatagtc ggttttggag gtgtgggaaa gactaccatt     660

gccacagcat tgtacagaaa agtcagtgat aaatttcagt gccgggcatc agtagctgtg     720

tctcagaact atgaccaagg caaagtcctc aatagtattc tgagtcaagt cagcaatcag 780

gagcagggca gcagcacaac aattagtgag aaaaagaacc tcacctcagg cgctaagagc 840

atgttgaaga cagccctgtc actgctcaga ggtaattgta tatgtcagcc agaaaatgat 900

ggaaaccctg ataatacacc aatcaggctg caggaaacaa cggacgatga tcaaaacccc 960

agaaaactgg aacagctcct ggccgaaaag aggtaccttt ttttgtaaat aaaattgctt 1020

tgcttatctg taaattaact tactcatccc actctaaatc taatgtttat ttccttctat 1080

acacagcaca actccatctt ttgaatgggt tttatttttc tcacttgtgc tcattttttt 1140

tttatcatct ctgcagttat atcctcttga ttgatgacat ttggtctgcc gaaacatggg 1200

agagtatcag atcgattttg cctaaaaata ataaaggcgg tagaataata gtgactacaa 1260

gatttcaagc tgttggttca acatgctccc ctcttgaaac tgatcgtttg catacagttg 1320

attttctcac cgatgacgag tcccaaaact tattcaatac aagtatttgt gaatcaaaga 1380

taagaaaaga tagcaacaaa gtagacgagc aagtccctga ggaaatatgg aaaatatgtg 1440

ggggattgcc tttggccata gtcaccatgg ctggtcttgt cgcctgcaac ccaaggaaag 1500

cctgctgcga ttggagtaaa ctttgcaaat cattatttcc agagcaagaa actcctctta 1560

ccctcgatgg tgttacaagg atactggatt gttgttacaa tgatttgcct gcggatctga 1620

agacttgctt attgtacttg agtatatttc cgaagggttg gaaaattagt aggaaacgtt 1680

tgtcccggcg atggatagct gaaggttttg ctaatgagaa gcaaggttta acccaggaaa 1740

gagttgcaga ggcatacttt aatcaactca caagaaggaa cttagtacgt cccatggagc 1800

atggcagcaa tgggaaggta aaaacgtttc aagttcatga catggttctt gaatacatca 1860

tgtccaaatc aatcgaagag aattttatta ctgtggttgg tggacactgg cagatgactg 1920

caccaagcaa taaagtccgt cgactgtcga tgcaaagcag tggatccaat cgtggaagtt 1980

caacaaaagg cctgaacttg gctcaagtga gatcactgac ggtgtttggg aacctgaacc 2040

atatgccatt ccattcattc aactatggga taatacaggt gctggatctt gaggactgga 2100

agggtttgaa agagagacat atgacggaga tatgtcaaat gcttttactc aagtatttga 2160

gcatccgacg aacagaaatt tccaaaattc cctccaagat tcagaaactt gagtacttgg 2220

aaactcttga cataagggag acatatgtca gggacctgcc taagtcaata gtccagctaa 2280

aacggatcat tagcatactt ggagggaata aaaacacacg gaaggggctg aggttgcctc 2340

aagaaaaaag taagaagcca attaaaaacc cgtcgcctca aggaaaaaca aaggagcccg 2400

caaagaaagg attcttatcc caagaaaaag gtaaaggcgc aatgaaagca ctccgtgtac 2460

tgtcagggat tgagattgtt gaggaatcat cagaagtagc tgcaggcctt catcagttga 2520

cagggctaag gaagcttgcc atatacaagc tcaatataac aaagggtggt gataccttca 2580

aacaattaca gtcctccatt gagtaccttg gcagctgtgg tctgcagact ctggccatca 2640

atgatgagaa ttctgaattt atcaactcac tgggcgacat gcccgcgcct ccaagatatc 2700

ttgtcgccct tgagctgtct ggcaagttgg agaagctacc caagtggatc accagcatca 2760

ctactctcaa caagctaacc atatctgtaa cagttcttag gactgaaact ttggagatcc 2820

tccacatttt accttcattg ttttccctca ccttcgcctt ttcacttagt gcagcgaagc 2880

aggatcagga cataataaag gacatccttg agaataataa attggacagt gatggggaaa 2940

tcgtcattcc agctgaagga ttcaagagtc ttaagctgct tcgcttcttt gcacctttag 3000

tgccgaagct cagctttttg gacaagaatg caatgccagc actcgaaatc attgaaatgc 3060

ggtttaaaga cttcgaaggt ctatttggca tcgaaatcct tgaaaatctc cgtgaggtgc 3120

atctcaaagt tagtgatggg gcagaagcaa taaccaagtt ccttgtaaat gatttgaagg 3180

ttaatactga gaaaccaaaa gtatttgttg atggcatcgt cactgcatga 3230

<210> 178
<211> 3432
<212> DNA
<213> Artificial Sequence

<220>
<223> cDNA

<400> 178
atggaggcgg ctgccatggc cgtaaccgca gccacggggg ccttggcgcc cgtgctagtg 60

aagctggccg ctttgctgga cgacggggag tgcaatcttc tggaggggag ccggagcgac 120

gcagagttca tcagatccga gctggaggcc gtgcactctc tcctcacccc aaatatcttg 180

gggaggatgg gggatgacga tgcggcgtgc aaggatggct tgattgcgga ggtccgggag 240

ctgtcctacg acctggatga tgccgtcgac gacttcttgg agctcaattt cgagcagcga 300

agaagcgcaa gccctttcgg tgagctcaag gcaagagttg aggagcgtgt ctccaatcgc 360

ttctctgact ggaagctacc ggcggcgagc cttccgccgt cgtcggtaca ccgtcgagct 420

ggcttgccgc caccagatgc agggctggtg gggatgcaca aacgtaagga agagctcatc 480

gagttgctgg aacaagggag cagtgatgct tcacgatggc gcaagcgaaa acctcatgtc 540

cccctcagga taatgggagg ggaaatgcaa aaaatcgtgt tcaagattcc catggtggac 600

gataagagcc gtacaaaagc aatgtcattg gttgcaagta cggttggagt gcactcggtt 660

gcaatcgccg gtgacctaag agaccaggtt gtggtggtcg gtgatggcat tgactccatc 720

aatctggtct ctgcgctccg gaagaaggtg ggccctgcga tgtttctgga ggtcagccaa 780

gtaaaggaag atgtgaagga gataacggcg atgcttgcgc cggtgaaatc catatgtgaa 840

tttcacgagg tcaaaacaat ttgcatcctt ggattgccag ggggaggcaa aacaacgatt 900

gcccgagtac tatatcatgc attgggaacg cagttccaat gccgggtttt cgcatcaatc 960
tctccaagtt ccagccccag tcccaatcta acagagactc ttgcagacat tttcgctcaa 1020
gcacaactag gagtaactga tacacttagc acaccatatg gtgggagtgg gaccgggaga 1080
gctcttcaac aacatctcat cgacaacata tcagctttcc tcctcaacaa aaagtatctc 1140
attgtaatcg atgacatttg gcattgggaa gaatgggaag tcatcagaaa gtccattccc 1200
aagaatgatc tgggtggtag aataatcatg actactcgtc ttaattcaat agctgagaag 1260
tgccacactg atgacaatga tgtttttgtc tacgaagttg gggatctaga taataatgat 1320
gcttggtcgt tgtcttgggg gatagcaaca aagtctgggg caggcaacag gatcggaact 1380
ggagaggata attcatgcta tgatattgtg aacatgtgtt atggtatgcc tttagcactt 1440
atttggctgt cgtcagcatt ggttggagag atagaagaat taggtggtgc tgaagtgaaa 1500
aaatgtaggg atttgagaca catagaggat ggtattttgg acatcccatc cttacaacca 1560
ttggcggaga gtttatgcct tggttataac catcttcctc tttatctgag gactttgttg 1620
ttgtactgta gtgcatacca ttggtctaac agaatcgaaa ggggtcgtct ggtcaggagg 1680
tggattgcgg aaggatttgt gtcggaagag aaagaagcag aaggttactt tggcgagctt 1740
attaacagag gatggattac gcagcacgga gacaacaaca gttataatta ctatgagatc 1800
caccccgtga tgctggcctt cctgagatgc aagtccaagg agtacaattt tttaacatgc 1860
ttgggtctgg gatctgatac tagtactagt gcatcctccc caaggttgat tcgccggctg 1920
tctcttcagg gggggtatcc agtggactgc ttgtcaagca tgagtatgga tgtgtcacac 1980
acttgcagcc ttgtcgtcct tggtgacgtg gcgcgaccca agggaatccc cttctatatg 2040
tttaagcgct tgcgagtgtt ggaccttgaa gataataagg atatacagga ttctcatctg 2100
cagggcatat gtgaacagtt aagcctcaga gtgaggtacc ttggtctcaa gggaacgcgg 2160
atccgaaagc tccctcagga gatgaggaag ctgaagcatt tggagatttt gtatgtgggg 2220
agcactcgga tcagtgaact tccgcaagag attggagagc tgaagcatct gcggattctg 2280
gacgtgagaa acacggacat cactgagctc ccactgcaga tacgggagct gcagcatctg 2340
cacactctgg acgtgaggaa cactccaatc agtgagctcc cgccgcaggt tggcaagctg 2400
cagaatctca agattatgtg cgtgaggagc actggggtta gggagctccc aaaggagatt 2460
ggggagctga atcatctaca gactctggac gtgagaaaca cgagggtgag agagctgcca 2520
tggcaagctg gccagatctc ccaatcgttg cgcgtgcttg ccggtgacag tggcgatggc 2580
gtgcggttgc ccgaaggcgt ctgcgaagct ctgatcaacg gtattccagg ggctacgcgt 2640
gcaaaatgca gggaggttct gtccatcgcg atcatcgatc gtttcggacc tccccttgtt 2700
gggatattca aagttcccgg cagtcatatg cgtatcccga agatgatcaa agaccacttc 2760
cgcgttcttt cttgcctaga catcaggctc tgccacaagc ttgaggatga tgaccaaaag 2820

ttcctcgccg agatgcccaa cctgcagacg ctcgtgctga ggttcgaggc cctaccaaga 2880

caacccataa ccatcaacgg cacaggcttc cagatgctgg agagcttccg tgtcgacagc 2940

cgggtgccaa ggatagcctt ccatgaagac gccatgccca acctcaagct tctcgagttc 3000

aagttctacg ccggcccagc aagcaacgat gccatcggca tcaccaacct gaagagcctc 3060

caaaaggtgg tctttcggtg ctcgccatgg tacaagagcg acgcccctgg catcagcgcc 3120

accattgacg tcgtgaagaa agaagccgag gagcatccca accggccgat caccctcctc 3180

atcaatgctg ggtataagga gatatcaact gagtcacacg ggagcagtga aaacattgcg 3240

ggcagcagtg ggatcgatac tgagcctgca caggcacagc atgataatct ccctgctgtt 3300

cgagatgact acaagggaaa agggattctt cttgatggca ggtgtcctac ctgcggccga 3360

gcgactaaaa ttgaagagga aacccaagat cgagtagcag atattgaaat tcaaacagaa 3420

actactagct ag 3432

<210> 179
<211> 1143
<212> PRT
<213> Oryza sativa

<400> 179
Met Glu Ala Ala Ala Met Ala Val Thr Ala Ala Thr Gly Ala Leu Ala
1               5                   10                  15
Pro Val Leu Val Lys Leu Ala Ala Leu Leu Asp Asp Gly Glu Cys Asn
            20                  25                  30
Leu Leu Glu Gly Ser Arg Ser Asp Ala Glu Phe Ile Arg Ser Glu Leu
        35                  40                  45
Glu Ala Val His Ser Leu Leu Thr Pro Asn Ile Leu Gly Arg Met Gly
    50                  55                  60
Asp Asp Asp Ala Ala Cys Lys Asp Gly Leu Ile Ala Glu Val Arg Glu
65                  70                  75                  80
Leu Ser Tyr Asp Leu Asp Asp Ala Val Asp Asp Phe Leu Glu Leu Asn
                85                  90                  95
Phe Glu Gln Arg Arg Ser Ala Ser Pro Phe Gly Glu Leu Lys Ala Arg
            100                 105                 110
Val Glu Glu Arg Val Ser Asn Arg Phe Ser Asp Trp Lys Leu Pro Ala
        115                 120                 125
Ala Ser Leu Pro Pro Ser Ser Val His Arg Arg Ala Gly Leu Pro Pro
    130                 135                 140
Pro Asp Ala Gly Leu Val Gly Met His Lys Arg Lys Glu Glu Leu Ile
145                 150                 155                 160
Glu Leu Leu Glu Gln Gly Ser Ser Asp Ala Ser Arg Trp Arg Lys Arg
                165                 170                 175
Lys Pro His Val Pro Leu Arg Ile Met Gly Gly Glu Met Gln Lys Ile
            180                 185                 190
Val Phe Lys Ile Pro Met Val Asp Asp Lys Ser Arg Thr Lys Ala Met
        195                 200                 205
Ser Leu Val Ala Ser Thr Val Gly Val His Ser Val Ala Ile Ala Gly
    210                 215                 220
Asp Leu Arg Asp Gln Val Val Val Val Gly Asp Gly Ile Asp Ser Ile
225                 230                 235                 240
Asn Leu Val Ser Ala Leu Arg Lys Lys Val Gly Pro Ala Met Phe Leu
                245                 250                 255

Glu Val Ser Gln Val Lys Glu Asp Val Lys Glu Ile Thr Ala Met Leu
            260                 265                 270
Ala Pro Val Lys Ser Ile Cys Glu Phe His Glu Val Lys Thr Ile Cys
        275                 280                 285
Ile Leu Gly Leu Pro Gly Gly Gly Lys Thr Thr Ile Ala Arg Val Leu
    290                 295                 300
Tyr His Ala Leu Gly Thr Gln Phe Gln Cys Arg Val Phe Ala Ser Ile
305                 310                 315                 320
Ser Pro Ser Ser Ser Pro Ser Pro Asn Leu Thr Glu Thr Leu Ala Asp
                325                 330                 335
Ile Phe Ala Gln Ala Gln Leu Gly Val Thr Asp Thr Leu Ser Thr Pro
            340                 345                 350
Tyr Gly Gly Ser Gly Thr Gly Arg Ala Leu Gln Gln His Leu Ile Asp
        355                 360                 365
Asn Ile Ser Ala Phe Leu Leu Asn Lys Lys Tyr Leu Ile Val Ile Asp
    370                 375                 380
Asp Ile Trp His Trp Glu Glu Trp Glu Val Ile Arg Lys Ser Ile Pro
385                 390                 395                 400
Lys Asn Asp Leu Gly Gly Arg Ile Ile Met Thr Thr Arg Leu Asn Ser
                405                 410                 415
Ile Ala Glu Lys Cys His Thr Asp Asp Asn Asp Val Phe Val Tyr Glu
            420                 425                 430
Val Gly Asp Leu Asp Asn Asn Asp Ala Trp Ser Leu Ser Trp Gly Ile
        435                 440                 445
Ala Thr Lys Ser Gly Ala Gly Asn Arg Ile Gly Thr Gly Glu Asp Asn
    450                 455                 460
Ser Cys Tyr Asp Ile Val Asn Met Cys Tyr Gly Met Pro Leu Ala Leu
465                 470                 475                 480
Ile Trp Leu Ser Ser Ala Leu Val Gly Glu Ile Glu Glu Leu Gly Gly
                485                 490                 495
Ala Glu Val Lys Lys Cys Arg Asp Leu Arg His Ile Glu Asp Gly Ile
            500                 505                 510
Leu Asp Ile Pro Ser Leu Gln Pro Leu Ala Glu Ser Leu Cys Leu Gly
        515                 520                 525
Tyr Asn His Leu Pro Leu Tyr Leu Arg Thr Leu Leu Leu Tyr Cys Ser
    530                 535                 540
Ala Tyr His Trp Ser Asn Arg Ile Glu Arg Gly Arg Leu Val Arg Arg
545                 550                 555                 560
Trp Ile Ala Glu Gly Phe Val Ser Glu Glu Lys Glu Ala Glu Gly Tyr
                565                 570                 575
Phe Gly Glu Leu Ile Asn Arg Gly Trp Ile Thr Gln His Gly Asp Asn
            580                 585                 590
Asn Ser Tyr Asn Tyr Tyr Glu Ile His Pro Val Met Leu Ala Phe Leu
        595                 600                 605
Arg Cys Lys Ser Lys Glu Tyr Asn Phe Leu Thr Cys Leu Gly Leu Gly
    610                 615                 620
Ser Asp Thr Ser Thr Ser Ala Ser Ser Pro Arg Leu Ile Arg Arg Leu
625                 630                 635                 640
Ser Leu Gln Gly Gly Tyr Pro Val Asp Cys Leu Ser Ser Met Ser Met
                645                 650                 655
Asp Val Ser His Thr Cys Ser Leu Val Val Leu Gly Asp Val Ala Arg
            660                 665                 670
Pro Lys Gly Ile Pro Phe Tyr Met Phe Lys Arg Leu Arg Val Leu Asp
        675                 680                 685
Leu Glu Asp Asn Lys Asp Ile Gln Asp Ser His Leu Gln Gly Ile Cys
    690                 695                 700
Glu Gln Leu Ser Leu Arg Val Arg Tyr Leu Gly Leu Lys Gly Thr Arg
705                 710                 715                 720
Ile Arg Lys Leu Pro Gln Glu Met Arg Lys Leu Lys His Leu Glu Ile
                725                 730                 735
Leu Tyr Val Gly Ser Thr Arg Ile Ser Glu Leu Pro Gln Glu Ile Gly
            740                 745                 750
Glu Leu Lys His Leu Arg Ile Leu Asp Val Arg Asn Thr Asp Ile Thr

755                 760                 765
Glu Leu Pro Leu Gln Ile Arg Glu Leu Gln His Leu His Thr Leu Asp
    770                 775                 780
Val Arg Asn Thr Pro Ile Ser Glu Leu Pro Pro Gln Val Gly Lys Leu
785                 790                 795                 800
Gln Asn Leu Lys Ile Met Cys Val Arg Ser Thr Gly Val Arg Glu Leu
                805                 810                 815
Pro Lys Glu Ile Gly Glu Leu Asn His Leu Gln Thr Leu Asp Val Arg
            820                 825                 830
Asn Thr Arg Val Arg Glu Leu Pro Trp Gln Ala Gly Gln Ile Ser Gln
        835                 840                 845
Ser Leu Arg Val Leu Ala Gly Asp Ser Gly Asp Gly Val Arg Leu Pro
    850                 855                 860
Glu Gly Val Cys Glu Ala Leu Ile Asn Gly Ile Pro Gly Ala Thr Arg
865                 870                 875                 880
Ala Lys Cys Arg Glu Val Leu Ser Ile Ala Ile Ile Asp Arg Phe Gly
                885                 890                 895
Pro Pro Leu Val Gly Ile Phe Lys Val Pro Gly Ser His Met Arg Ile
            900                 905                 910
Pro Lys Met Ile Lys Asp His Phe Arg Val Leu Ser Cys Leu Asp Ile
        915                 920                 925
Arg Leu Cys His Lys Leu Glu Asp Asp Asp Gln Lys Phe Leu Ala Glu
    930                 935                 940
Met Pro Asn Leu Gln Thr Leu Val Leu Arg Phe Glu Ala Leu Pro Arg
945                 950                 955                 960
Gln Pro Ile Thr Ile Asn Gly Thr Gly Phe Gln Met Leu Glu Ser Phe
                965                 970                 975
Arg Val Asp Ser Arg Val Pro Arg Ile Ala Phe His Glu Asp Ala Met
            980                 985                 990
Pro Asn Leu Lys Leu Leu Glu Phe Lys Phe Tyr Ala Gly Pro Ala Ser
        995                 1000                1005
Asn Asp Ala Ile Gly Ile Thr Asn Leu Lys Ser Leu Gln Lys Val Val
    1010                1015                1020
Phe Arg Cys Ser Pro Trp Tyr Lys Ser Asp Ala Pro Gly Ile Ser Ala
1025                1030                1035                1040
Thr Ile Asp Val Val Lys Lys Glu Ala Glu Glu His Pro Asn Arg Pro
                1045                1050                1055
Ile Thr Leu Leu Ile Asn Ala Gly Tyr Lys Glu Ile Ser Thr Glu Ser
            1060                1065                1070
His Gly Ser Ser Glu Asn Ile Ala Gly Ser Ser Gly Ile Asp Thr Glu
        1075                1080                1085
Pro Ala Gln Ala Gln His Asp Asn Leu Pro Ala Val Arg Asp Asp Tyr
    1090                1095                1100
Lys Gly Lys Gly Ile Leu Leu Asp Gly Arg Cys Pro Thr Cys Gly Arg
1105                1110                1115                1120
Ala Thr Lys Ile Glu Glu Glu Thr Gln Asp Arg Val Ala Asp Ile Glu
                1125                1130                1135
Ile Gln Thr Glu Thr Thr Ser
            1140

<210> 180
<211> 6319
<212> DNA
<213> Oryza sativa


<400> 180
   atggaggcgg ctgccatggc cgtaaccgca gccacggggg ccttggcgcc cgtgctagtg      60
   aagctggccg ctttgctgga cgacggggag tgcaatcttc tggaggggag ccggagcgac     120
   gcagagttca tcagatccga gctggaggcc gtgcactctc tcctcacccc aaatatcttg     180
   gggaggatgg gggatgacga tgcggcgtgc aaggatggct tgattgcgga ggtccgggag 240
   ctgtcctacg acctggatga tgccgtcgac gacttcttgg agctcaattt cgagcagcga 300
   agaagcgcaa gccctttcgg tgagctcaag gcaagagttg aggagcgtgt ctccaatcgc 360
   ttctctgact ggaagctacc ggcggcgagc cttccgccgt cgtcggtaca ccgtcgagct 420
   ggcttgccgc caccagatgc agggctggtg ggatgcacaa aacgtaagga agagctcatc 480
   gagttgctgg aacaagggag cagtgatgct tcacgatggc gcaagcgaaa acctcatgtc 540
   cccctcagga taatggaggg gaaatggtac cggtggatct cgattcctca acaaatagct 600
   tcgcttcctt ggatggcgct gccaataaat ttgtttactt tttctcttga ttcttcaact 660
   tggaagtact tcattttttg ttgcagcaaa aaatcgtgtt caagattccc atggtggacg 720
   ataagagccg tacaaaagca atgtcattgg ttgcaagtac ggttggagtg cactcggttg 780
   caatcgccgg tgacctaaga gaccaggttg tggtggtcgg tgatggcatt gactccatca 840
   atctggtctc tgcgctccgg aagaaggtgg gccctgcgat gtttctggag gtcagccaag 900
   taaaggaaga tgtgaaggag ataacggcga tgcttgcgcc ggtgaaatcc atatgtgaat 960
   ttcacgaggt caaaacaatt tgcatccttg gattgccagg gggaggcaaa acaacgattg 1020
   cccgagtact atatcatgca ttgggaacgc agttccaatg ccgggttttc gcatcaatct 1080
   ctccaagttc cagccccagt cccaatctaa cagagactct tgcagacatt ttcgctcaag 1140
   cacaactagg agtaactgat acacttagca caccatatgg tgggagtggg accgggagag 1200
   ctcttcaaca acatctcatc gacaacatat cagctttcct cctcaacaaa aagtaagcaa 1260
   tatcttatat ctcttatcta gcttgtcctt aatccggctt cccaaattaa agtgaacagg 1320
   ctatatgtcc atataatgtt gggttttttt tttctgtcct cgatgaggta tatatcaaga 1380
   tatgccaaac tttttttaat tagcattact ttattctacc tagtaaatag atacagaaat 1440
   ggggcaggcc cctcctttgt gacaggccaa tggttttatg atgtggaaac aactccctta 1500
   tcactgaagc tcttgctatt actcgtgatg gtactgatct tgcaaaggct tgcagtattt 1560
   gtaaatccac agggagtggt catctttttt ttttaagata atgaatagaa atctggcctc 1620
   tatatagaaa gccaaaggtt acacaagtgc atacactcca aatctcaaag ctgagaaaca 1680
   cgaaaaacaa acgaccaaaa agaaaagacg ataagattaa gtttcaagct tttcctcctt 1740
   gcttcagaaa acagggaaat gcagagctag cccgctcttc ccctgccatg ccctctcact 1800
   gccattgcaa ctaccacaaa agctcccctg tttcgccgtg agcgaaggga ggggatggaa 1860
   ctagtctgct gctttgttgt cgccaagcac tggtttcatg ctgcccaaac aacctatgcc 1920
   ccgctagcga taacgagtct gttgccgcca tgctgttgtc gctggagagc acttgtcgct 1980
   gctcagctat gtgttaagta agctactaac aatctctcca ttctcttgta gactgcttat 2040
   aaaagattgt acttcaactt tttattaatg aaatagagtt cctgccttac aacaacaaga 2100
   acgagagaga gagagagagc caatatcatg agttagcact gtgaagactt cctgactgtt 2160
   atgtcttcaa gaacagtata aaagcatttg ccactataaa aatatgatag ccctcaagat 2220
   cattaaaaat atgtcatgtt cttttacctt cataacaacc agtatcacaa atatgaaaac 2280
   atttaaagga aaatcagtat ttgccattta tgtaaagaaa aactagacta gaactctggg 2340
   cttttatttt agtgcacact gtatacatgt gtgttctttg acgtatatac ttgatatttt 2400
   ttcaaaaact tttgggggca tatctgtaac ccatgcccca tgcatccaaa gtagtagtga 2460
   gacaactttc gtatcaatgg acatgtggca ttaaatttaa tcccaatatt tgttctctcg 2520
   aagtcctgaa catataattg gacaaaatta tataatttaa ttaggccaat tgagccatat 2580
   atacacacac tataagaaac ttctacaaaa caaacattcg ctccttttag gttttgacca 2640
   gtctatacat gaaccagcca acggtgtgct ggtctgacta taaatgtttt tttagagagt 2700
   aaaggcacct tccagtacct ctctcgagga cagtaaaaac acccatgctt taaggttatg 2760
   aacaacaaag catagaacaa ccacaagaaa aaaaaacaac tagaacaagt tctggaacta 2820
   gttagggatt cgggaagcca ttgcttgctg gcttgggaca agctatgcct gccattggac 2880
   tatggtggag actgggcata aaggatctca atctcttgga gattggccta tgtcctaggt 2940
   ggccatggct gactgcaagg agatatattt cttgcaacgg aaattttcat tgcaaaagct 3000
   gaaaatcgtt gtgatatgta cctattgtga cataattgaa tccattggga gccgtagcaa 3060
   taaatcccgt ggtaataggc tattgcaatg attttccgat ggttgcataa aaaggtgcta 3120
   ttacaatggt tttgcattgc tattgcaaca acttgatatg tcataggtat ttattgcaac 3180
   agtttgtacc atgctgcaat aagatttcgc aacacagacc tattcattaa atagcctatt 3240
   tccacacatt catttttgtg gcaataattc agtgcgacaa gcatgttcta tagctataac 3300
   ctgatgcaac gggttagact tgttgcagta acttattagc acatttaatt ttttactaaa 3360
   tttatattga ttcaactatt tcaacatcat tcaatacagt aaatactgga aaatgcaagc 3420
   aatctccaaa aattcaccaa taagagaaac gaattaaatc atatacacaa ttatttcaca 3480
   ccgtacagtg cacttaagtg taggaaatat aatgctatat acatttccaa ccctcaatac 3540
   aggttctgta aataatgaaa agaaacatca atttgatttc ttgaccggtc cctctgagac 3600
   acacagtgct ttcctcagca acatcgttgc cgatctatgt aacttgtaaa ctccttgtgc 3660
   ttaataaatt ctggccaggg ttacttcagc ctgcatggcg aaaaaaaaga acttgttagg 3720
   gagatcgcaa acaaatgtta gctgccccat ttcttctcta ctgaccataa aagctctagg 3780
   atcaggtcat gaaaacaatg ccgtcagacc ggaaatactc aagattcaga acacgactcc 3840
   taggaactgc aagtttaaat ttcaacaaaa gcaaatacaa accagatgac aaactaatat 3900
   atatcagctg actgttcatc tcaagtggac tcacatctgt gaaattgtta gctctatgca 3960
   tatatgctaa tgtaatacta atatgctatt gaggcttatt actcttctcg acttttatag 4020
   gtatctcatt gtaatcgatg acatttggca ttgggaagaa tgggaagtca tcagaaagtc 4080
   cattcccaag aatgatctgg gtggtagaat aatcatgact actcgtctta attcaatagc 4140
   tgagaagtgc cacactgatg acaatgatgt ttttgtctac gaagttgggg atctagataa 4200
   taatgatgct tggtcgttgt cttgggggat agcaacaaag tctggggcag gcaacaggat 4260
   cggaactgga gaggataatt catgctatga tattgtgaac atgtgttatg gtatgccttt 4320
   agcacttatt tggctgtcgt cagcattggt tggagagata gaagaattag gtggtgctga 4380
   agtgaaaaaa tgtagggatt tgagacacat agaggatggt attttggaca tcccatcctt 4440
   acaaccattg gcggagagtt tatgccttgg ttataaccat cttcctcttt atctgaggac 4500
   tttgttgttg tactgtagtg cataccattg gtctaacaga atcgaaaggg gtcgtctggt 4560
   caggaggtgg attgcggaag gatttgtgtc ggaagagaaa gaagcagaag gttactttgg 4620
   cgagcttatt aacagaggat ggattacgca gcacggagac aacaacagtt ataattacta 4680
   tgagatccac cccgtgatgc tggccttcct gagatgcaag tccaaggagt acaatttttt 4740
   aacatgcttg ggtctgggat ctgatactag tactagtgca tcctccccaa ggttgattcg 4800
   ccggctgtct cttcaggggg ggtatccagt ggactgcttg tcaagcatga gtatggatgt 4860
   gtcacacact tgcagccttg tcgtccttgg tgacgtggcg cgacccaagg gaatcccctt 4920
   ctatatgttt aagcgcttgc gagtgttgga ccttgaagat aataaggata tacaggattc 4980
   tcatctgcag ggcatatgtg aacagttaag cctcagagtg aggtaccttg gtctcaaggg 5040
   aacgcggatc cgaaagctcc ctcaggagat gaggaagctg aagcatttgg agattttgta 5100
   tgtggggagc actcggatca gtgaacttcc gcaagagatt ggagagctga agcatctgcg 5160
   gattctggac gtgagaaaca cggacatcac tgagctccca ctgcagatac gggagctgca 5220
   gcatctgcac actctggacg tgaggaacac tccaatcagt gagctcccgc cgcaggttgg 5280
   caagctgcag aatctcaaga ttatgtgcgt gaggagcact ggggttaggg agctcccaaa 5340
   ggagattggg gagctgaatc atctacagac tctggacgtg agaaacacga gggtgagaga 5400
   gctgccatgg caagctggcc agatctccca atcgttgcgc gtgcttgccg gtgacagtgg 5460
   cgatggcgtg cggttgcccg aaggcgtctg cgaagctctg atcaacggta ttccaggggc 5520
   tacgcgtgca aaatgcaggg aggttctgtc catcgcgatc atcgatcgtt tcggacctcc 5580
   ccttgttggg atattcaaag ttcccggcag tcatatgcgt atcccgaaga tgatcaaaga 5640
   ccacttccgc gttctttctt gcctagacat caggctctgc cacaagcttg aggatgatga 5700
   ccaaaagttc ctcgccgaga tgcccaacct gcagacgctc gtgctgaggt tcgaggccct 5760
   accaagacaa cccataacca tcaacggcac aggcttccag atgctggaga gcttccgtgt 5820
   cgacagccgg gtgccaagga tagccttcca tgaagacgcc atgcccaacc tcaagcttct 5880
   cgagttcaag ttctacgccg gcccagcaag caacgatgcc atcggcatca ccaacctgaa 5940
   gagcctccaa aaggtggtct ttcggtgctc gccatggtac aagagcgacg cccctggcat 6000
   cagcgccacc attgacgtcg tgaagaaaga agccgaggag catcccaacc ggccgatcac 6060
   cctcctcatc aatgctgggt ataaggagat atcaactgag tcacacggga gcagtgaaaa 6120
   cattgcgggc agcagtggga tcgatactga gcctgcacag gcacagcatg ataatctccc 6180
   tgctgttcga gatgactaca agggaaaagg gattcttctt gatggcaggt gtcctacctg 6240
   cggccgagcg actaaaattg aagaggaaac ccaagatcga gtagcagata ttgaaattca 6300
   aacagaaact actagctag 6319

<210> 181
<211> 3066
<212> DNA
<213> Artificial Sequence

<220>
<223> cDNA

<400> 181
atggagttgg tggtaggtgc ttccgaagcc accatgaaat ctctcttggg caagctgggc 60

aatcttctag cccaggagta tgctctcatc agcggtatcc gtggtgacat ccagtacatc 120

aatgacgagc ttgccagcat gcaggccttc ctccgtgatc tcagcaacgt gccagagggt 180

cacagtcatg gccaccggat gaaggactgg atgaagcaga tccgagacat cgcctatgat 240

gttgaggact gtatcgatga ctttgcccac cgcctccctc aggattccat cagcgatgcc 300

aaatggtcct tcctactcac aaaaatctat gaactatgga catggtggcc acgtcgtgtg 360

attgcttcca acattgccca actcaaggta cgggcacaac agatcgcaga tcgacgtagt 420

agatacggag tgaacaaccc agaacacctt gacagtagca gcagtgccag gacccgtgct 480

gtcaattacg aaattgctga gtatcaggtc acaagccctc agatcattgg tataaaggag 540

cctgtgggga tgaagacggt catggaggag cttgaggttt ggttaactaa tcctcaagct 600

gaaaatgggc aagctgttct gtccatagtc ggttttggag gtgtgggaaa gactaccatt 660

gccacagcat tgtacagaaa agtcagtgaa aaatttcagt gccgggcatc agtagctgtg 720

tctcagaact atgaccaagg caaagtcctc aatagtattc tgagtcaagt cagcaatcag 780

gagcagggca gcagcacaac aattagtgag aaaaagaacc tcacctcagg cgctaagagc 840

atgttgaaga cagccctgtc actgctcaga ggtaattgta tatgtcagcc agaaaatgat 900

ggaaaccctg ataatacacc aatcaggctg caggaaacaa cggacgatga tcaaaacccc 960

agaaaactgg aacagctcct ggccgaaaag agttatatcc tcttgattga tgacatttgg 1020

tctgccgaaa catgggagag tatcagatcg attttgccta aaaataataa aggcggtaga 1080

ataatagtga ctacaagatt tcaagctgtt ggttcaacat gctcccctct tgaaactgat 1140

cgtttgcata cagttgattt tctcaccgat gacgagtccc aaaacttatt caatacaagt 1200

atttgtgaat caaagataag aaaagatagc aacaaagtag acgagcaagt ccctgaggaa 1260

atatggaaaa tatgtggggg attgcctttg gccatagtca gcatggctgg tcttgtcgcc 1320

tgcaacccaa ggaaagcctg ctgcgattgg agtaaacttt gcaaatcatt atttccagag 1380

caagaaactc ctcttaccct cgatggtgtt acaaggatac tggattgttg ttacaatgat 1440

ttgcctgcgg atctgaagac ttgcttattg tacttgagta tatttccgaa gggttggaaa 1500

attagtagga aacgtttgtc ccggcgatgg atagctgaag gttttgctaa tgagaagcaa 1560

gggttaaccc aggaaagagt tgcagaggca tactttaatc aactcacaag aaggaactta 1620

gtacgtccca tggagcatgg cagcaatggg aaggtaaaaa cgtttcaagt tcatgacatg 1680

gttcttgaat acatcatgtc caaatcaatc gaagagaatt ttattactgt ggttggtgga 1740

cactggcaga tgactgcacc aagcaataaa gtccgtcgac tgtcgatgca aagcagtgga 1800

tccaatcgtg gaagttcaac aaaaggcctg aacttggctc aagtgagatc actgacggtg 1860

tttgggaacc tgaaccatgt gccattccat tcattcaact atgggataat acaggtgctg 1920

gatcttgagg actggaaggg tttgaaagag agacatatga cggagatatg tcaaatgctt 1980

ttactcaagt atttgagcat ccgacgaaca gaaatttcca aaattccctc caagattcag 2040

aaacttgagt acttggaaac tcttgacata agggagacat atgtcaggga cctgcctaag 2100

tcaatagtcc agctaaaacg gatcattagc atacttggag ggaataaaaa cacacggaag 2160

gggctgaggt tgcctcaaga aaaaagtaag aagccaatta aaaacccgtc gcctcaagga 2220

aaaacaaagg agcccgcaaa gaaaggattc ttatcccaag aaaaaggtaa aggcgcaatg 2280

aaagcactcc gtgtactgtc agggattgag attgttgagg aatcatcaga agtagctgca 2340

ggccttcatc agttgacagg gctaaggaag cttgccatat acaagctcaa tataacaaag 2400

ggtggtgata ccttcaaaca attacagtcc tccattgagt accttggcag ctgtggtctg 2460

cagactctgg ccatcaatga tgagaattct gaatttatca actcactggg cgacatgccc 2520

gcgcctccaa gatatcttgt cgcccttgag ctgtctggca agttggagaa gctacccaag 2580

tggatcacca gcatcactac tctcaacaag ctaaccatat ctgtaacagt tcttaggact 2640

gaaactttgg agatcctcca cattttacct tcattgtttt ccctcacctt cgccttttca 2700

cttagtgcag cgaagcagga tcaggacata ataaaggaca tccttgagaa taataaattg 2760

gacagtgatg gggaaatcgt cattccagct gaaggattca agagtcttaa gctgcttcgc 2820

ttctttgcac ctttagtgcc gaagctcagc tttttggaca agaatgcaat gccagcactc 2880

gaaatcattg aaatgcggtt taaagacttc gaaggtctat ttggcatcga aatccttgaa 2940

aatctccgtg aggtgcatct caaagttagt gatggggcag aagcaataac caagttcctt 3000

gtaaatgatt tgaaggttaa tactgagaaa ccaaaagtat ttgttgatgg catcgtcact 3060

gcatga 3066

<210> 182
<211> 1021
<212> PRT
<213> Oryza sativa

<220>
<223> cDNA

<400> 182
Met Glu Leu Val Val Gly Ala Ser Glu Ala Thr Met Lys Ser Leu Leu
1               5                   10                  15
Gly Lys Leu Gly Asn Leu Leu Ala Gln Glu Tyr Ala Leu Ile Ser Gly
            20                  25                  30
Ile Arg Gly Asp Ile Gln Tyr Ile Asn Asp Glu Leu Ala Ser Met Gln
        35                  40                  45
Ala Phe Leu Arg Asp Leu Ser Asn Val Pro Glu Gly His Ser His Gly
    50                  55                  60
His Arg Met Lys Asp Trp Met Lys Gln Ile Arg Asp Ile Ala Tyr Asp
65                  70                  75                  80
Val Glu Asp Cys Ile Asp Asp Phe Ala His Arg Leu Pro Gln Asp Ser
                85                  90                  95
Ile Ser Asp Ala Lys Trp Ser Phe Leu Leu Thr Lys Ile Tyr Glu Leu
            100                 105                 110
Trp Thr Trp Trp Pro Arg Arg Val Ile Ala Ser Asn Ile Ala Gln Leu
        115                 120                 125
Lys Val Arg Ala Gln Gln Ile Ala Asp Arg Arg Ser Arg Tyr Gly Val
    130                 135                 140
Asn Asn Pro Glu His Leu Asp Ser Ser Ser Ser Ala Arg Thr Arg Ala
145                 150                 155                 160
Val Asn Tyr Glu Ile Ala Glu Tyr Gln Val Thr Ser Pro Gln Ile Ile
                165                 170                 175
Gly Ile Lys Glu Pro Val Gly Met Lys Thr Val Met Glu Glu Leu Glu
            180                 185                 190
Val Trp Leu Thr Asn Pro Gln Ala Glu Asn Gly Gln Ala Val Leu Ser
        195                 200                 205
Ile Val Gly Phe Gly Gly Val Gly Lys Thr Thr Ile Ala Thr Ala Leu
    210                 215                 220
Tyr Arg Lys Val Ser Glu Lys Phe Gln Cys Arg Ala Ser Val Ala Val
225                 230                 235                 240
Ser Gln Asn Tyr Asp Gln Gly Lys Val Leu Asn Ser Ile Leu Ser Gln
                245                 250                 255
Val Ser Asn Gln Glu Gln Gly Ser Ser Thr Thr Ile Ser Glu Lys Lys
            260                 265                 270
Asn Leu Thr Ser Gly Ala Lys Ser Met Leu Lys Thr Ala Leu Ser Leu
        275                 280                 285
Leu Arg Gly Asn Cys Ile Cys Gln Pro Glu Asn Asp Gly Asn Pro Asp
    290                 295                 300
Asn Thr Pro Ile Arg Leu Gln Glu Thr Thr Asp Asp Asp Gln Asn Pro
305                 310                 315                 320
Arg Lys Leu Glu Gln Leu Leu Ala Glu Lys Ser Tyr Ile Leu Leu Ile
                325                 330                 335
Asp Asp Ile Trp Ser Ala Glu Thr Trp Glu Ser Ile Arg Ser Ile Leu
            340                 345                 350
Pro Lys Asn Asn Lys Gly Gly Arg Ile Ile Val Thr Thr Arg Phe Gln
        355                 360                 365

Ala Val Gly Ser Thr Cys Ser Pro Leu Glu Thr Asp Arg Leu His Thr
    370                 375                 380
Val Asp Phe Leu Thr Asp Asp Glu Ser Gln Asn Leu Phe Asn Thr Ser
385                 390                 395                 400
Ile Cys Glu Ser Lys Ile Arg Lys Asp Ser Asn Lys Val Asp Glu Gln
                405                 410                 415
Val Pro Glu Glu Ile Trp Lys Ile Cys Gly Gly Leu Pro Leu Ala Ile
            420                 425                 430
Val Ser Met Ala Gly Leu Val Ala Cys Asn Pro Arg Lys Ala Cys Cys
        435                 440                 445
Asp Trp Ser Lys Leu Cys Lys Ser Leu Phe Pro Glu Gln Glu Thr Pro
    450                 455                 460
Leu Thr Leu Asp Gly Val Thr Arg Ile Leu Asp Cys Cys Tyr Asn Asp
465                 470                 475                 480
Leu Pro Ala Asp Leu Lys Thr Cys Leu Leu Tyr Leu Ser Ile Phe Pro
                485                 490                 495
Lys Gly Trp Lys Ile Ser Arg Lys Arg Leu Ser Arg Arg Trp Ile Ala
            500                 505                 510
Glu Gly Phe Ala Asn Glu Lys Gln Gly Leu Thr Gln Glu Arg Val Ala
        515                 520                 525
Glu Ala Tyr Phe Asn Gln Leu Thr Arg Arg Asn Leu Val Arg Pro Met
    530                 535                 540
Glu His Gly Ser Asn Gly Lys Val Lys Thr Phe Gln Val His Asp Met
545                 550                 555                 560
Val Leu Glu Tyr Ile Met Ser Lys Ser Ile Glu Glu Asn Phe Ile Thr
                565                 570                 575
Val Val Gly Gly His Trp Gln Met Thr Ala Pro Ser Asn Lys Val Arg
            580                 585                 590
Arg Leu Ser Met Gln Ser Ser Gly Ser Asn Arg Gly Ser Ser Thr Lys
        595                 600                 605
Gly Leu Asn Leu Ala Gln Val Arg Ser Leu Thr Val Phe Gly Asn Leu
    610                 615                 620
Asn His Val Pro Phe His Ser Phe Asn Tyr Gly Ile Ile Gln Val Leu
625                 630                 635                 640
Asp Leu Glu Asp Trp Lys Gly Leu Lys Glu Arg His Met Thr Glu Ile
                645                 650                 655
Cys Gln Met Leu Leu Leu Lys Tyr Leu Ser Ile Arg Arg Thr Glu Ile
            660                 665                 670
Ser Lys Ile Pro Ser Lys Ile Gln Lys Leu Glu Tyr Leu Glu Thr Leu
        675                 680                 685
Asp Ile Arg Glu Thr Tyr Val Arg Asp Leu Pro Lys Ser Ile Val Gln
    690                 695                 700
Leu Lys Arg Ile Ile Ser Ile Leu Gly Gly Asn Lys Asn Thr Arg Lys
705                 710                 715                 720
Gly Leu Arg Leu Pro Gln Glu Lys Ser Lys Lys Pro Ile Lys Asn Pro
                725                 730                 735
Ser Pro Gln Gly Lys Thr Lys Glu Pro Ala Lys Lys Gly Phe Leu Ser
            740                 745                 750
Gln Glu Lys Gly Lys Gly Ala Met Lys Ala Leu Arg Val Leu Ser Gly
        755                 760                 765
Ile Glu Ile Val Glu Glu Ser Ser Glu Val Ala Ala Gly Leu His Gln
    770                 775                 780
Leu Thr Gly Leu Arg Lys Leu Ala Ile Tyr Lys Leu Asn Ile Thr Lys
785                 790                 795                 800
Gly Gly Asp Thr Phe Lys Gln Leu Gln Ser Ser Ile Glu Tyr Leu Gly
                805                 810                 815
Ser Cys Gly Leu Gln Thr Leu Ala Ile Asn Asp Glu Asn Ser Glu Phe
            820                 825                 830
Ile Asn Ser Leu Gly Asp Met Pro Ala Pro Pro Arg Tyr Leu Val Ala
        835                 840                 845
Leu Glu Leu Ser Gly Lys Leu Glu Lys Leu Pro Lys Trp Ile Thr Ser
    850                 855                 860
Ile Thr Thr Leu Asn Lys Leu Thr Ile Ser Val Thr Val Leu Arg Thr

865                 870                 875                 880
Glu Thr Leu Glu Ile Leu His Ile Leu Pro Ser Leu Phe Ser Leu Thr
                885                 890                 895
Phe Ala Phe Ser Leu Ser Ala Ala Lys Gln Asp Gln Asp Ile Ile Lys
            900                 905                 910
Asp Ile Leu Glu Asn Asn Lys Leu Asp Ser Asp Gly Glu Ile Val Ile
        915                 920                 925
Pro Ala Glu Gly Phe Lys Ser Leu Lys Leu Leu Arg Phe Phe Ala Pro
    930                 935                 940
Leu Val Pro Lys Leu Ser Phe Leu Asp Lys Asn Ala Met Pro Ala Leu
945                 950                 955                 960
Glu Ile Ile Glu Met Arg Phe Lys Asp Phe Glu Gly Leu Phe Gly Ile
                965                 970                 975
Glu Ile Leu Glu Asn Leu Arg Glu Val His Leu Lys Val Ser Asp Gly
            980                 985                 990
Ala Glu Ala Ile Thr Lys Phe Leu Val Asn Asp Leu Lys Val Asn Thr
        995                 1000                1005
Glu Lys Pro Lys Val Phe Val Asp Gly Ile Val Thr Ala
    1010                1015                1020

<210> 183
<211> 3229
<212> DNA
<213> Oryza sativa


<400> 183
atggagttgg tggtaggtgc ttccgaagcc accatgaaat ctctcttggg caagctgggc      60

aatcttctag cccaggagta tgctctcatc agcggtatcc gtggtgacat ccagtacatc     120

aatgacgagc ttgccagcat gcaggccttc ctccgtgatc tcagcaacgt gccagagggt     180

cacagtcatg gccaccggat gaaggactgg atgaagcaga tccgagacat cgcctatgat     240

gttgaggact gtatcgatga ctttgcccac cgcctccctc aggattccat cagcgatgcc     300

aaatggtcct tcctactcac aaaaatctat gaactatgga catggtggcc acgtcgtgtg     360

attgcttcca acattgccca actcaaggta cgggcacaac agatcgcaga tcgacgtagt     420

agatacggag tgaacaaccc agaacacctt gacagtagca gcagtgccag gacccgtgct     480

gtcaattacg aaattgctga gtatcaggtc acaagccctc agatcattgg tataaaggag     540

cctgtgggga tgaagacggt catggaggag cttgaggttt ggttaactaa tcctcaagct     600

gaaaatgggc aagctgttct gtccatagtc ggttttggag gtgtgggaaa gactaccatt     660

gccacagcat tgtacagaaa agtcagtgaa aaatttcagt gccgggcatc agtagctgtg     720

tctcagaact atgaccaagg caaagtcctc aatagtattc tgagtcaagt cagcaatcag     780

gagcagggca gcagcacaac aattagtgag aaaaagaacc tcacctcagg cgctaagagc     840

atgttgaaga cagccctgtc actgctcaga ggtaattgta tatgtcagcc agaaaatgat     900

ggaaaccctg ataatacacc aatcaggctg caggaaacaa cggacgatga tcaaaacccc     960

agaaaactgg aacagctcct ggccgaaaag aggtaccttt ttttgtaaat aaaattgctt    1020

tgcttatctg taaattaact tactcatccc actctaaatc taatgtttat ttccttctat    1080

acacagcaca actccatctt ttgaatgggt tttatttttc tcacttgtgc tcattttttt 1140

ttatcatctc tgcagttata tcctcttgat tgatgacatt tggtctgccg aaacatggga 1200

gagtatcaga tcgattttgc ctaaaaataa taaaggcggt agaataatag tgactacaag 1260

atttcaagct gttggttcaa catgctcccc tcttgaaact gatcgtttgc atacagttga 1320

ttttctcacc gatgacgagt cccaaaactt attcaataca agtatttgtg aatcaaagat 1380

aagaaaagat agcaacaaag tagacgagca agtccctgag gaaatatgga aaatatgtgg 1440

gggattgcct ttggccatag tcagcatggc tggtcttgtc gcctgcaacc caaggaaagc 1500

ctgctgcgat tggagtaaac tttgcaaatc attatttcca gagcaagaaa ctcctcttac 1560

cctcgatggt gttacaagga tactggattg ttgttacaat gatttgcctg cggatctgaa 1620

gacttgctta ttgtacttga gtatatttcc gaagggttgg aaaattagta ggaaacgttt 1680

gtcccggcga tggatagctg aaggttttgc taatgagaag caagggttaa cccaggaaag 1740

agttgcagag gcatacttta atcaactcac aagaaggaac ttagtacgtc ccatggagca 1800

tggcagcaat gggaaggtaa aaacgtttca agttcatgac atggttcttg aatacatcat 1860

gtccaaatca atcgaagaga attttattac tgtggttggt ggacactggc agatgactgc 1920

accaagcaat aaagtccgtc gactgtcgat gcaaagcagt ggatccaatc gtggaagttc 1980

aacaaaaggc ctgaacttgg ctcaagtgag atcactgacg gtgtttggga acctgaacca 2040

tgtgccattc cattcattca actatgggat aatacaggtg ctggatcttg aggactggaa 2100

gggtttgaaa gagagacata tgacggagat atgtcaaatg cttttactca agtatttgag 2160

catccgacga acagaaattt ccaaaattcc ctccaagatt cagaaacttg agtacttgga 2220

aactcttgac ataaggagaa catatgtcag ggacctgcct aagtcaatag tccagctaaa 2280

acggatcatt agcatacttg gagggaataa aaacacacgg aaggggctga ggttgcctca 2340

agaaaaaagt aagaagccaa ttaaaaaccc gtcgcctcaa ggaaaaacaa aggagcccgc 2400

aaagaaagga ttcttatccc aagaaaaagg taaaggcgca atgaaagcac tccgtgtact 2460

gtcagggatt gagattgttg aggaatcatc agaagtagct gcaggccttc atcagttgac 2520

agggctaagg aagcttgcca tatacaagct caatataaca aagggtggtg ataccttcaa 2580

acaattacag tcctccattg agtaccttgg cagctgtggt ctgcagactc tggccatcaa 2640

tgatgagaat tctgaattta tcaactcact gggcgacatg cccgcgcctc caagatatct 2700

tgtcgccctt gagctgtctg gcaagttgga gaagctaccc aagtggatca ccagcatcac 2760

tactctcaac aagctaacca tatctgtaac agttcttagg actgaaactt tggagatcct 2820

ccacatttta ccttcattgt tttccctcac cttcgccttt tcacttagtg cagcgaagca 2880

ggatcaggac ataataaagg acatccttga gaataataaa ttggacagtg atggggaaat 2940

cgtcattcca gctgaaggat tcaagagtct taagctgctt cgcttctttg cacctttagt 3000

gccgaagctc agctttttgg acaagaatgc aatgccagca ctcgaaatca ttgaaatgcg 3060

gtttaaagac ttcgaaggtc tatttggcat cgaaatcctt gaaaatctcc gtgaggtgca 3120

tctcaaagtt agtgatgggg cagaagcaat aaccaagttc cttgtaaatg atttgaaggt 3180

taatactgag aaaccaaaag tatttgttga tggcatcgtc actgcatga 3229

<210> 184
<211> 342
<212> DNA
<213> Artificial Sequence

<220>
<223> cDNA

<400> 184
atgcgtgtta ccacttttaa cacattcctt ctcactttgg gaactgtcgc tgtcgtcaat 60

gccgaaacgg gcaacaaata tatagaaaaa cgcgctatcg acctaagtcg agagcgagac 120

cctaactttt tcgaccaccc tggtattcct gtacccgaat gtttttggtt tatgtttaaa 180

aacaacgtac gtcaagatgc tggaacctgt tacagctctt ggaaaatgga catgaaagtt 240

ggtccaaact gggtccatat taaatcagac gataattgca atttgtcggg cgacttccct 300

ccaggttgga ttgttttggg gaaaaaaagg cccggctttt aa 342

<210> 185
<211> 113
<212> PRT
<213> Magnaporthe oryzae

<400> 185
Met Arg Val Thr Thr Phe Asn Thr Phe Leu Leu Thr Leu Gly Thr Val
1               5                   10                  15
Ala Val Val Asn Ala Glu Thr Gly Asn Lys Tyr Ile Glu Lys Arg Ala
            20                  25                  30
Ile Asp Leu Ser Arg Glu Arg Asp Pro Asn Phe Phe Asp His Pro Gly
        35                  40                  45
Ile Pro Val Pro Glu Cys Phe Trp Phe Met Phe Lys Asn Asn Val Arg
    50                  55                  60
Gln Asp Ala Gly Thr Cys Tyr Ser Ser Trp Lys Met Asp Met Lys Val
65                  70                  75                  80
Gly Pro Asn Trp Val His Ile Lys Ser Asp Asp Asn Cys Asn Leu Ser
                85                  90                  95
Gly Asp Phe Pro Pro Gly Trp Ile Val Leu Gly Lys Lys Arg Pro Gly
            100                 105                 110
Phe

<210> 186
<211> 3429
<212> DNA
<213> Artificial Sequence

<220>
<223> cDNA

<400> 186
atggaggcgg ctgccatggc cgtaaccgca gccacggggg ccttggcgcc cgtgctagtg      60

aagctggccg ctttgctgga cgacggggag tgcaatcttc tggaggggag ccggagcgac     120

gcagagttca tcagatccga gctggaggcc gttcattctc tcctcacccc aaatatcttg     180

gggaggatgg gggatgacga tgcggcgtgc aaggatggct tgattgcgga ggtccgggag     240

ctgtcctacg acctggatga tgccgtcgac gacttcttgg agctcaattt cgagcagcga     300

agaagcgcaa gccctttcgg tgagctcaag gcaagagttg aggagcatgt ctccaatcgc     360

ttctctgact ggaagctacc ggcggcgagc cttccgccgt cgtcggtaca ccgccgagct     420

ggcttgccgc caccagatgc agagctggtg gggatggaca aacgtatgga agagctcacc     480

aaattgctgg aacaagggag caatgatgct tcacgatggc gcaagcgaaa accgcatttc     540

ccgctcagaa aaacagggct aaagcaaaaa atcgtgatca aggttgccat ggagggcaat     600

aattgccgtt caaaagcaat ggctttagtt gcgagcactg gaggagtgga ctcggttgcg     660

ctcgtaggtg atctaagaga caagatagag gtggtcggtt atggcattga ccccatcaag     720

ctgatctccg cgctccggaa gaaggtgggc gatgcggagt tgctgcaggt cagccaagca     780

aataaagatg tgaaggagac gacgccgatg cttgcgccgg tgaaatccat atgtgaattt     840

cacaaggtca aaacagtttg catccttgga ttgccaggtg gaggcaaaac aacggttgcc     900

agagaattat atgacgcctt gggaacgcac ttcccatgcc gggttttcgt gtcagtctct     960

ccaagttcta gtcccagtcc caatctcaca aagactcttg cagacatttt cgctcaagca    1020

caactaggag taaccgatac acttagcaca tcatatggtg ggagtgggac cgggagagct    1080

cttcaacaac atctcatcga caacatatca gctttcctcc tcaacaaaaa gtatctcatt    1140

gtaatcgatg acatttggca ttgggaagaa tgggaagtca tcagaaagtc cattcccaag    1200

aatgatctgg gtggtagaat aatcatgact actcgtctta attcaatagc tgagaagtgc    1260

cacactgatg acaatgatgt ttttgtctac gaagttgggg atctagataa taatgatgct    1320

ttgtcgttgt cttgggggat agcaacaaag tctggggcag gcaacaggat cggaactgga    1380

gaggataatc catgctatga tattgtgaac atgtgttatg gtatgccttt agcacttatt    1440

tggctgtcgt cagcattggt tggagagata gaagaattag gtggtgctga agtgaaaaaa    1500

tgtagggatt tgagacacat agaggatggt attttggaca tcccatcctt acaaccattg    1560

gcggagagtt tatgccttgg ttataaccat cttcctcttt atctgaggac tttgttgttg    1620

tactgtagtg cataccattg gtctaacaga atcgaaaggg gtcgtctggt caggaggtgg    1680

attgcggaag gatttgtgtc ggaagagaaa gaagcagaag gttactttgg cgagcttatt    1740

aacagaggat ggattacgca gcacggagac aacaacagtt ataattacta tgagatccac    1800

cccgtgatgc tggccttcct gagatgcaag tccaaggagt acaatttttt aacatgcttg 1860

ggtctgggat ctgatactag tactagtgca tcctccccaa ggttgattcg ccggctgtct 1920

cttcaggggg ggtatccagt ggactgcttg tcaagcatga gtatggatgt gtcacacact 1980

tgcagccttg tcgtccttgg tgacgtggcg cgacccaagg gaatcccctt ctatatgttt 2040

aagcgcttgc gagtgttgga ccttgaagat aataaggata tacaggattc tcatctgcag 2100

ggcatatgtg aacagttaag cctcagagtg aggtaccttg gtctcaaggg aacgcggatc 2160

cgaaagctcc ctcaggagat gaggaagctg aagcatttgg agattttgta tgtggggagc 2220

actcggatca gtgaacttcc gcaagagatt ggagagctga agcatctgcg gattctggac 2280

gtgagaaaca cggacatcac tgagctccca ctgcagatac gggagctgca gcatctgcac 2340

actctggacg tgaggaacac tccaatcagt gagctcccgc cgcaggttgg caagctgcag 2400

aatctcaaga ttatgtgcgt gaggagcact ggggttaggg agctcccaaa ggagattggg 2460

gagctgaatc atctacagac tctggacgtg agaaacacga gggtgagaga gctgccatgg 2520

caagctggcc agatctccca atcgttgcgc gtgcttgccg gtgacagtgg cgatggcgtg 2580

cggttgcccg aaggcgtctg cgaagctctg atcaacggta ttccaggggc tacgcgtgca 2640

aaatgcaggg aggttctgtc catcgcgatc atcgatcgtt tcggacctcc ccttgttggg 2700

atattcaaag ttcccggcag tcatatgcgt atcccgaaga tgatcaaaga ccacttccgc 2760

gttctttctt gcctagacat caggctctgc cacaagcttg aggatgatga ccaaaagttc 2820

ctcgccgaga tgcccaacct gcagacgctc gtgctgaggt tcgaggccct accaagacaa 2880

cccataacca tcaacggcac aggcttccag atgctggaga gcttccgtgt cgacagccgg 2940

gtgccaagga tagccttcca tgaagacgcc atgcccaacc tcaagcttct cgagttcaag 3000

ttctacgccg gcccagcaag caacgatgcc atcggcatca ccaacctgaa gagcctccaa 3060

aaggtggtct ttcggtgctc gccatggtac aagagcgacg cccctggcat cagcgccacc 3120

attgacgtcg tgaagaaaga agccgaggag catcccaacc ggccgatcac cctcctcatc 3180

aatgctgggt ataaggagat atcaactgag tcacacggga gcagtgaaaa cattgcgggc 3240

agcagtggga tcgatactga gcctgcacag gcacagcatg ataatctccc tgctgttcga 3300

gatgactaca agggaaaagg gattcttctt gatggcaggt gtcctacctg cggccgagcg 3360

actaaaattg aagaggaaac ccaagatcga gtagcagata ttgaaattca aacagaaact 3420

actagctag 3429

<210> 187
<211> 1142
<212> PRT
<213> Oryza sativa

<400> 187
Met Glu Ala Ala Ala Met Ala Val Thr Ala Ala Thr Gly Ala Leu Ala
1               5                   10                  15
Pro Val Leu Val Lys Leu Ala Ala Leu Leu Asp Asp Gly Glu Cys Asn
            20                  25                  30
Leu Leu Glu Gly Ser Arg Ser Asp Ala Glu Phe Ile Arg Ser Glu Leu
        35                  40                  45
Glu Ala Val His Ser Leu Leu Thr Pro Asn Ile Leu Gly Arg Met Gly
    50                  55                  60
Asp Asp Asp Ala Ala Cys Lys Asp Gly Leu Ile Ala Glu Val Arg Glu
65                  70                  75                  80
Leu Ser Tyr Asp Leu Asp Asp Ala Val Asp Asp Phe Leu Glu Leu Asn
                85                  90                  95
Phe Glu Gln Arg Arg Ser Ala Ser Pro Phe Gly Glu Leu Lys Ala Arg
            100                 105                 110
Val Glu Glu His Val Ser Asn Arg Phe Ser Asp Trp Lys Leu Pro Ala
        115                 120                 125
Ala Ser Leu Pro Pro Ser Ser Val His Arg Arg Ala Gly Leu Pro Pro
    130                 135                 140
Pro Asp Ala Glu Leu Val Gly Met Asp Lys Arg Met Glu Glu Leu Thr
145                 150                 155                 160
Lys Leu Leu Glu Gln Gly Ser Asn Asp Ala Ser Arg Trp Arg Lys Arg
                165                 170                 175
Lys Pro His Phe Pro Leu Arg Lys Thr Gly Leu Lys Gln Lys Ile Val
            180                 185                 190
Ile Lys Val Ala Met Glu Gly Asn Asn Cys Arg Ser Lys Ala Met Ala
        195                 200                 205
Leu Val Ala Ser Thr Gly Gly Val Asp Ser Val Ala Leu Val Gly Asp
    210                 215                 220
Leu Arg Asp Lys Ile Glu Val Val Gly Tyr Gly Ile Asp Pro Ile Lys
225                 230                 235                 240
Leu Ile Ser Ala Leu Arg Lys Lys Val Gly Asp Ala Glu Leu Leu Gln
                245                 250                 255
Val Ser Gln Ala Asn Lys Asp Val Lys Glu Thr Thr Pro Met Leu Ala
            260                 265                 270
Pro Val Lys Ser Ile Cys Glu Phe His Lys Val Lys Thr Val Cys Ile
        275                 280                 285
Leu Gly Leu Pro Gly Gly Gly Lys Thr Thr Val Ala Arg Glu Leu Tyr
    290                 295                 300
Asp Ala Leu Gly Thr His Phe Pro Cys Arg Val Phe Val Ser Val Ser
305                 310                 315                 320
Pro Ser Ser Ser Pro Ser Pro Asn Leu Thr Lys Thr Leu Ala Asp Ile
                325                 330                 335
Phe Ala Gln Ala Gln Leu Gly Val Thr Asp Thr Leu Ser Thr Ser Tyr
            340                 345                 350
Gly Gly Ser Gly Thr Gly Arg Ala Leu Gln Gln His Leu Ile Asp Asn
        355                 360                 365
Ile Ser Ala Phe Leu Leu Asn Lys Lys Tyr Leu Ile Val Ile Asp Asp
    370                 375                 380
Ile Trp His Trp Glu Glu Trp Glu Val Ile Arg Lys Ser Ile Pro Lys
385                 390                 395                 400
Asn Asp Leu Gly Gly Arg Ile Ile Met Thr Thr Arg Leu Asn Ser Ile
                405                 410                 415
Ala Glu Lys Cys His Thr Asp Asp Asn Asp Val Phe Val Tyr Glu Val
            420                 425                 430
Gly Asp Leu Asp Asn Asn Asp Ala Leu Ser Leu Ser Trp Gly Ile Ala
        435                 440                 445
Thr Lys Ser Gly Ala Gly Asn Arg Ile Gly Thr Gly Glu Asp Asn Pro
    450                 455                 460
Cys Tyr Asp Ile Val Asn Met Cys Tyr Gly Met Pro Leu Ala Leu Ile
465                 470                 475                 480
Trp Leu Ser Ser Ala Leu Val Gly Glu Ile Glu Glu Leu Gly Gly Ala

                485                 490                 495
Glu Val Lys Lys Cys Arg Asp Leu Arg His Ile Glu Asp Gly Ile Leu
            500                 505                 510
Asp Ile Pro Ser Leu Gln Pro Leu Ala Glu Ser Leu Cys Leu Gly Tyr
        515                 520                 525
Asn His Leu Pro Leu Tyr Leu Arg Thr Leu Leu Leu Tyr Cys Ser Ala
    530                 535                 540
Tyr His Trp Ser Asn Arg Ile Glu Arg Gly Arg Leu Val Arg Arg Trp
545                 550                 555                 560
Ile Ala Glu Gly Phe Val Ser Glu Glu Lys Glu Ala Glu Gly Tyr Phe
                565                 570                 575
Gly Glu Leu Ile Asn Arg Gly Trp Ile Thr Gln His Gly Asp Asn Asn
            580                 585                 590
Ser Tyr Asn Tyr Tyr Glu Ile His Pro Val Met Leu Ala Phe Leu Arg
        595                 600                 605
Cys Lys Ser Lys Glu Tyr Asn Phe Leu Thr Cys Leu Gly Leu Gly Ser
    610                 615                 620
Asp Thr Ser Thr Ser Ala Ser Ser Pro Arg Leu Ile Arg Arg Leu Ser
625                 630                 635                 640
Leu Gln Gly Gly Tyr Pro Val Asp Cys Leu Ser Ser Met Ser Met Asp
                645                 650                 655
Val Ser His Thr Cys Ser Leu Val Val Leu Gly Asp Val Ala Arg Pro
            660                 665                 670
Lys Gly Ile Pro Phe Tyr Met Phe Lys Arg Leu Arg Val Leu Asp Leu
        675                 680                 685
Glu Asp Asn Lys Asp Ile Gln Asp Ser His Leu Gln Gly Ile Cys Glu
    690                 695                 700
Gln Leu Ser Leu Arg Val Arg Tyr Leu Gly Leu Lys Gly Thr Arg Ile
705                 710                 715                 720
Arg Lys Leu Pro Gln Glu Met Arg Lys Leu Lys His Leu Glu Ile Leu
                725                 730                 735
Tyr Val Gly Ser Thr Arg Ile Ser Glu Leu Pro Gln Glu Ile Gly Glu
            740                 745                 750
Leu Lys His Leu Arg Ile Leu Asp Val Arg Asn Thr Asp Ile Thr Glu
        755                 760                 765
Leu Pro Leu Gln Ile Arg Glu Leu Gln His Leu His Thr Leu Asp Val
    770                 775                 780
Arg Asn Thr Pro Ile Ser Glu Leu Pro Pro Gln Val Gly Lys Leu Gln
785                 790                 795                 800
Asn Leu Lys Ile Met Cys Val Arg Ser Thr Gly Val Arg Glu Leu Pro
                805                 810                 815
Lys Glu Ile Gly Glu Leu Asn His Leu Gln Thr Leu Asp Val Arg Asn
            820                 825                 830
Thr Arg Val Arg Glu Leu Pro Trp Gln Ala Gly Gln Ile Ser Gln Ser
        835                 840                 845
Leu Arg Val Leu Ala Gly Asp Ser Gly Asp Gly Val Arg Leu Pro Glu
    850                 855                 860
Gly Val Cys Glu Ala Leu Ile Asn Gly Ile Pro Gly Ala Thr Arg Ala
865                 870                 875                 880
Lys Cys Arg Glu Val Leu Ser Ile Ala Ile Ile Asp Arg Phe Gly Pro
                885                 890                 895
Pro Leu Val Gly Ile Phe Lys Val Pro Gly Ser His Met Arg Ile Pro
            900                 905                 910
Lys Met Ile Lys Asp His Phe Arg Val Leu Ser Cys Leu Asp Ile Arg
        915                 920                 925
Leu Cys His Lys Leu Glu Asp Asp Asp Gln Lys Phe Leu Ala Glu Met
    930                 935                 940
Pro Asn Leu Gln Thr Leu Val Leu Arg Phe Glu Ala Leu Pro Arg Gln
945                 950                 955                 960
Pro Ile Thr Ile Asn Gly Thr Gly Phe Gln Met Leu Glu Ser Phe Arg
                965                 970                 975
Val Asp Ser Arg Val Pro Arg Ile Ala Phe His Glu Asp Ala Met Pro
            980                 985                 990

Asn Leu Lys Leu Leu Glu Phe Lys Phe Tyr Ala Gly Pro Ala Ser Asn
        995                 1000                1005
Asp Ala Ile Gly Ile Thr Asn Leu Lys Ser Leu Gln Lys Val Val Phe
    1010                1015                1020
Arg Cys Ser Pro Trp Tyr Lys Ser Asp Ala Pro Gly Ile Ser Ala Thr
1025                1030                1035                1040
Ile Asp Val Val Lys Lys Glu Ala Glu Glu His Pro Asn Arg Pro Ile
                1045                1050                1055
Thr Leu Leu Ile Asn Ala Gly Tyr Lys Glu Ile Ser Thr Glu Ser His
            1060                1065                1070
Gly Ser Ser Glu Asn Ile Ala Gly Ser Ser Gly Ile Asp Thr Glu Pro
        1075                1080                1085
Ala Gln Ala Gln His Asp Asn Leu Pro Ala Val Arg Asp Asp Tyr Lys
    1090                1095                1100
Gly Lys Gly Ile Leu Leu Asp Gly Arg Cys Pro Thr Cys Gly Arg Ala
1105                1110                1115                1120
Thr Lys Ile Glu Glu Glu Thr Gln Asp Arg Val Ala Asp Ile Glu Ile
                1125                1130                1135
Gln Thr Glu Thr Thr Ser
            1140

<210> 188
<211> 6350
<212> DNA
<213> Oryza sativa


<400> 188
atggaggcgg ctgccatggc cgtaaccgca gccacggggg ccttggcgcc cgtgctagtg      60

aagctggccg ctttgctgga cgacggggag tgcaatcttc tggaggggag ccggagcgac     120

gcagagttca tcagatccga gctggaggcc gttcattctc tcctcacccc aaatatcttg     180

gggaggatgg gggatgacga tgcggcgtgc aaggatggct tgattgcgga ggtccgggag     240

ctgtcctacg acctggatga tgccgtcgac gacttcttgg agctcaattt cgagcagcga     300

agaagcgcaa gccctttcgg tgagctcaag gcaagagttg aggagcatgt ctccaatcgc     360

ttctctgact ggaagctacc ggcggcgagc cttccgccgt cgtcggtaca ccgccgagct     420

ggcttgccgc caccagatgc agagctggtg gggatggaca aacgtatgga agagctcacc     480

aaattgctgg aacaagggag caatgatgct tcacgatggc gcaagcgaaa accgcatttc     540

ccgctcagaa aaacagggct aaaggtacgg ttggatctcg attcttcaca aatatagctt     600

cctccgatag cagtgccaat gaatttgttt acttctctct tgattcttta atttggaagt     660

actgtataac aaacatggag ggatcgtcat ttaacttaat tttttttgtt gcagcaaaaa     720

atcgtgatca aggttgccat ggagggcaat aattgccgtt caaaagcaat ggctttagtt     780

gcgagcactg gaggagtgga ctcggttgcg ctcgtaggtg atctaagaga caagatagag     840

gtggtcggtt atggcattga ccccatcaag ctgatctccg cgctccggaa gaaggtgggc     900

gatgcggagt tgctgcaggt cagccaagca aataaagatg tgaaggagac gacgccgatg     960

cttgcgccgg tgaaatccat atgtgaattt cacaaggtca aaacagtttg catccttgga    1020

ttgccaggtg gaggcaaaac aacggttgcc agagaattat atgacgcctt gggaacgcac    1080

ttcccatgcc gggttttcgt gtcagtctct ccaagttcta gtcccagtcc caatctcaca 1140

aagactcttg cagacatttt cgctcaagca caactaggag taaccgatac acttagcaca 1200

tcatatggtg ggagtgggac cgggagagct cttcaacaac atctcatcga caacatatca 1260

gctttcctcc tcaacaaaaa gtaagcaata tcttatatct cttatctagc ttgtccttaa 1320

tccggcttcc caaattaaag tgaacaggct atatgtccat ataatgttgg gttttttttt 1380

tctgtcctcg atgaggtata tatcaagata tgccaaactt tttttaatta gcgttacttt 1440

attctaccta gtaaatagat acagaaatgg ggcaggcccc tcctttgtga caggccaatg 1500

gttttatgat gtggaaacaa ctcccttatc actgaagctc ttgctattac tcgtgatggt 1560

actgatcttg caaaggcttg cagtatttgt aaatccacag ggagtggtca tctttttttt 1620

tttaagataa tgaatagaaa tctggcctct atatagaaag ccaaaggtta cacaagtgca 1680

tacactccaa atctcaaagc tgagaaacac gaaaaacaaa cgaccaaaaa gaaaagacga 1740

taagattaag tttcaagctt ttcctccttg cttcagaaaa cagggaaatg cagagctagc 1800

ccgctcttcc cctgccatgc cctctcactg ccattgcaac taccacaaaa gctcccctgt 1860

ttcgccgtga gcgaagggag gggatggaac tagtctgctg ctttgttgtc gccaagcact 1920

ggtttcatgc tgcccaaaca acctatgccc cgctagcgat aacgagtctg ttgccgccat 1980

gctgttgtcg ctggagagca cttgtcgctg ctcagctatg tgttaagtaa gctactaaca 2040

atctctccat tctcttgtag actgcttata aaagattgta cttcaacttt ttattaatga 2100

aatagagttc ctgccttaca acaacaagaa cgagagagag agagagagcc aatatcacga 2160

gttagcactg tgaagacttc ctgactgtta tgtcttcaag aacagtataa aagcatttgc 2220

cactataaaa atatgatagc cctcaagatc attaaaaata tgtcatgttc ttttaccttc 2280

ataacaacca gtatcacaaa tatgaaaaca tttaaaggaa aatcagtatt tgccatttat 2340

gtaaagaaaa actagactag aactctgggc ttttatttta gtgcacactg tatacatgtg 2400

tgttctttga cgtatatact tgatattttt tcaaaaactt ttgggggcat atctgtaacc 2460

catgccccat gcatccaaag tagtagtgag acaactttcg tatcaatgga catgtggcat 2520

taaatttaat cccaatattt gttctctcga agtcctgaac atataattgg acaaaattat 2580

ataatttaat taggccaatt gagccatata tacacacact ataagaaact tctacaaaac 2640

aaacattcgc tccttttagg ttttgaccag tctatacatg aaccagccaa cggtgtgctg 2700

gtctgactat aaatgttttt ttagagagta aaggcacctt ccagtacctc tctcgaggac 2760

agtaaaaaca cccatgcttt aaggttatga acaacaaagc atagaacaac cacaagaaaa 2820

aaaaaaacaa ctagaacaag ttctggaact agttagggat tcgggaagcc attgcttgct 2880

ggcttgggac aagctatgcc tgccattgga ctatggtgga gactgggcat aaaggatctc 2940

aatctcttgg agattggcct atgtcctagg tggccatggc tgactgcaag gagatatatt 3000
tcttgcaacg gaaattttca ttgcaaaagc tgaaaatcgt tgcgatatgt acctattgtg 3060
acataattga atccattggg agccgtagca ataaatcccg tggtaatagg ctattgcaat 3120
gattttccga tggttgcata aaaaggtgct attacaatgg ttttgcattg ctattgcaac 3180
aacttgatat gtcataggta tttattgcaa cagtttgtac catgctgcaa taagatttcg 3240
caacacagac ctattcatta aatagcctat ttccacacat tcatttttgt ggcaataatt 3300
cagtgcgaca agcatgttct atagctataa cctgatgcaa cgggttagac ttgttgcagt 3360
aacttattag cacatttaat tttttactaa atttatattg attcaactat ttcaacatca 3420
ttcaatacag taaatactgg aaaatgcaag caatctccaa aaattcacca ataagagaaa 3480
cgaattaaat catatacaca attatttcac accgtacagt gcacttaagt gtaggaaata 3540
taatgctata tacatttcca accctcaata caggttctgt aaataatgaa aagaaacatc 3600
aatttgattt cttgaccggt ccctctgaga cacacagtgc tttcctcagc aacatcgttg 3660
ccgatctatg taacttgtaa actccttgtg cttaataaat tctggccagg gttacttcag 3720
cctgcatggc gaaaaaaaag aacttgttag ggagatcgca aacaaatgtt agctgcccca 3780
tttcttctct actgaccata aaagctctag gatcaggtca tgaaaacaat gccgtcagac 3840
cggaaatact caagattcag aacacgactc ctaggaactg caagtttaaa tttcaacaaa 3900
agcaaaatac aaccagatga caaactaata tatatcagct gactgttcat ctcaagtgga 3960
ctcacatctg tgaaattgtt agttctatgc atatatgcta atgtaatact aatatgctat 4020
tgaggcttat tactcttctc gacttttata ggtatctcat tgtaatcgat gacatttggc 4080
attgggaaga atgggaagtc atcagaaagt ccattcccaa gaatgatctg ggtggtagaa 4140
taatcatgac tactcgtctt aattcaatag ctgagaagtg ccacactgat gacaatgatg 4200
tttttgtcta cgaagttggg gatctagata ataatgatgc tttgtcgttg tcttggggga 4260
tagcaacaaa gtctggggca ggcaacagga tcggaactgg agaggataat ccatgctatg 4320
atattgtgaa catgtgttat ggtatgcctt tagcacttat ttggctgtcg tcagcattgg 4380
ttggagagat agaagaatta ggtggtgctg aagtgaaaaa atgtagggat ttgagacaca 4440
tagaggatgg tattttggac atcccatcct tacaaccatt ggcggagagt ttatgccttg 4500
gttataacca tcttcctctt tatctgagga ctttgttgtt gtactgtagt gcataccatt 4560
ggtctaacag aatcgaaagg ggtcgtctgg tcaggaggtg gattgcggaa ggatttgtgt 4620
cggaagagaa agaagcagaa ggttactttg gcgagcttat taacagagga tggattacgc 4680
agcacggaga caacaacagt tataattact atgagatcca ccccgtgatg ctggccttcc 4740
tgagatgcaa gtccaaggag tacaattttt taacatgctt gggtctggga tctgatacta 4800
gtactagtgc atcctcccca aggttgattc gccggctgtc tcttcagggg gggtatccag 4860

tggactgctt gtcaagcatg agtatggatg tgtcacacac ttgcagcctt gtcgtccttg 4920

gtgacgtggc gcgacccaag ggaatcccct tctatatgtt taagcgcttg cgagtgttgg 4980

accttgaaga taataaggat atacaggatt ctcatctgca gggcatatgt gaacagttaa 5040

gcctcagagt gaggtacctt ggtctcaagg gaacgcggat ccgaaagctc cctcaggaga 5100

tgaggaagct gaagcatttg gagattttgt atgtggggag cactcggatc agtgaacttc 5160

cgcaagagat tggagagctg aagcatctgc ggattctgga cgtgagaaac acggacatca 5220

ctgagctccc actgcagata cgggagctgc agcatctgca cactctggac gtgaggaaca 5280

ctccaatcag tgagctcccg ccgcaggttg gcaagctgca gaatctcaag attatgtgcg 5340

tgaggagcac tggggttagg gagctcccaa aggagattgg ggagctgaat catctacaga 5400

ctctggacgt gagaaacacg agggtgagag agctgccatg gcaagctggc cagatctccc 5460

aatcgttgcg cgtgcttgcc ggtgacagtg gcgatggcgt gcggttgccc gaaggcgtct 5520

gcgaagctct gatcaacggt attccagggg ctacgcgtgc aaaatgcagg gaggttctgt 5580

ccatcgcgat catcgatcgt ttcggacctc cccttgttgg gatattcaaa gttcccggca 5640

gtcatatgcg tatcccgaag atgatcaaag accacttccg cgttctttct tgcctagaca 5700

tcaggctctg ccacaagctt gaggatgatg accaaaagtt cctcgccgag atgcccaacc 5760

tgcagacgct cgtgctgagg ttcgaggccc taccaagaca acccataacc atcaacggca 5820

caggcttcca gatgctggag agcttccgtg tcgacagccg ggtgccaagg atagccttcc 5880

atgaagacgc catgcccaac ctcaagcttc tcgagttcaa gttctacgcc ggcccagcaa 5940

gcaacgatgc catcggcatc accaacctga agagcctcca aaaggtggtc tttcggtgct 6000

cgccatggta caagagcgac gcccctggca tcagcgccac cattgacgtc gtgaagaaag 6060

aagccgagga gcatcccaac cggccgatca ccctcctcat caatgctggg tataaggaga 6120

tatcaactga gtcacacggg agcagtgaaa acattgcggg cagcagtggg atcgatactg 6180

agcctgcaca ggcacagcat gataatctcc ctgctgttcg agatgactac aagggaaaag 6240

ggattcttct tgatggcagg tgtcctacct gcggccgagc gactaaaatt gaagaggaaa 6300

cccaagatcg agtagcagat attgaaattc aaacagaaac tactagctag 6350

<210> 189
<211> 3066
<212> DNA
<213> Artificial Sequence

<220>
<223> cDNA

<400> 189
atggagttgg tggtaggtgc ttccgaagcc accatgaaat ctctcttggg caagctgggc 60

aatcttctag cccaggagta tgctctcatc agcggtatcc gtggtgacat ccagtacatc 120

aatgacgagc ttgccagcat gcaggccttc ctccgtgatc tcagcaacgt gccagagggt 180

cacagtcatg gccaccggat gaaggactgg atgaagcaga tccgagacat cgcctatgat 240

gttgaggact gtatcgatga ctttgcccac cgcctccctc aggattccat cagcgatgcc 300

aaatggtcct tcctactcac aaaaatctat gaactatgga catggtggcc acgtcgtgtg 360

attgcttcca acattgccca actcaaggta cgggcacaac agatcgcaga tcgacgtagt 420

agatacggag tgaacaaccc agaacacctt gacagtagca gcagtgccag gacccgtgct 480

gtcaattacg aaattgctga gtatcaggtc acaagccctc agatcattgg tataaaggag 540

cctgtgggga tgaagacggt catggaggag cttgaggttt ggttaactaa tcctcaagct 600

gaaaatgggc aagctgttct gtccatagtc ggttttggag gtgtgggaaa gactaccatt 660

gccacagcat tgtacagaaa agtcagtgat aaatttcagt gccgggcatc agtagctgtg 720

tctcagaact atgaccaagg caaagtcctc aatagtattc tgagtcaagt cagcaatcag 780

gagcagggca gcagcacaac aattagtgag aaaaagaacc tcacctcagg cgctaagagc 840

atgttgaaga cagccctgtc actgctcaga ggtaattgta tatgtcagcc agaaaatgat 900

ggaaaccctg ataatacacc aatcaggctg caggaaacaa cggacgatga tcaaaacccc 960

agaaaactgg aacagctcct ggccgaaaag agttatatcc tcttgattga tgacatttgg 1020

tctgccgaaa catgggagag tatcagatcg attttgccta aaaataataa aggcggtaga 1080

ataatagtga ctacaagatt tcaagctgtt ggttcaacat gctcccctct tgaaactgat 1140

cgtttgcata cagttgattt tctcaccgat gacgagtccc aaaacttatt caatacaagt 1200

atttgtgaat caaagataag aaaagatagc aacaaagtag acgagcaagt ccctgaggaa 1260

atatggaaaa tatgtggggg attgcctttg gccatagtca ccatggctgg tcttgtcgcc 1320

tgcaacccaa ggaaagcctg ctgcgattgg agtaaacttt gcaaatcatt atttccagag 1380

caagaaactc ctcttaccct cgatggtgtt acaaggatac tggattgttg ttacaatgat 1440

ttgcctgcgg atctgaagac ttgcttattg tacttgagta tatttccgaa gggttggaaa 1500

attagtagga aacgtttgtc ccggcgatgg atagctgaag gttttgctaa tgagaagcaa 1560

gggttaaccc aggaaagagt tgcagaggca tactttaatc aactcacaag aaggaactta 1620

gtacgtccca tggagcatgg cagcaatggg aaggtaaaaa cgtttcaagt tcatgacatg 1680

gttcttgaat acatcatgtc caaatcaatc gaagagaatt ttattactgt ggttggtgga 1740

cactggcaga tgactgcacc aagcaataaa gtccgtcgac tgtcgatgca aagcagtgga 1800

tccaatcgtg gaagttcaac aaaaggcctg aacttggctc aagtgagatc actgacggtg 1860

tttgggaacc tgaaccatat gccattccat tcattcaact atgggataat acaggtgctg 1920

gatcttgagg actggaaggg tttgaaagag agacatatga cggagatatg tcaaatgctt 1980

ttactcaagt atttgagcat ccgacgaaca gaaatttcca aaattccctc caagattcag 2040

aaacttgagt acttggaaac tcttgacata agggagacat atgtcaggga cctgcctaag 2100

tcaatagtcc agctaaaacg gatcattagc atacttggag ggaataaaaa cacacggaag 2160

gggctgaggt tgcctcaaga aaaaagtaag aagccaatta aaaacccgtc gcctcaagga 2220

aaaacaaagg agcccgcaaa gaaaggattc ttatcccaag aaaaaggtaa aggcgcaatg 2280

aaagcactcc gtgtactgtc agggattgag attgttgagg aatcatcaga agtagctgca 2340

ggccttcatc agttgacagg gctaaggaag cttgccatat acaagctcaa tataacaaag 2400

ggtggtgata ccttcaaaca attacagtcc tccattgagt accttggcag ctgtggtctg 2460

cagactctgg ccatcaatga tgagaattct gaatttatca actcactggg cgacatgccc 2520

gcgcctccaa gatatcttgt cgcccttgag ctgtctggca agttggagaa gctacccaag 2580

tggatcacca gcatcactac tctcaacaag ctaaccatat ctgtaacagt tcttaggact 2640

gaaactttgg agatcctcca cattttacct tcattgtttt ccctcacctt cgccttttca 2700

cttagtgcag cgaagcagga tcaggacata ataaaggaca tccttgagaa taataaattg 2760

gacagtgatg gggaaatcgt cattccagct gaaggattca agagtcttaa gctgcttcgc 2820

ttctttgcac ctttagtgcc gaagctcagc tttttggaca agaatgcaat gccagcactc 2880

gaaatcattg aaatgcggtt taaagacttc gaaggtctat ttggcatcga aatccttgaa 2940

aatctccgtg aggtgcatct caaagttagt gatggggcag aagcaataac caagttcctt 3000

gtaaatgatt tgaaggttaa tactgagaaa ccaaaagtat ttgttgatgg catcgtcact 3060

gcatga 3066

<210> 190
<211> 1021
<212> PRT
<213> Oryza sativa

<400> 190
   Met Glu Leu Val Val Gly Ala Ser Glu Ala Thr Met Lys Ser Leu Leu
   1 5 10 15
   Gly Lys Leu Gly Asn Leu Leu Ala Gln Glu Tyr Ala Leu Ile Ser Gly
   20 25 30
   Ile Arg Gly Asp Ile Gln Tyr Ile Asn Asp Glu Leu Ala Ser Met Gln
   35 40 45
   Ala Phe Leu Arg Asp Leu Ser Asn Val Pro Glu Gly His Ser His Gly
   50 55 60
   His Arg Met Lys Asp Trp Met Lys Gln Ile Arg Asp Ile Ala Tyr Asp
   65 70 75 80
   Val Glu Asp Cys Ile Asp Asp Phe Ala His Arg Leu Pro Gln Asp Ser
   85 90 95
   Ile Ser Asp Ala Lys Trp Ser Phe Leu Leu Thr Lys Ile Tyr Glu Leu
   100 105 110
   Trp Thr Trp Trp Pro Arg Arg Val Ile Ala Ser Asn Ile Ala Gln Leu
                   115                     120                     125
   Lys Val Arg Ala Gln Gln Ile Ala Asp Arg Arg Ser Arg Tyr Gly Val
       130                     135                     140
   Asn Asn Pro Glu His Leu Asp Ser Ser Ser Ser Ala Arg Thr Arg Ala
   145                     150                     155                     160
   Val Asn Tyr Glu Ile Ala Glu Tyr Gln Val Thr Ser Pro Gln Ile Ile
                   165                     170                     175
   Gly Ile Lys Glu Pro Val Gly Met Lys Thr Val Met Glu Glu Leu Glu
               180                     185                     190
   Val Trp Leu Thr Asn Pro Gln Ala Glu Asn Gly Gln Ala Val Leu Ser
           195                     200                     205
   Ile Val Gly Phe Gly Gly Val Gly Lys Thr Thr Ile Ala Thr Ala Leu
       210                     215                     220
   Tyr Arg Lys Val Ser Asp Lys Phe Gln Cys Arg Ala Ser Val Ala Val
   225                     230                     235                     240
   Ser Gln Asn Tyr Asp Gln Gly Lys Val Leu Asn Ser Ile Leu Ser Gln
                   245                     250                     255
   Val Ser Asn Gln Glu Gln Gly Ser Ser Thr Thr Ile Ser Glu Lys Lys
               260                     265                     270
   Asn Leu Thr Ser Gly Ala Lys Ser Met Leu Lys Thr Ala Leu Ser Leu
           275                     280                     285
   Leu Arg Gly Asn Cys Ile Cys Gln Pro Glu Asn Asp Gly Asn Pro Asp
       290                     295                     300
   Asn Thr Pro Ile Arg Leu Gln Glu Thr Thr Asp Asp Asp Gln Asn Pro
   305                     310                     315                     320
   Arg Lys Leu Glu Gln Leu Leu Ala Glu Lys Ser Tyr Ile Leu Leu Ile
                   325                     330                     335
   Asp Asp Ile Trp Ser Ala Glu Thr Trp Glu Ser Ile Arg Ser Ile Leu
               340                     345                     350
   Pro Lys Asn Asn Lys Gly Gly Arg Ile Ile Val Thr Thr Arg Phe Gln
           355                     360                     365
   Ala Val Gly Ser Thr Cys Ser Pro Leu Glu Thr Asp Arg Leu His Thr
       370                     375                     380
   Val Asp Phe Leu Thr Asp Asp Glu Ser Gln Asn Leu Phe Asn Thr Ser
   385                     390                     395                     400
   Ile Cys Glu Ser Lys Ile Arg Lys Asp Ser Asn Lys Val Asp Glu Gln
                   405                     410                     415
   Val Pro Glu Glu Ile Trp Lys Ile Cys Gly Gly Leu Pro Leu Ala Ile
               420                     425                     430
   Val Thr Met Ala Gly Leu Val Ala Cys Asn Pro Arg Lys Ala Cys Cys
           435                     440                     445
   Asp Trp Ser Lys Leu Cys Lys Ser Leu Phe Pro Glu Gln Glu Thr Pro
       450                     455                     460
   Leu Thr Leu Asp Gly Val Thr Arg Ile Leu Asp Cys Cys Tyr Asn Asp
   465                     470                     475                     480
   Leu Pro Ala Asp Leu Lys Thr Cys Leu Leu Tyr Leu Ser Ile Phe Pro
                   485                     490                     495
   Lys Gly Trp Lys Ile Ser Arg Lys Arg Leu Ser Arg Arg Trp Ile Ala
               500                     505                     510
   Glu Gly Phe Ala Asn Glu Lys Gln Gly Leu Thr Gln Glu Arg Val Ala
           515                     520                     525
   Glu Ala Tyr Phe Asn Gln Leu Thr Arg Arg Asn Leu Val Arg Pro Met
       530                     535                     540
   Glu His Gly Ser Asn Gly Lys Val Lys Thr Phe Gln Val His Asp Met
   545                     550                     555                     560
   Val Leu Glu Tyr Ile Met Ser Lys Ser Ile Glu Glu Asn Phe Ile Thr
                   565                     570                     575
   Val Val Gly Gly His Trp Gln Met Thr Ala Pro Ser Asn Lys Val Arg
               580                     585                     590
   Arg Leu Ser Met Gln Ser Ser Gly Ser Asn Arg Gly Ser Ser Thr Lys
           595                     600                     605
   Gly Leu Asn Leu Ala Gln Val Arg Ser Leu Thr Val Phe Gly Asn Leu
       610                     615                     620
   Asn His Met Pro Phe His Ser Phe Asn Tyr Gly Ile Ile Gln Val Leu
   625                 630                 635                 640
   Asp Leu Glu Asp Trp Lys Gly Leu Lys Glu Arg His Met Thr Glu Ile
                   645                 650                 655
   Cys Gln Met Leu Leu Leu Lys Tyr Leu Ser Ile Arg Arg Thr Glu Ile
               660                 665                 670
   Ser Lys Ile Pro Ser Lys Ile Gln Lys Leu Glu Tyr Leu Glu Thr Leu
           675                 680                 685
   Asp Ile Arg Glu Thr Tyr Val Arg Asp Leu Pro Lys Ser Ile Val Gln
       690                 695                 700
   Leu Lys Arg Ile Ile Ser Ile Leu Gly Gly Asn Lys Asn Thr Arg Lys
   705                 710                 715                 720
   Gly Leu Arg Leu Pro Gln Glu Lys Ser Lys Lys Pro Ile Lys Asn Pro
                   725                 730                 735
   Ser Pro Gln Gly Lys Thr Lys Glu Pro Ala Lys Lys Gly Phe Leu Ser
               740                 745                 750
   Gln Glu Lys Gly Lys Gly Ala Met Lys Ala Leu Arg Val Leu Ser Gly
           755                 760                 765
   Ile Glu Ile Val Glu Glu Ser Ser Glu Val Ala Ala Gly Leu His Gln
       770                 775                 780
   Leu Thr Gly Leu Arg Lys Leu Ala Ile Tyr Lys Leu Asn Ile Thr Lys
   785                 790                 795                 800
   Gly Gly Asp Thr Phe Lys Gln Leu Gln Ser Ser Ile Glu Tyr Leu Gly
                   805                 810                 815
   Ser Cys Gly Leu Gln Thr Leu Ala Ile Asn Asp Glu Asn Ser Glu Phe
               820                 825                 830
   Ile Asn Ser Leu Gly Asp Met Pro Ala Pro Pro Arg Tyr Leu Val Ala
           835                 840                 845
   Leu Glu Leu Ser Gly Lys Leu Glu Lys Leu Pro Lys Trp Ile Thr Ser
       850                 855                 860
   Ile Thr Thr Leu Asn Lys Leu Thr Ile Ser Val Thr Val Leu Arg Thr
   865                 870                 875                 880
   Glu Thr Leu Glu Ile Leu His Ile Leu Pro Ser Leu Phe Ser Leu Thr
                   885                 890                 895
   Phe Ala Phe Ser Leu Ser Ala Ala Lys Gln Asp Gln Asp Ile Ile Lys
               900                 905                 910
   Asp Ile Leu Glu Asn Asn Lys Leu Asp Ser Asp Gly Glu Ile Val Ile
           915                 920                 925
   Pro Ala Glu Gly Phe Lys Ser Leu Lys Leu Leu Arg Phe Phe Ala Pro
       930                 935                 940
   Leu Val Pro Lys Leu Ser Phe Leu Asp Lys Asn Ala Met Pro Ala Leu
   945                 950                 955                 960
   Glu Ile Ile Glu Met Arg Phe Lys Asp Phe Glu Gly Leu Phe Gly Ile
                   965                 970                 975
   Glu Ile Leu Glu Asn Leu Arg Glu Val His Leu Lys Val Ser Asp Gly
               980                 985                 990
   Ala Glu Ala Ile Thr Lys Phe Leu Val Asn Asp Leu Lys Val Asn Thr
           995                 1000                1005
   Glu Lys Pro Lys Val Phe Val Asp Gly Ile Val Thr Ala
       1010                1015                1020

<210> 191
<211> 3230
<212> DNA
<213> Oryza sativa


<400> 191
atggagttgg tggtaggtgc ttccgaagcc accatgaaat ctctcttggg caagctgggc      60

aatcttctag cccaggagta tgctctcatc agcggtatcc gtggtgacat ccagtacatc     120

aatgacgagc ttgccagcat gcaggccttc ctccgtgatc tcagcaacgt gccagagggt     180

cacagtcatg gccaccggat gaaggactgg atgaagcaga tccgagacat cgcctatgat 240

gttgaggact gtatcgatga ctttgcccac cgcctccctc aggattccat cagcgatgcc 300

aaatggtcct tcctactcac aaaaatctat gaactatgga catggtggcc acgtcgtgtg 360

attgcttcca acattgccca actcaaggta cgggcacaac agatcgcaga tcgacgtagt 420

agatacggag tgaacaaccc agaacacctt gacagtagca gcagtgccag gacccgtgct 480

gtcaattacg aaattgctga gtatcaggtc acaagccctc agatcattgg tataaaggag 540

cctgtgggga tgaagacggt catggaggag cttgaggttt ggttaactaa tcctcaagct 600

gaaaatgggc aagctgttct gtccatagtc ggttttggag gtgtgggaaa gactaccatt 660

gccacagcat tgtacagaaa agtcagtgat aaatttcagt gccgggcatc agtagctgtg 720

tctcagaact atgaccaagg caaagtcctc aatagtattc tgagtcaagt cagcaatcag 780

gagcagggca gcagcacaac aattagtgag aaaaagaacc tcacctcagg cgctaagagc 840

atgttgaaga cagccctgtc actgctcaga ggtaattgta tatgtcagcc agaaaatgat 900

ggaaaccctg ataatacacc aatcaggctg caggaaacaa cggacgatga tcaaaacccc 960

agaaaactgg aacagctcct ggccgaaaag aggtaccttt ttttgtaaat aaaattgctt 1020

tgcttatctg taaattaact tactcatccc actctaaatc taatgtttat ttccttctat 1080

acacagcaca actccatctt ttgaatgggt tttatttttc tcacttgtgc tcattttttt 1140

tttatcatct ctgcagttat atcctcttga ttgatgacat ttggtctgcc gaaacatggg 1200

agagtatcag atcgattttg cctaaaaata ataaaggcgg tagaataata gtgactacaa 1260

gatttcaagc tgttggttca acatgctccc ctcttgaaac tgatcgtttg catacagttg 1320

attttctcac cgatgacgag tcccaaaact tattcaatac aagtatttgt gaatcaaaga 1380

taagaaaaga tagcaacaaa gtagacgagc aagtccctga ggaaatatgg aaaatatgtg 1440

ggggattgcc tttggccata gtcaccatgg ctggtcttgt cgcctgcaac ccaaggaaag 1500

cctgctgcga ttggagtaaa ctttgcaaat cattatttcc agagcaagaa actcctctta 1560

ccctcgatgg tgttacaagg atactggatt gttgttacaa tgatttgcct gcggatctga 1620

agacttgctt attgtacttg agtatatttc cgaagggttg gaaaattagt aggaaacgtt 1680

tgtcccggcg atggatagct gaaggttttg ctaatgagaa gcaaggttta acccaggaaa 1740

gagttgcaga ggcatacttt aatcaactca caagaaggaa cttagtacgt cccatggagc 1800

atggcagcaa tgggaaggta aaaacgtttc aagttcatga catggttctt gaatacatca 1860

tgtccaaatc aatcgaagag aattttatta ctgtggttgg tggacactgg cagatgactg 1920

caccaagcaa taaagtccgt cgactgtcga tgcaaagcag tggatccaat cgtggaagtt 1980

caacaaaagg cctgaacttg gctcaagtga gatcactgac ggtgtttggg aacctgaacc 2040

atatgccatt ccattcattc aactatggga taatacaggt gctggatctt gaggactgga 2100

agggtttgaa agagagacat atgacggaga tatgtcaaat gcttttactc aagtatttga 2160

gcatccgacg aacagaaatt tccaaaattc cctccaagat tcagaaactt gagtacttgg 2220

aaactcttga cataagggag acatatgtca gggacctgcc taagtcaata gtccagctaa 2280

aacggatcat tagcatactt ggagggaata aaaacacacg gaaggggctg aggttgcctc 2340

aagaaaaaag taagaagcca attaaaaacc cgtcgcctca aggaaaaaca aaggagcccg 2400

caaagaaagg attcttatcc caagaaaaag gtaaaggcgc aatgaaagca ctccgtgtac 2460

tgtcagggat tgagattgtt gaggaatcat cagaagtagc tgcaggcctt catcagttga 2520

cagggctaag gaagcttgcc atatacaagc tcaatataac aaagggtggt gataccttca 2580

aacaattaca gtcctccatt gagtaccttg gcagctgtgg tctgcagact ctggccatca 2640

atgatgagaa ttctgaattt atcaactcac tgggcgacat gcccgcgcct ccaagatatc 2700

ttgtcgccct tgagctgtct ggcaagttgg agaagctacc caagtggatc accagcatca 2760

ctactctcaa caagctaacc atatctgtaa cagttcttag gactgaaact ttggagatcc 2820

tccacatttt accttcattg ttttccctca ccttcgcctt ttcacttagt gcagcgaagc 2880

aggatcagga cataataaag gacatccttg agaataataa attggacagt gatggggaaa 2940

tcgtcattcc agctgaagga ttcaagagtc ttaagctgct tcgcttcttt gcacctttag 3000

tgccgaagct cagctttttg gacaagaatg caatgccagc actcgaaatc attgaaatgc 3060

ggtttaaaga cttcgaaggt ctatttggca tcgaaatcct tgaaaatctc cgtgaggtgc 3120

atctcaaagt tagtgatggg gcagaagcaa taaccaagtt ccttgtaaat gatttgaagg 3180

ttaatactga gaaaccaaaa gtatttgttg atggcatcgt cactgcatga 3230

<210> 192
<211> 3432
<212> DNA
<213> Artificial Sequence

<220>
<223> cDNA

<400> 192
atggaggcgg ctgccatggc cgtaaccgca gccacggggg ccttggcgcc cgtgctagtg 60

aagctggccg ctttgctgga cgacggggag tgcaatcttc tggaggggag ccggagcgac 120

gcagagttca tcagatccga gctggaggcc gtgcactctc tcctcacccc aaatatcttg 180

gggaggatgg gggatgacga tgcggcgtgc aaggatggct tgattgcgga ggtccgggag 240

ctgtcctacg acctggatga tgccgtcgac gacttcttgg agctcaattt cgagcagcga 300

agaagcgcaa gccctttcgg tgagctcaag gcaagagttg aggagcgtgt ctccaatcgc 360

ttctctgact ggaagctacc ggcggcgagc cttccgccgt cgtcggtaca ccgtcgagct 420

ggcttgccgc caccagatgc agggctggtg gggatgcaca aacgtaagga agagctcatc 480

gagttgctgg aacaagggag cagtgatgct tcacgatggc gcaagcgaaa acctcatgtc 540

cccctcagga taatggaggg ggaaatgcaa aaaatcgtgt tcaagattcc catggtggac 600

gataagagcc gtacaaaagc aatgtcattg gttgcaagta cggttggagt gcactcggtt 660

gcaatcgccg gtgacctaag agacgaggtt gtggtggtcg gtgatggcat tgactccatc 720

aatctggtct ctgcgctccg gaagaaggtg ggccctgcga tgtttctgga ggtcagccaa 780

gcaaaggaag atgtgaagga gataacggcg atgcttgcgc cggtgaaatc catatgtgaa 840

tttcacgagg tcaaaacaat ttgcatcctt ggattgccag ggggaggcaa aacaacgatt 900

gcccgagtac tatatcatgc attgggaacg cagttccaat gccgggtttt cgcatcaatc 960

tctccaagtt ccagccccag tcccaatcta acagagactc ttgcagacat tttcgctcaa 1020

gcacaactag gagtaactga tacacttagc acaccatatg gtgggagtgg gaccgggaga 1080

gctcttcaac aacatctcat cgacaacata tcagctttcc tcctcaacaa aaagtatctc 1140

attgtaatcg atgacatttg gcattgggaa gaatgggaag tcatcagaaa gtccattccc 1200

aagaatgatc tgggtggtag aataatcatg actactcgtc ttaattcaat agctgagaag 1260

tgccacactg atgacaatga tgtttttgtc tacgaagttg gggatctaga taataatgat 1320

gcttggtcgt tgtcttgggg gatagcaaca aagtctgggg caggcaacag gatcggaact 1380

ggagaggata attcatgcta tgatattgtg aacatgtgtt atggtatgcc tttagcactt 1440

atttggctgt cgtcagcatt ggttggagag atagaagaat taggtggtgc tgaagtgaaa 1500

aaatgtaggg atttgagaca catagaggat ggtattttgg acatcccatc cttacaacca 1560

ttggcggaga gtttatgcct tggttataac catcttcctc tttatctgag gactttgttg 1620

ttgtactgta gtgcatacca ttggtctaac agaatcgaaa ggggtcgtct ggtcaggagg 1680

tggattgcgg aaggatttgt gtcggaagag aaagaagcag aaggttactt tggcgagctt 1740

attaacagag gatggattac gcagcacgga gacaacaaca gttataatta ctatgagatc 1800

caccccgtga tgctggcctt cctgagatgc aagtccaagg agtacaattt tttaacatgc 1860

ttgggtctgg gatctgatac tagtactagt gcatcctccc caaggttgat tcgccggctg 1920

tctcttcagg gggggtatcc agtggactgc ttgtcaagca tgagtatgga tgtgtcacac 1980

acttgcagcc ttgtcgtcct tggtgacgtg gcgcgaccca agggaatccc cttctatatg 2040

tttaagcgct tgcgagtgtt ggaccttgaa gataataagg atatacagga ttctcatctg 2100

cagggcatat gtgaacagtt aagcctcaga gtgaggtacc ttggtctcaa gggaacgcgg 2160

atccgaaagc tccctcagga gatgaggaag ctgaagcatt tggagatttt gtatgtgggg 2220

agcactcgga tcagtgaact tccgcaagag attggagagc tgaagcatct gcggattctg 2280

gacgtgagaa acacggacat cactgagctc ccactgcaga tacgggagct gcagcatctg 2340

cacactctgg acgtgaggaa cactccaatc agtgagctcc cgccgcaggt tggcaagctg 2400

cagaatctca agattatgtg cgtgaggagc actggggtta gggagctccc aaaggagatt 2460

ggggagctga atcatctaca gactctggac gtgagaaaca cgagggtgag agagctgcca 2520

tggcaagctg gccagatctc ccaatcgttg cgcgtgcttg ccggtgacag tggcgatggc 2580

gtgcggttgc ccgaaggcgt ctgcgaagct ctgatcaacg gtattccagg ggctacgcgt 2640

gcaaaatgca gggaggttct gtccatcgcg atcatcgatc gtttcggacc tccccttgtt 2700

gggatattca aagttcccgg cagtcatatg cgtatcccga agatgatcaa agaccacttc 2760

cgcgttcttt cttgcctaga catcaggctc tgccacaagc ttgaggatga tgaccaaaag 2820

ttcctcgccg agatgcccaa cctgcagacg ctcgtgctga ggttcgaggc cctaccaaga 2880

caacccataa ccatcaacgg cacaggcttc cagatgctgg agagcttccg tgtcgacagc 2940

cgggtgccaa ggatagcctt ccatgaagac gccatgccca acctcaagct tctcgagttc 3000

aagttctacg ccggcccagc aagcaacgat gccatcggca tcaccaacct gaagagcctc 3060

caaaaggtgg tctttcggtg ctcgccatgg tacaagagcg acgcccctgg catcagcgcc 3120

accattgacg tcgtgaagaa agaagccgag gagcatccca accggccgat caccctcctc 3180

atcaatgctg ggtataagga gatatcaact gagtcacacg ggagcagtga aaacattgcg 3240

ggcagcagtg ggatcgatac tgagcctgca caggcacagc atgataatct ccctgctgtt 3300

cgagatgact acaagggaaa agggattctt cttgatggca ggtgtcctac ctgcggccga 3360

gcgactaaaa ttgaagagga aacccaagat cgagtagcag atattgaaat tcaaacagaa 3420

actactagct ag 3432

<210> 193
<211> 1143
<212> PRT
<213> Oryza sativa

<400> 193
Met Glu Ala Ala Ala Met Ala Val Thr Ala Ala Thr Gly Ala Leu Ala
1               5                   10                  15
Pro Val Leu Val Lys Leu Ala Ala Leu Leu Asp Asp Gly Glu Cys Asn
            20                  25                  30
Leu Leu Glu Gly Ser Arg Ser Asp Ala Glu Phe Ile Arg Ser Glu Leu
        35                  40                  45
Glu Ala Val His Ser Leu Leu Thr Pro Asn Ile Leu Gly Arg Met Gly
    50                  55                  60
Asp Asp Asp Ala Ala Cys Lys Asp Gly Leu Ile Ala Glu Val Arg Glu
65                  70                  75                  80
Leu Ser Tyr Asp Leu Asp Asp Ala Val Asp Asp Phe Leu Glu Leu Asn
                85                  90                  95
Phe Glu Gln Arg Arg Ser Ala Ser Pro Phe Gly Glu Leu Lys Ala Arg
            100                 105                 110

Val Glu Glu Arg Val Ser Asn Arg Phe Ser Asp Trp Lys Leu Pro Ala
        115                 120                 125
Ala Ser Leu Pro Pro Ser Ser Val His Arg Arg Ala Gly Leu Pro Pro
    130                 135                 140
Pro Asp Ala Gly Leu Val Gly Met His Lys Arg Lys Glu Glu Leu Ile
145                 150                 155                 160
Glu Leu Leu Glu Gln Gly Ser Ser Asp Ala Ser Arg Trp Arg Lys Arg
                165                 170                 175
Lys Pro His Val Pro Leu Arg Ile Met Gly Gly Glu Met Gln Lys Ile
            180                 185                 190
Val Phe Lys Ile Pro Met Val Asp Asp Lys Ser Arg Thr Lys Ala Met
        195                 200                 205
Ser Leu Val Ala Ser Thr Val Gly Val His Ser Val Ala Ile Ala Gly
    210                 215                 220
Asp Leu Arg Asp Glu Val Val Val Val Gly Asp Gly Ile Asp Ser Ile
225                 230                 235                 240
Asn Leu Val Ser Ala Leu Arg Lys Lys Val Gly Pro Ala Met Phe Leu
                245                 250                 255
Glu Val Ser Gln Ala Lys Glu Asp Val Lys Glu Ile Thr Ala Met Leu
            260                 265                 270
Ala Pro Val Lys Ser Ile Cys Glu Phe His Glu Val Lys Thr Ile Cys
        275                 280                 285
Ile Leu Gly Leu Pro Gly Gly Gly Lys Thr Thr Ile Ala Arg Val Leu
    290                 295                 300
Tyr His Ala Leu Gly Thr Gln Phe Gln Cys Arg Val Phe Ala Ser Ile
305                 310                 315                 320
Ser Pro Ser Ser Ser Pro Ser Pro Asn Leu Thr Glu Thr Leu Ala Asp
                325                 330                 335
Ile Phe Ala Gln Ala Gln Leu Gly Val Thr Asp Thr Leu Ser Thr Pro
            340                 345                 350
Tyr Gly Gly Ser Gly Thr Gly Arg Ala Leu Gln Gln His Leu Ile Asp
        355                 360                 365
Asn Ile Ser Ala Phe Leu Leu Asn Lys Lys Tyr Leu Ile Val Ile Asp
    370                 375                 380
Asp Ile Trp His Trp Glu Glu Trp Glu Val Ile Arg Lys Ser Ile Pro
385                 390                 395                 400
Lys Asn Asp Leu Gly Gly Arg Ile Ile Met Thr Thr Arg Leu Asn Ser
                405                 410                 415
Ile Ala Glu Lys Cys His Thr Asp Asp Asn Asp Val Phe Val Tyr Glu
            420                 425                 430
Val Gly Asp Leu Asp Asn Asn Asp Ala Trp Ser Leu Ser Trp Gly Ile
        435                 440                 445
Ala Thr Lys Ser Gly Ala Gly Asn Arg Ile Gly Thr Gly Glu Asp Asn
    450                 455                 460
Ser Cys Tyr Asp Ile Val Asn Met Cys Tyr Gly Met Pro Leu Ala Leu
465                 470                 475                 480
Ile Trp Leu Ser Ser Ala Leu Val Gly Glu Ile Glu Glu Leu Gly Gly
                485                 490                 495
Ala Glu Val Lys Lys Cys Arg Asp Leu Arg His Ile Glu Asp Gly Ile
            500                 505                 510
Leu Asp Ile Pro Ser Leu Gln Pro Leu Ala Glu Ser Leu Cys Leu Gly
        515                 520                 525
Tyr Asn His Leu Pro Leu Tyr Leu Arg Thr Leu Leu Leu Tyr Cys Ser
    530                 535                 540
Ala Tyr His Trp Ser Asn Arg Ile Glu Arg Gly Arg Leu Val Arg Arg
545                 550                 555                 560
Trp Ile Ala Glu Gly Phe Val Ser Glu Glu Lys Glu Ala Glu Gly Tyr
                565                 570                 575
Phe Gly Glu Leu Ile Asn Arg Gly Trp Ile Thr Gln His Gly Asp Asn
            580                 585                 590
Asn Ser Tyr Asn Tyr Tyr Glu Ile His Pro Val Met Leu Ala Phe Leu
        595                 600                 605
Arg Cys Lys Ser Lys Glu Tyr Asn Phe Leu Thr Cys Leu Gly Leu Gly

610                 615                 620
Ser Asp Thr Ser Thr Ser Ala Ser Ser Pro Arg Leu Ile Arg Arg Leu
625                 630                 635                 640
Ser Leu Gln Gly Gly Tyr Pro Val Asp Cys Leu Ser Ser Met Ser Met
                645                 650                 655
Asp Val Ser His Thr Cys Ser Leu Val Val Leu Gly Asp Val Ala Arg
            660                 665                 670
Pro Lys Gly Ile Pro Phe Tyr Met Phe Lys Arg Leu Arg Val Leu Asp
        675                 680                 685
Leu Glu Asp Asn Lys Asp Ile Gln Asp Ser His Leu Gln Gly Ile Cys
    690                 695                 700
Glu Gln Leu Ser Leu Arg Val Arg Tyr Leu Gly Leu Lys Gly Thr Arg
705                 710                 715                 720
Ile Arg Lys Leu Pro Gln Glu Met Arg Lys Leu Lys His Leu Glu Ile
                725                 730                 735
Leu Tyr Val Gly Ser Thr Arg Ile Ser Glu Leu Pro Gln Glu Ile Gly
            740                 745                 750
Glu Leu Lys His Leu Arg Ile Leu Asp Val Arg Asn Thr Asp Ile Thr
        755                 760                 765
Glu Leu Pro Leu Gln Ile Arg Glu Leu Gln His Leu His Thr Leu Asp
    770                 775                 780
Val Arg Asn Thr Pro Ile Ser Glu Leu Pro Pro Gln Val Gly Lys Leu
785                 790                 795                 800
Gln Asn Leu Lys Ile Met Cys Val Arg Ser Thr Gly Val Arg Glu Leu
                805                 810                 815
Pro Lys Glu Ile Gly Glu Leu Asn His Leu Gln Thr Leu Asp Val Arg
            820                 825                 830
Asn Thr Arg Val Arg Glu Leu Pro Trp Gln Ala Gly Gln Ile Ser Gln
        835                 840                 845
Ser Leu Arg Val Leu Ala Gly Asp Ser Gly Asp Gly Val Arg Leu Pro
    850                 855                 860
Glu Gly Val Cys Glu Ala Leu Ile Asn Gly Ile Pro Gly Ala Thr Arg
865                 870                 875                 880
Ala Lys Cys Arg Glu Val Leu Ser Ile Ala Ile Ile Asp Arg Phe Gly
                885                 890                 895
Pro Pro Leu Val Gly Ile Phe Lys Val Pro Gly Ser His Met Arg Ile
            900                 905                 910
Pro Lys Met Ile Lys Asp His Phe Arg Val Leu Ser Cys Leu Asp Ile
        915                 920                 925
Arg Leu Cys His Lys Leu Glu Asp Asp Asp Gln Lys Phe Leu Ala Glu
    930                 935                 940
Met Pro Asn Leu Gln Thr Leu Val Leu Arg Phe Glu Ala Leu Pro Arg
945                 950                 955                 960
Gln Pro Ile Thr Ile Asn Gly Thr Gly Phe Gln Met Leu Glu Ser Phe
                965                 970                 975
Arg Val Asp Ser Arg Val Pro Arg Ile Ala Phe His Glu Asp Ala Met
            980                 985                 990
Pro Asn Leu Lys Leu Leu Glu Phe Lys Phe Tyr Ala Gly Pro Ala Ser
        995                 1000                1005
Asn Asp Ala Ile Gly Ile Thr Asn Leu Lys Ser Leu Gln Lys Val Val
    1010                1015                1020
Phe Arg Cys Ser Pro Trp Tyr Lys Ser Asp Ala Pro Gly Ile Ser Ala
1025                1030                1035                1040
Thr Ile Asp Val Val Lys Lys Glu Ala Glu Glu His Pro Asn Arg Pro
                1045                1050                1055
Ile Thr Leu Leu Ile Asn Ala Gly Tyr Lys Glu Ile Ser Thr Glu Ser
            1060                1065                1070
His Gly Ser Ser Glu Asn Ile Ala Gly Ser Ser Gly Ile Asp Thr Glu
        1075                1080                1085
Pro Ala Gln Ala Gln His Asp Asn Leu Pro Ala Val Arg Asp Asp Tyr
    1090                1095                1100
Lys Gly Lys Gly Ile Leu Leu Asp Gly Arg Cys Pro Thr Cys Gly Arg
1105                1110                1115                1120

Ala Thr Lys Ile Glu Glu Glu Thr Gln Asp Arg Val Ala Asp Ile Glu
                1125                1130                1135
Ile Gln Thr Glu Thr Thr Ser
            1140

<210> 194
<211> 6319
<212> DNA
<213> Oryza sativa

<400> 194
atggaggcgg ctgccatggc cgtaaccgca gccacggggg ccttggcgcc cgtgctagtg      60

aagctggccg ctttgctgga cgacggggag tgcaatcttc tggaggggag ccggagcgac     120

gcagagttca tcagatccga gctggaggcc gtgcactctc tcctcacccc aaatatcttg     180

gggaggatgg gggatgacga tgcggcgtgc aaggatggct tgattgcgga ggtccgggag     240

ctgtcctacg acctggatga tgccgtcgac gacttcttgg agctcaattt cgagcagcga     300

agaagcgcaa gccctttcgg tgagctcaag gcaagagttg aggagcgtgt ctccaatcgc     360

ttctctgact ggaagctacc ggcggcgagc cttccgccgt cgtcggtaca ccgtcgagct     420

ggcttgccgc caccagatgc agggctggtg gggatgcaca aacgtaagga agagctcatc     480

gagttgctgg aacaagggag cagtgatgct tcacgatggc gcaagcgaaa acctcatgtc     540

cccctcagga taatggaggg ggaaatggta ccggtggatc tcgattcctc acaaatagct     600

tcgcttcctt ggatggcgct gccaataaat ttgtttactt tttctcttga ttcttcaact     660

tggaagtact tcattttttg ttgcagcaaa aaatcgtgtt caagattccc atggtggacg     720

ataagagccg tacaaaagca atgtcattgg ttgcaagtac ggttggagtg cactcggttg     780

caatcgccgg tgacctaaga gacgaggttg tggtggtcgg tgatggcatt gactccatca     840

atctggtctc tgcgctccgg aagaaggtgg gccctgcgat gtttctggag gtcagccaag     900

caaaggaaga tgtgaaggag ataacggcga tgcttgcgcc ggtgaaatcc atatgtgaat     960

ttcacgaggt caaaacaatt tgcatccttg gattgccagg gggaggcaaa acaacgattg    1020

cccgagtact atatcatgca ttgggaacgc agttccaatg ccgggttttc gcatcaatct    1080

ctccaagttc cagccccagt cccaatctaa cagagactct tgcagacatt ttcgctcaag    1140

cacaactagg agtaactgat acacttagca caccatatgg tgggagtggg accgggagag    1200

ctcttcaaca acatctcatc gacaacatat cagctttcct cctcaacaaa aagtaagcaa    1260

tatcttatat ctcttatcta gcttgtcctt aatccggctt cccaaattaa agtgaacagg    1320

ctatatgtcc atataatgtt gggttttttt tttctgtcct cgatgaggta tatatcaaga    1380

tatgccaaac tttttttaat tagcattact ttattctacc tagtaaatag atacagaaat    1440

ggggcaggcc cctcctttgt gacaggccaa tggttttatg atgtggaaac aactccctta    1500

tcactgaagc tcttgctatt actcgtgatg gtactgatct tgcaaaggct tgcagtattt    1560

gtaaatccac agggagtggt catctttttt ttttaagata atgaatagaa atctggcctc 1620

tatatagaaa gccaaaggtt acacaagtgc atacactcca aatctcaaag ctgagaaaca 1680

cgaaaaacaa acgaccaaaa agaaaagacg ataagattaa gtttcaagct tttcctcctt 1740

gcttcagaaa acagggaaat gcagagctag cccgctcttc ccctgccatg ccctctcact 1800

gccattgcaa ctaccacaaa agctcccctg tttcgccgtg agcgaaggga ggggatggaa 1860

ctagtctgct gctttgttgt cgccaagcac tggtttcatg ctgcccaaac aacctatgcc 1920

ccgctagcga taacgagtct gttgccgcca tgctgttgtc gctggagagc acttgtcgct 1980

gctcagctat gtgttaagta agctactaac aatctctcca ttctcttgta gactgcttat 2040

aaaagattgt acttcaactt tttattaatg aaatagagtt cctgccttac aacaacaaga 2100

acgagagaga gagagagagc caatatcatg agttagcact gtgaagactt cctgactgtt 2160

atgtcttcaa gaacagtata aaagcatttg ccactataaa aatatgatag ccctcaagat 2220

cattaaaaat atgtcatgtt cttttacctt cataacaacc agtatcacaa atatgaaaac 2280

atttaaagga aaatcagtat ttgccattta tgtaaagaaa aactagacta gaactctggg 2340

cttttatttt agtgcacact gtatacatgt gtgttctttg acgtatatac ttgatatttt 2400

ttcaaaaact tttgggggca tatctgtaac ccatgcccca tgcatccaaa gtagtagtga 2460

gacaactttc gtatcaatgg acatgtggca ttaaatttaa tcccaatatt tgttctctcg 2520

aagtcctgaa catataattg gacaaaatta tataatttaa ttaggccaat tgagccatat 2580

atacacacac tataagaaac ttctacaaaa caaacattcg ctccttttag gttttgacca 2640

gtctatacat gaaccagcca acggtgtgct ggtctgacta taaatgtttt tttagagagt 2700

aaaggcacct tccagtacct ctctcgagga cagtaaaaac acccatgctt taaggttatg 2760

aacaacaaag catagaacaa ccacaagaaa aaaaaacaac tagaacaagt tctggaacta 2820

gttagggatt cgggaagcca ttgcttgctg gcttgggaca agctatgcct gccattggac 2880

tatggtggag actgggcata aaggatctca atctcttgga gattggccta tgtcctaggt 2940

ggccatggct gactgcaagg agatatattt cttgcaacgg aaattttcat tgcaaaagct 3000

gaaaatcgtt gtgatatgta cctattgtga cataattgaa tccattggga gccgtagcaa 3060

taaatcccgt ggtaataggc tattgcaatg attttccgat ggttgcataa aaaggtgcta 3120

ttacaatggt tttgcattgc tattgcaaca acttgatatg tcataggtat ttattgcaac 3180

agtttgtacc atgctgcaat aagatttcgc aacacagacc tattcattaa atagcctatt 3240

tccacacatt catttttgtg gcaataattc agtgcgacaa gcatgttcta tagctataac 3300

ctgatgcaac gggttagact tgttgcagta acttattagc acatttaatt ttttactaaa 3360

tttatattga ttcaactatt tcaacatcat tcaatacagt aaatactgga aaatgcaagc 3420

aatctccaaa aattcaccaa taagagaaac gaattaaatc atatacacaa ttatttcaca 3480
ccgtacagtg cacttaagtg taggaaatat aatgctatat acatttccaa ccctcaatac 3540
aggttctgta aataatgaaa agaaacatca atttgatttc ttgaccggtc cctctgagac 3600
acacagtgct ttcctcagca acatcgttgc cgatctatgt aacttgtaaa ctccttgtgc 3660
ttaataaatt ctggccaggg ttacttcagc ctgcatggcg aaaaaaaaga acttgttagg 3720
gagatcgcaa acaaatgtta gctgccccat ttcttctcta ctgaccataa aagctctagg 3780
atcaggtcat gaaaacaatg ccgtcagacc ggaaatactc aagattcaga acacgactcc 3840
taggaactgc aagtttaaat ttcaacaaaa gcaaaataca accagatgac aaactaatat 3900
atatcagctg actgttcatc tcaagtggac tcacatctgt gaaattgtta gctctatgca 3960
tatatgctaa tgtaatacta atatgctatt gaggcttatt actcttctcg acttttatag 4020
gtatctcatt gtaatcgatg acatttggca ttgggaagaa tgggaagtca tcagaaagtc 4080
cattcccaag aatgatctgg gtggtagaat aatcatgact actcgtctta attcaatagc 4140
tgagaagtgc cacactgatg acaatgatgt ttttgtctac gaagttgggg atctagataa 4200
taatgatgct tggtcgttgt cttgggggat agcaacaaag tctggggcag gcaacaggat 4260
cggaactgga gaggataatt catgctatga tattgtgaac atgtgttatg gtatgccttt 4320
agcacttatt tggctgtcgt cagcattggt tggagagata gaagaattag gtggtgctga 4380
agtgaaaaaa tgtagggatt tgagacacat agaggatggt attttggaca tcccatcctt 4440
acaaccattg gcggagagtt tatgccttgg ttataaccat cttcctcttt atctgaggac 4500
tttgttgttg tactgtagtg cataccattg gtctaacaga atcgaaaggg gtcgtctggt 4560
caggaggtgg attgcggaag gatttgtgtc ggaagagaaa gaagcagaag gttactttgg 4620
cgagcttatt aacagaggat ggattacgca gcacggagac aacaacagtt ataattacta 4680
tgagatccac cccgtgatgc tggccttcct gagatgcaag tccaaggagt acaatttttt 4740
aacatgcttg ggtctgggat ctgatactag tactagtgca tcctccccaa ggttgattcg 4800
ccggctgtct cttcaggggg ggtatccagt ggactgcttg tcaagcatga gtatggatgt 4860
gtcacacact tgcagccttg tcgtccttgg tgacgtggcg cgacccaagg gaatcccctt 4920
ctatatgttt aagcgcttgc gagtgttgga ccttgaagat aataaggata tacaggattc 4980
tcatctgcag ggcatatgtg aacagttaag cctcagagtg aggtaccttg gtctcaaggg 5040
aacgcggatc cgaaagctcc ctcaggagat gaggaagctg aagcatttgg agattttgta 5100
tgtggggagc actcggatca gtgaacttcc gcaagagatt ggagagctga agcatctgcg 5160
gattctggac gtgagaaaca cggacatcac tgagctccca ctgcagatac gggagctgca 5220
gcatctgcac actctggacg tgaggaacac tccaatcagt gagctcccgc cgcaggttgg 5280
caagctgcag aatctcaaga ttatgtgcgt gaggagcact ggggttaggg agctcccaaa 5340

ggagattggg gagctgaatc atctacagac tctggacgtg agaaacacga gggtgagaga 5400

gctgccatgg caagctggcc agatctccca atcgttgcgc gtgcttgccg gtgacagtgg 5460

cgatggcgtg cggttgcccg aaggcgtctg cgaagctctg atcaacggta ttccaggggc 5520

tacgcgtgca aaatgcaggg aggttctgtc catcgcgatc atcgatcgtt tcggacctcc 5580

ccttgttggg atattcaaag ttcccggcag tcatatgcgt atcccgaaga tgatcaaaga 5640

ccacttccgc gttctttctt gcctagacat caggctctgc cacaagcttg aggatgatga 5700

ccaaaagttc ctcgccgaga tgcccaacct gcagacgctc gtgctgaggt tcgaggccct 5760

accaagacaa cccataacca tcaacggcac aggcttccag atgctggaga gcttccgtgt 5820

cgacagccgg gtgccaagga tagccttcca tgaagacgcc atgcccaacc tcaagcttct 5880

cgagttcaag ttctacgccg gcccagcaag caacgatgcc atcggcatca ccaacctgaa 5940

gagcctccaa aaggtggtct ttcggtgctc gccatggtac aagagcgacg cccctggcat 6000

cagcgccacc attgacgtcg tgaagaaaga agccgaggag catcccaacc ggccgatcac 6060

cctcctcatc aatgctgggt ataaggagat atcaactgag tcacacggga gcagtgaaaa 6120

cattgcgggc agcagtggga tcgatactga gcctgcacag gcacagcatg ataatctccc 6180

tgctgttcga gatgactaca agggaaaagg gattcttctt gatggcaggt gtcctacctg 6240

cggccgagcg actaaaattg aagaggaaac ccaagatcga gtagcagata ttgaaattca 6300

aacagaaact actagctag 6319

<210> 195
<211> 3066
<212> DNA
<213> Artificial Sequence

<220>
<223> cDNA

<400> 195
atggagttgg tggtaggtgc ttccgaagcc accatgaaat ctctcttggg caagctgggc 60

aatcttctag cccaggagta tgctctcatc agcggtatcc gtggtgacat ccagtacatc 120

aatgacgagc ttgccagcat gcaggccttc ctccgtgatc tcagcaacgt gccagagggt 180

cacagtcatg gccaccggat gaaggactgg atgaagcaga tccgagacat cgcctatgat 240

gttgaggact gtatcgatga ctttgcccac cgcctccctc aggattccat cagcgatgcc 300

aaatggtcct tcctactcac aaaaatctat gaactatgga catggtggcc acgtcgtgtg 360

attgcttcca acattgccca actcaaggta cgggcacaac agatcgcaga tcgacgtagt 420

agatacggag tgaacaaccc agaacacctt gacagtagca gcagtgccag gacccgtgct 480

gtcaattacg aaattgctga gtatcaggtc acaagccctc agatcattgg tataaaggag 540

cctgtgggga tgaagacggt catggaggag cttgaggttt ggttaactaa tcctcaagct 600
gaaaatgggc aagctgttct gtccatagtc ggttttggag gtgtgggaaa gactaccatt 660
gccacagcat tgtacagaaa agtcagtgaa aaatttcagt gccgggcatc agtagctgtg 720
tctcagaact atgaccaagg caaagtcctc aatagtattc tgagtcaagt cagcaatcag 780
gagcagggca gcagcacaac aattagtgag aaaaagaacc tcacctcagg cgctaagagc 840
atgttgaaga cagccctgtc actgctcaga ggtaattgta tatgtcagcc agaaaatgat 900
ggaaaccctg ataatacacc aatcaggctg caggaaacaa cggacgatga tcaaaacccc 960
agaaaactgg aacagctcct ggccgaaaag agttatatcc tcttgattga tgacatttgg 1020
tctgccgaaa catgggagag tatcagatcg attttgccta aaaataataa aggcggtaga 1080
ataatagtga ctacaagatt tcaagctgtt ggttcaacat gctcccctct tgaaactgat 1140
cgtttgcata cagttgattt tctcaccgat gacgagtccc aaaacttatt caatacaagt 1200
atttgtgaat caaagataag aaaagatagc aacaaagtag acgagcaagt ccctgaggaa 1260
atatggaaaa tatgtggggg attgcctttg gccatagtca gcatggctgg tcttgtcgcc 1320
tgcaacccaa ggaaagcctg ctgcgattgg agtaaacttt gcaaatcatt atttccagag 1380
caagaaactc ctcttaccct cgatggtgtt acaaggatac tggattgttg ttacaatgat 1440
ttgcctgcgg atctgaagac ttgcttattg tacttgagta tatttccgaa gggttggaaa 1500
attagtagga aacgtttgtc ccggcgatgg atagctgaag gttttgctaa tgagaagcaa 1560
gggttaaccc aggaaagagt tgcagaggca tactttaatc aactcacaag aaggaactta 1620
gtacgtccca tggagcatgg cagcaatggg aaggtaaaaa cgtttcaagt tcatgacatg 1680
gttcttgaat acatcatgtc caaatcaatc gaagagaatt ttattactgt ggttggtgga 1740
cactggcaga tgactgcacc aagcaataaa gtccgtcgac tgtcgatgca aagcagtgga 1800
tccaatcgtg gaagttcaac aaaaggcctg aacttggctc aagtgagatc actgacggtg 1860
tttgggaacc tgaaccatgt gccattccat tcattcaact atgggataat acaggtgctg 1920
gatcttgagg actggaaggg tttgaaagag agacatatga cggagatatg tcaaatgctt 1980
ttactcaagt atttgagcat ccgacgaaca gaaatttcca aaattccctc caagattcag 2040
aaacttgagt acttggaaac tcttgacata agggagacat atgtcaggga cctgcctaag 2100
tcaatagtcc agctaaaacg gatcattagc atacttggag ggaataaaaa cacacggaag 2160
gggctgaggt tgcctcaaga aaaaagtaag aagccaatta aaaacccgtc gcctcaagga 2220
aaaacaaagg agcccgcaaa gaaaggattc ttatcccaag aaaaaggtaa aggcgcaatg 2280
aaagcactcc gtgtactgtc agggattgag attgttgagg aatcatcaga agtagctgca 2340
ggccttcatc agttgacagg gctaaggaag cttgccatat acaagctcaa tataacaaag 2400

ggtggtgata ccttcaaaca attacagtcc tccattgagt accttggcag ctgtggtctg 2460

cagactctgg ccatcaatga tgagaattct gaatttatca actcactggg cgacatgccc 2520

gcgcctccaa gatatcttgt cgcccttgag ctgtctggca agttggagaa gctacccaag 2580

tggatcacca gcatcactac tctcaacaag ctaaccatat ctgtaacagt tcttaggact 2640

gaaactttgg agatcctcca cattttacct tcattgtttt ccctcacctt cgccttttca 2700

cttagtgcag cgaagcagga tcaggacata ataaaggaca tccttgagaa taataaattg 2760

gacagtgatg gggaaatcgt cattccagct gaaggattca agagtcttaa gctgcttcgc 2820

ttctttgcac ctttagtgcc gaagctcagc tttttggaca agaatgcaat gccagcactc 2880

gaaatcattg aaatgcggtt taaagacttc gaaggtctat ttggcatcga aatccttgaa 2940

aatctccgtg aggtgcatct caaagttagt gatggggcag aagcaataac caagttcctt 3000

gtaaatgatt tgaaggttaa tactgagaaa ccaaaagtat ttgttgatgg catcgtcact 3060

gcatga 3066

<210> 196
<211> 1021
<212> PRT
<213> Oryza sativa

<400> 196
Met Glu Leu Val Val Gly Ala Ser Glu Ala Thr Met Lys Ser Leu Leu
1               5                   10                  15
Gly Lys Leu Gly Asn Leu Leu Ala Gln Glu Tyr Ala Leu Ile Ser Gly
            20                  25                  30
Ile Arg Gly Asp Ile Gln Tyr Ile Asn Asp Glu Leu Ala Ser Met Gln
        35                  40                  45
Ala Phe Leu Arg Asp Leu Ser Asn Val Pro Glu Gly His Ser His Gly
    50                  55                  60
His Arg Met Lys Asp Trp Met Lys Gln Ile Arg Asp Ile Ala Tyr Asp
65                  70                  75                  80
Val Glu Asp Cys Ile Asp Asp Phe Ala His Arg Leu Pro Gln Asp Ser
                85                  90                  95
Ile Ser Asp Ala Lys Trp Ser Phe Leu Leu Thr Lys Ile Tyr Glu Leu
            100                 105                 110
Trp Thr Trp Trp Pro Arg Arg Val Ile Ala Ser Asn Ile Ala Gln Leu
        115                 120                 125
Lys Val Arg Ala Gln Gln Ile Ala Asp Arg Arg Ser Arg Tyr Gly Val
    130                 135                 140
Asn Asn Pro Glu His Leu Asp Ser Ser Ser Ser Ala Arg Thr Arg Ala
145                 150                 155                 160
Val Asn Tyr Glu Ile Ala Glu Tyr Gln Val Thr Ser Pro Gln Ile Ile
                165                 170                 175
Gly Ile Lys Glu Pro Val Gly Met Lys Thr Val Met Glu Glu Leu Glu
            180                 185                 190
Val Trp Leu Thr Asn Pro Gln Ala Glu Asn Gly Gln Ala Val Leu Ser
        195                 200                 205
Ile Val Gly Phe Gly Gly Val Gly Lys Thr Thr Ile Ala Thr Ala Leu
    210                 215                 220
Tyr Arg Lys Val Ser Glu Lys Phe Gln Cys Arg Ala Ser Val Ala Val
225                 230                 235                 240
Ser Gln Asn Tyr Asp Gln Gly Lys Val Leu Asn Ser Ile Leu Ser Gln

245                 250                 255
Val Ser Asn Gln Glu Gln Gly Ser Ser Thr Thr Ile Ser Glu Lys Lys
            260                 265                 270
Asn Leu Thr Ser Gly Ala Lys Ser Met Leu Lys Thr Ala Leu Ser Leu
        275                 280                 285
Leu Arg Gly Asn Cys Ile Cys Gln Pro Glu Asn Asp Gly Asn Pro Asp
    290                 295                 300
Asn Thr Pro Ile Arg Leu Gln Glu Thr Thr Asp Asp Asp Gln Asn Pro
305                 310                 315                 320
Arg Lys Leu Glu Gln Leu Leu Ala Glu Lys Ser Tyr Ile Leu Leu Ile
                325                 330                 335
Asp Asp Ile Trp Ser Ala Glu Thr Trp Glu Ser Ile Arg Ser Ile Leu
            340                 345                 350
Pro Lys Asn Asn Lys Gly Gly Arg Ile Ile Val Thr Thr Arg Phe Gln
        355                 360                 365
Ala Val Gly Ser Thr Cys Ser Pro Leu Glu Thr Asp Arg Leu His Thr
    370                 375                 380
Val Asp Phe Leu Thr Asp Asp Glu Ser Gln Asn Leu Phe Asn Thr Ser
385                 390                 395                 400
Ile Cys Glu Ser Lys Ile Arg Lys Asp Ser Asn Lys Val Asp Glu Gln
                405                 410                 415
Val Pro Glu Glu Ile Trp Lys Ile Cys Gly Gly Leu Pro Leu Ala Ile
            420                 425                 430
Val Ser Met Ala Gly Leu Val Ala Cys Asn Pro Arg Lys Ala Cys Cys
        435                 440                 445
Asp Trp Ser Lys Leu Cys Lys Ser Leu Phe Pro Glu Gln Glu Thr Pro
    450                 455                 460
Leu Thr Leu Asp Gly Val Thr Arg Ile Leu Asp Cys Cys Tyr Asn Asp
465                 470                 475                 480
Leu Pro Ala Asp Leu Lys Thr Cys Leu Leu Tyr Leu Ser Ile Phe Pro
                485                 490                 495
Lys Gly Trp Lys Ile Ser Arg Lys Arg Leu Ser Arg Arg Trp Ile Ala
            500                 505                 510
Glu Gly Phe Ala Asn Glu Lys Gln Gly Leu Thr Gln Glu Arg Val Ala
        515                 520                 525
Glu Ala Tyr Phe Asn Gln Leu Thr Arg Arg Asn Leu Val Arg Pro Met
    530                 535                 540
Glu His Gly Ser Asn Gly Lys Val Lys Thr Phe Gln Val His Asp Met
545                 550                 555                 560
Val Leu Glu Tyr Ile Met Ser Lys Ser Ile Glu Glu Asn Phe Ile Thr
                565                 570                 575
Val Val Gly Gly His Trp Gln Met Thr Ala Pro Ser Asn Lys Val Arg
            580                 585                 590
Arg Leu Ser Met Gln Ser Ser Gly Ser Asn Arg Gly Ser Ser Thr Lys
        595                 600                 605
Gly Leu Asn Leu Ala Gln Val Arg Ser Leu Thr Val Phe Gly Asn Leu
    610                 615                 620
Asn His Val Pro Phe His Ser Phe Asn Tyr Gly Ile Ile Gln Val Leu
625                 630                 635                 640
Asp Leu Glu Asp Trp Lys Gly Leu Lys Glu Arg His Met Thr Glu Ile
                645                 650                 655
Cys Gln Met Leu Leu Leu Lys Tyr Leu Ser Ile Arg Arg Thr Glu Ile
            660                 665                 670
Ser Lys Ile Pro Ser Lys Ile Gln Lys Leu Glu Tyr Leu Glu Thr Leu
        675                 680                 685
Asp Ile Arg Glu Thr Tyr Val Arg Asp Leu Pro Lys Ser Ile Val Gln
    690                 695                 700
Leu Lys Arg Ile Ile Ser Ile Leu Gly Gly Asn Lys Asn Thr Arg Lys
705                 710                 715                 720
Gly Leu Arg Leu Pro Gln Glu Lys Ser Lys Lys Pro Ile Lys Asn Pro
                725                 730                 735
Ser Pro Gln Gly Lys Thr Lys Glu Pro Ala Lys Lys Gly Phe Leu Ser
            740                 745                 750

Gln Glu Lys Gly Lys Gly Ala Met Lys Ala Leu Arg Val Leu Ser Gly
        755                 760                 765
Ile Glu Ile Val Glu Glu Ser Ser Glu Val Ala Ala Gly Leu His Gln
    770                 775                 780
Leu Thr Gly Leu Arg Lys Leu Ala Ile Tyr Lys Leu Asn Ile Thr Lys
785                 790                 795                 800
Gly Gly Asp Thr Phe Lys Gln Leu Gln Ser Ser Ile Glu Tyr Leu Gly
                805                 810                 815
Ser Cys Gly Leu Gln Thr Leu Ala Ile Asn Asp Glu Asn Ser Glu Phe
            820                 825                 830
Ile Asn Ser Leu Gly Asp Met Pro Ala Pro Pro Arg Tyr Leu Val Ala
        835                 840                 845
Leu Glu Leu Ser Gly Lys Leu Glu Lys Leu Pro Lys Trp Ile Thr Ser
    850                 855                 860
Ile Thr Thr Leu Asn Lys Leu Thr Ile Ser Val Thr Val Leu Arg Thr
865                 870                 875                 880
Glu Thr Leu Glu Ile Leu His Ile Leu Pro Ser Leu Phe Ser Leu Thr
                885                 890                 895
Phe Ala Phe Ser Leu Ser Ala Ala Lys Gln Asp Gln Asp Ile Ile Lys
            900                 905                 910
Asp Ile Leu Glu Asn Asn Lys Leu Asp Ser Asp Gly Glu Ile Val Ile
        915                 920                 925
Pro Ala Glu Gly Phe Lys Ser Leu Lys Leu Leu Arg Phe Phe Ala Pro
    930                 935                 940
Leu Val Pro Lys Leu Ser Phe Leu Asp Lys Asn Ala Met Pro Ala Leu
945                 950                 955                 960
Glu Ile Ile Glu Met Arg Phe Lys Asp Phe Glu Gly Leu Phe Gly Ile
                965                 970                 975
Glu Ile Leu Glu Asn Leu Arg Glu Val His Leu Lys Val Ser Asp Gly
            980                 985                 990
Ala Glu Ala Ile Thr Lys Phe Leu Val Asn Asp Leu Lys Val Asn Thr
        995                 1000                1005
Glu Lys Pro Lys Val Phe Val Asp Gly Ile Val Thr Ala
    1010                1015                1020

<210> 197
<211> 3229
<212> DNA
<213> Oryza sativa


<400> 197
atggagttgg tggtaggtgc ttccgaagcc accatgaaat ctctcttggg caagctgggc      60

aatcttctag cccaggagta tgctctcatc agcggtatcc gtggtgacat ccagtacatc     120

aatgacgagc ttgccagcat gcaggccttc ctccgtgatc tcagcaacgt gccagagggt     180

cacagtcatg gccaccggat gaaggactgg atgaagcaga tccgagacat cgcctatgat     240

gttgaggact gtatcgatga ctttgcccac cgcctccctc aggattccat cagcgatgcc     300

aaatggtcct tcctactcac aaaaatctat gaactatgga catggtggcc acgtcgtgtg     360

attgcttcca acattgccca actcaaggta cgggcacaac agatcgcaga tcgacgtagt     420

agatacggag tgaacaaccc agaacacctt gacagtagca gcagtgccag gacccgtgct     480

gtcaattacg aaattgctga gtatcaggtc acaagccctc agatcattgg tataaaggag     540

cctgtgggga tgaagacggt catggaggag cttgaggttt ggttaactaa tcctcaagct     600

gaaaatgggc aagctgttct gtccatagtc ggttttggag gtgtgggaaa gactaccatt     660

gccacagcat tgtacagaaa agtcagtgaa aaatttcagt gccgggcatc agtagctgtg 720

tctcagaact atgaccaagg caaagtcctc aatagtattc tgagtcaagt cagcaatcag 780

gagcagggca gcagcacaac aattagtgag aaaaagaacc tcacctcagg cgctaagagc 840

atgttgaaga cagccctgtc actgctcaga ggtaattgta tatgtcagcc agaaaatgat 900

ggaaaccctg ataatacacc aatcaggctg caggaaacaa cggacgatga tcaaaacccc 960

agaaaactgg aacagctcct ggccgaaaag aggtaccttt ttttgtaaat aaaattgctt 1020

tgcttatctg taaattaact tactcatccc actctaaatc taatgtttat ttccttctat 1080

acacagcaca actccatctt ttgaatgggt tttatttttc tcacttgtgc tcattttttt 1140

ttatcatctc tgcagttata tcctcttgat tgatgacatt tggtctgccg aaacatggga 1200

gagtatcaga tcgattttgc ctaaaaataa taaaggcggt agaataatag tgactacaag 1260

atttcaagct gttggttcaa catgctcccc tcttgaaact gatcgtttgc atacagttga 1320

ttttctcacc gatgacgagt cccaaaactt attcaataca agtatttgtg aatcaaagat 1380

aagaaaagat agcaacaaag tagacgagca agtccctgag gaaatatgga aaatatgtgg 1440

gggattgcct ttggccatag tcagcatggc tggtcttgtc gcctgcaacc caaggaaagc 1500

ctgctgcgat tggagtaaac tttgcaaatc attatttcca gagcaagaaa ctcctcttac 1560

cctcgatggt gttacaagga tactggattg ttgttacaat gatttgcctg cggatctgaa 1620

gacttgctta ttgtacttga gtatatttcc gaagggttgg aaaattagta ggaaacgttt 1680

gtcccggcga tggatagctg aaggttttgc taatgagaag caagggttaa cccaggaaag 1740

agttgcagag gcatacttta atcaactcac aagaaggaac ttagtacgtc ccatggagca 1800

tggcagcaat gggaaggtaa aaacgtttca agttcatgac atggttcttg aatacatcat 1860

gtccaaatca atcgaagaga attttattac tgtggttggt ggacactggc agatgactgc 1920

accaagcaat aaagtccgtc gactgtcgat gcaaagcagt ggatccaatc gtggaagttc 1980

aacaaaaggc ctgaacttgg ctcaagtgag atcactgacg gtgtttggga acctgaacca 2040

tgtgccattc cattcattca actatgggat aatacaggtg ctggatcttg aggactggaa 2100

gggtttgaaa gagagacata tgacggagat atgtcaaatg cttttactca agtatttgag 2160

catccgacga acagaaattt ccaaaattcc ctccaagatt cagaaacttg agtacttgga 2220

aactcttgac ataaggagga catatgtcag ggacctgcct aagtcaatag tccagctaaa 2280

acggatcatt agcatacttg gagggaataa aaacacacgg aaggggctga ggttgcctca 2340

agaaaaaagt aagaagccaa ttaaaaaccc gtcgcctcaa ggaaaaacaa aggagcccgc 2400

aaagaaagga ttcttatccc aagaaaaagg taaaggcgca atgaaagcac tccgtgtact 2460

gtcagggatt gagattgttg aggaatcatc agaagtagct gcaggccttc atcagttgac 2520

agggctaagg aagcttgcca tatacaagct caatataaca aagggtggtg ataccttcaa 2580

acaattacag tcctccattg agtaccttgg cagctgtggt ctgcagactc tggccatcaa 2640

tgatgagaat tctgaattta tcaactcact gggcgacatg cccgcgcctc caagatatct 2700

tgtcgccctt gagctgtctg gcaagttgga gaagctaccc aagtggatca ccagcatcac 2760

tactctcaac aagctaacca tatctgtaac agttcttagg actgaaactt tggagatcct 2820

ccacatttta ccttcattgt tttccctcac cttcgccttt tcacttagtg cagcgaagca 2880

ggatcaggac ataataaagg acatccttga gaataataaa ttggacagtg atggggaaat 2940

cgtcattcca gctgaaggat tcaagagtct taagctgctt cgcttctttg cacctttagt 3000

gccgaagctc agctttttgg acaagaatgc aatgccagca ctcgaaatca ttgaaatgcg 3060

gtttaaagac ttcgaaggtc tatttggcat cgaaatcctt gaaaatctcc gtgaggtgca 3120

tctcaaagtt agtgatgggg cagaagcaat aaccaagttc cttgtaaatg atttgaaggt 3180

taatactgag aaaccaaaag tatttgttga tggcatcgtc actgcatga 3229

<210> 198
<211> 3690
<212> DNA
<213> Artificial Sequence

<220>
<223> cDNA

<400> 198
atggctgcag cactggtcgg tggtgccttt ctctctgctt tccttgatgt gcttttcgac 60

aggctggctt cacctgactt tgttgacttg atccttggaa agaagcttag caagaagttg 120

cttcgaaagt tggagaccac tctcagagtg gttggagctg tgcttgatga tgccgagaag 180

aaacagatca caaacaccaa tgtcaaacac tggctcaatg ctctcaaaga tgctgtctat 240

gaagccgatg acttactcga ccatgttttc accaaagctg ccacccaaaa caaggtaaga 300

aacttgtttt ctcgcttttc cgatcggaag atcgttagta agttggagga catagttgtc 360

accctagagt ctcatttaaa actcaaggag agtcttgatt tgaaagagag tgcagtggag 420

aacttgtcat ggaaagctcc atcaacatct ctggaagatg gatctcatat atatggtagg 480

gagaaagata gggaggccat aatcaagttg ttgtcggagg ataacagtga cggtagtgaa 540

gtgtctgtgg ttcctattgt gggcatgggt ggggttggaa aaactacttt ggcccaattg 600

gtgtacaatg atgagaattt gaaagagaaa tttgattttg attttaaggc atgggtttgt 660

gtttctcaag aatttgatgt tctgaaggtc acaaaaacta taatacaggc ggttactgga 720

aatccttgta aattgaatga tctgaatcta cttcatcttg aattgatgga caagctgaaa 780

gataaaaaat tcctaattgt tttggatgat gtttggacag aggattatgt tgattggagt 840

cttcttaaga aaccatttca atgtgggatt attaggagaa gtaaaattct tctaacaacc 900

cgcagtgaaa agacagcctc cgtagtccaa acggttcaaa cctatcatct aaaccaattg 960

tcgaatgaag attgttggtc agtgtttgcg aaccatgcgt gtctttcctt ggaatcgaac 1020

gagaacacaa cactagaaaa aattggaaag gagattgtta aaaagtgcga tggactgcct 1080

ttagcagcac agtcccttgg aggcatgttg agaagaaagc atgacattgg ggattggtat 1140

aatattctga atagtgacat ttgggaactt tctgaaagtg agtgtaaagt tattccagca 1200

ctgagactta gttatcatta tctccctcca catttaaaac ggtgctttgt ttattgttcg 1260

ttgtatcccc aagattacga atttgataaa aatgaattaa tcttgttgtg gatggctgaa 1320

gatcttttga agaaaccaag gaaaggtagg actttagaag aggttggtca tgagtatttt 1380

gatgatttgg tttcgagatc attttttcaa cgttcaagta caaatagaag tagttggcct 1440

tatggtgaat gttttgtgat gcatgacctc atgcatgatc tagccaaatc actcggtgga 1500

gatttttatt ttagatcaga agaacttggg aaagaaacaa agatcaatac taagactcgt 1560

catttgtcat ttaccaaatt caattcttca gtcttggaca actttgatgt tgttggtaga 1620

gcaaaatttc tgagaacttt cttgtccatt atcaattttg aagctgctcc attcaacaac 1680

gaggaggcac aatgtatcat tgtgtcgaag cttatgtact tgagagtttt atcattttgt 1740

gacttccaaa gtttggattc tttgcctgat tcaataggta aattgatcca tctgcgctat 1800

ttagatcttt ctcattcaag tgtagaaaca ctgccaaagt cattgtgtaa tttatacaat 1860

ctgcaaactt tgaagttgtg tagttgcaga aagctgacta agttgcctag tgacatgtgc 1920

aatcttgtta acttgcgtca tcttgagata cgtgaaactc ctatagaaga gatgccgaga 1980

ggaatgagta aattaaatca tctacaacat ctggatttct ttgttgtggg caagcacaaa 2040

gagaatggga tcaaagaatt gggaggactt tcaaatcttc gtggtcgact taaaattagg 2100

aacttggaga atgtttccca aagtgatgaa gcgtcggagg caaggatgat ggataaaaaa 2160

cacattaata gtttatggtt ggaatggtct agatgtaata acaacagtac caacttccaa 2220

ctagaaatag atgtgctttg caagttacag cctcacttta acattgaatc gttgagaata 2280

aaaggctata aaggaaccag atttccagat tggatgggaa attcttccta ctgcaatatg 2340

atgagtctaa aattgcgtga ttgtgacaac tgtagtatgc ttccttcact tggacaacta 2400

ccttctctca aggtccttaa gattgcacga ttgaataggc tgaagactat tgatgcaggt 2460

ttttacaaga acgaagattg tcgttctggg acgccctttc cctcccttga atctctggcc 2520

attcatcaaa tgccttgttg ggaggtgtgg agttccttcg attcagaagc ttttcctgtg 2580

cttgaaattc ttgaaatacg tgactgcccc aaactagagg gaagtttgcc gaatcacctt 2640

cctgctctga aaacacttac aattagaaat tgtgagctgc ttggctcttc tctcccaacg 2700

gctcccgcca ttcaaagttt ggagatacgt aaaagcaata aagtagcact gcatgcgttt 2760

cctctcttgg tagaaactat aaaagtagaa ggaagcccaa tggtggagtc catgatggag 2820

gccatcacaa acatccaacc aacttgtctc cggtctttaa cattaaggga ttgctcgtca 2880

gccgtgtcat ttccgggtgg tcgtttacct gaatcactga agagtctgta tatctcggat 2940

cttaaaaaac tggaattccc gacgcaacac aaacatgagt tactggaaac actgtcaata 3000

gaaagcagtt gtgattcact cacatctctt ccattggtta cctttccaaa tctcagagat 3060

cttgaaatca gaaactgtga aaatatggaa tctcttttgg tatcattctg gagagaagga 3120

ttgcctgcgc ccaacttgat tactttccaa gtgtggggct ctgacaagtt gaagtcgttg 3180

cctgatgaga tgagtactct tctcccaaag ttagaacgtc tcctcatatc caactgccca 3240

gaaattgagt cgtttccaaa acggggtatg ccacctaacc tgagaatagt ttggattttc 3300

aattgtgaga aactactgag cagcctagca tggccatcca tgggcatgct tactcatctc 3360

tatgttgggg gtcgatgtga tggcatcaag tccttcccta aggagggttt gctgcctccc 3420

tcccttacgt atctgtatct aagtggattc tcaaatctgg agatgttgga ctgcacgggg 3480

cttctccatc tcacatccct gcaacaatta accatagacg gatgtccttt gctggaaaat 3540

atggtgggag aaaggcttcc tgactctcta ataaaattaa ccataaagag ttgtcctttg 3600

ctgaaaaaac gatgccggaa gaagcaccct caaatttggc ctaaaatttc ccacatccct 3660

ggcattaagg ttgacaatag atggatttag 3690

<210> 199
<211> 1229
<212> PRT
<213> Glycine max

<400> 199
Met Ala Ala Ala Leu Val Gly Gly Ala Phe Leu Ser Ala Phe Leu Asp
1               5                   10                  15
Val Leu Phe Asp Arg Leu Ala Ser Pro Asp Phe Val Asp Leu Ile Leu
            20                  25                  30
Gly Lys Lys Leu Ser Lys Lys Leu Leu Arg Lys Leu Glu Thr Thr Leu
        35                  40                  45
Arg Val Val Gly Ala Val Leu Asp Asp Ala Glu Lys Lys Gln Ile Thr
    50                  55                  60
Asn Thr Asn Val Lys His Trp Leu Asn Ala Leu Lys Asp Ala Val Tyr
65                  70                  75                  80
Glu Ala Asp Asp Leu Leu Asp His Val Phe Thr Lys Ala Ala Thr Gln
                85                  90                  95
Asn Lys Val Arg Asn Leu Phe Ser Arg Phe Ser Asp Arg Lys Ile Val
            100                 105                 110
Ser Lys Leu Glu Asp Ile Val Val Thr Leu Glu Ser His Leu Lys Leu
        115                 120                 125
Lys Glu Ser Leu Asp Leu Lys Glu Ser Ala Val Glu Asn Leu Ser Trp
    130                 135                 140
Lys Ala Pro Ser Thr Ser Leu Glu Asp Gly Ser His Ile Tyr Gly Arg
145                 150                 155                 160
Glu Lys Asp Arg Glu Ala Ile Ile Lys Leu Leu Ser Glu Asp Asn Ser
                165                 170                 175

Asp Gly Ser Glu Val Ser Val Val Pro Ile Val Gly Met Gly Gly Val
            180                 185                 190
Gly Lys Thr Thr Leu Ala Gln Leu Val Tyr Asn Asp Glu Asn Leu Lys
        195                 200                 205
Glu Lys Phe Asp Phe Asp Phe Lys Ala Trp Val Cys Val Ser Gln Glu
    210                 215                 220
Phe Asp Val Leu Lys Val Thr Lys Thr Ile Ile Gln Ala Val Thr Gly
225                 230                 235                 240
Asn Pro Cys Lys Leu Asn Asp Leu Asn Leu Leu His Leu Glu Leu Met
                245                 250                 255
Asp Lys Leu Lys Asp Lys Lys Phe Leu Ile Val Leu Asp Asp Val Trp
            260                 265                 270
Thr Glu Asp Tyr Val Asp Trp Ser Leu Leu Lys Lys Pro Phe Gln Cys
        275                 280                 285
Gly Ile Ile Arg Arg Ser Lys Ile Leu Leu Thr Thr Arg Ser Glu Lys
    290                 295                 300
Thr Ala Ser Val Val Gln Thr Val Gln Thr Tyr His Leu Asn Gln Leu
305                 310                 315                 320
Ser Asn Glu Asp Cys Trp Ser Val Phe Ala Asn His Ala Cys Leu Ser
                325                 330                 335
Leu Glu Ser Asn Glu Asn Thr Thr Leu Glu Lys Ile Gly Lys Glu Ile
            340                 345                 350
Val Lys Lys Cys Asp Gly Leu Pro Leu Ala Ala Gln Ser Leu Gly Gly
        355                 360                 365
Met Leu Arg Arg Lys His Asp Ile Gly Asp Trp Tyr Asn Ile Leu Asn
    370                 375                 380
Ser Asp Ile Trp Glu Leu Ser Glu Ser Glu Cys Lys Val Ile Pro Ala
385                 390                 395                 400
Leu Arg Leu Ser Tyr His Tyr Leu Pro Pro His Leu Lys Arg Cys Phe
                405                 410                 415
Val Tyr Cys Ser Leu Tyr Pro Gln Asp Tyr Glu Phe Asp Lys Asn Glu
            420                 425                 430
Leu Ile Leu Leu Trp Met Ala Glu Asp Leu Leu Lys Lys Pro Arg Lys
        435                 440                 445
Gly Arg Thr Leu Glu Glu Val Gly His Glu Tyr Phe Asp Asp Leu Val
    450                 455                 460
Ser Arg Ser Phe Phe Gln Arg Ser Ser Thr Asn Arg Ser Ser Trp Pro
465                 470                 475                 480
Tyr Gly Glu Cys Phe Val Met His Asp Leu Met His Asp Leu Ala Lys
                485                 490                 495
Ser Leu Gly Gly Asp Phe Tyr Phe Arg Ser Glu Glu Leu Gly Lys Glu
            500                 505                 510
Thr Lys Ile Asn Thr Lys Thr Arg His Leu Ser Phe Thr Lys Phe Asn
        515                 520                 525
Ser Ser Val Leu Asp Asn Phe Asp Val Val Gly Arg Ala Lys Phe Leu
    530                 535                 540
Arg Thr Phe Leu Ser Ile Ile Asn Phe Glu Ala Ala Pro Phe Asn Asn
545                 550                 555                 560
Glu Glu Ala Gln Cys Ile Ile Val Ser Lys Leu Met Tyr Leu Arg Val
                565                 570                 575
Leu Ser Phe Cys Asp Phe Gln Ser Leu Asp Ser Leu Pro Asp Ser Ile
            580                 585                 590
Gly Lys Leu Ile His Leu Arg Tyr Leu Asp Leu Ser His Ser Ser Val
        595                 600                 605
Glu Thr Leu Pro Lys Ser Leu Cys Asn Leu Tyr Asn Leu Gln Thr Leu
    610                 615                 620
Lys Leu Cys Ser Cys Arg Lys Leu Thr Lys Leu Pro Ser Asp Met Cys
625                 630                 635                 640
Asn Leu Val Asn Leu Arg His Leu Glu Ile Arg Glu Thr Pro Ile Glu
                645                 650                 655
Glu Met Pro Arg Gly Met Ser Lys Leu Asn His Leu Gln His Leu Asp
            660                 665                 670
Phe Phe Val Val Gly Lys His Lys Glu Asn Gly Ile Lys Glu Leu Gly

675                 680                 685
Gly Leu Ser Asn Leu Arg Gly Arg Leu Lys Ile Arg Asn Leu Glu Asn
    690                 695                 700
Val Ser Gln Ser Asp Glu Ala Ser Glu Ala Arg Met Met Asp Lys Lys
705                 710                 715                 720
His Ile Asn Ser Leu Trp Leu Glu Trp Ser Arg Cys Asn Asn Asn Ser
                725                 730                 735
Thr Asn Phe Gln Leu Glu Ile Asp Val Leu Cys Lys Leu Gln Pro His
            740                 745                 750
Phe Asn Ile Glu Ser Leu Arg Ile Lys Gly Tyr Lys Gly Thr Arg Phe
        755                 760                 765
Pro Asp Trp Met Gly Asn Ser Ser Tyr Cys Asn Met Met Ser Leu Lys
    770                 775                 780
Leu Arg Asp Cys Asp Asn Cys Ser Met Leu Pro Ser Leu Gly Gln Leu
785                 790                 795                 800
Pro Ser Leu Lys Val Leu Lys Ile Ala Arg Leu Asn Arg Leu Lys Thr
                805                 810                 815
Ile Asp Ala Gly Phe Tyr Lys Asn Glu Asp Cys Arg Ser Gly Thr Pro
            820                 825                 830
Phe Pro Ser Leu Glu Ser Leu Ala Ile His Gln Met Pro Cys Trp Glu
        835                 840                 845
Val Trp Ser Ser Phe Asp Ser Glu Ala Phe Pro Val Leu Glu Ile Leu
    850                 855                 860
Glu Ile Arg Asp Cys Pro Lys Leu Glu Gly Ser Leu Pro Asn His Leu
865                 870                 875                 880
Pro Ala Leu Lys Thr Leu Thr Ile Arg Asn Cys Glu Leu Leu Gly Ser
                885                 890                 895
Ser Leu Pro Thr Ala Pro Ala Ile Gln Ser Leu Glu Ile Arg Lys Ser
            900                 905                 910
Asn Lys Val Ala Leu His Ala Phe Pro Leu Leu Val Glu Thr Ile Lys
        915                 920                 925
Val Glu Gly Ser Pro Met Val Glu Ser Met Met Glu Ala Ile Thr Asn
    930                 935                 940
Ile Gln Pro Thr Cys Leu Arg Ser Leu Thr Leu Arg Asp Cys Ser Ser
945                 950                 955                 960
Ala Val Ser Phe Pro Gly Gly Arg Leu Pro Glu Ser Leu Lys Ser Leu
                965                 970                 975
Tyr Ile Ser Asp Leu Lys Lys Leu Glu Phe Pro Thr Gln His Lys His
            980                 985                 990
Glu Leu Leu Glu Thr Leu Ser Ile Glu Ser Ser Cys Asp Ser Leu Thr
        995                 1000                1005
Ser Leu Pro Leu Val Thr Phe Pro Asn Leu Arg Asp Leu Glu Ile Arg
    1010                1015                1020
Asn Cys Glu Asn Met Glu Ser Leu Leu Val Ser Phe Trp Arg Glu Gly
1025                1030                1035                1040
Leu Pro Ala Pro Asn Leu Ile Thr Phe Gln Val Trp Gly Ser Asp Lys
                1045                1050                1055
Leu Lys Ser Leu Pro Asp Glu Met Ser Thr Leu Leu Pro Lys Leu Glu
            1060                1065                1070
Arg Leu Leu Ile Ser Asn Cys Pro Glu Ile Glu Ser Phe Pro Lys Arg
        1075                1080                1085
Gly Met Pro Pro Asn Leu Arg Ile Val Trp Ile Phe Asn Cys Glu Lys
    1090                1095                1100
Leu Leu Ser Ser Leu Ala Trp Pro Ser Met Gly Met Leu Thr His Leu
1105                1110                1115                1120
Tyr Val Gly Gly Arg Cys Asp Gly Ile Lys Ser Phe Pro Lys Glu Gly
                1125                1130                1135
Leu Leu Pro Pro Ser Leu Thr Tyr Leu Tyr Leu Ser Gly Phe Ser Asn
            1140                1145                1150
Leu Glu Met Leu Asp Cys Thr Gly Leu Leu His Leu Thr Ser Leu Gln
        1155                1160                1165
Gln Leu Thr Ile Asp Gly Cys Pro Leu Leu Glu Asn Met Val Gly Glu
    1170                1175                1180

Arg Leu Pro Asp Ser Leu Ile Lys Leu Thr Ile Lys Ser Cys Pro Leu
1185                1190                1195                1200
Leu Lys Lys Arg Cys Arg Lys Lys His Pro Gln Ile Trp Pro Lys Ile
                1205                1210                1215
Ser His Ile Pro Gly Ile Lys Val Asp Asn Arg Trp Ile
            1220                1225

<210> 200
<211> 840
<212> DNA
<213> Artificial Sequence

<220>
<223> cDNA

<400> 200
atgatgcaat ggagcgcaat cctcatccgc acttgtttca gtggcagtgg aggcgaggct      60

ctcacttgcg ccacctccga gcaacagaca cgacctgaat tatgtttctt cttcagcgtg     120

cgttcctcct ggccatcgac catctccgat ggtgcctgtt tggcactcgt atcggccgaa     180

caaggcgcca cggccgggcg caatacctta tccctgcgat cgatgatggc tactgaagac     240

atggcaacct cgacaagatc actacgatct caagctacga acgtggacga cgatgctaac     300

gtttcgattg aaaacagagg gatgaaccct tcagtcctga ccaagctcgg cgaatttgct     360

tcgacgttga cggctggcaa tacggcgaac aagttatggc tgatggctga cgtggaccca     420

aagtccgcgt tcaagttgct gggtcttgac atgccagggg tccgctttat cgacaacccg     480

aagatgttac agtggctcaa gttcacaaaa gcgtatttag acatgaaaaa gtcggggttt     540

ggcgagacaa gcgcacatgc tcttctgtat gaaaaaattg gaggacccga cttgtcacta     600

cttctcctaa gcctgaagga cgctccggat gcgaacagct tggttcaaaa actgacgaat     660

tctcagtttg gtatgtggca tgacgctcga atcgaaccag aacagctcgc acaaacggtg     720

ttcaaaattc aagacgtcag gaaactacct aaaaacgacc caaaacttca agttatcgat     780

gactacgcga agtatcacag aaagcaccgg aagtttctga acagcatcat gattatctga     840

<210> 201
<211> 279
<212> PRT
<213> Phytophthora sojae

<400> 201
Met Met Gln Trp Ser Ala Ile Leu Ile Arg Thr Cys Phe Ser Gly Ser
1               5                   10                  15
Gly Gly Glu Ala Leu Thr Cys Ala Thr Ser Glu Gln Gln Thr Arg Pro
            20                  25                  30
Glu Leu Cys Phe Phe Phe Ser Val Arg Ser Ser Trp Pro Ser Thr Ile
        35                  40                  45
Ser Asp Gly Ala Cys Leu Ala Leu Val Ser Ala Glu Gln Gly Ala Thr
    50                  55                  60
Ala Gly Arg Asn Thr Leu Ser Leu Arg Ser Met Met Ala Thr Glu Asp
65                  70                  75                  80

Met Ala Thr Ser Thr Arg Ser Leu Arg Ser Gln Ala Thr Asn Val Asp
                85                  90                  95
Asp Asp Ala Asn Val Ser Ile Glu Asn Arg Gly Met Asn Pro Ser Val
            100                 105                 110
Leu Thr Lys Leu Gly Glu Phe Ala Ser Thr Leu Thr Ala Gly Asn Thr
        115                 120                 125
Ala Asn Lys Leu Trp Leu Met Ala Asp Val Asp Pro Lys Ser Ala Phe
    130                 135                 140
Lys Leu Leu Gly Leu Asp Met Pro Gly Val Arg Phe Ile Asp Asn Pro
145                 150                 155                 160
Lys Met Leu Gln Trp Leu Lys Phe Thr Lys Ala Tyr Leu Asp Met Lys
                165                 170                 175
Lys Ser Gly Phe Gly Glu Thr Ser Ala His Ala Leu Leu Tyr Glu Lys
            180                 185                 190
Ile Gly Gly Pro Asp Leu Ser Leu Leu Leu Leu Ser Leu Lys Asp Ala
        195                 200                 205
Pro Asp Ala Asn Ser Leu Val Gln Lys Leu Thr Asn Ser Gln Phe Gly
    210                 215                 220
Met Trp His Asp Ala Arg Ile Glu Pro Glu Gln Leu Ala Gln Thr Val
225                 230                 235                 240
Phe Lys Ile Gln Asp Val Arg Lys Leu Pro Lys Asn Asp Pro Lys Leu
                245                 250                 255
Gln Val Ile Asp Asp Tyr Ala Lys Tyr His Arg Lys His Arg Lys Phe
            260                 265                 270
Leu Asn Ser Ile Met Ile Ile
        275

<210> 202
<211> 417
<212> DNA
<213> Artificial Sequence

<220>
<223> cDNA

<400> 202
atgcgtctat cttttgtgct ttctcttgtc gtggccattg gctacgtcgt gacctgcaac      60

gcaactgagt actccgacga aaccaatatc gccatggtgg aatctccaga tctcgtccgt     120

cgctcgctca ggaacggcga cattgccggt ggaagatttc ttcgagctca tgaagaggac     180

gatgcggggg agcggacctt cagcgtgact gacctgtgga acaaggtggc ggccaaaaag     240

ttggccaagg cgatgctggc ggacccttca aaggagcaga aagcgtacga gaagtgggca     300

aagaaggggt acagcctgga taagatcaag aactggttgg caatcgcgga ccccaagcag     360

aaggggaagt acgaccgtat ctacaacgga tacacctttc acctgtatca gagctga        417

<210> 203
<211> 138
<212> PRT
<213> Phytophthora sojae

<400> 203
Met Arg Leu Ser Phe Val Leu Ser Leu Val Val Ala Ile Gly Tyr Val
1               5                   10                  15
Val Thr Cys Asn Ala Thr Glu Tyr Ser Asp Glu Thr Asn Ile Ala Met
            20                  25                  30

Val Glu Ser Pro Asp Leu Val Arg Arg Ser Leu Arg Asn Gly Asp Ile
        35                  40                  45
Ala Gly Gly Arg Phe Leu Arg Ala His Glu Glu Asp Asp Ala Gly Glu
    50                  55                  60
Arg Thr Phe Ser Val Thr Asp Leu Trp Asn Lys Val Ala Ala Lys Lys
65                  70                  75                  80
Leu Ala Lys Ala Met Leu Ala Asp Pro Ser Lys Glu Gln Lys Ala Tyr
                85                  90                  95
Glu Lys Trp Ala Lys Lys Gly Tyr Ser Leu Asp Lys Ile Lys Asn Trp
            100                 105                 110
Leu Ala Ile Ala Asp Pro Lys Gln Lys Gly Lys Tyr Asp Arg Ile Tyr
        115                 120                 125
Asn Gly Tyr Thr Phe His Leu Tyr Gln Ser
    130                 135

<210> 204
<211> 3750
<212> DNA
<213> Artificial Sequence

<220>
<223> cDNA

<400> 204
atggcagcag cactggtcgg tggtgccttc ctctctgctt ttcttgatgt ggttttcgac      60

aggctggctt cacctgagtt tgttgacttg atccgtggaa agaagcttag caagaagttg     120

cttcaaaagt tggagaccac tctcagagtg gttggagctg tgcttgatga tgccgagaag     180

aaacagatca caaacaccaa tgtcaaacac tggctcaatg atctcaaaca tgctgtctat     240

gaagccgatg acttactcga ccatgttttc accaaagctg ccacccaaaa caaggtaaga     300

gacttgtttt ctcgcttttc cgataggaag atcgttagta agttggagga catagttgtc     360

acacttgagt ctcatttaaa actcaaggag agtcttgatt tgaaagagag tgcagtggag     420

aacttgtcat ggaaagctcc atcaacatct ctggaagatg gatctcatat atatggtagg     480

gagaaagata aggaggccat aatcaagttg ttgtcggagg ataacagtga cggtagagaa     540

gtgtctgtgg ttcctattgt gggcatgggt ggggttggaa aaactacttt ggcccaattg     600

gtgtacaacg atgagaattt gaaacagata tttgattttg attttaaggc atgggtttgt     660

gtttctcaag aatttgatgt tctcaaggtc acaaaaacta taatagaggc ggtgactgga     720

aaggcttgta aattgaatga tctgaatcta cttcatcttg aattgatgga caagctgaaa     780

gataaaaaat tcttaattgt tttggatgat gtttggacag aggattatgt tgattggcgt     840

cttcttaaga aaccatttaa ccgtgggatt attaggagaa gtaaaattct tctaacaacc     900

cgcagtgaaa aaacagcatc tgtagtccaa actgttcaca cctatcatct aaaccaattg     960

tcgaatgaag attgttggtc agtgtttgcg aaccatgcat gtctttccac ggaatctaac    1020

gagaacacag caacactaga aaaaattgga aaggagattg ttaaaaagtg caacggactg    1080

cctttagcag cagagtcgct tggaggcatg ttgagaagaa agcatgacat tggtgattgg    1140

aataatattc tcaatagtga catttgggaa ctttctgaaa gtgagtgtaa agttattcca 1200
gcactgagac ttagttatca ttatctccct ccacatttaa aacgatgctt tgtttattgt 1260
tcgttgtatc cacaagatta cgaatttgaa aaaaatgaat taatcttgtt gtggatggct 1320
gaagatcttt tgaagaaacc aaggaaaggt aggactttag aagaggttgg tcatgagtat 1380
tttgatgatt tggtttcgag atcgtttttc caacgttcaa gaacaagtag ttggcctcat 1440
cgcaaatgtt ttgtgatgca tgacctcatg catgatctag ccacatcact cggtggagat 1500
ttttatttta gatcagaaga acttgggaaa gaaacaaaga tcaataccaa gactcgtcat 1560
ttgtcatttg ccaaattcaa ttcttcagtc ttggacaact ttgatgttat tggtagagca 1620
aaatttctga gaaccttctt gtccattatc aattttgaag ctgctccatt caacaatgag 1680
gaggcacaat gtatcataat gtcgaagctt atgtacttga gagttttatc attttgtgac 1740
ttccaaagtc tggattcttt gcctgattca ataggtaaat tgatccatct gcgctattta 1800
gatctctctt tttcaagaat agaaacactg ccaaagtcat tgtgtaattt gtacaatctg 1860
caaactttga agttgtgtag ttgcagaaag ctgactaagt tgcccagtga catgcgcaat 1920
cttgttaact tgcgtcatct tggtattgct tatactccta taaaagagat gccgagagga 1980
atgggtaaat taaatcattt acaacatctg gatttctttg ttgtgggcaa gcacgaagag 2040
aatggaatca aagaattggg aggactttca aatcttcgtg gtcagcttga aattaggaag 2100
ttggagaatg tttcccaaag tgatgaagcg ttggaggcaa ggatgatgga taaaaaacac 2160
attaatagtt tacagttgga atggtctgga tgtaacaaca acagtaccaa cttccaactt 2220
gaaatagatg tgctttgcaa gttacagcct cactttaaca ttgaatcgtt ggaaataaaa 2280
ggttatgaag gaaccagatt tccagattgg atgggaaatt cttcctactg caatatgatt 2340
agtctaaaat tgcgtgattg tcacaactgt agtatgcttc cttcacttgg acaactacct 2400
tctctcaagg accttggaat tgcacgattg aataggctga agactattga tgcaggtttc 2460
tacaagaatg aagaatgtcg ttctgggacg tcctttccct cccttgaatc tctgtccatt 2520
gatgacatgc cttgttggga ggtgtggagt tccttcgatt cagaagcttt tcctgtgctt 2580
aacagtcttg aaatacgtga ctgccccaaa ctagaggaag tttgccgaat caccttcct 2640
gctctgacaa aacttgtgat tagaaattgc gagctgcttg tctcttctct cccaacggct 2700
cccgccattc aaagtttgga gatatgtaaa agcaataaag tagcactgca tgcgtttcct 2760
ctcttggtag aaactataga agtagaagga agcccaatgg tggagtccgt gatcgaggcc 2820
atcactaaca tccaaccaac ttgtctccgg tctttaacat taagggattg ttcgtcagcc 2880
gtgtcatttc cgggtggtcg tttacctgaa tcactgaaga gtctgagtat caaggatctt 2940
aaaaaactgg aattcccgac gcaacacaaa catgagttac tggaaacact gtcaatagaa 3000
agcagttgtg attcactcac atctcttcca ttggttacct ttccaaatct cagatatctc 3060

agcatcgaaa agtgtgaaaa tatggaatat cttttggttt caggggcaga gtcatttaag 3120

agtctgtgtt atttgttaat ttacaaatgc cccaactttg tatcattctg gagagaagga 3180

ttgcctgcgc ccaacttgat tactttcagt gtttggggct ctgacaagtt gaagtcgttg 3240

cctgatgaga tgagtactct tctcccaaag ttagaagatc tcaccatatc caactgccca 3300

gaaattgagt cctttccaaa acggggtatg ccacctaacc tgagaagagt tgagattgtc 3360

aattgtgaga aactactgag cggcctagca tggccatcca tgggcatgct tactcatctc 3420

aatgttgggg gtccatgtga tggcatcaag tccttcccta aagagggttt gctgcctccc 3480

tcccttacgt ctctgtctct atatgacttg tcaaatctgg agatgttgga ctgcacgggg 3540

cttctccatc tcacatccct gcaacaatta caaatttttg gatgtccaaa gctggagaat 3600

atggctggag aaagtcttcc tttctctcta ataaaattaa ccatggtgga atgtcctttg 3660

ctggaaaaac gatgccgcat gaagcaccct caaatttggc ctaaagtttc ccacatccct 3720

ggcattaagg ttggcaatag atggatttag 3750

<210> 205
<211> 1249
<212> PRT
<213> Glycine max

<400> 205
Met Ala Ala Ala Leu Val Gly Gly Ala Phe Leu Ser Ala Phe Leu Asp
1               5                   10                  15
Val Val Phe Asp Arg Leu Ala Ser Pro Glu Phe Val Asp Leu Ile Arg
            20                  25                  30
Gly Lys Lys Leu Ser Lys Lys Leu Leu Gln Lys Leu Glu Thr Thr Leu
        35                  40                  45
Arg Val Val Gly Ala Val Leu Asp Asp Ala Glu Lys Lys Gln Ile Thr
    50                  55                  60
Asn Thr Asn Val Lys His Trp Leu Asn Asp Leu Lys His Ala Val Tyr
65                  70                  75                  80
Glu Ala Asp Asp Leu Leu Asp His Val Phe Thr Lys Ala Ala Thr Gln
                85                  90                  95
Asn Lys Val Arg Asp Leu Phe Ser Arg Phe Ser Asp Arg Lys Ile Val
            100                 105                 110
Ser Lys Leu Glu Asp Ile Val Val Thr Leu Glu Ser His Leu Lys Leu
        115                 120                 125
Lys Glu Ser Leu Asp Leu Lys Glu Ser Ala Val Glu Asn Leu Ser Trp
    130                 135                 140
Lys Ala Pro Ser Thr Ser Leu Glu Asp Gly Ser His Ile Tyr Gly Arg
145                 150                 155                 160
Glu Lys Asp Lys Glu Ala Ile Ile Lys Leu Leu Ser Glu Asp Asn Ser
                165                 170                 175
Asp Gly Arg Glu Val Ser Val Val Pro Ile Val Gly Met Gly Gly Val
            180                 185                 190
Gly Lys Thr Thr Leu Ala Gln Leu Val Tyr Asn Asp Glu Asn Leu Lys
        195                 200                 205
Gln Ile Phe Asp Phe Asp Phe Lys Ala Trp Val Cys Val Ser Gln Glu
    210                 215                 220
Phe Asp Val Leu Lys Val Thr Lys Thr Ile Ile Glu Ala Val Thr Gly
225                 230                 235                 240

Lys Ala Cys Lys Leu Asn Asp Leu Asn Leu Leu His Leu Glu Leu Met
                245                 250                 255
Asp Lys Leu Lys Asp Lys Lys Phe Leu Ile Val Leu Asp Asp Val Trp
            260                 265                 270
Thr Glu Asp Tyr Val Asp Trp Arg Leu Leu Lys Lys Pro Phe Asn Arg
        275                 280                 285
Gly Ile Ile Arg Arg Ser Lys Ile Leu Leu Thr Thr Arg Ser Glu Lys
    290                 295                 300
Thr Ala Ser Val Val Gln Thr Val His Thr Tyr His Leu Asn Gln Leu
305                 310                 315                 320
Ser Asn Glu Asp Cys Trp Ser Val Phe Ala Asn His Ala Cys Leu Ser
                325                 330                 335
Thr Glu Ser Asn Glu Asn Thr Ala Thr Leu Glu Lys Ile Gly Lys Glu
            340                 345                 350
Ile Val Lys Lys Cys Asn Gly Leu Pro Leu Ala Ala Glu Ser Leu Gly
        355                 360                 365
Gly Met Leu Arg Arg Lys His Asp Ile Gly Asp Trp Asn Asn Ile Leu
    370                 375                 380
Asn Ser Asp Ile Trp Glu Leu Ser Glu Ser Glu Cys Lys Val Ile Pro
385                 390                 395                 400
Ala Leu Arg Leu Ser Tyr His Tyr Leu Pro Pro His Leu Lys Arg Cys
                405                 410                 415
Phe Val Tyr Cys Ser Leu Tyr Pro Gln Asp Tyr Glu Phe Glu Lys Asn
            420                 425                 430
Glu Leu Ile Leu Leu Trp Met Ala Glu Asp Leu Leu Lys Lys Pro Arg
        435                 440                 445
Lys Gly Arg Thr Leu Glu Glu Val Gly His Glu Tyr Phe Asp Asp Leu
    450                 455                 460
Val Ser Arg Ser Phe Phe Gln Arg Ser Arg Thr Ser Ser Trp Pro His
465                 470                 475                 480
Arg Lys Cys Phe Val Met His Asp Leu Met His Asp Leu Ala Thr Ser
                485                 490                 495
Leu Gly Gly Asp Phe Tyr Phe Arg Ser Glu Glu Leu Gly Lys Glu Thr
            500                 505                 510
Lys Ile Asn Thr Lys Thr Arg His Leu Ser Phe Ala Lys Phe Asn Ser
        515                 520                 525
Ser Val Leu Asp Asn Phe Asp Val Ile Gly Arg Ala Lys Phe Leu Arg
    530                 535                 540
Thr Phe Leu Ser Ile Ile Asn Phe Glu Ala Ala Pro Phe Asn Asn Glu
545                 550                 555                 560
Glu Ala Gln Cys Ile Ile Met Ser Lys Leu Met Tyr Leu Arg Val Leu
                565                 570                 575
Ser Phe Cys Asp Phe Gln Ser Leu Asp Ser Leu Pro Asp Ser Ile Gly
            580                 585                 590
Lys Leu Ile His Leu Arg Tyr Leu Asp Leu Ser Phe Ser Arg Ile Glu
        595                 600                 605
Thr Leu Pro Lys Ser Leu Cys Asn Leu Tyr Asn Leu Gln Thr Leu Lys
    610                 615                 620
Leu Cys Ser Cys Arg Lys Leu Thr Lys Leu Pro Ser Asp Met Arg Asn
625                 630                 635                 640
Leu Val Asn Leu Arg His Leu Gly Ile Ala Tyr Thr Pro Ile Lys Glu
                645                 650                 655
Met Pro Arg Gly Met Gly Lys Leu Asn His Leu Gln His Leu Asp Phe
            660                 665                 670
Phe Val Val Gly Lys His Glu Glu Asn Gly Ile Lys Glu Leu Gly Gly
        675                 680                 685
Leu Ser Asn Leu Arg Gly Gln Leu Glu Ile Arg Lys Leu Glu Asn Val
    690                 695                 700
Ser Gln Ser Asp Glu Ala Leu Glu Ala Arg Met Met Asp Lys Lys His
705                 710                 715                 720
Ile Asn Ser Leu Gln Leu Glu Trp Ser Gly Cys Asn Asn Asn Ser Thr
                725                 730                 735
Asn Phe Gln Leu Glu Ile Asp Val Leu Cys Lys Leu Gln Pro His Phe

740                 745                 750
Asn Ile Glu Ser Leu Glu Ile Lys Gly Tyr Glu Gly Thr Arg Phe Pro
        755                 760                 765
Asp Trp Met Gly Asn Ser Ser Tyr Cys Asn Met Ile Ser Leu Lys Leu
    770                 775                 780
Arg Asp Cys His Asn Cys Ser Met Leu Pro Ser Leu Gly Gln Leu Pro
785                 790                 795                 800
Ser Leu Lys Asp Leu Gly Ile Ala Arg Leu Asn Arg Leu Lys Thr Ile
                805                 810                 815
Asp Ala Gly Phe Tyr Lys Asn Glu Glu Cys Arg Ser Gly Thr Ser Phe
            820                 825                 830
Pro Ser Leu Glu Ser Leu Ser Ile Asp Asp Met Pro Cys Trp Glu Val
        835                 840                 845
Trp Ser Ser Phe Asp Ser Glu Ala Phe Pro Val Leu Asn Ser Leu Glu
    850                 855                 860
Ile Arg Asp Cys Pro Lys Leu Glu Gly Ser Leu Pro Asn His Leu Pro
865                 870                 875                 880
Ala Leu Thr Lys Leu Val Ile Arg Asn Cys Glu Leu Leu Val Ser Ser
                885                 890                 895
Leu Pro Thr Ala Pro Ala Ile Gln Ser Leu Glu Ile Cys Lys Ser Asn
            900                 905                 910
Lys Val Ala Leu His Ala Phe Pro Leu Leu Val Glu Thr Ile Glu Val
        915                 920                 925
Glu Gly Ser Pro Met Val Glu Ser Val Ile Glu Ala Ile Thr Asn Ile
    930                 935                 940
Gln Pro Thr Cys Leu Arg Ser Leu Thr Leu Arg Asp Cys Ser Ser Ala
945                 950                 955                 960
Val Ser Phe Pro Gly Gly Arg Leu Pro Glu Ser Leu Lys Ser Leu Ser
                965                 970                 975
Ile Lys Asp Leu Lys Lys Leu Glu Phe Pro Thr Gln His Lys His Glu
            980                 985                 990
Leu Leu Glu Thr Leu Ser Ile Glu Ser Ser Cys Asp Ser Leu Thr Ser
        995                 1000                1005
Leu Pro Leu Val Thr Phe Pro Asn Leu Arg Tyr Leu Ser Ile Glu Lys
    1010                1015                1020
Cys Glu Asn Met Glu Tyr Leu Leu Val Ser Gly Ala Glu Ser Phe Lys
1025                1030                1035                1040
Ser Leu Cys Tyr Leu Leu Ile Tyr Lys Cys Pro Asn Phe Val Ser Phe
                1045                1050                1055
Trp Arg Glu Gly Leu Pro Ala Pro Asn Leu Ile Thr Phe Ser Val Trp
            1060                1065                1070
Gly Ser Asp Lys Leu Lys Ser Leu Pro Asp Glu Met Ser Thr Leu Leu
        1075                1080                1085
Pro Lys Leu Glu Asp Leu Thr Ile Ser Asn Cys Pro Glu Ile Glu Ser
    1090                1095                1100
Phe Pro Lys Arg Gly Met Pro Pro Asn Leu Arg Arg Val Glu Ile Val
1105                1110                1115                1120
Asn Cys Glu Lys Leu Leu Ser Gly Leu Ala Trp Pro Ser Met Gly Met
                1125                1130                1135
Leu Thr His Leu Asn Val Gly Gly Pro Cys Asp Gly Ile Lys Ser Phe
            1140                1145                1150
Pro Lys Glu Gly Leu Leu Pro Pro Ser Leu Thr Ser Leu Ser Leu Tyr
        1155                1160                1165
Asp Leu Ser Asn Leu Glu Met Leu Asp Cys Thr Gly Leu Leu His Leu
    1170                1175                1180
Thr Ser Leu Gln Gln Leu Gln Ile Phe Gly Cys Pro Lys Leu Glu Asn
1185                1190                1195                1200
Met Ala Gly Glu Ser Leu Pro Phe Ser Leu Ile Lys Leu Thr Met Val
                1205                1210                1215
Glu Cys Pro Leu Leu Glu Lys Arg Cys Arg Met Lys His Pro Gln Ile
            1220                1225                1230
Trp Pro Lys Val Ser His Ile Pro Gly Ile Lys Val Gly Asn Arg Trp
        1235                1240                1245

Ile

<210> 206
<211> 3987
<212> DNA
<213> Artificial Sequence

<220>
<223> cDNA

<400> 206
atggagattg gcttagcagt tggtggtgca tttctgtctt cagctttgaa tgttctcttt 60

gataggcttg ctcctcatgg tgatctgctc aacatgtttc agaagaataa gaatgatgtt 120

cgactcttaa agaagctgaa aatgactttg gttggccttc aggttgtgtt aagtgatgca 180

gagaataaac aggcatcaaa tcaacacgtg agccagtggt ttaatgaact tcgaggtgct 240

gtggatggcg ctgaaaactt aatggaacta gtcaattatg aggctttgag gcgtaaggtg 300

gaaggtcggc atcaaaatct tgcagaaaca agcaaccaac aagtaagtga ccgcaaactg 360

aacttgagtg atgattattt tctcgacata aaggagaagt tggaagaaac cattgaaaca 420

ttggaggatt tgcaaaagca aatcggtgac cttggcttac agaagcattt agatttgggt 480

aagaaactag aaactagaac accttcaact tctttggtcg atgaatctaa aatccttggt 540

agaatgattg aaaaagagcg attgattgac cgtttattgt caagtgattc aaatggagaa 600

aatctgactg ttgtccctat tgttggaatg gggggcgtgg gcaagacaac acttgctaaa 660

attgtttaca acgataagaa ggtgaaagat cattttgact tgaaagcttg gttttgtgtt 720

tcagaggcat atgatgcttt cagaataaca aaaggattac ttcaagaaat tggatcattc 780

gacctaaagg atgacaacaa tcttaatcag ctacaggtaa aattgaagga aagcctaaag 840

ggaaaaagat ttcttgttgt ccttgatgac ttgtggaatg atgactgtga tgagtgggat 900

gacttgaaaa atctttttgt acaaggagct atgggaagta agatccttgt gacgacacgt 960

aaggaggatg ttgccttgat gatgggtaat ggggcaatca atgtggagac tctgtctgat 1020

gaagtctctt gggatctatt caaacagcat tcattaaaaa acagggatcc ggaggaacat 1080

ccagaacttg aagaggttgg aaagcgaatt gcagacaagt gcaaaggatt gcctttagct 1140

ctaaaggcac ttgctggtat tttatgccgc aaatcagaag tgtacgagtg gaaaaacgtc 1200

ttaagaagtg aaatatggga gctgccaagg cgtaagaatg gtatattacc agagttgatg 1260

ttgagctaca atgatcttcc tgcacatttg aagcaatgtt ttgctttttg tgcaatatat 1320

cccaaagatt ataaattttg caaagagcaa gttattcacc tgtggattgc taatggtctg 1380

gtacaacagt tgcattctgg taaccaatac tttaatgagt tgagatcaag atcattgttc 1440

gaaagggtcc cagagtcttc tgaaagatac ggtgggaaat tcttaatgca tgatcttgtc 1500

aatgatttgg cccaaattgc atcttcaaaa ctttgtgtca ggttggaaga gtgccaagga 1560
tctcatatat tggaacaaag tcggcacaca tcgtattcaa tgggcagaga tggtgacttt 1620
gagaaattga aaccgctctc taagtcagag cagctgagga cattgcttcc aatctctatc 1680
cagttccttt accgccctaa actaagcaag agggtgctgc ataacatatt gccaagacta 1740
acttacttaa gggcattatc attgtcatgt tatgcgattg tggagcttcc aaaagatttg 1800
tttatcaaat ttaagctcct gagatttttg gacctttctc aaacagagat tacaaaattg 1860
cccgattcta tctgtgcgtt gtataacttg gagacacttc tcctgtcatc ttgtgatgat 1920
cttgaggagc taccgctgca gatggagaag ttgatcaact tgcggcacct tgacatcagc 1980
aacacttctc gcttgaagat gccgctacat ctgagcaagt tgaaaagcct ccaagtgcta 2040
gtgggagcca agtttctact aggtggcccc tgtggttgga gaatggaaga tttgggtgaa 2100
gcacattaca tgtatggatc tctatcaatt ttagaattgc aaaatgtggt tgatagaagg 2160
gaagctcaga aggcgaagat gagggataag aagaagaatc atgttgagaa gttatcgttg 2220
gagtggagtg gaagtgatgc ggacaattca caaaccgaaa gagacatact tgatgagcta 2280
cgtccacaca caaaaataaa agaagtcgaa atctccggat atagagggac acaattccca 2340
aattggctag ctgatgattc gtttcttaag ctgctagtac aattgtctct tagcaactgc 2400
aaggactgtt tttccttgcc agcgctagga caacttcctt gtttgaaatt cctttccatt 2460
agaaagatgc atcgaataac agaggtgacg gaagaattct atggtagtcc gtcctctgaa 2520
aagcctttta actctcttga gaagcttgaa tttgcagaga tgcctgagtg gaagcagtgg 2580
cacgtactag ggatcggaga gtttcctgca cttcgggacc tttcaattga agattgcccc 2640
aagttggttg gaaatttcct cgagaatctt tgttctctga cgaaattgag aatctcaata 2700
tgtcccgaac tcaatttgga gacacccatc caactttcaa gtctaaaatg gtttgaagtt 2760
tctggttctt ctaaggctgg atttattttt gatgaagctg aactgttcac cttaaatatt 2820
ttgaattgca actctcttac ctccttgcct attagcactc tgccgagtac gttgaagaca 2880
atatggatat gtcgttgccg gaaattgaaa ttggaggcac cagatagtag taggatgatt 2940
tcagacatgt ttctggagga attgagactg gaggaatgtg attctatatc atcacctgag 3000
ttggtcccaa gagcacgcac tttgactgtg aagaggtgcc aaaaccttac taggtttttg 3060
attcctaatg ggactgaaag actcgatatt tggggttgtg agaatcttga aatattttcg 3120
gtggtatgtg ggacccagat gacgtttttg aatatccact cttgcgcaaa gctgaagcgg 3180
ctgccagaat gtatgcagga actccttcca tctcttaagg aactgcatct gtggaattgt 3240
ccagaaatag agtcctttcc tgatggagga ttgcccttca atttacaact cctcgtgatc 3300
aactattgcg agaaactggt gaacggtcga aaggagtggc gtctacagag actccattct 3360
ctcagagagt tgttcatcaa ccacgatggc agtgacgaag agattgttgg tggtgagaat 3420

tgggagttgc cttgctctat tcaaaggctt gtcatagtca atctgaaaac attaagcagc 3480

caacttctca aaagcctcac ctctcttgaa tctctagata ttcgtaattt acctcaaatt 3540

cggtcactgc tggaacaagg gcttccctcc tctttttcta agctatattt atacagccat 3600

gatgagctcc attcactaca aggtcttcag cacctcaatt cggttcaaag tctattgatc 3660

tggaattgcc ctaatctcca atctcttgca gaatcagcgc tgccctcctg cctctctaag 3720

ttgaccatca gggattgccc taatctccaa tcccttccca aatcagcatt tccctcctcc 3780

ctctctgagc tgaccatcga gaattgccct aatctccaat cccttccagt aaaagggatg 3840

ccctcttccc tctctatact atctatttac aaatgcccat ttctcgaacc actcctagaa 3900

tttgacaagg gggagtactg gccaaaaatt gctcatattc ctgaaatata tatcggtcgc 3960

actatgtttg actgtgaatg cctgtga 3987

<210> 207
<211> 1328
<212> PRT
<213> Capsicum chinense

<400> 207
Met Glu Ile Gly Leu Ala Val Gly Gly Ala Phe Leu Ser Ser Ala Leu
1               5                   10                  15
Asn Val Leu Phe Asp Arg Leu Ala Pro His Gly Asp Leu Leu Asn Met
            20                  25                  30
Phe Gln Lys Asn Lys Asn Asp Val Arg Leu Leu Lys Lys Leu Lys Met
        35                  40                  45
Thr Leu Val Gly Leu Gln Val Val Leu Ser Asp Ala Glu Asn Lys Gln
    50                  55                  60
Ala Ser Asn Gln His Val Ser Gln Trp Phe Asn Glu Leu Arg Gly Ala
65                  70                  75                  80
Val Asp Gly Ala Glu Asn Leu Met Glu Leu Val Asn Tyr Glu Ala Leu
                85                  90                  95
Arg Arg Lys Val Glu Gly Arg His Gln Asn Leu Ala Glu Thr Ser Asn
            100                 105                 110
Gln Gln Val Ser Asp Arg Lys Leu Asn Leu Ser Asp Asp Tyr Phe Leu
        115                 120                 125
Asp Ile Lys Glu Lys Leu Glu Glu Thr Ile Glu Thr Leu Glu Asp Leu
    130                 135                 140
Gln Lys Gln Ile Gly Asp Leu Gly Leu Gln Lys His Leu Asp Leu Gly
145                 150                 155                 160
Lys Lys Leu Glu Thr Arg Thr Pro Ser Thr Ser Leu Val Asp Glu Ser
                165                 170                 175
Lys Ile Leu Gly Arg Met Ile Glu Lys Glu Arg Leu Ile Asp Arg Leu
            180                 185                 190
Leu Ser Ser Asp Ser Asn Gly Glu Asn Leu Thr Val Val Pro Ile Val
        195                 200                 205
Gly Met Gly Gly Val Gly Lys Thr Thr Leu Ala Lys Ile Val Tyr Asn
    210                 215                 220
Asp Lys Lys Val Lys Asp His Phe Asp Leu Lys Ala Trp Phe Cys Val
225                 230                 235                 240
Ser Glu Ala Tyr Asp Ala Phe Arg Ile Thr Lys Gly Leu Leu Gln Glu
                245                 250                 255
Ile Gly Ser Phe Asp Leu Lys Asp Asp Asn Asn Leu Asn Gln Leu Gln
            260                 265                 270

Val Lys Leu Lys Glu Ser Leu Lys Gly Lys Arg Phe Leu Val Val Leu
        275                 280                 285
Asp Asp Leu Trp Asn Asp Asp Cys Asp Glu Trp Asp Asp Leu Lys Asn
    290                 295                 300
Leu Phe Val Gln Gly Ala Met Gly Ser Lys Ile Leu Val Thr Thr Arg
305                 310                 315                 320
Lys Glu Asp Val Ala Leu Met Met Gly Asn Gly Ala Ile Asn Val Glu
                325                 330                 335
Thr Leu Ser Asp Glu Val Ser Trp Asp Leu Phe Lys Gln His Ser Leu
            340                 345                 350
Lys Asn Arg Asp Pro Glu Glu His Pro Glu Leu Glu Glu Val Gly Lys
        355                 360                 365
Arg Ile Ala Asp Lys Cys Lys Gly Leu Pro Leu Ala Leu Lys Ala Leu
    370                 375                 380
Ala Gly Ile Leu Cys Arg Lys Ser Glu Val Tyr Glu Trp Lys Asn Val
385                 390                 395                 400
Leu Arg Ser Glu Ile Trp Glu Leu Pro Arg Arg Lys Asn Gly Ile Leu
                405                 410                 415
Pro Glu Leu Met Leu Ser Tyr Asn Asp Leu Pro Ala His Leu Lys Gln
            420                 425                 430
Cys Phe Ala Phe Cys Ala Ile Tyr Pro Lys Asp Tyr Lys Phe Cys Lys
        435                 440                 445
Glu Gln Val Ile His Leu Trp Ile Ala Asn Gly Leu Val Gln Gln Leu
    450                 455                 460
His Ser Gly Asn Gln Tyr Phe Asn Glu Leu Arg Ser Arg Ser Leu Phe
465                 470                 475                 480
Glu Arg Val Pro Glu Ser Ser Glu Arg Tyr Gly Gly Lys Phe Leu Met
                485                 490                 495
His Asp Leu Val Asn Asp Leu Ala Gln Ile Ala Ser Ser Lys Leu Cys
            500                 505                 510
Val Arg Leu Glu Glu Cys Gln Gly Ser His Ile Leu Glu Gln Ser Arg
        515                 520                 525
His Thr Ser Tyr Ser Met Gly Arg Asp Gly Asp Phe Glu Lys Leu Lys
    530                 535                 540
Pro Leu Ser Lys Ser Glu Gln Leu Arg Thr Leu Leu Pro Ile Ser Ile
545                 550                 555                 560
Gln Phe Leu Tyr Arg Pro Lys Leu Ser Lys Arg Val Leu His Asn Ile
                565                 570                 575
Leu Pro Arg Leu Thr Tyr Leu Arg Ala Leu Ser Leu Ser Cys Tyr Ala
            580                 585                 590
Ile Val Glu Leu Pro Lys Asp Leu Phe Ile Lys Phe Lys Leu Leu Arg
        595                 600                 605
Phe Leu Asp Leu Ser Gln Thr Glu Ile Thr Lys Leu Pro Asp Ser Ile
    610                 615                 620
Cys Ala Leu Tyr Asn Leu Glu Thr Leu Leu Leu Ser Ser Cys Asp Asp
625                 630                 635                 640
Leu Glu Glu Leu Pro Leu Gln Met Glu Lys Leu Ile Asn Leu Arg His
                645                 650                 655
Leu Asp Ile Ser Asn Thr Ser Arg Leu Lys Met Pro Leu His Leu Ser
            660                 665                 670
Lys Leu Lys Ser Leu Gln Val Leu Val Gly Ala Lys Phe Leu Leu Gly
        675                 680                 685
Gly Pro Cys Gly Trp Arg Met Glu Asp Leu Gly Glu Ala His Tyr Met
    690                 695                 700
Tyr Gly Ser Leu Ser Ile Leu Glu Leu Gln Asn Val Val Asp Arg Arg
705                 710                 715                 720
Glu Ala Gln Lys Ala Lys Met Arg Asp Lys Lys Lys Asn His Val Glu
                725                 730                 735
Lys Leu Ser Leu Glu Trp Ser Gly Ser Asp Ala Asp Asn Ser Gln Thr
            740                 745                 750
Glu Arg Asp Ile Leu Asp Glu Leu Arg Pro His Thr Lys Ile Lys Glu
        755                 760                 765
Val Glu Ile Ser Gly Tyr Arg Gly Thr Gln Phe Pro Asn Trp Leu Ala

770                 775                 780
Asp Asp Ser Phe Leu Lys Leu Leu Val Gln Leu Ser Leu Ser Asn Cys
785                 790                 795                 800
Lys Asp Cys Phe Ser Leu Pro Ala Leu Gly Gln Leu Pro Cys Leu Lys
                805                 810                 815
Phe Leu Ser Ile Arg Lys Met His Arg Ile Thr Glu Val Thr Glu Glu
            820                 825                 830
Phe Tyr Gly Ser Pro Ser Ser Glu Lys Pro Phe Asn Ser Leu Glu Lys
        835                 840                 845
Leu Glu Phe Ala Glu Met Pro Glu Trp Lys Gln Trp His Val Leu Gly
    850                 855                 860
Ile Gly Glu Phe Pro Ala Leu Arg Asp Leu Ser Ile Glu Asp Cys Pro
865                 870                 875                 880
Lys Leu Val Gly Asn Phe Leu Glu Asn Leu Cys Ser Leu Thr Lys Leu
                885                 890                 895
Arg Ile Ser Ile Cys Pro Glu Leu Asn Leu Glu Thr Pro Ile Gln Leu
            900                 905                 910
Ser Ser Leu Lys Trp Phe Glu Val Ser Gly Ser Ser Lys Ala Gly Phe
        915                 920                 925
Ile Phe Asp Glu Ala Glu Leu Phe Thr Leu Asn Ile Leu Asn Cys Asn
    930                 935                 940
Ser Leu Thr Ser Leu Pro Ile Ser Thr Leu Pro Ser Thr Leu Lys Thr
945                 950                 955                 960
Ile Trp Ile Cys Arg Cys Arg Lys Leu Lys Leu Glu Ala Pro Asp Ser
                965                 970                 975
Ser Arg Met Ile Ser Asp Met Phe Leu Glu Glu Leu Arg Leu Glu Glu
            980                 985                 990
Cys Asp Ser Ile Ser Ser Pro Glu Leu Val Pro Arg Ala Arg Thr Leu
        995                 1000                1005
Thr Val Lys Arg Cys Gln Asn Leu Thr Arg Phe Leu Ile Pro Asn Gly
    1010                1015                1020
Thr Glu Arg Leu Asp Ile Trp Gly Cys Glu Asn Leu Glu Ile Phe Ser
1025                1030                1035                1040
Val Val Cys Gly Thr Gln Met Thr Phe Leu Asn Ile His Ser Cys Ala
                1045                1050                1055
Lys Leu Lys Arg Leu Pro Glu Cys Met Gln Glu Leu Leu Pro Ser Leu
            1060                1065                1070
Lys Glu Leu His Leu Trp Asn Cys Pro Glu Ile Glu Ser Phe Pro Asp
        1075                1080                1085
Gly Gly Leu Pro Phe Asn Leu Gln Leu Leu Val Ile Asn Tyr Cys Glu
    1090                1095                1100
Lys Leu Val Asn Gly Arg Lys Glu Trp Arg Leu Gln Arg Leu His Ser
1105                1110                1115                1120
Leu Arg Glu Leu Phe Ile Asn His Asp Gly Ser Asp Glu Glu Ile Val
                1125                1130                1135
Gly Gly Glu Asn Trp Glu Leu Pro Cys Ser Ile Gln Arg Leu Val Ile
            1140                1145                1150
Val Asn Leu Lys Thr Leu Ser Ser Gln Leu Leu Lys Ser Leu Thr Ser
        1155                1160                1165
Leu Glu Ser Leu Asp Ile Arg Asn Leu Pro Gln Ile Arg Ser Leu Leu
    1170                1175                1180
Glu Gln Gly Leu Pro Ser Ser Phe Ser Lys Leu Tyr Leu Tyr Ser His
1185                1190                1195                1200
Asp Glu Leu His Ser Leu Gln Gly Leu Gln His Leu Asn Ser Val Gln
                1205                1210                1215
Ser Leu Leu Ile Trp Asn Cys Pro Asn Leu Gln Ser Leu Ala Glu Ser
            1220                1225                1230
Ala Leu Pro Ser Cys Leu Ser Lys Leu Thr Ile Arg Asp Cys Pro Asn
        1235                1240                1245
Leu Gln Ser Leu Pro Lys Ser Ala Phe Pro Ser Ser Leu Ser Glu Leu
    1250                1255                1260
Thr Ile Glu Asn Cys Pro Asn Leu Gln Ser Leu Pro Val Lys Gly Met
1265                1270                1275                1280

Pro Ser Ser Leu Ser Ile Leu Ser Ile Tyr Lys Cys Pro Phe Leu Glu
                1285                1290                1295
Pro Leu Leu Glu Phe Asp Lys Gly Glu Tyr Trp Pro Lys Ile Ala His
            1300                1305                1310
Ile Pro Glu Ile Tyr Ile Gly Arg Thr Met Phe Asp Cys Glu Cys Leu
        1315                1320                1325

<210> 208
<211> 474
<212> DNA
<213> Artificial Sequence

<220>
<223> cDNA

<400> 208
atggcttaca cagtttccag tgccaatcaa ttagtgtatt taggttctgt atgggctgat      60

ccattagagt tacaaaatct gtgtacttcg gcgttaggca atcagtttca aacacaacag     120

gctagaacta cggttcaaca gcagttctct gatgtgtgga agactattcc gaccgctaca     180

gttagatttc ctgctactgg tttcaaagtt ttccgatata atgccgtgct agattctcta     240

gtgtcggcac ttctcggagc ctttgatact aggaacagga taatagaagt tgaaaatccg     300

caaaatccta caactgccga gacgcttgat gcgacgaggc gggtagatga tgcgacggtg     360

gccattaggg ccagtataag taacctcatg aatgagttag ttcgtggcac gggaatgtac     420

aatcaagctc tgttcgagag cgcgagtgga ctaacctggg ctacaactcc ttaa           474

<210> 209
<211> 157
<212> PRT
<213> Pepper mild mottle virus

<400> 209
Met Ala Tyr Thr Val Ser Ser Ala Asn Gln Leu Val Tyr Leu Gly Ser
1               5                   10                  15
Val Trp Ala Asp Pro Leu Glu Leu Gln Asn Leu Cys Thr Ser Ala Leu
            20                  25                  30
Gly Asn Gln Phe Gln Thr Gln Gln Ala Arg Thr Thr Val Gln Gln Gln
        35                  40                  45
Phe Ser Asp Val Trp Lys Thr Ile Pro Thr Ala Thr Val Arg Phe Pro
    50                  55                  60
Ala Thr Gly Phe Lys Val Phe Arg Tyr Asn Ala Val Leu Asp Ser Leu
65                  70                  75                  80
Val Ser Ala Leu Leu Gly Ala Phe Asp Thr Arg Asn Arg Ile Ile Glu
                85                  90                  95
Val Glu Asn Pro Gln Asn Pro Thr Thr Ala Glu Thr Leu Asp Ala Thr
            100                 105                 110
Arg Arg Val Asp Asp Ala Thr Val Ala Ile Arg Ala Ser Ile Ser Asn
        115                 120                 125
Leu Met Asn Glu Leu Val Arg Gly Thr Gly Met Tyr Asn Gln Ala Leu
    130                 135                 140
Phe Glu Ser Ala Ser Gly Leu Thr Trp Ala Thr Thr Pro
145                 150                 155

<210> 210
<211> 4143
<212> DNA
<213> Artificial Sequence

<220>
<223> cDNA

<400> 210
atggagattg gcttagcagt tggaggtgca tttctatctt cagctttgaa tgttctcttt 60

gataggcttg ctcctcaggg cgagctgctg aagatgtttc agagggacaa acatgatgtt 120

cgattattaa agaagctgag aattaccttg cttggccttc aggctgttct atgtgatgca 180

gagaataaga aggcatcaaa tcaatacgtg agccagtggc ttattgagct tcaagatgct 240

gtggacagtg ctgaaaactt aatggaagaa atcaattatg aagttctgag agttaaggtg 300

gaaggtcagt atcaaaatct tggagaaaca agcaaccagc aagtaagtga cctcaacttg 360

tgcttgagtg atgaattttt ccttaacata aaggagaagt tggaggacgc cattgaaaca 420

ttggaggagt tggaaaagca aatcggtcgc cttgatctaa caaagtatct tgattcagat 480

aaacaagaaa ctagaagact ttcaacttct gtggttgatg actctaatat ctttggcagg 540

cagaatgaaa tagaggaatt ggtgggccgt ttactatctg ttgctgtaaa tggcaaaaat 600

ttgacagtaa ttcctattgt tggaatggct gggattggca agacaacact tgctaaagcc 660

gtttacaacg atgagaaggt gaaataccat tttgatttga aagcttggtt ttgtgtgtct 720

gaaccatatg atgctttcag aataacaaaa gggttacttc aagaaatagg ttcatttgac 780

ttaaagatgg ataacaatct taatcagcta caagtcaaat tgaaggaaag cttaaagggc 840

aaaaaatttc ttattgttct ggatgatgtt tggaatgaca actataatgc atgggaggac 900

ttgaaaaatc tttttgttca aggaaatgca ggaagtacga tcattgtgac gacacgtaag 960

aaaagtgttg ccaagacgat gggtaatgag caaattagca tggatacttt gtctagtgat 1020

gtctcttggt ctttattcaa aagacatgca tttgacaaca tggatcctaa ggaacatcta 1080

gaacatgtag aagttgggaa agaaatcgta gctaagtgca aaggattgcc tttagctctg 1140

aagacgcttg ctggcatttt acgttccaaa tcagagattg aagggtggaa acgtattttg 1200

agaagtgaag tatgggagct gccagacaat ggcatattac cagtattgat gttgagctac 1260

agcgatcttc ctgcacattt aaagcaatgt ttttcctact gtgcaatatt tccgaaagat 1320

tatccatttc ggaaaaaaca agtcattcag ttgtggattg ccaatggtct agtacagggg 1380

ttgcaaaaat atgaaacaat tgaagattta ggcaacctat tctttctcga gttgcaatca 1440

aggtcacttt tcgaaagggt cccagagtct tctaaaaata atgcagagaa attcttaatg 1500

catgaccttg tcaatgattt ggcccaagtt gcatcttcaa aactttgtgt taggttggaa 1560

gagtaccaag agtctcatat gttgaaacga agtcgacaca tgtcctattc aatgggctat 1620

ggtgactttg aaaagttgca acctctctac aaattggagc agctcaggac attgcttcca 1680

atctacaata tcgagctata tggttcttct ctaagcaaga gggtgttgct taacatattg 1740

ccaagattaa catccttaag ggcactatca ttgtctcgtt ataatatcaa ggagttacca 1800

gatgtcttgt ttatcaaatt aaaactccta agattggtgg acctttcttt gacacagata 1860

atacagttac cggattcaat ttgtgtgctc tataacttgg agatacttct cttgtcatct 1920

tgtgaatttc ttaaggagtt accgcggcag atggagaagt tgataaactt gcgtcacctt 1980

gacattagcg gcagttctcg cttgatgatg ccgctacatt tgaccaagtt gaaaagcctt 2040

catgtgttat tgggagctaa atttcttgta ggtgatcgaa gtggttcaag aatggaagat 2100

ttgggtgaac tatgcaactt gtatggaact ctatcaattc aacagttgga aaatgtggcg 2160

gatagaaggg aagctttgaa agcaaacatg agcggtaagg aacatattga gaagttatta 2220

ttggagtgga gtgtaagtat tgcggacagt tcacaaaatg aaagagacat acttggtgag 2280

gtacatccaa atccaaacat aaaagaactt gagatcaacg gatatagagg gacaaacttt 2340

ccaaattggc tagctgatta ttcattttct gagctggtgg aattgtctct aagtaattgc 2400

aaggactgtt attccttgcc agcattaggc cagcttcctt ctttgaaatt ccttgcaatt 2460

cgagggatgc atcgaataat agaggtgact gaagaatttt atggtggttc gtcctccaaa 2520

aagcctttta actctcttga aaagcttgat tttgcagaga tgttggcgtg ggagcagtgg 2580

catgtactag gaaatgggga gttccctgta cttcaacacc tttcaattga agattgcccc 2640

aagttgattg gaaagttgcc tgaaaatctt tgttctctga caaaattgac aatttcacat 2700

tgtccgaaac tcaatctgga gacacctgta aaatttccaa gtctaaaaaa gtttgaagtt 2760

gaaggttctc ctaaggttgg agttcttttt gatcatgcgg aactgttttt gtctcaactt 2820

cagggaatga aacagatagt tgaattatac atcagtgatt gtcactctct tacctccttg 2880

cctattagca gtctgccaaa taccttgaaa gaaataagga taaagcgttg tgagaaattg 2940

aaattggagt cgtcaattgg taagatgatt tcaagaggaa gtaacatgtt tcttgaaagt 3000

ttggaactgg aagaatgtga ttctatagat gatgtatcac ctgagctggt cccatgtgca 3060

cgctatctga gagtagaaag ttgtcaaagc cttactagac tttttattcc caatggggct 3120

gaagatctca aaatcaataa atgtgagaat cttgaaatgc tttcggtggc tcagacgacg 3180

cccttgtgta acttgtttat tagcaactgc gagaagctga agtcgctgcc agaacatatg 3240

caggagctct ttccatctct tagagatctg tacctgaaaa attgtccaga aatagagtcc 3300

tttcctgaag gaggattgcc cttcaattta gaaatcctcg ggatcaggga ttgctgtgaa 3360

ctggtgaatg gccgaaagga gtggcattta cagggactcc cctctctcac atatttagac 3420

atctaccatc acggttctga aaactgggat attatgtggg agttgccttg ctctattcga 3480

agtcttacca tagacaattt gaaaacattt agcagccaag ttctcaaaag cctcacctct 3540

cttgaatctc tatgtacttc taatttacct caaattcagt cactcttgga agaagggctt 3600

cccacatctc ttttaaagct aacattatct gaccatggtg agctccattc cctaccgacc 3660

gacggtcttc agcgcctcat ttctcttcaa cgtctacgca tcgataattg ccctaatctc 3720

caatatgttc cagaatcaac gtttccctct tccctctctg agctacatat tagtagctgt 3780

agttttctcc aatcgcttcg agaatcagcg ctgtcctcct ccctctctaa tcttttcatc 3840

tacagttgcc ctaatctcca atctctaatg ctgccctcct ccctctttga gctgcatatc 3900

attgattgcc gtaatctcca atctcttcca gaatcagcgc tgcccccctc cctctctaag 3960

ttaatcatcc ttacatgccc taatctccaa tctcttccag taaaagggat gccctcttcc 4020

atctcttttc tgtctattat tgactgccca ttgctcaaac caagtctaga atttgagaag 4080

ggcgaatact ggccaaatat tgctcatatt cccaacatag tgatcgattg tgaatgcctg 4140

tga 4143

<210> 211
<211> 1380
<212> PRT
<213> Nicotiana sylvestris

<400> 211
Met Glu Ile Gly Leu Ala Val Gly Gly Ala Phe Leu Ser Ser Ala Leu
1               5                   10                  15
Asn Val Leu Phe Asp Arg Leu Ala Pro Gln Gly Glu Leu Leu Lys Met
            20                  25                  30
Phe Gln Arg Asp Lys His Asp Val Arg Leu Leu Lys Lys Leu Arg Ile
        35                  40                  45
Thr Leu Leu Gly Leu Gln Ala Val Leu Cys Asp Ala Glu Asn Lys Lys
    50                  55                  60
Ala Ser Asn Gln Tyr Val Ser Gln Trp Leu Ile Glu Leu Gln Asp Ala
65                  70                  75                  80
Val Asp Ser Ala Glu Asn Leu Met Glu Glu Ile Asn Tyr Glu Val Leu
                85                  90                  95
Arg Val Lys Val Glu Gly Gln Tyr Gln Asn Leu Gly Glu Thr Ser Asn
            100                 105                 110
Gln Gln Val Ser Asp Leu Asn Leu Cys Leu Ser Asp Glu Phe Phe Leu
        115                 120                 125
Asn Ile Lys Glu Lys Leu Glu Asp Ala Ile Glu Thr Leu Glu Glu Leu
    130                 135                 140
Glu Lys Gln Ile Gly Arg Leu Asp Leu Thr Lys Tyr Leu Asp Ser Asp
145                 150                 155                 160
Lys Gln Glu Thr Arg Arg Leu Ser Thr Ser Val Val Asp Asp Ser Asn
                165                 170                 175
Ile Phe Gly Arg Gln Asn Glu Ile Glu Glu Leu Val Gly Arg Leu Leu
            180                 185                 190
Ser Val Ala Val Asn Gly Lys Asn Leu Thr Val Ile Pro Ile Val Gly
        195                 200                 205
Met Ala Gly Ile Gly Lys Thr Thr Leu Ala Lys Ala Val Tyr Asn Asp
    210                 215                 220
Glu Lys Val Lys Tyr His Phe Asp Leu Lys Ala Trp Phe Cys Val Ser
225                 230                 235                 240

Glu Pro Tyr Asp Ala Phe Arg Ile Thr Lys Gly Leu Leu Gln Glu Ile
                245                 250                 255
Gly Ser Phe Asp Leu Lys Met Asp Asn Asn Leu Asn Gln Leu Gln Val
            260                 265                 270
Lys Leu Lys Glu Ser Leu Lys Gly Lys Lys Phe Leu Ile Val Leu Asp
        275                 280                 285
Asp Val Trp Asn Asp Asn Tyr Asn Ala Trp Glu Asp Leu Lys Asn Leu
    290                 295                 300
Phe Val Gln Gly Asn Ala Gly Ser Thr Ile Ile Val Thr Thr Arg Lys
305                 310                 315                 320
Lys Ser Val Ala Lys Thr Met Gly Asn Glu Gln Ile Ser Met Asp Thr
                325                 330                 335
Leu Ser Ser Asp Val Ser Trp Ser Leu Phe Lys Arg His Ala Phe Asp
            340                 345                 350
Asn Met Asp Pro Lys Glu His Leu Glu His Val Glu Val Gly Lys Glu
        355                 360                 365
Ile Val Ala Lys Cys Lys Gly Leu Pro Leu Ala Leu Lys Thr Leu Ala
    370                 375                 380
Gly Ile Leu Arg Ser Lys Ser Glu Ile Glu Gly Trp Lys Arg Ile Leu
385                 390                 395                 400
Arg Ser Glu Val Trp Glu Leu Pro Asp Asn Gly Ile Leu Pro Val Leu
                405                 410                 415
Met Leu Ser Tyr Ser Asp Leu Pro Ala His Leu Lys Gln Cys Phe Ser
            420                 425                 430
Tyr Cys Ala Ile Phe Pro Lys Asp Tyr Pro Phe Arg Lys Lys Gln Val
        435                 440                 445
Ile Gln Leu Trp Ile Ala Asn Gly Leu Val Gln Gly Leu Gln Lys Tyr
    450                 455                 460
Glu Thr Ile Glu Asp Leu Gly Asn Leu Phe Phe Leu Glu Leu Gln Ser
465                 470                 475                 480
Arg Ser Leu Phe Glu Arg Val Pro Glu Ser Ser Lys Asn Asn Ala Glu
                485                 490                 495
Lys Phe Leu Met His Asp Leu Val Asn Asp Leu Ala Gln Val Ala Ser
            500                 505                 510
Ser Lys Leu Cys Val Arg Leu Glu Glu Tyr Gln Glu Ser His Met Leu
        515                 520                 525
Lys Arg Ser Arg His Met Ser Tyr Ser Met Gly Tyr Gly Asp Phe Glu
    530                 535                 540
Lys Leu Gln Pro Leu Tyr Lys Leu Glu Gln Leu Arg Thr Leu Leu Pro
545                 550                 555                 560
Ile Tyr Asn Ile Glu Leu Tyr Gly Ser Ser Leu Ser Lys Arg Val Leu
                565                 570                 575
Leu Asn Ile Leu Pro Arg Leu Thr Ser Leu Arg Ala Leu Ser Leu Ser
            580                 585                 590
Arg Tyr Asn Ile Lys Glu Leu Pro Asp Val Leu Phe Ile Lys Leu Lys
        595                 600                 605
Leu Leu Arg Leu Val Asp Leu Ser Leu Thr Gln Ile Ile Gln Leu Pro
    610                 615                 620
Asp Ser Ile Cys Val Leu Tyr Asn Leu Glu Ile Leu Leu Leu Ser Ser
625                 630                 635                 640
Cys Glu Phe Leu Lys Glu Leu Pro Arg Gln Met Glu Lys Leu Ile Asn
                645                 650                 655
Leu Arg His Leu Asp Ile Ser Gly Ser Ser Arg Leu Met Met Pro Leu
            660                 665                 670
His Leu Thr Lys Leu Lys Ser Leu His Val Leu Leu Gly Ala Lys Phe
        675                 680                 685
Leu Val Gly Asp Arg Ser Gly Ser Arg Met Glu Asp Leu Gly Glu Leu
    690                 695                 700
Cys Asn Leu Tyr Gly Thr Leu Ser Ile Gln Gln Leu Glu Asn Val Ala
705                 710                 715                 720
Asp Arg Arg Glu Ala Leu Lys Ala Asn Met Ser Gly Lys Glu His Ile
                725                 730                 735
Glu Lys Leu Leu Leu Glu Trp Ser Val Ser Ile Ala Asp Ser Ser Gln

                740                 745                 750
Asn Glu Arg Asp Ile Leu Gly Glu Val His Pro Asn Pro Asn Ile Lys
        755                 760                 765
Glu Leu Glu Ile Asn Gly Tyr Arg Gly Thr Asn Phe Pro Asn Trp Leu
    770                 775                 780
Ala Asp Tyr Ser Phe Ser Glu Leu Val Glu Leu Ser Leu Ser Asn Cys
785                 790                 795                 800
Lys Asp Cys Tyr Ser Leu Pro Ala Leu Gly Gln Leu Pro Ser Leu Lys
                805                 810                 815
Phe Leu Ala Ile Arg Gly Met His Arg Ile Ile Glu Val Thr Glu Glu
            820                 825                 830
Phe Tyr Gly Gly Ser Ser Ser Lys Lys Pro Phe Asn Ser Leu Glu Lys
        835                 840                 845
Leu Asp Phe Ala Glu Met Leu Ala Trp Glu Gln Trp His Val Leu Gly
    850                 855                 860
Asn Gly Glu Phe Pro Val Leu Gln His Leu Ser Ile Glu Asp Cys Pro
865                 870                 875                 880
Lys Leu Ile Gly Lys Leu Pro Glu Asn Leu Cys Ser Leu Thr Lys Leu
                885                 890                 895
Thr Ile Ser His Cys Pro Lys Leu Asn Leu Glu Thr Pro Val Lys Phe
            900                 905                 910
Pro Ser Leu Lys Lys Phe Glu Val Glu Gly Ser Pro Lys Val Gly Val
        915                 920                 925
Leu Phe Asp His Ala Glu Leu Phe Leu Ser Gln Leu Gln Gly Met Lys
    930                 935                 940
Gln Ile Val Glu Leu Tyr Ile Ser Asp Cys His Ser Leu Thr Ser Leu
945                 950                 955                 960
Pro Ile Ser Ser Leu Pro Asn Thr Leu Lys Glu Ile Arg Ile Lys Arg
                965                 970                 975
Cys Glu Lys Leu Lys Leu Glu Ser Ser Ile Gly Lys Met Ile Ser Arg
            980                 985                 990
Gly Ser Asn Met Phe Leu Glu Ser Leu Glu Leu Glu Glu Cys Asp Ser
        995                 1000                1005
Ile Asp Asp Val Ser Pro Glu Leu Val Pro Cys Ala Arg Tyr Leu Arg
    1010                1015                1020
Val Glu Ser Cys Gln Ser Leu Thr Arg Leu Phe Ile Pro Asn Gly Ala
1025                1030                1035                1040
Glu Asp Leu Lys Ile Asn Lys Cys Glu Asn Leu Glu Met Leu Ser Val
                1045                1050                1055
Ala Gln Thr Thr Pro Leu Cys Asn Leu Phe Ile Ser Asn Cys Glu Lys
            1060                1065                1070
Leu Lys Ser Leu Pro Glu His Met Gln Glu Leu Phe Pro Ser Leu Arg
        1075                1080                1085
Asp Leu Tyr Leu Lys Asn Cys Pro Glu Ile Glu Ser Phe Pro Glu Gly
    1090                1095                1100
Gly Leu Pro Phe Asn Leu Glu Ile Leu Gly Ile Arg Asp Cys Cys Glu
1105                1110                1115                1120
Leu Val Asn Gly Arg Lys Glu Trp His Leu Gln Gly Leu Pro Ser Leu
                1125                1130                1135
Thr Tyr Leu Asp Ile Tyr His His Gly Ser Glu Asn Trp Asp Ile Met
            1140                1145                1150
Trp Glu Leu Pro Cys Ser Ile Arg Ser Leu Thr Ile Asp Asn Leu Lys
        1155                1160                1165
Thr Phe Ser Ser Gln Val Leu Lys Ser Leu Thr Ser Leu Glu Ser Leu
    1170                1175                1180
Cys Thr Ser Asn Leu Pro Gln Ile Gln Ser Leu Leu Glu Glu Gly Leu
1185                1190                1195                1200
Pro Thr Ser Leu Leu Lys Leu Thr Leu Ser Asp His Gly Glu Leu His
                1205                1210                1215
Ser Leu Pro Thr Asp Gly Leu Gln Arg Leu Ile Ser Leu Gln Arg Leu
            1220                1225                1230
Arg Ile Asp Asn Cys Pro Asn Leu Gln Tyr Val Pro Glu Ser Thr Phe
        1235                1240                1245

Pro Ser Ser Leu Ser Glu Leu His Ile Ser Ser Cys Ser Phe Leu Gln
    1250                1255                1260
Ser Leu Arg Glu Ser Ala Leu Ser Ser Ser Leu Ser Asn Leu Phe Ile
1265                1270                1275                1280
Tyr Ser Cys Pro Asn Leu Gln Ser Leu Met Leu Pro Ser Ser Leu Phe
                1285                1290                1295
Glu Leu His Ile Ile Asp Cys Arg Asn Leu Gln Ser Leu Pro Glu Ser
            1300                1305                1310
Ala Leu Pro Pro Ser Leu Ser Lys Leu Ile Ile Leu Thr Cys Pro Asn
        1315                1320                1325
Leu Gln Ser Leu Pro Val Lys Gly Met Pro Ser Ser Ile Ser Phe Leu
    1330                1335                1340
Ser Ile Ile Asp Cys Pro Leu Leu Lys Pro Ser Leu Glu Phe Glu Lys
1345                1350                1355                1360
Gly Glu Tyr Trp Pro Asn Ile Ala His Ile Pro Asn Ile Val Ile Asp
                1365                1370                1375
Cys Glu Cys Leu
            1380

<210> 212
<211> 2814
<212> DNA
<213> Artificial Sequence

<220>
<223> cDNA

<400> 212
atggcttatg ctgctgttac ttcccttatg agaaccatac atcaatcaat ggaacttact      60

ggatgtgatt tgcaaccgtt ttatgaaaag ctcaaatctt tgagagctat tctggagaaa     120

tcctgcaata taatgggcga tcatgagggg ttaacaatct tggaagttga aatcgtagag     180

gtagcataca caacagaaga tatggttgac tcggaatcaa gaaatgtttt tttagcacag     240

aatttggagg aaagaagcag ggctatgtgg gagatttttt tcgtcctgga acaagcacta     300

gaatgcattg attccaccgt gaaacagtgg atggcaacat cggacagcat gaaagatcta     360

aaaccacaaa ctagctcgct tgtcagttta cctgaacatg atgttgagca gcccgagaat     420

ataatggttg gccgtgaaaa tgaatttgag atgatgctgg atcaacttgc tagaggagga     480

agggaactag aagttgtctc aatcgtaggg atgggaggca tcgggaaaac aactttggct     540

acaaaactct atagtgatcc gtgcattatg tctcgatttg atattcgtgc aaaagcaact     600

gtttcacaag agtattgtgt gagaaatgta ctcctaggcc ttctttcttt gacaagtgat     660

gaacctgatg atcagctagc ggaccgactg caaaagcatc tgaaaggcag gagatacttg     720

gtagtcattg atgacatatg gactacagaa gcttgggatg atataaaact atgtttccca     780

gactgttata atggaagcag aatactcctg actactcgga atgtggaagt ggctgaatat     840

gctagttcag gtaagcctcc tcatcacatg cgcctcatga attttgacga aagttggaat     900

ttactacaca aaaagatctt tgaaaaagaa ggttcttatt ctcctgaatt tgaaaatatt     960

gggaaacaaa ttgcattaaa atgtggagga ttacctctag caattactgt gattgctgga    1020

cttctctcca aaatgggtca aagattagat gagtggcaaa gaattgggga aaatgtaagt 1080

tcggtcgtta gcacagatcc tgaagcacaa tgcatgagag tgttggcttt gagttaccat 1140

cacttgcctt ctcacctaaa accgtgtttt ctgtattttg caattttcac agaggatgaa 1200

cagatttctg taaatgaact tgttgagtta tggcctgtag agggattttt gaatgaagaa 1260

gagggaaaaa gcatagaaga ggtggcaaca acatgtataa acgaacttat agatagaagc 1320

ttaattttca tccacaattt tagttttcgt ggaacaatag aaagttgtgg aatgcatgat 1380

gtgacccgtg aactctgttt gagggaagct cgaaacatga attttgtgaa tgttatcaga 1440

ggaaagagtg atcaaaattc atgtgcacaa tccatgcagc gttcctttaa gagtcgaagt 1500

cggatcagaa tccataaggt ggaagaattg gcttggtgtc gtaacagtga ggctcattct 1560

attatcatgt tgggtggatt cgaatgcgtc acactggaat tgtctttcaa gctagtaaga 1620

gtactagatc ttggtttgaa tacatggcca atttttccca gtggagtact ttctctaatt 1680

catttgagat acctatcttt gcgttttaat ccttgcttac agcagtatca aggatcgaaa 1740

gaagctgttc cctcatcaat aatagacatt cctctatcga tatcaagcct atgctatctg 1800

caaactttta aacttaacct tccatttccc agttattatc ctttcatatt accatcggaa 1860

attttgacga tgccacaatt gaggacgctg tgtatgggct ggaattactt gcggagtcat 1920

gagcctacag agaacagatt ggttttgaaa aatttgcaat gcctcaatca attgaaccct 1980

cggtattgta cagggtcttt ttttagacta tttcccaatt taaagaagtt gcaagtattt 2040

ggcgtcccag aagactttcg caatagccag gacctgtatg attttcgcta cttatatcag 2100

ctcgaagaat tgacatttcg tttatattat ccatatgctg cttgctttct aaaaaacact 2160

gcaccttcag gttctacgca agatcctctg aggtttcaga cggaaatatt gcacaaagag 2220

attgatttcg ggggaactgc acctccaact ttactcttac ctcctccgga tgcttttcca 2280

caaaacctta agagtttaac ttttagggga gaattctctg tggcatggaa ggatttgagc 2340

attgttggta aattacccaa actcgaggtc cttatactat catggaatgc cttcataggc 2400

aaggagtggg aagtagttga ggaagggttt cctcacttga agttcttgtt tctggatgat 2460

gtatacattc gatactggag agctagtagt gatcactttc cgtaccttga acgagttatt 2520

cttagagatt gccgtaattt ggattcaatc cctcgagatt ttgcagatat aaccacacta 2580

gctcttattg atatagatta ctgtcaacaa tctgttgtga attccgccaa gcaaattcaa 2640

caggacattc aagacaacta tggaagctct atcgaggtcc atactcgtca tcttttcatt 2700

cccaagagtg tgacaacagt tgaagatgat gatgatagtg tgacaacaga tgaagatgat 2760

gatgatgatg actttgagaa agaagttgct tcttgccgca ataatgtcga gtag 2814

<210> 213
<211> 937

<212> PRT
<213> Solanum tuberosum

<400> 213
Met Ala Tyr Ala Ala Val Thr Ser Leu Met Arg Thr Ile His Gln Ser
1               5                   10                  15
Met Glu Leu Thr Gly Cys Asp Leu Gln Pro Phe Tyr Glu Lys Leu Lys
            20                  25                  30
Ser Leu Arg Ala Ile Leu Glu Lys Ser Cys Asn Ile Met Gly Asp His
        35                  40                  45
Glu Gly Leu Thr Ile Leu Glu Val Glu Ile Val Glu Val Ala Tyr Thr
    50                  55                  60
Thr Glu Asp Met Val Asp Ser Glu Ser Arg Asn Val Phe Leu Ala Gln
65                  70                  75                  80
Asn Leu Glu Glu Arg Ser Arg Ala Met Trp Glu Ile Phe Phe Val Leu
                85                  90                  95
Glu Gln Ala Leu Glu Cys Ile Asp Ser Thr Val Lys Gln Trp Met Ala
            100                 105                 110
Thr Ser Asp Ser Met Lys Asp Leu Lys Pro Gln Thr Ser Ser Leu Val
        115                 120                 125
Ser Leu Pro Glu His Asp Val Glu Gln Pro Glu Asn Ile Met Val Gly
    130                 135                 140
Arg Glu Asn Glu Phe Glu Met Met Leu Asp Gln Leu Ala Arg Gly Gly
145                 150                 155                 160
Arg Glu Leu Glu Val Val Ser Ile Val Gly Met Gly Gly Ile Gly Lys
                165                 170                 175
Thr Thr Leu Ala Thr Lys Leu Tyr Ser Asp Pro Cys Ile Met Ser Arg
            180                 185                 190
Phe Asp Ile Arg Ala Lys Ala Thr Val Ser Gln Glu Tyr Cys Val Arg
        195                 200                 205
Asn Val Leu Leu Gly Leu Leu Ser Leu Thr Ser Asp Glu Pro Asp Asp
    210                 215                 220
Gln Leu Ala Asp Arg Leu Gln Lys His Leu Lys Gly Arg Arg Tyr Leu
225                 230                 235                 240
Val Val Ile Asp Asp Ile Trp Thr Thr Glu Ala Trp Asp Asp Ile Lys
                245                 250                 255
Leu Cys Phe Pro Asp Cys Tyr Asn Gly Ser Arg Ile Leu Leu Thr Thr
            260                 265                 270
Arg Asn Val Glu Val Ala Glu Tyr Ala Ser Ser Gly Lys Pro Pro His
        275                 280                 285
His Met Arg Leu Met Asn Phe Asp Glu Ser Trp Asn Leu Leu His Lys
    290                 295                 300
Lys Ile Phe Glu Lys Glu Gly Ser Tyr Ser Pro Glu Phe Glu Asn Ile
305                 310                 315                 320
Gly Lys Gln Ile Ala Leu Lys Cys Gly Gly Leu Pro Leu Ala Ile Thr
                325                 330                 335
Val Ile Ala Gly Leu Leu Ser Lys Met Gly Gln Arg Leu Asp Glu Trp
            340                 345                 350
Gln Arg Ile Gly Glu Asn Val Ser Ser Val Val Ser Thr Asp Pro Glu
        355                 360                 365
Ala Gln Cys Met Arg Val Leu Ala Leu Ser Tyr His His Leu Pro Ser
    370                 375                 380
His Leu Lys Pro Cys Phe Leu Tyr Phe Ala Ile Phe Thr Glu Asp Glu
385                 390                 395                 400
Gln Ile Ser Val Asn Glu Leu Val Glu Leu Trp Pro Val Glu Gly Phe
                405                 410                 415
Leu Asn Glu Glu Glu Gly Lys Ser Ile Glu Glu Val Ala Thr Thr Cys
            420                 425                 430
Ile Asn Glu Leu Ile Asp Arg Ser Leu Ile Phe Ile His Asn Phe Ser
        435                 440                 445
Phe Arg Gly Thr Ile Glu Ser Cys Gly Met His Asp Val Thr Arg Glu
    450                 455                 460

Leu Cys Leu Arg Glu Ala Arg Asn Met Asn Phe Val Asn Val Ile Arg
465                 470                 475                 480
Gly Lys Ser Asp Gln Asn Ser Cys Ala Gln Ser Met Gln Arg Ser Phe
                485                 490                 495
Lys Ser Arg Ser Arg Ile Arg Ile His Lys Val Glu Glu Leu Ala Trp
            500                 505                 510
Cys Arg Asn Ser Glu Ala His Ser Ile Ile Met Leu Gly Gly Phe Glu
        515                 520                 525
Cys Val Thr Leu Glu Leu Ser Phe Lys Leu Val Arg Val Leu Asp Leu
    530                 535                 540
Gly Leu Asn Thr Trp Pro Ile Phe Pro Ser Gly Val Leu Ser Leu Ile
545                 550                 555                 560
His Leu Arg Tyr Leu Ser Leu Arg Phe Asn Pro Cys Leu Gln Gln Tyr
                565                 570                 575
Gln Gly Ser Lys Glu Ala Val Pro Ser Ser Ile Ile Asp Ile Pro Leu
            580                 585                 590
Ser Ile Ser Ser Leu Cys Tyr Leu Gln Thr Phe Lys Leu Asn Leu Pro
        595                 600                 605
Phe Pro Ser Tyr Tyr Pro Phe Ile Leu Pro Ser Glu Ile Leu Thr Met
    610                 615                 620
Pro Gln Leu Arg Thr Leu Cys Met Gly Trp Asn Tyr Leu Arg Ser His
625                 630                 635                 640
Glu Pro Thr Glu Asn Arg Leu Val Leu Lys Asn Leu Gln Cys Leu Asn
                645                 650                 655
Gln Leu Asn Pro Arg Tyr Cys Thr Gly Ser Phe Phe Arg Leu Phe Pro
            660                 665                 670
Asn Leu Lys Lys Leu Gln Val Phe Gly Val Pro Glu Asp Phe Arg Asn
        675                 680                 685
Ser Gln Asp Leu Tyr Asp Phe Arg Tyr Leu Tyr Gln Leu Glu Glu Leu
    690                 695                 700
Thr Phe Arg Leu Tyr Tyr Pro Tyr Ala Ala Cys Phe Leu Lys Asn Thr
705                 710                 715                 720
Ala Pro Ser Gly Ser Thr Gln Asp Pro Leu Arg Phe Gln Thr Glu Ile
                725                 730                 735
Leu His Lys Glu Ile Asp Phe Gly Gly Thr Ala Pro Pro Thr Leu Leu
            740                 745                 750
Leu Pro Pro Pro Asp Ala Phe Pro Gln Asn Leu Lys Ser Leu Thr Phe
        755                 760                 765
Arg Gly Glu Phe Ser Val Ala Trp Lys Asp Leu Ser Ile Val Gly Lys
    770                 775                 780
Leu Pro Lys Leu Glu Val Leu Ile Leu Ser Trp Asn Ala Phe Ile Gly
785                 790                 795                 800
Lys Glu Trp Glu Val Val Glu Glu Gly Phe Pro His Leu Lys Phe Leu
                805                 810                 815
Phe Leu Asp Asp Val Tyr Ile Arg Tyr Trp Arg Ala Ser Ser Asp His
            820                 825                 830
Phe Pro Tyr Leu Glu Arg Val Ile Leu Arg Asp Cys Arg Asn Leu Asp
        835                 840                 845
Ser Ile Pro Arg Asp Phe Ala Asp Ile Thr Thr Leu Ala Leu Ile Asp
    850                 855                 860
Ile Asp Tyr Cys Gln Gln Ser Val Val Asn Ser Ala Lys Gln Ile Gln
865                 870                 875                 880
Gln Asp Ile Gln Asp Asn Tyr Gly Ser Ser Ile Glu Val His Thr Arg
                885                 890                 895
His Leu Phe Ile Pro Lys Ser Val Thr Thr Val Glu Asp Asp Asp Asp
            900                 905                 910
Ser Val Thr Thr Asp Glu Asp Asp Asp Asp Asp Asp Phe Glu Lys Glu
        915                 920                 925
Val Ala Ser Cys Arg Asn Asn Val Glu
    930                 935

<210> 214
<211> 3159

<212> DNA
<213> Solanum tuberosum

<400> 214
atggcttatg ctgctgttac ttcccttatg agaaccatac atcaatcaat ggaacttact 60

ggatgtgatt tgcaaccgtt ttatgaaaag ctcaaatctt tgagagctat tctggagaaa 120

tcctgcaata taatgggcga tcatgagggg ttaacaatct tggaagttga aatcgtagag 180

gtagcataca caacagaaga tatggttgac tcggaatcaa gaaatgtttt tttagcacag 240

aatttggagg aaagaagcag ggctatgtgg gagatttttt tcgtcctgga acaagcacta 300

gaatgcattg attccaccgt gaaacagtgg atggcaacat cggacagcat gaaagatcta 360

aaaccacaaa ctagctcgct tgtcagttta cctgaacatg atgttgagca gcccgagaat 420

ataatggttg gccgtgaaaa tgaatttgag atgatgctgg atcaacttgc tagaggagga 480

agggaactag aagttgtctc aatcgtaggg atgggaggca tcgggaaaac aactttggct 540

acaaaactct atagtgatcc gtgcattatg tctcgatttg atattcgtgc aaaagcaact 600

gtttcacaag agtattgtgt gagaaatgta ctcctaggcc ttctttcttt gacaagtgat 660

gaacctgatg atcagctagc ggaccgactg caaaagcatc tgaaaggcag gagatacttg 720

gtagtcattg atgacatatg gactacagaa gcttgggatg atataaaact atgtttccca 780

gactgttata atggaagcag aatactcctg actactcgga atgtggaagt ggctgaatat 840

gctagttcag gtaagcctcc tcatcacatg cgcctcatga attttgacga aagttggaat 900

ttactacaca aaaagatctt tgaaaaagaa ggttcttatt ctcctgaatt tgaaaatatt 960

gggaaacaaa ttgcattaaa atgtggagga ttacctctag caattactgt gattgctgga 1020

cttctctcca aaatgggtca aagattagat gagtggcaaa gaattgggga aaatgtaagt 1080

tcggtcgtta gcacagatcc tgaagcacaa tgcatgagag tgttggcttt gagttaccat 1140

cacttgcctt ctcacctaaa accgtgtttt ctgtattttg caattttcac agaggatgaa 1200

cagatttctg taaatgaact tgttgagtta tggcctgtag agggattttt gaatgaagaa 1260

gagggaaaaa gcatagaaga ggtggcaaca acatgtataa acgaacttat agatagaagc 1320

ttaattttca tccacaattt tagttttcgt ggaacaatag aaagttgtgg aatgcatgat 1380

gtgacccgtg aactctgttt gagggaagct cgaaacatga attttgtgaa tgttatcaga 1440

ggaaagagtg atcaaaattc atgtgcacaa tccatgcagc gttcctttaa gagtcgaagt 1500

cggatcagaa tccataaggt ggaagaattg gcttggtgtc gtaacagtga ggctcattct 1560

attatcatgt tgggtggatt cgaatgcgtc acactggaat tgtctttcaa gctagtaaga 1620

gtactagatc ttggtttgaa tacatggcca attttcccca gtggagtact ttctctaatt 1680

catttgagat acctatcttt gcgttttaat ccttgcttac agcagtatca aggatcgaaa 1740

gaagctgttc cctcatcaat aatagacatt cctctatcga tatcaagcct atgctatctg 1800

caaactttta aacttaacct tccatttccc agttattatc ctttcatatt accatcggaa 1860

attttgacga tgccacaatt gaggacgctg tgtatgggct ggaattactt gcggagtcat 1920

gagcctacag agaacagatt ggttttgaaa aatttgcaat gcctcaatca attgaaccct 1980

cggtattgta cagggtcttt ttttagacta tttcccaatt taaagaagtt gcaagtattt 2040

ggcgtcccag aagactttcg caatagccag gacctgtatg attttcgcta cttatatcag 2100

ctcgaagaat tgacatttcg tttatattat ccatatgctg cttgctttct aaaaaacact 2160

gcaccttcag gttctacgca agatcctctg aggtttcaga cggaaatatt gcacaaagag 2220

attgatttcg ggggaactgc acctccaact ttactcttac ctcctccgga tgcttttcca 2280

caaaacctta agagtttaac ttttagggga gaattctctg tggcatggaa ggatttgagc 2340

attgttggta aattacccaa actcgaggtc cttatactat catggaatgc cttcataggc 2400

aaggagtggg aagtagttga ggaagggttt cctcacttga agttcttgtt tctggatgat 2460

gtatacattc gatactggag agctagtagt gatcactttc cgtaccttga acgagttatt 2520

cttagagatt gccgtaattt ggattcaatc cctcgagatt ttgcagatat aaccacacta 2580

gctcttattg atatagatta ctgtcaacaa tctgttgtga attccgccaa gcaaattcaa 2640

caggacattc aagacaacta tggaagctct atcgaggtcc atactcgtca tcttttgtaa 2700

gacatcttct tccttgcttt acaacaataa ttaactcatc atcatagtaa actcgataat 2760

aatctggata atagccttag taagtcaaat tgcaccaatt caacaaaagt tcttgatgct 2820

gtcattgtgt ttgatttgaa tccttccaat attgtgtaac ttgttatact tgcatgttca 2880

ttcttgattt tgggaagtgt aacatttcat ttttcatctt ttgtggctag cattcccaag 2940

agtgtgacaa cagttgaaga tgatgatgat agtgtgacaa cagatgaaga tgatgatgat 3000

gatgactttg agaaagaagt tgcttcttgc cgcaataatg tgtaagttct tatacctgca 3060

tgctcattct tgctataatg ttctcttgtt ccttaattat gggacatctg acatattatt 3120

ttccatgttt tgcgtctttt atttttctgc agcgagtag 3159

<210> 215
<211> 714
<212> DNA
<213> Artificial Sequence

<220>
<223> cDNA

<400> 215
atgtcagcac cagctagcac aacacaggcc acagggtcaa ctacctcaac taccgcaaaa 60

actgcaggcg caactcctgc cacagcttca ggactgttca ccatcccgga tggggatttc 120

tttagtacag cccgcgctat agtagccagc aatgccgttg caacaaatga ggacctcagc 180

aagattgagg ctatctggaa ggacatgaag gtgcccacag acactatggc acaggctgcc 240

tgggacttag tcagacactg cgctgacgtg ggctcatctg ctcaaacaga aatgatagat 300

acaggtccct attccaacgg catcagcaga gccagactgg cagcagcaat taaagaggtg 360

tgcatactta ggcaattttg catgaagtat gccccagtgg tatggaactg gatgctgact 420

aacaacagtc cacctgctaa ctggcaagca caaggcttca agcctgagca caaattcgct 480

gcattcgact tcttcaatgg agtcaccaac ccagctgcca tcatgcccaa agaggggctc 540

atccggccac cgtctgaagc tgaaatgaat gctgcccaaa ctgctgcctt tgtgaagatt 600

acgaaggcca gggcacaatc caacgacttt gccagcctag atgcagctgt cactcgaggt 660

cgtatcactg gaacaacaac cgctgaggct gttgtcactc tcccaccacc ataa 714

<210> 216
<211> 237
<212> PRT
<213> Potato virus X

<400> 216
Met Ser Ala Pro Ala Ser Thr Thr Gln Ala Thr Gly Ser Thr Thr Ser
1               5                   10                  15
Thr Thr Ala Lys Thr Ala Gly Ala Thr Pro Ala Thr Ala Ser Gly Leu
            20                  25                  30
Phe Thr Ile Pro Asp Gly Asp Phe Phe Ser Thr Ala Arg Ala Ile Val
        35                  40                  45
Ala Ser Asn Ala Val Ala Thr Asn Glu Asp Leu Ser Lys Ile Glu Ala
    50                  55                  60
Ile Trp Lys Asp Met Lys Val Pro Thr Asp Thr Met Ala Gln Ala Ala
65                  70                  75                  80
Trp Asp Leu Val Arg His Cys Ala Asp Val Gly Ser Ser Ala Gln Thr
                85                  90                  95
Glu Met Ile Asp Thr Gly Pro Tyr Ser Asn Gly Ile Ser Arg Ala Arg
            100                 105                 110
Leu Ala Ala Ala Ile Lys Glu Val Cys Ile Leu Arg Gln Phe Cys Met
        115                 120                 125
Lys Tyr Ala Pro Val Val Trp Asn Trp Met Leu Thr Asn Asn Ser Pro
    130                 135                 140
Pro Ala Asn Trp Gln Ala Gln Gly Phe Lys Pro Glu His Lys Phe Ala
145                 150                 155                 160
Ala Phe Asp Phe Phe Asn Gly Val Thr Asn Pro Ala Ala Ile Met Pro
                165                 170                 175
Lys Glu Gly Leu Ile Arg Pro Pro Ser Glu Ala Glu Met Asn Ala Ala
            180                 185                 190
Gln Thr Ala Ala Phe Val Lys Ile Thr Lys Ala Arg Ala Gln Ser Asn
        195                 200                 205
Asp Phe Ala Ser Leu Asp Ala Ala Val Thr Arg Gly Arg Ile Thr Gly
    210                 215                 220
Thr Thr Thr Ala Glu Ala Val Val Thr Leu Pro Pro Pro
225                 230                 235

<210> 217
<211> 2817
<212> DNA
<213> Artificial Sequence

<220>
<223> cDNA

<400> 217
atggcttatg ctgctgttac ttcccttatg agaaccatac atcaatcaat ggaacttact      60

ggatgtgatt tgcaaccgtt ttatgaaaag ctcgaatctt taagagctat tctggagaaa     120

tcctgcaatg taacgggcga tcatgaggag ttaacaatct tggaagttga aatcttagag     180

gtagcataca caacagaaga tatggttgac tcggaatcaa gaagtgtttt tttagcacag     240

aatttggagg aaagaaacag ggctatgtgg gagatttttt tcgtcttgga acaagcacta     300

gaatgcattg attccaccgt gaaacagtgg atggcaacat cggacagcat gaaagatcta     360

aaaccacaaa ctagctcact tgtcagttta cctgaccatg cttttgagca gcctgagaat     420

ataatggttg gccgtgaaaa tgaatttgag atgatgctgg atcaacttgt tagaggagga     480

agggaactag aagttgtctc aatcgtaggg atgggaggta tcgggaaaac aactttggct     540

acaaaactct atagtgatcc ttacattatg tctcgatttg atattcgtgc aaaagcaact     600

gtttcacaag agtattgtgt gagaaatgta ctcctaggcc ttctttcttt gacaagtgat     660

gaacctgatg atcaactagc ggaccgactg caaaagcatc tgaaaggcag gagatacttg     720

gtagtcattg atgacatatg gactacaaaa gcttgggatg gtataaaact atgtttccca     780

gactgttata agggaagcag aatactcctg actactcgga atgtggaagt ggctgaatat     840

gctagttcag gtaagcctcc tcatcacatg cgcctcatga attttgacga aagttggaat     900

ttactacaca aaaagatctt tgaaaaagaa ggttcttatt ctcctgaatt tgaaaatatt     960

gggaaacaaa ttgcattaaa atgtggagga ttacctctag caattactgt gattgctgga    1020

cttctctcca aaatcagtaa aacattggat gagtggcaaa atgttgcgga gaatgtaagt    1080

tcggtggtaa gcacagatct tgaagcaaaa tgcatgagag tgttggcttt gagttaccat    1140

cacttgcctt ctcacctaaa accgtgtttt ctatattttg caattttcgc agaggatgaa    1200

cggatttctg taactaaact tgttgagtta tgggctgtag aggggttttt gaatgaagaa    1260

gagggaaaaa gcatagaaga ggtggcagaa acatgtataa acgagcttgt agatagaagt    1320

ctaatttcta tccacaattt gagttttgat gggaaaatag aaagttgtgg aatgcatgat    1380

gtgacccgtg aactctgttt gagggaagct cgaaacatga attttgtaaa tgttatcaga    1440

ggaaagagtg atcaaaattc atgtgcacaa tccatgcagc gttcctttaa gagtcgaagt    1500

cggatcagaa tccataagct ggaagaattg gcttggtgtc gtaacagtga ggctcattct    1560

attatcatgt tgggtggatt cgaatgcgtc acactggaat tgtctttcaa gctagtaaga    1620

gtactagatc ttggtttgaa tacatggcca atttttccca gtggagtact ttctctaatt    1680

catttgagat acctatcttt gcgttttaat ccttgcttac agcagtatca aggatcgaaa    1740

gaagctgttc cctcatcaat aatagacatt cctctatcga tatcaagcct atgctatctg 1800

caaactttta aacttaacct tccatttccc agttattatc ctttcatatt accatcggaa 1860

attttgacga tgccacaatt gaggacgctg tgtatggact ggaattactt gcggagtcat 1920

gagcctacag agaacagatt ggttttgaaa aatttgcaat gcctcaatca attgaaccct 1980

cggtattgta cagggtcttt ttttagacta tttcccaatt taaagaagtt gcaagtattt 2040

ggcgtcccag aagactttcg caatagccag gacctgtatg attttcgcta cttatatcag 2100

ctcgaagaat tgacatttcg tttatattat ccatatgctg cttgctttct aaaaaacact 2160

gcaccttcag gttctactcc gcaagatcct ctgaggtttc agacggaaat attgcacaaa 2220

gagattgatt tcgggggaac tgcacctcca actttactct tacctcctcc ggatgctttt 2280

ccacaaaacc ttaagagttt aacttttagg ggagaattct ctgtggcatg gaaggatttg 2340

agcattgttg gtaaattacc caaactcgag gtccttatac tatcatggaa tgccttcata 2400

ggcaaggagt gggaagtagt tgaggaaggg tttcctcact tgaacttctt gtttctggat 2460

gatgtataca ttcgatactg gagagctagt agtgatcact ttccgtacct tgaacgactt 2520

attcttagag attgccgtaa tttggattca atccctcgag attttgcaga tataaccaca 2580

ctagctctta ttgatataga ttactgtcaa caatctgttg tgaattccgc caagcaaatt 2640

caacaggaca ttcaagacaa ctatggaagc tctatcgagg tccatactcg tcatcttttc 2700

attcccaaga gtgtgacaac agttgaagat gatgatgata gtgtgacaac agatgaagat 2760

gatgatgatg atgactttga gaaagaagtt gcttcttgcc gcaataatgt cgagtag 2817

<210> 218
<211> 938
<212> PRT
<213> Solanum acaule

<400> 218
   Met Ala Tyr Ala Ala Val Thr Ser Leu Met Arg Thr Ile His Gln Ser
   1 5 10 15
   Met Glu Leu Thr Gly Cys Asp Leu Gln Pro Phe Tyr Glu Lys Leu Glu
   20 25 30
   Ser Leu Arg Ala Ile Leu Glu Lys Ser Cys Asn Val Thr Gly Asp His
   35 40 45
   Glu Glu Leu Thr Ile Leu Glu Val Glu Ile Leu Glu Val Ala Tyr Thr
   50 55 60
   Thr Glu Asp Met Val Asp Ser Glu Ser Arg Ser Val Phe Leu Ala Gln
   65 70 75 80
   Asn Leu Glu Glu Arg Asn Arg Ala Met Trp Glu Ile Phe Phe Val Leu
   85 90 95
   Glu Gln Ala Leu Glu Cys Ile Asp Ser Thr Val Lys Gln Trp Met Ala
   100 105 110
   Thr Ser Asp Ser Met Lys Asp Leu Lys Pro Gln Thr Ser Ser Leu Val
   115 120 125
   Ser Leu Pro Asp His Ala Phe Glu Gln Pro Glu Asn Ile Met Val Gly
   130 135 140
   Arg Glu Asn Glu Phe Glu Met Met Leu Asp Gln Leu Val Arg Gly Gly
   145                 150                 155                 160
   Arg Glu Leu Glu Val Val Ser Ile Val Gly Met Gly Gly Ile Gly Lys
                   165                 170                 175
   Thr Thr Leu Ala Thr Lys Leu Tyr Ser Asp Pro Tyr Ile Met Ser Arg
               180                 185                 190
   Phe Asp Ile Arg Ala Lys Ala Thr Val Ser Gln Glu Tyr Cys Val Arg
           195                 200                 205
   Asn Val Leu Leu Gly Leu Leu Ser Leu Thr Ser Asp Glu Pro Asp Asp
       210                 215                 220
   Gln Leu Ala Asp Arg Leu Gln Lys His Leu Lys Gly Arg Arg Tyr Leu
   225                 230                 235                 240
   Val Val Ile Asp Asp Ile Trp Thr Thr Lys Ala Trp Asp Gly Ile Lys
                   245                 250                 255
   Leu Cys Phe Pro Asp Cys Tyr Lys Gly Ser Arg Ile Leu Leu Thr Thr
               260                 265                 270
   Arg Asn Val Glu Val Ala Glu Tyr Ala Ser Ser Gly Lys Pro Pro His
           275                 280                 285
   His Met Arg Leu Met Asn Phe Asp Glu Ser Trp Asn Leu Leu His Lys
       290                 295                 300
   Lys Ile Phe Glu Lys Glu Gly Ser Tyr Ser Pro Glu Phe Glu Asn Ile
   305                 310                 315                 320
   Gly Lys Gln Ile Ala Leu Lys Cys Gly Gly Leu Pro Leu Ala Ile Thr
                   325                 330                 335
   Val Ile Ala Gly Leu Leu Ser Lys Ile Ser Lys Thr Leu Asp Glu Trp
               340                 345                 350
   Gln Asn Val Ala Glu Asn Val Ser Ser Val Val Ser Thr Asp Leu Glu
           355                 360                 365
   Ala Lys Cys Met Arg Val Leu Ala Leu Ser Tyr His His Leu Pro Ser
       370                 375                 380
   His Leu Lys Pro Cys Phe Leu Tyr Phe Ala Ile Phe Ala Glu Asp Glu
   385                 390                 395                 400
   Arg Ile Ser Val Thr Lys Leu Val Glu Leu Trp Ala Val Glu Gly Phe
                   405                 410                 415
   Leu Asn Glu Glu Glu Gly Lys Ser Ile Glu Glu Val Ala Glu Thr Cys
               420                 425                 430
   Ile Asn Glu Leu Val Asp Arg Ser Leu Ile Ser Ile His Asn Leu Ser
           435                 440                 445
   Phe Asp Gly Lys Ile Glu Ser Cys Gly Met His Asp Val Thr Arg Glu
       450                 455                 460
   Leu Cys Leu Arg Glu Ala Arg Asn Met Asn Phe Val Asn Val Ile Arg
   465                 470                 475                 480
   Gly Lys Ser Asp Gln Asn Ser Cys Ala Gln Ser Met Gln Arg Ser Phe
                   485                 490                 495
   Lys Ser Arg Ser Arg Ile Arg Ile His Lys Leu Glu Glu Leu Ala Trp
               500                 505                 510
   Cys Arg Asn Ser Glu Ala His Ser Ile Ile Met Leu Gly Gly Phe Glu
           515                 520                 525
   Cys Val Thr Leu Glu Leu Ser Phe Lys Leu Val Arg Val Leu Asp Leu
       530                 535                 540
   Gly Leu Asn Thr Trp Pro Ile Phe Pro Ser Gly Val Leu Ser Leu Ile
   545                 550                 555                 560
   His Leu Arg Tyr Leu Ser Leu Arg Phe Asn Pro Cys Leu Gln Gln Tyr
                   565                 570                 575
   Gln Gly Ser Lys Glu Ala Val Pro Ser Ser Ile Ile Asp Ile Pro Leu
               580                 585                 590
   Ser Ile Ser Ser Leu Cys Tyr Leu Gln Thr Phe Lys Leu Asn Leu Pro
           595                 600                 605
   Phe Pro Ser Tyr Tyr Pro Phe Ile Leu Pro Ser Glu Ile Leu Thr Met
       610                 615                 620
   Pro Gln Leu Arg Thr Leu Cys Met Asp Trp Asn Tyr Leu Arg Ser His
   625                 630                 635                 640
   Glu Pro Thr Glu Asn Arg Leu Val Leu Lys Asn Leu Gln Cys Leu Asn
   645 650 655
   Gln Leu Asn Pro Arg Tyr Cys Thr Gly Ser Phe Phe Arg Leu Phe Pro
   660 665 670
   Asn Leu Lys Lys Leu Gln Val Phe Gly Val Pro Glu Asp Phe Arg Asn
   675 680 685
   Ser Gln Asp Leu Tyr Asp Phe Arg Tyr Leu Tyr Gln Leu Glu Glu Leu
   690 695 700
   Thr Phe Arg Leu Tyr Tyr Pro Tyr Ala Ala Cys Phe Leu Lys Asn Thr
   705 710 715 720
   Ala Pro Ser Gly Ser Thr Pro Gln Asp Pro Leu Arg Phe Gln Thr Glu
   725 730 735
   Ile Leu His Lys Glu Ile Asp Phe Gly Gly Thr Ala Pro Pro Thr Leu
   740 745 750
   Leu Leu Pro Pro Pro Asp Ala Phe Pro Gln Asn Leu Lys Ser Leu Thr
   755 760 765
   Phe Arg Gly Glu Phe Ser Val Ala Trp Lys Asp Leu Ser Ile Val Gly
   770 775 780
   Lys Leu Pro Lys Leu Glu Val Leu Ile Leu Ser Trp Asn Ala Phe Ile
   785 790 795 800
   Gly Lys Glu Trp Glu Val Val Glu Glu Gly Phe Pro His Leu Asn Phe
   805 810 815
   Leu Phe Leu Asp Asp Val Tyr Ile Arg Tyr Trp Arg Ala Ser Ser Asp
   820 825 830
   His Phe Pro Tyr Leu Glu Arg Leu Ile Leu Arg Asp Cys Arg Asn Leu
   835 840 845
   Asp Ser Ile Pro Arg Asp Phe Ala Asp Ile Thr Thr Leu Ala Leu Ile
   850 855 860
   Asp Ile Asp Tyr Cys Gln Gln Ser Val Val Asn Ser Ala Lys Gln Ile
   865 870 875 880
   Gln Gln Asp Ile Gln Asp Asn Tyr Gly Ser Ser Ile Glu Val His Thr
   885 890 895
   Arg His Leu Phe Ile Pro Lys Ser Val Thr Thr Val Glu Asp Asp Asp
   900 905 910
   Asp Ser Val Thr Thr Asp Glu Asp Asp Asp Asp Asp Asp Phe Glu Lys
   915 920 925
   Glu Val Ala Ser Cys Arg Asn Asn Val Glu
   930 935

<210> 219
<211> 3162
<212> DNA
<213> Solanum acaule


<400> 219
atggcttatg ctgctgttac ttcccttatg agaaccatac atcaatcaat ggaacttact      60

ggatgtgatt tgcaaccgtt ttatgaaaag ctcgaatctt taagagctat tctggagaaa     120

tcctgcaatg taacgggcga tcatgaggag ttaacaatct tggaagttga aatcttagag     180

gtagcataca caacagaaga tatggttgac tcggaatcaa gaagtgtttt tttagcacag     240

aatttggagg aaagaaacag ggctatgtgg gagatttttt tcgtcttgga acaagcacta     300

gaatgcattg attccaccgt gaaacagtgg atggcaacat cggacagcat gaaagatcta     360

aaaccacaaa ctagctcact tgtcagttta cctgaccatg cttttgagca gcctgagaat     420

ataatggttg gccgtgaaaa tgaatttgag atgatgctgg atcaacttgt tagaggagga     480

agggaactag aagttgtctc aatcgtaggg atgggaggta tcgggaaaac aactttggct     540

acaaaactct atagtgatcc ttacattatg tctcgatttg atattcgtgc aaaagcaact 600

gtttcacaag agtattgtgt gagaaatgta ctcctaggcc ttctttcttt gacaagtgat 660

gaacctgatg atcaactagc ggaccgactg caaaagcatc tgaaaggcag gagatacttg 720

gtagtcattg atgacatatg gactacaaaa gcttgggatg gtataaaact atgtttccca 780

gactgttata agggaagcag aatactcctg actactcgga atgtggaagt ggctgaatat 840

gctagttcag gtaagcctcc tcatcacatg cgcctcatga atttgacgaa aagttggaat 900

ttactacaca aaaagatctt tgaaaaagaa ggttcttatt ctcctgaatt tgaaaatatt 960

gggaaacaaa ttgcattaaa atgtggagga ttacctctag caattactgt gattgctgga 1020

cttctctcca aaatcagtaa aacattggat gagtggcaaa atgttgcgga gaatgtaagt 1080

tcggtggtaa gcacagatct tgaagcaaaa tgcatgagag tgttggcttt gagttaccat 1140

cacttgcctt ctcacctaaa accgtgtttt ctatattttg caattttcgc agaggatgaa 1200

cggatttctg taactaaact tgttgagtta tgggctgtag aggggttttt gaatgaagaa 1260

gagggaaaaa gcatagaaga ggtggcagaa acatgtataa acgagcttgt agatagaagt 1320

ctaatttcta tccacaattt gagttttgat gggaaaatag aaagttgtgg aatgcatgat 1380

gtgacccgtg aactctgttt gagggaagct cgaaacatga attttgtaaa tgttatcaga 1440

ggaaagagtg atcaaaattc atgtgcacaa tccatgcagc gttcctttaa gagtcgaagt 1500

cggatcagaa tccataagct ggaagaattg gcttggtgtc gtaacagtga ggctcattct 1560

attatcatgt tgggtggatt cgaatgcgtc acactggaat tgtctttcaa gctagtaaga 1620

gtactagatc ttggtttgaa tacatggcca atttttccca gtggagtact ttctctaatt 1680

catttgagat acctatcttt gcgttttaat ccttgcttac agcagtatca aggatcgaaa 1740

gaagctgttc cctcatcaat aatagacatt cctctatcga tatcaagcct atgctatctg 1800

caaactttta aacttaacct tccatttccc agttattatc ctttcatatt accatcggaa 1860

attttgacga tgccacaatt gaggacgctg tgtatggact ggaattactt gcggagtcat 1920

gagcctacag agaacagatt ggttttgaaa aatttgcaat gcctcaatca attgaaccct 1980

cggtattgta cagggtcttt ttttagacta tttcccaatt taaagaagtt gcaagtattt 2040

ggcgtcccag aagactttcg caatagccag gacctgtatg attttcgcta cttatatcag 2100

ctcgaagaat tgacatttcg tttatattat ccatatgctg cttgctttct aaaaaacact 2160

gcaccttcag gttctactcc gcaagatcct ctgaggtttc agacggaaat attgcacaaa 2220

gagattgatt tcgggggaac tgcacctcca actttactct tacctcctcc ggatgctttt 2280

ccacaaaacc ttaagagttt aacttttagg ggagaattct ctgtggcatg gaaggatttg 2340

agcattgttg gtaaattacc caaactcgag gtccttatac tatcatggaa tgccttcata 2400

ggcaaggagt gggaagtagt tgaggaaggg tttcctcact tgaacttctt gtttctggat 2460

gatgtataca ttcgatactg gagagctagt agtgatcact ttccgtacct tgaacgactt 2520

attcttagag attgccgtaa tttggattca atccctcgag attttgcaga tataaccaca 2580

ctagctctta ttgatataga ttactgtcaa caatctgttg tgaattccgc caagcaaatt 2640

caacaggaca ttcaagacaa ctatggaagc tctatcgagg tccatactcg tcatcttttg 2700

taagacatct tcttccttgc tttacaacaa taattaactc atcatcatag taaactcgat 2760

aataatctgg ataatagcct tagtaagtca aattgcacca attcaacaaa agttcttgat 2820

gctgtcattg tgtttgattt gaatccttcc aatattgtgt aacttgttat acttgcatgt 2880

tcattcttga ttttgggaag tgtaacattt catttttcat cttttgtggc tagcattccc 2940

aagagtgtga caacagttga agatgatgat gatagtgtga caacagatga agatgatgat 3000

gatgatgact ttgagaaaga agttgcttct tgccgcaata atgtgtgagt tcttatacct 3060

gcatgctcat tcttgctata atgttctctt gttccttaat tatggggcat ctaacatatt 3120

attttccatt ttttgcgcct tttttttttc tgcagcgagt ag 3162

<210> 220
<211> 5238
<212> DNA
<213> Artificial Sequence

<220>
<223> cDNA

<400> 220
atggaaatcg taactgctat tttgagccca gtcgcagaac acttgatgat actgccagtt 60

gcgcgacaga ttggatattt gttttactat aggcgcaaca tcaggtcttt ggaaaatgaa 120

aataagaagc tggagggtat cagaagcggg gtgcagcaca gaaaggatgc tgaatggaga 180

aacttacaag tcatgccacc ccatgtcgag aattggttaa aggatgttaa tgaaactact 240

gctgaggtgg cggttttact acgacgtaga gctgaggttg aaagaggttg cttctacggt 300

tggtgtccaa acttgaagtc acgtcactcg ctgagcagga aagctaagaa aattgaacag 360

gctatgattg gtcttcaaga tgaaggcaca cgttatgctc cttcctcctg tccagcacca 420

cttgcagtta cagtgataca cagtgatgag tttgactcta gaaaacagaa ggaggaagag 480

gtcatgacag ctttgaaaga tgaggggatc actattgttg ggatatgtgg tatgggtggt 540

gttggtaaaa caacactggc tgagaaagta agagcaaggg caaaacaaga aaggttgttt 600

gattatgttg ttgtgctaac tatcagtcaa caacaaccag acattaaaaa aatacagcgc 660

gagatcgccg aaggagtcgg tctatcatta gagggggacg atttgttgga gcgtggagat 720

cggctgcgtt caaggttaat gcagaaggac agtcgcgtcc ttgtaatttt ggatgatgtt 780

tggaagaagg ttgatctaaa gagggttgga attcccagtg gtagcgatcg caactactgg 840

tgcaaagtga tattgacgac gcgtctccga gatgtttgtg acgatatgga ggctaaaaag 900

aaagtacatg ttgaaatctt atgtgaaaat gaagcatggc tcctttttag gcagaaagcc 960

ggtaattcag ctgatgatct ctctcttcct gatatagcag aagccgttgc caaagaatgc 1020

aaggggctgc cacttgcaat tgttactgtt gcaggagcac taaagggtaa aaccaagcgt 1080

tcatgggagg atgccctttt agaattaaaa aaagcagcac caagaaatat tcgaggagtg 1140

cttgcagatg tgtatcaacc tctgaagata agctataatc acttagggag tgatgaagcc 1200

aaggatgcct ttttgctttg ttccttgttt gaggaagata gtaatatttg gactgaagaa 1260

ttacttagat acggaatggg gcttggcatc ttttcggaac tcgaaaattt agaatgtgca 1320

agaaataggg tgtctaatct gttagaaaca ttgaaaaatt gtttcttgtt atcccaagtt 1380

ccaggcaaag attatgtcaa aatgcatgat gtggtccggg acatggctat acatattgca 1440

tctgagggta accatatttt tttggtgagc cacaatgtga actcaaaaga gttcccaaga 1500

aaagactctt acaagcaata cagtcatatg tcaattgttg caaataaatt tgatgcgcgt 1560

cccagaccaa tatcttgccc acgattgaag cttctgatgt taaaactccg tttcgaagag 1620

ggtttcaaat tacaggatga tttttttgat ggaatgagtg aactcagtgt cataaaactg 1680

agtggatatg atcgaaactc cattctgccc tttccatcat ccattcagag gttgtcaaat 1740

ctgagcacgc tttggctgag taatctaagg ttggatgacg tgtccattat tggggaactt 1800

gtcactttag aaattctcag catcagaggt tctgacttac aggagcttcc agtggagatc 1860

ggaaacttgg ccaatctaac tatgttagag tattggaata ctggttacag aaaccgtatg 1920

aggatttcac caggggtctt atcaagacta gttcgattgg aggaactaca tatggtggga 1980

gtagaagatt gtagttactc caccttgagg gagctggaat cgttatcgag attgactgca 2040

ctgacattag atggatgttc caaagatgtg atttacagta acttgggcct ttcctccaag 2100

ttgacacggt acgctcttaa aatgggtaga cactacacgt ttacttcatt catgcaaact 2160

tacaacaagg ctatctatct agacgtcacc aagggcaccc cattgggtga ttggatccgc 2220

ctcctgttga ggaatagcga atgtgtaagt tcaagcggaa aggggtccaa gaatgtagtg 2280

ttcgagttgc agaatgtgaa agatctcagg ctgtcttttt gtgattcatt gaatatccac 2340

tgtcagaata atattccatt ccccaaactt gaaaggctga aagtaaatgg gtgtgatcat 2400

ctacggcatc ttttttgtgt gtccttggct tgccctgatg aggggacctc tcgaaggaca 2460

cacatcagac ctgacgtaat caagttcccc aatttatatt acttggaact gggatttctg 2520

aaatgcttca cacacttttt cagtgacacc gttgagggca ttgagttccc tctgttacgg 2580

aaaatgcatt tctggagttt acaaaagttc caaaatttct ggcctagagt caacaatgct 2640

atcaccgact caaatcctct ttttaatgaa aaggtttctt gtcccaacct ggaattgcta 2700

cgactctgtg aggccaacag cttaactgct ctatgctctc accaacttcc aactgcctac 2760
ttcagcaaac ttgtaacctt gaatgtactt aggtgtggaa aactgagaaa cttgatgtct 2820
ccatcagtgg ccagaggtct tctgaatctc cgaatattac tattagaaga ctgtgaatca 2880
atggaagaag tgatcacaga agaggaacaa caaggagatg aaatcatgtg taatgaaccc 2940
ttatttcccc aattggaaga gctgggactt gaaaatctgc caaatctgag gcatttcatt 3000
ttgacaaagc aagctcttga atttccattc ctcagagaag tgcagattcg taactgccct 3060
gaaacgaaga tatttgtcca acagagatct gtcagtacat caagtctcaa aagtgtgaac 3120
aatgatgatg agttgaaagt tgttgatctc aacaaagcga tgtttaattc taaggtttct 3180
tgtcccaacc tggaagtgct acgactctat aaggctaaca gcataagtgc tctatgctct 3240
caccaacttc caactgccta cttcagcaaa cttaaggaac taggagtagg gaattgtgga 3300
aaattgagac acttgatgtc tccatcagtg gccagaggtc ttctgaatct ccgaaaacta 3360
tggataggag aatgccaatc aatggaagaa gtgatcacag aagaggaaca acaaggagat 3420
gaaatcatgt gtaatgaacc cttatttccc caattggaag agctgggact tgaaaatctg 3480
ccaaatctga ggcatttcat tttgacaaag caagctcttg aatttccatt cctcagagaa 3540
gtgctgattc gtaactgccc tgaaacgaag atatttgtcc aacagagatc tgtcagtaca 3600
tcaagtctca aaagtgtgaa caatgatgat gagttgaaag ttgttgatct caacaaagcg 3660
atgtttaatt ctaaggtttc ttgtcccaac ctggaagtgc tactactcta taaggctaac 3720
agcataagtg ctctatgctc tcaccaactt ccaactgcct acttcagcaa acttaaggaa 3780
ctaggagtat ggaattgtgg aaaattgaga cacttgatgt ctccatcagt ggccagaggt 3840
cttctgaatc tccgaaaact atggatacga gaatgccaat caatggaaga agtgatcaca 3900
gaagaggaac aacaaggaga tgaaatcatg tgtaatgaac ccttatttcc ccaattggaa 3960
gagctgggac ttgaaaatct gccaaatctg aggcatttca ttttgacaaa gcaagctctt 4020
gaatttccat tcctcagaga agtgctgatt cgtaactgcc ctgaaacgaa gatatttgtc 4080
caacagagat ctgtcagtac atcaagtctc aaaagtgtga acaatgatga tgagttgaaa 4140
gttgttgatc tcaacaaagc gatgtttaat tctaaggttt cttgtcccaa cctggaagtg 4200
ctactactct ataaggctaa cagcataagt gctctatgct ctcaccaact tccaactgcc 4260
tacttcagca aacttaagga actaggagta tggaattgtg gaaaattgag acacttgatg 4320
tctccatcag tggccagagg tcttctgaat ctccgaaaac tatggatacg agaatgccaa 4380
tcaatggaag aagtgatcac agaagaggaa caacaaggag atgaaatcat gtgtaatgaa 4440
cccttatttc cccaattgga agagctggga cttgaaaatc tgccaaatct gaggcatttc 4500
attttgacaa agcaagctct tgaatttcca ttcctcagag aagtgctgat tcgtaactgc 4560
cctgaaacga agatatttgt ccaacagaga tctgtcagta catcaagtct caaaagtgtg 4620

aacaatgatg atgagttgaa agttgttgat ctcaacaaag cgatgtttaa ttctaaggtt 4680

tcttgtccca acctggaaaa gctatatatc tattcggaca taactgctct atactctcac 4740

caacttccaa ctacctactt cagcaaactt gggacattga aagtagaaaa ttgtggaaaa 4800

ttgagacact tgatatctcc atcagtggcc agaggtcttc tgaatctccg aaaactacgg 4860

ataggagaat gccaatcaat ggaagaagtg atcacagaag aggaacaaca aggagatgaa 4920

atcatgtgta atgaaccctt atttccccaa ttggaaaagc tgaaacttga aaatctgcca 4980

aagctgaggc atttcatttt gacgaagcaa gctcttgaat ttccattcct caaattagtg 5040

gtgattcgta aatgccctga aatgaagata tttgtccaac atggatatgt gagtacacca 5100

agtcttgaaa ttgtgaacga tgatgatgag gtaaaagtag atgatttgaa cgaatggata 5160

catcagaggt tcaattctaa ggaagaagat ggaagtgaat ctgaatcttc tcagggagaa 5220

gattggaatc gaatctga 5238

<210> 221
<211> 1745
<212> PRT
<213> Capsicum annuum

<400> 221
Met Glu Ile Val Thr Ala Ile Leu Ser Pro Val Ala Glu His Leu Met
1               5                   10                  15
Ile Leu Pro Val Ala Arg Gln Ile Gly Tyr Leu Phe Tyr Tyr Arg Arg
            20                  25                  30
Asn Ile Arg Ser Leu Glu Asn Glu Asn Lys Lys Leu Glu Gly Ile Arg
        35                  40                  45
Ser Gly Val Gln His Arg Lys Asp Ala Glu Trp Arg Asn Leu Gln Val
    50                  55                  60
Met Pro Pro His Val Glu Asn Trp Leu Lys Asp Val Asn Glu Thr Thr
65                  70                  75                  80
Ala Glu Val Ala Val Leu Leu Arg Arg Arg Ala Glu Val Glu Arg Gly
                85                  90                  95
Cys Phe Tyr Gly Trp Cys Pro Asn Leu Lys Ser Arg His Ser Leu Ser
            100                 105                 110
Arg Lys Ala Lys Lys Ile Glu Gln Ala Met Ile Gly Leu Gln Asp Glu
        115                 120                 125
Gly Thr Arg Tyr Ala Pro Ser Ser Cys Pro Ala Pro Leu Ala Val Thr
    130                 135                 140
Val Ile His Ser Asp Glu Phe Asp Ser Arg Lys Gln Lys Glu Glu Glu
145                 150                 155                 160
Val Met Thr Ala Leu Lys Asp Glu Gly Ile Thr Ile Val Gly Ile Cys
                165                 170                 175
Gly Met Gly Gly Val Gly Lys Thr Thr Leu Ala Glu Lys Val Arg Ala
            180                 185                 190
Arg Ala Lys Gln Glu Arg Leu Phe Asp Tyr Val Val Val Leu Thr Ile
        195                 200                 205
Ser Gln Gln Gln Pro Asp Ile Lys Lys Ile Gln Arg Glu Ile Ala Glu
    210                 215                 220
Gly Val Gly Leu Ser Leu Glu Gly Asp Asp Leu Leu Glu Arg Gly Asp
225                 230                 235                 240
Arg Leu Arg Ser Arg Leu Met Gln Lys Asp Ser Arg Val Leu Val Ile
                245                 250                 255

Leu Asp Asp Val Trp Lys Lys Val Asp Leu Lys Arg Val Gly Ile Pro
            260                 265                 270
Ser Gly Ser Asp Arg Asn Tyr Trp Cys Lys Val Ile Leu Thr Thr Arg
        275                 280                 285
Leu Arg Asp Val Cys Asp Asp Met Glu Ala Lys Lys Lys Val His Val
    290                 295                 300
Glu Ile Leu Cys Glu Asn Glu Ala Trp Leu Leu Phe Arg Gln Lys Ala
305                 310                 315                 320
Gly Asn Ser Ala Asp Asp Leu Ser Leu Pro Asp Ile Ala Glu Ala Val
                325                 330                 335
Ala Lys Glu Cys Lys Gly Leu Pro Leu Ala Ile Val Thr Val Ala Gly
            340                 345                 350
Ala Leu Lys Gly Lys Thr Lys Arg Ser Trp Glu Asp Ala Leu Leu Glu
        355                 360                 365
Leu Lys Lys Ala Ala Pro Arg Asn Ile Arg Gly Val Leu Ala Asp Val
    370                 375                 380
Tyr Gln Pro Leu Lys Ile Ser Tyr Asn His Leu Gly Ser Asp Glu Ala
385                 390                 395                 400
Lys Asp Ala Phe Leu Leu Cys Ser Leu Phe Glu Glu Asp Ser Asn Ile
                405                 410                 415
Trp Thr Glu Glu Leu Leu Arg Tyr Gly Met Gly Leu Gly Ile Phe Ser
            420                 425                 430
Glu Leu Glu Asn Leu Glu Cys Ala Arg Asn Arg Val Ser Asn Leu Leu
        435                 440                 445
Glu Thr Leu Lys Asn Cys Phe Leu Leu Ser Gln Val Pro Gly Lys Asp
    450                 455                 460
Tyr Val Lys Met His Asp Val Val Arg Asp Met Ala Ile His Ile Ala
465                 470                 475                 480
Ser Glu Gly Asn His Ile Phe Leu Val Ser His Asn Val Asn Ser Lys
                485                 490                 495
Glu Phe Pro Arg Lys Asp Ser Tyr Lys Gln Tyr Ser His Met Ser Ile
            500                 505                 510
Val Ala Asn Lys Phe Asp Ala Arg Pro Arg Pro Ile Ser Cys Pro Arg
        515                 520                 525
Leu Lys Leu Leu Met Leu Lys Leu Arg Phe Glu Glu Gly Phe Lys Leu
    530                 535                 540
Gln Asp Asp Phe Phe Asp Gly Met Ser Glu Leu Ser Val Ile Lys Leu
545                 550                 555                 560
Ser Gly Tyr Asp Arg Asn Ser Ile Leu Pro Phe Pro Ser Ser Ile Gln
                565                 570                 575
Arg Leu Ser Asn Leu Ser Thr Leu Trp Leu Ser Asn Leu Arg Leu Asp
            580                 585                 590
Asp Val Ser Ile Ile Gly Glu Leu Val Thr Leu Glu Ile Leu Ser Ile
        595                 600                 605
Arg Gly Ser Asp Leu Gln Glu Leu Pro Val Glu Ile Gly Asn Leu Ala
    610                 615                 620
Asn Leu Thr Met Leu Glu Tyr Trp Asn Thr Gly Tyr Arg Asn Arg Met
625                 630                 635                 640
Arg Ile Ser Pro Gly Val Leu Ser Arg Leu Val Arg Leu Glu Glu Leu
                645                 650                 655
His Met Val Gly Val Glu Asp Cys Ser Tyr Ser Thr Leu Arg Glu Leu
            660                 665                 670
Glu Ser Leu Ser Arg Leu Thr Ala Leu Thr Leu Asp Gly Cys Ser Lys
        675                 680                 685
Asp Val Ile Tyr Ser Asn Leu Gly Leu Ser Ser Lys Leu Thr Arg Tyr
    690                 695                 700
Ala Leu Lys Met Gly Arg His Tyr Thr Phe Thr Ser Phe Met Gln Thr
705                 710                 715                 720
Tyr Asn Lys Ala Ile Tyr Leu Asp Val Thr Lys Gly Thr Pro Leu Gly
                725                 730                 735
Asp Trp Ile Arg Leu Leu Leu Arg Asn Ser Glu Cys Val Ser Ser Ser
            740                 745                 750
Gly Lys Gly Ser Lys Asn Val Val Phe Glu Leu Gln Asn Val Lys Asp

755                 760                 765
Leu Arg Leu Ser Phe Cys Asp Ser Leu Asn Ile His Cys Gln Asn Asn
    770                 775                 780
Ile Pro Phe Pro Lys Leu Glu Arg Leu Lys Val Asn Gly Cys Asp His
785                 790                 795                 800
Leu Arg His Leu Phe Cys Val Ser Leu Ala Cys Pro Asp Glu Gly Thr
                805                 810                 815
Ser Arg Arg Thr His Ile Arg Pro Asp Val Ile Lys Phe Pro Asn Leu
            820                 825                 830
Tyr Tyr Leu Glu Leu Gly Phe Leu Lys Cys Phe Thr His Phe Phe Ser
        835                 840                 845
Asp Thr Val Glu Gly Ile Glu Phe Pro Leu Leu Arg Lys Met His Phe
    850                 855                 860
Trp Ser Leu Gln Lys Phe Gln Asn Phe Trp Pro Arg Val Asn Asn Ala
865                 870                 875                 880
Ile Thr Asp Ser Asn Pro Leu Phe Asn Glu Lys Val Ser Cys Pro Asn
                885                 890                 895
Leu Glu Leu Leu Arg Leu Cys Glu Ala Asn Ser Leu Thr Ala Leu Cys
            900                 905                 910
Ser His Gln Leu Pro Thr Ala Tyr Phe Ser Lys Leu Val Thr Leu Asn
        915                 920                 925
Val Leu Arg Cys Gly Lys Leu Arg Asn Leu Met Ser Pro Ser Val Ala
    930                 935                 940
Arg Gly Leu Leu Asn Leu Arg Ile Leu Leu Leu Glu Asp Cys Glu Ser
945                 950                 955                 960
Met Glu Glu Val Ile Thr Glu Glu Glu Gln Gln Gly Asp Glu Ile Met
                965                 970                 975
Cys Asn Glu Pro Leu Phe Pro Gln Leu Glu Glu Leu Gly Leu Glu Asn
            980                 985                 990
Leu Pro Asn Leu Arg His Phe Ile Leu Thr Lys Gln Ala Leu Glu Phe
        995                 1000                1005
Pro Phe Leu Arg Glu Val Gln Ile Arg Asn Cys Pro Glu Thr Lys Ile
    1010                1015                1020
Phe Val Gln Gln Arg Ser Val Ser Thr Ser Ser Leu Lys Ser Val Asn
1025                1030                1035                1040
Asn Asp Asp Glu Leu Lys Val Val Asp Leu Asn Lys Ala Met Phe Asn
                1045                1050                1055
Ser Lys Val Ser Cys Pro Asn Leu Glu Val Leu Arg Leu Tyr Lys Ala
            1060                1065                1070
Asn Ser Ile Ser Ala Leu Cys Ser His Gln Leu Pro Thr Ala Tyr Phe
        1075                1080                1085
Ser Lys Leu Lys Glu Leu Gly Val Gly Asn Cys Gly Lys Leu Arg His
    1090                1095                1100
Leu Met Ser Pro Ser Val Ala Arg Gly Leu Leu Asn Leu Arg Lys Leu
1105                1110                1115                1120
Trp Ile Gly Glu Cys Gln Ser Met Glu Glu Val Ile Thr Glu Glu Glu
                1125                1130                1135
Gln Gln Gly Asp Glu Ile Met Cys Asn Glu Pro Leu Phe Pro Gln Leu
            1140                1145                1150
Glu Glu Leu Gly Leu Glu Asn Leu Pro Asn Leu Arg His Phe Ile Leu
        1155                1160                1165
Thr Lys Gln Ala Leu Glu Phe Pro Phe Leu Arg Glu Val Leu Ile Arg
    1170                1175                1180
Asn Cys Pro Glu Thr Lys Ile Phe Val Gln Gln Arg Ser Val Ser Thr
1185                1190                1195                1200
Ser Ser Leu Lys Ser Val Asn Asn Asp Asp Glu Leu Lys Val Val Asp
                1205                1210                1215
Leu Asn Lys Ala Met Phe Asn Ser Lys Val Ser Cys Pro Asn Leu Glu
            1220                1225                1230
Val Leu Leu Leu Tyr Lys Ala Asn Ser Ile Ser Ala Leu Cys Ser His
        1235                1240                1245
Gln Leu Pro Thr Ala Tyr Phe Ser Lys Leu Lys Glu Leu Gly Val Trp
    1250                1255                1260

Asn Cys Gly Lys Leu Arg His Leu Met Ser Pro Ser Val Ala Arg Gly
1265                1270                1275                1280
Leu Leu Asn Leu Arg Lys Leu Trp Ile Arg Glu Cys Gln Ser Met Glu
                1285                1290                1295
Glu Val Ile Thr Glu Glu Glu Gln Gln Gly Asp Glu Ile Met Cys Asn
            1300                1305                1310
Glu Pro Leu Phe Pro Gln Leu Glu Glu Leu Gly Leu Glu Asn Leu Pro
        1315                1320                1325
Asn Leu Arg His Phe Ile Leu Thr Lys Gln Ala Leu Glu Phe Pro Phe
    1330                1335                1340
Leu Arg Glu Val Leu Ile Arg Asn Cys Pro Glu Thr Lys Ile Phe Val
1345                1350                1355                1360
Gln Gln Arg Ser Val Ser Thr Ser Ser Leu Lys Ser Val Asn Asn Asp
                1365                1370                1375
Asp Glu Leu Lys Val Val Asp Leu Asn Lys Ala Met Phe Asn Ser Lys
            1380                1385                1390
Val Ser Cys Pro Asn Leu Glu Val Leu Leu Leu Tyr Lys Ala Asn Ser
        1395                1400                1405
Ile Ser Ala Leu Cys Ser His Gln Leu Pro Thr Ala Tyr Phe Ser Lys
    1410                1415                1420
Leu Lys Glu Leu Gly Val Trp Asn Cys Gly Lys Leu Arg His Leu Met
1425                1430                1435                1440
Ser Pro Ser Val Ala Arg Gly Leu Leu Asn Leu Arg Lys Leu Trp Ile
                1445                1450                1455
Arg Glu Cys Gln Ser Met Glu Glu Val Ile Thr Glu Glu Glu Gln Gln
            1460                1465                1470
Gly Asp Glu Ile Met Cys Asn Glu Pro Leu Phe Pro Gln Leu Glu Glu
        1475                1480                1485
Leu Gly Leu Glu Asn Leu Pro Asn Leu Arg His Phe Ile Leu Thr Lys
    1490                1495                1500
Gln Ala Leu Glu Phe Pro Phe Leu Arg Glu Val Leu Ile Arg Asn Cys
1505                1510                1515                1520
Pro Glu Thr Lys Ile Phe Val Gln Gln Arg Ser Val Ser Thr Ser Ser
                1525                1530                1535
Leu Lys Ser Val Asn Asn Asp Asp Glu Leu Lys Val Val Asp Leu Asn
            1540                1545                1550
Lys Ala Met Phe Asn Ser Lys Val Ser Cys Pro Asn Leu Glu Lys Leu
        1555                1560                1565
Tyr Ile Tyr Ser Asp Ile Thr Ala Leu Tyr Ser His Gln Leu Pro Thr
    1570                1575                1580
Thr Tyr Phe Ser Lys Leu Gly Thr Leu Lys Val Glu Asn Cys Gly Lys
1585                1590                1595                1600
Leu Arg His Leu Ile Ser Pro Ser Val Ala Arg Gly Leu Leu Asn Leu
                1605                1610                1615
Arg Lys Leu Arg Ile Gly Glu Cys Gln Ser Met Glu Glu Val Ile Thr
            1620                1625                1630
Glu Glu Glu Gln Gln Gly Asp Glu Ile Met Cys Asn Glu Pro Leu Phe
        1635                1640                1645
Pro Gln Leu Glu Lys Leu Lys Leu Glu Asn Leu Pro Lys Leu Arg His
    1650                1655                1660
Phe Ile Leu Thr Lys Gln Ala Leu Glu Phe Pro Phe Leu Lys Leu Val
1665                1670                1675                1680
Val Ile Arg Lys Cys Pro Glu Met Lys Ile Phe Val Gln His Gly Tyr
                1685                1690                1695
Val Ser Thr Pro Ser Leu Glu Ile Val Asn Asp Asp Asp Glu Val Lys
            1700                1705                1710
Val Asp Asp Leu Asn Glu Trp Ile His Gln Arg Phe Asn Ser Lys Glu
        1715                1720                1725
Glu Asp Gly Ser Glu Ser Glu Ser Ser Gln Gly Glu Asp Trp Asn Arg
    1730                1735                1740
Ile
1745

<210> 222
<211> 1557
<212> DNA
<213> Artificial Sequence

<220>
<223> cDNA

<400> 222
gcacacacat catcttggat gcttgaagtc ttgaaagaga atttgaaggc cattgcatat 60

atgaagagtc agctcgtcac caagcatgtt gtgaagggtg agtgtatgat gttcaaacag 120

tatttgcagg aaaaccccag ggcaaatgaa tttttccagc ctaagatgtg ggcgtatgga 180

aagagtatgt tgaataagga agcctatatc aaggatataa tgaaatattc aaaggttatt 240

gatgtaggag tagtcgattg cgaccggcat ttgaggaagc tatcgttaga gttattgtat 300

actcagatcc acggctttcg caaatgttct tacatcacag atgaagagga gatattcaag 360

gcattgaata taaccacagc tgttggagct atgtatgggg ggaagaaaaa ggagtacttt 420

gaaaagttca caacagagga taaggctgag attctccggc aaagctgttt gaggttgtac 480

acgggtaaac tgggtgtgtg ggaatgggct ctaaaagctg aactgagaag taaggaaaag 540

atagaagcca ataagacacg gactttcaca gcagccccaa ttgatacact attaggtggt 600

aaggtgtgtg tagatgatct caacaaccag ttttattcga aaaatattga atgttgttgg 660

acggttggaa tgaccaaatt ctatggtgga tgggataagc ttttgacagc tctgcctgct 720

ggatggatat attgcgatgc agatggttcg caattcgata gttcattgac accttatctc 780

ataaatgctg tattgactat acggtatgct tttatggaag attgggacat tgggtataag 840

atgttgcaaa acttgtacac agaaataatc tacacaccaa tatccacgcc tgatggaaca 900

attgtgaaga agttcagagg caataacagt gggcaacctt ccaccgttgt agacaactca 960

cttatggttg tgcttgctat gcattatgca tttgtacggg aaggtatagc atttgaagaa 1020

attgactcca tatgtaagtt cttcgttaat ggagacgatt tgctaatagc tgtgaaccca 1080

gaacgtgaga gcctattgga cacactgtcg aatcattttt ctgatttagg gctcaattat 1140

gatttctcat ctcggacgag gaataaatca gaattgtggt tcatgtcaca ttgtgggatt 1200

tctgttgaag gcacgtatat accgaagctt gaagaggagc gaattgtatc aattctccaa 1260

tgggaccgag cagagctacc agaatacaga ttggaggcta tttgtgcagc aatgattgaa 1320

tcatggggct acccacaatt aactcatgaa attcgaagat tctatagctg gttaattgag 1380

aagaacccat acgctgactt ggcatctgaa ggaaaagctc catacatttc tgaactagct 1440

ctaaagaagc tgtatctgaa tcaagatgta caaatgatga gcttcaggtc ttacctcaag 1500

tatttcgctg atgcagatga agagtttgaa tgcggtacat atgaagttcg tcatcag 1557

<210> 223
<211> 519
<212> PRT
<213> Pepper mottle virus

<400> 223
Ala His Thr Ser Ser Trp Met Leu Glu Val Leu Lys Glu Asn Leu Lys
1               5                   10                  15
Ala Ile Ala Tyr Met Lys Ser Gln Leu Val Thr Lys His Val Val Lys
            20                  25                  30
Gly Glu Cys Met Met Phe Lys Gln Tyr Leu Gln Glu Asn Pro Arg Ala
        35                  40                  45
Asn Glu Phe Phe Gln Pro Lys Met Trp Ala Tyr Gly Lys Ser Met Leu
    50                  55                  60
Asn Lys Glu Ala Tyr Ile Lys Asp Ile Met Lys Tyr Ser Lys Val Ile
65                  70                  75                  80
Asp Val Gly Val Val Asp Cys Asp Arg His Leu Arg Lys Leu Ser Leu
                85                  90                  95
Glu Leu Leu Tyr Thr Gln Ile His Gly Phe Arg Lys Cys Ser Tyr Ile
            100                 105                 110
Thr Asp Glu Glu Glu Ile Phe Lys Ala Leu Asn Ile Thr Thr Ala Val
        115                 120                 125
Gly Ala Met Tyr Gly Gly Lys Lys Lys Glu Tyr Phe Glu Lys Phe Thr
    130                 135                 140
Thr Glu Asp Lys Ala Glu Ile Leu Arg Gln Ser Cys Leu Arg Leu Tyr
145                 150                 155                 160
Thr Gly Lys Leu Gly Val Trp Glu Trp Ala Leu Lys Ala Glu Leu Arg
                165                 170                 175
Ser Lys Glu Lys Ile Glu Ala Asn Lys Thr Arg Thr Phe Thr Ala Ala
            180                 185                 190
Pro Ile Asp Thr Leu Leu Gly Gly Lys Val Cys Val Asp Asp Leu Asn
        195                 200                 205
Asn Gln Phe Tyr Ser Lys Asn Ile Glu Cys Cys Trp Thr Val Gly Met
    210                 215                 220
Thr Lys Phe Tyr Gly Gly Trp Asp Lys Leu Leu Thr Ala Leu Pro Ala
225                 230                 235                 240
Gly Trp Ile Tyr Cys Asp Ala Asp Gly Ser Gln Phe Asp Ser Ser Leu
                245                 250                 255
Thr Pro Tyr Leu Ile Asn Ala Val Leu Thr Ile Arg Tyr Ala Phe Met
            260                 265                 270
Glu Asp Trp Asp Ile Gly Tyr Lys Met Leu Gln Asn Leu Tyr Thr Glu
        275                 280                 285
Ile Ile Tyr Thr Pro Ile Ser Thr Pro Asp Gly Thr Ile Val Lys Lys
    290                 295                 300
Phe Arg Gly Asn Asn Ser Gly Gln Pro Ser Thr Val Val Asp Asn Ser
305                 310                 315                 320
Leu Met Val Val Leu Ala Met His Tyr Ala Phe Val Arg Glu Gly Ile
                325                 330                 335
Ala Phe Glu Glu Ile Asp Ser Ile Cys Lys Phe Phe Val Asn Gly Asp
            340                 345                 350
Asp Leu Leu Ile Ala Val Asn Pro Glu Arg Glu Ser Leu Leu Asp Thr
        355                 360                 365
Leu Ser Asn His Phe Ser Asp Leu Gly Leu Asn Tyr Asp Phe Ser Ser
    370                 375                 380
Arg Thr Arg Asn Lys Ser Glu Leu Trp Phe Met Ser His Cys Gly Ile
385                 390                 395                 400
Ser Val Glu Gly Thr Tyr Ile Pro Lys Leu Glu Glu Glu Arg Ile Val
                405                 410                 415
Ser Ile Leu Gln Trp Asp Arg Ala Glu Leu Pro Glu Tyr Arg Leu Glu
            420                 425                 430
Ala Ile Cys Ala Ala Met Ile Glu Ser Trp Gly Tyr Pro Gln Leu Thr
        435                 440                 445

His Glu Ile Arg Arg Phe Tyr Ser Trp Leu Ile Glu Lys Asn Pro Tyr
    450                 455                 460
Ala Asp Leu Ala Ser Glu Gly Lys Ala Pro Tyr Ile Ser Glu Leu Ala
465                 470                 475                 480
Leu Lys Lys Leu Tyr Leu Asn Gln Asp Val Gln Met Met Ser Phe Arg
                485                 490                 495
Ser Tyr Leu Lys Tyr Phe Ala Asp Ala Asp Glu Glu Phe Glu Cys Gly
            500                 505                 510
Thr Tyr Glu Val Arg His Gln
        515

<210> 224
<211> 3915
<212> DNA
<213> Artificial Sequence

<220>
<223> cDNA

<400> 224
atggaaagag taaaagaaaa tgaactagtg agaaaggaag aggaagcaaa caactcattg      60

gcgtcgtttt ctgctcttcg tgaggacgtt gtcaatgttc tggatttcat ggagaggtta     120

aagaacgaag aggatcgaaa agctgttgat gtggatctta ttgaaaagct gaaattattg     180

ctgacattta tttgtacgta tgtccagctt tcttattccg atttggatca gtttgaagat     240

ataatgactg acaaaagaca agaggttgag aatctgcttc agacaatttt ggatgatgtg     300

gacaacactg tcaggtgtaa atataacatg catcatgtcc ttcctagcct tgctgataat     360

atggatgact gtatcagctc acatcatcgt tctaaatcag atggcaccat gacggaggag     420

cgattgaact tccttctctt gaatctccat catctatcca agtatcatgc tgaaaagatt     480

tttccgttag tgactgaata tgggattctt cagaacgttt gtgccaacat aggagatttc     540

catgggttga tagtgaatgg ttgcattaag catgagatta ttgagaatgt cttacctctg     600

tttcaactga tggtcgagag tgtaggacac ttcctttggg aggatcaaac tgatggagac     660

tctcaactct ccgagctaga tgaggatgat cagactgatg gagactctga actctctgaa     720

ctatctgagg atgatcagac tgatggagac tctgaactct ctgaggatga tcagattgat     780

ggagattttc gactcttcaa gctagcacat ctactcatga agattattcc aattgaactg     840

gaggtgatgc acatatgtta tacaaatttg aaagcttcac catcagtaga agttggacgc     900

tttattaagc aactccttga aacctctccg gatgttctga gagaatatct gattcatcta     960

caagagcaca tggtaactgt tattaccgca agcacttcag gggctcgaaa cgttcatgtc    1020

attatagagt tcctattgat tatagttact gatgtgccca aggactttat tcatcatgac    1080

aaattgtttg atcttttggc acgtgttgga gcacttgtca gggaggtatc aactcttgtt    1140

cacgacttaa tagagaaatc aaggaataaa gagagaatcg accaaacaat ttgtgcaact    1200

cgagatttgc tggaaaatat tgaactcttc aaggaagatc tcaaaaatga ttatctgaaa    1260

gccccggact catatcaatg ttgcttcccc atgaatgatg gaccgctgtt catgcatctt 1320

ctactcatcc acttaaatgg tctgctagat tccaattctt attcaattgc tttgataaag 1380

gatgacatta gaatggtgaa aaaagccttt gaattgataa gatcgttctt cgtgaatttt 1440

gagcaaggat tgtataaaga tctctgggca catattttag atgtggctta tgaggctaaa 1500

gatgtcattg attcaattat tgttcgagat aatggtctct tacatcttat tttctcactt 1560

cccattacga taaaaaagat caagcttatc caagaagaag tctccaatat atctgagaag 1620

attcccaaga acaggagcct cactgtagta aactctacta agaagattgt tgaaagcaag 1680

tcactgacag ttggtaaaat aattgtaggt tttgaggagg agacaaattg gttaattagt 1740

aagctcacca gtggactaaa agatctagat gtcatctcaa tcactggtat gccgggttcg 1800

ggcaaaacta ctttggcata caaagtatac aatgatgagt ccgtttctag acgtttcgac 1860

atttgtgcat ggtgcacagt tggccaagaa tacgacaaaa taaatttgtt ggagaaagtt 1920

tataatcaag ttaccggccc tgattcaaaa ttgagtgaga atattgatgt tgctgatgag 1980

ctaaggagac atctgcttgg aaagaggtat cttattgtct tagatgactt gtgggatact 2040

gatgcatggg ataagttaac aagacctttt cctctagttg agaatggaag cagaataatt 2100

ttgacatctc gagatatggt agtgggtttg tatggaaagc gcaacactga tcctcttaac 2160

cttcgtttgc taaggccaga agaaagttgg gagttattag agaaaagggc atttggaaac 2220

aagagctgcc ctgatgaact attggatgtt ggaaaagaaa tcgcccaaaa ttgtaaggga 2280

cttcctttgg ttgttgatct gattgctgga gtcattgcag gtagggaaaa gacaaagtct 2340

gtatggcttg aaattcgaaa taatttgaat tcctttattt tgaatagtga agtttatgtg 2400

atgaaggttg tgaaattaag ttatgaccat ttacctcttc aactaaagcc atgctttctc 2460

taccttgcga ggtatccgaa ggacaatgca gtggatagaa attggttgaa aatgttttgg 2520

tgtgctgaag ggcttgttga acagacagag ctgaagagtt tggaagaagt gatggaaatt 2580

tatttggata atttaatttc cagtagcttg gtaattgctt tcaatgagat aggtaattac 2640

ccgacttgcc aactacatga tcttgtgcat gacttttgtt tgataaaagc aaaagaggaa 2700

aagttgtttg agaaggtaag ttcaagtgat ctatcttctt cttcagattt gatgtcgcat 2760

atagtgacca tttattatga taaggagcac tttgaatata acaatttcgt cctgctcgat 2820

tcaaaaatga aaaggcggta tggtaaacac ctctattctt tggtgataac tggagacgag 2880

atggaagatc gtttttctga tgcatgtcac ctaagagact tgaggcttct tagagtgttg 2940

tacctgaagc catcttttat gatggtgaaa gagtctttgc tgaatgaaat aggcatgttg 3000

aatcatttga ggttcttaag cattggggca gaagttaaag ctctgccttt atctttctca 3060

aacctttgga atctagaaac cctatgggtt aaaaacaaag gatcaacctt ggtactatta 3120

cctagtcttt gggatcttgt aaagttgcga gtgctgtcca ttgatgattg ttctttcttt 3180

gacttggata ctgttgaatc aatactgata actgtggact cacagttaga gaacttgaga 3240

gaattaggga aactcgtgct ttcctattca aaagaaacag aggttatttt caaaaggttc 3300

cccaatcttc aagagctttc atttgttctc aaggaatcat gggattactc aacagagcga 3360

tattggttcc cgaaatttga tttcctaact gaactagaac acctcacagt agattttaaa 3420

agttcaaatt ccaatgacag tggggcctct gtagcgacaa atcggtcttg ggattttcac 3480

ttcccttcca atttgagaac attgttgttg tatgactttc ctctggcatc cgattcacta 3540

tcaacaatag cgaaactgcc caatcttgaa gagttgttcc ttaggagaac aatcatccat 3600

ggggaggaat ggaacatgag ggaggaagac acctttgaga atctcaaata tttgaacttg 3660

actgaagtga ctcttgctaa gtgggaagtt ggagaggaat cctttcctgt gattgagaaa 3720

ttagtactgc ggaaatgtca tatgcttaag gagattccac ctagtttcgg tgatatttgt 3780

tcgttgaaaa tcatcaaact tgtagagagc cctcatgttg aagattctgc taagaagatt 3840

aaacaatatg ttgaagatat gggaggggac gagcttcagg tcctgggctg ggaggatatc 3900

cccttattta agtag 3915

<210> 225
<211> 1304
<212> PRT
<213> Capsicum annuum

<400> 225
Met Glu Arg Val Lys Glu Asn Glu Leu Val Arg Lys Glu Glu Glu Ala
1               5                   10                  15
Asn Asn Ser Leu Ala Ser Phe Ser Ala Leu Arg Glu Asp Val Val Asn
            20                  25                  30
Val Leu Asp Phe Met Glu Arg Leu Lys Asn Glu Glu Asp Arg Lys Ala
        35                  40                  45
Val Asp Val Asp Leu Ile Glu Lys Leu Lys Leu Leu Leu Thr Phe Ile
    50                  55                  60
Cys Thr Tyr Val Gln Leu Ser Tyr Ser Asp Leu Asp Gln Phe Glu Asp
65                  70                  75                  80
Ile Met Thr Asp Lys Arg Gln Glu Val Glu Asn Leu Leu Gln Thr Ile
                85                  90                  95
Leu Asp Asp Val Asp Asn Thr Val Arg Cys Lys Tyr Asn Met His His
            100                 105                 110
Val Leu Pro Ser Leu Ala Asp Asn Met Asp Asp Cys Ile Ser Ser His
        115                 120                 125
His Arg Ser Lys Ser Asp Gly Thr Met Thr Glu Glu Arg Leu Asn Phe
    130                 135                 140
Leu Leu Leu Asn Leu His His Leu Ser Lys Tyr His Ala Glu Lys Ile
145                 150                 155                 160
Phe Pro Leu Val Thr Glu Tyr Gly Ile Leu Gln Asn Val Cys Ala Asn
                165                 170                 175
Ile Gly Asp Phe His Gly Leu Ile Val Asn Gly Cys Ile Lys His Glu
            180                 185                 190
Ile Ile Glu Asn Val Leu Pro Leu Phe Gln Leu Met Val Glu Ser Val
        195                 200                 205
Gly His Phe Leu Trp Glu Asp Gln Thr Asp Gly Asp Ser Gln Leu Ser
    210                 215                 220

Glu Leu Asp Glu Asp Asp Gln Thr Asp Gly Asp Ser Glu Leu Ser Glu
225                 230                 235                 240
Leu Ser Glu Asp Asp Gln Thr Asp Gly Asp Ser Glu Leu Ser Glu Asp
                245                 250                 255
Asp Gln Ile Asp Gly Asp Phe Arg Leu Phe Lys Leu Ala His Leu Leu
            260                 265                 270
Met Lys Ile Ile Pro Ile Glu Leu Glu Val Met His Ile Cys Tyr Thr
        275                 280                 285
Asn Leu Lys Ala Ser Pro Ser Val Glu Val Gly Arg Phe Ile Lys Gln
    290                 295                 300
Leu Leu Glu Thr Ser Pro Asp Val Leu Arg Glu Tyr Leu Ile His Leu
305                 310                 315                 320
Gln Glu His Met Val Thr Val Ile Thr Ala Ser Thr Ser Gly Ala Arg
                325                 330                 335
Asn Val His Val Ile Ile Glu Phe Leu Leu Ile Ile Val Thr Asp Val
            340                 345                 350
Pro Lys Asp Phe Ile His His Asp Lys Leu Phe Asp Leu Leu Ala Arg
        355                 360                 365
Val Gly Ala Leu Val Arg Glu Val Ser Thr Leu Val His Asp Leu Ile
    370                 375                 380
Glu Lys Ser Arg Asn Lys Glu Arg Ile Asp Gln Thr Ile Cys Ala Thr
385                 390                 395                 400
Arg Asp Leu Leu Glu Asn Ile Glu Leu Phe Lys Glu Asp Leu Lys Asn
                405                 410                 415
Asp Tyr Leu Lys Ala Pro Asp Ser Tyr Gln Cys Cys Phe Pro Met Asn
            420                 425                 430
Asp Gly Pro Leu Phe Met His Leu Leu Leu Ile His Leu Asn Gly Leu
        435                 440                 445
Leu Asp Ser Asn Ser Tyr Ser Ile Ala Leu Ile Lys Asp Asp Ile Arg
    450                 455                 460
Met Val Lys Lys Ala Phe Glu Leu Ile Arg Ser Phe Phe Val Asn Phe
465                 470                 475                 480
Glu Gln Gly Leu Tyr Lys Asp Leu Trp Ala His Ile Leu Asp Val Ala
                485                 490                 495
Tyr Glu Ala Lys Asp Val Ile Asp Ser Ile Ile Val Arg Asp Asn Gly
            500                 505                 510
Leu Leu His Leu Ile Phe Ser Leu Pro Ile Thr Ile Lys Lys Ile Lys
        515                 520                 525
Leu Ile Gln Glu Glu Val Ser Asn Ile Ser Glu Lys Ile Pro Lys Asn
    530                 535                 540
Arg Ser Leu Thr Val Val Asn Ser Thr Lys Lys Ile Val Glu Ser Lys
545                 550                 555                 560
Ser Leu Thr Val Gly Lys Ile Ile Val Gly Phe Glu Glu Glu Thr Asn
                565                 570                 575
Trp Leu Ile Ser Lys Leu Thr Ser Gly Leu Lys Asp Leu Asp Val Ile
            580                 585                 590
Ser Ile Thr Gly Met Pro Gly Ser Gly Lys Thr Thr Leu Ala Tyr Lys
        595                 600                 605
Val Tyr Asn Asp Glu Ser Val Ser Arg Arg Phe Asp Ile Cys Ala Trp
    610                 615                 620
Cys Thr Val Gly Gln Glu Tyr Asp Lys Ile Asn Leu Leu Glu Lys Val
625                 630                 635                 640
Tyr Asn Gln Val Thr Gly Pro Asp Ser Lys Leu Ser Glu Asn Ile Asp
                645                 650                 655
Val Ala Asp Glu Leu Arg Arg His Leu Leu Gly Lys Arg Tyr Leu Ile
            660                 665                 670
Val Leu Asp Asp Leu Trp Asp Thr Asp Ala Trp Asp Lys Leu Thr Arg
        675                 680                 685
Pro Phe Pro Leu Val Glu Asn Gly Ser Arg Ile Ile Leu Thr Ser Arg
    690                 695                 700
Asp Met Val Val Gly Leu Tyr Gly Lys Arg Asn Thr Asp Pro Leu Asn
705                 710                 715                 720
Leu Arg Leu Leu Arg Pro Glu Glu Ser Trp Glu Leu Leu Glu Lys Arg

725                 730                 735
Ala Phe Gly Asn Lys Ser Cys Pro Asp Glu Leu Leu Asp Val Gly Lys
            740                 745                 750
Glu Ile Ala Gln Asn Cys Lys Gly Leu Pro Leu Val Val Asp Leu Ile
        755                 760                 765
Ala Gly Val Ile Ala Gly Arg Glu Lys Thr Lys Ser Val Trp Leu Glu
    770                 775                 780
Ile Arg Asn Asn Leu Asn Ser Phe Ile Leu Asn Ser Glu Val Tyr Val
785                 790                 795                 800
Met Lys Val Val Lys Leu Ser Tyr Asp His Leu Pro Leu Gln Leu Lys
                805                 810                 815
Pro Cys Phe Leu Tyr Leu Ala Arg Tyr Pro Lys Asp Asn Ala Val Asp
            820                 825                 830
Arg Asn Trp Leu Lys Met Phe Trp Cys Ala Glu Gly Leu Val Glu Gln
        835                 840                 845
Thr Glu Leu Lys Ser Leu Glu Glu Val Met Glu Ile Tyr Leu Asp Asn
    850                 855                 860
Leu Ile Ser Ser Ser Leu Val Ile Ala Phe Asn Glu Ile Gly Asn Tyr
865                 870                 875                 880
Pro Thr Cys Gln Leu His Asp Leu Val His Asp Phe Cys Leu Ile Lys
                885                 890                 895
Ala Lys Glu Glu Lys Leu Phe Glu Lys Val Ser Ser Ser Asp Leu Ser
            900                 905                 910
Ser Ser Ser Asp Leu Met Ser His Ile Val Thr Ile Tyr Tyr Asp Lys
        915                 920                 925
Glu His Phe Glu Tyr Asn Asn Phe Val Leu Leu Asp Ser Lys Met Lys
    930                 935                 940
Arg Arg Tyr Gly Lys His Leu Tyr Ser Leu Val Ile Thr Gly Asp Glu
945                 950                 955                 960
Met Glu Asp Arg Phe Ser Asp Ala Cys His Leu Arg Asp Leu Arg Leu
                965                 970                 975
Leu Arg Val Leu Tyr Leu Lys Pro Ser Phe Met Met Val Lys Glu Ser
            980                 985                 990
Leu Leu Asn Glu Ile Gly Met Leu Asn His Leu Arg Phe Leu Ser Ile
        995                 1000                1005
Gly Ala Glu Val Lys Ala Leu Pro Leu Ser Phe Ser Asn Leu Trp Asn
    1010                1015                1020
Leu Glu Thr Leu Trp Val Lys Asn Lys Gly Ser Thr Leu Val Leu Leu
1025                1030                1035                1040
Pro Ser Leu Trp Asp Leu Val Lys Leu Arg Val Leu Ser Ile Asp Asp
                1045                1050                1055
Cys Ser Phe Phe Asp Leu Asp Thr Val Glu Ser Ile Leu Ile Thr Val
            1060                1065                1070
Asp Ser Gln Leu Glu Asn Leu Arg Glu Leu Gly Lys Leu Val Leu Ser
        1075                1080                1085
Tyr Ser Lys Glu Thr Glu Val Ile Phe Lys Arg Phe Pro Asn Leu Gln
    1090                1095                1100
Glu Leu Ser Phe Val Leu Lys Glu Ser Trp Asp Tyr Ser Thr Glu Arg
1105                1110                1115                1120
Tyr Trp Phe Pro Lys Phe Asp Phe Leu Thr Glu Leu Glu His Leu Thr
                1125                1130                1135
Val Asp Phe Lys Ser Ser Asn Ser Asn Asp Ser Gly Ala Ser Val Ala
            1140                1145                1150
Thr Asn Arg Ser Trp Asp Phe His Phe Pro Ser Asn Leu Arg Thr Leu
        1155                1160                1165
Leu Leu Tyr Asp Phe Pro Leu Ala Ser Asp Ser Leu Ser Thr Ile Ala
    1170                1175                1180
Lys Leu Pro Asn Leu Glu Glu Leu Phe Leu Arg Arg Thr Ile Ile His
1185                1190                1195                1200
Gly Glu Glu Trp Asn Met Arg Glu Glu Asp Thr Phe Glu Asn Leu Lys
                1205                1210                1215
Tyr Leu Asn Leu Thr Glu Val Thr Leu Ala Lys Trp Glu Val Gly Glu
            1220                1225                1230

Glu Ser Phe Pro Val Ile Glu Lys Leu Val Leu Arg Lys Cys His Met
        1235                1240                1245
Leu Lys Glu Ile Pro Pro Ser Phe Gly Asp Ile Cys Ser Leu Lys Ile
    1250                1255                1260
Ile Lys Leu Val Glu Ser Pro His Val Glu Asp Ser Ala Lys Lys Ile
1265                1270                1275                1280
Lys Gln Tyr Val Glu Asp Met Gly Gly Asp Glu Leu Gln Val Leu Gly
                1285                1290                1295
Trp Glu Asp Ile Pro Leu Phe Lys
            1300

<210> 226
<211> 4041
<212> DNA
<213> Capsicum annuum

<400> 226
atggaaagag taaaagaaaa tgaactagtg agaaaggaag aggaagcaaa caactcattg      60

gtatgtcatg atcaatatat agcttatgta cagcattcta ttttgaagaa atccctcttc     120

tgtagctatg ttaagttctg tggctttgaa aatttgatag gaatgtaaca tttggtcttt     180

ttgcaggcgt cgttttctgc tcttcgtgag gacgttgtca atgttctgga tttcatggag     240

aggttaaaga acgaagagga tcgaaaagct gttgatgtgg atcttattga aaagctgaaa     300

ttattgctga catttatttg tacgtatgtc cagctttctt attccgattt ggatcagttt     360

gaagatataa tgactgacaa aagacaagag gttgagaatc tgcttcagac aattttggat     420

gatgtggaca acactgtcag gtgtaaatat aacatgcatc atgtccttcc tagccttgct     480

gataatatgg atgactgtat cagctcacat catcgttcta aatcagatgg caccatgacg     540

gaggagcgat tgaacttcct tctcttgaat ctccatcatc tatccaagta tcatgctgaa     600

aagatttttc cgttagtgac tgaatatggg attcttcaga acgtttgtgc caacatagga     660

gatttccatg ggttgatagt gaatggttgc attaagcatg agattattga gaatgtctta     720

cctctgtttc aactgatggt cgagagtgta ggacacttcc tttgggagga tcaaactgat     780

ggagactctc aactctccga gctagatgag gatgatcaga ctgatggaga ctctgaactc     840

tctgaactat ctgaggatga tcagactgat ggagactctg aactctctga ggatgatcag     900

attgatggag attttcgact cttcaagcta gcacatctac tcatgaagat tattccaatt     960

gaactggagg tgatgcacat atgttataca aatttgaaag cttcaccatc agtagaagtt    1020

ggacgcttta ttaagcaact ccttgaaacc tctccggatg ttctgagaga atatctgatt    1080

catctacaag agcacatggt aactgttatt accgcaagca cttcaggggc tcgaaacgtt    1140

catgtcatta tagagttcct attgattata gttactgatg tgcccaagga ctttattcat    1200

catgacaaat tgtttgatct tttggcacgt gttggagcac ttgtcaggga ggtatcaact    1260

cttgttcacg acttaataga gaaatcaagg aataaagaga gaatcgacca aacaatttgt    1320

gcaactcgag atttgctgga aaatattgaa ctcttcaagg aagatctcaa aaatgattat    1380

ctgaaagccc cggactcata tcaatgttgc ttccccatga atgatggacc gctgttcatg 1440

catcttctac tcatccactt aaatggtctg ctagattcca attcttattc aattgctttg 1500

ataaaggatg acattagaat ggtgaaaaaa gcctttgaat tgataagatc gttcttcgtg 1560

aattttgagc aaggattgta taaagatctc tgggcacata ttttagatgt ggcttatgag 1620

gctaaagatg tcattgattc aattattgtt cgagataatg gtctcttaca tcttattttc 1680

tcacttccca ttacgataaa aaagatcaag cttatccaag aagaagtctc caatatatct 1740

gagaagattc ccaagaacag gagcctcact gtagtaaact ctactaagaa gattgttgaa 1800

agcaagtcac tgacagttgg taaaataatt gtaggttttg aggaggagac aaattggtta 1860

attagtaagc tcaccagtgg actaaaagat ctagatgtca tctcaatcac tggtatgccg 1920

ggttcgggca aaactacttt ggcatacaaa gtatacaatg atgagtccgt ttctagacgt 1980

ttcgacattt gtgcatggtg cacagttggc caagaatacg acaaaataaa tttgttggag 2040

aaagtttata atcaagttac cggccctgat tcaaaattga gtgagaatat tgatgttgct 2100

gatgagctaa ggagacatct gcttggaaag aggtatctta ttgtcttaga tgacttgtgg 2160

gatactgatg catgggataa gttaacaaga ccttttcctc tagttgagaa tggaagcaga 2220

ataattttga catctcgaga tatggtagtg ggtttgtatg gaaagcgcaa cactgatcct 2280

cttaaccttc gtttgctaag gccagaagaa agttgggagt tattagagaa aagggcattt 2340

ggaaacaaga gctgccctga tgaactattg gatgttggaa aagaaatcgc ccaaaattgt 2400

aagggacttc ctttggttgt tgatctgatt gctggagtca ttgcaggtag ggaaaagaca 2460

aagtctgtat ggcttgaaat tcgaaataat ttgaattcct ttattttgaa tagtgaagtt 2520

tatgtgatga aggttgtgaa attaagttat gaccatttac ctcttcaact aaagccatgc 2580

tttctctacc ttgcgaggta tccgaaggac aatgcagtgg atagaaattg gttgaaaatg 2640

ttttggtgtg ctgaagggct tgttgaacag acagagctga agagtttgga agaagtgatg 2700

gaaatttatt tggataattt aatttccagt agcttggtaa ttgctttcaa tgagataggt 2760

aattacccga cttgccaact acatgatctt gtgcatgact tttgtttgat aaaagcaaaa 2820

gaggaaaagt tgtttgagaa ggtaagttca agtgatctat cttcttcttc agatttgatg 2880

tcgcatatag tgaccattta ttatgataag gagcactttg aatataacaa tttcgtcctg 2940

ctcgattcaa aaatgaaaag gcggtatggt aaacacctct attctttggt gataactgga 3000

gacgagatgg aagatcgttt ttctgatgca tgtcacctaa gagacttgag gcttcttaga 3060

gtgttgtacc tgaagccatc ttttatgatg gtgaaagagt ctttgctgaa tgaaataggc 3120

atgttgaatc atttgaggtt cttaagcatt ggggcagaag ttaaagctct gcctttatct 3180

ttctcaaacc tttggaatct agaaacccta tgggttaaaa acaaaggatc aaccttggta 3240

ctattaccta gtctttggga tcttgtaaag ttgcgagtgc tgtccattga tgattgttct 3300

ttctttgact tggatactgt tgaatcaata ctgataactg tggactcaca gttagagaac 3360

ttgagagaat tagggaaact cgtgctttcc tattcaaaag aaacagaggt tattttcaaa 3420

aggttcccca atcttcaaga gctttcattt gttctcaagg aatcatggga ttactcaaca 3480

gagcgatatt ggttcccgaa atttgatttc ctaactgaac tagaacacct cacagtagat 3540

tttaaaagtt caaattccaa tgacagtggg gcctctgtag cgacaaatcg gtcttgggat 3600

tttcacttcc cttccaattt gagaacattg ttgttgtatg actttcctct ggcatccgat 3660

tcactatcaa caatagcgaa actgcccaat cttgaagagt tgttccttag gagaacaatc 3720

atccatgggg aggaatggaa catgagggag gaagacacct ttgagaatct caaatatttg 3780

aacttgactg aagtgactct tgctaagtgg gaagttggag aggaatcctt tcctgtgatt 3840

gagaaattag tactgcggaa atgtcatatg cttaaggaga ttccacctag tttcggtgat 3900

atttgttcgt tgaaaatcat caaacttgta gagagccctc atgttgaaga ttctgctaag 3960

aagattaaac aatatgttga agatatggga ggggacgagc ttcaggtcct gggctgggag 4020

gatatcccct tatttaagta g 4041

<210> 227
<211> 2586
<212> DNA
<213> Artificial Sequence

<220>
<223> cDNA

<400> 227
atggctgaaa ttcttcttac atcagtaatc aataaatctg tagaaatagc tggaaattta 60

ctgattcaag aaggaaagcg tttatattgg ttgaaagagg atatcgattg gctccagaga 120

gaaatgagac acattcgatc ttatgttgac aacgcaaagg ccaaggaagc tggaggtgat 180

tcaagggtca aaaacttatt gaaagatatt caagaattgg caggtgatgt ggaggatctc 240

ttagatgact tccttccaaa aattcaacaa tccaataagt tcaattattg ccttaagagg 300

agttcttttg cggatgagtt tgctatggag attgagaaga taaagagaag ggttgttgac 360

attgaccgaa taaggaaaac ttacaacatc atagatacag ataacaataa tgatgattgt 420

gttctgctgg atcggagaag attattccta catgctgatg aaacagagat catcggtttg 480

gatgatgact tcaatatgct acaagccaaa ttacttaatc aagatttgca ttatggagtt 540

gtttccatag ttggcatgcc cggtctgggg aaaacaactc ttgccaagaa actttatagg 600

ctcattcgtg atcaatttga gtgttctgga ctggtctacg tttcacaaca gccaagagcg 660

agtgaaatct tacttgacat tgccaaacaa attggactga cggaacagaa aatgaaggaa 720

aatttggagg acaacctgcg atcactcttg aaaataaaaa ggtatgtttt cctcctagat 780

gacatttggg atgtggaaat ttgggatgat ctgaaacttg tccttcctga atgtgattca 840

aaagtcggca gtagaattat aatcacgtct cgaaatagta atgtaggcag atacatagga 900

ggggaatcct ccctccatgc attgcaaccc ctagaatccg agaaaagctt tgaactcttt 960

accaagaaaa tctttaattt tgatgataat aatagttggg ccaatgcttc acctgacttg 1020

gtgaatattg gtagaaatat agttgggaga tgtggaggta taccgctagc catagtggtg 1080

actgcaggca tgttaagggc aagagaaaga acagaacatg cgtggaacag agtacttgag 1140

agtatgggcc ataaagttca agatggatgt gctaaggtat tggctctcag ttacaatgat 1200

ttacctattg cctcaaggcc atgtttcttg tactttggcc tttaccccga ggaccatgaa 1260

attcgtgctt ttgatttgat aaatatgtgg attgctgaga agtttatagt agtaaatagt 1320

ggtaataggc gagaggctga ggatttggcg gaggacgtcc taaatgattt ggtttctaga 1380

aacttgattc aacttgccaa aaggacatat aatggaagaa tttcaagttg tcgcatacat 1440

gacttgttac atagtttgtg tgtggacttg gctaaggaaa gtaacttctt tcacaccgcg 1500

catgatgcat ttggtgatcc cggcaatgtt gctaggctcc gaaggattac attctactct 1560

gacaatgtca tgattgagtt cttccgttca aatcctaagc ttgagaagct tcgtgtactt 1620

ttctgtttcg caaaagaccc ttccatattt tctcatatgg cttattttga cttcaaattg 1680

ttgcacacat tggttgtagt catgtctcaa agttttcaag catatgtcac tatcccaagc 1740

aaatttggga acatgacttg cttacgctat ctgagattgg aggggaatat ttgtggaaaa 1800

ctgccaaata gtattgtcaa gctcacacgt ctagagacca tagacattga tcgacgtagc 1860

ctcattcaac ctccttctgg tgtttgggag tctaaacatt tgagacatct ttgttataga 1920

gattatggac aagcatgtaa cagttgcttt tctataagct cattttaccc aaatatttac 1980

tcattgcatc ctaacaatct acaaaccttg atgtggatac ctgataaatt ttttgaaccg 2040

aggttgttgc accgattgat caatttaaga aaactgggta tactgggagt gtccaattct 2100

accgttaaga tgttatcaat atttagccct gtgctcaagg cgctggaggt tctgaagctc 2160

agtttttcca gtgacccgag tgaacaaata aagttgtcat cgtatccaca tattgctaag 2220

ttgcatttga atgttaacag aacaatggcc ttgaactctc aatcatttcc tccaaatctc 2280

atcaagctta ctctagccaa ctttacggta gaccgttata tactggcagt acttaagaca 2340

tttcccaaat taagaaaact taaaatgttc atctgcaagt ataatgaaga aaagatggat 2400

ctctcgggcg aggcaaatgg ttatagcttt ccgcaacttg aagttttgca tattcatagc 2460

ccgaatgggt tgtctgaagt aacgtgcacg gatgatgtca gtatgcccaa attgaaaaag 2520

ctgttactta caggattcca ttgccgaatc agtttatcgg aacggcttaa aaagctgagt 2580

aaatga 2586

<210> 228
<211> 861
<212> PRT
<213> Solanum lycopersicum

<400> 228
Met Ala Glu Ile Leu Leu Thr Ser Val Ile Asn Lys Ser Val Glu Ile
1               5                   10                  15
Ala Gly Asn Leu Leu Ile Gln Glu Gly Lys Arg Leu Tyr Trp Leu Lys
            20                  25                  30
Glu Asp Ile Asp Trp Leu Gln Arg Glu Met Arg His Ile Arg Ser Tyr
        35                  40                  45
Val Asp Asn Ala Lys Ala Lys Glu Ala Gly Gly Asp Ser Arg Val Lys
    50                  55                  60
Asn Leu Leu Lys Asp Ile Gln Glu Leu Ala Gly Asp Val Glu Asp Leu
65                  70                  75                  80
Leu Asp Asp Phe Leu Pro Lys Ile Gln Gln Ser Asn Lys Phe Asn Tyr
                85                  90                  95
Cys Leu Lys Arg Ser Ser Phe Ala Asp Glu Phe Ala Met Glu Ile Glu
            100                 105                 110
Lys Ile Lys Arg Arg Val Val Asp Ile Asp Arg Ile Arg Lys Thr Tyr
        115                 120                 125
Asn Ile Ile Asp Thr Asp Asn Asn Asn Asp Asp Cys Val Leu Leu Asp
    130                 135                 140
Arg Arg Arg Leu Phe Leu His Ala Asp Glu Thr Glu Ile Ile Gly Leu
145                 150                 155                 160
Asp Asp Asp Phe Asn Met Leu Gln Ala Lys Leu Leu Asn Gln Asp Leu
                165                 170                 175
His Tyr Gly Val Val Ser Ile Val Gly Met Pro Gly Leu Gly Lys Thr
            180                 185                 190
Thr Leu Ala Lys Lys Leu Tyr Arg Leu Ile Arg Asp Gln Phe Glu Cys
        195                 200                 205
Ser Gly Leu Val Tyr Val Ser Gln Gln Pro Arg Ala Ser Glu Ile Leu
    210                 215                 220
Leu Asp Ile Ala Lys Gln Ile Gly Leu Thr Glu Gln Lys Met Lys Glu
225                 230                 235                 240
Asn Leu Glu Asp Asn Leu Arg Ser Leu Leu Lys Ile Lys Arg Tyr Val
                245                 250                 255
Phe Leu Leu Asp Asp Ile Trp Asp Val Glu Ile Trp Asp Asp Leu Lys
            260                 265                 270
Leu Val Leu Pro Glu Cys Asp Ser Lys Val Gly Ser Arg Ile Ile Ile
        275                 280                 285
Thr Ser Arg Asn Ser Asn Val Gly Arg Tyr Ile Gly Gly Glu Ser Ser
    290                 295                 300
Leu His Ala Leu Gln Pro Leu Glu Ser Glu Lys Ser Phe Glu Leu Phe
305                 310                 315                 320
Thr Lys Lys Ile Phe Asn Phe Asp Asp Asn Asn Ser Trp Ala Asn Ala
                325                 330                 335
Ser Pro Asp Leu Val Asn Ile Gly Arg Asn Ile Val Gly Arg Cys Gly
            340                 345                 350
Gly Ile Pro Leu Ala Ile Val Val Thr Ala Gly Met Leu Arg Ala Arg
        355                 360                 365
Glu Arg Thr Glu His Ala Trp Asn Arg Val Leu Glu Ser Met Gly His
    370                 375                 380
Lys Val Gln Asp Gly Cys Ala Lys Val Leu Ala Leu Ser Tyr Asn Asp
385                 390                 395                 400
Leu Pro Ile Ala Ser Arg Pro Cys Phe Leu Tyr Phe Gly Leu Tyr Pro
                405                 410                 415
Glu Asp His Glu Ile Arg Ala Phe Asp Leu Ile Asn Met Trp Ile Ala
            420                 425                 430

Glu Lys Phe Ile Val Val Asn Ser Gly Asn Arg Arg Glu Ala Glu Asp
        435                 440                 445
Leu Ala Glu Asp Val Leu Asn Asp Leu Val Ser Arg Asn Leu Ile Gln
    450                 455                 460
Leu Ala Lys Arg Thr Tyr Asn Gly Arg Ile Ser Ser Cys Arg Ile His
465                 470                 475                 480
Asp Leu Leu His Ser Leu Cys Val Asp Leu Ala Lys Glu Ser Asn Phe
                485                 490                 495
Phe His Thr Ala His Asp Ala Phe Gly Asp Pro Gly Asn Val Ala Arg
            500                 505                 510
Leu Arg Arg Ile Thr Phe Tyr Ser Asp Asn Val Met Ile Glu Phe Phe
        515                 520                 525
Arg Ser Asn Pro Lys Leu Glu Lys Leu Arg Val Leu Phe Cys Phe Ala
    530                 535                 540
Lys Asp Pro Ser Ile Phe Ser His Met Ala Tyr Phe Asp Phe Lys Leu
545                 550                 555                 560
Leu His Thr Leu Val Val Val Met Ser Gln Ser Phe Gln Ala Tyr Val
                565                 570                 575
Thr Ile Pro Ser Lys Phe Gly Asn Met Thr Cys Leu Arg Tyr Leu Arg
            580                 585                 590
Leu Glu Gly Asn Ile Cys Gly Lys Leu Pro Asn Ser Ile Val Lys Leu
        595                 600                 605
Thr Arg Leu Glu Thr Ile Asp Ile Asp Arg Arg Ser Leu Ile Gln Pro
    610                 615                 620
Pro Ser Gly Val Trp Glu Ser Lys His Leu Arg His Leu Cys Tyr Arg
625                 630                 635                 640
Asp Tyr Gly Gln Ala Cys Asn Ser Cys Phe Ser Ile Ser Ser Phe Tyr
                645                 650                 655
Pro Asn Ile Tyr Ser Leu His Pro Asn Asn Leu Gln Thr Leu Met Trp
            660                 665                 670
Ile Pro Asp Lys Phe Phe Glu Pro Arg Leu Leu His Arg Leu Ile Asn
        675                 680                 685
Leu Arg Lys Leu Gly Ile Leu Gly Val Ser Asn Ser Thr Val Lys Met
    690                 695                 700
Leu Ser Ile Phe Ser Pro Val Leu Lys Ala Leu Glu Val Leu Lys Leu
705                 710                 715                 720
Ser Phe Ser Ser Asp Pro Ser Glu Gln Ile Lys Leu Ser Ser Tyr Pro
                725                 730                 735
His Ile Ala Lys Leu His Leu Asn Val Asn Arg Thr Met Ala Leu Asn
            740                 745                 750
Ser Gln Ser Phe Pro Pro Asn Leu Ile Lys Leu Thr Leu Ala Asn Phe
        755                 760                 765
Thr Val Asp Arg Tyr Ile Leu Ala Val Leu Lys Thr Phe Pro Lys Leu
    770                 775                 780
Arg Lys Leu Lys Met Phe Ile Cys Lys Tyr Asn Glu Glu Lys Met Asp
785                 790                 795                 800
Leu Ser Gly Glu Ala Asn Gly Tyr Ser Phe Pro Gln Leu Glu Val Leu
                805                 810                 815
His Ile His Ser Pro Asn Gly Leu Ser Glu Val Thr Cys Thr Asp Asp
            820                 825                 830
Val Ser Met Pro Lys Leu Lys Lys Leu Leu Leu Thr Gly Phe His Cys
        835                 840                 845
Arg Ile Ser Leu Ser Glu Arg Leu Lys Lys Leu Ser Lys
    850                 855                 860

<210> 229
<211> 795
<212> DNA
<213> Artificial Sequence

<220>
<223> cDNA

<400> 229
atggctctag ttgttaaagg taaggtaaat attaatgagt ttatcgatct gtcaaagtct      60

gagaaacttc tcccgtcgat gttcacgcct gtaaagagtg ttatggtttc aaaggttgat     120

aagattatgg tccatgaaaa tgaatcattg tctgaagtaa atctcttaaa aggtgtaaaa     180

cttatagaag gtgggtatgt ttgcttagtg ggtcttgttg tgtccggtga gtggaattta     240

ccagataatt gccgtggtgg tgtgagtgtc tgcatggttg acaagagaat ggaaagagcg     300

gacgaagcca cactggggtc atattacact gctgctgcta aaaagcggtt tcagtttaaa     360

gtggtcccaa attacggtat tacaacaaag gatgcagaaa agaacatatg gcaggtctta     420

gtaaatatta aaaatgtaaa aatgagtgcg ggctactgcc ctttgtcatt agaatttgtg     480

tctgtgtgta ttgtttataa aaataatata aaattgggtt tgagggagaa agtaacgagt     540

gtgaacgatg gaggacccat ggaactttca gaagaagttg ttgatgagtt catggagaat     600

gttccaatgt cggttagact cgcaaagttt cgaaccaaat cctcaaaaag aggtccgaaa     660

aataataata atttgggtaa ggggcgttca ggcggaaggc ctaaaccaaa aagttttgat     720

gaagttgaaa aagagtttga taatttgatt gaagatgaag ccgagacgtc ggtcgcggat     780

tctgattcgt attaa                                                      795


<210> 230
<211> 264
<212> PRT
<213> Tomato mosaic virus


<400> 230
Met Ala Leu Val Val Lys Gly Lys Val Asn Ile Asn Glu Phe Ile Asp
1               5                   10                  15
Leu Ser Lys Ser Glu Lys Leu Leu Pro Ser Met Phe Thr Pro Val Lys
            20                  25                  30
Ser Val Met Val Ser Lys Val Asp Lys Ile Met Val His Glu Asn Glu
        35                  40                  45
Ser Leu Ser Glu Val Asn Leu Leu Lys Gly Val Lys Leu Ile Glu Gly
    50                  55                  60
Gly Tyr Val Cys Leu Val Gly Leu Val Val Ser Gly Glu Trp Asn Leu
65                  70                  75                  80
Pro Asp Asn Cys Arg Gly Gly Val Ser Val Cys Met Val Asp Lys Arg
                85                  90                  95
Met Glu Arg Ala Asp Glu Ala Thr Leu Gly Ser Tyr Tyr Thr Ala Ala
            100                 105                 110
Ala Lys Lys Arg Phe Gln Phe Lys Val Val Pro Asn Tyr Gly Ile Thr
        115                 120                 125
Thr Lys Asp Ala Glu Lys Asn Ile Trp Gln Val Leu Val Asn Ile Lys
    130                 135                 140
Asn Val Lys Met Ser Ala Gly Tyr Cys Pro Leu Ser Leu Glu Phe Val
145                 150                 155                 160
Ser Val Cys Ile Val Tyr Lys Asn Asn Ile Lys Leu Gly Leu Arg Glu
                165                 170                 175
Lys Val Thr Ser Val Asn Asp Gly Gly Pro Met Glu Leu Ser Glu Glu
            180                 185                 190
Val Val Asp Glu Phe Met Glu Asn Val Pro Met Ser Val Arg Leu Ala

195                 200                 205
Lys Phe Arg Thr Lys Ser Ser Lys Arg Gly Pro Lys Asn Asn Asn Asn
    210                 215                 220
Leu Gly Lys Gly Arg Ser Gly Gly Arg Pro Lys Pro Lys Ser Phe Asp
225                 230                 235                 240
Glu Val Glu Lys Glu Phe Asp Asn Leu Ile Glu Asp Glu Ala Glu Thr
                245                 250                 255
Ser Val Ala Asp Ser Asp Ser Tyr
                260

<210> 231
<211> 2586
<212> DNA
<213> Artificial Sequence


<220>
<223> cDNA

<400> 231
atggctgaaa ttcttcttac atcagtaatc aataaatctg tagaaatagc tggaaattta      60

ctgattcaag aaggaaagcg tttatattgg ttgaaagagg atatcgattg gctccagaga     120

gaaatgagac acattcgatc ttatgttgac aacgcaaagg ccaaggaagc tggaggtgat     180

tcaagggtca aaaacttatt gaaagatatt caagaattgg caggtgatgt ggaggatctc     240

ttagatgact tccttccaaa aattcaacaa tccaataagt tcaattattg ccttaagagg     300

agttcttttg cagatgagtt tgctatggag attgagaaga taaagagaag ggttgttgac     360

attgaccgaa taaggaaaac ttacaacatc atagatacag ataacaataa tgatgattgt     420

gttctgctgg atcggagaag attattccta catgctgatg aaacagagat catcggtttg     480

gatgatgact tcaatatgct acaagccaaa ttacttaatc aagatttgca ttatggagtt     540

gtttccatag ttggcatgcc cggtctgggg aaaacaactc ttgccaagaa actttatagg     600

ctcattcgtg atcaatttga gtgttctgga ctggtctacg tttcacaaca gccaagagcg     660

agtgaaatct tacttgacat tgccaaacaa attggactga cggaacagaa aatgaaggaa     720

aatttggagg acaacctgcg atcactcttg aaaataaaaa ggtatgttat cctcctagat     780

gacatttggg atgtggaaat ttgggatgat ctgaaacttg tccttcctga atgtgattca     840

aaagtcggca gtagaatgat aatcacgtct cgaaatagta atgtaggcag atacatagga     900

ggggaatcct ccctccatgc attgcaaccc ctagaatccg agaaaagctt tgaactcttt     960

accaagaaaa tctttaattt tgatgataat aatagttggg ccaatgcttc acctgacttg    1020

gtgaatattg gtagaaatat agttgggaga tgtggaggta taccgctagc catagtggtg    1080

actgcaggca tgttaagggc aagagaaaga acagaacatg cgtggaacag agtacttgag    1140

agtatgggcc ataaagttca agatggatgt gctaaggtat tggctctcag ttacaatgat    1200

ttacctattg cctcaaggcc atgtttcttg tactttggcc tttaccccga ggaccatgaa    1260

attcgtgctt ttgatttgat aaatatgtgg attgctgaga agtttatagt agtaaatagt    1320

ggtaataggc gagaggctga ggatttggcg gaggacgtcc taaatgattt ggtttctaga 1380

aacttgattc aacttgccaa aaggacatat aatggaagaa tttcaagttg tcgcatacat 1440

gacttgttac atagtttgtg tgtggacttg gctaaggaaa gtaacttctt tcacaccgcg 1500

catgatgcat ttggtgatcc cggcaatgtt gctaggctcc gaaggattac attctactct 1560

gacaatgtca tgattgagtt cttccgttca aatcctaagc ttgagaagct tcgtgtactt 1620

ttctgtttcg caaaagaccc ttccatattt tctcatatgg cttattttga cttcaaattg 1680

ttgcacacat tggttgtagt catgtctcaa agttttcaag catatgtcac tatcccaagc 1740

aaatttggga acatgacttg cttacgctat ctgagattgg aggggaatat ttgtggaaaa 1800

ctgccaaata gtattgtcaa gctcacacgt ctagagacca tagacattga tcgacgtagc 1860

ctcattcaac ctccttctgg tgtttgggag tctaaacatt tgagacatct ttgttataga 1920

gattatggac aagcatgtaa cagttgcttt tctataagct cattttaccc aaatatttac 1980

tcattgcatc ctaacaatct acaaaccttg atgtggatac ctgataaatt ttttgaaccg 2040

aggttgttgc accgattgat caatttaaga aaactgggta tactgggagt gtccaattct 2100

accgttaaga tgttatcaat atttagccct gtgcttaagg cgctggaggt tctgaagctc 2160

agtttttcca gtgacccgag tgaacaaata aagttgtcat cgtatccaca tattgctaag 2220

ttgcatttga atgttaacag aacaatggcc ttgaactctc aatcatttcc tccaaatctc 2280

atcaagctta ctctagccta ctttagtgta gaccgttata tactggcagt acttaagaca 2340

tttcccaaat taagaaaact taaaatgttc atctgcaagt ataatgaaga aaagatggat 2400

ctctcgggcg aggcaaatgg ttatagcttt ccgcaacttg aagttttgca tattcatagc 2460

ccgaatgggt tgtctgaagt aacgtgcacg gatgatgtca gtatgcccaa attgaaaaag 2520

ctgttactta caggattcca ttgccgaatc agtttatcgg aacggcttaa aaagctgagt 2580

aaatga 2586

<210> 232
<211> 861
<212> PRT
<213> Solanum lycopersicum

<400> 232
Met Ala Glu Ile Leu Leu Thr Ser Val Ile Asn Lys Ser Val Glu Ile
1               5                   10                  15
Ala Gly Asn Leu Leu Ile Gln Glu Gly Lys Arg Leu Tyr Trp Leu Lys
            20                  25                  30
Glu Asp Ile Asp Trp Leu Gln Arg Glu Met Arg His Ile Arg Ser Tyr
        35                  40                  45
Val Asp Asn Ala Lys Ala Lys Glu Ala Gly Gly Asp Ser Arg Val Lys
    50                  55                  60
Asn Leu Leu Lys Asp Ile Gln Glu Leu Ala Gly Asp Val Glu Asp Leu
65                  70                  75                  80

Leu Asp Asp Phe Leu Pro Lys Ile Gln Gln Ser Asn Lys Phe Asn Tyr
                85                  90                  95
Cys Leu Lys Arg Ser Ser Phe Ala Asp Glu Phe Ala Met Glu Ile Glu
            100                 105                 110
Lys Ile Lys Arg Arg Val Val Asp Ile Asp Arg Ile Arg Lys Thr Tyr
        115                 120                 125
Asn Ile Ile Asp Thr Asp Asn Asn Asn Asp Asp Cys Val Leu Leu Asp
    130                 135                 140
Arg Arg Arg Leu Phe Leu His Ala Asp Glu Thr Glu Ile Ile Gly Leu
145                 150                 155                 160
Asp Asp Asp Phe Asn Met Leu Gln Ala Lys Leu Leu Asn Gln Asp Leu
                165                 170                 175
His Tyr Gly Val Val Ser Ile Val Gly Met Pro Gly Leu Gly Lys Thr
            180                 185                 190
Thr Leu Ala Lys Lys Leu Tyr Arg Leu Ile Arg Asp Gln Phe Glu Cys
        195                 200                 205
Ser Gly Leu Val Tyr Val Ser Gln Gln Pro Arg Ala Ser Glu Ile Leu
    210                 215                 220
Leu Asp Ile Ala Lys Gln Ile Gly Leu Thr Glu Gln Lys Met Lys Glu
225                 230                 235                 240
Asn Leu Glu Asp Asn Leu Arg Ser Leu Leu Lys Ile Lys Arg Tyr Val
                245                 250                 255
Ile Leu Leu Asp Asp Ile Trp Asp Val Glu Ile Trp Asp Asp Leu Lys
            260                 265                 270
Leu Val Leu Pro Glu Cys Asp Ser Lys Val Gly Ser Arg Met Ile Ile
        275                 280                 285
Thr Ser Arg Asn Ser Asn Val Gly Arg Tyr Ile Gly Gly Glu Ser Ser
    290                 295                 300
Leu His Ala Leu Gln Pro Leu Glu Ser Glu Lys Ser Phe Glu Leu Phe
305                 310                 315                 320
Thr Lys Lys Ile Phe Asn Phe Asp Asp Asn Asn Ser Trp Ala Asn Ala
                325                 330                 335
Ser Pro Asp Leu Val Asn Ile Gly Arg Asn Ile Val Gly Arg Cys Gly
            340                 345                 350
Gly Ile Pro Leu Ala Ile Val Val Thr Ala Gly Met Leu Arg Ala Arg
        355                 360                 365
Glu Arg Thr Glu His Ala Trp Asn Arg Val Leu Glu Ser Met Gly His
    370                 375                 380
Lys Val Gln Asp Gly Cys Ala Lys Val Leu Ala Leu Ser Tyr Asn Asp
385                 390                 395                 400
Leu Pro Ile Ala Ser Arg Pro Cys Phe Leu Tyr Phe Gly Leu Tyr Pro
                405                 410                 415
Glu Asp His Glu Ile Arg Ala Phe Asp Leu Ile Asn Met Trp Ile Ala
            420                 425                 430
Glu Lys Phe Ile Val Val Asn Ser Gly Asn Arg Arg Glu Ala Glu Asp
        435                 440                 445
Leu Ala Glu Asp Val Leu Asn Asp Leu Val Ser Arg Asn Leu Ile Gln
    450                 455                 460
Leu Ala Lys Arg Thr Tyr Asn Gly Arg Ile Ser Ser Cys Arg Ile His
465                 470                 475                 480
Asp Leu Leu His Ser Leu Cys Val Asp Leu Ala Lys Glu Ser Asn Phe
                485                 490                 495
Phe His Thr Ala His Asp Ala Phe Gly Asp Pro Gly Asn Val Ala Arg
            500                 505                 510
Leu Arg Arg Ile Thr Phe Tyr Ser Asp Asn Val Met Ile Glu Phe Phe
        515                 520                 525
Arg Ser Asn Pro Lys Leu Glu Lys Leu Arg Val Leu Phe Cys Phe Ala
    530                 535                 540
Lys Asp Pro Ser Ile Phe Ser His Met Ala Tyr Phe Asp Phe Lys Leu
545                 550                 555                 560
Leu His Thr Leu Val Val Val Met Ser Gln Ser Phe Gln Ala Tyr Val
                565                 570                 575
Thr Ile Pro Ser Lys Phe Gly Asn Met Thr Cys Leu Arg Tyr Leu Arg

580                 585                 590
Leu Glu Gly Asn Ile Cys Gly Lys Leu Pro Asn Ser Ile Val Lys Leu
        595                 600                 605
Thr Arg Leu Glu Thr Ile Asp Ile Asp Arg Arg Ser Leu Ile Gln Pro
    610                 615                 620
Pro Ser Gly Val Trp Glu Ser Lys His Leu Arg His Leu Cys Tyr Arg
625                 630                 635                 640
Asp Tyr Gly Gln Ala Cys Asn Ser Cys Phe Ser Ile Ser Ser Phe Tyr
                645                 650                 655
Pro Asn Ile Tyr Ser Leu His Pro Asn Asn Leu Gln Thr Leu Met Trp
            660                 665                 670
Ile Pro Asp Lys Phe Phe Glu Pro Arg Leu Leu His Arg Leu Ile Asn
        675                 680                 685
Leu Arg Lys Leu Gly Ile Leu Gly Val Ser Asn Ser Thr Val Lys Met
    690                 695                 700
Leu Ser Ile Phe Ser Pro Val Leu Lys Ala Leu Glu Val Leu Lys Leu
705                 710                 715                 720
Ser Phe Ser Ser Asp Pro Ser Glu Gln Ile Lys Leu Ser Ser Tyr Pro
                725                 730                 735
His Ile Ala Lys Leu His Leu Asn Val Asn Arg Thr Met Ala Leu Asn
            740                 745                 750
Ser Gln Ser Phe Pro Pro Asn Leu Ile Lys Leu Thr Leu Ala Tyr Phe
        755                 760                 765
Ser Val Asp Arg Tyr Ile Leu Ala Val Leu Lys Thr Phe Pro Lys Leu
    770                 775                 780
Arg Lys Leu Lys Met Phe Ile Cys Lys Tyr Asn Glu Glu Lys Met Asp
785                 790                 795                 800
Leu Ser Gly Glu Ala Asn Gly Tyr Ser Phe Pro Gln Leu Glu Val Leu
                805                 810                 815
His Ile His Ser Pro Asn Gly Leu Ser Glu Val Thr Cys Thr Asp Asp
            820                 825                 830
Val Ser Met Pro Lys Leu Lys Lys Leu Leu Leu Thr Gly Phe His Cys
        835                 840                 845
Arg Ile Ser Leu Ser Glu Arg Leu Lys Lys Leu Ser Lys
    850                 855                 860

<210> 233
<211> 6351
<212> DNA
<213> Artificial Sequence

<220>
<223> cDNA

<400> 233
atggaaatgg taactaatat tttgagccca gtcgcagaac acttgatcat accgccagtt      60

gtgcgacaga ttgggtattt gttttactat aggcgcaaca tcaggtctct ggatgaagaa     120

tctaataagc tcgagaatat cagaagtggg gtgaatcaaa gagtggaggc tgcccggaga     180

aacttacaag tcatttcacc caatgttgag gcttggttaa ctagtgttga tactactact     240

gctgatgtgg cagttgtaat gggaggtcga actgaggttg aaaggggttg catctacggt     300

tggtgtccaa cattgaagtc acgttacttg ctgagcagga gagccaagaa aattacactg     360

gaagttacta aacttcaaaa ggaaggtaac gagtatgctg ttttctgcta tccggttcca     420

gctgatgaaa ttgaagctat acctagtaac actaatgagg agtttgtctc tagaaaacag     480

aaggaggaag aggtcatgac agctttgaaa gatgaggaga tcactattgt tgggatatgt     540

ggtatgggtg gtgttggtaa aacatcactg gctgagaaag taagagcaag ggcaaaacaa 600
gaaaggttat ttaatgatgt tgtcatggta actgtcagtc aacaacaaga cctgaaaaga 660
attcagggcg agatcgccgg agaggtcggg ctgacgtcat tgcaaggaga caatttgtta 720
agtcgtggag atcagctgcg tgcaaggtta atgcaagaag gaagtcgcgt ccttgtaatt 780
ttggatgatg tttgggaggc tcttgatgat ctagagaaac ttggaattcc cactggcagc 840
aaccacaact atcagtgcaa agtggcattg acgacgcgcc gccaagatgt ttgtgacgct 900
atgaaggctc aaaagactgt agatgttgta atcttatctg atgaagaagc atgggtcctt 960
tttaggcaga aagccggtta ttcagctgat gatccctctc tttctgaagt agcaaaagaa 1020
gttgccaaag aatgcaaggg gctgccactt gcaattgtta ttgttgcagg agcactaaag 1080
actaaaacca agccttcatg ggatgatgcc cttgtagaat taagaaaagc atcaccaaaa 1140
aatatcccaa gagtgcttac aaaggtgtat caacctctga agacaagcta tgatcactta 1200
gagagtgatg aagccaggta tgtctttttg ctttgttcct tgtttgagga agatagtgat 1260
atttggaccg aagaattact taaatatgga gtgggacttg gcatcttttc ggaactcgaa 1320
aatttagaat gtgcaagaaa tagggtgtgt aatctgttag aaacattgaa aaattgtttc 1380
ttgttatccc aaggtaaaga caaaaattat gtcaaaatgc atgatgtggt ccgtgacgtg 1440
gctatatata ttgcgtctga gggtgagcat aaatttctgg taaatcacaa tgtgaactca 1500
aacgtgttct tgagaaaagt ctcttatgag caatacagtc acatgtcaat tgttgcaaat 1560
aaatttgatg agcgtcccac accaatattt tgcccacgat tgaagcttct gatgttaaaa 1620
ctccgtttcg aagagggttt caaattacag gatgattttt ttgatggaat gagtgaactc 1680
agtgtcataa aactgagtgg atatgatcga aactccattc tgccctttcc atcatccatt 1740
cagaggttgt caaatctgag cacgctttgg ctgagtaatc taaggttgga tgacgtgtcc 1800
attattggga aacttgtcac tttagaaatt ctcagcatca gaggttctga cttacaggag 1860
cttccagtgg agatcggaaa cttggccaat ctaactatgt tagagtattg gaatactggt 1920
tacagaaaac gtatgaggat ttcaccaggg gtcttatcaa gactagttcg attggaggaa 1980
ctacatatgg tgggagtaga agattgtagt tactccacct tgagggagct ggaatcgtta 2040
tcgagattga ctgcactggc atttgatgaa tgttccgtag atgtgattta cagtaacttg 2100
ggcctttcct ccaagttgac acggtacgct cttaaaatgg gtagacacta cacgtttact 2160
tcattcatgg aaacttacaa caaggctatc gatctagacg tcaccaaggg caccccattg 2220
ggtgattgga tctgcctcct gttgaggaat agcgaagttg tacattcaag aggaaagggc 2280
tccaagaatg taatggtcga gttgcagaat gtgaaagatc tcatgctgtc tgattgtgat 2340
tcattgaata tccactatca gaataatatt tcattccccg aactcgaaag gctggaggta 2400

cgctattgtg attatctacg gcatcttttt tgtgtgtcct tggcttgccc tgatgagggg 2460

acctctcgaa ggacacacat cagacctgac gtaatcaagt tccccaattt acatagcttg 2520

acacttcgta atctggaatt cttcacacac ttttacagtg acactgttga gggcattgag 2580

ttccctctgt tacgggtaat atatctcagg ggattaccag agttccaaaa tttctggcca 2640

acagccaaca atgctatcac cgactcaaat cctcttttta atgaaaaggt ttcttgtccc 2700

aacctgaaag tgctaaaact ccatgaggct aacagcataa ctgctctgtg ctctcaccaa 2760

cttccaacta cctacttcag caaacttgag acattgaaag tagaaaattg tggaaaattg 2820

agacacttga tgtctccatc agtggccaga ggtcttctga atctccgaat attactatta 2880

gaatactgcg aatcaatgga agaagtgatc atagaagaag aacaagaagg agatgaaatc 2940

atgtgtaacg aacccttatt tccccaattg gaagagctgg aacttcaaaa tctgccaaag 3000

ctgaggcatt tcattttgac gaagcaagct cttgaatttc cattcctcat agaagtgaag 3060

attcgtaact gccctgaaat gaagatgctt gtccaacgga gatttgtcag tacatcaagt 3120

ctcaaaagtg tgaacaatga tgatgagttg aaagttgttg atctcaacaa agcgatgttt 3180

aattctaagg tttcttgtcc caacctgaaa gtgctaaaac tccatgaggc taacaacata 3240

actgctctgt gctctcacca acttccaact acctacttca gcaaacttga gacattggaa 3300

gtagaaaatt gtggaaaatt gagacacttg atgtctccat cagtggccag aggtcttctg 3360

aatctccgaa tattactatt aggatactgt gaatcaatgg aagaagtgat catagaagaa 3420

gaacaagaag gagaagaaaa catgactaat gagcccttat ttcccctgtt ggaagagctg 3480

atactcgata agctaccaaa gctggggcat ttctttctga cgaagcgtgc tcttgaattt 3540

ccattcctca gagaagtgaa gattcgtaaa tgccctgaaa tgaagatgct tgtccaacag 3600

agatctgtca gtacatcaag tctcaaaagt gtgaacaatg atgatgagtt gaaagttgtt 3660

gatctcaaca aagcgatgtt taattctaag gtttcttgtc ccaacctgaa agtgctaaaa 3720

ctccatgagg ctaacagcat aactgctctg tgctctcacc aacttccaac tacctacttc 3780

agcaaacttg agacattgga agtagaaaat tgtggaaaat tgagacactt gatgtctcca 3840

tcagtggcca gaggtcttct gaatctccga atattactat taggatactg tgaatcaatg 3900

gaagaagtga tcatagaaga agaacaagaa ggagaagaaa acatgactaa tgagccctta 3960

tttcccctgt tggaagagct gatactcgat aagctaccaa agctggggca tttctttctg 4020

acgaagcgtg ctcttgaatt tccattcctc agagaagtga agattcgtaa atgccctgaa 4080

atgaagatgc ttgtccaaca gagatctgtc agtacatcaa gtctcaaaag tgtgaacaat 4140

gatgatgagt tgaaagttgt tgatctcaac aaagcgatgt ttaattctaa ggtttcttgt 4200

cccaacctga aagtgctaaa actccatgag gctaacaaca taactgctct gtgctctcac 4260

caacttccaa ctacctactt cagcaaactt gagacattgg aagtagaaaa ttgtggaaaa 4320

ttgagacact tgatgtctcc atcagtggcc agaggtcttc tgaatctccg aatattacta 4380

ttaggatact gtgaatcaat ggaagaagtg atcatagaag aagaacaaga aggagaagaa 4440

aacatgacta atgagccctt atttcccctg ttggaagagc tgatactcga taagctacca 4500

aagctggggc atttctttct gacgaagcgt gctcttgaat ttccattcct cagagaagtg 4560

aagattcgta aatgccctga aatgaagatg cttgtccaac agagatctgt cagtacatca 4620

agtctcaaaa gtgtgaacaa tgatgatgag ttgaaagttg ttgatctcaa caaagcgatg 4680

tttaattcta aggtttcttg tcccaacctg aaagtgctaa aactccatga ggctaacaac 4740

ataactgctc tgtgctctca ccaacttcca actacctact tcagcaaact tgagacattg 4800

gaagtagaaa attgtggaaa attgagacac ttgatgtctc catcagtggc cagaggtctt 4860

ctgaatctcc gaatattact attaggatac tgtgaatcaa tggaagaagt gatcatagaa 4920

gaagaacaag aaggagaaga aaacatgact aatgagccct tatttcccct gttggaagag 4980

ctgatactcg ataagctacc aaagctgggg catttctttc tgacgaagcg tgctcttgaa 5040

tttccattcc tcagagaagt gaagattcgt aaatgccctg aaatgaagat gcttgtccaa 5100

cagagatctg tcagtacatc aagtctcaaa agtgtgaaca atgatgatga gttgaaagtt 5160

gttgatctca acaaagcgat gtttaattct aaggtttctt gtcccaacct ggaattgcta 5220

cgactctata aggctaacag cgtaggttct ctattctctc accaacttcc aactacctac 5280

ttcagcaaac ttgagacatt ggaagtagaa aattgtggaa aattgagaca cttgatgtct 5340

ccatcagtgg ccagaggtct tctgaatctc cgaatattaa tattaagaga ctgtgaatca 5400

atggaagaag tgatcacaga agaggaacaa gaaggagatg aaatcatgtg taatgaacca 5460

ttatttcccc aattggaaga gctgaaactt gaaaatctgc caaagctgag gcatttcatt 5520

ttgacaaagc aagctcttga aattcccttc ctcatagaag tgcagattcg taactgccct 5580

gaaatgaaga tgcttgtcca acagagatct gtcagtacat caagtctcaa aagtgtgaac 5640

aatgatgatg agttgaaagt tgttgatctc aacaaagcga tgtttaattc taaggtttct 5700

tgtcccaacc tggaattgct acgactctat aaggctaaca gcgtaggttc tctattctct 5760

caccaacttc caactaccta cttcagcaaa cttatgcaac taggaatacg gaattgtgga 5820

aaattgagac acttgatgtc tccatcagtg gccagaggtc ttctgaatct ccgaatatta 5880

atattaggag actgtgaatc aatggaagaa gtgatcacag aagaggaaca agaaggagaa 5940

gaaaacatga ctaatgagtc cttatttccc tgcttggaaa tgctgaaact tgaaaatctg 6000

ccaaagctgg ggcatttctt tctgacgaag cgtgctcttg aatttccatt cctcagagaa 6060

gtgaagattc gtacatgccc tgaaatgaag atatttgtcc aacatggatc tgtgagtaca 6120

ccaagtctcg aaattgtgaa caatgatgat gaggtaaaag tagatgatct gaacgaatgg 6180

atacatcaga ggttcaattc taaggaagaa gatggaagtg aatctgaatc ttctcaggaa 6240

gaagatggaa gcgaatctga agcttctcat gaagatggaa gcaaatgtga agcttctcag 6300

gaagaagata gaagcgaatc tgaagattca atgagtgacg gcagaagcta a 6351

<210> 234
<211> 2116
<212> PRT
<213> Capsicum chinense

<400> 234
Met Glu Met Val Thr Asn Ile Leu Ser Pro Val Ala Glu His Leu Ile
1               5                   10                  15
Ile Pro Pro Val Val Arg Gln Ile Gly Tyr Leu Phe Tyr Tyr Arg Arg
            20                  25                  30
Asn Ile Arg Ser Leu Asp Glu Glu Ser Asn Lys Leu Glu Asn Ile Arg
        35                  40                  45
Ser Gly Val Asn Gln Arg Val Glu Ala Ala Arg Arg Asn Leu Gln Val
    50                  55                  60
Ile Ser Pro Asn Val Glu Ala Trp Leu Thr Ser Val Asp Thr Thr Thr
65                  70                  75                  80
Ala Asp Val Ala Val Val Met Gly Gly Arg Thr Glu Val Glu Arg Gly
                85                  90                  95
Cys Ile Tyr Gly Trp Cys Pro Thr Leu Lys Ser Arg Tyr Leu Leu Ser
            100                 105                 110
Arg Arg Ala Lys Lys Ile Thr Leu Glu Val Thr Lys Leu Gln Lys Glu
        115                 120                 125
Gly Asn Glu Tyr Ala Val Phe Cys Tyr Pro Val Pro Ala Asp Glu Ile
    130                 135                 140
Glu Ala Ile Pro Ser Asn Thr Asn Glu Glu Phe Val Ser Arg Lys Gln
145                 150                 155                 160
Lys Glu Glu Glu Val Met Thr Ala Leu Lys Asp Glu Glu Ile Thr Ile
                165                 170                 175
Val Gly Ile Cys Gly Met Gly Gly Val Gly Lys Thr Ser Leu Ala Glu
            180                 185                 190
Lys Val Arg Ala Arg Ala Lys Gln Glu Arg Leu Phe Asn Asp Val Val
        195                 200                 205
Met Val Thr Val Ser Gln Gln Gln Asp Leu Lys Arg Ile Gln Gly Glu
    210                 215                 220
Ile Ala Gly Glu Val Gly Leu Thr Ser Leu Gln Gly Asp Asn Leu Leu
225                 230                 235                 240
Ser Arg Gly Asp Gln Leu Arg Ala Arg Leu Met Gln Glu Gly Ser Arg
                245                 250                 255
Val Leu Val Ile Leu Asp Asp Val Trp Glu Ala Leu Asp Asp Leu Glu
            260                 265                 270
Lys Leu Gly Ile Pro Thr Gly Ser Asn His Asn Tyr Gln Cys Lys Val
        275                 280                 285
Ala Leu Thr Thr Arg Arg Gln Asp Val Cys Asp Ala Met Lys Ala Gln
    290                 295                 300
Lys Thr Val Asp Val Val Ile Leu Ser Asp Glu Glu Ala Trp Val Leu
305                 310                 315                 320
Phe Arg Gln Lys Ala Gly Tyr Ser Ala Asp Asp Pro Ser Leu Ser Glu
                325                 330                 335
Val Ala Lys Glu Val Ala Lys Glu Cys Lys Gly Leu Pro Leu Ala Ile
            340                 345                 350
Val Ile Val Ala Gly Ala Leu Lys Thr Lys Thr Lys Pro Ser Trp Asp
        355                 360                 365
Asp Ala Leu Val Glu Leu Arg Lys Ala Ser Pro Lys Asn Ile Pro Arg
    370                 375                 380
Val Leu Thr Lys Val Tyr Gln Pro Leu Lys Thr Ser Tyr Asp His Leu

385                 390                 395                 400
Glu Ser Asp Glu Ala Arg Tyr Val Phe Leu Leu Cys Ser Leu Phe Glu
                405                 410                 415
Glu Asp Ser Asp Ile Trp Thr Glu Glu Leu Leu Lys Tyr Gly Val Gly
            420                 425                 430
Leu Gly Ile Phe Ser Glu Leu Glu Asn Leu Glu Cys Ala Arg Asn Arg
        435                 440                 445
Val Cys Asn Leu Leu Glu Thr Leu Lys Asn Cys Phe Leu Leu Ser Gln
    450                 455                 460
Gly Lys Asp Lys Asn Tyr Val Lys Met His Asp Val Val Arg Asp Val
465                 470                 475                 480
Ala Ile Tyr Ile Ala Ser Glu Gly Glu His Lys Phe Leu Val Asn His
                485                 490                 495
Asn Val Asn Ser Asn Val Phe Leu Arg Lys Val Ser Tyr Glu Gln Tyr
            500                 505                 510
Ser His Met Ser Ile Val Ala Asn Lys Phe Asp Glu Arg Pro Thr Pro
        515                 520                 525
Ile Phe Cys Pro Arg Leu Lys Leu Leu Met Leu Lys Leu Arg Phe Glu
    530                 535                 540
Glu Gly Phe Lys Leu Gln Asp Asp Phe Phe Asp Gly Met Ser Glu Leu
545                 550                 555                 560
Ser Val Ile Lys Leu Ser Gly Tyr Asp Arg Asn Ser Ile Leu Pro Phe
                565                 570                 575
Pro Ser Ser Ile Gln Arg Leu Ser Asn Leu Ser Thr Leu Trp Leu Ser
            580                 585                 590
Asn Leu Arg Leu Asp Asp Val Ser Ile Ile Gly Lys Leu Val Thr Leu
        595                 600                 605
Glu Ile Leu Ser Ile Arg Gly Ser Asp Leu Gln Glu Leu Pro Val Glu
    610                 615                 620
Ile Gly Asn Leu Ala Asn Leu Thr Met Leu Glu Tyr Trp Asn Thr Gly
625                 630                 635                 640
Tyr Arg Lys Arg Met Arg Ile Ser Pro Gly Val Leu Ser Arg Leu Val
                645                 650                 655
Arg Leu Glu Glu Leu His Met Val Gly Val Glu Asp Cys Ser Tyr Ser
            660                 665                 670
Thr Leu Arg Glu Leu Glu Ser Leu Ser Arg Leu Thr Ala Leu Ala Phe
        675                 680                 685
Asp Glu Cys Ser Val Asp Val Ile Tyr Ser Asn Leu Gly Leu Ser Ser
    690                 695                 700
Lys Leu Thr Arg Tyr Ala Leu Lys Met Gly Arg His Tyr Thr Phe Thr
705                 710                 715                 720
Ser Phe Met Glu Thr Tyr Asn Lys Ala Ile Asp Leu Asp Val Thr Lys
                725                 730                 735
Gly Thr Pro Leu Gly Asp Trp Ile Cys Leu Leu Leu Arg Asn Ser Glu
            740                 745                 750
Val Val His Ser Arg Gly Lys Gly Ser Lys Asn Val Met Val Glu Leu
        755                 760                 765
Gln Asn Val Lys Asp Leu Met Leu Ser Asp Cys Asp Ser Leu Asn Ile
    770                 775                 780
His Tyr Gln Asn Asn Ile Ser Phe Pro Glu Leu Glu Arg Leu Glu Val
785                 790                 795                 800
Arg Tyr Cys Asp Tyr Leu Arg His Leu Phe Cys Val Ser Leu Ala Cys
                805                 810                 815
Pro Asp Glu Gly Thr Ser Arg Arg Thr His Ile Arg Pro Asp Val Ile
            820                 825                 830
Lys Phe Pro Asn Leu His Ser Leu Thr Leu Arg Asn Leu Glu Phe Phe
        835                 840                 845
Thr His Phe Tyr Ser Asp Thr Val Glu Gly Ile Glu Phe Pro Leu Leu
    850                 855                 860
Arg Val Ile Tyr Leu Arg Gly Leu Pro Glu Phe Gln Asn Phe Trp Pro
865                 870                 875                 880
Thr Ala Asn Asn Ala Ile Thr Asp Ser Asn Pro Leu Phe Asn Glu Lys
                885                 890                 895

Val Ser Cys Pro Asn Leu Lys Val Leu Lys Leu His Glu Ala Asn Ser
            900                 905                 910
Ile Thr Ala Leu Cys Ser His Gln Leu Pro Thr Thr Tyr Phe Ser Lys
        915                 920                 925
Leu Glu Thr Leu Lys Val Glu Asn Cys Gly Lys Leu Arg His Leu Met
    930                 935                 940
Ser Pro Ser Val Ala Arg Gly Leu Leu Asn Leu Arg Ile Leu Leu Leu
945                 950                 955                 960
Glu Tyr Cys Glu Ser Met Glu Glu Val Ile Ile Glu Glu Glu Gln Glu
                965                 970                 975
Gly Asp Glu Ile Met Cys Asn Glu Pro Leu Phe Pro Gln Leu Glu Glu
            980                 985                 990
Leu Glu Leu Gln Asn Leu Pro Lys Leu Arg His Phe Ile Leu Thr Lys
        995                 1000                1005
Gln Ala Leu Glu Phe Pro Phe Leu Ile Glu Val Lys Ile Arg Asn Cys
    1010                1015                1020
Pro Glu Met Lys Met Leu Val Gln Arg Arg Phe Val Ser Thr Ser Ser
1025                1030                1035                1040
Leu Lys Ser Val Asn Asn Asp Asp Glu Leu Lys Val Val Asp Leu Asn
                1045                1050                1055
Lys Ala Met Phe Asn Ser Lys Val Ser Cys Pro Asn Leu Lys Val Leu
            1060                1065                1070
Lys Leu His Glu Ala Asn Asn Ile Thr Ala Leu Cys Ser His Gln Leu
        1075                1080                1085
Pro Thr Thr Tyr Phe Ser Lys Leu Glu Thr Leu Glu Val Glu Asn Cys
    1090                1095                1100
Gly Lys Leu Arg His Leu Met Ser Pro Ser Val Ala Arg Gly Leu Leu
1105                1110                1115                1120
Asn Leu Arg Ile Leu Leu Leu Gly Tyr Cys Glu Ser Met Glu Glu Val
                1125                1130                1135
Ile Ile Glu Glu Glu Gln Glu Gly Glu Glu Asn Met Thr Asn Glu Pro
            1140                1145                1150
Leu Phe Pro Leu Leu Glu Glu Leu Ile Leu Asp Lys Leu Pro Lys Leu
        1155                1160                1165
Gly His Phe Phe Leu Thr Lys Arg Ala Leu Glu Phe Pro Phe Leu Arg
    1170                1175                1180
Glu Val Lys Ile Arg Lys Cys Pro Glu Met Lys Met Leu Val Gln Gln
1185                1190                1195                1200
Arg Ser Val Ser Thr Ser Ser Leu Lys Ser Val Asn Asn Asp Asp Glu
                1205                1210                1215
Leu Lys Val Val Asp Leu Asn Lys Ala Met Phe Asn Ser Lys Val Ser
            1220                1225                1230
Cys Pro Asn Leu Lys Val Leu Lys Leu His Glu Ala Asn Ser Ile Thr
        1235                1240                1245
Ala Leu Cys Ser His Gln Leu Pro Thr Thr Tyr Phe Ser Lys Leu Glu
    1250                1255                1260
Thr Leu Glu Val Glu Asn Cys Gly Lys Leu Arg His Leu Met Ser Pro
1265                1270                1275                1280
Ser Val Ala Arg Gly Leu Leu Asn Leu Arg Ile Leu Leu Leu Gly Tyr
                1285                1290                1295
Cys Glu Ser Met Glu Glu Val Ile Ile Glu Glu Glu Gln Glu Gly Glu
            1300                1305                1310
Glu Asn Met Thr Asn Glu Pro Leu Phe Pro Leu Leu Glu Glu Leu Ile
        1315                1320                1325
Leu Asp Lys Leu Pro Lys Leu Gly His Phe Phe Leu Thr Lys Arg Ala
    1330                1335                1340
Leu Glu Phe Pro Phe Leu Arg Glu Val Lys Ile Arg Lys Cys Pro Glu
1345                1350                1355                1360
Met Lys Met Leu Val Gln Gln Arg Ser Val Ser Thr Ser Ser Leu Lys
                1365                1370                1375
Ser Val Asn Asn Asp Asp Glu Leu Lys Val Val Asp Leu Asn Lys Ala
            1380                1385                1390
Met Phe Asn Ser Lys Val Ser Cys Pro Asn Leu Lys Val Leu Lys Leu

            1395                1400                1405
His Glu Ala Asn Asn Ile Thr Ala Leu Cys Ser His Gln Leu Pro Thr
    1410                1415                1420
Thr Tyr Phe Ser Lys Leu Glu Thr Leu Glu Val Glu Asn Cys Gly Lys
1425                1430                1435                1440
Leu Arg His Leu Met Ser Pro Ser Val Ala Arg Gly Leu Leu Asn Leu
                1445                1450                1455
Arg Ile Leu Leu Leu Gly Tyr Cys Glu Ser Met Glu Glu Val Ile Ile
            1460                1465                1470
Glu Glu Glu Gln Glu Gly Glu Glu Asn Met Thr Asn Glu Pro Leu Phe
        1475                1480                1485
Pro Leu Leu Glu Glu Leu Ile Leu Asp Lys Leu Pro Lys Leu Gly His
    1490                1495                1500
Phe Phe Leu Thr Lys Arg Ala Leu Glu Phe Pro Phe Leu Arg Glu Val
1505                1510                1515                1520
Lys Ile Arg Lys Cys Pro Glu Met Lys Met Leu Val Gln Gln Arg Ser
                1525                1530                1535
Val Ser Thr Ser Ser Leu Lys Ser Val Asn Asn Asp Asp Glu Leu Lys
            1540                1545                1550
Val Val Asp Leu Asn Lys Ala Met Phe Asn Ser Lys Val Ser Cys Pro
        1555                1560                1565
Asn Leu Lys Val Leu Lys Leu His Glu Ala Asn Asn Ile Thr Ala Leu
    1570                1575                1580
Cys Ser His Gln Leu Pro Thr Thr Tyr Phe Ser Lys Leu Glu Thr Leu
1585                1590                1595                1600
Glu Val Glu Asn Cys Gly Lys Leu Arg His Leu Met Ser Pro Ser Val
                1605                1610                1615
Ala Arg Gly Leu Leu Asn Leu Arg Ile Leu Leu Leu Gly Tyr Cys Glu
            1620                1625                1630
Ser Met Glu Glu Val Ile Ile Glu Glu Glu Gln Glu Gly Glu Glu Asn
        1635                1640                1645
Met Thr Asn Glu Pro Leu Phe Pro Leu Leu Glu Glu Leu Ile Leu Asp
    1650                1655                1660
Lys Leu Pro Lys Leu Gly His Phe Phe Leu Thr Lys Arg Ala Leu Glu
1665                1670                1675                1680
Phe Pro Phe Leu Arg Glu Val Lys Ile Arg Lys Cys Pro Glu Met Lys
                1685                1690                1695
Met Leu Val Gln Gln Arg Ser Val Ser Thr Ser Ser Leu Lys Ser Val
            1700                1705                1710
Asn Asn Asp Asp Glu Leu Lys Val Val Asp Leu Asn Lys Ala Met Phe
        1715                1720                1725
Asn Ser Lys Val Ser Cys Pro Asn Leu Glu Leu Leu Arg Leu Tyr Lys
    1730                1735                1740
Ala Asn Ser Val Gly Ser Leu Phe Ser His Gln Leu Pro Thr Thr Tyr
1745                1750                1755                1760
Phe Ser Lys Leu Glu Thr Leu Glu Val Glu Asn Cys Gly Lys Leu Arg
                1765                1770                1775
His Leu Met Ser Pro Ser Val Ala Arg Gly Leu Leu Asn Leu Arg Ile
            1780                1785                1790
Leu Ile Leu Arg Asp Cys Glu Ser Met Glu Glu Val Ile Thr Glu Glu
        1795                1800                1805
Glu Gln Glu Gly Asp Glu Ile Met Cys Asn Glu Pro Leu Phe Pro Gln
    1810                1815                1820
Leu Glu Glu Leu Lys Leu Glu Asn Leu Pro Lys Leu Arg His Phe Ile
1825                1830                1835                1840
Leu Thr Lys Gln Ala Leu Glu Ile Pro Phe Leu Ile Glu Val Gln Ile
                1845                1850                1855
Arg Asn Cys Pro Glu Met Lys Met Leu Val Gln Gln Arg Ser Val Ser
            1860                1865                1870
Thr Ser Ser Leu Lys Ser Val Asn Asn Asp Asp Glu Leu Lys Val Val
        1875                1880                1885
Asp Leu Asn Lys Ala Met Phe Asn Ser Lys Val Ser Cys Pro Asn Leu
    1890                1895                1900

Glu Leu Leu Arg Leu Tyr Lys Ala Asn Ser Val Gly Ser Leu Phe Ser
1905                1910                1915                1920
His Gln Leu Pro Thr Thr Tyr Phe Ser Lys Leu Met Gln Leu Gly Ile
                1925                1930                1935
Arg Asn Cys Gly Lys Leu Arg His Leu Met Ser Pro Ser Val Ala Arg
            1940                1945                1950
Gly Leu Leu Asn Leu Arg Ile Leu Ile Leu Gly Asp Cys Glu Ser Met
        1955                1960                1965
Glu Glu Val Ile Thr Glu Glu Glu Gln Glu Gly Glu Glu Asn Met Thr
    1970                1975                1980
Asn Glu Ser Leu Phe Pro Cys Leu Glu Met Leu Lys Leu Glu Asn Leu
1985                1990                1995                2000
Pro Lys Leu Gly His Phe Phe Leu Thr Lys Arg Ala Leu Glu Phe Pro
                2005                2010                2015
Phe Leu Arg Glu Val Lys Ile Arg Thr Cys Pro Glu Met Lys Ile Phe
            2020                2025                2030
Val Gln His Gly Ser Val Ser Thr Pro Ser Leu Glu Ile Val Asn Asn
        2035                2040                2045
Asp Asp Glu Val Lys Val Asp Asp Leu Asn Glu Trp Ile His Gln Arg
    2050                2055                2060
Phe Asn Ser Lys Glu Glu Asp Gly Ser Glu Ser Glu Ser Ser Gln Glu
2065                2070                2075                2080
Glu Asp Gly Ser Glu Ser Glu Ala Ser His Glu Asp Gly Ser Lys Cys
                2085                2090                2095
Glu Ala Ser Gln Glu Glu Asp Arg Ser Glu Ser Glu Asp Ser Met Ser
            2100                2105                2110
Asp Gly Arg Ser
        2115

<210> 235
<211> 1404
<212> DNA
<213> Artificial Sequence

<220>
<223> cDNA

<400> 235
atgtcttcaa gtgtttatga gtcgatcatt cagacaagag cttcagtctg gggatcaact      60

gcatctggta aagctgttgt agattcttac tggattcacg aacttggtac tggttctcaa     120

ctagttcaga cccagctgta ttctgattca agaagcaaaa gtagctttgg ctatactgca     180

aaagtagggg atcttccttg tgaagaagaa gagattcttt ctcagcatgt gtatatcccc     240

atttttgatg atattgattt tagcatcaat attgatgact ctgttctggc actatctgtt     300

tgctcaaata cagtcaatgc taacggagtg aaacatcaag gtcacttgaa ggttttgtct     360

cctgctcagc tccactctat tggatctatc atgaacagat ctgatattac agaccgattc     420

cagctccaag aaaaagacat aattcccaat gacagataca ttgaagctgc aaacaaaggc     480

tctttgtctt gtgtcaaaga gcatacctat aagatcgaga tgtgctataa tcaagcttta     540

ggcaaagtga atgttctatc tcctaacaga aatgtccatg aatggctgta cagtttcaag     600

ccaaatttca atcaagttga aagcaacaac agaactgtga attctcttgc agtgaaatct     660

ctgctcatgt cagcagaaaa caacatcatg cctaactctc aagcttttgt caaagcttcc     720

actgattctc atttcaagct gagcctctgg ctaagggttc caaaggtttt gaagcagatt 780

tccattcaga aattgttcaa agttgcagga gatgagacaa ataaaacatt ttatttatct 840

attgcctgca ttccaaacca taacagtgtt gagacagctt taaacattac tgttatttgc 900

aagcatcagc ttccaattcg taaatgtaaa gctccttttg aattatcaat gatgttttct 960

gatttaaagg agccttacaa cattgttcat gatccttcat atccccaaag gattgttcat 1020

gctttgcttg aaactcacac atcttttgca caggttcttt gcaacaactt gcaagaagat 1080

gtgatcatct acaccttgaa caaccatgag ctaacttctg gaaagctaga tttaggtgaa 1140

agaaccttga attacagtga agatgcctac aaaaggaaac atttcctttc aaaaacactt 1200

gaatgtcttc catctaacac acaaactatg tcttatttag acagcatcca aattccttcc 1260

tggaagatag actttgccag gggagaaatt aaaatttctc cgcaatctat ttcagttgca 1320

aaatctttgt taaagcttga tttaagcggg atcaaaaaga aagaatctaa gattgaggaa 1380

gcatatgctt caggatcaaa atga 1404

<210> 236
<211> 467
<212> PRT
<213> Tomato spotted wilt virus

<400> 236
Met Ser Ser Ser Val Tyr Glu Ser Ile Ile Gln Thr Arg Ala Ser Val
1               5                   10                  15
Trp Gly Ser Thr Ala Ser Gly Lys Ala Val Val Asp Ser Tyr Trp Ile
            20                  25                  30
His Glu Leu Gly Thr Gly Ser Gln Leu Val Gln Thr Gln Leu Tyr Ser
        35                  40                  45
Asp Ser Arg Ser Lys Ser Ser Phe Gly Tyr Thr Ala Lys Val Gly Asp
    50                  55                  60
Leu Pro Cys Glu Glu Glu Glu Ile Leu Ser Gln His Val Tyr Ile Pro
65                  70                  75                  80
Ile Phe Asp Asp Ile Asp Phe Ser Ile Asn Ile Asp Asp Ser Val Leu
                85                  90                  95
Ala Leu Ser Val Cys Ser Asn Thr Val Asn Ala Asn Gly Val Lys His
            100                 105                 110
Gln Gly His Leu Lys Val Leu Ser Pro Ala Gln Leu His Ser Ile Gly
        115                 120                 125
Ser Ile Met Asn Arg Ser Asp Ile Thr Asp Arg Phe Gln Leu Gln Glu
    130                 135                 140
Lys Asp Ile Ile Pro Asn Asp Arg Tyr Ile Glu Ala Ala Asn Lys Gly
145                 150                 155                 160
Ser Leu Ser Cys Val Lys Glu His Thr Tyr Lys Ile Glu Met Cys Tyr
                165                 170                 175
Asn Gln Ala Leu Gly Lys Val Asn Val Leu Ser Pro Asn Arg Asn Val
            180                 185                 190
His Glu Trp Leu Tyr Ser Phe Lys Pro Asn Phe Asn Gln Val Glu Ser
        195                 200                 205
Asn Asn Arg Thr Val Asn Ser Leu Ala Val Lys Ser Leu Leu Met Ser
    210                 215                 220
Ala Glu Asn Asn Ile Met Pro Asn Ser Gln Ala Phe Val Lys Ala Ser
225                 230                 235                 240
Thr Asp Ser His Phe Lys Leu Ser Leu Trp Leu Arg Val Pro Lys Val

245                 250                 255
Leu Lys Gln Ile Ser Ile Gln Lys Leu Phe Lys Val Ala Gly Asp Glu
            260                 265                 270
Thr Asn Lys Thr Phe Tyr Leu Ser Ile Ala Cys Ile Pro Asn His Asn
        275                 280                 285
Ser Val Glu Thr Ala Leu Asn Ile Thr Val Ile Cys Lys His Gln Leu
    290                 295                 300
Pro Ile Arg Lys Cys Lys Ala Pro Phe Glu Leu Ser Met Met Phe Ser
305                 310                 315                 320
Asp Leu Lys Glu Pro Tyr Asn Ile Val His Asp Pro Ser Tyr Pro Gln
                325                 330                 335
Arg Ile Val His Ala Leu Leu Glu Thr His Thr Ser Phe Ala Gln Val
            340                 345                 350
Leu Cys Asn Asn Leu Gln Glu Asp Val Ile Ile Tyr Thr Leu Asn Asn
        355                 360                 365
His Glu Leu Thr Ser Gly Lys Leu Asp Leu Gly Glu Arg Thr Leu Asn
    370                 375                 380
Tyr Ser Glu Asp Ala Tyr Lys Arg Lys His Phe Leu Ser Lys Thr Leu
385                 390                 395                 400
Glu Cys Leu Pro Ser Asn Thr Gln Thr Met Ser Tyr Leu Asp Ser Ile
                405                 410                 415
Gln Ile Pro Ser Trp Lys Ile Asp Phe Ala Arg Gly Glu Ile Lys Ile
            420                 425                 430
Ser Pro Gln Ser Ile Ser Val Ala Lys Ser Leu Leu Lys Leu Asp Leu
        435                 440                 445
Ser Gly Ile Lys Lys Lys Glu Ser Lys Ile Glu Glu Ala Tyr Ala Ser
    450                 455                 460
Gly Ser Lys
465

<210> 237
<211> 3741
<212> DNA
<213> Artificial Sequence

<220>
<223> cDNA

<400> 237
atggctgaaa atgaaattga ggaaatgtta gagcacctga gaaggatcaa gagtggaggt      60

gatctggatt ggctcgacat attgcgaatt gaggaacttg aaatggtgct aagagttttt     120

agaaccttta caaagtataa tgatgttctt ttgcctgatt ccttagtcga actcacaaag     180

agggccaaat tgattgggga aatacttcac cggttgtttg gtaggattcc acataaatgt     240

aaaactaacc ttaatctaga aaggctagaa tcacatttgt tggaattctt tcaaggtaat     300

acggcaagtt taagtcacaa ttatgagttg aataattttg atctgtcgaa atatatggat     360

tgtctggaaa attttctaaa tgatgtactg atgatgttct tgcaaaagga taggttcttc     420

cattccagag aacaacttgc aaaacatcga tcaataaagg aactgaaaat tgttcaaaag     480

aaaataagat ttttgaaata catatatgcc acagagataa atggttatgt cgactatgag     540

aagcaggaat gtttggagaa tcgaattcag ttcatgacta acactgtggg acaatattgt     600

ttggcagtat tagattatgt cactgagggt aaacttaatg aagaaaatga caactttagt     660

aaacctcctt acctattatc attgattgtg ttagtggagc tggaaatgaa gaagattttt     720

catggtgaag taaaggcttc aaagtttact caatcaaaaa ctttcaagga caagaaatta 780

ccaaaaggat tttcacatca tctccacaat ctgttgatgt atctcagaaa caaaaagctc 840

gagaattttc ctaataatat cgctgctcaa aatattgatg tggcaataga gttcttgttg 900

gttttccttg atgctgatgt gtcaaatcat gttattaatg gtaactggtt gaaaaaggtc 960

ttgttaaagg ttggagctat agcgggtgat attctatatg taattcaaaa gcttcttcct 1020

agatcaataa acaaagatga aactagcaac ataagtcttt gctcaataca gatattggag 1080

aagactaaag atctgaaggc acaagttgag acgtactaca aatccttaaa atttactcca 1140

tctcagttcc ccacctttgg tggattgagc tttctgaatt ctcttttaag gaaactgaat 1200

gagatgtcga catctaagtc cggattaggt ttcctgatga aacctctttt agggaatttg 1260

gagaaagagc tatcatctct tacatccatt ttagagaagg agctctcatc cattttccgt 1320

gatgtcgtgc accatgaaca taacattcct aaagatcttc agaggcgtac catcaatttg 1380

tcatatgagg ctgaggttgc tattgattct attcttgctc agtataatgc ttttttgcat 1440

attttttgct cacttcctac aattgtaaaa gagatcaagc aaattaatgc agaggtgact 1500

gagatgtggt cagcggacat tcctcttaat cctcactatg tggctgctcc attaaaacat 1560

ctgccggatc gacatagcaa tcttgtaact gatgaggagg tagtgggttt tgagaataaa 1620

gcagaagaac taattgatta tctgattaga ggtacaaatg agctagacgt tgtcccaatt 1680

gtaggcatgg gaggacaagg gaaaacgaca attgctagaa agttgtacaa taatgacatt 1740

attgtttctc gctttgatgt tcgagcatgg tgcatcattt ctcaaacgta taatcggaga 1800

gagttattac aagatatttt cagtcaagtt acaggttccg acgacaatgg agctacggtt 1860

gatgttcttg ccgacatgtt gaggagaaaa ttaatgggaa agagatatct cattgtattg 1920

gatgatatgt gggattgtat ggtatgggat gacttaaggc tttcttttcc agatgatgga 1980

attagaagca gaatagtcgt aacaactcga cttgaagaag tgggtaagca agtcaagtac 2040

catactgatc cttattctct tccattcctc acaacagaag agagttgcca attgttgcag 2100

aaaaaagtgt ttcaaaagga agattgcccg cctgaactac aagatgtgag tcaagcagta 2160

gcagaaaaat gcaaaggact gcccctagtg gttgtcttgg tagctggaat aatcaaaaaa 2220

aggaaaatgg aagaatcttg gtggaatgag gtgaaagatg ctttatttga ctatcttgac 2280

agtgagttcg aagaatacag tctggcaact atgcagttga gttttgataa cttaccccac 2340

tgtttaaagc cttgtcttct ttatatggga atgttttcgg aggacgcaag aattccagca 2400

tctacattga taagtttatg gattgctgaa ggattcgtgg agaacactga atctgggaga 2460

ttaatggaag aggaagctga aggttacttg atggatctca ttagcagtaa cttggtaatg 2520

ctttcaaaga gaacttataa gggtagagtc aaatactgtc aggttcatga tgttgtgcat 2580

cacttttgct tggaaaagag tagagaagca aagtttatgc ttgcagtgaa gggtcaatat 2640

atccattttc aaccttcgga ttggaaggga actcgagtga gcttcagttt tagtgaagag 2700

ctttccaagt ttgcatctct ggtctccaaa acacagaagc ctttccatca acacttgagg 2760

tcattgataa cgaccaatcg agcaaaatct attaatgata ttttctcctg tcagattagt 2820

gaattgcgac ttcttaaagt cttggatttg agttcttata ttgtggagtt tttgtcgtta 2880

gctacattca aaccactaaa tcagctgaag tacctcgcag ttcaggcttt tgaattctat 2940

tttgatccag gatcacatct tccccatata gaaactttca ttgtaatgaa tcttccttat 3000

tatgatatat tgttaccagt gtctttttgg gaaatgaaaa aattaaggca tgctcatttt 3060

ggtaaggctg aatttgacaa gcaggggctc tctgaaggat cctctaaatt ggaaaatttg 3120

aggatattaa agaatattgt tggatttgat agggtggatg tgttatcaag gaggtgtcct 3180

aatcttcaac aacttcaaat cacatatttt gggaataatg aagagccttt ttgtcccaaa 3240

ttggagaatc ttacccagct tcaacaactt caactttcct ttgcgcgtcc ccgcactcta 3300

tccgggttac agttgccttc aaatttaaat aagttggtac ttgaaggaat tcatatagga 3360

tgtgttattc ccttcattgc gggactacca agcctggaat atctccagtt acatgatgtg 3420

tgttttcctc aatcagaaga gtggtgcctt ggagatatca cgttccataa acttaagttg 3480

ttgaaactgg taaagttaaa tatatcaagg tgggatgtct cagaggaatc atttccgttg 3540

cttgaaacac tcgttataaa gaagtgcatt gacctagagg agattccact tagctttgct 3600

gatattccaa cattggaaca gattaaattg attgggtcct ggaaagtatc tctggaggat 3660

tcagctgtga gaatgaagga agaaatcaaa gacactgaag gatgtgatcg tttacacctc 3720

gtcaaacaac gctcagattg a 3741

<210> 238
<211> 1246
<212> PRT
<213> Solanum lycopersicum

<400> 238
Met Ala Glu Asn Glu Ile Glu Glu Met Leu Glu His Leu Arg Arg Ile
1               5                   10                  15
Lys Ser Gly Gly Asp Leu Asp Trp Leu Asp Ile Leu Arg Ile Glu Glu
            20                  25                  30
Leu Glu Met Val Leu Arg Val Phe Arg Thr Phe Thr Lys Tyr Asn Asp
        35                  40                  45
Val Leu Leu Pro Asp Ser Leu Val Glu Leu Thr Lys Arg Ala Lys Leu
    50                  55                  60
Ile Gly Glu Ile Leu His Arg Leu Phe Gly Arg Ile Pro His Lys Cys
65                  70                  75                  80
Lys Thr Asn Leu Asn Leu Glu Arg Leu Glu Ser His Leu Leu Glu Phe
                85                  90                  95
Phe Gln Gly Asn Thr Ala Ser Leu Ser His Asn Tyr Glu Leu Asn Asn
            100                 105                 110
Phe Asp Leu Ser Lys Tyr Met Asp Cys Leu Glu Asn Phe Leu Asn Asp

115                 120                 125
Val Leu Met Met Phe Leu Gln Lys Asp Arg Phe Phe His Ser Arg Glu
    130                 135                 140
Gln Leu Ala Lys His Arg Ser Ile Lys Glu Leu Lys Ile Val Gln Lys
145                 150                 155                 160
Lys Ile Arg Phe Leu Lys Tyr Ile Tyr Ala Thr Glu Ile Asn Gly Tyr
                165                 170                 175
Val Asp Tyr Glu Lys Gln Glu Cys Leu Glu Asn Arg Ile Gln Phe Met
            180                 185                 190
Thr Asn Thr Val Gly Gln Tyr Cys Leu Ala Val Leu Asp Tyr Val Thr
        195                 200                 205
Glu Gly Lys Leu Asn Glu Glu Asn Asp Asn Phe Ser Lys Pro Pro Tyr
    210                 215                 220
Leu Leu Ser Leu Ile Val Leu Val Glu Leu Glu Met Lys Lys Ile Phe
225                 230                 235                 240
His Gly Glu Val Lys Ala Ser Lys Phe Thr Gln Ser Lys Thr Phe Lys
                245                 250                 255
Asp Lys Lys Leu Pro Lys Gly Phe Ser His His Leu His Asn Leu Leu
            260                 265                 270
Met Tyr Leu Arg Asn Lys Lys Leu Glu Asn Phe Pro Asn Asn Ile Ala
        275                 280                 285
Ala Gln Asn Ile Asp Val Ala Ile Glu Phe Leu Leu Val Phe Leu Asp
    290                 295                 300
Ala Asp Val Ser Asn His Val Ile Asn Gly Asn Trp Leu Lys Lys Val
305                 310                 315                 320
Leu Leu Lys Val Gly Ala Ile Ala Gly Asp Ile Leu Tyr Val Ile Gln
                325                 330                 335
Lys Leu Leu Pro Arg Ser Ile Asn Lys Asp Glu Thr Ser Asn Ile Ser
            340                 345                 350
Leu Cys Ser Ile Gln Ile Leu Glu Lys Thr Lys Asp Leu Lys Ala Gln
        355                 360                 365
Val Glu Thr Tyr Tyr Lys Ser Leu Lys Phe Thr Pro Ser Gln Phe Pro
    370                 375                 380
Thr Phe Gly Gly Leu Ser Phe Leu Asn Ser Leu Leu Arg Lys Leu Asn
385                 390                 395                 400
Glu Met Ser Thr Ser Lys Ser Gly Leu Gly Phe Leu Met Lys Pro Leu
                405                 410                 415
Leu Gly Asn Leu Glu Lys Glu Leu Ser Ser Leu Thr Ser Ile Leu Glu
            420                 425                 430
Lys Glu Leu Ser Ser Ile Phe Arg Asp Val Val His His Glu His Asn
        435                 440                 445
Ile Pro Lys Asp Leu Gln Arg Arg Thr Ile Asn Leu Ser Tyr Glu Ala
    450                 455                 460
Glu Val Ala Ile Asp Ser Ile Leu Ala Gln Tyr Asn Ala Phe Leu His
465                 470                 475                 480
Ile Phe Cys Ser Leu Pro Thr Ile Val Lys Glu Ile Lys Gln Ile Asn
                485                 490                 495
Ala Glu Val Thr Glu Met Trp Ser Ala Asp Ile Pro Leu Asn Pro His
            500                 505                 510
Tyr Val Ala Ala Pro Leu Lys His Leu Pro Asp Arg His Ser Asn Leu
        515                 520                 525
Val Thr Asp Glu Glu Val Val Gly Phe Glu Asn Lys Ala Glu Glu Leu
    530                 535                 540
Ile Asp Tyr Leu Ile Arg Gly Thr Asn Glu Leu Asp Val Val Pro Ile
545                 550                 555                 560
Val Gly Met Gly Gly Gln Gly Lys Thr Thr Ile Ala Arg Lys Leu Tyr
                565                 570                 575
Asn Asn Asp Ile Ile Val Ser Arg Phe Asp Val Arg Ala Trp Cys Ile
            580                 585                 590
Ile Ser Gln Thr Tyr Asn Arg Arg Glu Leu Leu Gln Asp Ile Phe Ser
        595                 600                 605
Gln Val Thr Gly Ser Asp Asp Asn Gly Ala Thr Val Asp Val Leu Ala
    610                 615                 620

Asp Met Leu Arg Arg Lys Leu Met Gly Lys Arg Tyr Leu Ile Val Leu
625                 630                 635                 640
Asp Asp Met Trp Asp Cys Met Val Trp Asp Asp Leu Arg Leu Ser Phe
                645                 650                 655
Pro Asp Asp Gly Ile Arg Ser Arg Ile Val Val Thr Thr Arg Leu Glu
            660                 665                 670
Glu Val Gly Lys Gln Val Lys Tyr His Thr Asp Pro Tyr Ser Leu Pro
        675                 680                 685
Phe Leu Thr Thr Glu Glu Ser Cys Gln Leu Leu Gln Lys Lys Val Phe
    690                 695                 700
Gln Lys Glu Asp Cys Pro Pro Glu Leu Gln Asp Val Ser Gln Ala Val
705                 710                 715                 720
Ala Glu Lys Cys Lys Gly Leu Pro Leu Val Val Val Leu Val Ala Gly
                725                 730                 735
Ile Ile Lys Lys Arg Lys Met Glu Glu Ser Trp Trp Asn Glu Val Lys
            740                 745                 750
Asp Ala Leu Phe Asp Tyr Leu Asp Ser Glu Phe Glu Glu Tyr Ser Leu
        755                 760                 765
Ala Thr Met Gln Leu Ser Phe Asp Asn Leu Pro His Cys Leu Lys Pro
    770                 775                 780
Cys Leu Leu Tyr Met Gly Met Phe Ser Glu Asp Ala Arg Ile Pro Ala
785                 790                 795                 800
Ser Thr Leu Ile Ser Leu Trp Ile Ala Glu Gly Phe Val Glu Asn Thr
                805                 810                 815
Glu Ser Gly Arg Leu Met Glu Glu Glu Ala Glu Gly Tyr Leu Met Asp
            820                 825                 830
Leu Ile Ser Ser Asn Leu Val Met Leu Ser Lys Arg Thr Tyr Lys Gly
        835                 840                 845
Arg Val Lys Tyr Cys Gln Val His Asp Val Val His His Phe Cys Leu
    850                 855                 860
Glu Lys Ser Arg Glu Ala Lys Phe Met Leu Ala Val Lys Gly Gln Tyr
865                 870                 875                 880
Ile His Phe Gln Pro Ser Asp Trp Lys Gly Thr Arg Val Ser Phe Ser
                885                 890                 895
Phe Ser Glu Glu Leu Ser Lys Phe Ala Ser Leu Val Ser Lys Thr Gln
            900                 905                 910
Lys Pro Phe His Gln His Leu Arg Ser Leu Ile Thr Thr Asn Arg Ala
        915                 920                 925
Lys Ser Ile Asn Asp Ile Phe Ser Cys Gln Ile Ser Glu Leu Arg Leu
    930                 935                 940
Leu Lys Val Leu Asp Leu Ser Ser Tyr Ile Val Glu Phe Leu Ser Leu
945                 950                 955                 960
Ala Thr Phe Lys Pro Leu Asn Gln Leu Lys Tyr Leu Ala Val Gln Ala
                965                 970                 975
Phe Glu Phe Tyr Phe Asp Pro Gly Ser His Leu Pro His Ile Glu Thr
            980                 985                 990
Phe Ile Val Met Asn Leu Pro Tyr Tyr Asp Ile Leu Leu Pro Val Ser
        995                 1000                1005
Phe Trp Glu Met Lys Lys Leu Arg His Ala His Phe Gly Lys Ala Glu
    1010                1015                1020
Phe Asp Lys Gln Gly Leu Ser Glu Gly Ser Ser Lys Leu Glu Asn Leu
1025                1030                1035                1040
Arg Ile Leu Lys Asn Ile Val Gly Phe Asp Arg Val Asp Val Leu Ser
                1045                1050                1055
Arg Arg Cys Pro Asn Leu Gln Gln Leu Gln Ile Thr Tyr Phe Gly Asn
            1060                1065                1070
Asn Glu Glu Pro Phe Cys Pro Lys Leu Glu Asn Leu Thr Gln Leu Gln
        1075                1080                1085
Gln Leu Gln Leu Ser Phe Ala Arg Pro Arg Thr Leu Ser Gly Leu Gln
    1090                1095                1100
Leu Pro Ser Asn Leu Asn Lys Leu Val Leu Glu Gly Ile His Ile Gly
1105                1110                1115                1120
Cys Val Ile Pro Phe Ile Ala Gly Leu Pro Ser Leu Glu Tyr Leu Gln

                    1125                1130                1135
Leu His Asp Val Cys Phe Pro Gln Ser Glu Glu Trp Cys Leu Gly Asp
            1140                1145                1150
Ile Thr Phe His Lys Leu Lys Leu Leu Lys Leu Val Lys Leu Asn Ile
        1155                1160                1165
Ser Arg Trp Asp Val Ser Glu Glu Ser Phe Pro Leu Leu Glu Thr Leu
    1170                1175                1180
Val Ile Lys Lys Cys Ile Asp Leu Glu Glu Ile Pro Leu Ser Phe Ala
1185                1190                1195                1200
Asp Ile Pro Thr Leu Glu Gln Ile Lys Leu Ile Gly Ser Trp Lys Val
                1205                1210                1215
Ser Leu Glu Asp Ser Ala Val Arg Met Lys Glu Glu Ile Lys Asp Thr
            1220                1225                1230
Glu Gly Cys Asp Arg Leu His Leu Val Lys Gln Arg Ser Asp
        1235                1240                1245

<210> 239
<211> 909
<212> DNA
<213> Artificial Sequence


<220>
<223> cDNA

<400> 239
atgttgactt ttttcagcaa taaggggtct tctaagtctg ccaaaaagga tgaaggtcct      60

ttagtttcac tagctaaaca taatggtaat gtagaagtct caaaaccatg gtcttcttct     120

gatgaaaagc ttgctttgac taaagctatg gatacatcca aaggaaagat actgttgaac     180

acagagggaa catcttcctt tggaacctat gaatctgatt ctatcacaga atcagagggt     240

tatgatcttt ctgcaagaat gatagtagat acaaaccatc atatctcaaa ctggaaaaat     300

gatctttttg ttggcaatgg gaagcagaac gctaataagg tcatcaagat ctgtccaact     360

tgggacagca gaaaacaata catgatggtt tccaggattg tgatatgggt ctgccccact     420

ataccaaacc ctacagggaa acttgtggtt gccctagttg atcccaacat gccatctgaa     480

aagcaagtca ttctgaaggg tcaaggacaa ataactgatc ctatatgttt tgtcttttat     540

ctgaactggt ctattccaaa aataaacaac actccagaaa actgctgtca gctgcatttg     600

atgtgcagcc aagaatacaa gaaaggggtt tcttttggta gtgtcatgta ttcttggaca     660

aaggagtttt gtgattcacc cagagctgat aaagataaaa gttgtgtggt tatacctcta     720

aacagagcta ttagagctag atctcaagca ttcattgagg cttgcaagct gataattcct     780

aaaggaaaca gtgagaagca gatcaaaaaa cagcttaaag agttgagctc aaatcttgag     840

agatcagttg aagaagaaga ggaagggatt tctgatagtg ttgctcagtt atcttttgat     900

gaaatatag                                                             909

<210> 240
<211> 302
<212> PRT
<213> Tomato spotted wilt virus

<400> 240
Met Leu Thr Phe Phe Ser Asn Lys Gly Ser Ser Lys Ser Ala Lys Lys
1               5                   10                  15
Asp Glu Gly Pro Leu Val Ser Leu Ala Lys His Asn Gly Asn Val Glu
            20                  25                  30
Val Ser Lys Pro Trp Ser Ser Ser Asp Glu Lys Leu Ala Leu Thr Lys
        35                  40                  45
Ala Met Asp Thr Ser Lys Gly Lys Ile Leu Leu Asn Thr Glu Gly Thr
    50                  55                  60
Ser Ser Phe Gly Thr Tyr Glu Ser Asp Ser Ile Thr Glu Ser Glu Gly
65                  70                  75                  80
Tyr Asp Leu Ser Ala Arg Met Ile Val Asp Thr Asn His His Ile Ser
                85                  90                  95
Asn Trp Lys Asn Asp Leu Phe Val Gly Asn Gly Lys Gln Asn Ala Asn
            100                 105                 110
Lys Val Ile Lys Ile Cys Pro Thr Trp Asp Ser Arg Lys Gln Tyr Met
        115                 120                 125
Met Val Ser Arg Ile Val Ile Trp Val Cys Pro Thr Ile Pro Asn Pro
    130                 135                 140
Thr Gly Lys Leu Val Val Ala Leu Val Asp Pro Asn Met Pro Ser Glu
145                 150                 155                 160
Lys Gln Val Ile Leu Lys Gly Gln Gly Thr Ile Thr Asp Pro Ile Cys
                165                 170                 175
Phe Val Phe Tyr Leu Asn Trp Ser Ile Pro Lys Ile Asn Asn Thr Pro
            180                 185                 190
Glu Asn Cys Cys Gln Leu His Leu Met Cys Ser Gln Glu Tyr Lys Lys
        195                 200                 205
Gly Val Ser Phe Gly Ser Val Met Tyr Ser Trp Thr Lys Glu Phe Cys
    210                 215                 220
Asp Ser Pro Arg Ala Asp Lys Asp Lys Ser Cys Val Val Ile Pro Leu
225                 230                 235                 240
Asn Arg Ala Ile Arg Ala Arg Ser Gln Ala Phe Ile Glu Ala Cys Lys
                245                 250                 255
Leu Ile Ile Pro Lys Gly Asn Ser Glu Lys Gln Ile Lys Lys Gln Leu
            260                 265                 270
Lys Glu Leu Ser Ser Asn Leu Glu Arg Ser Val Glu Glu Glu Glu Glu
        275                 280                 285
Gly Ile Ser Asp Ser Val Ala Gln Leu Ser Phe Asp Glu Ile
    290                 295                 300

<210> 241
<211> 3390
<212> DNA
<213> Artificial Sequence

<220>
<223> cDNA

<400> 241
atggcactag aattggttgg tggtgctctt ctttctgctt tccttcaggt tgcgtttgag      60

aagcttgctt ctcctcaagt tctggacttc tttcgtggaa gaaagcttga tgaaaagctg     120

ctcaacaact tggaaatcaa gctgaactcc atacaggctc tggctgatga tgcagaacta     180

aagcagttca gagatccacc tgtcaggaac tggcttctta aggtcaaaga tgctttgttt     240

gacgcagagg atctcttgga tgaaatacaa catgagatct ccaaatgcca ggtcgaagct     300

gaagctgaag ctgagtctca aacctgcact tgcaaggtac caaatttctt caaatcttct     360

cctgttggtt cctttaacaa ggaaattaaa tcaaggatgg aacaggtcct tgaggacctt 420

gaaaatcttg ctagccaaag tggctatcta ggtttgcaaa atgcaagtgg tgttggatca 480

ggatttggtg gtgcagtatc actgcattca gaatcaacat ctttggttgt tgaaagtgtt 540

atttatggca gagatgatga caaagaaatg atctttaatt ggctcacatc tgacattgac 600

aattgtaaca aactatcaat actttctatt gtgggcatgg gtgggttggg taagaccaca 660

cttgcccaac atgtattcaa tgacccaagg atagaaaata aatttgatat caaagcttgg 720

gtctgtgttt cagatgaatt tgatgttttc aatgtaacaa gaacaattct ggaggcagtc 780

actaaatcaa ctgatgatag tagaaaccga gaaacggttc agggaaggtt gagagaaaaa 840

ttgacaggaa ataaattttt tcttgttttg gatgatgttt ggaacagaaa tcaaaaggaa 900

tggaaagatt tgcaaactcc tcttaattat ggggcttcag ggagtaaaat tgttgtcact 960

acacgtgaca agaaagttgc ttcaatagtg ggatcaaata aaacacattg cttagagctg 1020

ttgcaagatg atcattgctg gcgattgttc actaaacatg cattccgaga tgatagtcat 1080

caaccaaatc cagacttcaa ggagattggt acgaagatag tagaaaaatg taaaggactg 1140

cctctggcct tgacaacaat tggaagtcta ttacaccaaa aatcatctat ttcagaatgg 1200

gaaggcatat tgaaaagcga gatatgggaa ttttcagaag aggatagctc tattgtccct 1260

gctttagcac taagctatca ccaccttcct tcccatctca agagatgttt tgcttattgc 1320

gccttattcc ccaaagatta caggtttgac aaggagggtt taattcagct gtggatggcg 1380

gaaaactttt tacaatgtca tcaacagagt aggagtccag aaaaagtcgg tgaacaatac 1440

ttcaacgatc ttttgtcaag gtccttattt caacaatcaa gcaccgtaga gagaacacct 1500

tttgtcatgc atgaccttct caatgatttg gcaaaatatg tttgtgggga catctgcttc 1560

aggttggaaa atgatcaagc aacaaatata ccgaagacaa cccgtcattt ttcagttgca 1620

agcgatcacg ttacatgctt tgatgggttt cgaactttat acaatgctga aaggttacgc 1680

acatttatgt cattaagtga ggaaatgagt tttcgtaact acaatccttg gtattgcaag 1740

atgtcaacac gtgaattatt ttccaagttt aagttcttac gtgtcttatc tctgtctggt 1800

tattataacc taacaaaggt gcctaactct gtgggtaatc ttaaatatct cagttcgtta 1860

gacttatccc atactgaaat agtaaaacta cctgaatcaa tatgttcact ctacaacttg 1920

caaatactga agctaaatgg ttgtgaacat ttgaaggagc tgccctcaaa tttgcataaa 1980

ctcactgatt tgcatcgcct tgaattaata gatactgaag tgagaaaggt accagcacat 2040

ttgggaaaac tgaagtatct tcaagtatta atgagttcgt ttaatgttgg caaaagtagg 2100

gagttcagta tccagcagct aggagaactc aatcttcatg gaagtctatc aataaggcaa 2160

ctgcagaatg ttgagaatcc atcagatgca ttagcggtgg atttaaaaaa taaaacacac 2220

cttgtcgagc tagagttaga atgggattca gactggaacc ctgatgattc aacaaaagaa 2280

agggatgtaa tcgagaatct acagccttcc aaacacttgg agaagttgac gatgagcaac 2340

tatgggggta aacaatttcc aaggtggtta ttcaacaatt cattattgcg tgtggtatcc 2400

ttaaccttga agaactgtaa aggtttccta tgtttgcctc cccttggacg tttgccatct 2460

ctgaaggagc tttcaattga agggcttgat gggattgtga gtattaatgc tgattttttc 2520

gggagtagct cttgttcatt tacatccttg gaatctttgg agttctccga tatgaaggaa 2580

tgggaagaat gggaatgtaa aggtgtgaca ggtgcttttc cacgtcttca acgtctttct 2640

ataatgcgtt gtcccaagct gaaagggcac ttgccagagc aactatgtca tttaaattat 2700

ctaaagattt ctgggtggga ctctctaacg actattccgc tagatatctt cccaatactc 2760

aaggagcttc agatctggga gtgtcctaat ctacagagga tttcacaggg gcaggctctt 2820

aatcatctcg agactctgag tatgagagag tgcccccaat tagaatcatt gcctgaagga 2880

atgcatgtcc tccttccatc tcttgattct ctgtggatag atgattgtcc aaaagttgaa 2940

atgtttcccg aaggaggttt gccatcaaat ttaaaaagta tgggtctcta tggtggtagt 3000

tacaaactta tctccttatt gaaaagtgcc ttgggaggca atcactctct agaaaggtta 3060

gttattggag gagtggatgt tgagtgtctt cctgacgaag gtgtactgcc acactctctt 3120

gttaatctat ggataaggga gtgtggagat ctaaaaagac tggactacaa aggtctctgc 3180

cacctctcct ctctcaagac attgactctt tgggactgcc ccaggctcca atgcttacca 3240

gaggagggtc tgcccaaatc catttcaact ttgggaattc tcaattgtcc gttgctcaaa 3300

caacgttgcc gggaacccga aggcgaagac tggccaaaga ttgctcacat taaacgcgtg 3360

tggttattag gtaatgatgt tgatgtttaa 3390

<210> 242
<211> 1129
<212> PRT
<213> Glycine max

<400> 242
Met Ala Leu Glu Leu Val Gly Gly Ala Leu Leu Ser Ala Phe Leu Gln
1               5                   10                  15
Val Ala Phe Glu Lys Leu Ala Ser Pro Gln Val Leu Asp Phe Phe Arg
            20                  25                  30
Gly Arg Lys Leu Asp Glu Lys Leu Leu Asn Asn Leu Glu Ile Lys Leu
        35                  40                  45
Asn Ser Ile Gln Ala Leu Ala Asp Asp Ala Glu Leu Lys Gln Phe Arg
    50                  55                  60
Asp Pro Pro Val Arg Asn Trp Leu Leu Lys Val Lys Asp Ala Leu Phe
65                  70                  75                  80
Asp Ala Glu Asp Leu Leu Asp Glu Ile Gln His Glu Ile Ser Lys Cys
                85                  90                  95
Gln Val Glu Ala Glu Ala Glu Ala Glu Ser Gln Thr Cys Thr Cys Lys
            100                 105                 110
Val Pro Asn Phe Phe Lys Ser Ser Pro Val Gly Ser Phe Asn Lys Glu

115                 120                 125
Ile Lys Ser Arg Met Glu Gln Val Leu Glu Asp Leu Glu Asn Leu Ala
    130                 135                 140
Ser Gln Ser Gly Tyr Leu Gly Leu Gln Asn Ala Ser Gly Val Gly Ser
145                 150                 155                 160
Gly Phe Gly Gly Ala Val Ser Leu His Ser Glu Ser Thr Ser Leu Val
                165                 170                 175
Val Glu Ser Val Ile Tyr Gly Arg Asp Asp Asp Lys Glu Met Ile Phe
            180                 185                 190
Asn Trp Leu Thr Ser Asp Ile Asp Asn Cys Asn Lys Leu Ser Ile Leu
        195                 200                 205
Ser Ile Val Gly Met Gly Gly Leu Gly Lys Thr Thr Leu Ala Gln His
    210                 215                 220
Val Phe Asn Asp Pro Arg Ile Glu Asn Lys Phe Asp Ile Lys Ala Trp
225                 230                 235                 240
Val Cys Val Ser Asp Glu Phe Asp Val Phe Asn Val Thr Arg Thr Ile
                245                 250                 255
Leu Glu Ala Val Thr Lys Ser Thr Asp Asp Ser Arg Asn Arg Glu Thr
            260                 265                 270
Val Gln Gly Arg Leu Arg Glu Lys Leu Thr Gly Asn Lys Phe Phe Leu
        275                 280                 285
Val Leu Asp Asp Val Trp Asn Arg Asn Gln Lys Glu Trp Lys Asp Leu
    290                 295                 300
Gln Thr Pro Leu Asn Tyr Gly Ala Ser Gly Ser Lys Ile Val Val Thr
305                 310                 315                 320
Thr Arg Asp Lys Lys Val Ala Ser Ile Val Gly Ser Asn Lys Thr His
                325                 330                 335
Cys Leu Glu Leu Leu Gln Asp Asp His Cys Trp Arg Leu Phe Thr Lys
            340                 345                 350
His Ala Phe Arg Asp Asp Ser His Gln Pro Asn Pro Asp Phe Lys Glu
        355                 360                 365
Ile Gly Thr Lys Ile Val Glu Lys Cys Lys Gly Leu Pro Leu Ala Leu
    370                 375                 380
Thr Thr Ile Gly Ser Leu Leu His Gln Lys Ser Ser Ile Ser Glu Trp
385                 390                 395                 400
Glu Gly Ile Leu Lys Ser Glu Ile Trp Glu Phe Ser Glu Glu Asp Ser
                405                 410                 415
Ser Ile Val Pro Ala Leu Ala Leu Ser Tyr His His Leu Pro Ser His
            420                 425                 430
Leu Lys Arg Cys Phe Ala Tyr Cys Ala Leu Phe Pro Lys Asp Tyr Arg
        435                 440                 445
Phe Asp Lys Glu Gly Leu Ile Gln Leu Trp Met Ala Glu Asn Phe Leu
    450                 455                 460
Gln Cys His Gln Gln Ser Arg Ser Pro Glu Lys Val Gly Glu Gln Tyr
465                 470                 475                 480
Phe Asn Asp Leu Leu Ser Arg Ser Leu Phe Gln Gln Ser Ser Thr Val
                485                 490                 495
Glu Arg Thr Pro Phe Val Met His Asp Leu Leu Asn Asp Leu Ala Lys
            500                 505                 510
Tyr Val Cys Gly Asp Ile Cys Phe Arg Leu Glu Asn Asp Gln Ala Thr
        515                 520                 525
Asn Ile Pro Lys Thr Thr Arg His Phe Ser Val Ala Ser Asp His Val
    530                 535                 540
Thr Cys Phe Asp Gly Phe Arg Thr Leu Tyr Asn Ala Glu Arg Leu Arg
545                 550                 555                 560
Thr Phe Met Ser Leu Ser Glu Glu Met Ser Phe Arg Asn Tyr Asn Pro
                565                 570                 575
Trp Tyr Cys Lys Met Ser Thr Arg Glu Leu Phe Ser Lys Phe Lys Phe
            580                 585                 590
Leu Arg Val Leu Ser Leu Ser Gly Tyr Tyr Asn Leu Thr Lys Val Pro
        595                 600                 605
Asn Ser Val Gly Asn Leu Lys Tyr Leu Ser Ser Leu Asp Leu Ser His
    610                 615                 620

Thr Glu Ile Val Lys Leu Pro Glu Ser Ile Cys Ser Leu Tyr Asn Leu
625                 630                 635                 640
Gln Ile Leu Lys Leu Asn Gly Cys Glu His Leu Lys Glu Leu Pro Ser
                645                 650                 655
Asn Leu His Lys Leu Thr Asp Leu His Arg Leu Glu Leu Ile Asp Thr
            660                 665                 670
Glu Val Arg Lys Val Pro Ala His Leu Gly Lys Leu Lys Tyr Leu Gln
        675                 680                 685
Val Leu Met Ser Ser Phe Asn Val Gly Lys Ser Arg Glu Phe Ser Ile
    690                 695                 700
Gln Gln Leu Gly Glu Leu Asn Leu His Gly Ser Leu Ser Ile Arg Gln
705                 710                 715                 720
Leu Gln Asn Val Glu Asn Pro Ser Asp Ala Leu Ala Val Asp Leu Lys
                725                 730                 735
Asn Lys Thr His Leu Val Glu Leu Glu Leu Glu Trp Asp Ser Asp Trp
            740                 745                 750
Asn Pro Asp Asp Ser Thr Lys Glu Arg Asp Val Ile Glu Asn Leu Gln
        755                 760                 765
Pro Ser Lys His Leu Glu Lys Leu Thr Met Ser Asn Tyr Gly Gly Lys
    770                 775                 780
Gln Phe Pro Arg Trp Leu Phe Asn Asn Ser Leu Leu Arg Val Val Ser
785                 790                 795                 800
Leu Thr Leu Lys Asn Cys Lys Gly Phe Leu Cys Leu Pro Pro Leu Gly
                805                 810                 815
Arg Leu Pro Ser Leu Lys Glu Leu Ser Ile Glu Gly Leu Asp Gly Ile
            820                 825                 830
Val Ser Ile Asn Ala Asp Phe Phe Gly Ser Ser Ser Cys Ser Phe Thr
        835                 840                 845
Ser Leu Glu Ser Leu Glu Phe Ser Asp Met Lys Glu Trp Glu Glu Trp
    850                 855                 860
Glu Cys Lys Gly Val Thr Gly Ala Phe Pro Arg Leu Gln Arg Leu Ser
865                 870                 875                 880
Ile Met Arg Cys Pro Lys Leu Lys Gly His Leu Pro Glu Gln Leu Cys
                885                 890                 895
His Leu Asn Tyr Leu Lys Ile Ser Gly Trp Asp Ser Leu Thr Thr Ile
            900                 905                 910
Pro Leu Asp Ile Phe Pro Ile Leu Lys Glu Leu Gln Ile Trp Glu Cys
        915                 920                 925
Pro Asn Leu Gln Arg Ile Ser Gln Gly Gln Ala Leu Asn His Leu Glu
    930                 935                 940
Thr Leu Ser Met Arg Glu Cys Pro Gln Leu Glu Ser Leu Pro Glu Gly
945                 950                 955                 960
Met His Val Leu Leu Pro Ser Leu Asp Ser Leu Trp Ile Asp Asp Cys
                965                 970                 975
Pro Lys Val Glu Met Phe Pro Glu Gly Gly Leu Pro Ser Asn Leu Lys
            980                 985                 990
Ser Met Gly Leu Tyr Gly Gly Ser Tyr Lys Leu Ile Ser Leu Leu Lys
        995                 1000                1005
Ser Ala Leu Gly Gly Asn His Ser Leu Glu Arg Leu Val Ile Gly Gly
    1010                1015                1020
Val Asp Val Glu Cys Leu Pro Asp Glu Gly Val Leu Pro His Ser Leu
1025                1030                1035                1040
Val Asn Leu Trp Ile Arg Glu Cys Gly Asp Leu Lys Arg Leu Asp Tyr
                1045                1050                1055
Lys Gly Leu Cys His Leu Ser Ser Leu Lys Thr Leu Thr Leu Trp Asp
            1060                1065                1070
Cys Pro Arg Leu Gln Cys Leu Pro Glu Glu Gly Leu Pro Lys Ser Ile
        1075                1080                1085
Ser Thr Leu Gly Ile Leu Asn Cys Pro Leu Leu Lys Gln Arg Cys Arg
    1090                1095                1100
Glu Pro Glu Gly Glu Asp Trp Pro Lys Ile Ala His Ile Lys Arg Val
1105                1110                1115                1120
Trp Leu Leu Gly Asn Asp Val Asp Val

                1125

<210> 243
<211> 1197
<212> DNA
<213> Artificial Sequence


<220>
<223> cDNA

<400> 243
ggtgaagtgc aacagagaat gaagtgtgaa acagcactta taacaagcat tttcaaacct 60

aagagaatga ttcaaatcct tgaaaatgac ccatacattc tcttgatggg cttggtttca 120

ccttctatct tgattcacat gtatcgtatg aagcattttg agaaaggggt ggagttgtgg 180

ataagtaaag aacatagtgt ggcaaagatt ttcatcatat tggaacaact cactaagagg 240

gtcgctgcaa atgatgtgct acttgagcaa ctcgaaatga tctcagaaac ttctgagagg 300

ttcatgagta tcttagagga ttgccctcaa gcaccacatt catacaagac agcaaaagat 360

ttgttgacaa tgtacataga aagaaaagca tccaacaacc aattggtgga gaatggtttt 420

gtagacatga atgacaaatt gtacatggca tatgaaaaaa tctactcaga tcgcttgaag 480

caggaatggc gcgcattaag ctggttggaa aaattttcta taacatggca attgaaaaga 540

tttgctccac atacggagaa atgtttgaca aagaaagttg tagaagaaag cagcgcatct 600

tcaggaaact ttgcgagtgt gtgcttcatg aatgcccagt cacacctaag aaatgtaaga 660

aatacacttt tccaaaaatg tgaccaggtt tggactgcat cggtgcgagc ctttgtgaag 720

ctcataattt caacacttca caggtgctac agtgatatag tttatctggt aaacatctgt 780

ataatctttt cattgcttgt ccaaatgact agtgtactgc agggcattgt caacacagta 840

aggagagaca aagcactctt aagtggatgg aaaaggaaag aagatgaaga ggccgtgatt 900

catttgtatg aaatgtgtga aaagatggaa ggtggacatc caagtattga gaaatttttg 960

gaccatgtca agggagttag acctgatcta ctccctgtgg cagtaagcat gacaggacaa 1020

tcagaagatg tctccgcaca ggccaaaaca gcaactcaat tgcaacttga gaaaattgtg 1080

gcattcatgg ctttgttgac catgtgtatt gataatgaaa ggagtgatgc ggttttcaaa 1140

gtattgagca agttaaaggc atttttcagc acaatgggtg aggatgttaa agtgcag 1197


<210> 244
<211> 399
<212> PRT
<213> Soybean mosaic virus


<400> 244
Gly Glu Val Gln Gln Arg Met Lys Cys Glu Thr Ala Leu Ile Thr Ser
1               5                   10                  15
Ile Phe Lys Pro Lys Arg Met Ile Gln Ile Leu Glu Asn Asp Pro Tyr

20                  25                  30
Ile Leu Leu Met Gly Leu Val Ser Pro Ser Ile Leu Ile His Met Tyr
        35                  40                  45
Arg Met Lys His Phe Glu Lys Gly Val Glu Leu Trp Ile Ser Lys Glu
    50                  55                  60
His Ser Val Ala Lys Ile Phe Ile Ile Leu Glu Gln Leu Thr Lys Arg
65                  70                  75                  80
Val Ala Ala Asn Asp Val Leu Leu Glu Gln Leu Glu Met Ile Ser Glu
                85                  90                  95
Thr Ser Glu Arg Phe Met Ser Ile Leu Glu Asp Cys Pro Gln Ala Pro
            100                 105                 110
His Ser Tyr Lys Thr Ala Lys Asp Leu Leu Thr Met Tyr Ile Glu Arg
        115                 120                 125
Lys Ala Ser Asn Asn Gln Leu Val Glu Asn Gly Phe Val Asp Met Asn
    130                 135                 140
Asp Lys Leu Tyr Met Ala Tyr Glu Lys Ile Tyr Ser Asp Arg Leu Lys
145                 150                 155                 160
Gln Glu Trp Arg Ala Leu Ser Trp Leu Glu Lys Phe Ser Ile Thr Trp
                165                 170                 175
Gln Leu Lys Arg Phe Ala Pro His Thr Glu Lys Cys Leu Thr Lys Lys
            180                 185                 190
Val Val Glu Glu Ser Ser Ala Ser Ser Gly Asn Phe Ala Ser Val Cys
        195                 200                 205
Phe Met Asn Ala Gln Ser His Leu Arg Asn Val Arg Asn Thr Leu Phe
    210                 215                 220
Gln Lys Cys Asp Gln Val Trp Thr Ala Ser Val Arg Ala Phe Val Lys
225                 230                 235                 240
Leu Ile Ile Ser Thr Leu His Arg Cys Tyr Ser Asp Ile Val Tyr Leu
                245                 250                 255
Val Asn Ile Cys Ile Ile Phe Ser Leu Leu Val Gln Met Thr Ser Val
            260                 265                 270
Leu Gln Gly Ile Val Asn Thr Val Arg Arg Asp Lys Ala Leu Leu Ser
        275                 280                 285
Gly Trp Lys Arg Lys Glu Asp Glu Glu Ala Val Ile His Leu Tyr Glu
    290                 295                 300
Met Cys Glu Lys Met Glu Gly Gly His Pro Ser Ile Glu Lys Phe Leu
305                 310                 315                 320
Asp His Val Lys Gly Val Arg Pro Asp Leu Leu Pro Val Ala Val Ser
                325                 330                 335
Met Thr Gly Gln Ser Glu Asp Val Ser Ala Gln Ala Lys Thr Ala Thr
            340                 345                 350
Gln Leu Gln Leu Glu Lys Ile Val Ala Phe Met Ala Leu Leu Thr Met
        355                 360                 365
Cys Ile Asp Asn Glu Arg Ser Asp Ala Val Phe Lys Val Leu Ser Lys
    370                 375                 380
Leu Lys Ala Phe Phe Ser Thr Met Gly Glu Asp Val Lys Val Gln
385                 390                 395

<210> 245
<211> 3834
<212> DNA
<213> Artificial Sequence

<220>
<223> cDNA

<400> 245
atggctgcct ctgccgcact cgtttttgca gggaaatctg tggcgacttc ggccatatcc      60

ttcttggtca acaaggcttt cagctacatc gatgagtact gcaagtccga aaacatggat     120

gagttgaaga atcggctcct gcgggccatg ccacatatcc aggcggtgtt cgatgtcgtc     180

aacccggaac ttgtcaggga gcagagcagc ggcctcgacg cgtggctctg gcagctcagg 240

gacgcggtcg aggcggcgga ggacgccatc gatgagcttg aatactacga gctcgaggag 300

aaggccaagg accaaaaggt tagtgaatgg ggttcccctc ttggtaagat gaagcacaag 360

tttgtcagat cttttggccc tgctatcaac aagactatca agaaaattag tcaccgtgac 420

actctgaaga ggttgatgaa atctgtggat gatttagaca aggctgccgt aggtgttagt 480

gatttcctca agctcacaga ccatctcagt ggaggttctt ctaccagcag ccagcagctc 540

atggacaatg atcgccagac tagttcaaca tcaagtgcaa ccaattttgt tggacgagaa 600

aaggagaaag aacagattat tgggtggcta gccaacacct cagatgaatt gggtgaaact 660

atggtcataa agaccaatag tattccaatt atttcagtta ttggtcatgg tggcatgggg 720

aagactgcct tggctcagag catatgtggt caagatgagg tagtaaagca ttttaaggtc 780

atctggacca ccatatctac tagctttgat gcgacatcgg tgacaagtaa aatactggaa 840

gacgctacgc gcgtgaaacc gagcagcgat catttggaac cactacaacg ggacctcaaa 900

gagaaactcg catccattaa ttttttgtta gttctagatg acgtttggga agataagaag 960

agagatgaat gggagaagtt atttgctcct ctgaaggaac tgaacactac tgggagcaaa 1020

attctgttga caacccgaat gcaatctgta gcagacatgg ctgcaaaggt gatgggagtc 1080

gaaagggata agttcttgac attacaagga ctggaagatg ataaaaatct caagctcttc 1140

ggtcatcatg ccttttccgg tttgaatcca agagatgatg catatttgat gttaattgga 1200

gaacaaattg caaagaaact gaggggatgt cccctggtaa cgaaggttgt cgccgagcac 1260

ctgcaaagca atataacacc tgaatactgg aggaaattct tgcatcaagg attggaacat 1320

tttaaaggaa ccgaaaaaga tattatgaag gttctcagat tgagctatta ccacttacca 1380

acagagctac agatttgctt tcggtactgt tgcctatttc cgcaggacta tatatttgaa 1440

aagaaagagt tggtgcaaat gtggattggt tcaggattga ttgcaagtgg cattcaatct 1500

ttggaggata ctgcagaaca gttcttggct cagctaacta gaaagtcgtt ctttgataag 1560

aaacctatat gggcacaaag tgaattttat gtaatgcatg acctgatgca tgaattagca 1620

agcaatgtgt ccaccggtga atgtgcaaga atagttgacc ctgttcaact tcaagatgag 1680

aagtatacag tccgacacct atgcattgcc aatattcaca gtttctctgc tgatgaggtc 1740

aagaaaatct cccattttaa gaatctgcgc actattatca ttgattgtat gccgccactt 1800

gaaaatgata ctctttgcgc acttcaaatg atagttgaga cctcaaaatc actgcgacta 1860

ttccactctg cacaattatg gaacacatcc cgttttgctg tcaattttgg taacttaaaa 1920

catcttcgct atatctgcgt gagttcgata ccacaagaca agatatgtgg ggtcgcaaga 1980

ctttatcact tgatggtcct tcaccatggt tcttctggga ccaaaataga tgaagcaaga 2040

tatttaggga accttgaacg tttgcggtat gtatcctatg gtgtgcatgg atttggtaat 2100

ttccccataa gcaggctcac ttctcttcag gaattacata attaccgggt agaagaaaga 2160

acatgcaacc aaataagtgc agttgggagc ttaagagatc ttcgtagatt gggtctgcag 2220

ggtcttgaga atgtcaagaa ttgggaagaa gctaagaatg ccaagttgaa ggaaaaacaa 2280

tatctcaact cattatttat ggagtggtcg acacctgatc aaatcatgac agataactta 2340

gttcttgacc accttgagcc acatgttaat atcagagaat tgcaaattgg gggttatcca 2400

ggtctcaaaa ttccatcttg gatcgagaac tgctcagtca gaaacttgat atcactcaag 2460

ttgataagct gcataaattg ggactacatt ccctctctct gccagttggt attgctggag 2520

cttttagtgt tggacaatct tcctaagcta cggcagattg gtcgatcatc tgatatgtcc 2580

aagagtagtt ccatggagtt gttgttacct caaagcctta atactttgga agtaagcaaa 2640

tgccagaaac tgagggaatt accacttctg cctccaagcc taatatcatt gaaaatacac 2700

gatgtttggc tgaccaagtt tcctattatt ggcaaggtat cgagtgagag catcgagtca 2760

aaatcatcta agttgattaa cgtaagggtc accaactgtc ctcatttaac ttcgttggaa 2820

gggagccttt tggagcaaaa actgtacatg ggaactcttc gtgtcctaac aattgttgat 2880

tgtaaacatc tggagtccgc atccatacca tttgaagaaa tgaaacaact taaggaactt 2940

gcagtaatgc aatgtccaaa gttgaggacg atgagagatg ccaaagataa gcttgtgcca 3000

tcgtcactaa gagaactagc tattggcggg tgtggtgacc tggaacttcc actacttggg 3060

tcactgcagc tgctcaccaa cctatctggg ctcgagctgc agaactgctc gagcctgtta 3120

tctctcccct ccgcggatgt gttcaagagt ttcaggtcgc tgcagaacat acatgtaacg 3180

cgatgcgaga atctctcgtc cctgggaggg ctcggatcac tgtcatccct tcagcagtta 3240

gctatctcca agtgcagtaa actcacggag gctgggtctt ccttgctatc tggttctggt 3300

gatgcagaag agcatctggt ggagcccggc aactcactgc ggataagtgt tcttagctta 3360

gatctgccgt ccttgctgct tcatgagcct ctcaagagtc tgtgccacac tgaatattta 3420

tgcattaatg gctgttcaga gacgaagagc ttacctgaac gatggctgct acagaaccgc 3480

tcatccctcc gacacctggg tatactgaaa gcagaatcct tggaatcgct cccaccgagc 3540

atgcaagacc tttgctctct caagtcatta taccttgatg gtgctctgct actccgatca 3600

cttccatatc ttccctcctc cttgaagaat ctgaacatta cggaatgcca tccagacctg 3660

gagaagaaga tcacaaaaca tggaagctct gaattgaaca agattgccca catccctgat 3720

gtgacaatag gaggttggca tttccttatg ggcaagaaat tcagcagaga ggcttaccat 3780

aaactgagtg atagtgaccg caacagattt atgtccgagt cgtcgttgca ctag 3834

<210> 246
<211> 1277

<212> PRT
<213> Triticum aestivum

<400> 246
Met Ala Ala Ser Ala Ala Leu Val Phe Ala Gly Lys Ser Val Ala Thr
1               5                   10                  15
Ser Ala Ile Ser Phe Leu Val Asn Lys Ala Phe Ser Tyr Ile Asp Glu
            20                  25                  30
Tyr Cys Lys Ser Glu Asn Met Asp Glu Leu Lys Asn Arg Leu Leu Arg
        35                  40                  45
Ala Met Pro His Ile Gln Ala Val Phe Asp Val Val Asn Pro Glu Leu
    50                  55                  60
Val Arg Glu Gln Ser Ser Gly Leu Asp Ala Trp Leu Trp Gln Leu Arg
65                  70                  75                  80
Asp Ala Val Glu Ala Ala Glu Asp Ala Ile Asp Glu Leu Glu Tyr Tyr
                85                  90                  95
Glu Leu Glu Glu Lys Ala Lys Asp Gln Lys Val Ser Glu Trp Gly Ser
            100                 105                 110
Pro Leu Gly Lys Met Lys His Lys Phe Val Arg Ser Phe Gly Pro Ala
        115                 120                 125
Ile Asn Lys Thr Ile Lys Lys Ile Ser His Arg Asp Thr Leu Lys Arg
    130                 135                 140
Leu Met Lys Ser Val Asp Asp Leu Asp Lys Ala Ala Val Gly Val Ser
145                 150                 155                 160
Asp Phe Leu Lys Leu Thr Asp His Leu Ser Gly Gly Ser Ser Thr Ser
                165                 170                 175
Ser Gln Gln Leu Met Asp Asn Asp Arg Gln Thr Ser Ser Thr Ser Ser
            180                 185                 190
Ala Thr Asn Phe Val Gly Arg Glu Lys Glu Lys Glu Gln Ile Ile Gly
        195                 200                 205
Trp Leu Ala Asn Thr Ser Asp Glu Leu Gly Glu Thr Met Val Ile Lys
    210                 215                 220
Thr Asn Ser Ile Pro Ile Ile Ser Val Ile Gly His Gly Gly Met Gly
225                 230                 235                 240
Lys Thr Ala Leu Ala Gln Ser Ile Cys Gly Gln Asp Glu Val Val Lys
                245                 250                 255
His Phe Lys Val Ile Trp Thr Thr Ile Ser Thr Ser Phe Asp Ala Thr
            260                 265                 270
Ser Val Thr Ser Lys Ile Leu Glu Asp Ala Thr Arg Val Lys Pro Ser
        275                 280                 285
Ser Asp His Leu Glu Pro Leu Gln Arg Asp Leu Lys Glu Lys Leu Ala
    290                 295                 300
Ser Ile Asn Phe Leu Leu Val Leu Asp Asp Val Trp Glu Asp Lys Lys
305                 310                 315                 320
Arg Asp Glu Trp Glu Lys Leu Phe Ala Pro Leu Lys Glu Leu Asn Thr
                325                 330                 335
Thr Gly Ser Lys Ile Leu Leu Thr Thr Arg Met Gln Ser Val Ala Asp
            340                 345                 350
Met Ala Ala Lys Val Met Gly Val Glu Arg Asp Lys Phe Leu Thr Leu
        355                 360                 365
Gln Gly Leu Glu Asp Asp Lys Asn Leu Lys Leu Phe Gly His His Ala
    370                 375                 380
Phe Ser Gly Leu Asn Pro Arg Asp Asp Ala Tyr Leu Met Leu Ile Gly
385                 390                 395                 400
Glu Gln Ile Ala Lys Lys Leu Arg Gly Cys Pro Leu Val Thr Lys Val
                405                 410                 415
Val Ala Glu His Leu Gln Ser Asn Ile Thr Pro Glu Tyr Trp Arg Lys
            420                 425                 430
Phe Leu His Gln Gly Leu Glu His Phe Lys Gly Thr Glu Lys Asp Ile
        435                 440                 445
Met Lys Val Leu Arg Leu Ser Tyr Tyr His Leu Pro Thr Glu Leu Gln
    450                 455                 460

Ile Cys Phe Arg Tyr Cys Cys Leu Phe Pro Gln Asp Tyr Ile Phe Glu
465                 470                 475                 480
Lys Lys Glu Leu Val Gln Met Trp Ile Gly Ser Gly Leu Ile Ala Ser
                485                 490                 495
Gly Ile Gln Ser Leu Glu Asp Thr Ala Glu Gln Phe Leu Ala Gln Leu
            500                 505                 510
Thr Arg Lys Ser Phe Phe Asp Lys Lys Pro Ile Trp Ala Gln Ser Glu
        515                 520                 525
Phe Tyr Val Met His Asp Leu Met His Glu Leu Ala Ser Asn Val Ser
    530                 535                 540
Thr Gly Glu Cys Ala Arg Ile Val Asp Pro Val Gln Leu Gln Asp Glu
545                 550                 555                 560
Lys Tyr Thr Val Arg His Leu Cys Ile Ala Asn Ile His Ser Phe Ser
                565                 570                 575
Ala Asp Glu Val Lys Lys Ile Ser His Phe Lys Asn Leu Arg Thr Ile
            580                 585                 590
Ile Ile Asp Cys Met Pro Pro Leu Glu Asn Asp Thr Leu Cys Ala Leu
        595                 600                 605
Gln Met Ile Val Glu Thr Ser Lys Ser Leu Arg Leu Phe His Ser Ala
    610                 615                 620
Gln Leu Trp Asn Thr Ser Arg Phe Ala Val Asn Phe Gly Asn Leu Lys
625                 630                 635                 640
His Leu Arg Tyr Ile Cys Val Ser Ser Ile Pro Gln Asp Lys Ile Cys
                645                 650                 655
Gly Val Ala Arg Leu Tyr His Leu Met Val Leu His His Gly Ser Ser
            660                 665                 670
Gly Thr Lys Ile Asp Glu Ala Arg Tyr Leu Gly Asn Leu Glu Arg Leu
        675                 680                 685
Arg Tyr Val Ser Tyr Gly Val His Gly Phe Gly Asn Phe Pro Ile Ser
    690                 695                 700
Arg Leu Thr Ser Leu Gln Glu Leu His Asn Tyr Arg Val Glu Glu Arg
705                 710                 715                 720
Thr Cys Asn Gln Ile Ser Ala Val Gly Ser Leu Arg Asp Leu Arg Arg
                725                 730                 735
Leu Gly Leu Gln Gly Leu Glu Asn Val Lys Asn Trp Glu Glu Ala Lys
            740                 745                 750
Asn Ala Lys Leu Lys Glu Lys Gln Tyr Leu Asn Ser Leu Phe Met Glu
        755                 760                 765
Trp Ser Thr Pro Asp Gln Ile Met Thr Asp Asn Leu Val Leu Asp His
    770                 775                 780
Leu Glu Pro His Val Asn Ile Arg Glu Leu Gln Ile Gly Gly Tyr Pro
785                 790                 795                 800
Gly Leu Lys Ile Pro Ser Trp Ile Glu Asn Cys Ser Val Arg Asn Leu
                805                 810                 815
Ile Ser Leu Lys Leu Ile Ser Cys Ile Asn Trp Asp Tyr Ile Pro Ser
            820                 825                 830
Leu Cys Gln Leu Val Leu Leu Glu Leu Leu Val Leu Asp Asn Leu Pro
        835                 840                 845
Lys Leu Arg Gln Ile Gly Arg Ser Ser Asp Met Ser Lys Ser Ser Ser
    850                 855                 860
Met Glu Leu Leu Leu Pro Gln Ser Leu Asn Thr Leu Glu Val Ser Lys
865                 870                 875                 880
Cys Gln Lys Leu Arg Glu Leu Pro Leu Leu Pro Pro Ser Leu Ile Ser
                885                 890                 895
Leu Lys Ile His Asp Val Trp Leu Thr Lys Phe Pro Ile Ile Gly Lys
            900                 905                 910
Val Ser Ser Glu Ser Ile Glu Ser Lys Ser Ser Lys Leu Ile Asn Val
        915                 920                 925
Arg Val Thr Asn Cys Pro His Leu Thr Ser Leu Glu Gly Ser Leu Leu
    930                 935                 940
Glu Gln Lys Leu Tyr Met Gly Thr Leu Arg Val Leu Thr Ile Val Asp
945                 950                 955                 960
Cys Lys His Leu Glu Ser Ala Ser Ile Pro Phe Glu Glu Met Lys Gln

965                 970                 975
Leu Lys Glu Leu Ala Val Met Gln Cys Pro Lys Leu Arg Thr Met Arg
            980                 985                 990
Asp Ala Lys Asp Lys Leu Val Pro Ser Ser Leu Arg Glu Leu Ala Ile
        995                 1000                1005
Gly Gly Cys Gly Asp Leu Glu Leu Pro Leu Leu Gly Ser Leu Gln Leu
    1010                1015                1020
Leu Thr Asn Leu Ser Gly Leu Glu Leu Gln Asn Cys Ser Ser Leu Leu
1025                1030                1035                1040
Ser Leu Pro Ser Ala Asp Val Phe Lys Ser Phe Arg Ser Leu Gln Asn
                1045                1050                1055
Ile His Val Thr Arg Cys Glu Asn Leu Ser Ser Leu Gly Gly Leu Gly
            1060                1065                1070
Ser Leu Ser Ser Leu Gln Gln Leu Ala Ile Ser Lys Cys Ser Lys Leu
        1075                1080                1085
Thr Glu Ala Gly Ser Ser Leu Leu Ser Gly Ser Gly Asp Ala Glu Glu
    1090                1095                1100
His Leu Val Glu Pro Gly Asn Ser Leu Arg Ile Ser Val Leu Ser Leu
1105                1110                1115                1120
Asp Leu Pro Ser Leu Leu Leu His Glu Pro Leu Lys Ser Leu Cys His
                1125                1130                1135
Thr Glu Tyr Leu Cys Ile Asn Gly Cys Ser Glu Thr Lys Ser Leu Pro
            1140                1145                1150
Glu Arg Trp Leu Leu Gln Asn Arg Ser Ser Leu Arg His Leu Gly Ile
        1155                1160                1165
Leu Lys Ala Glu Ser Leu Glu Ser Leu Pro Pro Ser Met Gln Asp Leu
    1170                1175                1180
Cys Ser Leu Lys Ser Leu Tyr Leu Asp Gly Ala Leu Leu Leu Arg Ser
1185                1190                1195                1200
Leu Pro Tyr Leu Pro Ser Ser Leu Lys Asn Leu Asn Ile Thr Glu Cys
                1205                1210                1215
His Pro Asp Leu Glu Lys Lys Ile Thr Lys His Gly Ser Ser Glu Leu
            1220                1225                1230
Asn Lys Ile Ala His Ile Pro Asp Val Thr Ile Gly Gly Trp His Phe
        1235                1240                1245
Leu Met Gly Lys Lys Phe Ser Arg Glu Ala Tyr His Lys Leu Ser Asp
    1250                1255                1260
Ser Asp Arg Asn Arg Phe Met Ser Glu Ser Ser Leu His
1265                1270                1275

<210> 247
<211> 4406
<212> DNA
<213> Triticum aestivum

<400> 247
atggctgcct ctgccgcact cgtttttgca gggaaatctg tggcgacttc ggccatatcc      60

ttcttggtca acaaggcttt cagctacatc gatgagtact gcaagtccga aaacatggat     120

gagttgaaga atcggctcct gcgggccatg ccacatatcc aggcggtgtt cgatgtcgtc     180

aacccggaac ttgtcaggga gcagagcagc ggcctcgacg cgtggctctg gcagctcagg     240

gacgcggtcg aggcggcgga ggacgccatc gatgagcttg aatactacga gctcgaggag     300

aaggccaagg accaaaaggt tagtgaatgg ggttcccctc ttggtaagat gaagcacaag     360

tttgtcagat cttttggccc tgctatcaac aagactatca agaaaattag tcaccgtgac     420

actctgaaga ggttgatgaa atctgtggat gatttagaca aggctgccgt aggtgttagt     480

gatttcctca agctcacaga ccatctcagt ggaggttctt ctaccagcag ccagcagctc 540

atggacaatg atcgccagac tagttcaaca tcaagtgcaa ccaattttgt tggacgagaa 600

aaggagaaag aacagattat tgggtggcta gccaacacct cagatgaatt gggtgaaact 660

atggtcataa agaccaatag tattccaatt atttcagtta ttggtcatgg tggcatgggg 720

aagactgcct tggctcagag catatgtggt caagatgagg tagtaaagca ttttaaggtc 780

atctggacca ccatatctac tagctttgat gcgacatcgg tgacaagtaa aatactggaa 840

gacgctacgc gcgtgaaacc gagcagcgat catttggaac cactacaacg ggacctcaaa 900

gagaaactcg catccattaa ttttttgtta gttctagatg acgtttggga agataagaag 960

agagatgaat gggagaagtt atttgctcct ctgaaggaac tgaacactac tgggagcaaa 1020

attctgttga caacccgaat gcaatctgta gcagacatgg ctgcaaaggt gatgggagtc 1080

gaaagggata agttcttgac attacaagga ctggaagatg ataaaaatct caagctcttc 1140

ggtcatcatg ccttttccgg tttgaatcca agagatgatg catatttgat gttaattgga 1200

gaacaaattg caaagaaact gaggggatgt cccctggtaa cgaaggttgt cgccgagcac 1260

ctgcaaagca atataacacc tgaatactgg aggaaattct tgcatcaagg attggaacat 1320

tttaaaggaa ccgaaaaaga tattatgaag gttctcagat tgagctatta ccacttacca 1380

acagagctac agatttgctt tcggtactgt tgcctatttc cgcaggacta tatatttgaa 1440

aagaaagagt tggtgcaaat gtggattggt tcaggattga ttgcaagtgg cattcaatct 1500

ttggaggata ctgcagaaca gttcttggct cagctaacta gaaagtcgtt ctttgataag 1560

aaacctatat gggcacaaag tgaattttat gtaatgcatg acctgatgca tgaattagca 1620

agcaatgtgt ccaccggtga atgtgcaaga atagttgacc ctgttcaact tcaagatgag 1680

aagtatacag tccgacacct atgcattgcc aatattcaca gtttctctgc tgatgaggtc 1740

aagaaaatct cccattttaa gaatctgcgc actattatca ttgattgtat gccgccactt 1800

gaaaatgata ctctttgcgc acttcaaatg atagttgaga cctcaaaatc actgcgacta 1860

ttccactctg cacaattatg gaacacatcc cgttttgctg tcaattttgg taacttaaaa 1920

catcttcgct atatctgcgt gagttcgata ccacaagaca agatatgtgg ggtcgcaaga 1980

ctttatcact tgatggtcct tcaccatggt tcttctggga ccaaaataga tgaagcaaga 2040

tatttaggga accttgaacg tttgcggtat gtatcctatg gtgtgcatgg atttggtaat 2100

ttccccataa gcaggctcac ttctcttcag gaattacata attaccgggt agaagaaaga 2160

acatgcaacc aaataagtgc agttgggagc ttaagagatc ttcgtagatt gggtctgcag 2220

ggtcttgaga atgtcaagaa ttgggaagaa gctaagaatg ccaagttgaa ggaaaaacaa 2280

tatctcaact cattatttat ggagtggtcg acacctgatc aaatcatgac agataactta 2340

gttcttgacc accttgagcc acatgttaat atcagagaat tgcaaattgg gggttatcca 2400

ggtctcaaaa ttccatcttg gatcgagaac tgctcagtca gaaacttgat atcactcaag 2460

ttgataagct gcataaattg ggactacatt ccctctctct gccagttggt attgctggag 2520

cttttagtgt tggacaatct tcctaagcta cggcagattg gtcgatcatc tgatatgtcc 2580

aagagtagtt ccatggagtt gttgttacct caaagcctta atactttgga agtaagcaaa 2640

tgccagaaac tgagggaatt accacttctg cctccaagcc taatatcatt gaaaatacac 2700

gatgtttggc tgaccaagtt tcctattatt ggcaaggtat cgagtgagag catcgagtca 2760

aaatcatcta agttgattaa cgtaagggtc accaactgtc ctcatttaac ttcgttggaa 2820

gggagccttt tggagcaaaa actgtacatg ggaactcttc gtgtcctaac aattgttgat 2880

tgtaaacatc tggagtccgc atccatacca tttgaagaaa tgaaacaact taaggaactt 2940

gcagtaatgc aatgtccaaa gttgaggacg atgagagatg ccaaagataa gcttgtgcca 3000

tcgtcactaa gagaactagc tattggcggg tgtggtgacc tggaacttcc actacttggg 3060

tcactgcagc tgctcaccaa cctatctggg ctcgagctgc agaactgctc gagcctgtta 3120

tctctcccct ccgcggatgt gttcaagagt ttcaggtcgc tgcagaacat acatgtaacg 3180

cgatgcgaga atctctcgtc cctgggaggg ctcggatcac tgtcatccct tcagcagtta 3240

gctatctcca agtgcagtaa actcacggag gctgggtctt ccttgctatc tggttctggt 3300

gatgcagaag agcatctggt ggagcccggc aactcactgc ggataagtgt tcttagctta 3360

gatctgccgt ccttgctgct tcatgagcct ctcaagagtc tgtgccacac tgaatattta 3420

tgcattaatg gctgttcaga gacgaagagc ttacctgaac gatggctgct acagaaccgc 3480

tcatccctcc gacacctggg tatactgaaa gcagaatcct tggaatcgct cccaccgagc 3540

atgcaagacc tttgctctct caagtcatta taccttgatg gtgctctgct actccgatca 3600

cttccatatc ttccctcctc cttgaagaat ctgaacatta cggaatgcca tccagacctg 3660

gagaagaaga tcacaaaaca tggaagctct gaattgaaca agattgccca catccctgat 3720

gtgacaatag gtaactccgc ccttccactc tcgtcctcac ttgcttaatt tactgcttgg 3780

acctctgtac tgctgaaacc tactgatcct gatctgactg tgaaccgatg acaggaggtt 3840

ggcatttcct tatgggcaag aaattcagca gagaggctta ccataaactg aggtaattaa 3900

taatcttctg agaaagacat tcaatagtat cctctttttg ctctgaataa gggttggtgc 3960

gcaaagtgct atggcagaca ctcatatata atggcacaac acccccgcat ggccgcattg 4020

cacgaaatat gatcaacgaa ctgaccatgc aaaacatatc aatatatgac ggcgaacaca 4080

tagcaaaacc tggtgtaagc tcacagactc actagataag acccctatca aagaattgag 4140

agttgtaaaa ggagagtaat cgtatctttt gcttctcctt gagcagcatt ttattaccat 4200

tgcaaccgta ctgttgaagt gcaactatgc ttattcatat cctaggatac gtttccattt 4260

tagtatttga cactccaaat gcatgctatc atttatttcc ccttgccgca aacaaatatc     4320

tttgttcttg aaaactgaaa cttctgtttt tggtcatcag tgatagtgac cgcaacagat     4380

ttatgtccga gtcgtcgttg cactag                                          4406


<210> 248
<211> 360
<212> DNA
<213> Artificial Sequence


<220>
<223> cDNA

<400> 248
atgagaccat tccaactttt gtctgctatt gcagtcttca atagtttgaa gatagtagcg      60

ggtgaatctt attgggattg taagggtatt ccaattttat ttcgaactgt ccatgctgcg     120

gtggaattgg cttttacttc gcagccaggt tctattagtg gttacccctc aatttgccgt     180

acaacacctc tacggactgg accagacgaa cgtcgacagt ttccactaac agatactggc     240

gcaaggtggc aaggaggagg tataacctat tacgttgaag ccactagaga taagagacat     300

tgcgaagtct ttggaacagc aggaggtgtc tataaatgta cattagttct gcgagactag     360


<210> 249
<211> 119
<212> PRT
<213> Blumeria graminis


<400> 249
Met Arg Pro Phe Gln Leu Leu Ser Ala Ile Ala Val Phe Asn Ser Leu
1               5                   10                  15
Lys Ile Val Ala Gly Glu Ser Tyr Trp Asp Cys Lys Gly Ile Pro Ile
            20                  25                  30
Leu Phe Arg Thr Val His Ala Ala Val Glu Leu Ala Phe Thr Ser Gln
        35                  40                  45
Pro Gly Ser Ile Ser Gly Tyr Pro Ser Ile Cys Arg Thr Thr Pro Leu
    50                  55                  60
Arg Thr Gly Pro Asp Glu Arg Arg Gln Phe Pro Leu Thr Asp Thr Gly
65                  70                  75                  80
Ala Arg Trp Gln Gly Gly Gly Ile Thr Tyr Tyr Val Glu Ala Thr Arg
                85                  90                  95
Asp Lys Arg His Cys Glu Val Phe Gly Thr Ala Gly Gly Val Tyr Lys
            100                 105                 110
Cys Thr Leu Val Leu Arg Asp
        115

<210> 250
<211> 413
<212> DNA
<213> Blumeria graminis


<400> 250
atgagaccat tccaactttt gtctgctatt gcagtcttca atagtttgaa gatagtagcg      60

ggtgaatctt attgggattg taagggtatt ccaattttat ttcgaactgt ccatgctgcg 120

gtggaattgg cttttacttc gcagccaggt tctattagtg gttacccctc aatttgccgt 180

acaacacctc tacggactgg accagacgaa cgtcgacagt ttccactaac agatactggc 240

gcaaggtggc aaggttggtt atttggttct cttccaccaa tgaaaacacg ctaataattt 300

tggtaggagg aggtataacc tattacgttg aagccactag agataagaga cattgcgaag 360

tctttggaac agcaggaggt gtctataaat gtacattagt tctgcgagac tag 413

<210> 251
<211> 4248
<212> DNA
<213> Artificial Sequence

<220>
<223> cDNA

<400> 251
atggcagagc gggtggtcac catggcgatc gggccactgg tgtccatgct gaaggacaag 60

gcgtccagct acctcctgga ccagtacaag gtcatggagg gaatggagga gcagcacaag 120

attctcaaac gcaagcttcc ggccatcctc gacgtcatca ccgatgtcga ggagcaggcc 180

atggcacaga gagaaggtgc caaagcctgg cttcaggagc tcaggacagt cgcctatgtg 240

gcaaatgaag tcttcgacga attcaagtac gaagccctac gccgtgaagc caagaagaat 300

gggcactaca taaagctcgg cttcgatgta attaaactct tccctactca caaccgtgtt 360

gcgttccgtt acaaaatggg tcgcaagctt tgcctgattc tgcaagccgt tgaggtcctc 420

atagcagaga tgcaggtctt tgggttcaag taccaaccac agccaccggt gtccaaagag 480

tggaggcata cagattatgt tagcattgac ccacaagaaa ttgccagcag atccagacac 540

gaagataaga agaacattat tggtatacta gttgatgaag ctagcaatgc agatctcaca 600

gttgttcctg tggttgcaat gggggccttt ggcaagacca cattagcgca actcatatac 660

aatgatcctg aaattcagaa gcatttccag ttgctgctct gggtttgtgt ctctgatacc 720

tttgatgtga actccctggc caagagtata gttgaagcat cccccaataa gaatgttgat 780

acagacaaac caccattgga tagacttcaa aaactggtca gcggacagcg gtatctcctt 840

gtattggatg atgtttggga caacaaagag ttacgtaagt gggaaaggct gaaggtatgt 900

ctccagcatg gtggcatggg cagtgcagtg ttgacaacaa ctcgtgataa acgagtttct 960

gaaattatgg gtgcagatag agctgcctac aatctcaatg ctttggagga tcacttcata 1020

aaggaaatta ttgaggctag agcgttcagt tcaaagaaag aaaagcctat tgaactagtg 1080

gaggtagttg acgagattgt gaagagatgt tgtggctctc cattagctgc aacggcactg 1140

ggctctgtac tttgtaccaa gaccagtgtg aaagaatgga aagctgtatc atctggaacc 1200

agcgtttgca ctgaggaaac tggaatcttg ccaatactca agcttagcta caacgatttg 1260

ccatcgcaca tgaagcagtg ctttgctttt tgtgctgtat ttccaaagga ttacaagatt 1320

gatgtagcga agttgatcca actatggatc gcaaatggct ttatccctga acacaaggaa 1380

gatagtcttg aaaccattgg acaacttatt tttgatgagc ttgcttcaag gtcattcttt 1440

ctggatatag agaagagtaa agaagactgg gagtattatt ccagaacaac atgtaaaatc 1500

cacgatctta tgcatgatat tgcaatgtct gttatggaaa aggaatgtgt tgttgcaact 1560

atggaaccaa gtgaaattga gtggcttcca gatactgctc ggcatttgtt cttgtcatgt 1620

gaagaaacgg aacgtatttt gaatgattct atggaggaaa gatcccctgc tattcaaaca 1680

ttgctatgtg atagtaatgt gttcagccca ttgaagcatc tatcaaaata tagctctttg 1740

cacgccttga agctctgtat tagaggcaca gaatcatttc tactcaaacc aaagtatctg 1800

catcacctga ggtaccttga tctctcagaa agtcgtatga aagcacttcc tgaggatata 1860

agtattctat ataacctgca agtgttggac ctttcctact gtaattatct tgatcgactt 1920

ccaaggcaaa tgaagtatat gacttccctc tgccacctct acactcatgg atgtcggaac 1980

ttgaagagca tgcctccagg acttgaaaat ctcactaagc tgcagacact cacagttttt 2040

gtagcaggag ttcctggccc tgattgcgct gatgttggag agctgcatgg tctaaacatt 2100

ggtggtcggc tagagctatg ccaggtagag aatgttgaaa aagcagaagc agaagtggca 2160

aaccttggtg gtcagctaga gctgcagcat ttaaaccttg gtgatcagct agagctacgc 2220

cgggtagaga atgttaaaaa agcagaggca aaagtggcga atcttggaaa caagaaggat 2280

ctccgtgaac tgacattaag atggactgag gttggcgaca gcaaggtgct cgacaagttc 2340

gaacctcatg gtgggctgca ggttctgaag atatataaat atggagggaa gtgtatgggt 2400

atgttgcaaa acatggttga gatccatctt tctggttgtg aaagattgca ggttttgttc 2460

agttgtggta catccttcac gtttccaaaa ctgaaggtgc ttacactaga gcatttattg 2520

gattttgaga gatggtggga aataaatgag gcacaagaag aacagataat atttcctctg 2580

cttgagaagt tgtttattcg gcattgtgga aagctgatag cattacctga agcaccattg 2640

cttggagaac caagtcgtgg aggtaataga ctggtatgca caccattttc tctgcttgag 2700

aacttgttta tttggtattg tggaaagctg gtaccattgc gtgaagcaag gttggttcat 2760

gaaaattgta gtggaggtta tagattggta cagtcagcat ttcctgctct aaaggtactt 2820

gcattggaag acttggagag ttttcagaaa tgggatgcag ctatcgaagg agaaccgata 2880

ttgtttcctc aacttgagac actatcagtt cagaaatgcc caaagctagt agatttaccc 2940

gaagcaccaa aactcagtgt actagtaatt gaagatggca agcaagaggt gttccatttt 3000

gtagacaggt atttatcttc attgaccaat ctgacactga ggctagaaca cagagaaaca 3060

acatcagagg ctgaatgcac ttcaattgta cctgtggaca gcaaagagaa atggaaccag 3120

aaatcccctc ttacagttct ggagttagga tgctgcaact cattctttgg accaggtgca 3180

ctagagccgt gggactattt tgtacacctt gaaaagttgg aaattgatag atgtgatgtg 3240

ctcgtccact ggccagagaa agtgttccaa agcttggtat cgttgaggag attagtgatt 3300

agaaactgcg aaaatctgac tggatatgca caagctcctc ttgagccatt ggcatccgaa 3360

aggagtgaac acctgagagg tctggagtct cttcgcatag aaaactgccc aagtttagta 3420

gagatgttca acgtcccggc atctctcaag aaaatggata ttcttgagtg cgataagctt 3480

gagtccatat tcggcaagca acagggcatg gcagagttag tccaagtatc ttctagcagt 3540

gaggcaatca tgcctgcaac tgtatcagag ttgccatcca cacccatgaa tcacttttgt 3600

ccatgcctag aagatctatg cttatcagca tgtggaagct taccagcggt tctaaatctg 3660

cctccatcct taaagacctt agaaatggat cgttgtagta gtattcaagt cctatcatgc 3720

cagctgggtg ggctccagaa accagaagcc actacctcca gaagcagaag tcctatcatg 3780

ccacagccac tagcagcagc aacagcacca gctgcaagag agcatttact tcctccccat 3840

ctcgaatatc taacaatact gaactgtgct ggcatgttgg gtgggactct ccgtctgcct 3900

gcacccctca agagactgtt cattatgggc aacagtgggc tgacatcgct ggagtgtctg 3960

tcgggagagc accccccatc gctgaaaatc cttgatctta gaagctgcag taccctggca 4020

tccctgccga atgagccgca agtatacagg tctctctggt ctcttgaaat tacaggctgc 4080

cctgctataa agaagctccc tagatgcctg cagcagcaac tgggcagcat caaacgcaaa 4140

tggctagatg cccgttatga agtaacggaa ttcaaaccat tgaaaccgaa gacatggaag 4200

gaaataccga ggctagtccg tgagcggagg caggcctgcc ggagctga 4248

<210> 252
<211> 1415
<212> PRT
<213> Triticum aestivum

<400> 252
   Met Ala Glu Arg Val Val Thr Met Ala Ile Gly Pro Leu Val Ser Met
   1               5                   10                  15
   Leu Lys Asp Lys Ala Ser Ser Tyr Leu Leu Asp Gln Tyr Lys Val Met
               20                  25                  30
   Glu Gly Met Glu Glu Gln His Lys Ile Leu Lys Arg Lys Leu Pro Ala
           35                  40                  45
   Ile Leu Asp Val Ile Thr Asp Val Glu Glu Gln Ala Met Ala Gln Arg
       50                  55                  60
   Glu Gly Ala Lys Ala Trp Leu Gln Glu Leu Arg Thr Val Ala Tyr Val
   65                  70                  75                  80
   Ala Asn Glu Val Phe Asp Glu Phe Lys Tyr Glu Ala Leu Arg Arg Glu
                   85                  90                  95
   Ala Lys Lys Asn Gly His Tyr Ile Lys Leu Gly Phe Asp Val Ile Lys
               100                 105                 110
   Leu Phe Pro Thr His Asn Arg Val Ala Phe Arg Tyr Lys Met Gly Arg
           115                 120                 125
   Lys Leu Cys Leu Ile Leu Gln Ala Val Glu Val Leu Ile Ala Glu Met
       130                 135                 140
   Gln Val Phe Gly Phe Lys Tyr Gln Pro Gln Pro Pro Val Ser Lys Glu
   145                 150                 155                 160
   Trp Arg His Thr Asp Tyr Val Ser Ile Asp Pro Gln Glu Ile Ala Ser
                   165                 170                 175
   Arg Ser Arg His Glu Asp Lys Lys Asn Ile Ile Gly Ile Leu Val Asp
               180                 185                 190
   Glu Ala Ser Asn Ala Asp Leu Thr Val Val Pro Val Val Ala Met Gly
           195                 200                 205
   Gly Leu Gly Lys Thr Thr Leu Ala Gln Leu Ile Tyr Asn Asp Pro Glu
       210                 215                 220
   Ile Gln Lys His Phe Gln Leu Leu Leu Trp Val Cys Val Ser Asp Thr
   225                 230                 235                 240
   Phe Asp Val Asn Ser Leu Ala Lys Ser Ile Val Glu Ala Ser Pro Asn
                   245                 250                 255
   Lys Asn Val Asp Thr Asp Lys Pro Pro Leu Asp Arg Leu Gln Lys Leu
               260                 265                 270
   Val Ser Gly Gln Arg Tyr Leu Leu Val Leu Asp Asp Val Trp Asp Asn
           275                 280                 285
   Lys Glu Leu Arg Lys Trp Glu Arg Leu Lys Val Cys Leu Gln His Gly
       290                 295                 300
   Gly Met Gly Ser Ala Val Leu Thr Thr Thr Arg Asp Lys Arg Val Ser
   305                 310                 315                 320
   Glu Ile Met Gly Ala Asp Arg Ala Ala Tyr Asn Leu Asn Ala Leu Glu
                   325                 330                 335
   Asp His Phe Ile Lys Glu Ile Ile Glu Ala Arg Ala Phe Ser Ser Lys
               340                 345                 350
   Lys Glu Lys Pro Ile Glu Leu Val Glu Val Val Asp Glu Ile Val Lys
           355                 360                 365
   Arg Cys Cys Gly Ser Pro Leu Ala Ala Thr Ala Leu Gly Ser Val Leu
       370                 375                 380
   Cys Thr Lys Thr Ser Val Lys Glu Trp Lys Ala Val Ser Ser Gly Thr
   385                 390                 395                 400
   Ser Val Cys Thr Glu Glu Thr Gly Ile Leu Pro Ile Leu Lys Leu Ser
                   405                 410                 415
   Tyr Asn Asp Leu Pro Ser His Met Lys Gln Cys Phe Ala Phe Cys Ala
               420                 425                 430
   Val Phe Pro Lys Asp Tyr Lys Ile Asp Val Ala Lys Leu Ile Gln Leu
           435                 440                 445
   Trp Ile Ala Asn Gly Phe Ile Pro Glu His Lys Glu Asp Ser Leu Glu
       450                 455                 460
   Thr Ile Gly Gln Leu Ile Phe Asp Glu Leu Ala Ser Arg Ser Phe Phe
   465                 470                 475                 480
   Leu Asp Ile Glu Lys Ser Lys Glu Asp Trp Glu Tyr Tyr Ser Arg Thr
                   485                 490                 495
   Thr Cys Lys Ile His Asp Leu Met His Asp Ile Ala Met Ser Val Met
               500                 505                 510
   Glu Lys Glu Cys Val Val Ala Thr Met Glu Pro Ser Glu Ile Glu Trp
           515                 520                 525
   Leu Pro Asp Thr Ala Arg His Leu Phe Leu Ser Cys Glu Glu Thr Glu
       530                 535                 540
   Arg Ile Leu Asn Asp Ser Met Glu Glu Arg Ser Pro Ala Ile Gln Thr
   545                 550                 555                 560
   Leu Leu Cys Asp Ser Asn Val Phe Ser Pro Leu Lys His Leu Ser Lys
                   565                 570                 575
   Tyr Ser Ser Leu His Ala Leu Lys Leu Cys Ile Arg Gly Thr Glu Ser
               580                 585                 590
   Phe Leu Leu Lys Pro Lys Tyr Leu His His Leu Arg Tyr Leu Asp Leu
           595                 600                 605
   Ser Glu Ser Arg Met Lys Ala Leu Pro Glu Asp Ile Ser Ile Leu Tyr
       610                 615                 620
   Asn Leu Gln Val Leu Asp Leu Ser Tyr Cys Asn Tyr Leu Asp Arg Leu
   625                 630                 635                 640
   Pro Arg Gln Met Lys Tyr Met Thr Ser Leu Cys His Leu Tyr Thr His
                   645                 650                 655
   Gly Cys Arg Asn Leu Lys Ser Met Pro Pro Gly Leu Glu Asn Leu Thr
               660                 665                 670
   Lys Leu Gln Thr Leu Thr Val Phe Val Ala Gly Val Pro Gly Pro Asp
           675                 680                 685
   Cys Ala Asp Val Gly Glu Leu His Gly Leu Asn Ile Gly Gly Arg Leu
       690                 695                 700
   Glu Leu Cys Gln Val Glu Asn Val Glu Lys Ala Glu Ala Glu Val Ala
   705                 710                 715                 720
   Asn Leu Gly Gly Gln Leu Glu Leu Gln His Leu Asn Leu Gly Asp Gln
                   725                 730                 735
   Leu Glu Leu Arg Arg Val Glu Asn Val Lys Lys Ala Glu Ala Lys Val
               740                 745                 750
   Ala Asn Leu Gly Asn Lys Lys Asp Leu Arg Glu Leu Thr Leu Arg Trp
           755                 760                 765
   Thr Glu Val Gly Asp Ser Lys Val Leu Asp Lys Phe Glu Pro His Gly
       770                 775                 780
   Gly Leu Gln Val Leu Lys Ile Tyr Lys Tyr Gly Gly Lys Cys Met Gly
   785                 790                 795                 800
   Met Leu Gln Asn Met Val Glu Ile His Leu Ser Gly Cys Glu Arg Leu
                   805                 810                 815
   Gln Val Leu Phe Ser Cys Gly Thr Ser Phe Thr Phe Pro Lys Leu Lys
               820                 825                 830
   Val Leu Thr Leu Glu His Leu Leu Asp Phe Glu Arg Trp Trp Glu Ile
           835                 840                 845
   Asn Glu Ala Gln Glu Glu Gln Ile Ile Phe Pro Leu Leu Glu Lys Leu
       850                 855                 860
   Phe Ile Arg His Cys Gly Lys Leu Ile Ala Leu Pro Glu Ala Pro Leu
   865                 870                 875                 880
   Leu Gly Glu Pro Ser Arg Gly Gly Asn Arg Leu Val Cys Thr Pro Phe
                   885                 890                 895
   Ser Leu Leu Glu Asn Leu Phe Ile Trp Tyr Cys Gly Lys Leu Val Pro
               900                 905                 910
   Leu Arg Glu Ala Arg Leu Val His Glu Asn Cys Ser Gly Gly Tyr Arg
           915                 920                 925
   Leu Val Gln Ser Ala Phe Pro Ala Leu Lys Val Leu Ala Leu Glu Asp
       930                 935                 940
   Leu Glu Ser Phe Gln Lys Trp Asp Ala Ala Ile Glu Gly Glu Pro Ile
   945                 950                 955                 960
   Leu Phe Pro Gln Leu Glu Thr Leu Ser Val Gln Lys Cys Pro Lys Leu
                   965                 970                 975
   Val Asp Leu Pro Glu Ala Pro Lys Leu Ser Val Leu Val Ile Glu Asp
               980                 985                 990
   Gly Lys Gln Glu Val Phe His Phe Val Asp Arg Tyr Leu Ser Ser Leu
           995                 1000                1005
   Thr Asn Leu Thr Leu Arg Leu Glu His Arg Glu Thr Thr Ser Glu Ala
       1010                1015                1020
   Glu Cys Thr Ser Ile Val Pro Val Asp Ser Lys Glu Lys Trp Asn Gln
   1025                1030                1035                1040
   Lys Ser Pro Leu Thr Val Leu Glu Leu Gly Cys Cys Asn Ser Phe Phe
                   1045                1050                1055
   Gly Pro Gly Ala Leu Glu Pro Trp Asp Tyr Phe Val His Leu Glu Lys
               1060                1065                1070
   Leu Glu Ile Asp Arg Cys Asp Val Leu Val His Trp Pro Glu Lys Val
           1075                1080                1085
   Phe Gln Ser Leu Val Ser Leu Arg Arg Leu Val Ile Arg Asn Cys Glu
       1090                1095                1100
   Asn Leu Thr Gly Tyr Ala Gln Ala Pro Leu Glu Pro Leu Ala Ser Glu
   1105                1110                1115                1120
   Arg Ser Glu His Leu Arg Gly Leu Glu Ser Leu Arg Ile Glu Asn Cys
                   1125                1130                1135
   Pro Ser Leu Val Glu Met Phe Asn Val Pro Ala Ser Leu Lys Lys Met
               1140                1145                1150
   Asp Ile Leu Glu Cys Asp Lys Leu Glu Ser Ile Phe Gly Lys Gln Gln
           1155                1160                1165
   Gly Met Ala Glu Leu Val Gln Val Ser Ser Ser Ser Glu Ala Ile Met
       1170                1175                1180
   Pro Ala Thr Val Ser Glu Leu Pro Ser Thr Pro Met Asn His Phe Cys
   1185                1190                1195                1200
   Pro Cys Leu Glu Asp Leu Cys Leu Ser Ala Cys Gly Ser Leu Pro Ala
                   1205                1210                1215
   Val Leu Asn Leu Pro Pro Ser Leu Lys Thr Leu Glu Met Asp Arg Cys
               1220                1225                1230
   Ser Ser Ile Gln Val Leu Ser Cys Gln Leu Gly Gly Leu Gln Lys Pro
           1235                1240                1245
   Glu Ala Thr Thr Ser Arg Ser Arg Ser Pro Ile Met Pro Gln Pro Leu
       1250                1255                1260
   Ala Ala Ala Thr Ala Pro Ala Ala Arg Glu His Leu Leu Pro Pro His
   1265                1270                1275                1280
   Leu Glu Tyr Leu Thr Ile Leu Asn Cys Ala Gly Met Leu Gly Gly Thr
                   1285                1290                1295
   Leu Arg Leu Pro Ala Pro Leu Lys Arg Leu Phe Ile Met Gly Asn Ser
               1300                1305                1310
   Gly Leu Thr Ser Leu Glu Cys Leu Ser Gly Glu His Pro Pro Ser Leu
           1315                1320                1325
   Lys Ile Leu Asp Leu Arg Ser Cys Ser Thr Leu Ala Ser Leu Pro Asn
       1330                1335                1340
   Glu Pro Gln Val Tyr Arg Ser Leu Trp Ser Leu Glu Ile Thr Gly Cys
   1345                1350                1355                1360
   Pro Ala Ile Lys Lys Leu Pro Arg Cys Leu Gln Gln Gln Leu Gly Ser
                   1365                1370                1375
   Ile Lys Arg Lys Trp Leu Asp Ala Arg Tyr Glu Val Thr Glu Phe Lys
               1380                1385                1390
   Pro Leu Lys Pro Lys Thr Trp Lys Glu Ile Pro Arg Leu Val Arg Glu
           1395                1400                1405
   Arg Arg Gln Ala Cys Arg Ser
       1410                1415

<210> 253
<211> 4448
<212> DNA
<213> Triticum aestivum


<400> 253
atggcagagc gggtggtcac catggcgatc gggccactgg tgtccatgct gaaggacaag      60

gcgtccagct acctcctgga ccagtacaag gtcatggagg gaatggagga gcagcacaag     120

attctcaaac gcaagcttcc ggccatcctc gacgtcatca ccgatgtcga ggagcaggcc     180

atggcacaga gagaaggtgc caaagcctgg cttcaggagc tcaggacagt cgcctatgtg     240

gcaaatgaag tcttcgacga attcaagtac gaagccctac gccgtgaagc caagaagaat     300

gggcactaca taaagctcgg cttcgatgta attaaactct tccctactca caaccgtgtt     360

gcgttccgtt acaaaatggg tcgcaagctt tgcctgattc tgcaagccgt tgaggtcctc     420

atagcagaga tgcaggtctt tgggttcaag taccaaccac agccaccggt gtccaaagag     480

tggaggcata cagattatgt tagcattgac ccacaagaaa ttgccagcag atccagacac     540

gaagataaga agaacattat tggtatacta gttgatgaag ctagcaatgc agatctcaca     600

gttgttcctg tggttgcaat gggggccttt ggcaagacca cattagcgca actcatatac 660

aatgatcctg aaattcagaa gcatttccag ttgctgctct gggtttgtgt ctctgatacc 720

tttgatgtga actccctggc caagagtata gttgaagcat cccccaataa gaatgttgat 780

acagacaaac caccattgga tagacttcaa aaactggtca gcggacagcg gtatctcctt 840

gtattggatg atgtttggga caacaaagag ttacgtaagt gggaaaggct gaaggtatgt 900

ctccagcatg gtggcatggg cagtgcagtg ttgacaacaa ctcgtgataa acgagtttct 960

gaaattatgg gtgcagatag agctgcctac aatctcaatg ctttggagga tcacttcata 1020

aaggaaatta ttgaggctag agcgttcagt tcaaagaaag aaaagcctat tgaactagtg 1080

gaggtagttg acgagattgt gaagagatgt tgtggctctc cattagctgc aacggcactg 1140

ggctctgtac tttgtaccaa gaccagtgtg aaagaatgga aagctgtatc atctggaacc 1200

agcgtttgca ctgaggaaac tggaatcttg ccaatactca agcttagcta caacgatttg 1260

ccatcgcaca tgaagcagtg ctttgctttt tgtgctgtat ttccaaagga ttacaagatt 1320

gatgtagcga agttgatcca actatggatc gcaaatggct ttatccctga acacaaggaa 1380

gatagtcttg aaaccattgg acaacttatt tttgatgagc ttgcttcaag gtcattcttt 1440

ctggatatag agaagagtaa agaagactgg gagtattatt ccagaacaac atgtaaaatc 1500

cacgatctta tgcatgatat tgcaatgtct gttatggaaa aggaatgtgt tgttgcaact 1560

atggaaccaa gtgaaattga gtggcttcca gatactgctc ggcatttgtt cttgtcatgt 1620

gaagaaacgg aacgtatttt gaatgattct atggaggaaa gatcccctgc tattcaaaca 1680

ttgctatgtg atagtaatgt gttcagccca ttgaagcatc tatcaaaata tagctctttg 1740

cacgccttga agctctgtat tagaggcaca gaatcatttc tactcaaacc aaagtatctg 1800

catcacctga ggtaccttga tctctcagaa agtcgtatga aagcacttcc tgaggatata 1860

agtattctat ataacctgca agtgttggac ctttcctact gtaattatct tgatcgactt 1920

ccaaggcaaa tgaagtatat gacttccctc tgccacctct acactcatgg atgtcggaac 1980

ttgaagagca tgcctccagg acttgaaaat ctcactaagc tgcagacact cacagttttt 2040

gtagcaggag ttcctggccc tgattgcgct gatgttggag agctgcatgg tctaaacatt 2100

ggtggtcggc tagagctatg ccaggtagag aatgttgaaa aagcagaagc agaagtggca 2160

aaccttggtg gtcagctaga gctgcagcat ttaaaccttg gtgatcagct agagctacgc 2220

cgggtagaga atgttaaaaa agcagaggca aaagtggcga atcttggaaa caagaaggat 2280

ctccgtgaac tgacattaag atggactgag gttggcgaca gcaaggtgct cgacaagttc 2340

gaacctcatg gtgggctgca ggttctgaag atatataaat atggagggaa gtgtatgggt 2400

atgttgcaaa acatggttga gatccatctt tctggttgtg aaagattgca ggttttgttc 2460

agttgtggta catccttcac gtttccaaaa ctgaaggtgc ttacactaga gcatttattg 2520

gattttgaga gatggtggga aataaatgag gcacaagaag aacagataat atttcctctg 2580

cttgagaagt tgtttattcg gcattgtgga aagctgatag cattacctga agcaccattg 2640

cttggagaac caagtcgtgg aggtaataga ctggtatgca caccattttc tctgcttgag 2700

aacttgttta tttggtattg tggaaagctg gtaccattgc gtgaagcaag gttggttcat 2760

gaaaattgta gtggaggtta tagattggta cagtcagcat ttcctgctct aaaggtactt 2820

gcattggaag acttggagag ttttcagaaa tgggatgcag ctatcgaagg agaaccgata 2880

ttgtttcctc aacttgagac actatcagtt cagaaatgcc caaagctagt agatttaccc 2940

gaagcaccaa aactcagtgt actagtaatt gaagatggca agcaagaggt gttccatttt 3000

gtagacaggt atttatcttc attgaccaat ctgacactga ggctagaaca cagagaaaca 3060

acatcagagg ctgaatgcac ttcaattgta cctgtggaca gcaaagagaa atggaaccag 3120

aaatcccctc ttacagttct ggagttagga tgctgcaact cattctttgg accaggtgca 3180

ctagagccgt gggactattt tgtacacctt gaaaagttgg aaattgatag atgtgatgtg 3240

ctcgtccact ggccagagaa agtgttccaa agcttggtat cgttgaggag attagtgatt 3300

agaaactgcg aaaatctgac tggatatgca caagctcctc ttgagccatt ggcatccgaa 3360

aggagtgaac acctgagagg tctggagtct cttcgcatag aaaactgccc aagtttagta 3420

gagatgttca acgtcccggc atctctcaag aaaatggata ttcttgagtg cgataagctt 3480

gagtccatat tcggcaagca acagggcatg gcagagttag tccaagtatc ttctagcagt 3540

gaggcaatca tgcctgcaac tgtatcagag ttgccatcca cacccatgaa tcacttttgt 3600

ccatgcctag aagatctatg cttatcagca tgtggaagct taccagcggt tctaaatctg 3660

cctccatcct taaagacctt agaaatggat cgttgtagta gtattcaagt cctatcatgc 3720

cagctgggtg ggctccagaa accagaagcc actacctcca gaagcagaag tcctatcatg 3780

ccacagccac tagcagcagc aacagcacca gctgcaagag agcatttact tcctccccat 3840

ctcgaatatc taacaatact gaactgtgct ggcatgttgg gtgggactct ccgtctgcct 3900

gcacccctca agagactgtt cattatgggc aacagtgggc tgacatcgct ggagtgtctg 3960

tcgggagagc accccccatc gctgaaaatc cttgatctta gaagctgcag taccctggca 4020

tccctgccga atgagccgca agtatacagg tctctctggt ctcttgaaat tacaggctgc 4080

cctgctataa agaagctccc tagatgcctg cagcagcaac tgggcagcat caaacgcaaa 4140

tggctagatg cccgttatga aggtatgctt gcagcacgtc cttctatctt ggccacattt 4200

aataaacaga tgcagtcatc cattccatcc cttgtacata atggtcaaca cgtttgcaaa 4260

agtttcttct ccattatttg aatgccatca tgctatacat tatgatataa cacattagtt 4320

tgtacaagtt tttttttctg acatcgcttg tggtatgtgc agtaacggaa ttcaaaccat 4380

tgaaaccgaa gacatggaag gaaataccga ggctagtccg tgagcggagg caggcctgcc 4440

ggagctga 4448

<210> 254
<211> 393
<212> DNA
<213> Artificial Sequence

<220>
<223> cDNA

<400> 254
atgagaacct tcagtcttgt gtcactggcg gctattttaa attatctaac gcttccatct 60

gccgttggga gtggccctgt cgctaacgct tcaagttaca agtgtcatga cagggtcatt 120

ggtccggtta ccttaaatga tcagatagaa aaggcatata gagaagcact agaagcaggc 180

acttcaccta acggactaag gaaaggacag aaatttggtt caagatactt taatgtaatc 240

ttaaaaagag gagaagaaaa tattaaagtc gaattttttg tgggaatcaa ctatttgaag 300

gaaattattt atctacaggc ttatgtccag agcgtattgc tagattgtta tccaacaaca 360

gagaggcctc aattaaacat aattctgcac tag 393

<210> 255
<211> 130
<212> PRT
<213> Blumeria graminis

<400> 255
Met Arg Thr Phe Ser Leu Val Ser Leu Ala Ala Ile Leu Asn Tyr Leu
1               5                   10                  15
Thr Leu Pro Ser Ala Val Gly Ser Gly Pro Val Ala Asn Ala Ser Ser
            20                  25                  30
Tyr Lys Cys His Asp Arg Val Ile Gly Pro Val Thr Leu Asn Asp Gln
        35                  40                  45
Ile Glu Lys Ala Tyr Arg Glu Ala Leu Glu Ala Gly Thr Ser Pro Asn
    50                  55                  60
Gly Leu Arg Lys Gly Gln Lys Phe Gly Ser Arg Tyr Phe Asn Val Ile
65                  70                  75                  80
Leu Lys Arg Gly Glu Glu Asn Ile Lys Val Glu Phe Phe Val Gly Ile
                85                  90                  95
Asn Tyr Leu Lys Glu Ile Ile Tyr Leu Gln Ala Tyr Val Gln Ser Val
            100                 105                 110
Leu Leu Asp Cys Tyr Pro Thr Thr Glu Arg Pro Gln Leu Asn Ile Ile
        115                 120                 125
Leu His
    130

<210> 256
<211> 445
<212> DNA
<213> Blumeria graminis

<400> 256
atgagaacct tcagtcttgt gtcactggcg gctattttaa attatctaac gcttccatct      60

gccgttggga gtggccctgt cgctaacgct tcaagttaca agtgtcatga cagggtcatt     120

ggtccggtta ccttaaatga tcagatagaa aaggcatata gagaagcact agaagcaggc     180

acttcaccta acggactaag gaaaggacag aaatttggtt caagatactt taatgtaatc     240

ttaaaaagag gagaaggtat gaagtttcat tccaaaaaac tttatttcct actaattaat     300

atcttcagaa aatattaaag tcgaattttt tgtgggaatc aactatttga aggaaattat     360

ttatctacag gcttatgtcc agagcgtatt gctagattgt tatccaacaa cagagaggcc     420

tcaattaaac ataattctgc actag                                           445

<210> 257
<211> 4245
<212> DNA
<213> Artificial Sequence

<220>
<223> cDNA

<400> 257
atggcagagc gggtggtcac catggcgatc gggccactgg tgtccatgct gaaggacaag      60

gcgtccagct acctcctgga ccagtacaag gtcatggagg gaatggagga gcagcacaag     120

attctcaaac gcaagcttcc ggccatcctc gacgtcatca ccgatgtcga ggagcaggcc     180

atggcacaga gagaaggtgc caaagcctgg cttcaggagc tcaggacagt cgcctatgtg     240

gcaaatgaag tcttcgacga attcaagtac gaagccctac gccgtgaagc caagaagaat     300

gggcactaca taaagctcgg cttcgatgta attaaactct tccctactca caaccgtgtt     360

gcgttccgtt acaaaatggg tcgcaagctt tgcctgattc tgcaagccgt tgaggtcctc     420

atagcagaga tgcaggtctt tgggttcaag taccaaccac agccaccggt gtccaaagag     480

tggaggcata cagattatgt tagcattgac ccacaagaaa ttgccagcag atccagacac     540

gaagataaga agaacattat tggtatacta gttgatgaag ctagcaatgc agatctcaca     600

gttgttcctg tggttgcaat gggggccctt ggcaagacca cattagcgca actcatatac     660

aatgatcctg aaattcagaa gcatttccag ttgctgctct gggtttgtgt ctctgatacc     720

tttgatgtga actccctggc caagagtata gttgaagcat cccccaataa gaatgttgat     780

acagacaaac caccattgga tagacttcaa aaactggtca gcggacagcg gtatctcctt     840

gtattggatg atgtttggga caacaaagag ttacgtaagt gggaaaggct gaaggtatgt     900

ctccagcatg gtggcatggg cagtgcagtg ttgacaacaa ctcgtgataa acgagtttct     960

gaaattatgg gtgcagatag agctgcctac aatctcaatg ctttggagga tcacttcata    1020

aaggaaatta ttgaggctag agcgttcagt tcaaagaaag aaaagcctat tgaactagtg    1080

gaggtagttg acgagattgt gaagagatgt tgtggctctc cattagctgc aacggcactg 1140

ggctctgtac tttgtaccaa gaccagtgtg aaagaatgga aagctgtatc atctggaacc 1200

agcgtttgca ctgatgaaac tggaattttg ccaatactca agcttagcta caacgacttg 1260

cccgcacaca tgaagcagtg ctttgctttc tgtgctgtgt ttcccaagga ttacaagatc 1320

aatgtggaga agcttatcca actatggatt gcaaacggct ttatcctaga atacaaggaa 1380

gatagtcccg aaacatttgg aaaacatatt ttcgatgagc ttgtctcaag gtcattcttt 1440

ctggacctag aggaaagtaa ggactacagc gggtattatt ccagcacatg taaaattcat 1500

gatcttatgc atgatattgc aatgtctgtt atggaaaagg aatgtgttgt tgcaactatg 1560

gaaccaagtg aaattgagtg gcttccagat actgctcggc atttgttctt gtcatgtgaa 1620

gaagcagaac gtattttgaa tgattctatg gaggaaagat cccctgctat tcaaacattg 1680

ctatgtgata gtaatgtgtt cagcccattg aagcatctat caaaatatag ctctttgcac 1740

gccttgaagc tctgtattag aggcacagaa tcatttctac tcaaaccaaa gtatctgcat 1800

cacctgaggt accttgatct ctcagaaagt cgtatgaaag cacttcctga ggatataagt 1860

attctatata acctgcaagt gttggacctt tcctactgta attatcttga tcgacttcca 1920

aggcaaatga agtatatgac ttccctctgc cacctctaca ctcatggatg tcggaacttg 1980

aagagcatgc ctccaggact tgaaaatctc actaagctgc agacactcac agtttttgta 2040

gcaggagttc ctggccctga ttgcgctgat gttggagagc tgcatggtct aaacattggt 2100

ggtcggctag agctatgcca ggtagagaat gttgaaaaag cagaagcaga agtggcaaac 2160

cttggtggtc agctagagct gcagcattta aaccttggtg atcagctaga gctacgccgg 2220

gtagagaatg ttaaaaaagc agaggcaaaa gtggcgaatc ttggaaacaa gaaggatctc 2280

cgtgaactga cattaagatg gactgaggtt ggcgacagca aggtgctcga caagttcgaa 2340

cctcatggtg ggctgcaggt tctgaagata tataaatatg gagggaagtg tatgggtatg 2400

ttgcaaaaca tggttgagat ccatctttct ggttgtgaaa gattgcaggt tttgttcagt 2460

tgtggtacat ccttcacgtt tccaaaactg aaggtgctta cactagagca tttattggat 2520

tttgagagat ggtgggaaat aaatgaggca caagaagaac agataatatt tcctctgctt 2580

gagaagttgt ttattcggca ttgtggaaag ctgatagcat tacctgaagc accattgctt 2640

ggagaaccaa gtcgtggagg taatagactg gtatgcacac cattttctct gcttgagaac 2700

ttgtttattt ggtattgtgg aaagctggta ccattgcgtg aagcaaggtt ggttcatgaa 2760

aattgtagtg gaggttatag attggtacag tcagcatttc ctgctctaaa ggtacttgca 2820

ttggaagact tggagagttt tcagaaatgg gatgcagcta tcgaaggaga accgatattg 2880

tttcctcaac ttgagacact atcagttcag aaatgcccaa agctagtaga tttacccgaa 2940

gcaccaaaac tcagtgtact agtaattgaa gatggcaagc aagaggtgtt ccattttgta 3000

gacaggtatt tatcttcatt gaccaatctg acactgaggc tagaacacag agaaacaaca 3060

tcagaggctg aatgcacttc aattgtacct gtggacagca aagagaaatg gaaccagaaa 3120

tcccctctta cagttctgga gttaggatgc tgcaactcat tctttggacc aggtgcacta 3180

gagccgtggg actattttgt acaccttgaa aagttggaaa ttgatagatg tgatgtgctc 3240

gtccactggc cagagaatgt gttccaaagc ttggtatcct tgaggacatt actgattaga 3300

aactgcaaaa atctgactgg atatgcacaa gctcctcttg agccgttggc gtctgaaagg 3360

agtcagcacc cgagaggtct ggagtctctt tgcttaagaa actgcccaag tttagtagag 3420

atgttcaacg tcccggcatc tctcaagaaa atgactattg gtgggtgcat taagcttgag 3480

tccatattcg gcaagcaaca gggcatggca gagttagtcc aagtatcttc tagcagtgag 3540

gcaatcatgc ctgcaactgt atcagagttg ccatccacac ccatgaatca cttttgtcca 3600

tgcctagaag atctatgctt atcagcatgt ggaagcttac cagcggttct aaatctgcct 3660

ccatccttaa agaccttaga aatggatcgt tgtagtagta ttcaagtcct atcatgccag 3720

ctgggtgggc tccagaaacc agaagccact acctccagaa gcagaagtcc tatcatgcca 3780

cagccactag cagcagcaac agcaccagct gcaagagagc atttacttcc tccccatctc 3840

gaatatctaa caatactgaa ctgtgctggc atgttgggtg ggactctccg tctgcctgca 3900

cccctcaaga gactgttcat tatgggcaac agtgggctga catcgctgga gtgtctgtcg 3960

ggagagcacc ccccatcgct gaaaatcctt gatcttagaa gctgcagtac cctggcatcc 4020

ctgccgaatg agccgcaagt atacaggtct ctctggtctc ttgaaattac aggctgccct 4080

gctataaaga agctccctag atgcctgcag cagcaactgg gcagcatcaa acgcaaatgg 4140

ctagatgccc gttatgaagt aacggaattc aaaccattga aaccgaagac atggaaggaa 4200

ataccgaggc tagtccgtga gcggaggcag gcctgccgga gctga 4245

<210> 258
<211> 1414
<212> PRT
<213> Triticum aestivum

<400> 258
Met Ala Glu Arg Val Val Thr Met Ala Ile Gly Pro Leu Val Ser Met
1               5                   10                  15
Leu Lys Asp Lys Ala Ser Ser Tyr Leu Leu Asp Gln Tyr Lys Val Met
            20                  25                  30
Glu Gly Met Glu Glu Gln His Lys Ile Leu Lys Arg Lys Leu Pro Ala
        35                  40                  45
Ile Leu Asp Val Ile Thr Asp Val Glu Glu Gln Ala Met Ala Gln Arg
    50                  55                  60
Glu Gly Ala Lys Ala Trp Leu Gln Glu Leu Arg Thr Val Ala Tyr Val
65                  70                  75                  80
Ala Asn Glu Val Phe Asp Glu Phe Lys Tyr Glu Ala Leu Arg Arg Glu

                85                  90                  95
Ala Lys Lys Asn Gly His Tyr Ile Lys Leu Gly Phe Asp Val Ile Lys
            100                 105                 110
Leu Phe Pro Thr His Asn Arg Val Ala Phe Arg Tyr Lys Met Gly Arg
        115                 120                 125
Lys Leu Cys Leu Ile Leu Gln Ala Val Glu Val Leu Ile Ala Glu Met
    130                 135                 140
Gln Val Phe Gly Phe Lys Tyr Gln Pro Gln Pro Pro Val Ser Lys Glu
145                 150                 155                 160
Trp Arg His Thr Asp Tyr Val Ser Ile Asp Pro Gln Glu Ile Ala Ser
                165                 170                 175
Arg Ser Arg His Glu Asp Lys Lys Asn Ile Ile Gly Ile Leu Val Asp
            180                 185                 190
Glu Ala Ser Asn Ala Asp Leu Thr Val Val Pro Val Val Ala Met Gly
        195                 200                 205
Gly Leu Gly Lys Thr Thr Leu Ala Gln Leu Ile Tyr Asn Asp Pro Glu
    210                 215                 220
Ile Gln Lys His Phe Gln Leu Leu Leu Trp Val Cys Val Ser Asp Thr
225                 230                 235                 240
Phe Asp Val Asn Ser Leu Ala Lys Ser Ile Val Glu Ala Ser Pro Asn
                245                 250                 255
Lys Asn Val Asp Thr Asp Lys Pro Pro Leu Asp Arg Leu Gln Lys Leu
            260                 265                 270
Val Ser Gly Gln Arg Tyr Leu Leu Val Leu Asp Asp Val Trp Asp Asn
        275                 280                 285
Lys Glu Leu Arg Lys Trp Glu Arg Leu Lys Val Cys Leu Gln His Gly
    290                 295                 300
Gly Met Gly Ser Ala Val Leu Thr Thr Thr Arg Asp Lys Arg Val Ser
305                 310                 315                 320
Glu Ile Met Gly Ala Asp Arg Ala Ala Tyr Asn Leu Asn Ala Leu Glu
                325                 330                 335
Asp His Phe Ile Lys Glu Ile Ile Glu Ala Arg Ala Phe Ser Ser Lys
            340                 345                 350
Lys Glu Lys Pro Ile Glu Leu Val Glu Val Val Asp Glu Ile Val Lys
        355                 360                 365
Arg Cys Cys Gly Ser Pro Leu Ala Ala Thr Ala Leu Gly Ser Val Leu
    370                 375                 380
Cys Thr Lys Thr Ser Val Lys Glu Trp Lys Ala Val Ser Ser Gly Thr
385                 390                 395                 400
Ser Val Cys Thr Asp Glu Thr Gly Ile Leu Pro Ile Leu Lys Leu Ser
                405                 410                 415
Tyr Asn Asp Leu Pro Ala His Met Lys Gln Cys Phe Ala Phe Cys Ala
            420                 425                 430
Val Phe Pro Lys Asp Tyr Lys Ile Asn Val Glu Lys Leu Ile Gln Leu
        435                 440                 445
Trp Ile Ala Asn Gly Phe Ile Leu Glu Tyr Lys Glu Asp Ser Pro Glu
    450                 455                 460
Thr Phe Gly Lys His Ile Phe Asp Glu Leu Val Ser Arg Ser Phe Phe
465                 470                 475                 480
Leu Asp Leu Glu Glu Ser Lys Asp Tyr Ser Gly Tyr Tyr Ser Ser Thr
                485                 490                 495
Cys Lys Ile His Asp Leu Met His Asp Ile Ala Met Ser Val Met Glu
            500                 505                 510
Lys Glu Cys Val Val Ala Thr Met Glu Pro Ser Glu Ile Glu Trp Leu
        515                 520                 525
Pro Asp Thr Ala Arg His Leu Phe Leu Ser Cys Glu Glu Ala Glu Arg
    530                 535                 540
Ile Leu Asn Asp Ser Met Glu Glu Arg Ser Pro Ala Ile Gln Thr Leu
545                 550                 555                 560
Leu Cys Asp Ser Asn Val Phe Ser Pro Leu Lys His Leu Ser Lys Tyr
                565                 570                 575
Ser Ser Leu His Ala Leu Lys Leu Cys Ile Arg Gly Thr Glu Ser Phe
            580                 585                 590

Leu Leu Lys Pro Lys Tyr Leu His His Leu Arg Tyr Leu Asp Leu Ser
        595                 600                 605
Glu Ser Arg Met Lys Ala Leu Pro Glu Asp Ile Ser Ile Leu Tyr Asn
    610                 615                 620
Leu Gln Val Leu Asp Leu Ser Tyr Cys Asn Tyr Leu Asp Arg Leu Pro
625                 630                 635                 640
Arg Gln Met Lys Tyr Met Thr Ser Leu Cys His Leu Tyr Thr His Gly
                645                 650                 655
Cys Arg Asn Leu Lys Ser Met Pro Pro Gly Leu Glu Asn Leu Thr Lys
            660                 665                 670
Leu Gln Thr Leu Thr Val Phe Val Ala Gly Val Pro Gly Pro Asp Cys
        675                 680                 685
Ala Asp Val Gly Glu Leu His Gly Leu Asn Ile Gly Gly Arg Leu Glu
    690                 695                 700
Leu Cys Gln Val Glu Asn Val Glu Lys Ala Glu Ala Glu Val Ala Asn
705                 710                 715                 720
Leu Gly Gly Gln Leu Glu Leu Gln His Leu Asn Leu Gly Asp Gln Leu
                725                 730                 735
Glu Leu Arg Arg Val Glu Asn Val Lys Lys Ala Glu Ala Lys Val Ala
            740                 745                 750
Asn Leu Gly Asn Lys Lys Asp Leu Arg Glu Leu Thr Leu Arg Trp Thr
        755                 760                 765
Glu Val Gly Asp Ser Lys Val Leu Asp Lys Phe Glu Pro His Gly Gly
    770                 775                 780
Leu Gln Val Leu Lys Ile Tyr Lys Tyr Gly Gly Lys Cys Met Gly Met
785                 790                 795                 800
Leu Gln Asn Met Val Glu Ile His Leu Ser Gly Cys Glu Arg Leu Gln
                805                 810                 815
Val Leu Phe Ser Cys Gly Thr Ser Phe Thr Phe Pro Lys Leu Lys Val
            820                 825                 830
Leu Thr Leu Glu His Leu Leu Asp Phe Glu Arg Trp Trp Glu Ile Asn
        835                 840                 845
Glu Ala Gln Glu Glu Gln Ile Ile Phe Pro Leu Leu Glu Lys Leu Phe
    850                 855                 860
Ile Arg His Cys Gly Lys Leu Ile Ala Leu Pro Glu Ala Pro Leu Leu
865                 870                 875                 880
Gly Glu Pro Ser Arg Gly Gly Asn Arg Leu Val Cys Thr Pro Phe Ser
                885                 890                 895
Leu Leu Glu Asn Leu Phe Ile Trp Tyr Cys Gly Lys Leu Val Pro Leu
            900                 905                 910
Arg Glu Ala Arg Leu Val His Glu Asn Cys Ser Gly Gly Tyr Arg Leu
        915                 920                 925
Val Gln Ser Ala Phe Pro Ala Leu Lys Val Leu Ala Leu Glu Asp Leu
    930                 935                 940
Glu Ser Phe Gln Lys Trp Asp Ala Ala Ile Glu Gly Glu Pro Ile Leu
945                 950                 955                 960
Phe Pro Gln Leu Glu Thr Leu Ser Val Gln Lys Cys Pro Lys Leu Val
                965                 970                 975
Asp Leu Pro Glu Ala Pro Lys Leu Ser Val Leu Val Ile Glu Asp Gly
            980                 985                 990
Lys Gln Glu Val Phe His Phe Val Asp Arg Tyr Leu Ser Ser Leu Thr
        995                 1000                1005
Asn Leu Thr Leu Arg Leu Glu His Arg Glu Thr Thr Ser Glu Ala Glu
    1010                1015                1020
Cys Thr Ser Ile Val Pro Val Asp Ser Lys Glu Lys Trp Asn Gln Lys
1025                1030                1035                1040
Ser Pro Leu Thr Val Leu Glu Leu Gly Cys Cys Asn Ser Phe Phe Gly
                1045                1050                1055
Pro Gly Ala Leu Glu Pro Trp Asp Tyr Phe Val His Leu Glu Lys Leu
            1060                1065                1070
Glu Ile Asp Arg Cys Asp Val Leu Val His Trp Pro Glu Asn Val Phe
        1075                1080                1085
Gln Ser Leu Val Ser Leu Arg Thr Leu Leu Ile Arg Asn Cys Lys Asn

1090                1095                1100
Leu Thr Gly Tyr Ala Gln Ala Pro Leu Glu Pro Leu Ala Ser Glu Arg
1105                1110                1115                1120
Ser Gln His Pro Arg Gly Leu Glu Ser Leu Cys Leu Arg Asn Cys Pro
                1125                1130                1135
Ser Leu Val Glu Met Phe Asn Val Pro Ala Ser Leu Lys Lys Met Thr
            1140                1145                1150
Ile Gly Gly Cys Ile Lys Leu Glu Ser Ile Phe Gly Lys Gln Gln Gly
        1155                1160                1165
Met Ala Glu Leu Val Gln Val Ser Ser Ser Ser Glu Ala Ile Met Pro
    1170                1175                1180
Ala Thr Val Ser Glu Leu Pro Ser Thr Pro Met Asn His Phe Cys Pro
1185                1190                1195                1200
Cys Leu Glu Asp Leu Cys Leu Ser Ala Cys Gly Ser Leu Pro Ala Val
                1205                1210                1215
Leu Asn Leu Pro Pro Ser Leu Lys Thr Leu Glu Met Asp Arg Cys Ser
            1220                1225                1230
Ser Ile Gln Val Leu Ser Cys Gln Leu Gly Gly Leu Gln Lys Pro Glu
        1235                1240                1245
Ala Thr Thr Ser Arg Ser Arg Ser Pro Ile Met Pro Gln Pro Leu Ala
    1250                1255                1260
Ala Ala Thr Ala Pro Ala Ala Arg Glu His Leu Leu Pro Pro His Leu
1265                1270                1275                1280
Glu Tyr Leu Thr Ile Leu Asn Cys Ala Gly Met Leu Gly Gly Thr Leu
                1285                1290                1295
Arg Leu Pro Ala Pro Leu Lys Arg Leu Phe Ile Met Gly Asn Ser Gly
            1300                1305                1310
Leu Thr Ser Leu Glu Cys Leu Ser Gly Glu His Pro Pro Ser Leu Lys
        1315                1320                1325
Ile Leu Asp Leu Arg Ser Cys Ser Thr Leu Ala Ser Leu Pro Asn Glu
    1330                1335                1340
Pro Gln Val Tyr Arg Ser Leu Trp Ser Leu Glu Ile Thr Gly Cys Pro
1345                1350                1355                1360
Ala Ile Lys Lys Leu Pro Arg Cys Leu Gln Gln Gln Leu Gly Ser Ile
                1365                1370                1375
Lys Arg Lys Trp Leu Asp Ala Arg Tyr Glu Val Thr Glu Phe Lys Pro
            1380                1385                1390
Leu Lys Pro Lys Thr Trp Lys Glu Ile Pro Arg Leu Val Arg Glu Arg
        1395                1400                1405
Arg Gln Ala Cys Arg Ser
    1410

<210> 259
<211> 4445
<212> DNA
<213> Triticum aestivum


<400> 259
atggcagagc gggtggtcac catggcgatc gggccactgg tgtccatgct gaaggacaag      60

gcgtccagct acctcctgga ccagtacaag gtcatggagg gaatggagga gcagcacaag     120

attctcaaac gcaagcttcc ggccatcctc gacgtcatca ccgatgtcga ggagcaggcc     180

atggcacaga gagaaggtgc caaagcctgg cttcaggagc tcaggacagt cgcctatgtg     240

gcaaatgaag tcttcgacga attcaagtac gaagccctac gccgtgaagc caagaagaat     300

gggcactaca taaagctcgg cttcgatgta attaaactct tccctactca caaccgtgtt     360

gcgttccgtt acaaaatggg tcgcaagctt tgcctgattc tgcaagccgt tgaggtcctc     420

atagcagaga tgcaggtctt tgggttcaag taccaaccac agccaccggt gtccaaagag 480
tggaggcata cagattatgt tagcattgac ccacaagaaa ttgccagcag atccagacac 540
gaagataaga agaacattat tggtatacta gttgatgaag ctagcaatgc agatctcaca 600
gttgttcctg tggttgcaat gggggccctt ggcaagacca cattagcgca actcatatac 660
aatgatcctg aaattcagaa gcatttccag ttgctgctct gggtttgtgt ctctgatacc 720
tttgatgtga actccctggc caagagtata gttgaagcat cccccaataa gaatgttgat 780
acagacaaac caccattgga tagacttcaa aaactggtca gcggacagcg gtatctcctt 840
gtattggatg atgtttggga caacaaagag ttacgtaagt gggaaaggct gaaggtatgt 900
ctccagcatg gtggcatggg cagtgcagtg ttgacaacaa ctcgtgataa acgagtttct 960
gaaattatgg gtgcagatag agctgcctac aatctcaatg ctttggagga tcacttcata 1020
aaggaaatta ttgaggctag agcgttcagt tcaaagaaag aaaagcctat tgaactagtg 1080
gaggtagttg acgagattgt gaagagatgt tgtggctctc cattagctgc aacggcactg 1140
ggctctgtac tttgtaccaa gaccagtgtg aaagaatgga aagctgtatc atctggaacc 1200
agcgtttgca ctgatgaaac tggaattttg ccaatactca agcttagcta caacgacttg 1260
cccgcacaca tgaagcagtg ctttgctttc tgtgctgtgt ttcccaagga ttacaagatc 1320
aatgtggaga agcttatcca actatggatt gcaaacggct ttatcctaga atacaaggaa 1380
gatagtcccg aaacatttgg aaaacatatt ttcgatgagc ttgtctcaag gtcattcttt 1440
ctggacctag aggaaagtaa ggactacagc gggtattatt ccagcacatg taaaattcat 1500
gatcttatgc atgatattgc aatgtctgtt atggaaaagg aatgtgttgt tgcaactatg 1560
gaaccaagtg aaattgagtg gcttccagat actgctcggc atttgttctt gtcatgtgaa 1620
gaagcagaac gtattttgaa tgattctatg gaggaaagat cccctgctat tcaaacattg 1680
ctatgtgata gtaatgtgtt cagcccattg aagcatctat caaaatatag ctctttgcac 1740
gccttgaagc tctgtattag aggcacagaa tcatttctac tcaaaccaaa gtatctgcat 1800
cacctgaggt accttgatct ctcagaaagt cgtatgaaag cacttcctga ggatataagt 1860
attctatata acctgcaagt gttggacctt tcctactgta attatcttga tcgacttcca 1920
aggcaaatga agtatatgac ttccctctgc cacctctaca ctcatggatg tcggaacttg 1980
aagagcatgc ctccaggact tgaaaatctc actaagctgc agacactcac agtttttgta 2040
gcaggagttc ctggccctga ttgcgctgat gttggagagc tgcatggtct aaacattggt 2100
ggtcggctag agctatgcca ggtagagaat gttgaaaaag cagaagcaga agtggcaaac 2160
cttggtggtc agctagagct gcagcattta aaccttggtg atcagctaga gctacgccgg 2220
gtagagaatg ttaaaaaagc agaggcaaaa gtggcgaatc ttggaaacaa gaaggatctc 2280
cgtgaactga cattaagatg gactgaggtt ggcgacagca aggtgctcga caagttcgaa 2340

cctcatggtg ggctgcaggt tctgaagata tataaatatg gagggaagtg tatgggtatg 2400
ttgcaaaaca tggttgagat ccatctttct ggttgtgaaa gattgcaggt tttgttcagt 2460
tgtggtacat ccttcacgtt tccaaaactg aaggtgctta cactagagca tttattggat 2520
tttgagagat ggtgggaaat aaatgaggca caagaagaac agataatatt tcctctgctt 2580
gagaagttgt ttattcggca ttgtggaaag ctgatagcat tacctgaagc accattgctt 2640
ggagaaccaa gtcgtggagg taatagactg gtatgcacac cattttctct gcttgagaac 2700
ttgtttattt ggtattgtgg aaagctggta ccattgcgtg aagcaaggtt ggttcatgaa 2760
aattgtagtg gaggttatag attggtacag tcagcatttc ctgctctaaa ggtacttgca 2820
ttggaagact tggagagttt tcagaaatgg gatgcagcta tcgaaggaga accgatattg 2880
tttcctcaac ttgagacact atcagttcag aaatgcccaa agctagtaga tttacccgaa 2940
gcaccaaaac tcagtgtact agtaattgaa gatggcaagc aagaggtgtt ccattttgta 3000
gacaggtatt tatcttcatt gaccaatctg acactgaggc tagaacacag agaaacaaca 3060
tcagaggctg aatgcacttc aattgtacct gtggacagca aagagaaatg gaaccagaaa 3120
tcccctctta cagttctgga gttaggatgc tgcaactcat tctttggacc aggtgcacta 3180
gagccgtggg actattttgt acaccttgaa aagttggaaa ttgatagatg tgatgtgctc 3240
gtccactggc cagagaatgt gttccaaagc ttggtatcct tgaggacatt actgattaga 3300
aactgcaaaa atctgactgg atatgcacaa gctcctcttg agccgttggc gtctgaaagg 3360
agtcagcacc cgagaggtct ggagtctctt tgcttaagaa actgcccaag tttagtagag 3420
atgttcaacg tcccggcatc tctcaagaaa atgactattg gtgggtgcat taagcttgag 3480
tccatattcg gcaagcaaca gggcatggca gagttagtcc aagtatcttc tagcagtgag 3540
gcaatcatgc ctgcaactgt atcagagttg ccatccacac ccatgaatca cttttgtcca 3600
tgcctagaag atctatgctt atcagcatgt ggaagcttac cagcggttct aaatctgcct 3660
ccatccttaa agaccttaga aatggatcgt tgtagtagta ttcaagtcct atcatgccag 3720
ctgggtgggc tccagaaacc agaagccact acctccagaa gcagaagtcc tatcatgcca 3780
cagccactag cagcagcaac agcaccagct gcaagagagc atttacttcc tccccatctc 3840
gaatatctaa caatactgaa ctgtgctggc atgttgggtg ggactctccg tctgcctgca 3900
cccctcaaga gactgttcat tatgggcaac agtgggctga catcgctgga gtgtctgtcg 3960
ggagagcacc ccccatcgct gaaaatcctt gatcttagaa gctgcagtac cctggcatcc 4020
ctgccgaatg agccgcaagt atacaggtct ctctggtctc ttgaaattac aggctgccct 4080
gctataaaga agctccctag atgcctgcag cagcaactgg gcagcatcaa acgcaaatgg 4140
ctagatgccc gttatgaagg tatgcttgca gcacgtcctt ctatcttggc cacatttaat 4200

aaacagatgc agtcatccat tccatccctt gtacataatg gtcaacacgt ttgcaaaagt 4260

ttcttctcca ttatttgaat gccatcatgc tatacattat gatataacac attagtttgt 4320

acaagttttt ttttctgaca tcgcttgtgg tatgtgcagt aacggaattc aaaccattga 4380

aaccgaagac atggaaggaa ataccgaggc tagtccgtga gcggaggcag gcctgccgga 4440

gctga 4445

<210> 260
<211> 564
<212> DNA
<213> Beta vulgaris

<220>
<223> Bv123_#187

<400> 260
atgggaataa taggagaaac aattctatca gcatttctac aagtgctgtt tgagaagtta 60

ttttcccgag gagtaaattt gtttatgggg ttagtgaaag gaggaatcca acaaaaaacc 120

atcaaacaat gggagaaaca actaagaatt atagaggcag ttttaagtga tgctgagcaa 180

aagcaagtcc aaagcaatgc tgtgaagtca tggatgcagg acctccaaga tttggcgtat 240

gacttagaag atgtgttgga tgcatttgca actcaagctc aaatttacga attgtcgatc 300

atgaatgata gtcatagctc cgaattgaaa gataatgaca ccaagtttat ggcggaaagc 360

attattgttg gcggttcaaa agctatagag catttatctt ttatgtttaa ctctggccaa 420

gatttttcgg ggaggataga agagattagt caacaattgg aagaaatttt aagccaaacc 480

gagggtttag ggctatcagt tcatgtgatg catcagcaag tagtacccac aacaacagca 540

caacgttggc aaagctggcg atga 564

<210> 261
<211> 187
<212> PRT
<213> Beta vulgaris

<220>
<223> Bv123_#187

<400> 261
Met Gly Ile Ile Gly Glu Thr Ile Leu Ser Ala Phe Leu Gln Val Leu
1               5                   10                  15
Phe Glu Lys Leu Phe Ser Arg Gly Val Asn Leu Phe Met Gly Leu Val
            20                  25                  30
Lys Gly Gly Ile Gln Gln Lys Thr Ile Lys Gln Trp Glu Lys Gln Leu
        35                  40                  45
Arg Ile Ile Glu Ala Val Leu Ser Asp Ala Glu Gln Lys Gln Val Gln
    50                  55                  60
Ser Asn Ala Val Lys Ser Trp Met Gln Asp Leu Gln Asp Leu Ala Tyr
65                  70                  75                  80
Asp Leu Glu Asp Val Leu Asp Ala Phe Ala Thr Gln Ala Gln Ile Tyr

85                  90                  95
Glu Leu Ser Ile Met Asn Asp Ser His Ser Ser Glu Leu Lys Asp Asn
            100                 105                 110
Asp Thr Lys Phe Met Ala Glu Ser Ile Ile Val Gly Gly Ser Lys Ala
        115                 120                 125
Ile Glu His Leu Ser Phe Met Phe Asn Ser Gly Gln Asp Phe Ser Gly
    130                 135                 140
Arg Ile Glu Glu Ile Ser Gln Gln Leu Glu Glu Ile Leu Ser Gln Thr
145                 150                 155                 160
Glu Gly Leu Gly Leu Ser Val His Val Met His Gln Gln Val Val Pro
                165                 170                 175
Thr Thr Thr Ala Gln Arg Trp Gln Ser Trp Arg
            180                 185

<210> 262
<211> 531
<212> DNA
<213> Beta vulgaris

<220>
<223> Bv165_#176

<400> 262
atggtgatag gcggagagat ttttctatca gcgtttcttc aagtgctctt tgaaaagctt      60

gcatcaggag ggataagtct attcctaaaa agagaaaaag gaataggacc taaagttatt     120

caaagatgga acaaaaaatt gagattaata gaggcggttt taagtgacgc cgagcaaaag     180

cagtttcaca acaatgccgt caaattgtgg cttcgagatc ttcaagagtt cgcgtacgat     240

ttagaggaca tcttagacga atttgataca gatgctcgac ttaaggagtt caatgatcag     300

cctcagccac agccacagga acatcaaccc aaatcttctt gttctccttt caataaggta     360

caaagctgtc tttcttgtgg tttcccaact ttaaataaga aaactacaaa gtattcaact     420

agcattgaag agatgagcac tcgcttggaa gatcttctgg accaagtaag gtctttggct     480

ctctcaacgc agatacactc aagggtagag caacatgaag aacggaggtg a              531

<210> 263
<211> 176
<212> PRT
<213> Beta vulgaris

<220>
<223> Bv165_#176

<400> 263
Met Val Ile Gly Gly Glu Ile Phe Leu Ser Ala Phe Leu Gln Val Leu
1               5                   10                  15
Phe Glu Lys Leu Ala Ser Gly Gly Ile Ser Leu Phe Leu Lys Arg Glu
            20                  25                  30
Lys Gly Ile Gly Pro Lys Val Ile Gln Arg Trp Asn Lys Lys Leu Arg
        35                  40                  45
Leu Ile Glu Ala Val Leu Ser Asp Ala Glu Gln Lys Gln Phe His Asn
    50                  55                  60
Asn Ala Val Lys Leu Trp Leu Arg Asp Leu Gln Glu Phe Ala Tyr Asp
65                  70                  75                  80

Leu Glu Asp Ile Leu Asp Glu Phe Asp Thr Asp Ala Arg Leu Lys Glu
                85                  90                  95
Phe Asn Asp Gln Pro Gln Pro Gln Pro Gln Glu His Gln Pro Lys Ser
            100                 105                 110
Ser Cys Ser Pro Phe Asn Lys Val Gln Ser Cys Leu Ser Cys Gly Phe
        115                 120                 125
Pro Thr Leu Asn Lys Lys Thr Thr Lys Tyr Ser Thr Ser Ile Glu Glu
    130                 135                 140
Met Ser Thr Arg Leu Glu Asp Leu Leu Asp Gln Val Arg Ser Leu Ala
145                 150                 155                 160
Leu Ser Thr Gln Ile His Ser Arg Val Glu Gln His Glu Glu Arg Arg
                165                 170                 175

<210> 264
<211> 468
<212> DNA
<213> Solanum tuberosum

<220>
<223> StR3a-#1-155

<400> 264
atggagattg gcttagcagt tggtggtgca tttctctctt cagctttgaa tgttctcttt      60

gataggcttg ctcctcacgg tgatctgctc aacatgtttc agaagcataa ggatcatgtt     120

aagctcttaa agaagctgga ggacattttg ctcggtcttc agattgtgct aagtgatgca     180

gagaataaac aagcatcaaa tcgacatgtg agccagtggt tcaataagct tcagaatgct     240

gtggacggtg ctgagaactt gatagaacaa gtcaattatg aagctttgag gcttaaggtg     300

gaaggccagc atcaaaatct tgcagaaaca agcaaccagc aagtaagtga ccttaacctg     360

tgcttcagtg atgatttctt tcttaacata aaggataagt tggaagaaac cattgaaaca     420

ttggaggtgt tggaaaagca aattggtcgc cttgtaatca ctagctag                  468

<210> 265
<211> 155
<212> PRT
<213> Solanum tuberosum

<220>
<223> StR3a-#1-155

<400> 265
Met Glu Ile Gly Leu Ala Val Gly Gly Ala Phe Leu Ser Ser Ala Leu
1               5                   10                  15
Asn Val Leu Phe Asp Arg Leu Ala Pro His Gly Asp Leu Leu Asn Met
            20                  25                  30
Phe Gln Lys His Lys Asp His Val Lys Leu Leu Lys Lys Leu Glu Asp
        35                  40                  45
Ile Leu Leu Gly Leu Gln Ile Val Leu Ser Asp Ala Glu Asn Lys Gln
    50                  55                  60
Ala Ser Asn Arg His Val Ser Gln Trp Phe Asn Lys Leu Gln Asn Ala
65                  70                  75                  80
Val Asp Gly Ala Glu Asn Leu Ile Glu Gln Val Asn Tyr Glu Ala Leu

85                  90                  95
Arg Leu Lys Val Glu Gly Gln His Gln Asn Leu Ala Glu Thr Ser Asn
            100                 105                 110
Gln Gln Val Ser Asp Leu Asn Leu Cys Phe Ser Asp Asp Phe Phe Leu
        115                 120                 125
Asn Ile Lys Asp Lys Leu Glu Glu Thr Ile Glu Thr Leu Glu Val Leu
    130                 135                 140
Glu Lys Gln Ile Gly Arg Leu Val Ile Thr Ser
145                 150                 155

<210> 266
<211> 863
<212> PRT
<213> Solanum demissum

<400> 266
   Met Ala Glu Ile Leu Leu Thr Ala Val Ile Asn Lys Ser Val Glu Ile
   1               5                   10                  15
   Ala Ala Asn Val Leu Phe Gln Gln Gly Ser Arg Leu Asn Phe Leu Lys
               20                  25                  30
   Glu Asp Ile Asp Trp Leu Gln Arg Val Leu Arg His Ile Arg Ser Tyr
           35                  40                  45
   Val Asp Asp Ala Lys Ala Lys Glu Val Gly Gly Asp Ser Arg Val Lys
       50                  55                  60
   Asn Leu Leu Lys Asp Ile Gln Glu Leu Ala Gly Asp Val Glu Asp Leu
   65                  70                  75                  80
   Leu Asp Glu Phe Leu Pro Lys Ile Gln Gln Ser Asn Lys Phe Lys Gly
                   85                  90                  95
   Ala Ile Cys Cys Leu Lys Thr Val Ser Phe Ala Asp Glu Phe Ala Val
               100                 105                 110
   Glu Ile Glu Lys Ile Arg Arg Arg Val Ala Asp Ile Asp Ser Leu Arg
           115                 120                 125
   Thr Thr Phe Asn Ile Thr Asp Thr Ser Asn Asn Asn Asn Asp Cys Ile
       130                 135                 140
   Pro Met Glu Gln Arg Arg Lys Phe Leu His Ala Asp Glu Thr Glu Val
   145                 150                 155                 160
   Ile Gly Leu Asp Asp Asp Phe Asn Lys Leu Gln Asp Lys Leu Leu Val
                   165                 170                 175
   Gln Asp Leu Cys Asn Gly Val Val Ser Ile Val Gly Met Pro Gly Leu
               180                 185                 190
   Gly Lys Thr Thr Leu Ala Lys Lys Leu Tyr Arg His Val Arg His Gln
           195                 200                 205
   Phe Glu Cys Ser Ala Leu Val Tyr Val Ser Gln Gln Pro Arg Ala Gly
       210                 215                 220
   Glu Ile Leu Leu Asp Ile Ala Lys Gln Val Gly Leu Thr Asp Glu Gly
   225                 230                 235                 240
   Arg Lys Glu His Leu Glu Asp Asn Leu Arg Ser Leu Leu Glu Thr Lys
                   245                 250                 255
   Arg Tyr Val Ile Leu Leu Asp Asp Ile Trp Asp Thr Lys Ile Trp Asp
               260                 265                 270
   Ala Leu Asn Arg Val Leu Arg Pro Glu Cys Asp Ser Lys Ile Gly Ser
           275                 280                 285
   Arg Ile Ile Ile Thr Ser Arg Tyr His His Val Gly Arg Tyr Ile Gly
       290                 295                 300
   Glu Asp Phe Ser Leu His Glu Leu Gln Pro Leu Asp Ser Glu Lys Ser
   305                 310                 315                 320
   Phe Glu Leu Phe Thr Lys Lys Ile Phe Ile Phe Asp Asn Asn Asn Asn
                   325                 330                 335
   Trp Ala Asn Ala Ser Pro Val Leu Val Asp Ile Gly Lys Ser Ile Val
               340                 345                 350
   Arg Arg Cys Gly Gly Ile Pro Leu Ala Ile Val Val Thr Ala Gly Met
           355                 360                 365
   Leu Arg Ala Arg Glu Arg Thr Glu His Ala Trp Asn Arg Val Leu Glu
       370                 375                 380
   Arg Ile Gly His Asn Ile Gln Asp Gly Cys Ala Lys Ala Leu Ala Leu
   385                 390                 395                 400
   Ser Tyr Asn Asp Leu Pro Ile Ala Leu Arg Pro Cys Phe Leu Tyr Phe
                   405                 410                 415
   Gly Leu Tyr Pro Glu Asp His Glu Ile Arg Ala Phe Asp Leu Thr Asn
               420                 425                 430
   Met Trp Ile Ala Glu Lys Leu Ile Val Val Asn Ser Gly Asn Gly Arg
           435                 440                 445
   Glu Ala Glu Ser Leu Ala Asp Asp Val Leu Asn Asp Leu Val Ser Arg
       450                 455                 460
   Asn Leu Ile Gln Val Ala Lys Arg Thr Tyr Asp Gly Arg Ile Ser Ser
   465                 470                 475                 480
   Cys Arg Ile His Asp Leu Leu His Ser Leu Cys Val Asp Leu Ala Lys
                   485                 490                 495
   Glu Ser Asn Phe Phe His Thr Glu His Asn Ala Phe Gly Asp Pro Gly
               500                 505                 510
   Asn Val Ser Arg Leu Arg Arg Ile Thr Phe Tyr Ser Asp Asn Asn Ala
           515                 520                 525
   Met Asn Glu Phe Phe Arg Ser Asn Pro Lys Leu Glu Lys Leu Arg Ala
       530                 535                 540
   Leu Phe Cys Phe Thr Lys Gly Asp Ser Cys Ile Phe Ser His Leu Ala
   545                 550                 555                 560
   His His Asp Phe Lys Leu Leu Gln Val Leu Val Val Val Gln Pro Arg
                   565                 570                 575
   Lys Asn Tyr Asp Phe Ser Ile Ser Gln Ile Lys Ile Gly Asn Met Ser
               580                 585                 590
   Cys Leu Arg Tyr Leu Arg Phe Glu Gly Asp Ile Tyr Gly Lys Leu Pro
           595                 600                 605
   Asn Cys Met Val Lys Leu Lys His Leu Glu Thr Leu Asp Ile Ser Lys
       610                 615                 620
   Ser Phe Ile Ile Lys Leu Pro Thr Gly Val Trp Lys Thr Thr Gln Leu
   625                 630                 635                 640
   Arg His Leu Arg Ser Asn Gly Tyr Asn Leu Ala Pro Tyr Ser Tyr Phe
                   645                 650                 655
   Cys Ile Ser Pro Phe Phe Pro Asn Val Pro Pro Asn Asn Val Gln Thr
               660                 665                 670
   Leu Met Trp Met Asp Gly Glu Phe Phe Glu Pro Arg Trp Leu His Arg
           675                 680                 685
   Phe Ile Asn Leu Arg Lys Leu Gly Leu Gln Glu Val Ser Asp Ser Thr
       690                 695                 700
   Ile Lys Lys Leu Ser Thr Leu Ser Pro Val Pro Thr Thr Leu Glu Val
   705                 710                 715                 720
   Leu Lys Leu Ser Ser Phe Phe Ser Glu Leu Arg Glu Gln Ile Asn Leu
                   725                 730                 735
   Ser Ser Tyr Pro Asn Ile Val Lys Leu His Leu Asn Gly Arg Ile Pro
               740                 745                 750
   Leu Asn Val Ser Glu Ser Phe Pro Pro Asn Leu Val Lys Leu Thr Leu
           755                 760                 765
   Cys Asn Leu Met Val Asp Gly His Val Val Ala Val Leu Lys Lys Leu
       770                 775                 780
   Pro Lys Leu Lys Ile Leu Thr Leu His Arg Cys Arg His Asp Ala Glu
   785                 790                 795                 800
   Lys Met Asp Leu Ser Gly Asp Gly Asp Ser Phe Pro Gln Leu Glu Val
                   805                 810                 815
   Leu His Ile Lys Asp Pro Val Cys Leu Ser Glu Val Thr Cys Thr Asp
               820                 825                 830
   Asp Val Gly Met Pro Lys Leu Lys Lys Leu Leu Leu Ile Glu Arg Thr
           835                 840                 845
   Asp Ser Asn Val Arg Leu Ser Glu Arg Leu Ala Lys Leu Arg Val
       850                 855                 860

<210> 267
<211> 147
<212> PRT
<213> Phytophthora infestans

<400> 267
Met Arg Leu Ala Ile Met Leu Ser Ala Thr Ala Val Ala Ile Asn Phe
1               5                   10                  15
Ala Thr Ser Ser Ala Ile Asp Gln Thr Lys Val Leu Val Tyr Gly Thr
            20                  25                  30
Pro Ala His Tyr Ile His Asp Ser Ala Gly Arg Arg Leu Leu Arg Lys
        35                  40                  45
Asn Glu Glu Asn Glu Glu Thr Ser Glu Glu Arg Ala Pro Asn Phe Asn
    50                  55                  60
Leu Ala Asn Leu Asn Glu Glu Met Phe Asn Val Ala Ala Leu Thr Lys
65                  70                  75                  80
Arg Ala Asp Ala Lys Lys Leu Ala Lys Gln Leu Met Gly Asn Asp Lys
                85                  90                  95
Leu Ala Asp Ala Ala Tyr Ile Trp Trp Gln His Asn Arg Val Thr Leu
            100                 105                 110
Asp Gln Ile Asp Thr Phe Leu Lys Leu Ala Ser Arg Lys Thr Gln Gly
        115                 120                 125
Ala Lys Tyr Asn Gln Ile Tyr Asn Ser Tyr Met Met His Leu Gly Leu
    130                 135                 140
Thr Gly Tyr
145

<210> 268
<211> 9623
<212> DNA
<213> Solanum tuberosum

<220>
<221> allele
<222> 7619
<223> /note="n = a or c or g or t"

<400> 268
ggatccttgg acgacgttta aacctagata atatgaaggt taggatacat gtcaccatgt      60

ctctttgttt ttttactaat tcaattaact tcagaaacct tgatgcattt gcaattccct     120

ttccagattg aaagtttgta tcctgaagaa atgggaccag atgtgatgcc tctggaagac     180

tttctagtga atggccttcc cttacttgat cagaaaattg aagataggat taaacaagct     240

tctgcaaatg ggaatgttct gcgctatgtt tgcttgattg atgatgcgag gtaattcttt     300

catttcattt ttagtttgct caatggataa tggcaactgg atcttttatt cttatcacgt     360

tctgcaagtc accatacctc tagacgctta aagcaaagct tgctcactga tgggagtgtt     420

atggcaagtt aaggtactga tgagtgatga gcataacaat tactactaat taagacaagt     480

ttatcttaga agatagtctc aattcctctt aacaaatgct ttagaaaatt atgacagcag     540

tcacccactt ggacatataa gtttgagagc tatctgcaaa cctgttgtaa gttcatctgt     600

tttatgttag tcatctgatc agctgttcac ctcttgtatc cgtaaccaat taaagaatga     660

gtcagaagta taaatttgta atggcataga tggggaaaaa ttgattattg aaaacacttt     720

tgtgtacatg acattaacat ctcacatttt cagatgtgaa gttggcatcc aagaagttcc 780

aaaagattct gcattgggaa ggttaagagg aagtgataat gtggtatgca ttgttcactt 840

tcacctccca ttttgctatt tctttggtcc ctcttcttct tgttccatta ctttgttttc 900

cgttgggaat tttcttgtaa ctgctatgga ggttcaacca tatcatacgc gtcgcaatgt 960

ttaaagccct tttgtgatgt catttagtgt agtgatgtat tacttcatgc tgtgatttaa 1020

agaattgctc atcttttacc catgcctagg ctgacgcctt actttgtctt tactctttac 1080

gcatcatctg gtgtcgccct gcaaaacttt atgtagttaa ggtctttgat tctactttaa 1140

tcgtaaaatg tttcccttgt acagttgtaa ttatgaggtc ataaaaacag taagattaat 1200

gatgattgag cgtctgcaat tcgtttgtct gcaggtggag atatacagcc gttgttacga 1260

gaaacaacct ttggttatcc aaggtgctgg agcaggaaat gataccacag cagctggtgt 1320

cctagctgat atccttgaca ttcaagattt attcccttga cagagtatta tacactgaga 1380

tacaaacatt cgatcctcaa aggacaaaaa atcgactctt cttgtccctc tgcttccaga 1440

ttggagattg aattcaggct aagaaagctc acgcattttt tgaaaaattt ctcgatacac 1500

gagatgcctc gtcagcttac agtagattat tacacaattt ctctcgtaca taaccgtctc 1560

aaatggaaca gttcaaactg agtcatttgc ctcagtgctg gactagtttt taggattttt 1620

cttcagctta taatcagttt ccttgtaatg accatttctt ttataatctg aaaaactgct 1680

gatatttctt tataatattt cttcagaaaa ccttatgatt gaacaccaca atattcacct 1740

tttcggtatt taatgttcct ttatgttatg tgttcttcta tttttccctt ttttttttaa 1800

agttaatcgt caattgggac atggacccag ataaaaacca taatgacatt aatatctctc 1860

aattgttgag ataaaattat gaaaagagaa aaatcaatta ttaacctatt cctatgacta 1920

ttatttgcat cttatgagaa gagaaaagac tttgaaatat gacgttggcc ttttcctttc 1980

ttcgactgtc aactatacaa aaacaaggaa ttcagttcta ttaaattcaa gattcaaaaa 2040

tgatttcttt tttattttgg agccccgccc cccccccccc ctcccccctc ttgcttcaat 2100

tattgaattg caaaagtaat gaatagtcat gaaaccaggc tggcagactc cataatgttt 2160

ttgctactct tctttttttt ctatggctgt gagccgtaca ctgtagataa gacatagttg 2220

taccacttac aagtaagacc actagcaaaa gaaagagaga aatcattctt gatcacatat 2280

cactcttagc tcttagcatt caacaggtaa ataaagttgt tagtaattga agattgaaac 2340

atttatcttc ttaaaatcat tttgtgttct ccttgttttg atttgcagat ttcagtaatg 2400

gagattggct tagcagttgg tggtgcattt ctctcttcag ctttgaatgt tctctttgat 2460

aggcttgctc ctcacggtga tctgctcaac atgtttcaga agcataagga tcatgttaag 2520

ctcttaaaga agctggagga cattttgctc ggtcttcaga ttgtgctaag tgatgcagag 2580

aataaacaag catcaaatcg acatgtgagc cagtggttca ataagcttca gaatgctgtg 2640

gacggtgctg agaacttgat agaacaagtc aattatgaag ctttgaggct taaggtggaa 2700

ggccagcatc aaaatcttgc agaaacaagc aaccagcaag taagtgacct taacctgtgc 2760

ttcagtgatg atttctttct taacataaag gataagttgg aagaaaccat tgaaacattg 2820

gaggtgttgg aaaagcaaat tggtcgcctt ggcttaaagg aacattttgg ttcgactaaa 2880

caagaaacta gaacaccttc aacttcttta gttgatgact ctgatatctt tggaaggcag 2940

aatgatatag aggatttgat tgaccgttta ttatctgaag atgcaagtgg aaaaaagcgg 3000

actgtagttc ctattgttgg aatgggtggt ctgggtaaga caacacttgc taaagcggtt 3060

tacaatgatg agagagtgca gatacatttt ggtttgaaag cttggttttg tgtttctgag 3120

gcatttgatg ctttcagaat aacaaaaggg ttacttcaag aaattggctc atttgacttg 3180

aaggctgatg acaatcttaa tcagctacaa gtcaaattga aggaaagatt aaagggaaag 3240

aagtttctta tagttttgga tgatgtgtgg aatgacaact acaacaagtg ggatgaattg 3300

agaaatgttt ttgtacaagg agatatagga agtaagatca ttgtgacgac acgtaaagag 3360

agtgttgcct tgatgatggg aaatgagcaa attagcatgg acaatttgtc tactgaatcc 3420

tcttggtctt tatttaaaac acatgcattt gaaaacatgg gtcctatggg acatccggaa 3480

cttgaagagg tcgggaaaca aattgcagct aagtgcaaag gactgccctt agctctgaag 3540

acgctcgctg gcatgttacg ctccaaatca gaggttgaag agtggaaacg tattttgaga 3600

agtgaaatat gggagctgcc acacaatgac atattaccag cgttgatgtt gagctacaat 3660

gatcttcccg cacatttaaa gcgatgtttt tccttttgtg caatatttcc taaagattat 3720

ccctttagga aagaacaagt tattcatctg tggattgcca atggtctcgt accacaggaa 3780

gatgtaataa ttgaagattc aggcaaccaa tactttctcg agttgaggtc aagatcatta 3840

ttcgaaaggg tcccaaatcc ttctcaaggg aacacagaga atttattctt aatgcatgac 3900

cttgtcaatg atttagcgca aattgcatct tcaaaacttt gtatcaggtt ggaagagagc 3960

caaggatctc atatgttgga acaaagtcaa cacttatcat attcaatggg atatggtggt 4020

gagtttgaga aattgacacc cctctacaaa ttggagcagc tgaggacatt gcttccgaca 4080

tgtattgatc tccctgattg ttgtcaccat ctaagcaaga gggtgctaca taacatactg 4140

ccaagactaa catccttaag ggcattatcg ttgtcatgct atgagattgt tgagttgcca 4200

aatgacttgt ttatcaaatt aaagctcctc agatttttgg atatttctcg gacagagatt 4260

aaaaggttgc cagattccat ttgtgcattg tataacttag agacacttct cctgtcatct 4320

tgttatgatc ttgaggagct accgctgcag atggagaagc tgattaactt gcgtcatctt 4380

gacataagca acactcgtct cttgaagatg ccgctacatc tgagcaagtt gaaaagcctc 4440

caagtgttag tgggagccaa atttcttata ggtggtttga gaatggaaga tttgggtgaa 4500

gtacataact tgtacggatc tctatcagtt gtagagttgc aaaatgtggt tgatagaagg 4560

gaagctgtga aggcaaagat gagggagaag aatcacgtcg acaggttata tttggagtgg 4620

agtggaagta gtagtgccga caattcacaa acagaaagag acatacttga tgagctacgc 4680

ccccataaaa acataaaagt agtcaaaatc actggatata gagggacaaa ctttcccaat 4740

tggctagctg atcctttgtt tcttaagctg gtaaaattgt ctcttagaaa ctgcaagaac 4800

tgttattcat tgccagcact aggacaactc ccttttctga aattcctttc gattagagag 4860

atgcatggaa taacagaggt gacggaagaa ttctatggca gttggtcctc caaaaagcct 4920

tttaactgtc ttgagaagct tgaatttaaa gatatgccgg agtggaagca atgggaccta 4980

ctaggaagtg gagagttccc tatacttgag aagcttttga ttgaaaattg ccctgaactc 5040

agtttggaga cggtacccat ccaactttca agtttaaaaa gttttgacgt aattggttct 5100

cccttggtta taaactttcc tttaagcata ctgcccacta ccttgaagag aataaagata 5160

tctgattgcc agaaattgaa attggaacag ccaactggtg agattagtat gtttctggag 5220

gaattgacac tgattaaatg tgattgtata gatgatatat cacctgagtt actcccaaga 5280

gcacgcaaat tgtgggtaca ggattggcac aaccttacta ggtttttgat tcctactgcc 5340

actgaaactc tcgatatttg gaattgtgag aatgttgaaa tactttcggt ggcatgtggg 5400

gggacccaga tgacgtcact gactattgcc tactgtaaga agctgaagtg gctgccagaa 5460

cgtatgcagg aactccttcc atctcttaag gaactgcatc tgtctaattg tccagaaata 5520

gagtcctttc ctgaaggtgg attgcccttc aatttacaac aacttgcgat cagatattgc 5580

aagaaactgg tgaacggccg aaaggagtgg catttacaga gacgactctg tctcacagcg 5640

ttaatcatct accatgatgg cagtgacgaa gagattgttg gtggtgagaa ttgggagttg 5700

ccttcctcta ttcaaaggct taccatagtg aatctgaaaa cattaagcag ccaacatctc 5760

aaaaacctca cctctcttca atatctattt attaggggta atttacctca gattcagcca 5820

atgctggaac aaggccagtg ttcgcacctc acttcgcttc aaagtctaca aatctcctcc 5880

ctccaatcac ttcctgaatc ggcactgccc tcctccctct ctcacctgga gatctcccat 5940

tgccctaatc tccaatcact tcctgaatcg gcactgccct cctccctctc tcagctgacc 6000

atcaataatt gccctaatct ccaatcactt tccgaatcaa cactgccctc ctccctctct 6060

cagctggaga tctccttttg ccctaatctc caatatcttc cactaaaagg gatgccctct 6120

tccctctctg aactatctat ttacaaatgc ccattgctca aaccacaact agaatttgac 6180

aagggggaat actggccaaa tattgctcaa tttcccacca taaagatcga tagggaatgc 6240

atgtgatgat taaaacgaat ggctcttcaa cttatgtaag ctattctttt cccttggaag 6300

ctttttattt ccgtttactt cactttgctt ttttgttaat tcttttcatt tttaatcgct 6360

aattctaaaa acatgccttg cacgtttatc ccaaaatatt tcatataatt tttgtatagc 6420
aaatgacata ttttatcccc ttcaaatcta ttggtattta ctaaaaaaat atccatcatt 6480
tttacgattg aaagtacata tattttccta ataaaaagaa tacaccctta attataaaat 6540
tgattaaaag tgcataaagt aaaaataact aattgttgta caaatacaat attcaaatga 6600
ggatactaaa ataccacatg ttaggttaat gtatacaaaa caactaacca atgtaaattc 6660
tcaatattaa taaccataaa acaataaaaa gttatacaat aacaacaaac aagaaacata 6720
aacattaaaa aaaataaaaa taaaaagagt caagaaaaga gagcatagag attctctttt 6780
tttttcaatt tgttttaact gtgtacaata ttgtgtcaaa tgtcactatt catagaataa 6840
gattgaagaa cacttaagag ttgttatgaa tataacatta atccatttga tcatggatga 6900
gaggtggtag taaaaatcat aatgaatata attagtggga gttgcatata tttacataat 6960
gaagagttag tggtaataaa tctatgtaat atacttaaca ttaatacaaa tatttatata 7020
caaaaaagaa tataatatag atctatatga tttttttccc tttttgttaa gggcaaacat 7080
aaccattcat atttccaatt ggaagatgaa caaaacaaaa aattcatgaa tttaactcca 7140
gcatgaaatt aanaaaaaga atacatattc aagaaacttt atggtggttg attcatgtac 7200
taatatgatg atttactatt aaatatttaa tttattaaat agtttataac atttaaattt 7260
aatgtagaaa tagaatcata atctaacttt tgcctaacat tctccccact tttagtcaat 7320
tttccttttt gcattataat atggattgca cacaacaact tctatttaaa aaaggaaaga 7380
caaaattaga aaaaatagta tcaataaatt ggaatgaaaa atgtaaaatt gtaataagag 7440
gaagagaaga aaaaatcagt agaatatatt tacctaacca tgaataagga agtatgttga 7500
gtaaaagaag agtaatgcaa aagtgtgagc tgatatatgg aggaaattga ctaatataat 7560
aatctgtatt gaataatgaa aagaccaatc tttaatttct gccaaatttt tattacgagc 7620
tcaagataat ataaatttat taatttaata atgacataaa taaataacat acattgtatg 7680
tatgtgtagt taatggggta gtgaaaaaga ctctttctaa aattattttt ctctttaaag 7740
tatttttaaa cttataatat tagtgctata gaaaatgcta tagaaaatca taagctactg 7800
caagttttta tagtaattat tttattaaat tatataccta ttatttataa tcattccgga 7860
aatatataaa caattttgga aagcttgtaa aatgagaaat tattatttct tatcgttatt 7920
ttagctatta ttaacattta gtatgacaac atttaaaaag tagcctcctt taaatattaa 7980
cggtttctaa attaacgtag ccaataattt gaaatgtttt gacgttttgt ttatctaaac 8040
acgcagaatt ttgtttttga acttgaaaca tgtaattttg ccttgggtga attataattt 8100
tatttttact atttaattaa ttaaatattt tataacattt taatataatg aactggtaaa 8160
atggtaatcc aactttaaag gttggagctt cccactttta gtaatatatg atatatgata 8220
tatgatatat gatgatatat gatgttgttg tgctcatcat caaacacata gctttatatt 8280

tttgcttcat agagaatcta aattttttaa aagataatca gggataacga tcacaagttt 8340

taggaaataa gtgcaacttc cattgtcaga ataagtagac gttatataat tctatatttc 8400

ttggatagcg accagacaac tacacagggc aatgcttgat gtaagagctt cagatataca 8460

tttgtagggt gtttatccaa cgagtaataa aatcacagtc ttctgatatc ttctcttgca 8520

tatacttttg caattttaaa ctttattttg aattgtgttg ttgctaactt gaacatgttc 8580

tgtgcttaat cagatgtgga ttttgaaggg ctagtacgac aagtctggtc catccattgt 8640

ccacaagaag tgcttataag gtgttgctgc tattttttac atatgttcat gagttctttt 8700

ttattttttt gttctttccg ctaaagttat tatgtcatcc acagtgaact caggtctgct 8760

gttataggca agtcttcgag atggggcaat accgctgata ctatttcatg tttccagtgc 8820

aagcctcttt tgtaagtaga caaactcgat ttgtaaatat gtttgggact caactagtgg 8880

ttagagtact gattttgtaa gatttgtgta cgaaaaatca aattagaaag ataacttgcg 8940

atgattgaat aagctcaaag aagtggatgt gaatttcaaa tcgtgtcttt tttttttcat 9000

gtgtgtgtgt gagaaatttg aagtgttgtt tagatttgtt ggaatagtct aaatagattg 9060

tctctataaa atttgaagac atttgatgga gatctgatta gatgattttg aacaacaagt 9120

gcaactgaag atggatatgt atattatttt cgtagttagc aacgtgttat atatttttat 9180

agtggatata ttgttttctc attcagtgtt tgatattcac attggagtcc gactaaattt 9240

gaatttgcgc atttcaaggg gcggcgttct tgacatgatt ttttccattc tacggctcgt 9300

acgtgctctt aaattctaat taaggataga aaaatctcaa ctatttcacc acaactcaaa 9360

ttagttatat tgggtataca agtgtaaact aagaaatata actatgtagg tgcaaatatt 9420

tttgaaaaac cccaaataat aagccaaaat aaagaaagaa tactaaatcg tacactcatt 9480

actacaacaa ctaaaaaata tttataatca ttgaactcct atgcacattc tactgcaaaa 9540

cataagtaga agaagttcaa attttggcca tgaaatgaca ccattttctt acactcaaaa 9600

tgatcttaaa tttgatcccc ggg 9623

<210> 269
<211> 1282
<212> PRT
<213> Solanum tuberosum

<400> 269
Met Glu Ile Gly Leu Ala Val Gly Gly Ala Phe Leu Ser Ser Ala Leu
1               5                   10                  15
Asn Val Leu Phe Asp Arg Leu Ala Pro His Gly Asp Leu Leu Asn Met
            20                  25                  30
Phe Gln Lys His Lys Asp His Val Lys Leu Leu Lys Lys Leu Glu Asp
        35                  40                  45
Ile Leu Leu Gly Leu Gln Ile Val Leu Ser Asp Ala Glu Asn Lys Gln
    50                  55                  60

Ala Ser Asn Arg His Val Ser Gln Trp Phe Asn Lys Leu Gln Asn Ala
65                  70                  75                  80
Val Asp Gly Ala Glu Asn Leu Ile Glu Gln Val Asn Tyr Glu Ala Leu
                85                  90                  95
Arg Leu Lys Val Glu Gly Gln His Gln Asn Leu Ala Glu Thr Ser Asn
            100                 105                 110
Gln Gln Val Ser Asp Leu Asn Leu Cys Phe Ser Asp Asp Phe Phe Leu
        115                 120                 125
Asn Ile Lys Asp Lys Leu Glu Glu Thr Ile Glu Thr Leu Glu Val Leu
    130                 135                 140
Glu Lys Gln Ile Gly Arg Leu Gly Leu Lys Glu His Phe Gly Ser Thr
145                 150                 155                 160
Lys Gln Glu Thr Arg Thr Pro Ser Thr Ser Leu Val Asp Asp Ser Asp
                165                 170                 175
Ile Phe Gly Arg Gln Asn Asp Ile Glu Asp Leu Ile Asp Arg Leu Leu
            180                 185                 190
Ser Glu Asp Ala Ser Gly Lys Lys Arg Thr Val Val Pro Ile Val Gly
        195                 200                 205
Met Gly Gly Leu Gly Lys Thr Thr Leu Ala Lys Ala Val Tyr Asn Asp
    210                 215                 220
Glu Arg Val Gln Ile His Phe Gly Leu Lys Ala Trp Phe Cys Val Ser
225                 230                 235                 240
Glu Ala Phe Asp Ala Phe Arg Ile Thr Lys Gly Leu Leu Gln Glu Ile
                245                 250                 255
Gly Ser Phe Asp Leu Lys Ala Asp Asp Asn Leu Asn Gln Leu Gln Val
            260                 265                 270
Lys Leu Lys Glu Arg Leu Lys Gly Lys Lys Phe Leu Ile Val Leu Asp
        275                 280                 285
Asp Val Trp Asn Asp Asn Tyr Asn Lys Trp Asp Glu Leu Arg Asn Val
    290                 295                 300
Phe Val Gln Gly Asp Ile Gly Ser Lys Ile Ile Val Thr Thr Arg Lys
305                 310                 315                 320
Glu Ser Val Ala Leu Met Met Gly Asn Glu Gln Ile Ser Met Asp Asn
                325                 330                 335
Leu Ser Thr Glu Ser Ser Trp Ser Leu Phe Lys Thr His Ala Phe Glu
            340                 345                 350
Asn Met Gly Pro Met Gly His Pro Glu Leu Glu Glu Val Gly Lys Gln
        355                 360                 365
Ile Ala Ala Lys Cys Lys Gly Leu Pro Leu Ala Leu Lys Thr Leu Ala
    370                 375                 380
Gly Met Leu Arg Ser Lys Ser Glu Val Glu Glu Trp Lys Arg Ile Leu
385                 390                 395                 400
Arg Ser Glu Ile Trp Glu Leu Pro His Asn Asp Ile Leu Pro Ala Leu
                405                 410                 415
Met Leu Ser Tyr Asn Asp Leu Pro Ala His Leu Lys Arg Cys Phe Ser
            420                 425                 430
Phe Cys Ala Ile Phe Pro Lys Asp Tyr Pro Phe Arg Lys Glu Gln Val
        435                 440                 445
Ile His Leu Trp Ile Ala Asn Gly Leu Val Pro Gln Glu Asp Val Ile
    450                 455                 460
Ile Glu Asp Ser Gly Asn Gln Tyr Phe Leu Glu Leu Arg Ser Arg Ser
465                 470                 475                 480
Leu Phe Glu Arg Val Pro Asn Pro Ser Gln Gly Asn Thr Glu Asn Leu
                485                 490                 495
Phe Leu Met His Asp Leu Val Asn Asp Leu Ala Gln Ile Ala Ser Ser
            500                 505                 510
Lys Leu Cys Ile Arg Leu Glu Glu Ser Gln Gly Ser His Met Leu Glu
        515                 520                 525
Gln Ser Gln His Leu Ser Tyr Ser Met Gly Tyr Gly Gly Glu Phe Glu
    530                 535                 540
Lys Leu Thr Pro Leu Tyr Lys Leu Glu Gln Leu Arg Thr Leu Leu Pro
545                 550                 555                 560
Thr Cys Ile Asp Leu Pro Asp Cys Cys His His Leu Ser Lys Arg Val

565                 570                 575
Leu His Asn Ile Leu Pro Arg Leu Thr Ser Leu Arg Ala Leu Ser Leu
            580                 585                 590
Ser Cys Tyr Glu Ile Val Glu Leu Pro Asn Asp Leu Phe Ile Lys Leu
        595                 600                 605
Lys Leu Leu Arg Phe Leu Asp Ile Ser Arg Thr Glu Ile Lys Arg Leu
    610                 615                 620
Pro Asp Ser Ile Cys Ala Leu Tyr Asn Leu Glu Thr Leu Leu Leu Ser
625                 630                 635                 640
Ser Cys Tyr Asp Leu Glu Glu Leu Pro Leu Gln Met Glu Lys Leu Ile
                645                 650                 655
Asn Leu Arg His Leu Asp Ile Ser Asn Thr Arg Leu Leu Lys Met Pro
            660                 665                 670
Leu His Leu Ser Lys Leu Lys Ser Leu Gln Val Leu Val Gly Ala Lys
        675                 680                 685
Phe Leu Ile Gly Gly Leu Arg Met Glu Asp Leu Gly Glu Val His Asn
    690                 695                 700
Leu Tyr Gly Ser Leu Ser Val Val Glu Leu Gln Asn Val Val Asp Arg
705                 710                 715                 720
Arg Glu Ala Val Lys Ala Lys Met Arg Glu Lys Asn His Val Asp Arg
                725                 730                 735
Leu Tyr Leu Glu Trp Ser Gly Ser Ser Ser Ala Asp Asn Ser Gln Thr
            740                 745                 750
Glu Arg Asp Ile Leu Asp Glu Leu Arg Pro His Lys Asn Ile Lys Val
        755                 760                 765
Val Lys Ile Thr Gly Tyr Arg Gly Thr Asn Phe Pro Asn Trp Leu Ala
    770                 775                 780
Asp Pro Leu Phe Leu Lys Leu Val Lys Leu Ser Leu Arg Asn Cys Lys
785                 790                 795                 800
Asn Cys Tyr Ser Leu Pro Ala Leu Gly Gln Leu Pro Phe Leu Lys Phe
                805                 810                 815
Leu Ser Ile Arg Glu Met His Gly Ile Thr Glu Val Thr Glu Glu Phe
            820                 825                 830
Tyr Gly Ser Trp Ser Ser Lys Lys Pro Phe Asn Cys Leu Glu Lys Leu
        835                 840                 845
Glu Phe Lys Asp Met Pro Glu Trp Lys Gln Trp Asp Leu Leu Gly Ser
    850                 855                 860
Gly Glu Phe Pro Ile Leu Glu Lys Leu Leu Ile Glu Asn Cys Pro Glu
865                 870                 875                 880
Leu Ser Leu Glu Thr Val Pro Ile Gln Leu Ser Ser Leu Lys Ser Phe
                885                 890                 895
Asp Val Ile Gly Ser Pro Leu Val Ile Asn Phe Pro Leu Ser Ile Leu
            900                 905                 910
Pro Thr Thr Leu Lys Arg Ile Lys Ile Ser Asp Cys Gln Lys Leu Lys
        915                 920                 925
Leu Glu Gln Pro Thr Gly Glu Ile Ser Met Phe Leu Glu Glu Leu Thr
    930                 935                 940
Leu Ile Lys Cys Asp Cys Ile Asp Asp Ile Ser Pro Glu Leu Leu Pro
945                 950                 955                 960
Arg Ala Arg Lys Leu Trp Val Gln Asp Trp His Asn Leu Thr Arg Phe
                965                 970                 975
Leu Ile Pro Thr Ala Thr Glu Thr Leu Asp Ile Trp Asn Cys Glu Asn
            980                 985                 990
Val Glu Ile Leu Ser Val Ala Cys Gly Gly Thr Gln Met Thr Ser Leu
        995                 1000                1005
Thr Ile Ala Tyr Cys Lys Lys Leu Lys Trp Leu Pro Glu Arg Met Gln
    1010                1015                1020
Glu Leu Leu Pro Ser Leu Lys Glu Leu His Leu Ser Asn Cys Pro Glu
1025                1030                1035                1040
Ile Glu Ser Phe Pro Glu Gly Gly Leu Pro Phe Asn Leu Gln Gln Leu
                1045                1050                1055
Ala Ile Arg Tyr Cys Lys Lys Leu Val Asn Gly Arg Lys Glu Trp His
            1060                1065                1070

Leu Gln Arg Arg Leu Cys Leu Thr Ala Leu Ile Ile Tyr His Asp Gly
        1075                1080                1085
Ser Asp Glu Glu Ile Val Gly Gly Glu Asn Trp Glu Leu Pro Ser Ser
    1090                1095                1100
Ile Gln Arg Leu Thr Ile Val Asn Leu Lys Thr Leu Ser Ser Gln His
1105                1110                1115                1120
Leu Lys Asn Leu Thr Ser Leu Gln Tyr Leu Phe Ile Arg Gly Asn Leu
                1125                1130                1135
Pro Gln Ile Gln Pro Met Leu Glu Gln Gly Gln Cys Ser His Leu Thr
            1140                1145                1150
Ser Leu Gln Ser Leu Gln Ile Ser Ser Leu Gln Ser Leu Pro Glu Ser
        1155                1160                1165
Ala Leu Pro Ser Ser Leu Ser His Leu Glu Ile Ser His Cys Pro Asn
    1170                1175                1180
Leu Gln Ser Leu Pro Glu Ser Ala Leu Pro Ser Ser Leu Ser Gln Leu
1185                1190                1195                1200
Thr Ile Asn Asn Cys Pro Asn Leu Gln Ser Leu Ser Glu Ser Thr Leu
                1205                1210                1215
Pro Ser Ser Leu Ser Gln Leu Glu Ile Ser Phe Cys Pro Asn Leu Gln
            1220                1225                1230
Tyr Leu Pro Leu Lys Gly Met Pro Ser Ser Leu Ser Glu Leu Ser Ile
        1235                1240                1245
Tyr Lys Cys Pro Leu Leu Lys Pro Gln Leu Glu Phe Asp Lys Gly Glu
    1250                1255                1260
Tyr Trp Pro Asn Ile Ala Gln Phe Pro Thr Ile Lys Ile Asp Arg Glu
1265                1270                1275                1280
Cys Met

<210> 270
<211> 876
<212> DNA
<213> Artificial Sequence

<220>
<223> 2xS4xD-NpCABE-Avr3a-terminator

<400> 270
cagccaccaa agaggaccca gaattctagt cagccaccaa agaggaccca gaattctagt      60

tacaattcaa acattgttca aacaaggaac ctctagttac aattcaaaca ttgttcaaac     120

aaggaacctc tagttacaat tcaaacattg ttcaaacaag gaacctctag ttacaattca     180

aacattgttc aaacaaggaa cctctagtgc cggcctgcag taaagccatt tatatacact     240

tagtgcaaag cccatgaaac tcaagcctca actcgagatg gaccaaacca aggtcctggt     300

gtatgggacg ccagctcact acatacacga ttcagccggc agaagacttc ttcgcaagaa     360

cgaagagaat gaagaaacgt ctgaggagcg agccccaaat ttcaatttgg cgaatctaaa     420

tgaggagatg tttaatgtgg ctgcgttgac gaagagagca gatgccaaaa agctagcgaa     480

acagcttatg ggtaatgata agctggcgga tgctgcatac atttggtggc agcacaacag     540

ggttacgcta gaccagattg acacgttcct gaagcttgca agccgcaaga cgcaaggcgc     600

aaagtacaat cagatctaca atagctacat gatgcacctg gggctcactg gatattaaat     660

cactagagtc cgcaaaaatc accagtctct ctctacaaat ctatctctct ctatttttct     720

ccagaataat gtgtgagtag ttcccagata agggaattag ggttcttata gggtttcgct     780

catgtgttga gcatataaga aacccttagt atgtatttgt atttgtaaaa tacttctatc     840

aataaaattt ctaattccta aaaccaaaat ccagtg                               876

<210> 271
<211> 1282
<212> PRT
<213> Artificial Sequence

<220>
<223> R3a - K35E + K37M

<400> 271
Met Glu Ile Gly Leu Ala Val Gly Gly Ala Phe Leu Ser Ser Ala Leu
1               5                   10                  15
Asn Val Leu Phe Asp Arg Leu Ala Pro His Gly Asp Leu Leu Asn Met
            20                  25                  30
Phe Gln Glu His Met Asp His Val Lys Leu Leu Lys Lys Leu Glu Asp
        35                  40                  45
Ile Leu Leu Gly Leu Gln Ile Val Leu Ser Asp Ala Glu Asn Lys Gln
    50                  55                  60
Ala Ser Asn Arg His Val Ser Gln Trp Phe Asn Lys Leu Gln Asn Ala
65                  70                  75                  80
Val Asp Gly Ala Glu Asn Leu Ile Glu Gln Val Asn Tyr Glu Ala Leu
                85                  90                  95
Arg Leu Lys Val Glu Gly Gln His Gln Asn Leu Ala Glu Thr Ser Asn
            100                 105                 110
Gln Gln Val Ser Asp Leu Asn Leu Cys Phe Ser Asp Asp Phe Phe Leu
        115                 120                 125
Asn Ile Lys Asp Lys Leu Glu Glu Thr Ile Glu Thr Leu Glu Val Leu
    130                 135                 140
Glu Lys Gln Ile Gly Arg Leu Gly Leu Lys Glu His Phe Gly Ser Thr
145                 150                 155                 160
Lys Gln Glu Thr Arg Thr Pro Ser Thr Ser Leu Val Asp Asp Ser Asp
                165                 170                 175
Ile Phe Gly Arg Gln Asn Asp Ile Glu Asp Leu Ile Asp Arg Leu Leu
            180                 185                 190
Ser Glu Asp Ala Ser Gly Lys Lys Arg Thr Val Val Pro Ile Val Gly
        195                 200                 205
Met Gly Gly Leu Gly Lys Thr Thr Leu Ala Lys Ala Val Tyr Asn Asp
    210                 215                 220
Glu Arg Val Gln Ile His Phe Gly Leu Lys Ala Trp Phe Cys Val Ser
225                 230                 235                 240
Glu Ala Phe Asp Ala Phe Arg Ile Thr Lys Gly Leu Leu Gln Glu Ile
                245                 250                 255
Gly Ser Phe Asp Leu Lys Ala Asp Asp Asn Leu Asn Gln Leu Gln Val
            260                 265                 270
Lys Leu Lys Glu Arg Leu Lys Gly Lys Lys Phe Leu Ile Val Leu Asp
        275                 280                 285
Asp Val Trp Asn Asp Asn Tyr Asn Lys Trp Asp Glu Leu Arg Asn Val
    290                 295                 300
Phe Val Gln Gly Asp Ile Gly Ser Lys Ile Ile Val Thr Thr Arg Lys
305                 310                 315                 320
Glu Ser Val Ala Leu Met Met Gly Asn Glu Gln Ile Ser Met Asp Asn
                325                 330                 335
Leu Ser Thr Glu Ser Ser Trp Ser Leu Phe Lys Thr His Ala Phe Glu
            340                 345                 350
Asn Met Gly Pro Met Gly His Pro Glu Leu Glu Glu Val Gly Lys Gln
        355                 360                 365

Ile Ala Ala Lys Cys Lys Gly Leu Pro Leu Ala Leu Lys Thr Leu Ala
    370                 375                 380
Gly Met Leu Arg Ser Lys Ser Glu Val Glu Glu Trp Lys Arg Ile Leu
385                 390                 395                 400
Arg Ser Glu Ile Trp Glu Leu Pro His Asn Asp Ile Leu Pro Ala Leu
                405                 410                 415
Met Leu Ser Tyr Asn Asp Leu Pro Ala His Leu Lys Arg Cys Phe Ser
            420                 425                 430
Phe Cys Ala Ile Phe Pro Lys Asp Tyr Pro Phe Arg Lys Glu Gln Val
        435                 440                 445
Ile His Leu Trp Ile Ala Asn Gly Leu Val Pro Gln Glu Asp Val Ile
    450                 455                 460
Ile Glu Asp Ser Gly Asn Gln Tyr Phe Leu Glu Leu Arg Ser Arg Ser
465                 470                 475                 480
Leu Phe Glu Arg Val Pro Asn Pro Ser Gln Gly Asn Thr Glu Asn Leu
                485                 490                 495
Phe Leu Met His Asp Leu Val Asn Asp Leu Ala Gln Ile Ala Ser Ser
            500                 505                 510
Lys Leu Cys Ile Arg Leu Glu Glu Ser Gln Gly Ser His Met Leu Glu
        515                 520                 525
Gln Ser Gln His Leu Ser Tyr Ser Met Gly Tyr Gly Gly Glu Phe Glu
    530                 535                 540
Lys Leu Thr Pro Leu Tyr Lys Leu Glu Gln Leu Arg Thr Leu Leu Pro
545                 550                 555                 560
Thr Cys Ile Asp Leu Pro Asp Cys Cys His His Leu Ser Lys Arg Val
                565                 570                 575
Leu His Asn Ile Leu Pro Arg Leu Thr Ser Leu Arg Ala Leu Ser Leu
            580                 585                 590
Ser Cys Tyr Glu Ile Val Glu Leu Pro Asn Asp Leu Phe Ile Lys Leu
        595                 600                 605
Lys Leu Leu Arg Phe Leu Asp Ile Ser Arg Thr Glu Ile Lys Arg Leu
    610                 615                 620
Pro Asp Ser Ile Cys Ala Leu Tyr Asn Leu Glu Thr Leu Leu Leu Ser
625                 630                 635                 640
Ser Cys Tyr Asp Leu Glu Glu Leu Pro Leu Gln Met Glu Lys Leu Ile
                645                 650                 655
Asn Leu Arg His Leu Asp Ile Ser Asn Thr Arg Leu Leu Lys Met Pro
            660                 665                 670
Leu His Leu Ser Lys Leu Lys Ser Leu Gln Val Leu Val Gly Ala Lys
        675                 680                 685
Phe Leu Ile Gly Gly Leu Arg Met Glu Asp Leu Gly Glu Val His Asn
    690                 695                 700
Leu Tyr Gly Ser Leu Ser Val Val Glu Leu Gln Asn Val Val Asp Arg
705                 710                 715                 720
Arg Glu Ala Val Lys Ala Lys Met Arg Glu Lys Asn His Val Asp Arg
                725                 730                 735
Leu Tyr Leu Glu Trp Ser Gly Ser Ser Ser Ala Asp Asn Ser Gln Thr
            740                 745                 750
Glu Arg Asp Ile Leu Asp Glu Leu Arg Pro His Lys Asn Ile Lys Val
        755                 760                 765
Val Lys Ile Thr Gly Tyr Arg Gly Thr Asn Phe Pro Asn Trp Leu Ala
    770                 775                 780
Asp Pro Leu Phe Leu Lys Leu Val Lys Leu Ser Leu Arg Asn Cys Lys
785                 790                 795                 800
Asn Cys Tyr Ser Leu Pro Ala Leu Gly Gln Leu Pro Phe Leu Lys Phe
                805                 810                 815
Leu Ser Ile Arg Glu Met His Gly Ile Thr Glu Val Thr Glu Glu Phe
            820                 825                 830
Tyr Gly Ser Trp Ser Ser Lys Lys Pro Phe Asn Cys Leu Glu Lys Leu
        835                 840                 845
Glu Phe Lys Asp Met Pro Glu Trp Lys Gln Trp Asp Leu Leu Gly Ser
    850                 855                 860
Gly Glu Phe Pro Ile Leu Glu Lys Leu Leu Ile Glu Asn Cys Pro Glu

865                 870                 875                 880
Leu Ser Leu Glu Thr Val Pro Ile Gln Leu Ser Ser Leu Lys Ser Phe
                885                 890                 895
Asp Val Ile Gly Ser Pro Leu Val Ile Asn Phe Pro Leu Ser Ile Leu
            900                 905                 910
Pro Thr Thr Leu Lys Arg Ile Lys Ile Ser Asp Cys Gln Lys Leu Lys
        915                 920                 925
Leu Glu Gln Pro Thr Gly Glu Ile Ser Met Phe Leu Glu Glu Leu Thr
    930                 935                 940
Leu Ile Lys Cys Asp Cys Ile Asp Asp Ile Ser Pro Glu Leu Leu Pro
945                 950                 955                 960
Arg Ala Arg Lys Leu Trp Val Gln Asp Trp His Asn Leu Thr Arg Phe
                965                 970                 975
Leu Ile Pro Thr Ala Thr Glu Thr Leu Asp Ile Trp Asn Cys Glu Asn
            980                 985                 990
Val Glu Ile Leu Ser Val Ala Cys Gly Gly Thr Gln Met Thr Ser Leu
        995                 1000                1005
Thr Ile Ala Tyr Cys Lys Lys Leu Lys Trp Leu Pro Glu Arg Met Gln
    1010                1015                1020
Glu Leu Leu Pro Ser Leu Lys Glu Leu His Leu Ser Asn Cys Pro Glu
1025                1030                1035                1040
Ile Glu Ser Phe Pro Glu Gly Gly Leu Pro Phe Asn Leu Gln Gln Leu
                1045                1050                1055
Ala Ile Arg Tyr Cys Lys Lys Leu Val Asn Gly Arg Lys Glu Trp His
            1060                1065                1070
Leu Gln Arg Arg Leu Cys Leu Thr Ala Leu Ile Ile Tyr His Asp Gly
        1075                1080                1085
Ser Asp Glu Glu Ile Val Gly Gly Glu Asn Trp Glu Leu Pro Ser Ser
    1090                1095                1100
Ile Gln Arg Leu Thr Ile Val Asn Leu Lys Thr Leu Ser Ser Gln His
1105                1110                1115                1120
Leu Lys Asn Leu Thr Ser Leu Gln Tyr Leu Phe Ile Arg Gly Asn Leu
                1125                1130                1135
Pro Gln Ile Gln Pro Met Leu Glu Gln Gly Gln Cys Ser His Leu Thr
            1140                1145                1150
Ser Leu Gln Ser Leu Gln Ile Ser Ser Leu Gln Ser Leu Pro Glu Ser
        1155                1160                1165
Ala Leu Pro Ser Ser Leu Ser His Leu Glu Ile Ser His Cys Pro Asn
    1170                1175                1180
Leu Gln Ser Leu Pro Glu Ser Ala Leu Pro Ser Ser Leu Ser Gln Leu
1185                1190                1195                1200
Thr Ile Asn Asn Cys Pro Asn Leu Gln Ser Leu Ser Glu Ser Thr Leu
                1205                1210                1215
Pro Ser Ser Leu Ser Gln Leu Glu Ile Ser Phe Cys Pro Asn Leu Gln
            1220                1225                1230
Tyr Leu Pro Leu Lys Gly Met Pro Ser Ser Leu Ser Glu Leu Ser Ile
        1235                1240                1245
Tyr Lys Cys Pro Leu Leu Lys Pro Gln Leu Glu Phe Asp Lys Gly Glu
    1250                1255                1260
Tyr Trp Pro Asn Ile Ala Gln Phe Pro Thr Ile Lys Ile Asp Arg Glu
1265                1270                1275                1280
Cys Met


<210> 272
<211> 14
<212> PRT
<213> Artificial Sequence


<220>
<223> Motif of LRR domain

<220>
<221> VARIANT
<222> 1..14
<223> X = A or R or N or D or C or Q or E or G or H or I or L or K or M
      or F or P or S or T or W or Y or V

<400> 272
Leu Xaa Xaa Leu Xaa Xaa Leu Xaa Leu Xaa Xaa Cys Xaa Xaa
1               5                   10

<210> 273
<211> 169
<212> PRT
<213> Artificial Sequence

<220>
<223> Consensus motif

<220>
<221> VARIANT
<222> 1..169
<223> X = A or R or N or D or C or Q or E or G or H or I or L or K or M
      or F or P or S or T or W or Y or V

<400> 273
Met Leu Ile Ile Gly Glu Xaa Phe Leu Ser Ala Phe Leu Gln Val Leu
1               5                   10                  15
Phe Glu Lys Leu Ala Ser Xaa Gly Ile Xaa Leu Phe Met Xaa Xaa Xaa
            20                  25                  30
Lys Gly Xaa Ile Xaa Xaa Lys Leu Ile Lys Lys Trp Glu Lys Xaa Leu
        35                  40                  45
Arg Ile Ile Glu Ala Val Leu Ser Asp Ala Glu Gln Lys Gln Xaa Xaa
    50                  55                  60
Asn Asn Ala Val Lys Xaa Trp Leu Asn Asp Leu Gln Asp Xaa Ala Tyr
65                  70                  75                  80
Asp Leu Glu Asp Ile Leu Asp Xaa Phe Xaa Thr Asp Ala Xaa Ile Xaa
                85                  90                  95
Glu Leu Xaa Xaa Xaa Asn Asp Gln His Gln Xaa Xaa Xaa Xaa Asp Xaa
            100                 105                 110
Asn Xaa Lys Xaa Xaa Xaa Xaa Xaa Xaa Xaa Xaa Xaa Gln Ser Xaa Ala
        115                 120                 125
Xaa Xaa Xaa Leu Xaa Phe Leu Xaa Xaa Xaa Xaa Xaa Xaa Phe Ser Xaa
    130                 135                 140
Lys Ile Glu Glu Ile Ser Xaa Xaa Leu Glu Asp Leu Leu Xaa Gln Ile
145                 150                 155                 160
Xaa Xaa Leu Ala Leu Ser Ser Xaa Ile
                165

<210> 274
<211> 3463
<212> DNA
<213> Beta vulgaris

<400> 274
gattcatact ctacttccat actttgtaaa aacaaaaaaa gataaaaatg gtgataggcg      60

gagagatttt tctatcagcg tttcttcaag tgctctttga aaagcttgca tcaggaggga     120

taagtctatt cctaaaaaga gaaaaaggaa taggacctaa agttattcaa agatggaaca     180

aaaaattgag attaatagag gcggttttaa gtgacgccga gcaaaagcag tttcacaaca     240

atgccgtcaa attgtggctt cgagatcttc aagagttcgc gtacgattta gaggacatct 300

tagacgaatt tgatacagat gctcgactta aggagttcaa tgatcagcct cagccacagc 360

cacaggaaca tcaacccaaa tcttcttgtt ctcctttcaa taaggtacaa agctgtcttt 420

cttgtggttt cccaacttta aataagaaaa ctacaaagta ttcaactagc attgaagaga 480

tgagcactcg cttggaagat cttctggacc aagtaaggtc tttggctctc tcaacgcaga 540

tacactcaag ggtagagcaa catgaagaac ggagggaaac atcatctatg atacgggagc 600

cagttgtata tgggagggac gatgagaaaa atcagataat ccaaagatta ttaaagacag 660

atgagccatt ttgtgaaaat tacaatgtaa ttcctattgt tggaacagga ggcattggga 720

agacgaccct cgctcaggct gtatacaatg atgagcaggt gaaggctcac tttgatgtaa 780

aagcatgggt gtgcatctcc gatgagtttg atgtcaaaca agtgacaaca agcattatca 840

cttcagccac tcgtgataca tgtaatttcg gtgttctaga tgaggcacaa gataaattaa 900

agaacttgct tgtagacaaa agattcttga tcgttcttga tgatatatgg agtgatgaat 960

atgatccttg ggaccaactt caaacccctt tttcatcagc gaagaaggga agtagagttc 1020

taatcacgac cagaatggag acggtggcaa agaacatggt taaaagacca gatcaaagcc 1080

ccatcatcaa gttaaaggtt ttatctgatg atgattgttg gcttctcttt caaaagcatg 1140

cagtcgtaga tgaagacctc attgtgatgc agaaggactt ggttgggttg ttcaaaggat 1200

tgcctttggc tgcaaaagct ctaggaggtc tcctaaggag agagcgtaag agtaattggg 1260

caaggatatt aaagagcaat atttggagtg aagaaggtgg tgttctgcca gtcttgaggc 1320

ttagttatca tcatctccca caaaacctca aacgtgcctt tgcttattgt tctatattcc 1380

cgaaagatta taaattcacg gagatgaatg ttatattaat gtggatagca gaaggcttgc 1440

tgccagagca cgacaaggag tgcaaggaag acattggtcg tgactatttt cttgatctag 1500

tatcaagatc attgtttgaa ccaaacagtc caatgtatat ggatggatct ttcattatgc 1560

atgacctaat ccatgaccta gctcaatggg ctgctggtga agtttgttgc acattgaata 1620

tccaaaaact ttcttctaga acacgttatt ggtctttctc tgaagaaata ctgatagatc 1680

aaacatggac ttcaaagaaa cttgtccagg tacgatcatt tgcttctttt ggtgcattag 1740

atgttcgaat tcctatgcaa ctactagatt ccattttccg tcagtttcaa tacttacgct 1800

tgttgcgtat gcataaggcg ggaataattg agttaccaaa tagtatgggt aatctgaaac 1860

acttgagact tcttgacctc tcatggaata caaaactaac tagattgcca aagtccacta 1920

gcaagctttg caacctccag acgctgctgt taagaggatg taagtctctt gtggagatag 1980

tccctctaat cgaacttcaa caccttgatg ttagtagtag gtatgctgct tcaccgttgg 2040

ggattggaaa attaacaaac ttgcagacat tgaagggatt tactttgaga agatactcta 2100

agacaagtat aagtgaattg aagaacttga aatgcctccg tggctcactg gacatccatg 2160

gcctagagaa tgtgtttagt agtgaagaag cacaagcagc aaggttgcac gaaaagacag 2220

gccttgataa gttggaaatg tattggggag ataatgccca tgatgttgat gacaacatta 2280

aaaaagatgt agttgatcga ttacagcctc cggaatccat caaagaatta acgttgaatg 2340

ggtacgacgg cttaacattc cctgcttggt tgggaaatcc ctcttacact aatatggttt 2400

tcatagaatt aagggagtgt aggagatgtg aattcttacc agcactaggg cagctgccct 2460

cattaaagaa catcataata gaagggatgg atgatattaa gactgtaggc cctgaatttt 2520

acggcaatga tgatggttgt tcaaacctgt ttcctgcatt gaaaagtcta cgattctatg 2580

gaatgggggg ttgggaggag tggttggccc catcagttga caatagcaat gcatttccct 2640

gccttgaata tattagcata tatgaatgtc cattgttgcg ggctaactta ccttcccatc 2700

tcccatccct aaaagacttg tccattggca aatgcaaaga gttgagattg tcacttccaa 2760

gctgcccttt gctccaaaaa ttgactatta taacatatga gacgccattt agtattgctg 2820

attgtttgcc ctcaactctt gagaccctgg aaatcttgca ctccgaaata gagcagccaa 2880

ttcaagagtg gaaacttgac ctccttactt ctcttaaatc tcttgatctc caaaacatag 2940

gcagagccgc agatacaata gagcatattc cccaaccaga ttttcatctt ccctcttcct 3000

tgtcttcctt atatatctcg gatttcaaga atttgaaatc cttgtcatgc tctacccttc 3060

ccaacctcac tgaaattaaa atctggaatt gtaagaagct ggaatcattg ggtgtcgatt 3120

tcccaccatc gaagcttcag gaagcgtatt ttagtgattg ccctttgatt tatcaacgat 3180

gcaaaccgga tccccatgga cttattgttt ttaattatat tgataaatac aaatattcgt 3240

acatttgaag attccagttt tcattccctg aagattgagt tggaggtgga gttttggggt 3300

ttttttcaga ttttgttttt gtttttttc atttctttta gtttatttgt atgaaactag 3360

aatatattca aggaattgtg ataaaaaaaa aaaaaaaagg gcggccgctc tagagtatcc 3420

ctcgaggggc ccaagcttac gcgacccagc tttcttacaa agg 3463

<210> 275
<211> 1066
<212> PRT
<213> Beta vulgaris

<400> 275
Met Val Ile Gly Gly Glu Ile Phe Leu Ser Ala Phe Leu Gln Val Leu
1               5                   10                  15
Phe Glu Lys Leu Ala Ser Gly Gly Ile Ser Leu Phe Leu Lys Arg Glu
            20                  25                  30
Lys Gly Ile Gly Pro Lys Val Ile Gln Arg Trp Asn Lys Lys Leu Arg
        35                  40                  45
Leu Ile Glu Ala Val Leu Ser Asp Ala Glu Gln Lys Gln Phe His Asn
    50                  55                  60
Asn Ala Val Lys Leu Trp Leu Arg Asp Leu Gln Glu Phe Ala Tyr Asp

65                  70                  75                  80
Leu Glu Asp Ile Leu Asp Glu Phe Asp Thr Asp Ala Arg Leu Lys Glu
                85                  90                  95
Phe Asn Asp Gln Pro Gln Pro Gln Pro Gln Glu His Gln Pro Lys Ser
            100                 105                 110
Ser Cys Ser Pro Phe Asn Lys Val Gln Ser Cys Leu Ser Cys Gly Phe
        115                 120                 125
Pro Thr Leu Asn Lys Lys Thr Thr Lys Tyr Ser Thr Ser Ile Glu Glu
    130                 135                 140
Met Ser Thr Arg Leu Glu Asp Leu Leu Asp Gln Val Arg Ser Leu Ala
145                 150                 155                 160
Leu Ser Thr Gln Ile His Ser Arg Val Glu Gln His Glu Glu Arg Arg
                165                 170                 175
Glu Thr Ser Ser Met Ile Arg Glu Pro Val Val Tyr Gly Arg Asp Asp
            180                 185                 190
Glu Lys Asn Gln Ile Ile Gln Arg Leu Leu Lys Thr Asp Glu Pro Phe
        195                 200                 205
Cys Glu Asn Tyr Asn Val Ile Pro Ile Val Gly Thr Gly Gly Ile Gly
    210                 215                 220
Lys Thr Thr Leu Ala Gln Ala Val Tyr Asn Asp Glu Gln Val Lys Ala
225                 230                 235                 240
His Phe Asp Val Lys Ala Trp Val Cys Ile Ser Asp Glu Phe Asp Val
                245                 250                 255
Lys Gln Val Thr Thr Ser Ile Ile Thr Ser Ala Thr Arg Asp Thr Cys
            260                 265                 270
Asn Phe Gly Val Leu Asp Glu Ala Gln Asp Lys Leu Lys Asn Leu Leu
        275                 280                 285
Val Asp Lys Arg Phe Leu Ile Val Leu Asp Asp Ile Trp Ser Asp Glu
    290                 295                 300
Tyr Asp Pro Trp Asp Gln Leu Gln Thr Pro Phe Ser Ser Ala Lys Lys
305                 310                 315                 320
Gly Ser Arg Val Leu Ile Thr Thr Arg Met Glu Thr Val Ala Lys Asn
                325                 330                 335
Met Val Lys Arg Pro Asp Gln Ser Pro Ile Ile Lys Leu Lys Val Leu
            340                 345                 350
Ser Asp Asp Asp Cys Trp Leu Leu Phe Gln Lys His Ala Val Val Asp
        355                 360                 365
Glu Asp Leu Ile Val Met Gln Lys Asp Leu Val Gly Leu Phe Lys Gly
    370                 375                 380
Leu Pro Leu Ala Ala Lys Ala Leu Gly Gly Leu Leu Arg Arg Glu Arg
385                 390                 395                 400
Lys Ser Asn Trp Ala Arg Ile Leu Lys Ser Asn Ile Trp Ser Glu Glu
                405                 410                 415
Gly Gly Val Leu Pro Val Leu Arg Leu Ser Tyr His His Leu Pro Gln
            420                 425                 430
Asn Leu Lys Arg Ala Phe Ala Tyr Cys Ser Ile Phe Pro Lys Asp Tyr
        435                 440                 445
Lys Phe Thr Glu Met Asn Val Ile Leu Met Trp Ile Ala Glu Gly Leu
    450                 455                 460
Leu Pro Glu His Asp Lys Glu Cys Lys Glu Asp Ile Gly Arg Asp Tyr
465                 470                 475                 480
Phe Leu Asp Leu Val Ser Arg Ser Leu Phe Glu Pro Asn Ser Pro Met
                485                 490                 495
Tyr Met Asp Gly Ser Phe Ile Met His Asp Leu Ile His Asp Leu Ala
            500                 505                 510
Gln Trp Ala Ala Gly Glu Val Cys Cys Thr Leu Asn Ile Gln Lys Leu
        515                 520                 525
Ser Ser Arg Thr Arg Tyr Trp Ser Phe Ser Glu Glu Ile Leu Ile Asp
    530                 535                 540
Gln Thr Trp Thr Ser Lys Lys Leu Val Gln Val Arg Ser Phe Ala Ser
545                 550                 555                 560
Phe Gly Ala Leu Asp Val Arg Ile Pro Met Gln Leu Leu Asp Ser Ile
                565                 570                 575

Phe Arg Gln Phe Gln Tyr Leu Arg Leu Leu Arg Met His Lys Ala Gly
            580                 585                 590
Ile Ile Glu Leu Pro Asn Ser Met Gly Asn Leu Lys His Leu Arg Leu
        595                 600                 605
Leu Asp Leu Ser Trp Asn Thr Lys Leu Thr Arg Leu Pro Lys Ser Thr
    610                 615                 620
Ser Lys Leu Cys Asn Leu Gln Thr Leu Leu Leu Arg Gly Cys Lys Ser
625                 630                 635                 640
Leu Val Glu Ile Val Pro Leu Ile Glu Leu Gln His Leu Asp Val Ser
                645                 650                 655
Ser Arg Tyr Ala Ala Ser Pro Leu Gly Ile Gly Lys Leu Thr Asn Leu
            660                 665                 670
Gln Thr Leu Lys Gly Phe Thr Leu Arg Arg Tyr Ser Lys Thr Ser Ile
        675                 680                 685
Ser Glu Leu Lys Asn Leu Lys Cys Leu Arg Gly Ser Leu Asp Ile His
    690                 695                 700
Gly Leu Glu Asn Val Phe Ser Ser Glu Glu Ala Gln Ala Ala Arg Leu
705                 710                 715                 720
His Glu Lys Thr Gly Leu Asp Lys Leu Glu Met Tyr Trp Gly Asp Asn
                725                 730                 735
Ala His Asp Val Asp Asp Asn Ile Lys Lys Asp Val Val Asp Arg Leu
            740                 745                 750
Gln Pro Pro Glu Ser Ile Lys Glu Leu Thr Leu Asn Gly Tyr Asp Gly
        755                 760                 765
Leu Thr Phe Pro Ala Trp Leu Gly Asn Pro Ser Tyr Thr Asn Met Val
    770                 775                 780
Phe Ile Glu Leu Arg Glu Cys Arg Arg Cys Glu Phe Leu Pro Ala Leu
785                 790                 795                 800
Gly Gln Leu Pro Ser Leu Lys Asn Ile Ile Ile Glu Gly Met Asp Asp
                805                 810                 815
Ile Lys Thr Val Gly Pro Glu Phe Tyr Gly Asn Asp Asp Gly Cys Ser
            820                 825                 830
Asn Leu Phe Pro Ala Leu Lys Ser Leu Arg Phe Tyr Gly Met Gly Gly
        835                 840                 845
Trp Glu Glu Trp Leu Ala Pro Ser Val Asp Asn Ser Asn Ala Phe Pro
    850                 855                 860
Cys Leu Glu Tyr Ile Ser Ile Tyr Glu Cys Pro Leu Leu Arg Ala Asn
865                 870                 875                 880
Leu Pro Ser His Leu Pro Ser Leu Lys Asp Leu Ser Ile Gly Lys Cys
                885                 890                 895
Lys Glu Leu Arg Leu Ser Leu Pro Ser Cys Pro Leu Leu Gln Lys Leu
            900                 905                 910
Thr Ile Ile Thr Tyr Glu Thr Pro Phe Ser Ile Ala Asp Cys Leu Pro
        915                 920                 925
Ser Thr Leu Glu Thr Leu Glu Ile Leu His Ser Glu Ile Glu Gln Pro
    930                 935                 940
Ile Gln Glu Trp Lys Leu Asp Leu Leu Thr Ser Leu Lys Ser Leu Asp
945                 950                 955                 960
Leu Gln Asn Ile Gly Arg Ala Ala Asp Thr Ile Glu His Ile Pro Gln
                965                 970                 975
Pro Asp Phe His Leu Pro Ser Ser Leu Ser Ser Leu Tyr Ile Ser Asp
            980                 985                 990
Phe Lys Asn Leu Lys Ser Leu Ser Cys Ser Thr Leu Pro Asn Leu Thr
        995                 1000                1005
Glu Ile Lys Ile Trp Asn Cys Lys Lys Leu Glu Ser Leu Gly Val Asp
    1010                1015                1020
Phe Pro Pro Ser Lys Leu Gln Glu Ala Tyr Phe Ser Asp Cys Pro Leu
1025                1030                1035                1040
Ile Tyr Gln Arg Cys Lys Pro Asp Pro His Gly Leu Ile Val Phe Asn
                1045                1050                1055
Tyr Ile Asp Lys Tyr Lys Tyr Ser Tyr Ile
            1060                1065

<210> 276
<211> 3241
<212> DNA
<213> Beta vulgaris

<400> 276
attgaattaa tctactacaa ttgaaaataa aagtgagtca atattcagta atatgggaat 60
aataggagaa acaattctat cagcatttct acaagtgctg tttgagaagt tattttcccg 120
aggagtaaat ttgtttatgg ggttagtgaa aggaggaatc caacaaaaaa ccatcaaaca 180
atgggagaaa caactaagaa ttatagaggc agttttaagt gatgctgagc aaaagcaagt 240
ccaaagcaat gctgtgaagt catggatgca ggacctccaa gatttggcgt atgacttaga 300
agatgtgttg gatgcatttg caactcaagc tcaaatttac gaattgtcga tcatgaatga 360
tagtcatagc tccgaattga aagataatga caccaagttt atggcggaaa gcattattgt 420
tggcggttca aaagctatag agcatttatc ttttatgttt aactctggcc aagatttttc 480
ggggaggata gaagagatta gtcaacaatt ggaagaaatt ttaagccaaa ccgagggttt 540
agggctatca gttcatgtga tgcatcagca agtagtaccc acaacaacag cacaacgttg 600
gcaaagctgg cgagaatcaa cttctttatt atgtgagcca tttgtatatg gacgggaagg 660
tgacaagaag gagatcatgc atcggttact acatgacaag aactcaagct ctgggaacta 720
tattgtgatt cctattgttg gaaaaggagg cattggtaag acgactcttg ctcaacttat 780
ctacaatgat gaagaagtgg acgtgttttc tttgaaagca tgggtttatg tatctcaagt 840
gtttgatgct aaaagggtaa caacatcaat tctgaattca atcactggtg aagccaattc 900
attttgtgat ttaaatcaag ctcaagtacg attgcagcaa gtgcttagaa acaaacgatt 960
cttgattata cttgatgaca tgtggagtgt gatgtacagt gattgggatg acctacgcac 1020
tccactaaga gcaggggcaa aaggaagcag ggtcatagtt accaccagaa ttcagaaagt 1080
tgcgagaatt atgaatccac aaatgaataa tatgatttta ttggagtgtc ttgcagatga 1140
tgactgttgg catctaattt tgcaacatgc ctttgatgat gccaatattg ccgtaagccc 1200
aaatattagt ctaatgagag ctgaaatggt aagggcgtgc aaagggatac ccctggtagc 1260
aaaaactctt ggaggccttt taaggaccaa agtggaggag gagagcgagt ggcaagagag 1320
tctcaacact gatattttga gcatgagtaa gagtagagtt ttacaggtat tggaattgag 1380
ttattatgac cttccatcac atttgaaacg ctgtttctct tactgcgcta tatttcccaa 1440
tgatcatgct tttacagagg aggatgtcat tttgttatgg atggctgaag gcttcctacc 1500
tgaaaatgta gggaacaagc gaattgaaga cataggccat gagtatttcc aggagctagt 1560
atcaagatca ttttttgaac ccagcacaaa aggatcaaaa tggttcaaaa tgcacaactt 1620
atatagtgat ttggcccgac aagttgttgg tgatttatgg ttggtgatga aggaatgttc 1680

agtaaggaaa gattatagag ctcgacattt atctttctcc aaactagatg aatgtgaaga 1740

tctatttcac ctcaagaaca taagccactt tcgtactttt gctcaatttg acaagcatcc 1800

cagttcctca agccgagtac ttgagcttat aggatatttt cagtacttaa gggtgctatt 1860

gctaccgagc ctgggaatta caaattttcc agaatctata ggtgacctca agctcttacg 1920

atatctcaat ctctcataca atgattttga agaattgccg aattcagtaa gcaaccttgt 1980

taatctccaa actttgttgc tgaagggttg cgttaaactt cagaaattat cgacgaatat 2040

gaggtgtcta atcaaccttc gccatcttga tattgatggt acctctctga atgagatgcc 2100

attggggtta gggaatctga cgaacctgca aacgttacac agatttgtct taggagctaa 2160

tgcacccata ataaaagagt tgaagaactt gaagcagctt cgaggaaagt taaccatagc 2220

tggcttagag aatttggtga gctccaacga tgcaggagaa gcaaggttga atgagaaatc 2280

gtgtcttcat gagttggaaa tggtatggga atggggaacc ttcactaatg ggattgatga 2340

caacactaag atagatgtgt tagatcagct acaccctcac agatccacaa aagtgttaac 2400

tctaagaaac tacaagggtt tgacattccc aagttggttg agtgaccctt ctttcactaa 2460

gatggttgtc ataaggctaa taggttgtga gagatgtgaa tcattaccat cactagggaa 2520

gcttccttca ttgaaggaac ttgtggttca agaaatgcgc ggtcttaaat cagtagatga 2580

tgacttttac ggtgttggtt gttcaaaccc ttttccggca ttgataactc tgaaattcaa 2640

gtctatgttt tcttgggaaa gttggttaat ttcatcagct gatactaagg ttttttctaa 2700

ccttcaagag ctttcaatca aggattctcc cctcttacaa catttatctt taccttcatg 2760

tctccctttg ttagagacac ttgagataca acattgtaag aagctagatg cctcacttcc 2820

aaccctccct catctccaga agttggtatt agaagattgc tcatcccttt tcatggttga 2880

tcagctatct acgactcttc gacaactggc tatttccaat tgccggaaac tacagttggt 2940

ggaattcaga agaccatgct tctctcttga tgaaaatatt tctctagagg ttgtagaagt 3000

tgtcaattgc caatctctgt taaatttttt cagtagagat ggtattgaag tgctacatca 3060

cgtgccaggc cacctatcag gtttacatac accaggaggt ctagggggaa gaatactgaa 3120

atgtagagtg agctggcaag aatgagctgg caggtggtta gtgcggaaat tcagggagtt 3180

ggttagagaa atatctgtta gagaaatatc tgtccagagg gaatgtagct gacattctag 3240

a 3241

<210> 277
<211> 1030
<212> PRT
<213> Beta vulgaris

<400> 277
Met Gly Ile Ile Gly Glu Thr Ile Leu Ser Ala Phe Leu Gln Val Leu

1               5                   10                  15
Phe Glu Lys Leu Phe Ser Arg Gly Val Asn Leu Phe Met Gly Leu Val
                20                  25                  30
Lys Gly Gly Ile Gln Gln Lys Thr Ile Lys Gln Trp Glu Lys Gln Leu
            35                  40                  45
Arg Ile Ile Glu Ala Val Leu Ser Asp Ala Glu Gln Lys Gln Val Gln
    50                  55                  60
Ser Asn Ala Val Lys Ser Trp Met Gln Asp Leu Gln Asp Leu Ala Tyr
65                  70                  75                  80
Asp Leu Glu Asp Val Leu Asp Ala Phe Ala Thr Gln Ala Gln Ile Tyr
                85                  90                  95
Glu Leu Ser Ile Met Asn Asp Ser His Ser Ser Glu Leu Lys Asp Asn
            100                 105                 110
Asp Thr Lys Phe Met Ala Glu Ser Ile Ile Val Gly Gly Ser Lys Ala
        115                 120                 125
Ile Glu His Leu Ser Phe Met Phe Asn Ser Gly Gln Asp Phe Ser Gly
    130                 135                 140
Arg Ile Glu Glu Ile Ser Gln Gln Leu Glu Glu Ile Leu Ser Gln Thr
145                 150                 155                 160
Glu Gly Leu Gly Leu Ser Val His Val Met His Gln Gln Val Val Pro
                165                 170                 175
Thr Thr Thr Ala Gln Arg Trp Gln Ser Trp Arg Glu Ser Thr Ser Leu
            180                 185                 190
Leu Cys Glu Pro Phe Val Tyr Gly Arg Glu Gly Asp Lys Lys Glu Ile
        195                 200                 205
Met His Arg Leu Leu His Asp Lys Asn Ser Ser Ser Gly Asn Tyr Ile
    210                 215                 220
Val Ile Pro Ile Val Gly Lys Gly Gly Ile Gly Lys Thr Thr Leu Ala
225                 230                 235                 240
Gln Leu Ile Tyr Asn Asp Glu Glu Val Asp Val Phe Ser Leu Lys Ala
                245                 250                 255
Trp Val Tyr Val Ser Gln Val Phe Asp Ala Lys Arg Val Thr Thr Ser
            260                 265                 270
Ile Leu Asn Ser Ile Thr Gly Glu Ala Asn Ser Phe Cys Asp Leu Asn
        275                 280                 285
Gln Ala Gln Val Arg Leu Gln Gln Val Leu Arg Asn Lys Arg Phe Leu
    290                 295                 300
Ile Ile Leu Asp Asp Met Trp Ser Val Met Tyr Ser Asp Trp Asp Asp
305                 310                 315                 320
Leu Arg Thr Pro Leu Arg Ala Gly Ala Lys Gly Ser Arg Val Ile Val
                325                 330                 335
Thr Thr Arg Ile Gln Lys Val Ala Arg Ile Met Asn Pro Gln Met Asn
            340                 345                 350
Asn Met Ile Leu Leu Glu Cys Leu Ala Asp Asp Asp Cys Trp His Leu
        355                 360                 365
Ile Leu Gln His Ala Phe Asp Asp Ala Asn Ile Ala Val Ser Pro Asn
    370                 375                 380
Ile Ser Leu Met Arg Ala Glu Met Val Arg Ala Cys Lys Gly Ile Pro
385                 390                 395                 400
Leu Val Ala Lys Thr Leu Gly Gly Leu Leu Arg Thr Lys Val Glu Glu
                405                 410                 415
Glu Ser Glu Trp Gln Glu Ser Leu Asn Thr Asp Ile Leu Ser Met Ser
            420                 425                 430
Lys Ser Arg Val Leu Gln Val Leu Glu Leu Ser Tyr Tyr Asp Leu Pro
        435                 440                 445
Ser His Leu Lys Arg Cys Phe Ser Tyr Cys Ala Ile Phe Pro Asn Asp
    450                 455                 460
His Ala Phe Thr Glu Glu Asp Val Ile Leu Leu Trp Met Ala Glu Gly
465                 470                 475                 480
Phe Leu Pro Glu Asn Val Gly Asn Lys Arg Ile Glu Asp Ile Gly His
                485                 490                 495
Glu Tyr Phe Gln Glu Leu Val Ser Arg Ser Phe Phe Glu Pro Ser Thr
            500                 505                 510

Lys Gly Ser Lys Trp Phe Lys Met His Asn Leu Tyr Ser Asp Leu Ala
        515                 520                 525
Arg Gln Val Val Gly Asp Leu Trp Leu Val Met Lys Glu Cys Ser Val
    530                 535                 540
Arg Lys Asp Tyr Arg Ala Arg His Leu Ser Phe Ser Lys Leu Asp Glu
545                 550                 555                 560
Cys Glu Asp Leu Phe His Leu Lys Asn Ile Ser His Phe Arg Thr Phe
                565                 570                 575
Ala Gln Phe Asp Lys His Pro Ser Ser Ser Ser Arg Val Leu Glu Leu
            580                 585                 590
Ile Gly Tyr Phe Gln Tyr Leu Arg Val Leu Leu Leu Pro Ser Leu Gly
        595                 600                 605
Ile Thr Asn Phe Pro Glu Ser Ile Gly Asp Leu Lys Leu Leu Arg Tyr
    610                 615                 620
Leu Asn Leu Ser Tyr Asn Asp Phe Glu Glu Leu Pro Asn Ser Val Ser
625                 630                 635                 640
Asn Leu Val Asn Leu Gln Thr Leu Leu Leu Lys Gly Cys Val Lys Leu
                645                 650                 655
Gln Lys Leu Ser Thr Asn Met Arg Cys Leu Ile Asn Leu Arg His Leu
            660                 665                 670
Asp Ile Asp Gly Thr Ser Leu Asn Glu Met Pro Leu Gly Leu Gly Asn
        675                 680                 685
Leu Thr Asn Leu Gln Thr Leu His Arg Phe Val Leu Gly Ala Asn Ala
    690                 695                 700
Pro Ile Ile Lys Glu Leu Lys Asn Leu Lys Gln Leu Arg Gly Lys Leu
705                 710                 715                 720
Thr Ile Ala Gly Leu Glu Asn Leu Val Ser Ser Asn Asp Ala Gly Glu
                725                 730                 735
Ala Arg Leu Asn Glu Lys Ser Cys Leu His Glu Leu Glu Met Val Trp
            740                 745                 750
Glu Trp Gly Thr Phe Thr Asn Gly Ile Asp Asp Asn Thr Lys Ile Asp
        755                 760                 765
Val Leu Asp Gln Leu His Pro His Arg Ser Thr Lys Val Leu Thr Leu
    770                 775                 780
Arg Asn Tyr Lys Gly Leu Thr Phe Pro Ser Trp Leu Ser Asp Pro Ser
785                 790                 795                 800
Phe Thr Lys Met Val Val Ile Arg Leu Ile Gly Cys Glu Arg Cys Glu
                805                 810                 815
Ser Leu Pro Ser Leu Gly Lys Leu Pro Ser Leu Lys Glu Leu Val Val
            820                 825                 830
Gln Glu Met Arg Gly Leu Lys Ser Val Asp Asp Asp Phe Tyr Gly Val
        835                 840                 845
Gly Cys Ser Asn Pro Phe Pro Ala Leu Ile Thr Leu Lys Phe Lys Ser
    850                 855                 860
Met Phe Ser Trp Glu Ser Trp Leu Ile Ser Ser Ala Asp Thr Lys Val
865                 870                 875                 880
Phe Ser Asn Leu Gln Glu Leu Ser Ile Lys Asp Ser Pro Leu Leu Gln
                885                 890                 895
His Leu Ser Leu Pro Ser Cys Leu Pro Leu Leu Glu Thr Leu Glu Ile
            900                 905                 910
Gln His Cys Lys Lys Leu Asp Ala Ser Leu Pro Thr Leu Pro His Leu
        915                 920                 925
Gln Lys Leu Val Leu Glu Asp Cys Ser Ser Leu Phe Met Val Asp Gln
    930                 935                 940
Leu Ser Thr Thr Leu Arg Gln Leu Ala Ile Ser Asn Cys Arg Lys Leu
945                 950                 955                 960
Gln Leu Val Glu Phe Arg Arg Pro Cys Phe Ser Leu Asp Glu Asn Ile
                965                 970                 975
Ser Leu Glu Val Val Glu Val Val Asn Cys Gln Ser Leu Leu Asn Phe
            980                 985                 990
Phe Ser Arg Asp Gly Ile Glu Val Leu His His Val Pro Gly His Leu
        995                 1000                1005
Ser Gly Leu His Thr Pro Gly Gly Leu Gly Gly Arg Ile Leu Lys Cys

1010                1015                1020
Arg Val Ser Trp Gln Glu
1025                1030

<210> 278
<211> 29
<212> DNA
<213> Phytophthora infestans

<400> 278
aatcagatct acaatagcta catgatgca                                         29

<210> 279
<211> 24
<212> DNA
<213> Phytophthora infestans

<400> 279
ccttatctgg gaactactca caca                                              24

<210> 280
<211> 20
<212> DNA
<213> Solanum tuberosum

<400> 280
taagaaaggc aaggctgcat                                                   20

<210> 281
<211> 20
<212> DNA
<213> Solanum tuberosum

<400> 281
tcccttgggc aagttaacag                                                   20